# EUROPEAN PATENT SPECIFICATION

(11) **EP 4 146 649 B1**
(45) Date of publication and mention of the grant of the patent: **05.08.2026**
(21) Application number: 21800220.2
(22) Date of filing: 07.05.2021
(51) Int. Cl.: C07D 471/04, C07D 471/14, C07D 498/14, C07D 519/00, A61K 31/437, A61P 35/00

(54) **AZASPIRO[3.5]NONANE DERIVATIVES AS ANTI-APOPTOTIC B-CELL LYMPHOMA-2 (BCL-2) INHIBITORS FOR THE TREATMENT OF CANCER**
AZASPIRO[3.5]NONAN-DERIVATE ALS ANTI-APOPTOTISCHE B-ZELL-LYMPHOM-2 (BCL-2)-INHIBITOREN ZUR BEHANDLUNG VON KREBS
DÉRIVÉS D'AZASPIRO[3.5]NONANE EN TANT QU'INHIBITEURS ANTI-APOPTOTIQUES DU LYMPHOME 2 À CELLULES B (BCL-2) POUR LE TRAITEMENT DU CANCER

(30) Priority: 08.05.2020 US 202063021816 P; 18.11.2020 US 202063115471 P; 06.02.2021 US 202163146601 P
(43) Date of publication of application: 15.03.2023
(73) Proprietor: Fochon Biosciences, Ltd., Chongqing 401121 (CN)
(72) Inventor: ZHANG, Weipeng, Chongqing 401121 (CN); LIU, Hongbin, Chongqing 401121 (CN); TAN, Rui, Chongqing 401121 (CN); LI, Zhifu, Chongqing 401121 (CN); RONG, Yue, Chongqing 401121 (CN); LIU, Qihong, Chongqing 401121 (CN); CHEN, Zhifang, Chongqing 401121 (CN); CHEN, Ling, Chongqing 401121 (CN); XU, Hua, Chongqing 401121 (CN); ZHOU, Zuwen, Chongqing 401121 (CN); YU, Jinhua, Chongqing 401121 (CN); TAN, Haohan, Chongqing 401121 (CN); LIU, Bin, Chongqing 401121 (CN); WANG, Yunling, Chongqing 401121 (CN); YANG, Lijun, Chongqing 401121 (CN); HE, Chengxi, Chongqing 401121 (CN); JIANG, Lihua, Chongqing 401121 (CN); LIN, Shu, San Leandro, California 94577 (US); ZHAO, Xingdong, Chongqing 401121 (CN); WANG, Weibo, Moraga, California 94556 (US)
(74) Representative: Müller, Christian Stefan Gerd
(86) International application number: PCT/CN2021/092104
(87) International publication number: WO 2021/223736

(56) References cited:
- WO-A1-2019/210828
- WO-A1-2021/083135
- WO-A1-2021/110102
- WO-A2-2020/140005
- CN-A- 103 562 202
- CN-A- 106 749 233

## Description

### FIELD OF THE INVENTION

Provided are certain compounds or pharmaceutically acceptable salts thereof which can inhibit anti-apoptotic B-cell lymphoma-2 (Bcl-2) family proteins and their drug-resistant mutations, and may be useful for the treatment of hyper-proliferative diseases like cancer and inflammation, or immune and autoimmune diseases.

### BACKGROUND OF THE INVENTION

Hyper-proliferative diseases like cancer and inflammation are attracting the scientific community to provide therapeutic benefits. In this regard, efforts have been made to identify and target specific mechanisms that drive the disease initiation and progression.

Protein-protein interactions (PPIs) control many biological processes, such as cell proliferation, growth, differentiation, signal transduction and apoptosis. Abnormal regulation of PPIs leads to different diseases. Thus, PPIs represent an important class of molecular targets for novel human therapeutics.

The Bcl-2 family of proteins are central to the regulation of apoptosis, which are vital for proper tissue development and cellular homeostasis. Apoptosis occurs via activation of two different pathways. The extrinsic pathway, triggered by activation of the intrinsic pathway involves members of the Bcl-2 family of proteins. The Bcl-2 family proteins include anti-apoptotic proteins, such as Bcl-2, Bcl-X_{L} and Mcl-1, and pro-apoptotic proteins, including Bid, Bim, Bad, Bak and Bax.

Anti-apoptotic Bcl-2 family members are often found to be up-regulated in cancers and are associated with stage of disease and prognosis. Therefore, Bcl-2 proteins are under investigation as potential therapeutic drug targets which include, for example, Bcl-2 and Bcl-X_{L}. Expression of Bcl-2 proteins is an independent indicator of poor prognosis in tumors including chronic lymphocytic leukemia (CLL), prostate cancer, and small cell lung cancer (SCLC). In other tumors such as colorectal cancer, Bcl-X_{L} expression is linked to grade and stage, and in hepatocellular cancer, Bcl-X_{L} expression is an independent marker of poorer overall and disease-free survival. Venetolax, as a potent first-generation Bcl-2 inhibitor, selectively inhibits Bcl-2 by binding its key hydrophobic groove, which is the same site that sequesters its physiological ligands (BH3 domain-containing pro-apoptotic proteins), thus attenuating the tumor progression. However, acquired drug resistance eventually emerges in cancer patients after receiving first-generation Bcl-2 inhibitors, resulting in unmet new medical needs. It has been reported that the mutations in the drug-binding sites of Bcl-2, such as G101V, D103Y, F104L, F104C, etc., is one of the key mechanisms driving drug resistance.

Therefore, a compound having inhibitory activities against Bcl-2 family proteins and their drug-resistant mutations will be useful for the prevention or treatment of cancer. Although Bcl-2 inhibitors were disclosed in the arts, e.g. WO 2011149492, many suffer from short half-life or toxicity. Therefore, there is a need for new Bcl-2 inhibitors that have at least one advantageous property selected from solubility, drug-drug interactions, potency, stability, selectivity, toxicity, drug resistance, pharmacokinetics and pharmacodynamics properties as an alternative for the treatment of hyper-proliferative diseases. In this regard, a novel class of Bcl-2 inhibitors is provided herein.

WO 2019/210828 A1 discloses anti-apoptotic B-cell lymphoma-2 (Bc1-2) inhibitors for the treatment of cancer and autoimmune diseases.

### DISCLOSURE OF THE INVENTION

The scope of the claimed invention is defined by the claims. The following description refers to the disclosure of the present invention and not necessarily to the claimed matter.

References in the present description to methods of medical treatment or medical uses have to be understood as references to the compounds of the present invention for use in methods of medical treatment. Methods of medical treatment or medical uses are not encompassed by the claimed invention.

Disclosed herein are certain novel compounds, pharmaceutically acceptable salts thereof, and pharmaceutical compositions thereof, and their use as pharmaceuticals.

In one aspect, disclosed herein is a compound of formula (I), 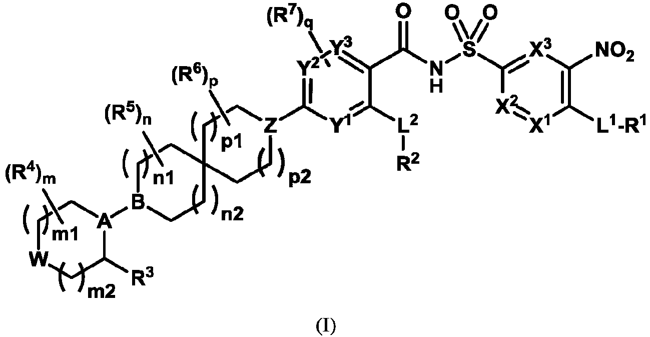 or a pharmaceutically acceptable salt thereof, wherein:
X¹ is selected from N and CR⁸;
X² is selected from N and CR⁹;
X³ is selected from N and CR¹⁰;
Y¹, Y² and Y³ are independently selected from N and CH;
A and B are independently selected from N and CH;
W is selected from -CR¹¹-, -NR¹²-, -O-, -S(O)ᵣ- and -S(O)(=NR¹²)-;
Z is selected from N and CH;
L¹ and L² are independently selected from a bond, -(CR^{C0}R^{D0})ᵤ-, -(CR^{C0}R^{D0})ᵤO(CR^{C0}R^{D0})ₜ-, -(CR^{C0}R^{D0})ᵤNR^{A0}(CR^{C0}R^{D0})ₜ- and -(CR^{C0}R^{D0})ᵤS(O)ᵣCCR^{C0}R^{D0})ₜ-;
R¹ is selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A1}R^{B1}, -OR^{A1}, -C(O)R^{A1}, -C(=NR^{E1})R^{A1}, -C(=N-OR^{B1})R^{A1}, -C(O)OR^{A1}, -OC(O)R^{A1}, -C(O)NR^{A1}R^{B1}, -NR^{A1}C(O)R^{B1}, -C(=NR^{E1})NR^{A1}R^{B1}, -NR^{A1}C(=NR^{E1})R^{B1}, -OC(O)NR^{A1}R^{B1}, -NR^{A1}C(O)OR^{B1}, -NR^{A1}C(O)NR^{A1}R^{B1}, -NR^{A1}C(S)NR^{A1}R^{B1}, -NR^{A1}C(=NR^{E1})NR^{A1}R^{B1}, -S(O)ᵣR^{A1}, -S(O)(=NR^{E1})R^{B1}, -N=S(O)R^{A1}R^{B1}, -S(O)₂OR^{A1}, -OS(O)₂R^{A1}, -NR^{A1}S(O)ᵣR^{B1}, -NR^{A1}S(O)(=NR^{E1})R^{B1}, -S(O)ᵣNR^{A1}R^{B1}, -S(O)(=NR^{E1})NR^{A1}R^{B1}, -NR^{A1}S(O)₂RNR^{A1}R^{B1}, -NR^{A1}S(O)(=NR^{E1})NR^{A1}R^{B1}, -P(O)R^{A1}R^{B1} and -P(O)(OR^{A1})(OR^{B1}), wherein alkyl, alkenyl, alkynyl, cycloalkyl, heterocyclyl, aryl and heteroaryl are each unsubstituted or substituted with at least one substituent, independently selected from R^{X};
R² and R³ are independently selected from aryl, heteroaryl and heterocyclyl, wherein aryl, heteroaryl and heterocyclyl are each unsubstituted or substituted with at least one substituent, independently selected from R^{X};
each R⁴ is independently selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A4}R^{B4}, -OR^{A4}, -C(O)R^{A4}, -C(=NR^{E4})R^{A4}, -C(=N-OR^{B4})R^{A4}, -C(O)OR^{A4}, -OC(O)R^{A4}, -C(O)NR^{A4}R^{B4}, -NR^{A4}C(O)R^{B4}, -C(=NR^{E4})NR^{A4}R^{B4}, -NR^{A4}C(=NR^{E4})R^{B4}, -OC(O)NR^{A4}R^{B4}, -NR^{A4}C(O)OR^{B4}, -NR^{A4}C(O)NR^{A4}R^{B4}, -NR^{A4}C(S)NR^{A4}R^{B4} -NR^{A4}C(=NR^{E4})NR^{A4}R^{B4}, -S(O)ᵣR^{A4}, -S(O)(=NR^{E4})R ^{B4}, -N=S(O)R^{A4}R^{B4}, -S(O)₂OR^{A4}, -OS(O)₂R^{A4}, -NR^{A4}S(O)ᵣR^{B4}, -NR^{A4}S(O)(=NR^{E4})R^{B4}, -S(O)ᵣNR^{A4}R^{B4}, -S(O)(=NR^{E4})NR^{A4}R^{B4}, -NR^{A4}S(O)₂NR^{A4}R^{B4}, -NR^{A4}S(O)(=NR^{E4})NR^{A4}R^{B4}, -P(O)R^{A4}R^{B4} and -P(O)(OR^{A4})(OR^{B4}), wherein alkyl, alkenyl, alkynyl, cycloalkyl, heterocyclyl, aryl and heteroaryl are each unsubstituted or substituted with at least one substituent, independently selected from R^{X};
or any two of R⁴ or "R⁴ and R¹¹" or "R⁴ and R¹²" together with the atoms to which they are attached form a C₃₋₁₀ cycloalkyl or heterocyclic ring of 4 to 12 members containing 1, 2 or 3 heteroatoms independently selected from oxygen, sulfur, nitrogen and phosphorus, and optionally substituted with 1, 2 or 3 R^{X} groups;
each R⁵ is independently selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A5}R^{B5}, -OR^{A5}, -C(O)R^{A5}, -C(=NR^{E5})R^{A5}, -C(=N-OR^{B5})R^{A5}, -C(O)OR^{A5}, -OC(O)R^{A5}, -C(O)NR^{A5}R^{B5}, -NR^{A5}C(O)R^{B5}, -C(=NR^{E5})NR^{A5}R^{B5}, -NR^{A5}C(=NR^{E5})R^{B5}, -OC(O)NR^{A5}R^{B5}, -NR^{A5}C(O)OR^{B5}, -NR^{A5}C(O)NR^{A5}R^{B5}, -NR^{A5}C(S)NR^{A5}R^{B5}, -NR^{A5}C(=NR^{E5})NR^{A5}R^{B5}, -S(O)ᵣR^{A5}, -S(O)(=NR^{E5})R^{B5}, -N=S(O)R^{A5}R^{B5}, -S(O)₂OR^{A5} , -OS(O)₂R^{A5}, -NR^{A5}S(O)ᵣR^{B5}, -NR^{A5}S(O)(=NR^{E5})R^{B5}, -S(O)ᵣNR^{A5}R^{B5}, -S(O)(=NR^{E5})NR^{A5}R^{B5}, -NR^{A5}S(O)₂NR^{A5}R^{B5}, -NR^{A5}S(O)(=NR^{E5})NR^{A5}R^{B5}, -P(O)R^{A5}R^{B5} and -P(O)(OR^{A5})(OR^{B5}), wherein alkyl, alkenyl, alkynyl, cycloalkyl, heterocyclyl, aryl and heteroaryl are each unsubstituted or substituted with at least one substituent, independently selected from R^{X};
or any two of R⁵ together with the carbon atoms to which they are attached form a C₃₋₁₀ cycloalkyl or heterocyclic ring of 4 to 12 members containing 1, 2 or 3 heteroatoms independently selected from oxygen, sulfur, nitrogen and phosphorus, and optionally substituted with 1, 2 or 3 R^{X} groups;
each R⁶ is independently selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A6}R^{B6}, -OR^{A6}, -C(O)R^{A6}, -C(=NR^{E6})R^{A6}, -C(=N-OR^{B6})R^{A6}, -C(O)OR^{A6}, -OC(O)R^{A6}, -C(O)NR^{A6}R^{B6}, -NR^{A6}C(O)R^{B6}, -C(=NR^{E6})NR^{A6}R^{B6}, -NR^{A6}C(=NR^{E6})R^{B6}, -OC(O)NR^{A6}R^{B6}, -NR^{A6}C(O)OR^{B6}, -NR^{A6}C(O)NR^{A6}R^{B6}, -NR^{A6}C(S)NR^{A6}R^{B6}, -NR^{A6}C(=NR^{E6})NR^{A6}R^{B6}, -S(O)ᵣR^{A6}, -S(O)(=NR^{E6})R^{B6}, -N=S(O)R^{A6}R^{B6}, -S(O)₂OR^{A6}, -OS(O)₂R^{A6}, -NR^{A6}S(O)ᵣR^{B6}, -NR^{A6}S(O)(=NR^{E6})R^{B6}, -S(O)ᵣNR^{A6}R^{B6}, -S(O)(=NR^{E6})NR^{A6}R^{B6}, -NR^{A6}S(O)₂NR^{A6}R^{B6}, -NR^{A6}S(O)(=NR^{E6})NR^{A6}R^{B6}, -P(O)R^{A6}R^{B6} and -P(O)(OR^{A6})(OR^{B6}), wherein alkyl, alkenyl, alkynyl, cycloalkyl, heterocyclyl, aryl and heteroaryl are each unsubstituted or substituted with at least one substituent, independently selected from R^{X};
or any two of R⁶ together with the carbon atoms to which they are attached form a C₃₋₁₀ cycloalkyl or heterocyclic ring of 4 to 12 members containing 1, 2 or 3 heteroatoms independently selected from oxygen, sulfur, nitrogen and phosphorus, and optionally substituted with 1, 2 or 3 R^{X} groups;
each R⁷ is independently selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A7}R^{B7}, -OR^{A7}, -C(O)R^{A7}, -C(=NR^{E7})R^{A7}, -C(=N-OR^{B7})R^{A7}, -C(O)OR^{A7}, -OC(O)R^{A7}, -C(O)NR^{A7}R^{B7}, -NR^{A7}C(O)R^{B7}, -C(=NR^{E7})NR^{A7}R^{B7}, -NR^{A7}C(=NR^{E7})R^{B7}, -OC(O)NR^{A7}R^{B7}, -NR^{A7}C(O)OR^{B7}, -NR^{A7}C(O)NR^{A7}R^{B7}, -NR^{A7}C(S)NR^{A7}R^{B7}, -NR^{A7}C(=NR^{E7})NR^{A7}R^{B7}, -S(O)ᵣR^{A7}, -S(O)(=NR^{E7})R^{B7}, -N=S(O)R^{A7}R^{B7}, -S(O)₂OR^{A7}, -OS(O)₂R^{A7}, -NR^{A7}S(O)ᵣR^{B7}, -NR^{A7}S(O)(=NR^{E7})R^{B7}, -S(O)ᵣNR^{A7}R^{B7}, -S(O)(=NR^{E7})NR^{A7}R^{B7}, -NR^{A7}S(O)₂NR^{A7}R^{B7}, -NR^{A7}S(O)(=NR^{E7})NR^{A7}R^{B7}, -P(O)R^{A7}R^{B7} and -P(O)(OR^{A7})(OR^{B7}), wherein alkyl, alkenyl, alkynyl, cycloalkyl, heterocyclyl, aryl and heteroaryl are each unsubstituted or substituted with at least one substituent, independently selected from R^{X};
R⁸ is selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A8}R^{B8}, -OR^{A8}, -C(O)R^{A8}, -C(=NR^{EB})R^{AB}, -C(=N-OR^{B8})R^{A8}, -C(O)OR^{A8}, -OC(O)R^{A8}, -C(O)NR^{A8}R^{B8}, -NR^{A8}C(O)R^{B8}, -C(=NR^{E8})NR^{A8}R^{B8}, -NR^{A8}C(=NR^{E8})R^{B8}, -OC(O)NR^{A8}R^{B8}, -NR^{AB}C(O)OR^{B8}, -NR^{A8}C(O)NR^{A8}R^{B8}, -NR^{A8}C(S)NR^{A8}R^{B8}, -NR^{A8}C(=NR^{E8})NR^{A8}R^{B8}, -S(O)ᵣR^{A8}, -S(O)(=NR^{E8})R^{B8}, -N=S(O)R^{A8}R^{B8}, -S(O)₂OR^{A8}, -OS(O)₂R^{A8}, -NR^{A8}S(O)ᵣR^{B8}, -NR^{A8}S(O)(=NR^{E8})R^{B8}, -S(O)ᵣNR^{A8}R^{B8}, -S(O)(=NR^{E8})NR^{A8}R^{B8}, -NR^{A8}S(O)₂NR^{A8}R^{B8}, -NR^{A8}S(O)(=NR^{E8})NR^{A8}R^{B8}, -P(O)R^{A8}R^{B8} and -P(O)(OR^{A8})(OR^{B8}), wherein alkyl, alkenyl, alkynyl, cycloalkyl, heterocyclyl, aryl and heteroaryl are each unsubstituted or substituted with at least one substituent, independently selected from R^{X};
or R¹ and R⁸ together with the atoms to which they are attached form a C₅₋₁₀cycloalkyl or heterocyclic ring of 5 to 12 members containing 1, 2 or 3 heteroatoms independently selected from oxygen, sulfur, nitrogen and phosphorus, and optionally substituted with 1, 2 or 3 R^{X} groups;
R⁹ is selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A9}R^{B9}, -OR^{A9}, -C(O)R^{A9}, -C(=NR^{E9})R^{A9}, -C(=N-OR^{B9})R^{A9}, -C(O)OR^{A9}, -OC(O)R^{A9}, -C(O)NR^{A9}R^{B9}, -NR^{A9}C(O)R^{B9}, -C(=NR^{E9})NR^{A9}R^{B9}, -NR^{A9}C(=NR^{E9})R^{B9}, -OC(O)NR^{A9}R^{B9}, -NR^{A9}C(O)OR^{B9}, -NR^{A9}C(O)NR^{A9}R^{B9}, -NR^{A9}C(S)NR^{A9}R^{B9}, -NR^{A9}C(=NR^{E9})NR^{A9}R^{B9}, -S(O)ᵣR^{A9}, -S(O)(=NR^{E9})R^{B9}, -N=S(O)R^{A9}R^{B9}, -S(O)₂OR^{A9}, -OS(O)₂R^{A9}, -NR^{A9}S(O)ᵣR^{B9}, -NR^{A9}S(O)(=NR^{E9})R^{B9}, -S(O)ᵣNR^{A9}R^{B9}, -S(O)(=NR^{E9})NR^{A9}R^{B9}, -NR^{A9}S(O)₂NR^{A9}R^{B9}, -NR^{A9}S(O)(=NR^{E9})NR^{A9}R^{B9}, -P(O)R^{A9}R^{B9} and -P(O)(OR^{A9})(OR^{B9}), wherein alkyl, alkenyl, alkynyl, cycloalkyl, heterocyclyl, aryl and heteroaryl are each unsubstituted or substituted with at least one substituent, independently selected from R^{X};
R¹⁰ is selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A10}R^{B10}, -OR^{A10}, -C(O)R^{A10}, -C(=NR^{E10})R^{A10}, -C(=N-OR^{B10})R^{A10}, -C(O)OR^{A10}, -OC(O)R^{A10}, -C(O)NR^{A10}R^{B10}, -NR^{A10}C(O)R^{B10}, -C(=NR^{E10})NR^{A10}R^{B10}, -NR^{A10}C(=NR^{E10})R^{B10}, -OC(O)NR^{A10}R^{B10}, -NR^{A10}C(O)OR^{B10}, -NR^{A10}NR^{A10}R^{B10}, -NR^{A10}C(S)NR^{A10}R^{B10}, -NR^{A10}C(=NR^{E10})NR^{A10}R^{B10}, -S(O)ᵣR^{A10}, -S(O)(=NR^{E10})R^{B10}, -N=S(O)R^{A10}R^{B10}, -S(O)₂OR^{A10}, -OS(O)₂R^{A10}, -NR^{A10}S(O)ᵣR^{B10}, -NR^{A10}S(O)(=NR^{E10})R^{B10}, -S(O)ᵣNR^{A10}R^{B10} -S(O)(=NR^{E10})NR^{A10}R^{B10}, -NR^{A10}S(O)₂NR^{A10}R^{B10}, -NR^{A10}S(O)(=NR^{E10})NR^{A10}R^{B10}, -P(O)R^{A10}R^{B10} and -P(O)(OR^{A10})(OR^{B10}), wherein alkyl, alkenyl, alkynyl, cycloalkyl, heterocyclyl, aryl and heteroaryl are each unsubstituted or substituted with at least one substituent, independently selected from R^{X};
R¹¹ is selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A11}R^{B11}, -OR^{A11}, -C(O)R^{A11}, -C(=NR^{E11})R^{A11}, -C(=N-OR^{B11})R^{A11}, -C(O)OR^{A11}, -OC(O)R^{A11}, -C(O)NR^{A11}R^{B11}, -NR^{A11}C(O)R^{B11}, -C(=NR^{E11})NR^{A11}R^{B11}, -NR^{A11}C(=NR^{E11})R^{B11}, -OC(O)NR^{A11}R^{B11}, -NR^{A11}C(O)OR^{B11}, -NR^{A11}C(O)NR^{A11}R^{B11}, -NR^{A11}C(S)NR^{A11}R^{B11}, -NR^{A11}C(=NR^{E11})NR^{A11}R^{B11}, -S(O)ᵣR^{A11}, -S(O)(=NR^{E11})R^{B11}, -N=S(O)R^{A11}R^{B11}, -S(O)₂OR^{A11}, -OS(O)₂R^{A11}, -NR^{A11}S(O)ᵣR^{B11}, -NR^{A11}S(O)(=NR^{E11})R^{B11}, -S(O)ᵣNR^{A11}R^{B11}, -S(O)(=NR^{E11})NR^{A11}R^{B11}, -NR^{A11}S(O)₂NR^{A11}R^{B11}, -NR^{A11}S(O)(=NR^{E11})NR^{A11}R^{B11}, -P(O)R^{A11}R^{B11} and -P(O)(OR^{A11})(OR^{B11}), wherein alkyl, alkenyl, alkynyl, cycloalkyl, heterocyclyl, aryl and heteroaryl are each unsubstituted or substituted with at least one substituent, independently selected from R^{X};
R¹² is selected from hydrogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, -C(O)R^{A12}, -C(-NR^{E12})R^{A12}, -C(-N-OR^{B12})R^{A12}, -C(O)OR^{A12}, -C(O)NR^{A12}R^{B12}, -C(=NR^{E12})NR^{A12}R^{B12}, -S(O)ᵣR^{A12}, -S(O)(=NR^{E12})R^{B12}, -S(O)₂OR^{A12}, -S(O)ᵣNR^{A12}R^{B12}, -S(O)(=NR^{E12})NR^{A12}R^{B12}, -P(O)R^{A12}R^{B12} and -P(O)(OR^{A12})(OR^{B12}), wherein alkyl, alkenyl, alkynyl, cycloalkyl, heterocyclyl, aryl and heteroaryl are each unsubstituted or substituted with at least one substituent, independently selected from R^{X};
each R^{A0}, R^{A1}, R^{A4}, R^{A5}, R^{A6}, R^{A7}, R^{A8}, R^{A9}, R^{A10}, R^{A11}, R^{A12}, R^{B1}, R^{B4}, R^{B5}, R^{B6}, R^{B7}, R^{B8}, R^{B9}, R^{B10}, R^{B11} and R^{B12} are independently selected from hydrogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl and heteroaryl-C₁₋₄ alkyl, wherein alkyl, alkenyl, alkynyl, cycloalkyl, heterocyclyl, aryl and heteroaryl are each unsubstituted or substituted with at least one substituent, independently selected from R^{X};
or "R^{A1} and R^{B1}" or "R^{A4} and R^{B4}" or "R^{A5} and R^{B5}" or "R^{A6} and R^{B6}" or "R^{A7} and R^{B7}" or "R^{A8} and R^{B8}" or "R^{A9} and R^{B9}" or "R^{A10} and R^{B10}" or "R^{A11}and R^{B11}" or "R^{A12} and R^{B12}" together with the atom(s) to which they are attached form a heterocyclic ring of 4 to 12 members containing 0, 1 or 2 additional heteroatoms independently selected from oxygen, sulfur, nitrogen and phosphorus and optionally substituted with 1, 2 or 3 R^{X} groups;
each R^{C0} and R^{D0} are independently selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl and heteroaryl-C₁₋₄ alkyl, wherein alkyl, alkenyl, alkynyl, cycloalkyl, heterocyclyl, aryl and heteroaryl are each unsubstituted or substituted with at least one substituent, independently selected from R^{X};
or each "R^{C0} and R^{D0}" together with the carbon atom(s) to which they are attached form a 3- to 12-membered ring containing 0, 1 or 2 heteroatoms independently selected from oxygen, sulfur and nitrogen, and optionally substituted with 1, 2 or 3 R^{X} groups;
each R^{E1}, R^{E4}, R^{E5}, R^{E6}, R^{E7}, R^{E8}, R^{E9}, R^{E10}, R^{E11} and R^{E12} are independently selected from hydrogen, C₁₋₁₀ alkyl, CN, NO₂, -OR^{a1}, -SR^{a1}, -S(O)ᵣR^{a1}, -C(O)R^{a1}, -C(O)OR^{a1}, -C(O)NR^{a1}R^{b1} and -S(O)ᵣNR^{a1}R^{b1}, wherein alkyl is unsubstituted or substituted with at least one substituent, independently selected from R^{X};
each R^{X} is independently selected from hydrogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, halogen, CN, NO₂, -(CR^{c1}R^{d1})ₜNR^{a1}R^{b1}, -(CR^{c1}R^{d1})ₜO_{R}^{b1}, -(CR^{c1}R^{d1})ₜC(O)R^{a1}, -(CR^{c1}R^{d1})ₜC(=NR^{e1})R^{a1}, -(CR^{c1}R^{d1})ₜC(=N-OR^{b1})R^{a1}, -(CR^{c1}R^{d1})ₜC(O)OR^{b1}, -(CR^{c1}R^{d1})ₜOC(O)R^{b1}, -(CR^{c1}R^{d1})ₜC(O)NR^{a1}R^{b1}, -(CR^{c1}R^{d1})ₜNR^{a1}C(O)R^{b1}, -(CR^{c1}R^{d1} )ₜC(=NR^{e1})NR^{a1}R^{b1}, -(CR^{c1}R^{d1})ₜNR^{a1}C(=NR^{e1})R^{b1}, -(CR^{c1}R^{d1})ₜOC(O)NR^{a1}R^{b1}, -(CR^{c1}R^{d1})ₜNR^{a1}C(O)OR^{b1}, -(CR^{c1}R^{a1})_{c}NR^{a1}C(O)NR^{a1}R^{b1}, -(CR^{c1}R^{d1})ₜNR^{a1}C(S)NR^{a1}R^{b1}, -(CR^{c1}R^{d1})ₜNR^{a1}C(=NR^{e1})NR^{a1}R^{b1}, -(CR^{c1}R^{d1})ₜS(O)ᵣR^{b1}, -(CR^{c1}R^{d1})ₜS(O)(=NR^{e1})R^{b1}, -(CR^{c1}R^{d1})ₜN=S(O)R^{a1}R^{b1}, -(CR^{c1}R^{d1})ₜS(O)₂OR^{b1}, -(CR^{c1}R^{d1})ₜOS(O)₂R^{b1}, -(CR^{c1}R^{d1})ₜNR^{a1}S(O)ᵣR^{b1}, -(CR^{c1}R^{d1})ₜNR^{a1}S(O)(=NR^{e1})R^{b1}, -(CR^{c1}R^{d1})ₜS(O)ᵣNR^{a1}R^{b1}, -(CR^{c1}R^{d1})ₜS(O)(=NR^{e1})NR^{a1}R^{b1}, -(CR^{c1}R^{d1})ₜNR^{a1}S(O)₂NR^{a1}R^{b1}, -(CR^{c1}R^{d1})ₜNR^{a1}S(O)(=NR^{e1})NR^{a1}R^{b1}, -(CR^{c1}R^{d1})ₜP(O)R^{a1}R^{b1} and -(CR^{c1}R^{d1})ₜP(O)(OR^{a1})(OR^{b1}), wherein alkyl, alkenyl, alkynyl, cycloalkyl, heterocyclyl, aryl and heteroaryl are each unsubstituted or substituted with at least one substituent, independently selected from R^{Y};
wherein
when X¹ is CR⁸; X² is CR⁹, X³ is CR¹⁰, R¹ and R⁸ together with the atoms to which they are attached form a C₅₋₁₀ cycloalkyl or heterocyclic ring of 5 to 12 members containing 1, 2 or 3 heteroatoms independently selected from oxygen, sulfur, nitrogen and phosphorus, and optionally substituted with 1, 2 or 3 R^{X} groups and L¹ is selected from -(CR^{C0}R^{D0})ᵤNR^{A0}(CR^{C0}R^{D0})ₜ- , L² is selected from a bond, -(CR^{C0}R^{D0})ᵤO(CR^{C0}R^{D0})ₜ-, -(CR^{C0}R^{D0})ᵤS(O)ᵣ(CR^{C0}R^{D0})ₜ-, wherein when L² is -O- and R² is 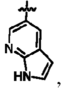 each substituent R^{X} of R² is selected from C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, halogen, CN, NO₂, -(CR^{c1}R^{d1})ₜNR^{a1}R^{b1}, -(CR^{c1}R^{d1})ₜOR^{b1}, -(CR^{c1}R^{d1})ₜC(O)R^{a1}, -(CR^{c1}R^{d1})ₜC(=NR^{e1})R^{a1}, -(CR^{c1}R^{d1})ₜC(=N-OR^{b1})R^{a1}, -(CR^{c1}R^{a1})ₜC(O)OR^{b1}, -(CR^{c1}R^{d1})ₜOC(O)R^{b1}, -(CR^{c1}R^{d1})ₜC(O)NR^{a1}R^{b1}, -(CR^{c1}R^{d1})ₜNR^{a1}C(O)R^{b1}, -(CR^{c1}R^{d1})ₜC(=NR^{e1})NR^{a1}R^{b1}, -(CR^{c1}R^{d1})ₜNR^{a1}C(=NR^{e1})R^{b1}, -(CR^{c1}R^{d1})ₜOC(O)NR^{a1}R^{b1}, -(CR^{c1}R^{d1})ₜNR^{a1}C(O)OR^{b1}, -(CR^{c1}R^{d1})ₜNR^{a1}C(O)NR^{a1}R^{b1}, -(CR^{c1}R^{d1})ₜNR^{a1}C(S)NR^{a1}R^{b1}, -(CR^{c1}R^{d1})ₜNR^{a1}C(=NR^{e1})NR^{a1}R^{b1}, -(CR^{c1}R^{d1})ₜS(O)ᵣR^{b1}, -(CR^{c1}R^{d1})ₜS(O)(=NR^{e1})R^{b1}, -(CR^{c1}R^{d1})ₜN=S(O)R^{a1}R^{b1}, -(CR^{c1}R^{d1})ₜS(O)₂OR^{b1}, -(CR^{c1}R^{d1})ₜOS(O)₂R^{b1}, -(CR^{c1}R^{d1})ₜNR^{a1}S(O)ᵣR^{b1}, -(CR^{c1}R^{d1})ₜNR^{a1}S(O)(=NR^{e1})R^{b1}, -(CR^{c1}R^{d1})ₜS(O)ᵣNR^{a1}R^{b1}, -(CR^{c1}R^{d1})ₜS(O)(=NR^{e1})NR^{a1}R^{b1}, -(CR^{c1}R^{d1})ₜNR^{a1}S(O)₂NR^{a1}R^{b1}, -(CR^{c1}R^{d1})ₜNR^{a1}S(O)(=NR^{e1})NR^{a1}R^{b1}, -(CR^{c1}R^{d1})ₜP(O)R^{a1}R^{b1} and -(CR^{c1}R^{d1})ₜP(O)(OR^{a1})(OR^{b1}), wherein alkyl, alkenyl, alkynyl, cycloalkyl, heterocyclyl, aryl and heteroaryl are each unsubstituted or substituted with at least one substituent, independently selected from R^{Y};
when X¹ is CR⁸; X² is CR⁹, X³ is CR¹⁰ and R¹ and R⁸ together with the atoms to which they are attached do not form a ring, A is CH. W is selected from -CR¹¹-, and L² is selected from -(CR^{C0}R^{D0})ᵤO(CR^{C0}R^{D0})ₜ- and -(CR^{C0}R^{D0})ᵤS(O)ᵣ(CR^{C0}R^{D0})ₜ-;
each R^{a1} and each R^{b1} are independently selected from hydrogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl and heteroaryl-C₁₋₄ alkyl, wherein alkyl, alkenyl, alkynyl, cycloalkyl, heterocyclyl, aryl and heteroaryl are each unsubstituted or substituted with at least one substituent, independently selected from R^{Y};
or R^{a1} and R^{b1} together with the atom(s) to which they are attached form a heterocyclic ring of 4 to 12 members containing 0, 1 or 2 additional heteroatoms independently selected from oxygen, sulfur, nitrogen and phosphorus, and optionally substituted with 1, 2 or 3 R^{Y} groups;
each R^{c1} and each R^{d1} are independently selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl and heteroaryl-C₁₋₄ alkyl, wherein alkyl, alkenyl, alkynyl, cycloalkyl, heterocyclyl, aryl and heteroaryl are each unsubstituted or substituted with at least one substituent, independently selected from R^{Y};
or R^{c1} and R^{d1} together with the carbon atom(s) to which they are attached form a ring of 3 to 12 members containing 0, 1 or 2 heteroatoms independently selected from oxygen, sulfur and nitrogen, and optionally substituted with 1, 2 or 3 R^{Y} groups;
each R^{e1} is independently selected from hydrogen, C₁₋₁₀ alkyl, C₃₋₁₀ cycloalkyl, C₃₋₁₀ cycloalkyl-C₁₋₄ alkyl, CN, NO₂, -OR^{a2}, -SR^{a2}, -S(O)ᵣR^{a2}, -C(O)R^{a2}, -C(O)OR^{a2}, -S(O)ᵣNR^{a2}R^{b2} and -C(O)NR^{a2}R^{b2};
each R^{Y} is independently selected from C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, halogen, CN, NO₂, -(CR^{c2}R^{d2})ₜNR^{a2}R^{b2}, -(CR^{c2}R^{d2})ₜOR^{b2}, -(CR^{c2}R^{d2})ₜC(O)R^{a2}, -(CR^{c2}R^{d2})ₜC(=NR^{e2})R^{a2}, -(CR^{c2}R^{d2})ₜC(=N-OR^{b2})R^{a2}, -(CR^{c2}R^{d2})ₜC(O)OR^{b2}, -(CR^{c2}R^{d2})ₜOC(O)R^{b2}, -(CR^{c2}R^{d2})ₜC(O)NR^{a2}R^{b2}, -(CR^{c2}R^{d2})ₜNR^{a2}C(O)R^{b2}, -(CR^{c2}R^{d2})ₜC(=NR^{e2})NR^{a2}R^{b2}, -(CR^{c2}R^{d2})ₜNR^{a2}C(=NR^{e2})R^{b2}, -(CR^{c2}R^{d2})ₜOC(O)NR^{a2}R^{b2}, -(CR^{c2}R ^{d2})ₜNR^{a2}C(O)OR^{b2}, -(CR^{c2}R^{d2})ₜNR^{a2}C(O)NR^{a2}R^{b2}, -(CR^{c2}R^{d2})ₜNR^{a2}C(S)NR^{a2}R^{b2}, -(CR^{c2}R^{d2})ₜNR^{a2}C(=NR^{e2})NR^{a2}R^{b2}, -(CR^{c2}R^{d2})ₜS(O)ᵣR^{b2}, -(CR^{c2}R^{d2})ₜS(O)(=NR^{e2})R^{b2}, -(CR^{c2}R^{d2})ₜN=S(O)R^{a2}R^{b2}, -(CR^{c2}R^{d2})ₜS(O)₂OR^{b2}, -(CR^{c2}R^{d2})ₜOS(O)2R^{b2}, -(CR^{c2}R^{d2})ₜNR^{a2}S(O)ᵣR^{b2}, -(CR^{c2}R^{d2})ₜNR^{a2}S(O)(=NR^{e2})R^{b2}, -(CR^{c2}R^{d2})ₜS(O)ᵣNR^{a2}R^{b2}, -(CR^{c2}R^{d2})ₜS(O)(=NR^{e2})NR^{a2}R^{b2}, -(CR^{c2}R^{d2})ₜNR^{a2}S(O)₂NR^{a2}R^{b2}, -(CR^{c2}R^{d2})ₜNR^{a2}S(O)(=NR^{e2})NR^{a2}R^{b2}, -(CR^{c2}R^{d2})ₜP(O)R^{a2}R^{b2} and -(CR^{c2}R^{d2})ₜP(O)(OR^{a2})(OR^{b2}), wherein alkyl, alkenyl, alkynyl, cycloalkyl, heterocyclyl, aryl and heteroaryl are each unsubstituted or substituted with at least one substituent, independently selected from OH, CN, amino, halogen, C₁₋₁₀ alkyl, C₂₋₁₀ alkenyl, C₂₋₁₀ alkynyl, C₃₋₁₀ cycloalkyl, C₁₋₁₀ alkoxy, C₃₋₁₀ cycloalkoxy, C₁₋₁₀ alkylthio, C₃₋₁₀ cycloalkylthio, C₁₋₁₀ alkylamino, C₃₋₁₀ cycloalkylamino and di(C₁₋₁₀ alkyl)amino;
each R^{a2} and each R^{b2} are independently selected from hydrogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, C₁₋₁₀ alkoxy, C₃₋₁₀ cycloalkoxy, C₁₋₁₀ alkylthio, C₃₋₁₀ cycloalkylthio, C₁₋₁₀ alkylamino, C₃₋₁₀ cycloalkylamino, di(C₁₋₁₀ alkyl)amino, heterocyclyl, heterocyclyl-C₁₋₄ alkyl, aryl, aryl-C₁₋₄ alkyl, heteroaryl and heteroaryl-C₁₋₄ alkyl, wherein alkyl, alkenyl, alkynyl, cycloalkyl, alkoxy, cycloalkoxy, alkylthio, cycloalkylthio, alkylamino, cycloalkylamino, heterocyclyl, aryl and heteroaryl are each unsubstituted or substituted with at least one substituent, independently selected from halogen, CN, C₁₋₁₀ alkyl, C₂₋₁₀ alkenyl, C₂₋₁₀ alkynyl, C₃₋₁₀ cycloalkyl, OH, C₁₋₁₀ alkoxy, C₃₋₁₀ cycloalkoxy, C₁₋₁₀ alkylthio, C₃₋₁₀ cycloalkylthio, amino, C₁₋₁₀ alkylamino, C₃₋₁₀ cycloalkylamino and di(C₁₋₁₀ alkyl)amino;
or R^{a2} and R^{b2} together with the atom(s) to which they are attached form a heterocyclic ring of 4 to 12 members containing 0, 1 or 2 additional heteroatoms independently selected from oxygen, sulfur, nitrogen and phosphorus, and optionally substituted with 1 or 2 substituents, independently selected from halogen, CN, C₁₋₁₀ alkyl, C₂₋₁₀ alkenyl, C₂₋₁₀ alkynyl, C₃₋₁₀ cycloalkyl, OH, C₁₋₁₀ alkoxy, C₃₋₁₀ cycloalkoxy, C₁₋₁₀ alkylthio, C₃₋₁₀ cycloalkylthio, amino, C₁₋₁₀ alkylamino, C₃₋₁₀ cycloalkylamino and di(C₁₋₁₀ alkyl)amino;
each R^{c2} and each R^{d2} are independently selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, C₁₋₁₀ alkoxy, C₃₋₁₀ cycloalkoxy, C₁₋₁₀ alkylthio, C₃₋₁₀ cycloalkylthio, C₁₋₁₀ alkylamino, C₃₋₁₀ cycloalkylamino, di(C₁₋₁₀ alkyl)amino, heterocyclyl, heterocyclyl-C₁₋₄ alkyl, aryl, aryl-C₁₋₄ alkyl, heteroaryl and heteroaryl-C₁₋₄ alkyl, wherein alkyl, alkenyl, alkynyl, cycloalkyl, alkoxy, cycloalkoxy, alkylthio, cycloalkylthio, alkylamino, cycloalkylamino, heterocyclyl, aryl and heteroaryl are each unsubstituted or substituted with at least one substituent, independently selected from halogen, CN, C₁₋₁₀ alkyl, C₂₋₁₀ alkenyl, C₂₋₁₀ alkynyl, C₃₋₁₀ cycloalkyl, OH, C₁₋₁₀ alkoxy, C₃₋₁₀ cycloalkoxy, C₁₋₁₀ alkylthio, C₃₋₁₀ cycloalkylthio, amino, C₁₋₁₀ alkylamino, C₃₋₁₀ cycloalkylamino and di(C₁₋₁₀ alkyl)amino;
or R^{c2} and R^{d2} together with the carbon atom(s) to which they are attached form a ring of 3 to 12 members containing 0, 1 or 2 heteroatoms independently selected from oxygen, sulfur and nitrogen, and optionally substituted with 1 or 2 substituents, independently selected from halogen, CN, C₁₋₁₀ alkyl, C₂₋₁₀ alkenyl, C₂₋₁₀ alkynyl, C₃₋₁₀ cycloalkyl, OH, C₁₋₁₀ alkoxy, C₃₋₁₀ cycloalkoxy, C₁₋₁₀ alkylthio, C₃₋₁₀ cycloalkylthio, amino, C₁₋₁₀ alkylamino, C₃₋₁₀ cycloalkylamino and di(C₁₋₁₀ alkyl)amino;
each R^{e2} is independently selected from hydrogen, CN, NO₂, C₁₋₁₀ alkyl, C₃₋₁₀ cycloalkyl, C₃₋₁₀ cycloalkyl-C₁₋₄ alkyl, C₁₋₁₀ alkoxy, C₃₋₁₀ cycloalkoxy, -C(O)C₁₋₄ alkyl, -C(O)C₃₋₁₀ cycloalkyl, -C(O)OC₁₋₄ alkyl, -C(O)OC₃₋₁₀ cycloalkyl, -C(O)N(C₁₋₄ alkyl)₂, -C(O)N(C₃₋₁₀ cycloalkyl)₂, -S(O)₂C₁₋₄ alkyl, -S(O)₂C₃₋₁₀ cycloalkyl, -S(O)₂N(C₁₋₄ alkyl)₂ and -S(O)₂N(C₃₋₁₀ cycloalkyl)₂;
m, m1, m2, n, n1, n2, p, p1, p2 and q are independently selected from 0, 1, 2 and 3;
each r is independently selected from 0, 1 and 2;
each t is independently selected from 0, 1, 2, 3 and 4;
each u is independently selected from 0, 1, 2, 3 and 4.

In another aspect, disclosed herein is a compound of formula (I) or a pharmaceutically acceptable salt thereof, wherein at least one of X¹, X² and X³ is N.

In another aspect, disclosed herein is a compound of formula (I) or a pharmaceutically acceptable salt thereof,
wherein X³ is CH, A is N, B is CH, Z is N, Y¹, Y² and Y³ are CH, shown as formula (II), 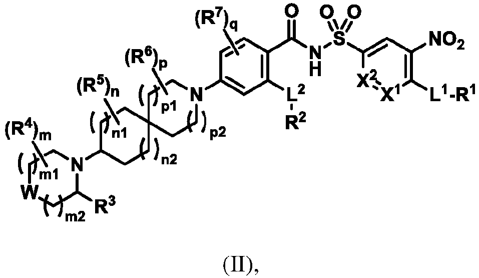
wherein
X¹ is selected from N and CR⁸; Y is selected from N and CR⁹, wherein at least one of X¹ and X² is N; W is selected from -CR¹¹-, -NR¹²-, -O- and -S(O)ᵣ-; L¹, L², R¹, R², R³, R⁴, R⁵, R⁶, R⁷, R⁸, R⁹, R¹¹, R¹², m, m1, m2, n, n1, n2, p, p1, p2 and q are as defined in formula (I).

In yet another aspect, the present disclosure provides pharmaceutical compositions comprising a compound of formula (I) or at least one pharmaceutically acceptable salt thereof and a pharmaceutically acceptable excipient.

In yet another aspect, the disclosure provides methods for modulating Bcl-2, comprising administering to a system or a subject in need thereof, a therapeutically effective amount of a compound of formula (I) or a pharmaceutically acceptable salt thereof or pharmaceutical compositions thereof, thereby modulating said Bcl-2.

In yet another aspect, disclosed is a method to treat, ameliorate or prevent a condition which responds to inhibition of Bcl-2 comprising administering to a system or subject in need of such treatment an effective amount of a compound of formula (I) or a pharmaceutically acceptable salt thereof or pharmaceutical compositions thereof, and optionally in combination with a second therapeutic agent, thereby treating said condition.

Alternatively, the present disclosure provides the use of a compound of formula (I) or a pharmaceutically acceptable salt thereof in the manufacture of a medicament for treating a condition mediated by Bcl-2. In particular embodiments, the compounds of the disclosure may be used alone or in combination with a second therapeutic agent to treat a condition mediated by Bcl-2.

Alternatively, disclosed is a compound of formula (I) or a pharmaceutically acceptable salt thereof for treating a condition mediated by Bcl-2.

Specifically, the condition herein includes but not limited to, an autoimmune disease, a transplantation disease, an infectious disease or a cell proliferative disorder.

Furthermore, the disclosure provides methods for treating a cell proliferative disorder, comprising administering to a system or subject in need of such treatment an effective amount of a compound of formula (I) or a pharmaceutically acceptable salt thereof or pharmaceutical compositions thereof, and optionally in combination with a second therapeutic agent, thereby treating said condition.

Alternatively, the present disclosure provides the use of a compound of formula (I) or a pharmaceutically acceptable salt thereof in the manufacture of a medicament for treating a cell-proliferative disorder. In particular examples, the compounds of the disclosure may be used alone or in combination with a chemotherapeutic agent to treat a cell proliferative disorder.

Specifically, the cell proliferative disorder disclosed herein includes but not limited to, lymphoma, osteosarcoma, melanoma, or a tumor of breast, renal, prostate, colorectal, thyroid, ovarian, pancreatic, neuronal, lung, uterine or gastrointestinal tumor.

In the above methods for using the compounds of the disclosure, a compound of formula (I) or a pharmaceutically acceptable salt thereof may be administered to a system comprising cells or tissues, or to a subject including a mammalian subject such as a human or animal subject.

### Certain Terminology

Unless defined otherwise, all technical and scientific terms used herein have the same meaning as is commonly understood by one of skill in the art to which the claimed subject matter belongs. In the event that there is a plurality of definitions for terms herein, those in this section prevail.

It is to be understood that the foregoing general description and the following detailed description are explanatory only and are not restrictive of any subject matter claimed. In this application, the use of the singular includes the plural unless specifically stated otherwise. It must be noted that, as used in the specification and the appended claims, the singular forms "a", "an" and "the" include plural referents unless the context clearly dictates otherwise. It should also be noted that use of "or" means "and/or" unless stated otherwise. Furthermore, use of the term "including" as well as other forms, such as "include", "includes", and "included" is not limiting. Likewise, use of the term "comprising" as well as other forms, such as "comprise", "comprises", and "comprised" is not limiting.

Unless otherwise indicated, conventional methods of mass spectroscopy, NMR, HPLC, IR and UV/Vis spectroscopy and pharmacology, within the skill of the art are employed. Unless specific definitions are provided, the nomenclature employed in connection with, and the laboratory procedures and techniques of, analytical chemistry, synthetic organic chemistry, and medicinal and pharmaceutical chemistry described herein are those known in the art. Standard techniques can be used for chemical syntheses, chemical analyses, pharmaceutical preparation, formulation, and delivery, and treatment of patients. Reactions and purification techniques can be performed e.g., using kits of manufacturer's specifications or as commonly accomplished in the art or as described herein. The foregoing techniques and procedures can be generally performed of conventional methods well known in the art and as described in various general and more specific references that are cited and discussed throughout the present specification. Throughout the specification, groups and substituents thereof can be chosen by one skilled in the field to provide stable moieties and compounds.

Where substituent groups are specified by their conventional chemical formulas, written from left to right, they equally encompass the chemically identical substituents that would result from writing the structure from right to left. As a non-limiting example, CH₂O is equivalent to OCH₂.

The term "substituted" means that a hydrogen atom is replaced by a substituent. It is to be understood that substitution at a given atom is limited by valency.

The term "Cᵢ₋ⱼ" or "i-j membered" used herein means that the moiety has i-j carbon atoms or i-j atoms. For example, "C₁₋₆ alkyl" means said alkyl has 1-6 carbon atoms. Likewise, C₃₋₁₀ cycloalkyl means said cycloalkyl has 3-10 carbon atoms.

When any variable (e.g. R) occurs at the structure of a compound over one time, it is defined independently at each case. Therefore, for example, if a group is substituted by 0-2 R, the group may be optionally substituted by at most two R and R has independent option at each case. Additionally, a combination of substituents and/or the variants thereof are allowed only if such a combination will result in a stable compound.

The expression "one or more" or "at least one" refers to one, two, three, four, five, six, seven, eight, nine or more.

Unless stated otherwise, the term "hetero" means heteroatom or heteroatom radical (i.e. a radical containing heteroatom). i.e. the atoms beyond carbon and hydrogen atoms or the radical containing such atoms. Preferably, the heteroatom(s) is independently selected from the group consisting of O, N, S, P and the like. In an embodiment wherein two or more heteroatoms are involved, the two or more heteroatoms may be the same, or part or all of the two or more heteroatoms may be different.

The term "alkyl", employed alone or in combination with other terms, refers to branched or straight-chain saturated aliphatic hydrocarbon groups having the specified number of carbon atoms. Unless otherwise specified, "alkyl" refers to C₁₋₁₀ alkyl. For example, C₁₋₆, as in "C₁₋₆ alkyl" is defined to include groups having 1, 2, 3, 4, 5, or 6 carbons in a linear or branched arrangement. For example, "C₁₋₈ alkyl" includes but is not limited to methyl, ethyl, n-propyl, i-propyl, n-butyl, t-butyl, i-butyl, pentyl, hexyl, heptyl, and octyl.

The term "cycloalkyl", employed alone or in combination with other terms, refers to a saturated monocyclic or multicyclic (e.g. bicyclic or tricyclic) hydrocarbon ring system, usually with 3 to 16 ring atoms. The ring atoms of cycloalkyl are all carbon and the cycloalkyl contains zero heteroatoms and zero double bonds. In a multicyclic cycloalkyl, two or more rings can be fused or bridged or spiro together. Examples of monocyclic ring systems include but are not limited to cyclopropyl, cyclobutyl, cyclopentyl, cyclohexyl, cycloheptyl, and cyclooctyl. The bridged cycloalkyl is a polycyclic ring system containing 3-10 carbon atoms, which contains one or two alkylene bridges, each alkylene bridge consisting of one, two, or three carbon atoms, each linking two non-adjacent carbon atoms of the ring system. Cycloalkyl can be fused with aryl or heteroaryl group. In some embodiments, cycloalkyl is benzocondensed. Representative examples of such bridged cycloalkyl ring systems include, but are not limited to, bicyclo[3.1.1]heptane, bicyclo[2.2.1]heptane, bicyclo[2.2.2]octane, bicyclo[3.2.2]nonane, bicyclo[3.3.1]nonane, bicyclo[4.2.1]nonane, tricyclo[3.3.1.03,7]nonane and tricyclo[3.3.1.13,7]decane (adamantane). The monocyclic or bridged cycloalkyl can be attached to the parent molecular moiety through any substitutable atom contained within the ring system.

The term "alkenyl", employed alone or in combination with other terms, refers to a non-aromatic hydrocarbon radical, straight, branched or cyclic, containing 2-10 carbon atoms and at least one carbon to carbon double bond. In some embodiments, the cyclic refers to monocyclic or multicyclic. In a multicyclic alkenyl, two or more rings can be fused or bridged or spiro together. In some embodiments, one carbon to carbon double bond is present, and up to four non-aromatic carbon-carbon double bonds may be present. Thus, "C₂₋₆ alkenyl" means an alkenyl radical having 2-6 carbon atoms. Alkenyl groups include but are not limited to ethenyl, propenyl, butenyl, 2-methylbutenyl and cyclohexenyl. The straight, branched or cyclic portion of the alkenyl group may contain double bonds and may be substituted if a substituted alkenyl group is indicated.

The term "alkynyl", employed alone or in combination with other terms, refers to a hydrocarbon radical, straight, branched or cyclic, containing 2-10 carbon atoms and at least one carbon to carbon triple bond. In some embodiments, up to three carbon-carbon triple bonds may be present. Thus, "C₂₋₆ alkynyl" means an alkynyl radical having 2-6 carbon atoms. Alkynyl groups include but are not limited to ethynyl, propynyl, butynyl, and 3-methylbutynyl. The straight, branched or cyclic portion of the alkynyl group may contain triple bonds and may be substituted if a substituted alkynyl group is indicated.

The term "halogen" (or "halo") refers to fluorine, chlorine, bromine and iodine.

The term "alkoxy", employed alone or in combination with other terms, refers to an alkyl as defined above, which is single bonded to an oxygen atom. The attachment point of an alkoxy radical to a molecule is through the oxygen atom. An alkoxy radical may be depicted as -O-alkyl. The term "C₁₋₁₀ alkoxy" refers to an alkoxy radical containing 1-10 carbon atoms, having straight or branched moieties. Alkoxy group includes but is not limited to, methoxy, ethoxy, propoxy, isopropoxy, butoxy, pentyloxy, hexyloxy, and the like.

The term "cycloalkoxy", employed alone or in combination with other terms, refers to cycloalkyl as defined above, which is single bonded to an oxygen atom. The attachment point of a cycloalkoxy radical to a molecule is through the oxygen atom. A cycloalkoxy radical may be depicted as -O-cycloalkyl. "C₃₋₁₀ cycloalkoxy" refers to a cycloalkoxy radical containing 3-10 carbon atoms. Cycloalkoxy can be fused with aryl or heteroaryl group. In some embodiments, cycloalkoxy is benzocondensed. Cycloalkoxy group includes but is not limited to, cyclopropoxy, cyclobutoxy, cyclopentyloxy, cyclohexyloxy, and the like.

The term "alkylthio", employed alone or in combination with other terms, refers to an alkyl radical as defined above, which is single bonded to a sulfur atom. The attachment point of an alkylthio radical to a molecule is through the sulfur atom. An alkylthio radical may be depicted as -S-alkyl. The term "C₁₋₁₀ alkylthio" refers to an alkylthio radical containing 1-10 carbon atoms, having straight or branched moieties. Alkylthio group includes but is not limited to, methylthio, ethylthio, propylthio, isopropylthio, butylthio, hexylthio, and the like.

The term "cycloalkylthio", employed alone or in combination with other terms, refers to cycloalkyl as defined above, which is single bonded to a sulfur atom. The attachment point of a cycloalkylthio radical to a molecule is through the sulfur atom. A cycloalkylthio radical may be depicted as -S-cycloalkyl. "C₃₋₁₀ cycloalkylthio" refers to a cycloalkylthio radical containing 3-10 carbon atoms. Cycloalkylthio can be fused with aryl or heteroaryl group. In some embodiments, cycloalkylthio is benzocondensed. Cycloalkylthio group includes but is not limited to, cyclopropylthio, cyclobutylthio, cyclohexylthio, and the like.

The term "alkylamino", employed alone or in combination with other terms, refers to an alkyl as defined above, which is single bonded to a nitrogen atom. The attachment point of an alkylamino radical to a molecule is through the nitrogen atom. An alkylamino radical may be depicted as -NH(alkyl). The term "C₁₋₁₀ alkylamino" refers to an alkylamino radical containing 1-10 carbon atoms, having straight or branched moieties. Alkylamino group includes but is not limited to, methylamino, ethylamino, propylamino, isopropylamino, butylamino, hexylamoino, and the like.

The term "cycloalkylamino", employed alone or in combination with other terms, refers to cycloalkyl as defined above, which is single bonded to a nitrogen atom. The attachment point of a cycloalkylamino radical to a molecule is through the nitrogen atom. A cycloalkylamino radical may be depicted as -NH(cycloalkyl). "C₃₋₁₀ cycloalkylamino" refers to a cycloalkylamino radical containing 3-10 carbon atoms. Cycloalkylamino can be fused with aryl or heteroaryl group. In some embodiments, cycloalkylamino is benzocondensed. Cycloalkylamino group includes but is not limited to, cyclopropylamino, cyclobutylamino, cyclohexylamino, and the like.

The term "di(alkyl)amino", employed alone or in combination with other terms, refers to two alkyl as defined above, which are single bonded to a nitrogen atom. The attachment point of an di(alkyl)amino radical to a molecule is through the nitrogen atom. A di(alkyl)amino radical may be depicted as -N(alkyl)₂. The term "di(C₁₋₁₀ alkyl)amino" refers to a di(C₁₋₁₀ alkyl)amino radical wherein the alkyl radicals each independently contains 1-10 carbon atoms, having straight or branched moieties.

The term "aryl", employed alone or in combination with other terms, refers to a monovalent, monocyclic- , bicyclic- or tricyclic aromatic hydrocarbon ring system having 6, 7, 8, 9, 10, 11, 12, 13 or 14 carbon atoms (a "C₆₋₁₄ aryl" group), particularly a ring having 6 carbon atoms (a "C₆ aryl" group), e.g. a phenyl group; or a ring having 10 carbon atoms (a "C₁₀ aryl" group), e.g. a naphthyl group; or a ring having 14 carbon atoms, (a "C₁₄ aryl" group), e.g. an anthranyl group. Aryl can be fused with cycloalkyl or heterocycle group.

Bivalent radicals formed from substituted benzene derivatives and having the free valences at ring atoms are named as substituted phenylene radicals. Bivalent radicals derived from univalent polycyclic hydrocarbon radicals whose names end in "-yl" by removal of one hydrogen atom from the carbon atom with the free valence are named by removing "-yl" and adding "-idene" to the name of the corresponding univalent radical, e.g., a naphthyl group with two points of attachment is termed naphthylidene.

The term "heteroaryl", employed alone or in combination with other terms, refers to a monovalent, monocyclic- , bicyclic- or tricyclic aromatic ring system having 5, 6, 7, 8, 9, 10, 11, 12, 13 or 14 ring atoms (a "5- to 14-membered heteroaryl" group), particularly 5 or 6 or 9 or 10 atoms, and which contains at least one heteroatom which may be identical or different, said heteroatom selected from N, O and S. Heteroaryl can be fused with cycloalkyl or heterocycle group. In some embodiments, "heteroaryl" refers to
a 5- to 8-membered monocyclic aromatic ring containing one or more, for example, from 1 to 4, or, in some embodiments, from 1 to 3, heteroatoms selected from N, O and S, with the remaining ring atoms being carbon; or
a 8- to 12-membered bicyclic aromatic ring system containing one or more, for example, from 1 to 6, or, in some embodiments, from 1 to 4, or, in some embodiments, from 1 to 3, heteroatoms selected from N, O and S, with the remaining ring atoms being carbon; or
a 11- to 14-membered tricyclic aromatic ring system containing one or more, for example, from 1 to 8, or, in some embodiments, from 1 to 6, or, in some embodiments, from 1 to 4, or in some embodiments, from 1 to 3, heteroatoms selected from N, O and S, with the remaining ring atoms being carbon.

When the total number of S and O atoms in the heteroaryl group exceeds 1, those heteroatoms are not adjacent to one another. In some embodiments, the total number of S and O atoms in the heteroaryl group is not more than 2. In some embodiments, the total number of S and O atoms in the aromatic heterocycle is not more than 1.

Examples of heteroaryl groups include, but are not limited to, pyrid-2-yl, pyrid-3-yl, pyrid-4-yl, pyrazin-2-yl, pyrazin-3-yl, pyrimidin-2-yl, pyrimidin-4-yl, pyrimidin-5-yl, pyrimidin-6-yl, pyrazol-1-yl, pyrazol-3-yl, pyrazol-4-yl, pyrazol-5-yl, imidazol-1-yl, imidazol-2-yl, imidazol-4-yl, imidazol-5-yl, pyridazinyl, triazinyl, pyrrolyl, oxazolyl, isoxazolyl, thiazolyl, isothiazolyl, thiadiazolyl, triazolyl, tetrazolyl, thienyl, furyl.

Further heteroaryl groups include but are not limited to indolyl, benzothienyl, benzofuryl, benzoimidazolyl, benzotriazolyl, quinoxalinyl, quinolinyl, and isoquinolinyl. "Heteroaryl" is also understood to include the N-oxide derivative of any nitrogen-containing heteroaryl.

Bivalent radicals derived from univalent heteroaryl radicals whose names end in "-yl" by removal of one hydrogen atom from the atom with the free valence are named by adding "-idene" to the name of the corresponding univalent radical, e.g., a pyridyl group with two points of attachment is a pyridylidene.

The term "heterocycle", employed alone or in combination with other terms, (and variations thereof such as "heterocyclic", or "heterocyclyl") broadly refers to a saturated or unsaturated mono- or multicyclic (e.g. bicyclic) aliphatic ring system, usually with 3 to 12 ring atoms (for example 3, 4, 5, 6, 7, 8, 9, 10, 11, 12, 4 to 12, 5 to 12, 4 to 8, 4 to 7, 4 to 6, 5 to 6, or 7 to 9 ring atoms), wherein at least one (e.g. 2, 3 or 4) ring atom is heteroatom independently selected from O, S, N and P (preferably O, S, N). In a multicyclic heterocycle, two or more rings can be fused or bridged or spiro together. Heterocycle can be fused with aryl or heteroaryl group. In some embodiments, heterocycle is benzocondensed. Heterocycle also includes ring systems substituted with one or more oxo or imino moieties. In some embodiments, the C, N, S and P atoms in the heterocycle ring are optionally substituted by oxo. In some embodiments, the C, S and P atoms in the heterocycle ring are optionally substituted by imino, and imino can be unsubstituted or substituted. The point of the attachment may be carbon atom or heteroatom in the heterocyclic ring, provided that attachment results in the creation of a stable structure. When the heterocyclic ring has substituents, it is understood that the substituents may be attached to any atom in the ring, whether a heteroatom or a carbon atom, provided that a stable chemical structure result.

Suitable heterocycles include, for example, pyrrolidin-1-yl, pyrrolidin-2-yl, pyrrolidin-3-yl, imidazolidin-1-yl, imidazolidin-2-yl, imidazolidin-3-yl, imidazolidin-4-yl, imidazolidin-5-yl, pyrazolidin-1-yl, pyrazolidin-2-yl, pyrazolidin-3-yl, pyrazolidin-4-yl, pyrazolidin-5-yl, piperidin-1-yl, piperidin-2-yl, piperidin-3-yl, piperidin-4-yl, piperazin-1-yl, piperazin-2-yl, piperazin-3-yl, hexahydropyridazin-1-yl, hexahydropyridazin-3-yl and hexahydropyridazin-4-yl. Morpholinyl groups are also contemplated, such as morpholin-1-yl, morpholin-2-yl, morpholin-3-yl and morpholin-4-yl. Examples of heterocycle with one or more oxo moieties include but are not limited to, piperidinyl N-oxide, morpholinyl-N-oxide, 1-oxo-thiomorpholinyl and 1,1-dioxo-thiomorpholinyl. Bicyclic heterocycles include, for example:

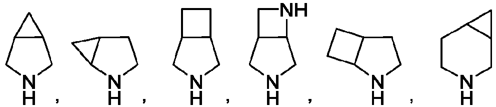

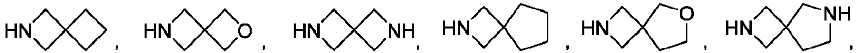

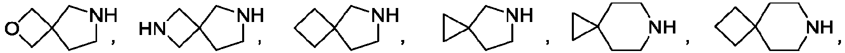

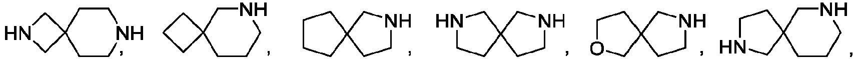

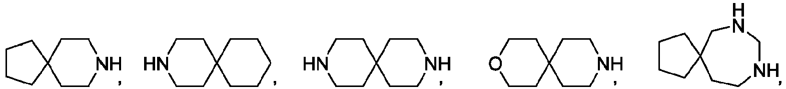

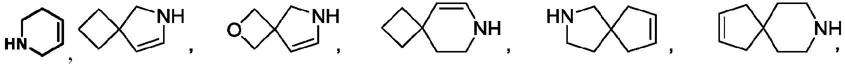

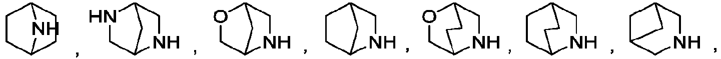

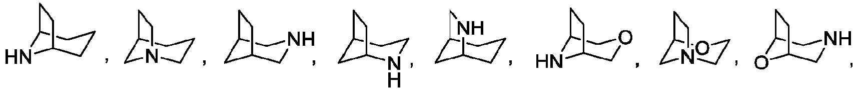

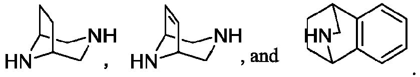

As used herein, "aryl-alkyl" refers to an alkyl moiety as defined above substituted by an aryl group as defined above. Exemplary aryl-alkyl groups include but are not limited to benzyl, phenethyl and naphthylmethyl groups. In some embodiments, aryl-alkyl groups have 7-20 or 7-11 carbon atoms. When used in the phrase "aryl-C₁₋₄ alkyl", the term "C₁₋₄" refers to the alkyl portion of the moiety and does not describe the number of atoms in the aryl portion of the moiety.

As used herein, "heterocyclyl-alkyl" refers to alkyl as defined above substituted by heterocyclyl as defined above. When used in the phrase "heterocyclyl-C₁₋₄ alkyl", the term "C₁₋₄" refers to the alkyl portion of the moiety and does not describe the number of atoms in the heterocyclyl portion of the moiety.

As used herein, "cycloalkyl-alkyl" refers to alkyl as defined above substituted by cycloalkyl as defined above. When used in the phrase "C₃₋₁₀ cycloalkyl-C₁₋₄ alkyl", the term "C₃₋₁₀" refers to the cycloalkyl portion of the moiety and does not describe the number of atoms in the alkyl portion of the moiety, and the term "C₁₋₄" refers to the alkyl portion of the moiety and does not describe the number of atoms in the cycloalkyl portion of the moiety.

As used herein, "heteroaryl-alkyl" refers to alkyl as defined above substituted by heteroaryl as defined above. When used in the phrase "heteroaryl-C₁₋₄ alkyl", the term "C₁₋₄" refers to the alkyl portion of the moiety and does not describe the number of atoms in the heteroaryl portion of the moiety.

For avoidance of doubt, reference, for example, to substitution of alkyl, cycloalkyl, heterocyclyl, aryl and/or heteroaryl refers to substitution of each of those groups individually as well as to substitutions of combinations of those groups. That is, if R is aryl-C₁₋₄ alkyl and may be unsubstituted or substituted with at least one substituent, such as one, two, three, or four substituents, independently selected from R^{X}, it should be understood that the aryl portion may be unsubstituted or substituted with at least one substituent, such as one, two, three, or four substituents, independently selected from R^{X} and the alkyl portion may also be unsubstituted or substituted with at least one substituent, such as one, two, three, or four substituents, independently selected from R^{X}.

The term "pharmaceutically acceptable salts" refers to salts prepared from pharmaceutically acceptable non-toxic bases or acids including inorganic or organic bases and inorganic or organic acids. Salts derived from inorganic bases may be selected, for example, from aluminum, ammonium, calcium, copper, ferric, ferrous, lithium, magnesium, manganic, manganous, potassium, sodium and zinc salts. Further, for example, the pharmaceutically acceptable salts derived from inorganic bases may be selected from ammonium, calcium, magnesium, potassium and sodium salts. Salts in the solid form may exist in one or more crystalline forms, or polymorphs, and may also be in the form of solvates, such as hydrates. Salts derived from pharmaceutically acceptable organic non-toxic bases may be selected, for example, from salts of primary, secondary and tertiary amines, substituted amines including naturally occurring substituted amines, cyclic amines and basic ion exchange resins, such as arginine, betaine, caffeine, choline, N,N'-dibenzylethylene-diamine, diethylamine, 2-diethylaminoethanol, 2-dimethylaminoethanol, ethanolamine, ethylenediamine, N-ethyl-morpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine and tripropylamine, tromethamine.

When the compound disclosed herein is basic, salts may be prepared using at least one pharmaceutically acceptable non-toxic acid, selected from inorganic and organic acids. Such acid may be selected, for example, from acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric and p-toluenesulfonic acids. In some embodiments, such acid may be selected, for example, from citric, hydrobromic, hydrochloric, maleic, phosphoric, sulfuric, fumaric and tartaric acids.

The terms "administration of" and or "administering" a compound or a pharmaceutically acceptable salt should be understood to mean providing a compound or a pharmaceutically acceptable salt thereof to the individual in recognized need of treatment.

The term "effective amount" means the amount of the a compound or a pharmaceutically acceptable salt that will elicit the biological or medical response of a tissue, system, animal or human that is being sought by the researcher, veterinarian, medical doctor or other clinician.

The term "composition" as used herein is intended to encompass a product comprising the specified ingredients in the specified amounts, as well as any product which results, directly or indirectly, from combination of the specified ingredients in the specified amounts. Such term in relation to a pharmaceutical composition is intended to encompass a product comprising the active ingredient (s) and the inert ingredient (s) that make up the carrier, as well as any product which results, directly or indirectly, from combination, complexation or aggregation of any two or more of the ingredients, or from dissociation of one or more of the ingredients, or from other types of reactions or interactions of one or more of the ingredients.

The term "pharmaceutically acceptable" it is meant compatible with the other ingredients of the formulation and not unacceptably deleterious to the recipient thereof.

The term "subject" as used herein in reference to individuals suffering from a disorder, a condition, and the like, encompasses mammals and non-mammals. Examples of mammals include, but are not limited to, any member of the Mammalian class: humans, non-human primates such as chimpanzees, and other apes and monkey species; farm animals such as cattle, horses, sheep, goats, swine; domestic animals such as rabbits, dogs and cats; laboratory animals including rodents, such as rats, mice and guinea pigs, and the like. Examples of non- mammals include, but are not limited to, birds, fish and the like. In one embodiment of the methods and compositions provided herein, the mammal is a human.

The terms "treat," "treating" or "treatment," and other grammatical equivalents as used herein, include alleviating, abating or ameliorating a disease or condition, preventing additional symptoms, ameliorating or preventing the underlying metabolic causes of symptoms, inhibiting the disease or condition, e.g., arresting the development of the disease or condition, relieving the disease or condition, causing regression of the disease or condition, relieving a condition caused by the disease or condition, or stopping the symptoms of the disease or condition, and are intended to include prophylaxis. The terms further include achieving a therapeutic benefit and/or a prophylactic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the patient, notwithstanding that the patient may still be afflicted with the underlying disorder. For prophylactic benefit, the compositions may be administered to a patient at risk of developing a particular disease, or to a patient reporting one or more of the physiological symptoms of a disease, even though a diagnosis of this disease may not have been made.

The term "protecting group" or "Pg" refers to a substituent that can be commonly employed to block or protect a certain functionality while reacting other functional groups on the compound. For example, an "amino-protecting group" is a substituent attached to an amino group that blocks or protects the amino functionality in the compound. Suitable amino-protecting groups include but are not limited to acetyl, trifluoroacetyl, t-butoxycarbonyl (BOC), benzyloxycarbonyl (CBZ) and 9-fluorenylmethylenoxycarbonyl (Fmoc). Similarly, a "hydroxy-protecting group" refers to a substituent of a hydroxy group that blocks or protects the hydroxy functionality. Suitable protecting groups include but are not limited to acetyl and silyl. A "carboxy-protecting group" refers to a substituent of the carboxy group that blocks or protects the carboxy functionality. Common carboxy-protecting groups include -CH₂CH₂SO₂Ph, cyanoethyl, 2-(trimethylsilyl)ethyl, 2-(trimethylsilyl)ethoxymethyl, 2-(p-toluenesulfonyl)ethyl, 2-(p-nitrophenylsulfenyl)ethyl, 2-(diphenylphosphino)-ethyl, nitroethyl and the like. For a general description of protecting groups and their use, see T. W. Greene, Protective Groups in Organic Synthesis, John Wiley & Sons, New York, 1991.

The term "NH protecting group" as used herein includes, but not limited to, trichloroethoxycarbonyl, tribromoethoxycarbonyl, benzyloxycarbonyl, para-nitrobenzylcarbonyl, ortho-bromobenzyloxycarbonyl, chloroacetyl, dichloroacetyl, trichloroacetyl, trifluoroacetyl, phenylacetyl, formyl, acetyl, benzoyl, tert-amyloxycarbonyl, tert-butoxycarbonyl, para-methoxybenzyloxycarbonyl, 3,4-dimethoxybenzyl-oxycarbonyl, 4-(phenylazo)-benzyloxycarbonyl, 2-furfuryloxycarbonyl, diphenylmethoxycarbonyl, 1,1-dimethylpropoxy-carbonyl, isopropoxycarbonyl, phthaloyl, succinyl, alanyl, leucyl, 1-adamantyloxycarbonyl, 8-quinolyloxycarbonyl, benzyl, diphenylmethyl, triphenylmethyl, 2-nitrophenylthio, methanesulfonyl, para-toluenesulfonyl, N,N-dimethylaminomethylene, benzylidene, 2-hydroxybenzylidene, 2-hydroxy-5-chlorobenzylidene, 2-hydroxy-1-naphthylmethylene, 3-hydroxy-4-pyridylmethylene, cyclohexylidene, 2-ethoxycarbonylcyclohexylidene, 2-ethoxycarbonylcyclopentylidene, 2-acetylcyclohexylidene, 3,3-dimethyl-5-oxycyclo-hexylidene, diphenylphosphoryl, dibenzylphosphoryl, 5-methyl-2-oxo-2H-1,3-dioxol-4-yl-methyl, trimethylsilyl, triethylsilyl and triphenylsilyl.

The term "C(O)OH protecting group" as used herein includes, but not limited to, methyl, ethyl, n-propyl, isopropyl, 1,1-dimethylpropyl, n-butyl, tert-butyl, phenyl, naphthyl, benzyl, diphenylmethyl, triphenylmethyl, para-nitrobenzyl, para-methoxybenzyl, bis(para-methoxyphenyl)methyl, acetylmethyl, benzoylmethyl, para-nitrobenzoylmethyl, para-bromobenzoylmethyl, para-methanesulfonylbenzoylmethyl, 2-tetrahydropyranyl, 2-tetrahydrofuranyl, 2,2,2-trichloro-ethyl, 2-(trimethylsilyl)ethyl, acetoxymethyl, propionyloxymethyl, pivaloyloxymethyl, phthalimidomethyl, succinimidomethyl, cyclopropyl, cyclobutyl, cyclopentyl, cyclohexyl, methoxymethyl, methoxyethoxymethyl, 2-(trimethylsilyl)ethoxymethyl, benzyloxymethyl, methylthiomethyl, 2-methylthioethyl, phenylthiomethyl, 1,1-dimethyl-2-propenyl, 3-methyl-3-butenyl, allyl, trimethylsilyl, triethylsilyl, triisopropylsilyl, diethylisopropylsilyl, tert-butyldimethylsilyl, tert-butyldiphenylsilyl, diphenylmethylsilyl and tert-butylmethoxyphenylsilyl.

The term "OH or SH protecting group" as used herein includes, but not limited to, benzyloxycarbonyl, 4-nitrobenzyloxycarbonyl, 4-bromobenzyloxycarbonyl, 4-methoxybenzyloxycarbonyl, 3,4-dimethoxybenzyloxycarbonyl, methoxycarbonyl, ethoxycarbonyl, tert-butoxycarbonyl, 1,1-dimethylpropoxycarbonyl, isopropoxycarbonyl, isobutyloxycarbonyl, diphenylmethoxycarbonyl, 2,2,2-trichloroethoxycarbonyl, 2,2,2-tribromoethoxycarbonyl, 2-(trimethylsilyl)ethoxycarbonyl, 2-(phenylsulfonyl)ethoxycarbonyl, 2-(triphenylphosphonio)ethoxycarbonyl, 2-furfuryloxycarbonyl, 1-adamantyloxycarbonyl, vinyloxycarbonyl, allyloxycarbonyl, 4-ethoxy-1-naphthyloxycarbonyl, 8-quinolyloxycarbonyl, acetyl, formyl, chloroacetyl, dichloroacetyl, trichloroacetyl, trifluoroacetyl, methoxyacetyl, phenoxyacetyl, pivaloyl, benzoyl, methyl, tert-butyl, 2,2,2-trichloroethyl, 2-trimethylsilylethyl, 1,1-dimethyl-2-propenyl, 3-methyl-3-butenyl, allyl, benzyl (phenylmethyl), para-methoxybenzyl, 3,4-dimethoxybenzyl, diphenylmethyl, triphenylmethyl, tetrahydrofuryl, tetrahydropyranyl, tetrahydrothiopyranyl, methoxymethyl, methylthiomethyl, benzyloxymethyl, 2-methoxyethoxymethyl, 2,2,2-trichloro-ethoxymethyl, 2-(trimethylsilyl)ethoxymethyl, 1-ethoxyethyl, methanesulfonyl, para-toluenesulfonyl, trimethylsilyl, triethylsilyl, triisopropylsilyl, diethylisopropylsilyl, tert-butyldimethylsilyl, tert-butyldiphenylsilyl, diphenylmethylsilyl and tert-butylmethoxyphenylsilyl.

Geometric isomers may exist in the present compounds. Compounds of this invention may contain carbon-carbon double bonds or carbon-nitrogen double bonds in the E or Z configuration, wherein the term "E" represents higher order substituents on opposite sides of the carbon-carbon or carbon-nitrogen double bond and the term "Z" represents higher order substituents on the same side of the carbon-carbon or carbon-nitrogen double bond as determined by the Cahn-Ingold-Prelog Priority Rules. The compounds of this invention may also exist as a mixture of "E" and "Z" isomers. Substituents around a cycloalkyl or heterocycloalkyl are designated as being of cis or trans configuration. Furthermore, the invention contemplates the various isomers and mixtures thereof resulting from the disposal of substituents around an adamantane ring system. Two substituents around a single ring within an adamantane ring system are designated as being of Z or E relative configuration. For examples, see C. D. Jones, M. Kaselj, R. N. Salvatore, W. J. le Noble J. Org. Chem. 1998, 63, 2758-2760.

Compounds of this invention may contain asymmetrically substituted carbon atoms in the R or S configuration, in which the terms "R" and "S" are as defined by the IUPAC 1974 Recommendations for Section E, Fundamental Stereochemistry, Pure Appl. Chem. (1976) 45, 13-10. Compounds having asymmetrically substituted carbon atoms with equal amounts of R and S configurations are racemic at those carbon atoms. Atoms with an excess of one configuration over the other are assigned the configuration present in the higher amount, preferably an excess of about 85-90%, more preferably an excess of about 95-99%, and still more preferably an excess greater than about 99%. Accordingly, this invention includes racemic mixtures, relative and absolute stereoisomers, and mixtures of relative and absolute stereoisomers.

### Isotope Enriched or Labeled Compounds.

Compounds of the invention can exist in isotope-labeled or -enriched form containing one or more atoms having an atomic mass or mass number different from the atomic mass or mass number most abundantly found in nature. Isotopes can be radioactive or non- radioactive isotopes. Isotopes of atoms such as hydrogen, carbon, nitrogen, oxygen, phosphorous, sulfur, fluorine, chlorine and iodine include, but are not limited to, ²H, ³H, ¹³C, ¹⁴C, ¹⁵N, ¹⁸O, ³²P, ³⁵S, ¹⁸F, ³⁶Cl and ¹²⁵I. Compounds that contain other isotopes of these and/or other atoms are within the scope of this invention.

In another embodiment, the isotope-labeled compounds contain deuterium (²H), tritium (³H) or ¹⁴C isotopes. Isotope-labeled compounds of this invention can be prepared by the general methods well known to persons having ordinary skill in the art. Such isotope-labeled compounds can be conveniently prepared by carrying out the procedures disclosed in the Examples disclosed herein and Schemes by substituting a readily available isotope-labeled reagent for a non-labeled reagent. In some instances, compounds may be treated with isotope-labeled reagents to exchange a normal atom with its isotope, for example, hydrogen for deuterium can be exchanged by the action of a deuterated acid such as D₂SO₄/D₂O.

The isotope-labeled compounds of the invention may be used as standards to determine the effectiveness of Bcl-2 inhibitors in binding assays. Isotope containing compounds have been used in pharmaceutical research to investigate the in vivo metabolic fate of the compounds by evaluation of the mechanism of action and metabolic pathway of the nonisotope-labeled parent compound (Blake et al. J. Pharm. Sci. 64, 3, 367-391 (1975)). Such metabolic studies are important in the design of safe, effective therapeutic drugs, either because the in vivo active compound administered to the patient or because the metabolites produced from the parent compound prove to be toxic or carcinogenic (Foster et al., Advances in Drug Research Vol. 14, pp. 2-36, Academic press, London, 1985; Kato et al, J. Labelled Compounds. Radiopharmaceuticals., 36(10), 927-932 (1995); Kushner et al., Can. J. Physiol. Pharmacology, 77, 79-88 (1999).

In addition, non-radioactive isotope containing drugs, such as deuterated drugs called "heavy drugs" can be used for the treatment of diseases and conditions related to Bcl-2 activity. Increasing the amount of an isotope present in a compound above its natural abundance is called enrichment. Examples of the amount of enrichment include but are not limited to from about 0.5, 1, 2, 3, 4, 5, 6, 7, 8, 9, 10, 12, 16, 21, 25, 29, 33, 37, 42, 46, 50, 54, 58, 63, 67, 71, 75, 79, 84, 88, 92, 96, to about 100 mol %.

Stable isotope labeling of a drug can alter its physico-chemical properties such as pKa and lipid solubility. These effects and alterations can affect the pharmacodynamic response of the drug molecule if the isotopic substitution affects a region involved in a ligand-receptor interaction. While some of the physical properties of a stable isotope-labeled molecule are different from those of the unlabeled one, the chemical and biological properties are the same, with one important exception: because of the increased mass of the heavy isotope, any bond involving the heavy isotope and another atom will be stronger than the same bond between the light isotope and that atom. Accordingly, the incorporation of an isotope at a site of metabolism or enzymatic transformation will slow said reactions potentially altering the pharmacokinetic profile or efficacy relative to the non-isotopic compound.

In an Embodiment (1), this invention provides to a compound of formula (I), 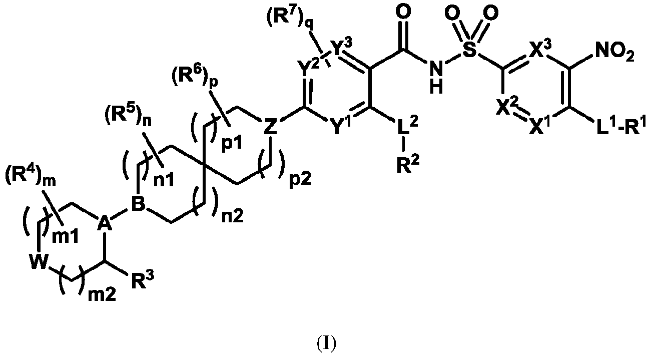 or a pharmaceutically acceptable salt thereof, wherein:
X¹ is selected from N and CR⁸;
X² is selected from N and CR⁹;
X³ is selected from N and CR¹⁰;
Y¹, Y² and Y³ are independently selected from N and CH;
A and B are independently selected from N and CH;
W is selected from -CR¹¹-, -NR¹²-, -O-, -S(O)ᵣ- and -S(O)(=NR¹²)-;
Z is selected from N and CH;
L¹ and L² are independently selected from a bond, -(CR^{C0}R^{D0})ᵤ-, -(CR^{C0}R^{D0})^{u}O(CR^{C0}R^{D0})ₜ-, -(CR^{C0}R^{D0})ᵤNR^{A0}(CR^{C0}R^{D0})ₜ- and -(CR^{C0}R^{D0})ᵤS(O)ᵣ(CR^{C0}R^{D0})ₜ-;
R¹ is selected from hydrogen, halogen, C_{1-1O} alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A1}R^{B1}, -OR^{A1}, -C(O)R^{A1}, -C(=NR^{E1})R^{A1}, -C(=N-OR^{B1})R^{A1}, -C(O)OR^{A1}, -OC(O)R^{A1}, -C(O)NR^{A1}R^{B1}, -NR^{A1}C(O)R^{B1}, -C(=NR^{E1})NR^{A1}R^{B1}, -NR^{A1}C(=NR^{E1})R^{B1}, -OC(O)NR^{A1}R^{B1}, -NR^{A1}C(O)OR^{B1}, -NR^{A1}C(O)NR^{A1}R^{B1}, -NR^{A1}C(S)NR^{A1}R^{B1}, -NR^{A1}C(=NR^{E1})NR^{A1}R^{B1}, -S(O)ᵣR^{A1}, -S(O)(=NR^{E1})R^{B1}, -N=S(O)R^{A1}R^{B1}, -S(O)₂OR^{A1}, -OS(O)₂R^{A1}, -NR^{A1}S(O)ᵣR^{B1}, -NR^{A1}S(O)(=NR^{E1})R^{B1}, -S(O)ᵣNR^{A1}R^{B1}, -S(O)(=NR^{E1})NR^{A1}R^{B1}, -NR^{A1}S(O)₂NR^{A1}R^{B1}, -NR^{A1}S(O)(=NR^{E1})NR^{A1}R^{B1}, -P(O)R^{A1}R^{B1} and -P(O)(OR^{A1})(OR^{B1}), wherein alkyl, alkenyl, alkynyl, cycloalkyl, heterocyclyl, aryl and heteroaryl are each unsubstituted or substituted with at least one substituent, independently selected from R^{X};
R² and R³ are independently selected from aryl, heteroaryl and heterocyclyl, wherein aryl, heteroaryl and heterocyclyl are each unsubstituted or substituted with at least one substituent, independently selected from R^{X};
each R⁴ is independently selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A4}R^{B4}, -OR^{A4}, -C(O)R^{A4}, -C(=NR^{E4})R^{A4}, -C(=N-OR^{B4})R^{A4}, -C(O)OR^{A4}, -OC(O)R^{A4}, -C(O)NR^{A4}R^{B4}, -NR^{A4}C(O)R^{B4}, -C(=NR^{E4})NR^{A4}R^{B4}, -NR^{A4}C(=NR^{E4})R^{B4}, -OC(O)NR^{A4}R^{B4}, -NR^{A4}C(O)OR^{B4}, -NR^{A4}C(O)NR^{A4}R^{B4}, -NR^{A4}C(S)NR^{A4}R^{B4}, -NR^{A4}C(=NR^{E4})NR^{A4}R^{B4}, -S(O)ᵣR^{A4}, -S(O)(=NR^{E4})R^{B4}, -N=S(O)R^{A4}R^{B4}, -S(O)₂OR^{A4}, -OS(O)₂R^{A4}, -NR^{A4}S(O)ᵣR^{B4}, -NR^{A4}S(O)(=NR^{E4})R^{B4}, -S(O)ᵣNR^{A4}R^{B4}, -S(O)(=NR^{E4})NR^{A4}R^{B4}, -NR^{A4}S(O)₂NR^{A4}R^{B4}, -NR^{A4}S(O)(=NR^{E4})NR^{A4}R^{B4}, -P(O)R^{A4}R^{B4} and -P(O)(OR^{A4})(OR^{B4}), wherein alkyl, alkenyl, alkynyl, cycloalkyl, heterocyclyl, aryl and heteroaryl are each unsubstituted or substituted with at least one substituent, independently selected from R^{X};
or any two of R⁴ or "R⁴ and R¹¹" or "R⁴ and R¹²" together with the atoms to which they are attached form a C₃₋₁₀ cycloalkyl or heterocyclic ring of 4 to 12 members containing 1, 2 or 3 heteroatoms independently selected from oxygen, sulfur, nitrogen and phosphorus, and optionally substituted with 1, 2 or 3 R^{X} groups;
each R⁵ is independently selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A5}R^{B5}, -OR^{A5}, -C(O)R^{A5}, -C(=NR^{E5})R^{A5}, -C(=N-OR^{B5})R^{A5}, -C(O)OR^{A5}, -OC(O)R^{A5}, -C(O)NR^{A5}R^{B5}, -NR^{A5}C(O)R^{B5}, -C(=NR^{E5})NR^{A5}R^{B5}, -NR^{A5}C(=NR^{E5})R^{B5}, -OC(O)NR^{A5}R^{B5}, -NR^{A5}C(O)OR^{B5}, -NR^{A5}C(O)NR^{A5}R^{B5}, -NR^{A5}C(S)NR^{A5}R^{B5}, -NR^{A5}C(=NR^{E5})NR^{A5}R^{B5}, -S(O)ᵣR^{A5}, -S(O)(=NR^{E5})R^{B5}, -N=S(O)R^{A5}R^{B5}, -S(O)₂OR^{A5}, -OS(O)₂R^{A5}, -NR^{AS}S(O)ᵣR^{B5}, -NR^{A5}S(O)(=NR^{E5})R^{B5}, -S(O)ᵣNR^{A5}R^{B5}, -S(O)(=NR^{E5})NR^{A5}R^{B5}, -NR^{A5}S(O)₂NR^{A5}R^{B5}, -NR^{A5}S(O)(=NR^{E5})NR^{A5}R^{B5}, -P(O)R^{A5}R^{B5} and -P(O)(OR^{A5})(OR^{B5}), wherein alkyl, alkenyl, alkynyl, cycloalkyl, heterocyclyl, aryl and heteroaryl are each unsubstituted or substituted with at least one substituent, independently selected from R^{X};
or any two of R⁵ together with the carbon atoms to which they are attached form a C₃₋₁₀ cycloalkyl or heterocyclic ring of 4 to 12 members containing 1, 2 or 3 heteroatoms independently selected from oxygen, sulfur, nitrogen and phosphorus, and optionally substituted with 1, 2 or 3 R^{X} groups;
each R⁶ is independently selected from hydrogen, halogen, C_{1-1O} alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A6}R^{B6}, -OR^{A6}, -C(O)R^{A6}, -C(=NR^{E6})R^{A6}, -C(=N-OR^{B6})R^{A6}, -C(O)OR^{A6}, -OC(O)R^{A6}, -C(O)NR^{A6}R^{B6}, -NR^{A6}C(O)R^{B6}, -C(=NR^{E6})NR^{A6}R^{B6}, -NR^{A6}C(=NR^{E6})R^{B6}, -OC(O)NR^{A6}R^{B6}, -NR^{A6}C(O)OR^{B6}, -NR^{A6}C(O)NR^{A6}R^{B6}, -NR^{A6}C(S)NR^{A6}R^{B6}, -NR^{A6}C(=NR^{E6})NR^{A6}R^{B6}, -S(O)ᵣR^{A6}, -S(O)(=NR^{E6})R^{B6}, -N=S(O)R^{A6}R^{B6}, -S(O)₂OR^{A6}, -OS(O)₂R^{A6}, -NR^{A6}S(O)ᵣR^{B6}, -NR^{A6}S(O)(=NR^{E6})R^{B6}, -S(O)ᵣNR^{A6}R^{B6}, -S(O)(=NR^{E6})NR^{A6}R^{B6}, -NR^{A6}S(O)₂NR^{A6}R^{B6}, -NR^{A6}S(O)(=NR^{E6})NR^{A6}R^{B6}, -P(O)R^{A6}R^{B6} and -P(O)(OR^{A6})(OR^{B6}), wherein alkyl, alkenyl, alkynyl, cycloalkyl, heterocyclyl, aryl and heteroaryl are each unsubstituted or substituted with at least one substituent, independently selected from R^{X};
or any two of R⁶ together with the carbon atoms to which they are attached form a C₃₋₁₀ cycloalkyl or heterocyclic ring of 4 to 12 members containing 1, 2 or 3 heteroatoms independently selected from oxygen, sulfur, nitrogen and phosphorus, and optionally substituted with 1, 2 or 3 R^{X} groups;
each R⁷ is independently selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A7}R^{B7}, -OR^{A7}, -C(O)R^{A7}, -C(=NR^{E7})R^{A7}, -C(=N-OR^{B7})R^{A7}, -C(O)OR^{A7}, -OC(O)R^{A7}, -C(O)NR^{A7}R^{B7}, -NR^{A7}C(O)R^{B7}, -C(=NR^{E7})NR^{A7}R^{B7}, -NR^{A7}C(=NR^{E7})R^{B7}, -OC(O)NR^{A7}R^{B7}, -NR^{A7}C(O)OR^{B7}, -NR^{A7}C(O)NR^{A7}R^{B7}, -NR^{A7}C(S)NR^{A7}R^{B7}, -NR^{A7}C(=NR^{E7})NR^{A7}R^{B7}, -S(O)ᵣR^{A7}, -S(O)(=NR^{E7})R^{B7}, -N=S(O)R^{A7}R^{B7}, -S(O)₂OR^{A7}, -OS(O)₂R^{A7}, -NR^{A7}S(O)ᵣR^{B7}, -NR^{A7}S(O)(=NR^{E7})R^{B7}, -S(O)ᵣNR^{A7}R^{B7}, -S(O)(=NR^{E7})NR^{A7}R^{B7}, -NR^{A7}S(O)₂NR^{A7}R^{B7}, -NR^{A7}S(O)(=NR^{E7})NR^{A7}R^{B7}, -P(O)R^{A7}R^{B7} and -P(O)(OR^{A7})(OR^{B7}), wherein alkyl, alkenyl, alkynyl, cycloalkyl, heterocyclyl, aryl and heteroaryl are each unsubstituted or substituted with at least one substituent, independently selected from R^{X};
R⁸ is selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A8}R^{B8}, -OR^{A8}, -C(O)R^{A8}, -C(=NR^{E8})R^{A8}, -C(=N-OR^{B8})R^{A8}, -C(O)OR^{A8}, -OC(O)R^{A8}, -C(O)NR^{A8}R^{B8}, -NR^{A8}C(O)R^{B8}, -C(=NR^{E8})NR^{A8}R^{B8}, -NR^{A8}C(=NR^{E8})R^{B8}, -OC(O)NR^{A8}R^{B8}, -NR^{A8}C(O)OR^{B8}, -NR^{A8}C(O)NR^{A8}R^{B8}, -NR^{A8}C(S)NR^{A8}R^{B8}, -NR^{A8}C(=NR^{E8})NR^{A8}R^{B8}, -S(O)ᵣR^{A8}, -S(O)(=NR^{E8})R^{B8}, -N=S(O)R^{A8}R^{B8}, -S(O)₂OR^{A8}, -OS(O)₂R^{A8}, -NR^{A8}S(O)ᵣR^{B8}, -NR^{A8}S(O)(=NR^{E8})R^{B8}, -S(O)ᵣNR^{A8}R^{B8}, -S(O)(=NR^{E8})NR^{A8}R^{B8}, -NR^{A8}S(O)₂NR^{A8}R^{B8}, -NR^{A8}S(O)(=NR^{E8})NR^{A8}R^{B8}, -P(O)R^{A8}R^{B8} and -P(O)(OR^{A8})(OR^{B8}), wherein alkyl, alkenyl, alkynyl, cycloalkyl, heterocyclyl, aryl and heteroaryl are each unsubstituted or substituted with at least one substituent, independently selected from R^{X};
or R¹ and R⁸ together with the atoms to which they are attached form a C₅₋₁₀ cycloalkyl or heterocyclic ring of 5 to 12 members containing 1, 2 or 3 heteroatoms independently selected from oxygen, sulfur, nitrogen and phosphorus, and optionally substituted with 1, 2 or 3 R^{X} groups;
R⁹ is selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A9}R^{B9}, -OR^{A9}, -C(O)R^{A9}, -C(=NR^{E9})R^{A9}, -C(=N-OR^{B9})R^{A9}, -C(O)OR^{A9}, -OC(O)R^{A9}, -C(O)NR^{A9}R^{B9}, -NR^{A9}C(O)R^{B9}, -C(=NR^{E9})NR^{A9}R^{B9}, -NR^{A9}C(=NR^{E9})R^{B9}, -OC(O)NR^{A9}R^{B9}, -NR^{A9}C(O)OR^{B9}, -NR^{A9}C(O)NR^{A9}R^{B9}, -NR^{A9}C(S)NR^{A9}R^{B9}, -NR^{A9}C(=NR^{E9})NR^{A9}R^{B9}, -S(O)ᵣR^{A9}, -S(O)(=NR^{E9})R^{B9}, -N=S(O)R^{A9}R^{B9}, -S(O)₂OR^{A9}, -OS(O)₂R^{A9}, -NR^{A9}S(O)ᵣR^{B9}, -NR^{A9}S(O)(=NR^{E9})R^{B9}, -S(O)ᵣNR^{A9}R^{B9}, -S(O)(=NR^{E9})NR^{A9}R^{B9}, -NR^{A9}S(O)₂NR^{A9}R^{B9}, -NR^{A9}S(O)(=NR^{E9})NR^{A9}R^{B9}, -P(O)R^{A9}R^{B9} and -P(O)(OR^{A9})(OR^{B9}), wherein alkyl, alkenyl, alkynyl, cycloalkyl, heterocyclyl, aryl and heteroaryl are each unsubstituted or substituted with at least one substituent, independently selected from R^{X};
R¹⁰ is selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A10}R^{B10}, -OR^{A10} -C(O)R^{A10}, -C(=NR^{E10})R^{A10}, -C(=N-OR^{B10})R^{A10}, -C(O)OR^{A10}, -OC(O)R^{A10}, -C(O)NR^{A10}R^{B10}, -NR^{A10}C(O)R^{B10}, -C(=NR^{E10})NR^{A10}R^{B10}, -NR^{A10}C(=NR^{E10})R^{B10}, -OC(O)NR^{A10}R^{B10}, -NR^{A10}C(O)OR^{B10}, -NR^{A10}C(O)NR^{A10}R^{B10}, -NR^{A10}C(S)NR^{A10}R^{B10}, -NR^{A10}C(=NR^{E10})NR^{A10})R^{B10}, -S(O)ᵣR^{A10}, -S(O)(=NR^{E10})R^{B10}, -N=S(O)R^{A10}R^{B10}, -S(O)₂OR^{A10}, -OS(O)₂R^{A10}, -NR^{A10}S(O)ᵣR^{B10}, -NR^{A10}S(O)(=NR^{E10})R^{B10}, -S(O)ᵣNR^{A10}R^{B10}, -S(O)(=NR^{E10})NR^{A10}R^{B10}, -NR^{A10}S(O)₂NR^{A10}R^{B10}, -NR^{A10}S(O)(=NR^{E10})NR^{A10}R^{B10}, -P(O)R^{A10}R^{B10} and -P(O)(OR^{A10})(OR^{B10}), wherein alkyl, alkenyl, alkynyl, cycloalkyl, heterocyclyl, aryl and heteroaryl are each unsubstituted or substituted with at least one substituent, independently selected from R^{X};
R¹¹ is selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A11}R^{B11}, -OR^{A11}, -C(O)R^{A11}, -C(=NR^{E11})R^{A11}, -C(=N-OR^{B11})R^{A11}, -C(O)OR^{A11}, -OC(O)R^{A11}, -C(O)NR^{A11}R^{B11}, -NR^{A11}C(O)R^{B11}, -C(=NR^{E11})NR^{A11}R^{B11}, -NR^{A11}C(=NR^{E11})R^{B11}, -OC(O)NR^{A11}R^{B11}, -NR^{A11}C(O)OR^{B11}, -NR^{A11}C(O)NR^{A11}R^{B11}, -NR^{A11}C(S)NR^{A11}R^{B11}, -NR^{A11}C(=NR^{E11})NR^{A11}R^{B11}, -S(O)ᵣR^{A11}, -S(O)(=NR^{E11})R^{B11}, -N=S(O)R^{A11}R^{B11}, -S(O)₂OR^{A11}, -OS(O)₂R^{A11}, -NR^{A11}S(O)ᵣR^{B11}, -NR^{A11}S(O)(=NR^{E11})R^{B11}, -S(O)ᵣNR^{A11}R^{B11}, -S(O)(=NR^{E11})R^{A11}R^{B11}, -NR^{A11}S(O)₂NR^{A11}R^{B11}, -NR^{A11}S(O)(=NR^{E11})NR^{A11}R^{B11}, -P(O)R^{A11}R^{B11} and -P(O)(OR^{A11})(OR^{B11}), wherein alkyl, alkenyl, alkynyl, cycloalkyl, heterocyclyl, aryl and heteroaryl are each unsubstituted or substituted with at least one substituent, independently selected from R^{X};
R¹² is selected from hydrogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, -C(O)R^{A12}, -C(=NR^{E12})R^{A12}, -C(=N-OR^{B12})R^{A12}, -C(O)OR^{A12}, -C(O)NR^{A12}R^{B12}, -C(=NR^{E12})NR^{A12}R^{B12}, -S(O)ᵣR^{A12}, -S(O)(=NR^{E12})R^{B12}, -S(O)₂OR^{A12}, -S(O)ᵣNR^{A12}R^{B12}, -S(O)(=NR^{E12})NR^{A12}R^{B12}, -P(O)R^{A12}R^{B12} and -P(O)(OR^{A12})(OR^{B12}), wherein alkyl, alkenyl, alkynyl, cycloalkyl, heterocyclyl, aryl and heteroaryl are each unsubstituted or substituted with at least one substituent, independently selected from R^{X};

each R^{A0}, R^{A1}, R^{A4}, R^{A5}, R^{A6}, R^{A7}, R^{A8}, R^{A9}, R^{A10}, R^{A11}, R^{A12}, R^{B1}, R^{B4}, R^{B5}, R^{B6}, R^{B7}, R^{B8}, R^{B9}, R^{B10}, R^{B11} and R^{B12} are independently selected from hydrogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl and heteroaryl-C₁₋₄ alkyl, wherein alkyl, alkenyl, alkynyl, cycloalkyl, heterocyclyl, aryl and heteroaryl are each unsubstituted or substituted with at least one substituent, independently selected from R^{X};
or "R^{A1} and R^{B1}" or "R^{A4} and R^{B4}" or "R^{A5} and R^{B5}" or "R^{A6} and R^{B6}" or "R^{A7} and R^{B7}" or "R^{A8} and R^{B8}" or "R^{A9} and R^{B9}" or "R^{A10} and R^{B10}" or "R^{A11} and R^{B11}" or "R^{A12} and R^{B12}" together with the atom(s) to which they are attached form a heterocyclic ring of 4 to 12 members containing 0, 1 or 2 additional heteroatoms independently selected from oxygen, sulfur, nitrogen and phosphorus and optionally substituted with 1, 2 or 3 R^{X} groups;
   each R^{C0} and R^{D0} are independently selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl and heteroaryl-C₁₋₄ alkyl, wherein alkyl, alkenyl, alkynyl, cycloalkyl, heterocyclyl, aryl and heteroaryl are each unsubstituted or substituted with at least one substituent, independently selected from R^{X};
   or each "R^{C0} and R^{D0}" together with the carbon atom(s) to which they are attached form a 3- to 12-membered ring containing 0, 1 or 2 heteroatoms independently selected from oxygen, sulfur and nitrogen, and optionally substituted with 1, 2 or 3 R^{X} groups;
   each R^{E1}, R^{E4}, R^{E5}, R^{E6}, R^{E7}, R^{E8}, R^{E9}, R^{E10}, R^{E11} and R^{E12} are independently selected from hydrogen, C₁₋₁₀ alkyl, CN, NO₂, -OR^{a1}, -SR^{a1}, -S(O)ᵣR^{a1}, -C(O)R^{a1}, -C(O)OR^{a1}, -C(O)NR^{a1}R^{b1} and -S(O)ᵣNR^{a1}R^{b1}, wherein alkyl is unsubstituted or substituted with at least one substituent, independently selected from R^{X};
   each R^{X} is independently selected from hydrogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, halogen, CN, NO₂, -(CR^{c1}R^{d1})ₜNR^{a1}R^{b1}, -(CR^{c1}R^{d1})ₜOR^{b1}, -(CR^{c1}R^{d1})ₜC(O)R^{a1}, -(CR^{c1}R^{d1})ₜC(=NR^{e1})R^{a1}, -(CR^{c1}R^{d1})ₜC(=N-OR^{b1})R^{a1}, -(CR^{c1}R^{d1})ₜC(O)OR^{b1}, -(CR^{c1}R^{d1})ₜOC(O)R^{b1}, -(CR^{c1}R^{d1})ₜC(O)NR^{a1}R^{b1}, -(CR^{c1}R^{d1})ₜNR^{a1}C(O)R^{b1}, -(CR^{c1}R^{d1})ₜC(=NR^{e1})NR^{a1}R^{b1}, -(CR^{c1}R^{d1})ₜNR^{a1}C(=NR^{e1})R^{b}, -(CR^{c1}R^{d1})ₜOC(O)NR^{a1}R^{b1}, -(CR^{c1}R^{d1})ₜNR^{a1}C(O)OR^{b1}, -(CR^{c1}R^{d1})ₜNR^{a1}C(O)NR^{a1}R^{b1}, -(CR^{c1}R^{d1})ₜNR^{a1}C(S)NR^{a1}R^{b1}, -(CR^{c1}R^{d1})ₜNR^{a1}C(=NR^{e1})NR^{a1}R^{b1}, -(CR^{c1}R^{d1})ₜS(O)ᵣR^{b1}, -(CR^{c1}R^{d1})ₜS(O)(=NR^{e1})R^{b1}, -(CR^{c1}R^{d1})ₜN=S(O)R^{a1}R^{b1}, -(CR^{c1}R^{d1})ₜS(O)₂OR^{b1}, -(CR^{c1}R^{d1})ₜOS(O)₂R^{b1}, -(CR^{c1}R^{d1})ₜNR^{a1}S(O)ᵣR^{b1}, -(CR^{c1}R^{d1})ₜNR^{a1}S(O)(=NR^{e1})R^{b1}, -(CR^{c1}R^{d1})ₜS(O)ᵣNR^{a1}R^{b1}, -(CR^{c1}R^{d1})ₜS(O)(=NR^{e1})NR^{a1}R^{b1}, -(CR^{c1}R^{d1})ₜNR^{a1}S(O)₂NR^{a1}R^{b1}, -(CR^{c1}R^{d1})ₜNR^{a1}S(O)(=NR^{e1})NR^{a1}R^{b1}, -(CR^{c1}R^{d1})ₜP(O)R^{a1}R^{b1} and -(CR^{c1}R^{d1})ₜP(O)(OR^{a1})(OR^{b1}), wherein alkyl, alkenyl, alkynyl, cycloalkyl, heterocyclyl, aryl and heteroaryl are each unsubstituted or substituted with at least one substituent, independently selected from R^{Y};
   wherein
   when X¹ is CR⁸, X² is CR⁹, X³ is CR¹⁰, R¹ and R⁸ together with the atoms to which they are attached form a C₅₋₁₀ cycloalkyl or heterocyclic ring of 5 to 12 members containing 1, 2 or 3 heteroatoms independently selected from oxygen, sulfur, nitrogen and phosphorus, and optionally substituted with 1, 2 or 3 R^{X} groups and L¹ is selected from -(CR^{C0}R^{D0})ᵤNR^{A0}(CR^{C0}R^{D0})ₜ-, L² is selected from a bond, -(CR^{C0}R^{D0})ᵤO(CR^{C0}R^{D0})ₜ-, -(CR^{C0}R^{D0})ᵤS(O)ᵣ(CR^{C0}R^{D0})ₜ-, wherein when L² is -O- and R² is 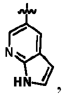 each substituent R^{X} of R² is selected from C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, halogen, CN, NO₂, -(CR^{c1}R^{d1})ₜNR^{a1}R^{b1}, -(CR^{c1}R^{d1})ₜOR^{b1}, -(CR^{c1}R^{d1})ₜC(O)R^{a1}, -(CR^{c1}R^{d1})ₜC(=NR^{e1})R^{a1}, -(CR^{c1}R^{d1})ₜC(=N-OR^{b1})R^{a1}, -(CR^{c1}R^{d1})ₜC(O)OR^{b1}, -(CR^{c1}R^{a1})ₜOC(O)R^{b1}, -(CR^{c1}R^{d1})ₜC(O)NR^{a1}R^{b1}, -(CR^{c1}R^{d1})ₜNR^{a1}C(O)R^{b1}, -(CR^{c1}R^{d1})ₜC(=NR^{e1})NR^{a1}R^{b1}, -(CR^{cl}R^{d1})ₜNR^{a1}C(=NR^{e1})R^{b1}, -(CR^{c1}R^{d1})ₜOC(O)NR^{a1}R^{b1}, -(CR^{c1}R^{d1})ₜNR^{a1}C(O)OR^{b1}, -(CR^{c1}R^{d1})ₜNR^{a1}C(O)NR^{a1}R^{b1}, -(CR^{c1}R^{d1})ₜNR^{a1}C(S)NR^{a1}R^{b1}, -(CR^{c1}R^{d1})ₜNR^{a1}C(=NR^{e1})NR^{a1}R^{b1}, -(CR^{c1}R^{d1})ₜS(O)ᵣR^{b1}, -(CR^{c1}R^{d1})ₜS(O)(=NR^{e1})R^{b1}, -(CR^{c1}R^{d1})ₜN=S(O)R^{a1}R^{b1}, -(CR^{c1}R^{a1})ₜS(O)₂OR^{b1}, -(CR^{c1}R^{d1})ₜOS(O)₂R^{b1}, -(CR^{c1}R^{d1})ₜNR^{a1}S(O)ᵣR^{b1}, -(CR^{c1}R^{d1})ₜNR^{a1}S(O)(=NR^{e1})R^{b1}, -(CR^{c1}R^{d1})ₜS(O)ᵣNR^{a1}R^{b1}, -(CR^{c1}R^{d1})ₜS(O)(=NR^{e1})NR^{a1}R^{b1}, -(CR^{c1}R^{d1})ₜNR^{a1}S(O)₂NR^{a1}R^{b1}, -(CR^{c1}R^{d1})ₜNR^{a1}S(O)(=NR^{e1})NR^{a1}R^{b1}, -(CR^{c1}R^{d1})ₜP(O)R^{a1}R^{b1} and -(CR^{c1}R^{d1})ₜP(O)(OR^{a1})(OR^{b1}), wherein alkyl, alkenyl, alkynyl, cycloalkyl, heterocyclyl, aryl and heteroaryl are each unsubstituted or substituted with at least one substituent, independently selected from R^{Y};
   when X¹ is CR⁸, X² is CR⁹, X³ is CR¹⁰ and R¹ and R⁸ together with the atoms to which they are attached do not form a ring, A is CH, W is selected from -CR¹¹-, and L² is selected from -(CR^{C0}R^{D0})ᵤO(CR^{C0}R^{D0})ₜ- and -(CR^{C0}R^{D0})ᵤS(O)ᵣ(CR^{C0}R^{D0})ₜ-;
   each R^{a1} and each R^{b1} are independently selected from hydrogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl and heteroaryl-C₁₋₄ alkyl, wherein alkyl, alkenyl, alkynyl, cycloalkyl, heterocyclyl, aryl and heteroaryl are each unsubstituted or substituted with at least one substituent, independently selected from R^{Y};
   or R^{a1} and R^{b1} together with the atom(s) to which they are attached form a heterocyclic ring of 4 to 12 members containing 0, 1 or 2 additional heteroatoms independently selected from oxygen, sulfur, nitrogen and phosphorus, and optionally substituted with 1, 2 or 3 R^{Y} groups;
   each R^{c1} and each R^{d1} are independently selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl and heteroaryl-C₁₋₄ alkyl, wherein alkyl, alkenyl, alkynyl, cycloalkyl, heterocyclyl, aryl and heteroaryl are each unsubstituted or substituted with at least one substituent, independently selected from R^{Y};
   or R^{c1} and R^{d1} together with the carbon atom(s) to which they are attached form a ring of 3 to 12 members containing 0, 1 or 2 heteroatoms independently selected from oxygen, sulfur and nitrogen, and optionally substituted with 1, 2 or 3 R^{Y} groups;
   each R^{e1} is independently selected from hydrogen, C₁₋₁₀ alkyl, C₃₋₁₀ cycloalkyl, C₃₋₁₀ cycloalkyl-C₁₋₄ alkyl, CN, NO₂, -OR^{a2}, -SR^{a2}, -S(O)ᵣR^{a2}, -C(O)R^{a2}, -C(O)OR^{a2}, -S(O)ᵣNR^{a2}R^{b2} and -C(O)NR^{a2}R^{b2};
   each R^{Y} is independently selected from C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, halogen, CN, NO₂, -(CR^{c2}R^{d2})ₜNR^{a2}R^{b2}, -(CR^{c2}R^{d2})ₜOR^{b2}, -(CR^{c2}R^{d2})ₜC(O)R^{a2}, -(CR^{c2}R^{d2})ₜC(=NR^{e2})R^{a2}, -(CR^{c2}R^{d2})ₜC(=N-OR^{b2})R^{a2}, -(CR^{c2}R^{d2})ₜC(O)OR^{b2}, -(CR^{c2}R^{d2})ₜOC(O)R^{b2}, -(CR^{c2}R^{d2})ₜC(O)NR^{a2}R^{b2}, -(CR^{c2}R^{d2})ₜNR^{a2}C(O)R^{b2}, -(CR^{c2}R^{d2})ₜC(=NR^{e2})NR^{a2}R^{b2}, -(CR^{c2}R^{d2})ₜNR^{a2}C(=NR^{e2})R^{b2}, -(CR^{c2}R^{d2})ₜOC(O)NR^{a2}R^{b2}, -(CR^{c2}R^{d2})ₜNR^{a2}C(O)OR^{b2}, -(CR^{c2}R^{d2})ₜNR^{a2}C(O)NR^{a2}R^{b2}, -(CR^{c2}R^{d2})ₜNR^{a2}C(S)NR^{a2}R^{b2}, -(CR^{c2}R^{d2})ₜNR^{a2}C(=NR^{e2})NR^{a2}R^{b2}, -(CR^{c2}R^{d2})ₜS(O)ᵣR^{b2}, -(CR^{c2}R^{d2})ₜS(O)(=NR^{e2})R^{b2}, -(CR^{c2}R^{d2})ₜN=S(O)R^{a2}R^{b2}, -(CR^{c2}R^{d2})ₜS(O)₂OR^{b2}, -(CR^{c2}R^{d2})ₜOS(O)₂R^{b2}, -(CR^{c2}R^{d2})ₜNR^{a2}S(O)ᵣR^{b2}, -(CR^{c2}R^{d2})ₜNR^{a2}S(O)(=NR^{e2})R^{b2}, -(CR^{c2}R^{d2})ₜS(O)ᵣNR^{a2}R^{b2}, -(CR^{c2}R^{d2})ₜS(O)(=NR^{e2})NR^{a2}R^{b2}, -(CR^{c2}R^{d2})ₜNR^{a2}S(O)₂NR^{a2}R^{b2}, -(CR^{c2}R^{d2})ₜNR^{a2}S(O)(=NR^{e2})NR^{a2}R^{b2}, -(CR^{c2}R^{d2})ₜP(O)R^{a2}R^{b2} and -(CR^{c2}R^{d2})ₜP(O)(OR^{a2})(OR^{b2}), wherein alkyl, alkenyl, alkynyl, cycloalkyl, heterocyclyl, aryl and heteroaryl are each unsubstituted or substituted with at least one substituent, independently selected from OH, CN, amino, halogen, C₁₋₁₀ alkyl, C₂₋₁₀ alkenyl, C₂₋₁₀ alkynyl, C₃₋₁₀ cycloalkyl, C₁₋₁₀ alkoxy, C₃₋₁₀ cycloalkoxy, C₁₋₁₀ alkylthio, C₃₋₁₀ cycloalkylthio, C₁₋₁₀ alkylamino, C₃₋₁₀ cycloalkylamino and di(C₁₋₁₀ alkyl)amino;
   each R^{a2} and each R^{b2} are independently selected from hydrogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, C₁₋₁₀ alkoxy, C₃₋₁₀ cycloalkoxy, C₁₋₁₀ alkylthio, C₃₋₁₀ cycloalkylthio, C₁₋₁₀ alkylamino, C₃₋₁₀ cycloalkylamino, di(C₁₋₁₀ alkyl)amino, heterocyclyl, heterocyclyl-C₁₋₄ alkyl, aryl, aryl-C₁₋₄ alkyl, heteroaryl and heteroaryl-C₁₋₄ alkyl, wherein alkyl, alkenyl, alkynyl, cycloalkyl, alkoxy, cycloalkoxy, alkylthio, cycloalkylthio, alkylamino, cycloalkylamino, heterocyclyl, aryl and heteroaryl are each unsubstituted or substituted with at least one substituent, independently selected from halogen, CN, C₁₋₁₀ alkyl, C₂₋₁₀ alkenyl, C₂₋₁₀ alkynyl, C₃₋₁₀ cycloalkyl, OH, C₁₋₁₀ alkoxy, C₃₋₁₀ cycloalkoxy, C₁₋₁₀ alkylthio, C₃₋₁₀ cycloalkylthio, amino, C₁₋₁₀ alkylamino, C₃₋₁₀ cycloalkylamino and di(C₁₋₁₀ alkyl)amino;
   or R^{a2} and R^{b2} together with the atom(s) to which they are attached form a heterocyclic ring of 4 to 12 members containing 0, 1 or 2 additional heteroatoms independently selected from oxygen, sulfur, nitrogen and phosphorus, and optionally substituted with 1 or 2 substituents, independently selected from halogen, CN, C₁₋₁₀ alkyl, C₂₋₁₀ alkenyl, C₂₋₁₀ alkynyl, C₃₋₁₀ cycloalkyl, OH, C₁₋₁₀ alkoxy, C₃₋₁₀ cycloalkoxy, C₁₋₁₀ alkylthio, C₃₋₁₀ cycloalkylthio, amino, C₁₋₁₀ alkylamino, C₃₋₁₀ cycloalkylamino and di(C₁₋₁₀ alkyl)amino;
   each R^{c2} and each R^{d2} are independently selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, C₁₋₁₀ alkoxy, C₃₋₁₀ cycloalkoxy, C₁₋₁₀ alkylthio, C₃₋₁₀ cycloalkylthio, C₁₋₁₀ alkylamino, C₃₋₁₀ cycloalkylamino, di(C₁₋₁₀ alkyl)amino, heterocyclyl, heterocyclyl-C₁₋₄ alkyl, aryl, aryl-C₁₋₄ alkyl, heteroaryl and heteroaryl-C₁₋₄ alkyl, wherein alkyl, alkenyl, alkynyl, cycloalkyl, alkoxy, cycloalkoxy, alkylthio, cycloalkylthio, alkylamino, cycloalkylamino, heterocyclyl, aryl and heteroaryl are each unsubstituted or substituted with at least one substituent, independently selected from halogen, CN, C₁₋₁₀ alkyl, C₂₋₁₀ alkenyl, C₂₋₁₀ alkynyl, C₃₋₁₀ cycloalkyl, OH, C₁₋₁₀ alkoxy, C₃₋₁₀ cycloalkoxy, C₁₋₁₀ alkylthio, C₃₋₁₀ cycloalkylthio, amino, C₁₋₁₀ alkylamino, C₃₋₁₀ cycloalkylamino and di(C₁₋₁₀ alkyl)amino;
   or R^{c2} and R^{d2} together with the carbon atom(s) to which they are attached form a ring of 3 to 12 members containing 0, 1 or 2 heteroatoms independently selected from oxygen, sulfur and nitrogen, and optionally substituted with 1 or 2 substituents, independently selected from halogen, CN, C₁₋₁₀ alkyl, C₂₋₁₀ alkenyl, C₂₋₁₀ alkynyl, C₃₋₁₀ cycloalkyl, OH, C₁₋₁₀ alkoxy, C₃₋₁₀ cycloalkoxy, C₁₋₁₀ alkylthio, C₃₋₁₀ cycloalkylthio, amino, C₁₋₁₀ alkylamino, C₃₋₁₀ cycloalkylamino and di(C₁₋₁₀ alkyl)amino;
   each R^{e2} is independently selected from hydrogen, CN, NO₂, C₁₋₁₀ alkyl, C₃₋₁₀ cycloalkyl, C₃₋₁₀ cycloalkyl-C₁₋₄ alkyl, C₁₋₁₀ alkoxy, C₃₋₁₀ cycloalkoxy, -C(O)C₁₋₄ alkyl, -C(O)C₃₋₁₀ cycloalkyl, -C(O)OC₁₋₄ alkyl, -C(O)OC₃₋₁₀ cycloalkyl, -C(O)N(C₁₋₄ alkyl)₂, -C(O)N(C₃₋₁₀ cycloalkyl)₂, -S(O)₂C₁₋₄ alkyl, -S(O)₂C₃₋₁₀ cycloalkyl, -S(O)₂N(C₁₋₄ alkyl)₂ and -S(O)₂N(C₃₋₁₀ cycloalkyl)₂;
   m, m1, m2, n, n1, n2, p, p1, p2 and q are independently selected from 0, 1, 2 and 3;
   each r is independently selected from 0, 1 and 2;
   each t is independently selected from 0, 1, 2, 3 and 4;
   each u is independently selected from 0, 1, 2, 3 and 4.

In another Embodiment (2), the invention provides a compound of Embodiment (1) or a pharmaceutically acceptable salt thereof, wherein at least one of X¹, X² and X³ is N.

In another Embodiment (3), the invention provides a compound of Embodiment (1) or (2) or a pharmaceutically acceptable salt thereof,
wherein X³ is CH, A is N, B is CH, Z is N, Y¹, Y² and Y³ are CH, shown as formula (II), 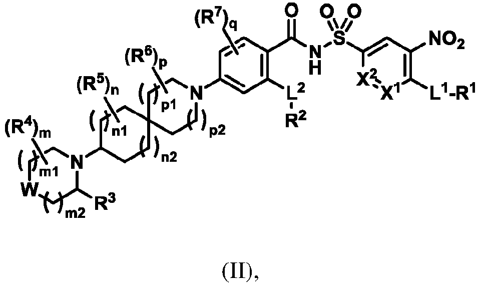
wherein
X¹ is selected from N and CR⁸, X² is selected from N and CR⁹, wherein at least one of X¹ and X² is N; W is selected from -CR¹¹-, -NR¹²-, -O- and -S(O)ᵣ-; L¹, L², W, R¹, R², R³, R⁴, R⁵, R⁶, R⁷, R⁸, R⁹, R¹¹, R¹², m, m1, m2, n, n1, n2, p, p1, p2 and q are as defined in formula (I).

In another Embodiment (4), the invention provides a compound of any one of Embodiments (1)-(3) or a pharmaceutically acceptable salt thereof, wherein L¹ and L² are independently selected from a bond, -(CR^{C0}R^{D0})ᵤ-, -(CR^{C0}R^{D0})ᵤO(CR^{C0}R^{D0})ₜ-, -(CR^{C0}R^{D0})ᵤNR^{A0}(CR^{C0}R^{D0})ₜ- and -S(O)ᵣ.

In another Embodiment (5), the invention provides a compound of any one of Embodiments (1)-(4) or a pharmaceutically acceptable salt thereof, wherein L¹ is selected from -(CR^{C0}R^{D0})ᵤ, -O-, -NH- and -S(O)ᵣ-, preferably, L¹ is selected from -CH₂-, -O-, -NH- and -S-. In an embodiment, u is 1. In an embodiment, L¹ is selected from -CH₂-, -O- and -S-. In an embodiment, L¹ is -NH-.

In another Embodiment (6), the invention provides a compound of any one of Embodiments (1)-(5) or a pharmaceutically acceptable salt thereof, wherein L² is selected from a bond, -O- and -S(O)ᵣ, preferably, L² is selected from a bond and -O-.

In another Embodiment (7), the invention provides a compound of any one of Embodiments (1)-(6) or a pharmaceutically acceptable salt thereof, wherein R¹ is selected from C₁₋₁₀ alkyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A1}R^{B1}, -OR^{A1}, -C(O)R^{A1}, -C(O)OR^{A1}, -OC(O)R^{A1}, -C(O)NR^{A1}R^{B1}, -NR^{A1}C(O)R^{B1}, -OC(O)NR^{A1}R^{B1}, -NR^{A1}C(O)OR^{B1}, -NR^{A1}C(O)NR^{A1}R^{B1} and -S(O)ᵣR^{A1}, wherein alkyl, cycloalkyl, heterocyclyl, aryl and heteroaryl are each unsubstituted or substituted with at least one substituent, independently selected from R^{X}.

In another Embodiment (8), the invention provides a compound of any one of Embodiments (1)-(7) or a pharmaceutically acceptable salt thereof, wherein R¹ is selected from C₁₋₁₀ alkyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, wherein alkyl, cycloalkyl, heterocyclyl, aryl and heteroaryl are each unsubstituted or substituted with at least one substituent, independently selected from R^{X}. In an embodiment, R¹ is selected from C₃₋₁₀ cycloalkyl, C₃₋₁₀ cycloalkyl-C₁₋₄ alkyl, heterocyclyl and heterocyclyl-C₁₋₄ alkyl, wherein alkyl, cycloalkyl and heterocyclyl are each unsubstituted or substituted with at least one substituent, independently selected from R^{X}.

In another Embodiment (9), the invention provides a compound of any one of Embodiments (1)-(8) or a pharmaceutically acceptable salt thereof, wherein R¹ is selected from 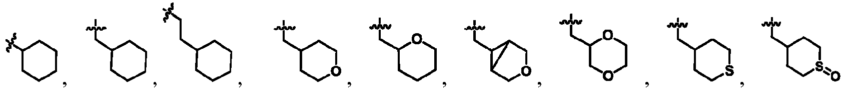 and 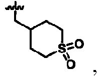 which are unsubstituted or substituted with at least one substituent, independently selected from R^{X}.

In another Embodiment (10), the invention provides a compound of any one of Embodiments (1)-(9) or a pharmaceutically acceptable salt thereof, wherein each substituent R^{X} of R¹ is independently selected from C₁₋₁₀ alkyl, C₃₋₁₀ cycloalkyl, halogen, CN, NO₂, -(CR^{c1}R^{d1})ₜNR^{a1}R^{b1}, -(CR^{c1}R^{d1})ₜOR^{b1}, -(CR^{c1}R^{d1})ₜC(O)R^{a1} and -(CR^{c1}R^{d1})N=S(O)R^{a1}R^{b1}, wherein alkyl, cycloalkyl are each unsubstituted or substituted with at least one substituent, independently selected from R^{Y}.

In another Embodiment (11), the invention provides a compound of any one of Embodiments (1)-(10) or a pharmaceutically acceptable salt thereof, wherein each R^{a1} and each R^{b1} are independently selected from hydrogen, C₁₋₁₀ alkyl, C₂₋₁₀ alkenyl and C₂₋₁₀ alkynyl, wherein alkyl, alkenyl and alkynyl are each unsubstituted or substituted with at least one substituent, independently selected from R^{Y};
or R^{a1} and R^{b1} together with the atom(s) to which they are attached form a heterocyclic ring of 4 to 8 members containing 0, 1 or 2 additional heteroatoms independently selected from oxygen, sulfur, nitrogen and phosphorus, and optionally substituted with 1, 2 or 3 R^{Y} groups.

In another Embodiment (12), the invention provides a compound of any one of Embodiments (1)-(11) or a pharmaceutically acceptable salt thereof, wherein each substituent R^{X} of R¹ is independently selected from OH, CN, halogen, C₁₋₁₀ alkyl,

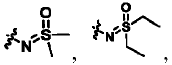

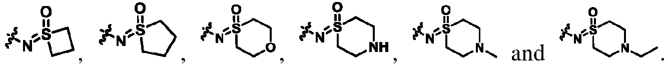

In another Embodiment (13), the invention provides a compound of any one of Embodiments (1)-(12) or a pharmaceutically acceptable salt thereof, wherein R¹ is selected from

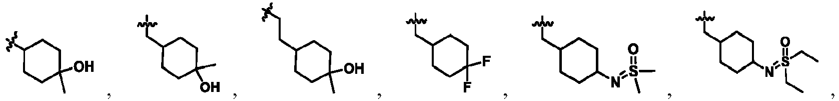

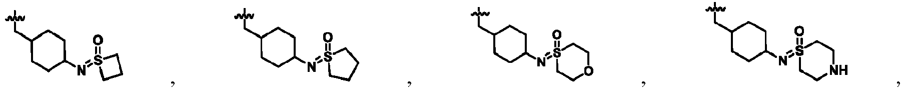

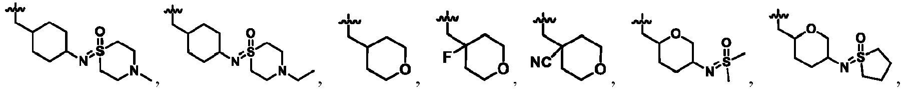

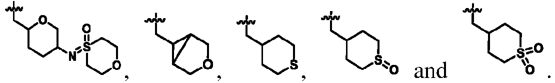

In another Embodiment (14), the invention provides a compound of any one of Embodiments (1)-(13) or a pharmaceutically acceptable salt thereof, wherein R⁸ is selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A8}R^{B8}, -OR^{A8}, -C(O)R^{A8}, -C(O)OR^{A8}, -OC(O)R^{A8}, -C(O)NR^{A8}R^{B8}, -NR^{A8}C(O)R^{B8}, -OC(O)NR^{A8}R^{B8}, -NR^{A8}C(O)OR^{B8}, -NR^{A8}C(O)NR^{A8}R^{B8}, -NR^{A8}C(S)NR^{A8}R^{B8}, -S(O)ᵣR^{A8}, -S(O)₂OR^{A8}, -OS(O)₂R^{A8}, -NR^{A8}S(O)ᵣR^{B8} and -S(O)ᵣNR^{A8}R^{B8}, wherein alkyl, alkenyl, alkynyl, cycloalkyl, heterocyclyl, aryl and heteroaryl are each unsubstituted or substituted with at least one substituent, independently selected from R^{X}.

In another Embodiment (15), the invention provides a compound of any one of Embodiments (1)-(14) or a pharmaceutically acceptable salt thereof, wherein R⁸ is selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, CN, NO₂, -NR^{A8}R^{B8}, -OR^{A8}, -C(O)R^{A8}, -C(O)OR^{A8}, -OC(O)R^{A8} and -S(O)ᵣR^{A8}, wherein alkyl, alkenyl, alkynyl, cycloalkyl and heterocyclyl are each unsubstituted or substituted with at least one substituent, independently selected from R^{X}, preferably, R⁸ is hydrogen.

In another Embodiment (16), the invention provides a compound of any one of Embodiments (1)-(15) or a pharmaceutically acceptable salt thereof, wherein R¹ and R⁸ together with the atoms to which they are attached form a C₅₋₈ cycloalkyl or heterocyclic ring of 5 to 12 members containing 1, 2 or 3 heteroatoms independently selected from oxygen, sulfur, nitrogen and phosphorus, and optionally substituted with 1, 2 or 3 R^{X} groups.

In another Embodiment (17), the invention provides a compound of any one of Embodiments (1)-(16) or a pharmaceutically acceptable salt thereof, wherein R¹ and R⁸ together with the atoms to which they are attached form a heterocyclic ring of 5 to 12 members containing 1, 2 or 3 heteroatoms independently selected from oxygen, sulfur and nitrogen, and optionally substituted with 1, 2 or 3 R^{X} groups.

In another Embodiment (18), the invention provides a compound of any one of Embodiments (1)-(17) or a pharmaceutically acceptable salt thereof, wherein the moiety 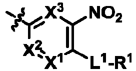 in Formula (I) and the moiety 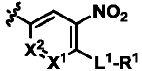 in Formula (II) are selected from 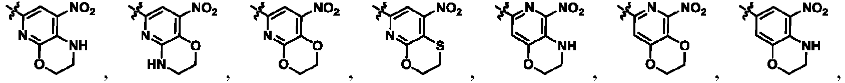 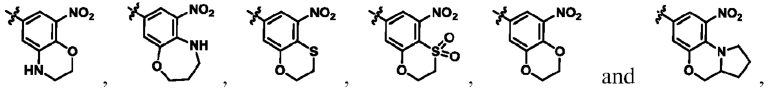 wherein the heterocyclic ring formed by R¹ and R⁸ is optionally substituted with 1, 2 or 3 R^{X} groups, wherein the 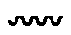 symbol indicates the point of attachment to the rest of the molecule. In another embodiment, the moiety 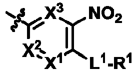 in Formula (I) and the moiety 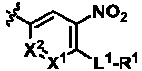 in Formula (II) are selected from 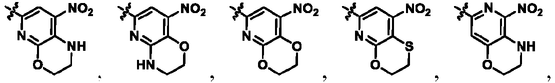 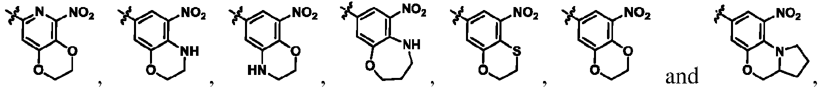 wherein the heterocyclic ring formed by R¹ and R⁸ is optionally substituted with 1, 2 or 3 R^{X} groups, wherein the 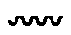 symbol indicates the point of attachment to the rest of the molecule.

In another Embodiment (19), the invention provides a compound of any one of Embodiment (1)-(18) or a pharmaceutically acceptable salt thereof, wherein each substituent R^{X} of the 5 to 12 membered heterocyclic ring is independently selected from C₁₋₁₀ alkyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, halogen, CN, NO₂, -(CR^{c1}R^{d1})ₜNR^{a1}R^{b1} and -(CR^{c1}R^{d1})ₜOR^{b1}, wherein alkyl, cycloalkyl, heterocyclyl, aryl and heteroaryl are each unsubstituted or substituted with at least one substituent, independently selected from R^{Y}.

In another Embodiment (20), the invention provides a compound of any one of Embodiment (1)-(19) or a pharmaceutically acceptable salt thereof, wherein each substituent R^{X} of the 5 to 12 membered heterocyclic ring is selected from halogen, CN, NO₂, OH, C₁₋₈ alkyl, 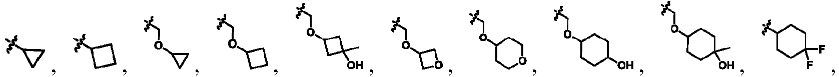 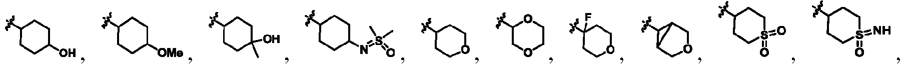 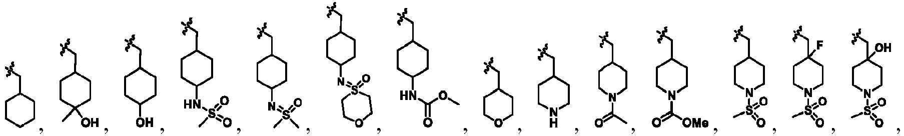 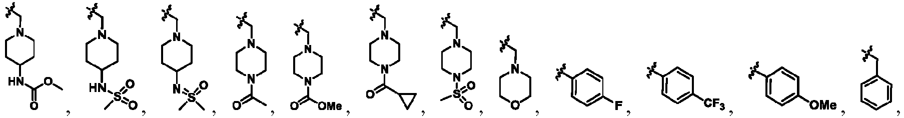 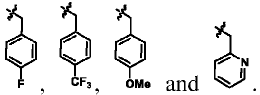 In another embodiment, the substituent R^{X} of 5 to 7 membered heterocyclic ring is selected from halogen, CN, NO₂, OH, C₁₋₈ alkyl, 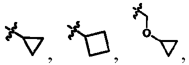 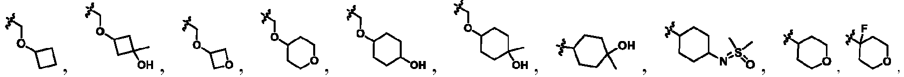 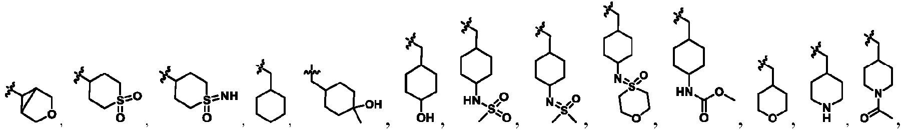 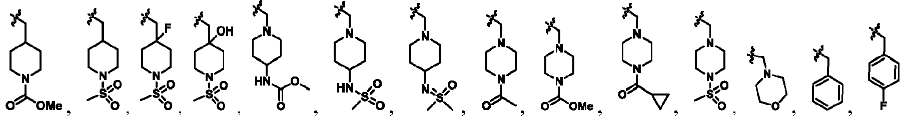 and

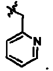

In another Embodiment (21), the invention provides a compound of any one of Embodiments (1)-(20) or a pharmaceutically acceptable salt thereof, wherein R² is selected from heterocyclyl and heteroaryl, wherein heterocyclyl and heteroaryl are each unsubstituted or substituted with at least one substituent, independently selected from R^{X}. In an embodiment, R² is a heteroaryl or heterocyclyl comprising a pyridyl condensed pyrrole structure.

In another Embodiment (22), the invention provides a compound of any one of Embodiments (1)-(21) or a pharmaceutically acceptable salt thereof, wherein R² is selected from 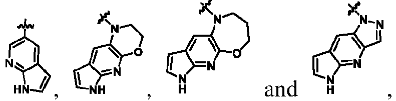 which are unsubstituted or substituted with at least one substituent, independently selected from R^{X}, preferably, R² is 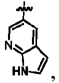 which are unsubstituted or substituted with at least one substituent, independently selected from R^{X}. In another embodiment, R² is selected from 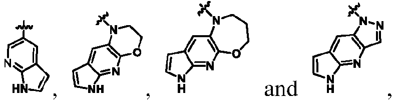 which are unsubstituted or substituted with at least one substituent, independently selected from R^{X}, preferably, R² is

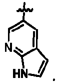

In another Embodiment (23), the invention provides a compound of any one of Embodiments (1)-(22) or a pharmaceutically acceptable salt thereof, wherein each substituent R^{X} of R² is independently selected from C₁₋₁₀ alkyl, C₂₋₁₀ alkenyl, C₂₋₁₀ alkynyl, C₃₋₁₀ cycloalkyl, heterocyclyl, heterocyclyl-C₁₋₄ alkyl, halogen, CN, NO₂, -(CR^{c1}R^{d1})ₜNR^{a1}R^{b1} , -(CR^{c1}R^{d1})ₜOR^{b1}, -(CR^{c1}R^{d1})ₜC(O)OR^{b1}, -(CR^{c1}R^{d1})ₜC(O)NR^{a1}R^{b1}, -(CR^{c1}R^{d1})ₜNR^{a1}C(O)R^{b1}, -(CR^{c1}R^{d1})ₜNR^{a1}C(O)OR^{b1}, -(CR^{c1}R^{d1})ₜNR^{a1}C(O)NR^{a1}R^{b1}, -(CR^{c1}R^{d1})ₜS(O)₂OR^{b1}, -(CR^{c1}R^{d1})ₜS(O)ᵣR^{b1}, -(CR^{c1}R^{d1})ₜNR^{a1}S(O)ᵣR^{b1}, -(CR^{c1}R^{d1})ₜS(O)ᵣNR^{a1}R^{b1} and -(CR^{c1}R^{d1})ₜNR^{a1}S(O)₂NR^{a1}R^{b1}, wherein alkyl, alkenyl, alkynyl, cycloalkyl and heterocyclyl are each unsubstituted or substituted with at least one substituent, independently selected from R^{Y}. In another embodiment, each substituent R^{X} of R² is independently selected from C₁₋₁₀ alkyl, C₂₋₁₀ alkenyl, C₂₋₁₀ alkynyl, C₃₋₁₀ cycloalkyl, heterocyclyl, heterocyclyl-C₁₋₄ alkyl, halogen, CN, NO₂, -(CR^{c1}R^{d1})ₜNR^{a1}R^{b1} , -(CR^{c1}R^{d1})ₜOR^{b1}, -(CR^{c1}R^{d1})ₜC(O)OR^{b1}, -(CR^{c1}R^{d1})ₜNR^{a1}C(O)R^{b1}, -(CR^{c1}R^{d1})ₜNR^{a1}C(O)OR^{b1}, -(CR^{c1}R^{d1})ₜNR^{a1}C(O)NR^{a1}R^{b1}, -(CR^{c1}R^{d1})ₜS(O)₂OR^{b1}, -(CR^{c1}R^{d1})ₜS(O)ᵣR^{b1}, -(CR^{c1}R^{d1})ₜNR^{a1}S(O)ᵣR^{b1}, -(CR^{c1}R^{d1})ₜS(O)ᵣNR^{a1}R^{b1} and -(CR^{c1}R^{d1})ₜNR^{a1}S(O)₂NR^{a1}R^{b1}, wherein alkyl, alkenyl, alkynyl, cycloalkyl and heterocyclyl are each unsubstituted or substituted with at least one substituent, independently selected from R^{Y}. In another embodiment, each substituent R^{X} of R² is independently selected from C₁₋₁₀ alkyl, C₃₋₁₀ cycloalkyl, halogen, CN, NO₂, -(CR^{c1}R^{d1})ₜNR^{a1}R^{b1} and -(CR^{c1}R^{d1})ₜOR^{b1}, wherein alkyl and cycloalkyl are each unsubstituted or substituted with at least one substituent, independently selected from R^{Y}, preferably, each substituent R^{X} of R² is selected from halogen, CN, NH₂, methyl, trifluoromethyl, cyclopropyl, methoxy, methoxyethoxy, and difluoromethoxy.

In another Embodiment (24), the invention provides a compound of any one of Embodiments (1)-(23) or a pharmaceutically acceptable salt thereof, wherein R^{a1} and R^{b1} are independently selected from hydrogen, C₁₋₁₀ alkyl, C₃₋₁₀ cycloalkyl, C₃₋₁₀ cycloalkyl-C₁₋₄ alkyl, heterocyclyl and heterocyclyl-C₁₋₄ alkyl, wherein alkyl, cycloalkyl and heterocyclyl are each unsubstituted or substituted with at least one substituent, independently selected from R^{Y}.

In another Embodiment (25), the invention provides a compound of any one of Embodiments (1)-(24) or a pharmaceutically acceptable salt thereof, wherein R² is selected from 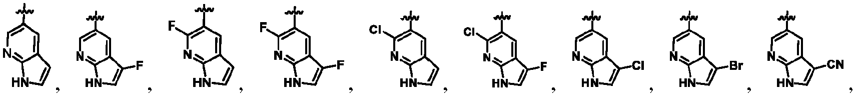 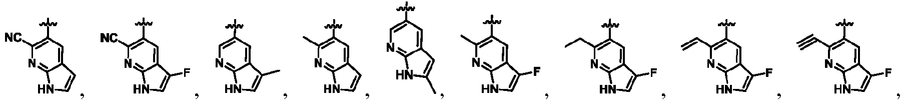 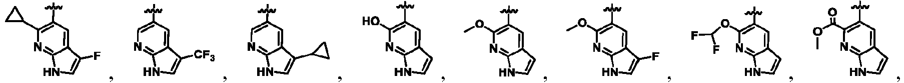 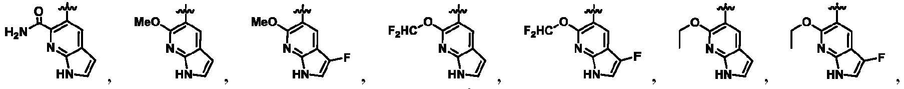 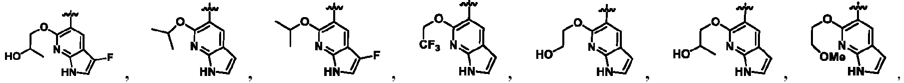 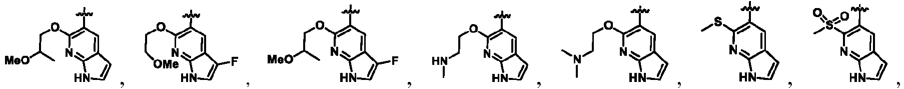 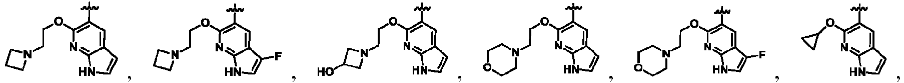 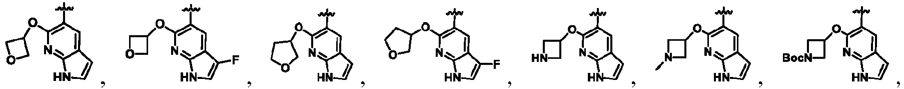 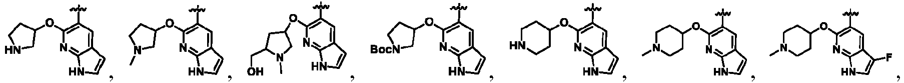 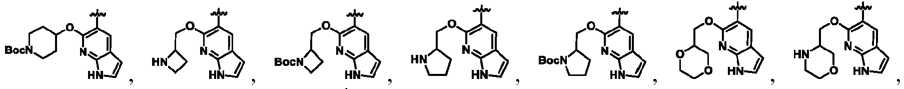 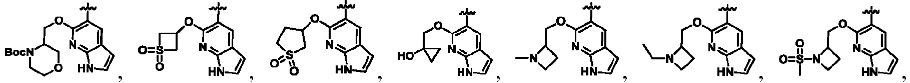 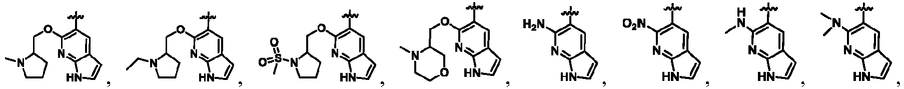 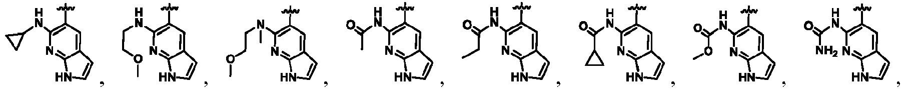 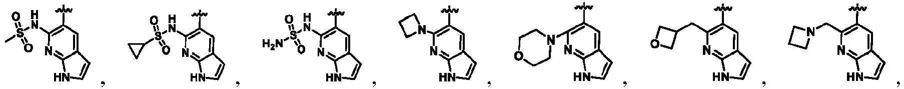 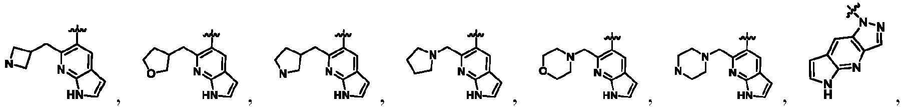 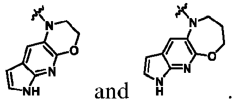 In another embodiment, R² is selected from 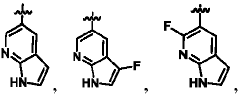 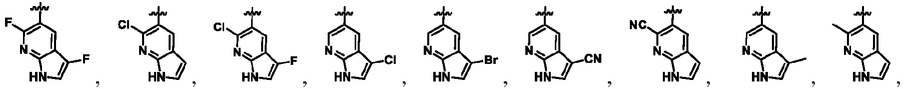 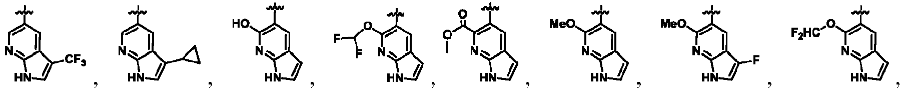 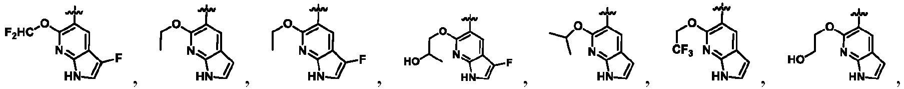 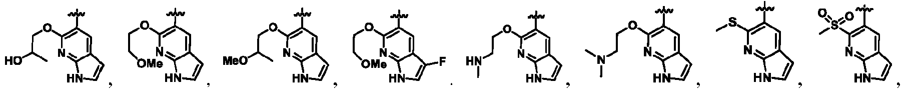 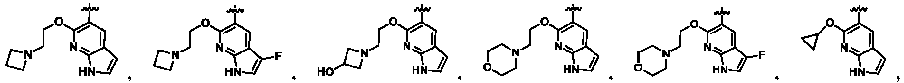 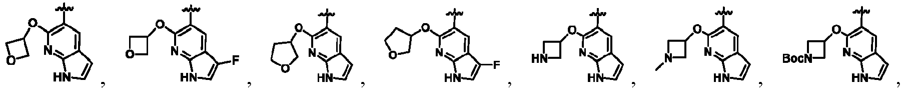 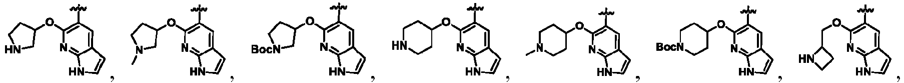 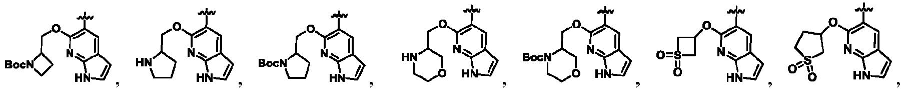 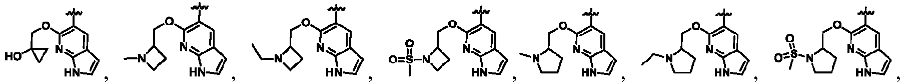 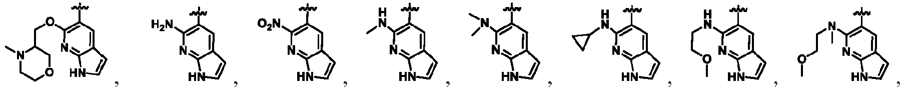 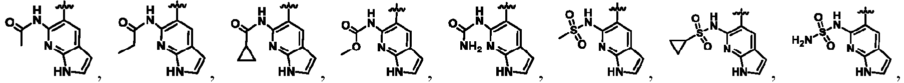 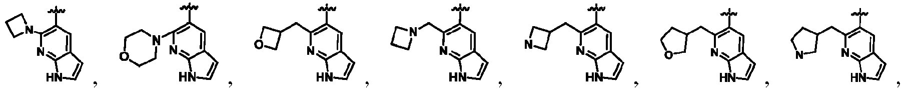 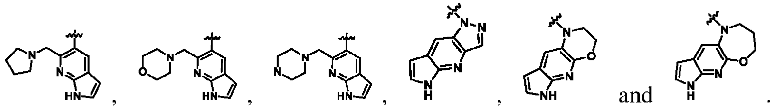 In another embodiment, R² is selected from

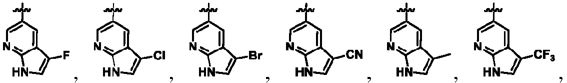

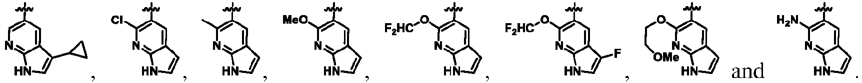

In another Embodiment (26), the invention provides a compound of any one of Embodiments (1)-(25) or a pharmaceutically acceptable salt thereof, wherein W is selected from -CH₂-, -NR¹²-, -O-, -S(O)ᵣ- and -S(O)(=NR¹²)-. In another embodiment, W is selected from -CH-, -NR¹²-, -O-, -S(O)ᵣ- and -S(O)(=NR¹²)-.

In another embodiment, X³ is CR¹⁰, and X¹ is N. In another embodiment, X³ is CR¹⁰, and X² is N.

In another Embodiment (27), the invention provides a compound of any one of Embodiment (1)-(26) or a pharmaceutically acceptable salt thereof, wherein X¹ is CR⁸, X² is CR⁹ and X³ is CR¹⁰.

In another Embodiment (28), the invention provides a compound of any one of Embodiments (1)-(27) or a pharmaceutically acceptable salt thereof, wherein Y¹, Y² and Y³ are CH.

In another Embodiment (29), the invention provides a compound of any one of Embodiment (1)-(28) or a pharmaceutically acceptable salt thereof, wherein R¹ and R⁸ together with the atoms to which they are attached form a C₅₋₈ cycloalkyl or heterocyclic ring of 5 to 12 members containing 1, 2 or 3 heteroatoms independently selected from oxygen, sulfur, nitrogen and phosphorus, and optionally substituted with 1, 2 or 3 R^{X} groups.

In another Embodiment (30), the invention provides a compound of any one of Embodiments (1)-(29) or a pharmaceutically acceptable salt thereof, wherein R¹ and R⁸ together with the atoms to which they are attached form a heterocyclic ring of 5 to 12 members containing 1, 2 or 3 heteroatoms independently selected from oxygen, sulfur and nitrogen, and optionally substituted with 1, 2 or 3 R^{X} groups.

In another Embodiment (31), the invention provides a compound of any one of Embodiments (1)-(30) or a pharmaceutically acceptable salt thereof, wherein the moiety 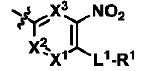 in Formula (I) is selected from 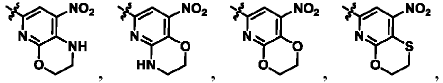 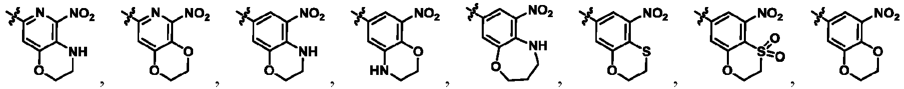 and 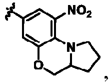 wherein the heterocyclic ring formed by R¹ and R⁸ is optionally substituted with 1, 2 or 3 R^{X} groups, wherein the 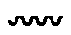 symbol indicates the point of attachment to the rest of the molecule. In another embodiment, the moiety 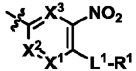 in Formula (I) is selected from 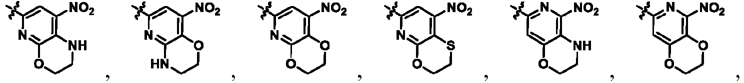 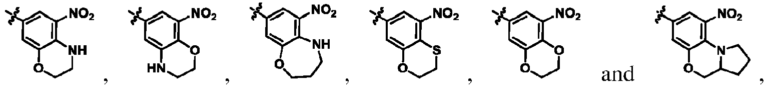 wherein the heterocyclic ring formed by R¹ and R⁸ is optionally substituted with 1, 2 or 3 R^{X} groups, wherein the 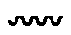 symbol indicates the point of attachment to the rest of the molecule. In another embodiment, the moiety 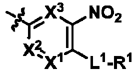 in Formula (I) is selected from 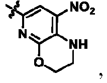 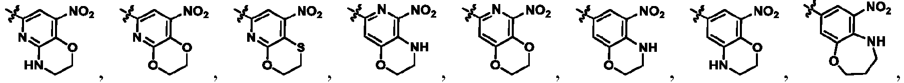 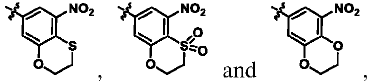 wherein the heterocyclic ring formed by R¹ and R⁸ is optionally substituted with 1, 2 or 3 R^{X} groups, wherein the 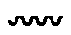 symbol indicates the point of attachment to the rest of the molecule.

In another Embodiment (32), the invention provides a compound of any one of Embodiments (1)-(31) or a pharmaceutically acceptable salt thereof, wherein each substituent R^{X} of the 5 to 12 membered heterocyclic ring is independently selected from C₁₋₁₀ alkyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, halogen, CN, NO₂, -(CR^{c1}R^{d1})ₜNR^{a1}R^{b1} and -(CR^{c1}R^{d1})ₜOR^{b1}, wherein alkyl, cycloalkyl, heterocyclyl, aryl and heteroaryl are each unsubstituted or substituted with at least one substituent, independently selected from R^{Y}.

In another Embodiment (33), the invention provides a compound of any one of Embodiments (1)-(32) or a pharmaceutically acceptable salt thereof, wherein each substituent R^{X} of the 5 to 12 membered heterocyclic ring is selected from halogen, CN, NO₂, OH, C₁₋₈ alkyl, 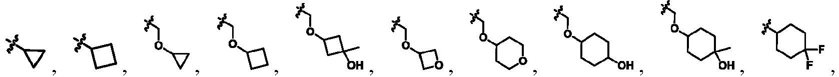 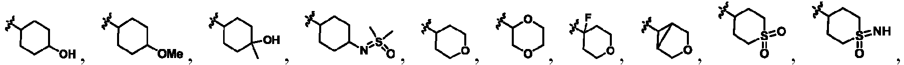 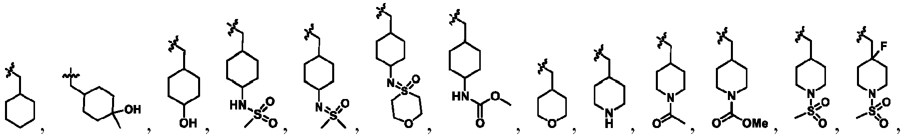 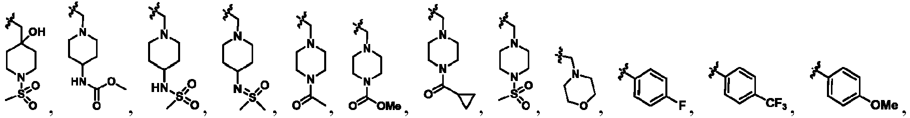 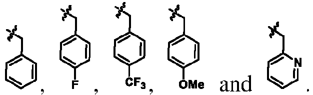 In another embodiment, each substituent R^{X} of the 5 to 7 membered heterocyclic ring is selected from halogen, CN, NO₂, OH, C₁₋₈ alkyl,

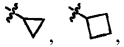

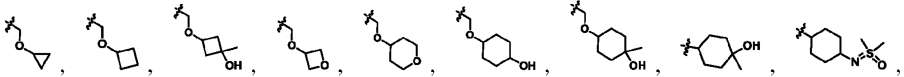

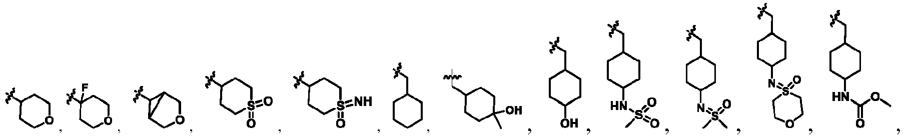

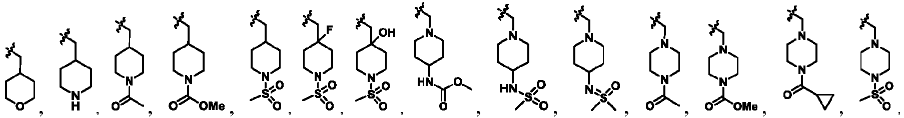

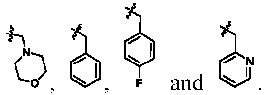

In another Embodiment (34), the invention provides a compound of any one of Embodiments (1)-(33) or a pharmaceutically acceptable salt thereof, wherein L¹ is selected from a bond, -(CR^{C0}R^{D0})ᵤ-, -(CR^{C0}R^{D0})ᵤ(CR^{C0}R^{D0})ₜ- and -(CR^{C0}R^{D0})ᵤS(O)ᵣ(CR^{C0}R^{D0})ₜ-, preferably, L¹ is selected from a bond, -CH₂-, -O- and -S-.

In another Embodiment (35), the invention provides a compound of any one of Embodiments (1)-(34) or a pharmaceutically acceptable salt thereof, wherein L¹ is selected from -(CR^{C0}R^{D0})ᵤNR^{A0}(CR^{C0}R^{D0})ₜ-, L² is selected from a bond, -(CR^{C0}R^{D0})ᵤO(CR^{C0}R^{D0})ₜ- and -(CR^{C0}R^{D0})ᵤS(O)ᵣ(CR^{C0}R^{D0})ₜ-, preferably, L² is selected from a bond and -O-.

In another Embodiment (36), the invention provides a compound of any one of Embodiments (1)-(35) or a pharmaceutically acceptable salt thereof, wherein Y¹, Y² and Y³ are CH, L¹ is -NH- and L² is O, shown as formula (III), 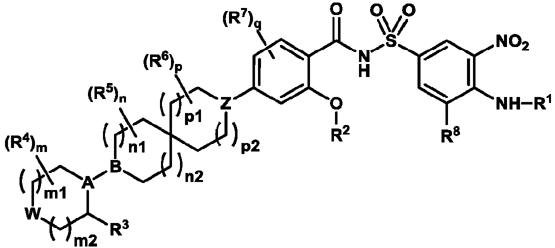 wherein
W, A, B, Z, R¹, R², R³, R⁴, R⁵, R⁶, R⁷, m, m1, m2, n, n1, n2, p, p1, p2 and q are as defined in formula (I).

In another Embodiment (37), the invention provides a compound of any one of Embodiments (1)-(36) or a pharmaceutically acceptable salt thereof, R² is selected from heteroaryl and heterocyclyl, wherein heteroaryl and heterocyclyl are each unsubstituted or substituted with at least one substituent, independently selected from R^{X}. In an embodiment, R² is a heteroaryl or heterocyclyl comprising a pyridyl condensed pyrrole structure.

In another Embodiment (38), the invention provides a compound of any one of Embodiments (1)-(37) or a pharmaceutically acceptable salt thereof, wherein R² is selected from 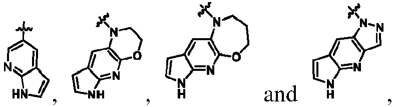 which are unsubstituted or substituted with at least one substituent, independently selected from R^{X}. In another embodiment, each substituent R^{X} of R² is selected from C₁₋₁₀ alkyl, C₂₋₁₀ alkenyl, C₂₋₁₀ alkynyl, C₃₋₁₀ cycloalkyl, halogen, CN, NO₂, -(CR^{c1}R^{d1})ₜNR^{a1}R^{b1}, -(CR^{c1}R^{d1})ₜOR^{b1}, -(CR^{c1}R^{d1})ₜS(O)₂OR^{b1} and -(CR^{c1}R^{d1})ₜNR^{a1}S(O)ᵣR^{b1}, wherein alkyl, alkenyl, alkynyl and cycloalkyl are each unsubstituted or substituted with at least one substituent, independently selected from R^{Y}, preferably, each substituent R^{X} of R² is selected from halogen, CN, NH₂, methyl, methoxy and difluoromethoxy.

In another Embodiment (39), the invention provides a compound of any one of Embodiment (1)-(38) or a pharmaceutically acceptable salt thereof, wherein when L² is -O- and R² is 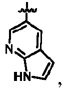 R² is substituted with at least one R^{X}.

In another Embodiment (40), the invention provides a compound of any one of Embodiments (1)-(39) or a pharmaceutically acceptable salt thereof, wherein each substituent R^{X} of R² is independently selected from C₁₋₁₀ alkyl, C₂₋₁₀ alkenyl, C₂₋₁₀ alkynyl, C₃₋₁₀ cycloalkyl, heterocyclyl, heterocyclyl-C₁₋₄ alkyl, halogen, CN, NO₂, -(CR^{c1}R^{d1})ₜNR^{a1}R^{b1}, -(CR^{c1}R^{d1})ₜOR^{b1}, -(CR^{c1}R^{d1})ₜC(O)OR^{b1}, -(CR^{c1}R^{d1})ₜC(O)NR^{a1}R^{b1}, -(CR^{c1}R^{d1})ₜNR^{a1}C(O)R^{b1}, -(CR^{c1}R^{d1})ₜNR^{a1}C(O)OR^{b1}, -(CR^{c1}R^{d1})ₜNR^{a1}C(O)NR^{a1}R^{b1}, -(CR^{c1}R^{d1})ₜS(O)₂OR^{b1}, -(CR^{c1}R^{d1})ₜS(O)ᵣR^{b1}, -(CR^{c1}R^{d1})ₜNR^{a1}S(O)ᵣR^{b1}, -(CR^{c1}R^{d1})ₜS(O)ᵣNR^{a1}R^{b1} and -(CR^{c1}R^{d1})ₜNR^{a1}S(O)₂NR^{a1}R^{b1}, wherein alkyl, alkenyl, alkynyl, cycloalkyl and heterocyclyl are each unsubstituted or substituted with at least one substituent, independently selected from R^{Y}. In another embodiment, each substituent R^{X} of R² is independently selected from C₁₋₁₀ alkyl, C₂₋₁₀ alkenyl, C₂₋₁₀ alkynyl, C₃₋₁₀ cycloalkyl, heterocyclyl, heterocyclyl-C₁₋₄ alkyl, halogen, CN, NO₂, -(CR^{c1}R^{d1})ₜNR^{a1}R^{b1}, -(CR^{c1}R^{d1})ₜOR^{b1}, -(CR^{c1}R^{d1})ₜC(O)OR^{b1}, -(CR^{c1}R^{d1})ₜNR^{a1}C(O)R^{b1}, -(CR^{c1}R^{d1})ₜNR^{a1}C(O)OR^{b1}, -(CR^{c1}R^{d1})ₜNR^{a1}C(O)NR^{a1}R^{b1}, -(CR^{c1}R^{d1})ₜS(O)₂OR^{b1}, -(CR^{c1}R^{d1})ₜS(O)ᵣR^{b1}, -(CR^{c1}R^{d1})ₜNR^{a1}S(O)ᵣR^{b1}, -(CR^{c1}R^{d1})ₜS(O)ᵣNR^{a1}R^{b1} and -(CR^{c1}R^{d1})ₜNR^{a1}S(O)₂NR^{a1}R^{b1}, wherein alkyl, alkenyl, alkynyl, cycloalkyl and heterocyclyl are each unsubstituted or substituted with at least one substituent, independently selected from R^{Y}.

In another embodiment, when L² is -O- and R² is 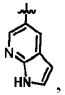 R² is substituted with at least one R^{X}, each substituent R^{X} of R² is independently selected from C₁₋₁₀ alkyl, C₃₋₁₀ cycloalkyl, halogen, CN, NO₂, -(CR^{c1}R^{d1})ₜNR^{a1}R^{b1} and -(CR^{c1}R^{d1})ₜOR^{b1}, wherein alkyl and cycloalkyl are each unsubstituted or substituted with at least one substituent, independently selected from R^{Y}, preferably, each substituent R^{X} of R² is selected from halogen, CN, NH₂, methyl, trifluoromethyl, cycloprpyl, methoxy, methoxyethoxy, and difluoromethoxy.

In another Embodiment (41), the invention provides a compound of any one of Embodiments (1)-(40) or a pharmaceutically acceptable salt thereof, wherein R² is selected from 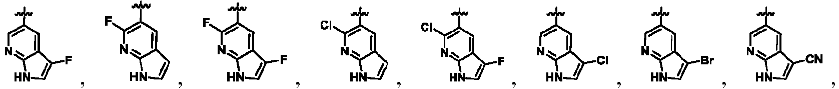 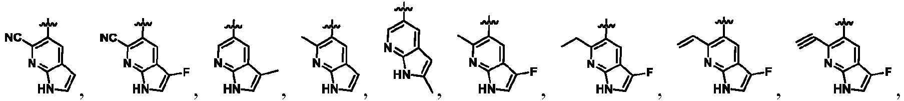 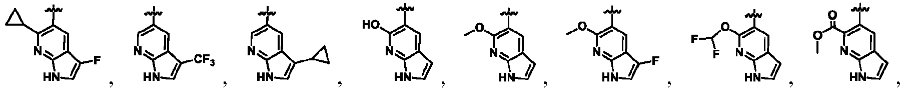 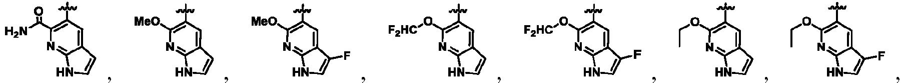 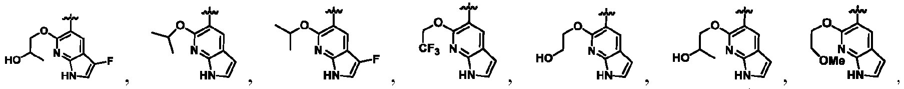 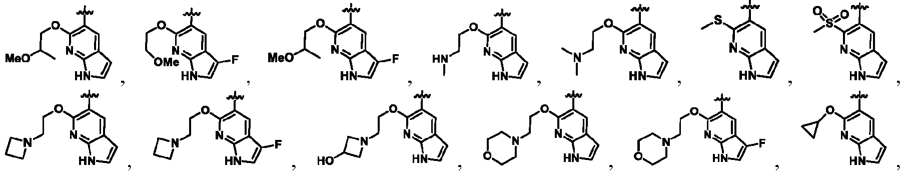 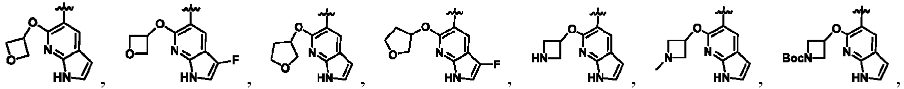 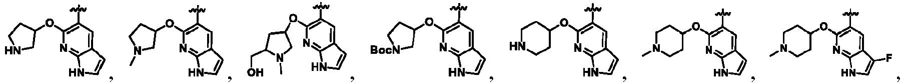 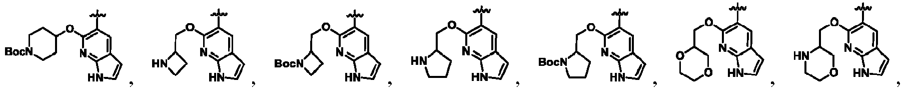 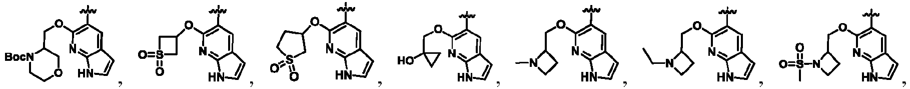 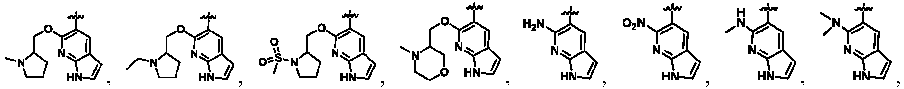 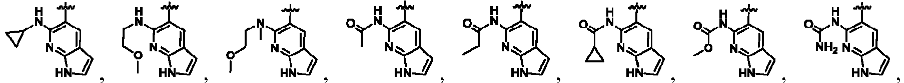 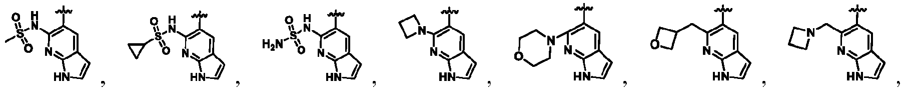 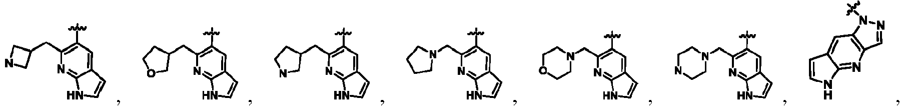 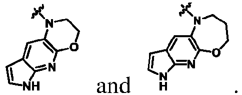 In another embodiment, R² is selected from 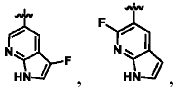 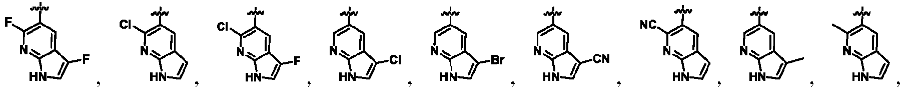 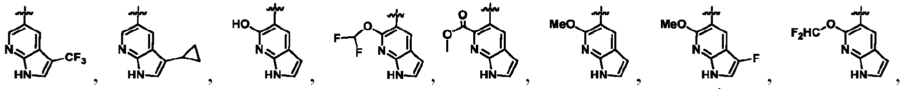 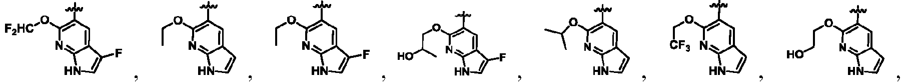 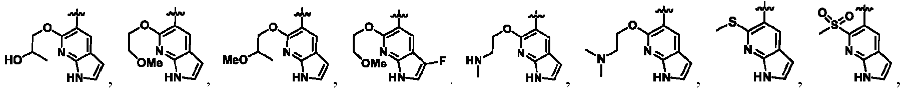 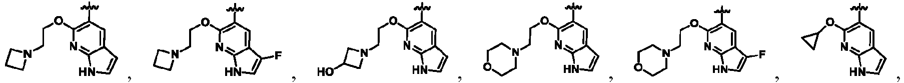 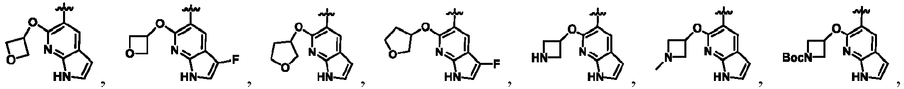 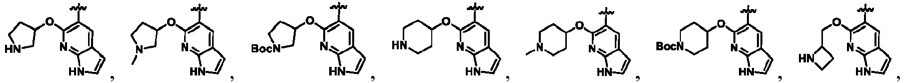 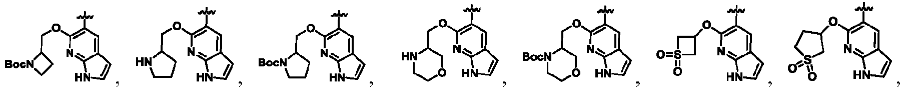 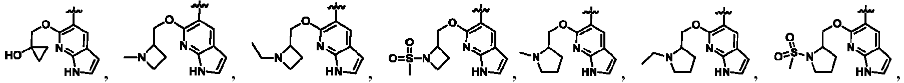 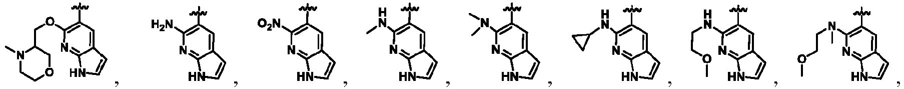 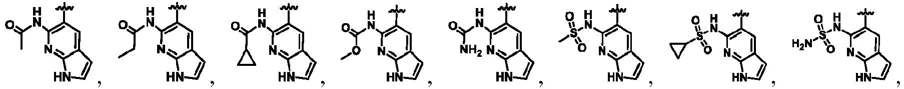 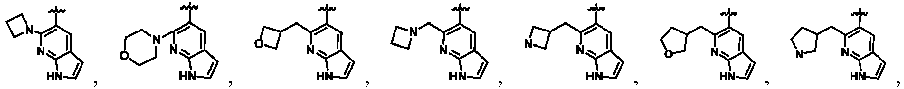 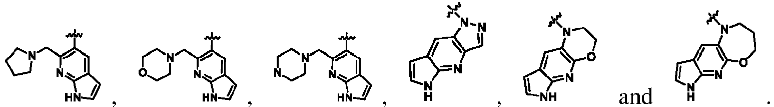 In another embodiment, R² is selected from

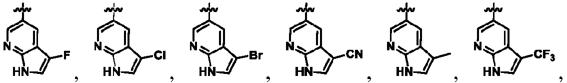

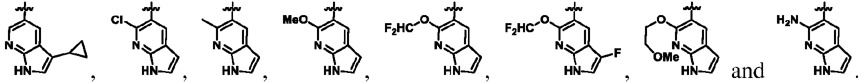

In another Embodiment (42), the invention provides a compound of any one of Embodiment (1)-(41) or a pharmaceutically acceptable salt thereof, wherein R¹ and R⁸ together with the atoms to which they are attached do not form a ring.

In another Embodiment (43), the invention provides a compound of any one of Embodiments (1)-(42) or a pharmaceutically acceptable salt thereof, wherein A is CH, W is selected from -CR¹¹-.

In another Embodiment (44), the invention provides a compound of any one of Embodiment (1)-(43) or a pharmaceutically acceptable salt thereof, wherein L² is selected from -(CR^{C0}R^{D0})ᵤO(CR^{C0}R^{D0})ₜ- and -(CR^{C0}R^{D0})ᵤS(O)ᵣ(CR^{C0}R^{D0})ₜ-, preferably, L² is selected from -O- and S(O)ᵣ.

In another Embodiment (45), the invention provides a compound of any one of Embodiments (1)-(44) or a pharmaceutically acceptable salt thereof, wherein R¹ is selected from C₁₋₁₀ alkyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A1}R^{B1}, -OR^{A1}, -C(O)R^{A1}, -C(O)OR^{A1}, -OC(O)R^{A1}, -C(O)NR^{A1}R^{B1}, -NR^{A1}C(O)R^{B1}, -OC(O)NR^{A1}R^{B1}, -NR^{A1}C(O)OR^{B1}, -NR^{A1}C(O)NR^{A1}R^{B1} and -S(O)ᵣR^{A1}, wherein alkyl, cycloalkyl, heterocyclyl, aryl and heteroaryl are each unsubstituted or substituted with at least one substituent, independently selected from R^{X}. In an embodiment, R¹ is selected from C₃₋₁₀ cycloalkyl, C₃₋₁₀ cycloalkyl-C₁₋₄ alkyl, heterocyclyl and heterocyclyl-C₁₋₄ alkyl, wherein alkyl, cycloalkyl and heterocyclyl are each unsubstituted or substituted with at least one substituent, independently selected from R^{X}.

In another Embodiment (46), the invention provides a compound of any one of Embodiments (1)-(45) or a pharmaceutically acceptable salt thereof, wherein R¹ is selected from C₁₋₁₀ alkyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, wherein alkyl, cycloalkyl, heterocyclyl, aryl and heteroaryl are each unsubstituted or substituted with at least one substituent, independently selected from R^{X}.

In another Embodiment (47), the invention provides a compound of any one of Embodiments (1)-(46) or a pharmaceutically acceptable salt thereof, wherein R¹ is selected from 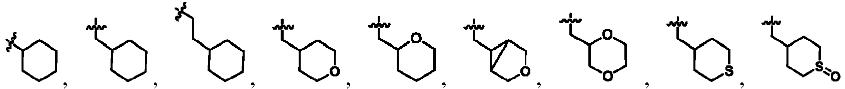 and 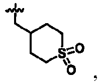 which are unsubstituted or substituted with at least one substituent, independently selected from R^{X}.

In another Embodiment (48), the invention provides a compound of any one of Embodiments (1)-(47) or a pharmaceutically acceptable salt thereof, wherein each substituent R^{X} of R¹ is independently selected from C₁₋₁₀ alkyl, C₃₋₁₀ cycloalkyl, halogen, CN, NO₂, -(CR^{c1}R^{d1})ₜNR^{a1}R^{b1}, -(CR^{c1}R^{d1})ₜOR^{b1}, -(CR^{c1}R^{d1})ₜC(O)R^{a1} and -(CR^{c1}R^{d1})N=S(O)R^{a1}R^{b1}, wherein alkyl, cycloalkyl are each unsubstituted or substituted with at least one substituent, independently selected from R^{Y}.

In another Embodiment (49), the invention provides a compound of any one of Embodiment (1)-(48) or a pharmaceutically acceptable salt thereof, wherein each R^{a1} and each R^{b1} are independently selected from hydrogen, C₁₋₁₀ alkyl, C₂₋₁₀ alkenyl and C₂₋₁₀ alkynyl, wherein alkyl, alkenyl and alkynyl are each unsubstituted or substituted with at least one substituent, independently selected from R^{Y};
or R^{a1} and R^{b1} together with the atom(s) to which they are attached form a heterocyclic ring of 4 to 8 members containing 0, 1 or 2 additional heteroatoms independently selected from oxygen, sulfur, nitrogen and phosphorus, and optionally substituted with 1, 2 or 3 R^{Y} groups.

In another Embodiment (50), the invention provides a compound of any one of Embodiments (1)-(49) or a pharmaceutically acceptable salt thereof, wherein each substituent R^{X} of R¹ is independently selected from OH, CN, halogen, C₁₋₁₀ alkyl,

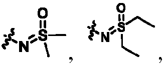

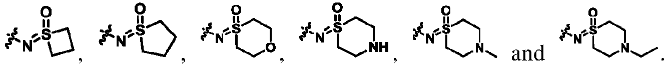

In another Embodiment (51), the invention provides a compound of any one of Embodiments (1)-(50) or a pharmaceutically acceptable salt thereof, wherein R¹ is selected from

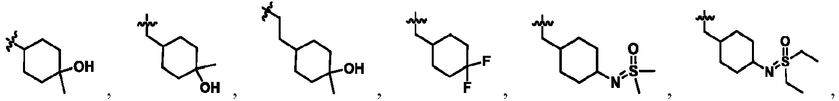

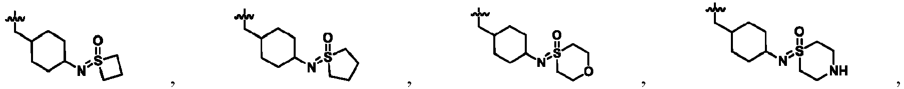

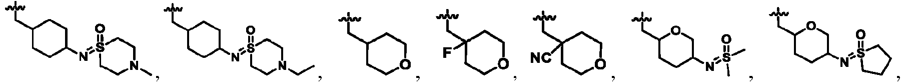

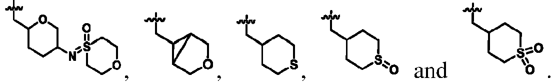

In another Embodiment (52), the invention provides a compound of any one of Embodiments (1)-(51) or a pharmaceutically acceptable salt thereof, wherein R⁸ is selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A8}R^{B8}, -OR^{A8}, -C(O)R^{A8}, -C(O)OR^{A8}, -OC(O)R^{A8}, -C(O)NR^{A8}R^{B8}, -NR^{A8}C(O)R^{B8}, -OC(O)NR^{A8}R^{B8}, -NR^{A8}C(O)OR^{B8}, -NR^{A8}C(O)NR^{A8}R^{B8}, -NR^{A8}C(S)NR^{A8}R^{B8}, -S(O)ᵣR^{A8}, -S(O)₂OR^{A8}, -OS(O)₂R^{A8}, -NR^{A8}S(O)ᵣR^{B8} and -S(O)ᵣNR^{A8}R^{B8}, wherein alkyl, alkenyl, alkynyl, cycloalkyl, heterocyclyl, aryl and heteroaryl are each unsubstituted or substituted with at least one substituent, independently selected from R^{X}.

In another Embodiment (53), the invention provides a compound of any one of Embodiments (1)-(52) or a pharmaceutically acceptable salt thereof, wherein R⁸ is selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, CN, NO₂, -NR^{A8}R^{B8}, -OR^{A8}, -C(O)R^{A8}, -C(O)OR^{A8}, -OC(O)R^{A8} and -S(O)ᵣR^{A8}, wherein alkyl, alkenyl, alkynyl, cycloalkyl and heterocyclyl are each unsubstituted or substituted with at least one substituent, independently selected from R^{X}, preferably, R⁸ is hydrogen.

In another Embodiment (54), the invention provides a compound of any one of Embodiments (1)-(53) or a pharmaceutically acceptable salt thereof, wherein R⁹ is selected from hydrogen, halogen, C₁₋₁₀ alkyl, C₂₋₁₀ alkenyl, C₃₋₁₀ cycloalkyl, C₃₋₁₀ cycloalkyl-C₁₋₄ alkyl, CN, NO₂, -NR^{A9}R^{B9}, -OR^{A9} and -C(O)R^{A9}, wherein alkyl, alkenyl and cycloalkyl are each unsubstituted or substituted with at least one substituent, independently selected from R^{X}.

In another Embodiment (55), the invention provides a compound of any one of Embodiment (1)-(54) or a pharmaceutically acceptable salt thereof, wherein R⁹ is selected from hydrogen, halogen, CN, OH, methyl, ethyl and trifluoromethyl.

In another Embodiment (56), the invention provides a compound of any one of Embodiments (1)-(55) or a pharmaceutically acceptable salt thereof, wherein R¹⁰ is selected from hydrogen, halogen, C₁₋₁₀ alkyl, C₂₋₁₀ alkenyl, C₃₋₁₀ cycloalkyl, C₃₋₁₀ cycloalkyl-C₁₋₄ alkyl, CN, NO₂, -NH₂ and -OH, wherein alkyl, alkenyl and cycloalkyl are each unsubstituted or substituted with at least one substituent, independently selected from R^{X}, preferably, R¹⁰ is selected from hydrogen, halogen, C₁₋₁₀ alkyl. CN, NO₂, -NH₂ and -OH, wherein alkyl is unsubstituted or substituted with at least one substituent, independently selected from R^{X}.

In another Embodiment (57), the invention provides a compound of any one of Embodiments (1)-(56) or a pharmaceutically acceptable salt thereof, wherein R¹¹ is selected from hydrogen, C₁₋₁₀ alkyl and C₃₋₁₀ cycloalkyl, wherein alkyl and cycloalkyl are each unsubstituted or substituted with at least one substituent, independently selected from R^{X}, preferably, R¹¹ is hydrogen.

In another Embodiment (58), the invention provides a compound of any one of Embodiments (1)-(57) or a pharmaceutically acceptable salt thereof, wherein R¹² is selected from hydrogen, C₁₋₁₀ alkyl and C₃₋₁₀ cycloalkyl, heterocyclyl, heterocyclyl-C₁₋₄ alkyl, aryl, aryl-C₁₋₄ alkyl, heteroaryl, -C(O)R^{A12}, -C(O)NR^{A12}R^{B12}, -C(O)OR^{A12}, -S(O)ᵣR^{A12} and -S(O)ᵣNR^{A12}R^{B12}, wherein alkyl, cycloalkyl heterocyclyl, aryl and heteroaryl are each unsubstituted or substituted with at least one substituent, independently selected from R^{X}. In another embodiment, R¹² is selected from hydrogen, C₁₋₁₀ alkyl and C₃₋₁₀ cycloalkyl, -C(O)R^{A12}, -C(O)OR^{A12}, -S(O)ᵣR^{A12}, wherein alkyl and cycloalkyl are each unsubstituted or substituted with at least one substituent, independently selected from R^{X}.

In another Embodiment (59), the invention provides a compound of any one of Embodiments (1)-(58) or a pharmaceutically acceptable salt thereof, wherein R¹² is selected from hydrogen, methyl, ethyl, cyclopropyl, -C(O)CH₃, -C(O)OCH₃, -S(O)₂CH₃, 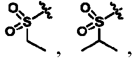 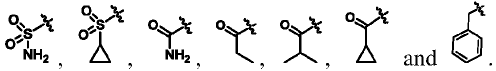 In another embodiment, R¹² is selected from hydrogen, methyl, ethyl, cyclopropyl, -C(O)CH₃, -C(O)OCH₃ and -S(O)₂CH₃.

In another Embodiment (60), the invention provides a compound of any one of Embodiments (1)-(59) or a pharmaceutically acceptable salt thereof, wherein "R⁴ and R¹¹" or "R⁴ and R¹²" together with the atoms to which they are attached form a C₃₋₁₀ cycloalkyl or heterocyclic ring of 4 to 12 members containing 1, 2 or 3 heteroatoms independently selected from oxygen, sulfur, nitrogen and phosphorus, and optionally substituted with 1, 2 or 3 R^{X} groups.

In another Embodiment (61), the invention provides a compound of any one of Embodiments (1)-(60) or a pharmaceutically acceptable salt thereof, wherein "R⁴ and R¹¹" or "R⁴ and R¹²" together with the atoms to which they are attached form a C₃₋₈ cycloalkyl or heterocyclic ring of 4 to 10 members containing 1, 2 or 3 heteroatoms independently selected from oxygen, sulfur, nitrogen and phosphorus, and optionally substituted with 1, 2 or 3 R^{X} groups.

In another Embodiment (62), the invention provides a compound of any one of Embodiments (1)-(61) or a pharmaceutically acceptable salt thereof, wherein m1 and m2 are independently selected from 0 and 1.

In another Embodiment (63), the invention provides a compound of any one of Embodiments (1)-(62) or a pharmaceutically acceptable salt thereof, wherein R³ is selected from aryl and heteroaryl, wherein aryl and heteroaryl are each unsubstituted or substituted with at least one substituent, independently selected from R^{X}.

In another Embodiment (64), the invention provides a compound of any one of Embodiments (1)-(63) or a pharmaceutically acceptable salt thereof, wherein R³ is selected from phenyl and pyridinyl, wherein phenyl and pyridinyl is unsubstituted or substituted with at least one substituent, independently selected from R^{X}. In an embodiment, the pyridinyl is pyrid-3-yl or pyrid-4-yl.

In another Embodiment (65), the invention provides a compound of any one of Embodiments (1)-(64) or a pharmaceutically acceptable salt thereof, each substituent R^{X} of R³ is selected from halogen, C₁₋₁₀ alkyl, C₂₋₁₀ alkenyl, C₃₋₁₀ cycloalkyl, wherein alkyl, alkenyl and cycloalkyl are each unsubstituted or substituted with at least one substituent, independently selected from R^{Y}.

In another Embodiment (66), the invention provides a compound of any one of Embodiments (1)-(65) or a pharmaceutically acceptable salt thereof, each substituent R^{X} of R³ is selected from halogen, methyl, ethyl, isopropyl, tert-butyl, propenyl and cyclopropyl, wherein methyl, ethyl, isopropyl, propenyl and cyclopropyl are each unsubstituted or substituted with at least one substituent, independently selected from halogen, C₁₋₁₀ alkyl, CN, NO₂, -NH₂ and -OH. In an embodiment, the propenyl is 1-propen-2-yl. In another embodiment, each substituent R^{X} of R³ is selected from halogen, methyl, ethyl, isopropyl, propenyl and cyclopropyl, wherein methyl, ethyl, isopropyl, propenyl and cyclopropyl are each unsubstituted or substituted with at least one substituent, independently selected from halogen.

In another Embodiment (67), the invention provides a compound of any one of Embodiments (1)-(66) or a pharmaceutically acceptable salt thereof, wherein R³ is selected from 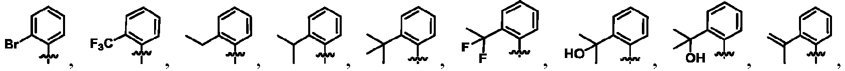 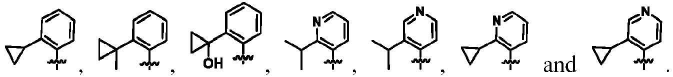 In another embodiment, R³ is selected from 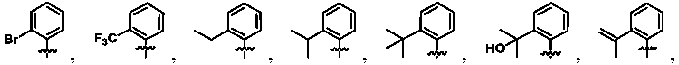 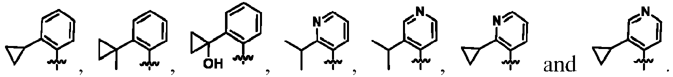 In another embodiment, R³ is selected from

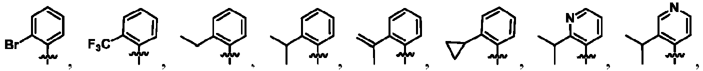

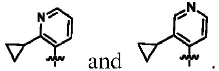

In another Embodiment (68), the invention provides a compound of any one of Embodiments (1)-(67) or a pharmaceutically acceptable salt thereof, wherein m is selected from 0, 1 and 2.

In another Embodiment (69), the invention provides a compound of any one of Embodiments (1)-(68) or a pharmaceutically acceptable salt thereof, wherein each R⁴ is independently selected from hydrogen, halogen, C₁₋₁₀ alkyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A4}R^{B4}, -OR^{A4}, -C(O)R^{A4}, -C(O)OR^{A4}, -OC(O)R^{A4}, -C(O)NR^{A4}R^{B4} and -S(O)ᵣR^{A4}, wherein alkyl, cycloalkyl, heterocyclyl, aryl and heteroaryl are each unsubstituted or substituted with at least one substituent, independently selected from R^{X};
any two of R⁴ together with the atoms to which they are attached form a C₃₋₈ cycloalkyl or heterocyclic ring of 4 to 8 members containing 1, 2 or 3 heteroatoms independently selected from oxygen, sulfur, nitrogen and phosphorus, and optionally substituted with 1, 2 or 3 R^{X} groups.

In another Embodiment (70), the invention provides a compound of any one of Embodiments (1)-(69) or a pharmaceutically acceptable salt thereof, wherein each R⁴ is independently selected from hydrogen, halogen, -OH, methyl, ethyl, isopropyl and cyclopropyl, wherein methyl, ethyl, isopropyl and cyclopropyl are each unsubstituted or substituted with at least one substituent, independently selected from R^{X};
or any two of R⁴ together with the atoms to which they are attached form cyclopropyl, wherein cyclopropyl is unsubstituted or substituted with 1, 2 or 3 R^{X} groups.

In another embodiment, each R⁴ is independently selected from hydrogen, halogen, methyl, ethyl, isopropyl and cyclopropyl, wherein methyl, ethyl, isopropyl and cyclopropyl are each unsubstituted or substituted with at least one substituent, independently selected from R^{X};
or any two of R⁴ together with the atoms to which they are attached form cyclopropyl, wherein cyclopropyl is unsubstituted or substituted with 1, 2 or 3 R^{X} groups.

In another Embodiment (71), the invention provides a compound of any one of Embodiments (1)-(70) or a pharmaceutically acceptable salt thereof, wherein the moiety 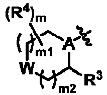 in Formula (I) and Formula (III) is selected from 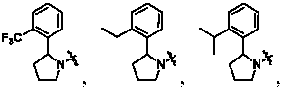 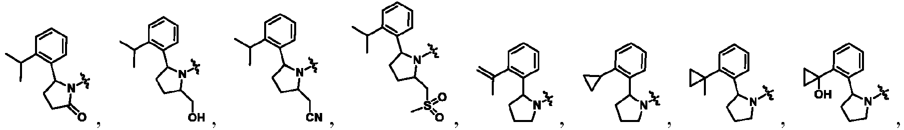 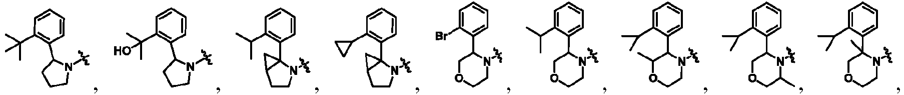 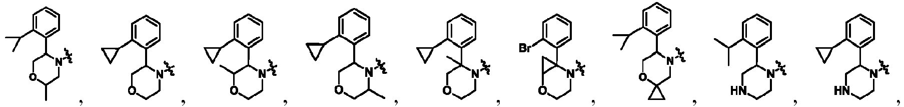 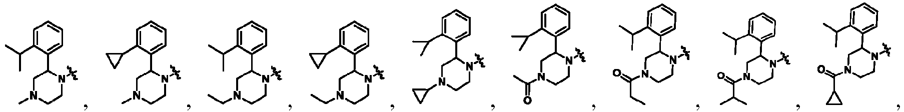 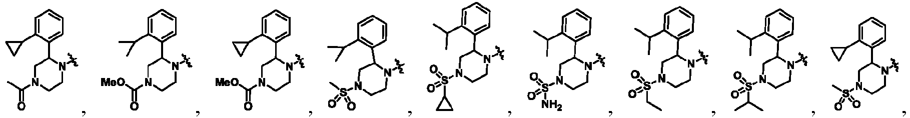 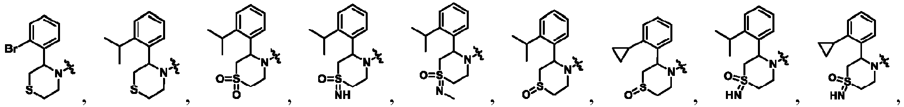 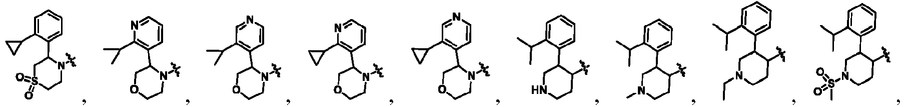 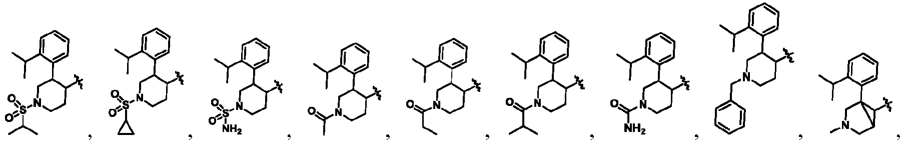 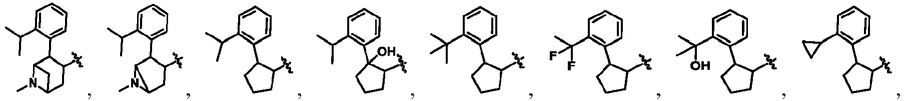 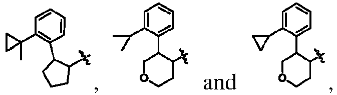 wherein the 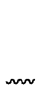 symbol indicates the point of attachment to the rest of the molecule. In an embodiment, the moiety 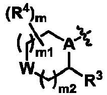 in Formula (I) and Formula (III) is selected from 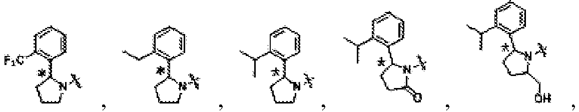 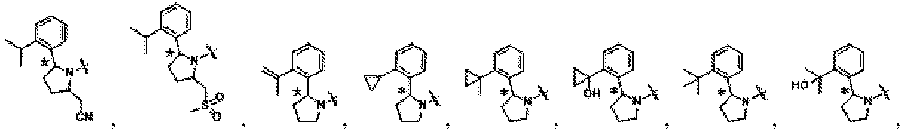 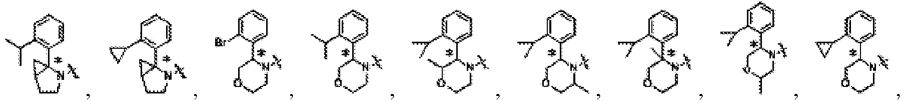 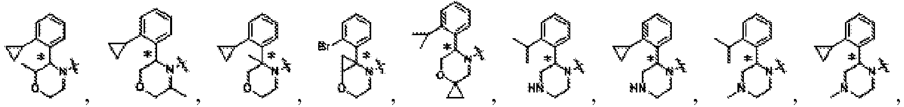 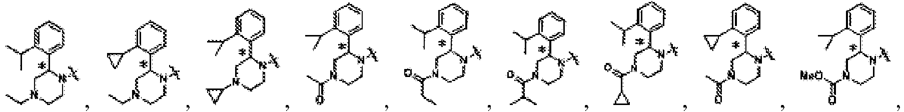 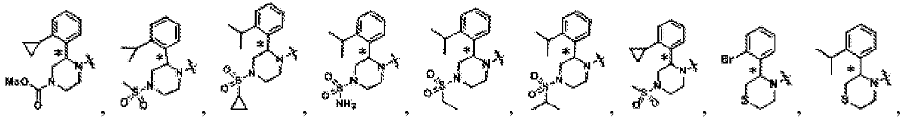 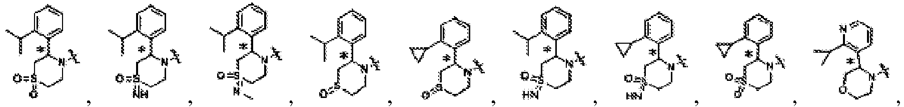 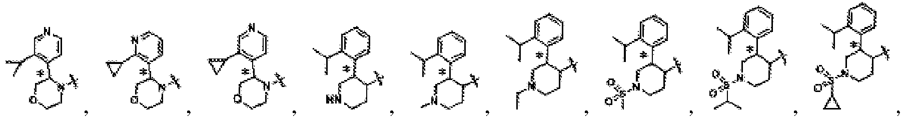 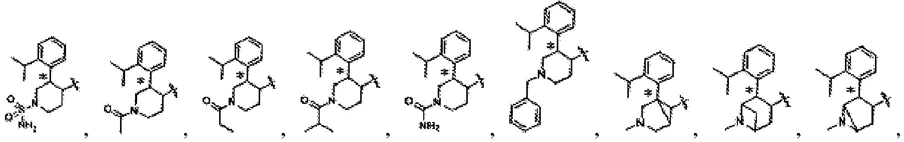 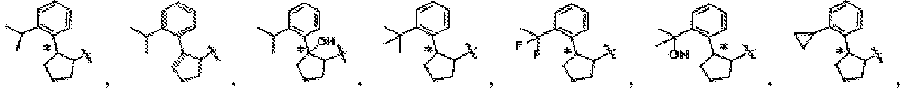 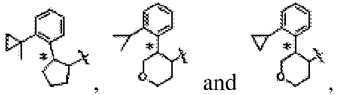 wherein the 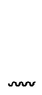 symbol indicates the point of attachment to the rest of the molecule, wherein the carbon marked with * is asymmetric center. In an embodiment, the asymmetric center is S configured carbon atom, enriched S configured carbon atom, R configured carbon atom, enriched R configured carbon atom or racemic carbon atom. In an embodiment, the asymmetric center is R configured carbon atom, enriched R configured carbon atom or racemic carbon atom, especially R configured carbon atom. In an embodiment, the asymmetric center is S configured carbon atom, enriched S configured carbon atom or racemic carbon atom, especially S configured carbon atom. In another embodiment, the moiety 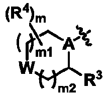 in Formula (I) is selected from 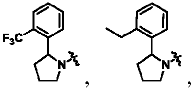 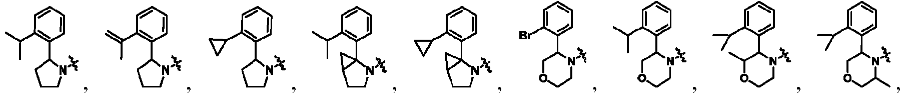 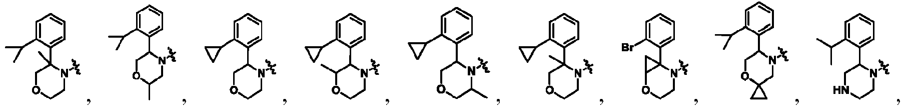 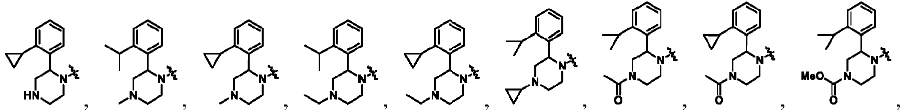 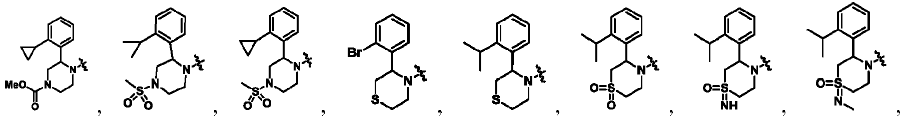 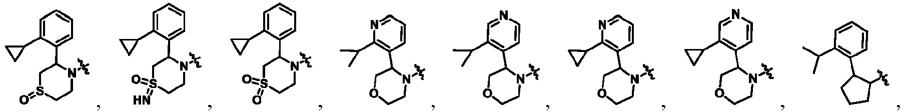 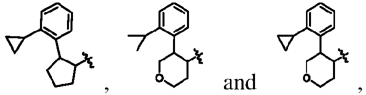 wherein the 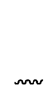 symbol indicates the point of attachment to the rest of the molecule.

In another Embodiment (72), the invention provides a compound of any one of Embodiments (1)-(71) or a pharmaceutically acceptable salt thereof, wherein the moiety 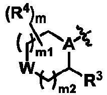 in Formula (I) is selected from 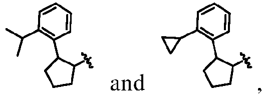 wherein the 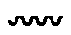 symbol indicates the point of attachment to the rest of the molecule.

In another Embodiment (73), the invention provides a compound of any one of Embodiments (1)-(72) or a pharmaceutically acceptable salt thereof, wherein each R⁵ is independently selected from hydrogen, halogen, C₁₋₁₀ alkyl, C₃₋₁₀ cycloalkyl, CN, NO₂, -NR^{A5}R^{B5}, -OR^{A5} and -C(O)R^{A5}, wherein alkyl and cycloalkyl are each unsubstituted or substituted with at least one substituent, independently selected from R^{X}; each R⁶ is independently selected from hydrogen, halogen, C₁₋₁₀ alkyl, C₃₋₁₀ cycloalkyl, CN, NO₂, -NR^{A5}R^{B5}, -OR^{A5} and -C(O)R^{A5}, wherein alkyl and cycloalkyl are each unsubstituted or substituted with at least one substituent, independently selected from R^{X}, preferably, each R⁵ and R⁶ are independently selected from hydrogen, halogen, C₁₋₁₀ alkyl, C₃₋₁₀ cycloalkyl, CN, NO₂, -NH₂ and -OH, wherein alkyl and cycloalkyl are each unsubstituted or substituted with at least one substituent, independently selected from R^{X}.

In another Embodiment (74), the invention provides a compound of any one of Embodiments (1)-(73) or a pharmaceutically acceptable salt thereof, wherein R⁵ and R⁶ are H.

In another Embodiment (75), the invention provides a compound of any one of Embodiments (1)-(74) or a pharmaceutically acceptable salt thereof, wherein the moiety 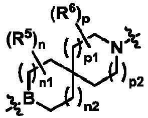 in Formula (I) and Formula (III) is selected from 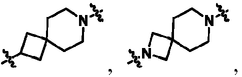 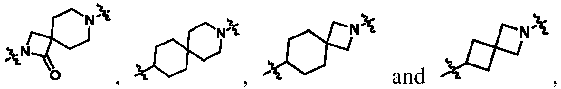 wherein the 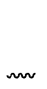 symbol indicates the point of attachment to the rest of the molecule. In another embodiment, the moiety 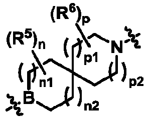 in Formula (I) and Formula (III) is selected from 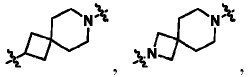 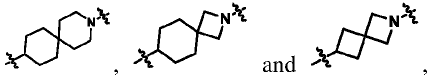 wherein the 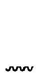 symbol indicates the point of attachment to the rest of the molecule.

In another Embodiment (76), the invention provides a compound of any one of Embodiments (1)-(75) or a pharmaceutically acceptable salt thereof, wherein each R⁷ is independently selected from hydrogen, halogen, C₁₋₁₀ alkyl, C₃₋₁₀ cycloalkyl, CN, NO₂, -NR^{A7}R^{B7}, -OR^{A7} and -C(O)R^{A7}, wherein alkyl and cycloalkyl are each unsubstituted or substituted with at least one substituent, independently selected from R^{X}, preferably, R⁷ is hydrogen.

In another embodiment, this invention provides to a compound of formula (I"), 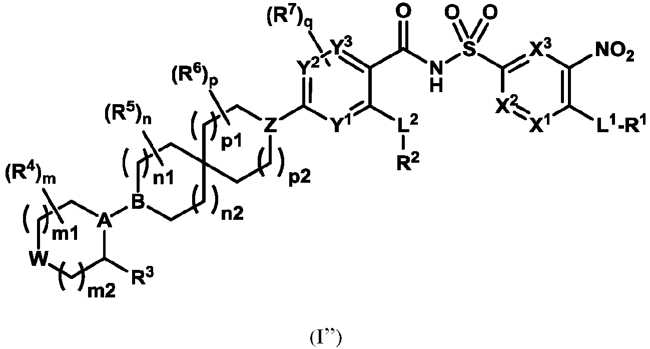 or a pharmaceutically acceptable salt thereof, wherein:
X¹ is selected from N and CR⁸;
X² is selected from N and CR⁹;
X³ is selected from N and CR¹⁰;
Y¹, Y² and Y³ are independently selected from N and CH;
A and B are independently selected from N and CH;
W is selected from -CR¹¹-, -NR¹²-, -O-, -S(O)ᵣ- and -S(O)(=NR¹²)-;
Z is selected from N and CH;
L¹ and L² are independently selected from a bond, -(CR^{C0}R^{D0})ᵤ-, -(CR^{C0}R^{D0})ᵤO(CR^{C0}R^{D0})ₜ-, -(CR^{C0}R^{D0})ᵤNR^{A0}(CR^{C0}R^{D0})ₜ- and -(CR^{C0}R^{D0})ᵤS(O)ᵣ(CR^{C0}R^{D0})ₜ-;
R¹ is selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A1}R^{B1}, -OR^{A1} -C(O)R^{A1}, -C(=NR^{E1})R^{A1}, -C(=N-OR^{B1})R^{A1}, -C(O)OR^{A1}, -OC(O)R^{A1}, -C(O)NR^{A1}R^{B1}, -NR^{A1}C(O)R^{B1}, -C(=NR^{E1})NR^{A1}R^{B1}, -NR^{A1}C(=NR^{E1})R^{B1}, -OC(O)NR^{A1}R^{B1}, -NR^{A1}C(O)OR^{B1}, -NR^{A1}C(O)NR^{A1}R^{B1}, -NR^{A1}C(S)NR^{A1}R^{B1}, -NR^{A1}C(=NR^{E1})NR^{A1}R^{B1}, -S(O)ᵣR^{A1}, -S(O)(=NR^{E1})R^{B1}, -N=S(O)R^{A1}R^{B1}, -S(O)₂OR^{A1}, -OS(O)₂R^{A1}, -NR^{A1}S(O)ᵣR^{B1}, -NR^{A1}S(O)(=NR^{E1})R^{B1}, -S(O)ᵣNR^{A1}R^{B1}, -S(O)(=NR^{E1})NR^{A1}R^{B1}, -NR^{A1}S(O)₂NR^{A1}R^{B1}, -NR^{A1}S(O)(=NR^{E1})NR^{A1}R^{B1}, -P(O)R^{A1}R^{B1} and -P(O)(OR^{A1})(OR^{B1}), wherein alkyl, alkenyl, alkynyl, cycloalkyl, heterocyclyl, aryl and heteroaryl are each unsubstituted or substituted with at least one substituent, independently selected from R^{X};
R² and R³ are independently selected from aryl, heteroaryl and heterocyclyl, wherein aryl, heteroaryl and heterocyclyl are each unsubstituted or substituted with at least one substituent, independently selected from R^{X};
each R⁴ is independently selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A4}R^{B4}, -OR^{A4}, -C(O)R^{A4}, -C(=NR^{E4})R^{A4}, -C(=N-OR^{B4})R^{A4}, -C(O)OR^{A4}, -OC(O)R^{A4}, -C(O)NR^{A4}R^{B4}, -NR^{A4}C(O)R^{B4}, -C(=NR^{E4})NR^{A4}R^{B4}, -NR^{A4}C(=NR^{E4})R^{B4}, -OC(O)NR^{A4}R^{B4}, -NR^{A4}C(O)OR^{B4}, -NR^{A4}C(O)NR^{A4}R^{B4}, -NR^{A4}C(S)NR^{A4}R^{B4}, -NR^{A4}C(=NR^{E4})NR^{A4}R^{B4}, -S(O)ᵣR^{A4}, -S(O)(=NR^{E4})R^{B4}, -N=S(O)R^{A4}R^{B4}, -S(O)₂OR^{A4}, -OS(O)₂R^{A4}, -NR^{A4}S(O)ᵣR^{B4}, -NR^{A4}S(O)(=NR^{E4})R^{B4}, -S(O)ᵣNR^{A4}R^{B4}, -S(O)(=NR^{E4})NR^{A4}R^{B4}, -NR^{A4}S(O)₂NR^{A4}R^{B4}, -NR^{A4}S(O)(=NR^{E4})NR^{A4}R^{B4}, -P(O)R^{A4}R^{B4} and -P(O)(OR^{A4})(OR^{B4}), wherein alkyl, alkenyl, alkynyl, cycloalkyl, heterocyclyl, aryl and heteroaryl are each unsubstituted or substituted with at least one substituent, independently selected from R^{X};
or any two of R⁴ or "R⁴ and R¹⁰" or "R⁴ and R¹¹" together with the atoms to which they are attached form a C₃₋₁₀ cycloalkyl or heterocyclic ring of 4 to 12 members containing 1, 2 or 3 heteroatoms independently selected from oxygen, sulfur, nitrogen and phosphorus, and optionally substituted with 1, 2 or 3 R^{X} groups;
each R⁵ is independently selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A5}R^{B5}, -OR^{A5}, -C(O)R^{A5}, -C(-NR^{E5})R^{A5}, -C(=N-OR^{B5})R^{A5}, -C(O)OR^{A5}, -OC(O)R^{A5}, -C(O)NR^{A5}R^{B5}, -NR^{A5}C(O)R^{B5}, -C(=NR^{E5})NR^{A5}R^{B5}, -NR^{A5}C(=NR^{E5})R^{B5}, -OC(O)NR^{A5}R^{B5}, -NR^{A5}C(O)OR^{B5}, -NR^{A5}C(O)NR^{A5}R^{B5}, -NR^{A5}C(S)NR^{A5}R^{B5}, -NR^{A5}C(=NR^{E5})NR^{A5}R^{B5}, -S(O)ᵣR^{A5}, -S(O)(=NR^{E5})R^{B5}, -N=S(O)R^{A5}R^{B5}, -S(O)₂OR^{A5}, -OS(O)₂R^{A5}, -NR^{A5}S(O)ᵣR^{B5}, -NR^{A5}S(O)(=NR^{E5})R^{B5}, -S(O)ᵣNR^{A5}R^{B5}, -S(O)(=NR^{E5})NR^{A5}R^{B5}, -NR^{A5}S(O)₂NR^{A5}R^{B5}, -NR^{A5}S(O)(=NR^{E5})NR^{A5}R^{B5}, -P(O)R^{A5}R^{B5} and -P(O)(OR^{A5})(OR^{B5}), wherein alkyl, alkenyl, alkynyl, cycloalkyl, heterocyclyl, aryl and heteroaryl are each unsubstituted or substituted with at least one substituent, independently selected from R^{X};
or any two of R⁵ together with the carbon atoms to which they are attached form a C₃₋₁₀ cycloalkyl or heterocyclic ring of 4 to 12 members containing 1, 2 or 3 heteroatoms independently selected from oxygen, sulfur, nitrogen and phosphorus, and optionally substituted with 1, 2 or 3 R^{X} groups;
each R⁶ is independently selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A6}R^{B6}, -OR^{A6}, -C(O)R^{A6}, -C(-NR^{E6})R^{A6}, -C(=N-OR^{B6})R^{A6}, -C(O)OR^{A6}, -OC(O)R^{A6}, -C(O)NR^{A6}R^{B6}, -NR^{A6}C(O)R^{B6}, -C(=NR^{E6})NR^{A6}R^{B6}, -NR^{A6}C(=NR^{E6})R^{B6}, -OC(O)NR^{A6}R^{B6}, -NR^{A6}C(O)OR^{B6}, -NR^{A6}C(O)NR^{A6}R^{B6}, -NR^{A6}C(S)NR^{A6}R^{B6}, -NR^{A6}C(=NR^{E6})NR^{A6}R^{B6}, -S(O)ᵣR^{A6}, -S(O)(=NR^{E6})R^{B6}, -N=S(O)R^{A6}R^{B6}, -S(O)₂OR^{A6}, -OS(O)₂R^{A6}, -NR^{A6}S(O)ᵣR^{B6}, -NR^{A6}S(O)(=NR^{E6})R^{B6}, -S(O)ᵣNR^{A6}R^{B6}, -S(O)(=NR^{E6})NR^{A6}R^{B6}, -NR^{A6}S(O)₂NR^{A6}R^{B6}, -NR^{A6}S(O)(=NR^{E6})NR^{A6}R^{A6}, -P(O)R^{A6}R^{B6} and -P(O)(OR^{A6})(OR^{B6}), wherein alkyl, alkenyl, alkynyl, cycloalkyl, heterocyclyl, aryl and heteroaryl are each unsubstituted or substituted with at least one substituent, independently selected from R^{X};
or any two of R⁶ together with the carbon atoms to which they are attached form a C₃₋₁₀ cycloalkyl or heterocyclic ring of 4 to 12 members containing 1, 2 or 3 heteroatoms independently selected from oxygen, sulfur, nitrogen and phosphorus, and optionally substituted with 1, 2 or 3 R^{X} groups;
each R⁷ is independently selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A7}R^{B7}, -OR^{A7}, -C(O)R^{A7}, -C(=NR^{E7})R^{A7}, -C(=N-OR^{B7})R^{A7}, -C(O)OR^{A7}, -OC(O)R^{A7}, -C(O)NR^{A7}R^{B7}, -NR^{A7}C(O)R^{B7}, -C(=NR^{E7})NR^{A7}R^{B7}, -NR^{A7}C(=NR^{E7})R^{B7}, -OC(O)NR^{A7}R^{B7}, -NR^{A7}C(O)OR^{B7}, -NR^{A7}C(O)NR^{A7}R^{B7}, -NR^{A7}C(S)NR^{A7}R^{B7}, -NR^{A7}C(=NR^{E7})NR^{A7}R^{B7}, -S(O)ᵣR^{A7}, -S(O)(=NR^{E7})R^{B7}, -N=S(O)R^{A7}R^{B7}, -S(O)₂OR^{A7}, -OS(O)₂R^{A7}, -NR^{A7}S(O)ᵣR^{B7}, -NR^{A7}S(O)(=NR^{E7})R^{B7}, -S(O)ᵣNR^{A7}R^{B7}, -S(O)(=NR^{E7})NR^{A7}R^{B7}, -NR^{A7}S(O)₂NR^{A7}R^{B7}, -NR^{A7}S(O)(=NR^{E7})NR^{A7}R^{B7}, -P(O)R^{A7}R^{B7} and -P(O)(OR^{A7})(OR^{B7}), wherein alkyl, alkenyl, alkynyl, cycloalkyl, heterocyclyl, aryl and heteroaryl are each unsubstituted or substituted with at least one substituent, independently selected from R^{X};
R⁸ is selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A8}R^{B8}, -OR^{A8}, -C(O)R^{A8}, -C(=NR^{E8})R^{A8}, -C(=N-OR^{B8})R^{A8}, -C(O)OR^{A8}, -OC(O)R^{A8}, -C(O)NR^{A8}R^{B8}, -NR^{A8}C(O)R^{B8}, -C(=NR^{E8})NR^{A8}R^{B8}, -NR^{A8}C(=NR^{E8})R^{B8}, -OC(O)NR^{A8}R^{B8}, -NR^{A8}C(O)OR^{B8}, -NR^{A8}C(O)NR^{A8}R^{B8}, -NR^{A8}C(S)NR^{A8}R^{B8}, -NR^{A8}C(=NR^{E8})NR^{A8}R^{B8}, -S(O)ᵣR^{A8}, -S(O)(=NR^{E8})R^{B8}, -N=S(O)R^{A8}R^{B8}, -S(O)₂OR^{A8}, -OS(O)₂R^{A8}, -NR^{A8}S(O)ᵣR^{B8}, -NR^{A8}S(O)(=NR^{E8})R^{B8}, -S(O)ᵣNR^{A8}R^{B8}, -S(O)(=NR^{E8})NR^{A8}R^{B8}, -NR^{A8}S(O)₂NR^{A8}R^{B8}, -NR^{A8}S(O)(=NR^{E8})NR^{A8}R^{B8}, -P(O)R^{A8}R^{B8} and -P(O)(OR^{A8})(OR^{B8}), wherein alkyl, alkenyl, alkynyl, cycloalkyl, heterocyclyl, aryl and heteroaryl are each unsubstituted or substituted with at least one substituent, independently selected from R^{X};
or R¹ and R⁸ together with the atoms to which they are attached form a C₅₋₁₀ cycloalkyl or heterocyclic ring of 5 to 12 members containing 1, 2 or 3 heteroatoms independently selected from oxygen, sulfur, nitrogen and phosphorus, and optionally substituted with 1, 2 or 3 R^{X} groups;
R⁹ is selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A9}R^{B9}, -OR^{A9}, -C(O)R^{A9}, -C(=NR^{E9})R^{A9}, -C(=N-OR^{B9})R^{A9}, -C(O)OR^{A9}, -OC(O)R^{A9}, -C(O)NR^{A9}R^{B9}, -NR^{A9}C(O)R^{B9}, -C(=NR^{E9})NR^{A9}R^{B9}, -NR^{A9}C(=NR^{E9})R^{B9}, -OC(O)NR^{A9}R^{B9}, -NR^{A9}C(O)OR^{B9}, -NR^{A9}C(O)NR^{A9}R^{B9}, -NR^{A9}C(S)NR^{A9}R^{B9}, -NR^{A9}C(=NR^{E9})NR^{A9}R^{B9}, -S(O)ᵣR^{A9}, -S(O)(=NR^{E9})R^{B9}, -N=S(O)R^{A9}R^{B9}, -S(O)₂OR^{A9}, -OS(O)₂R^{A9}, -NR^{A9}S(O)ᵣR^{B9}, -NR^{A9}S(O)(=NR^{E9})R^{B9}, -S(O)ᵣNR^{A9}R^{B9}, -S(O)(=NR^{E9})NR^{A9}R^{B9}, -NR^{A9}S(O)₂NR^{A9}R^{B9}, -NR^{A9}S(O)(=NR^{E9})NR^{A9}R^{B9}, -P(O)R^{A9}R^{B9} and -P(O)(OR^{A9})(OR^{B9}), wherein alkyl, alkenyl, alkynyl, cycloalkyl, heterocyclyl, aryl and heteroaryl are each unsubstituted or substituted with at least one substituent, independently selected from R^{X};
R¹⁰ is selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A10}R^{B10}, -OR^{A10}, -C(O)R^{A10}, -C(=NR^{E10})R^{A10}, -C(=N-OR^{B10})R^{A10}, -C(O)OR^{A10}, -OC(O)R^{A10}, -C(O)NR^{A10}R^{B10}, -NR^{A11}C(O)R^{B10}, -C(=NR^{E10})NR^{A10}R^{B10}, -NR^{A10}C(=NR^{E10})R^{B10}, -OC(O)NR^{A10}R^{B10}, -NR^{A10}C(O)OR^{B10}, -NR^{A10}C(O)NR^{A10}R^{B10}, -NR^{A10}C(S)NR^{A10}R^{B10}, -NR^{A10}C(=NR^{E10})NR^{A10}R^{B10}, -S(O)ᵣR^{A10}, -S(O)(=NR^{E10})R^{B10}, -N=S(O)R^{A10}R^{B10}, -S(O)₂OR^{A10}, -OS(O)₂R^{A10}, -NR^{A10}S(O)ᵣR^{B10}, -NR^{A10}S(O)(=NR^{E10})R^{B10}, -S(O)ᵣNR^{A10}R^{B10}, -S(O)(=NR^{E10})NR^{A10}R^{B10}, -NR^{A10}S(O)₂NR^{A10}R^{B10}, -NR^{A10}S(O)(=NR^{E10})NR^{A10}R^{B10}, -P(O)R^{A10}R^{B10} and -P(O)(OR^{A10})(OR^{B10}), wherein alkyl, alkenyl, alkynyl, cycloalkyl, heterocyclyl, aryl and heteroaryl are each unsubstituted or substituted with at least one substituent, independently selected from R^{X};
R¹¹ is selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A11}R^{B11}, -OR^{A11}, -C(O)R^{A11}, -C(=NR^{E11})R^{A11}, -C(=N-OR^{B11})R^{A11}, -C(O)OR^{A11}, -OC(O)R^{A11}, -C(O)NR^{A11}R^{B11}, -NR^{A11}C(O)R^{B11}, -C(=NR^{E11})NR^{A11}R^{B11}, -NR^{A11}C(=NR^{E11})R^{B11}, -OC(O)NR^{A11}R^{B11}, -NR^{A11}C(O)OR^{B11}, -NR^{A11}C(O)NR^{A11}R^{B11}, -NR^{A11}C(S)NR^{A11}R^{B11}, -NR^{A11}C(=NR^{E11})NR^{A11}R^{B11}, -S(O)ᵣR^{A11}, -S(O)(=NR^{E11})R^{B11}, -N=S(O)R^{A11}R^{B11}, -S(O)₂OR^{A11}, -OS(O)₂R^{A11}, -NR^{A11}S(O)ᵣR^{B11}, -NR^{A11}S(O)(=NR^{E11})R^{B11}, -S(O)ᵣNR^{A11}R^{B11}, -S(O)(=NR^{E11})NR^{A11}R^{B11}, -NR^{A11}S(O)₂NR^{A11}R^{B11}, -NR^{A11}S(O)(=NR^{E11})NR^{A11}R^{B11}, -P(O)R^{A11}R^{B11} and -P(O)(OR^{A11})(OR^{B11}), wherein alkyl, alkenyl, alkynyl, cycloalkyl, heterocyclyl, aryl and heteroaryl are each unsubstituted or substituted with at least one substituent, independently selected from R^{X};
R¹² is selected from hydrogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, -C(O)R^{A12}, -C(=NR^{E12})R^{A12}, -C(=N-OR^{B12})R^{A12}, -C(O)OR^{A12}, -C(O)NR^{A12}R^{B12}, -C(=NR^{E12})NR^{A12}R^{B12}, -S(O)ᵣR^{A12}, -S(O)(=NR^{E12})R^{B12}, -S(O)₂OR^{A12}, -S(O)ᵣNR^{A12}R^{B12}, -S(O)(=NR^{E12})NR^{A12}R^{B12}, -P(O)R^{A12}R^{B12} and -P(O)(OR^{A12})(OR^{B12}), wherein alkyl, alkenyl, alkynyl, cycloalkyl, heterocyclyl, aryl and heteroaryl are each unsubstituted or substituted with at least one substituent, independently selected from R^{X};
each R^{A0}, R^{A1}, R^{A4}, R^{A5}, R^{A6}, R^{A7}, R^{A8}, R^{A9}, R^{A10}, R^{A11}, R^{A12}, R^{B1}, R^{B4}, R^{B5}, R^{B6}, R^{B7}, R^{B8}, R^{B9}, R^{B10}, R^{B11} and R^{B12} are independently selected from hydrogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl and heteroaryl-C₁₋₄ alkyl, wherein alkyl, alkenyl, alkynyl, cycloalkyl, heterocyclyl, aryl and heteroaryl are each unsubstituted or substituted with at least one substituent, independently selected from R^{X};
or "R^{A1} and R^{B1}" or "R^{A4} and R^{B4}" or "R^{A5} and R^{B5}" or "R^{A6} and R^{B6}" or "R^{A7} and R^{B7}" or "R^{A8} and R^{B8}" or "R^{A9} and R^{B9}" or "R^{A10} and R^{B10}" or "R^{A11} and R^{B11}" or "R^{A12} and R^{B12}" together with the atom(s) to which they are attached form a heterocyclic ring of 4 to 12 members containing 0, 1 or 2 additional heteroatoms independently selected from oxygen, sulfur, nitrogen and phosphorus and optionally substituted with 1, 2 or 3 R^{X} groups;
each R^{C0} and R^{D0} are independently selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl and heteroaryl-C₁₋₄ alkyl, wherein alkyl, alkenyl, alkynyl, cycloalkyl, heterocyclyl, aryl and heteroaryl are each unsubstituted or substituted with at least one substituent, independently selected from R^{X};
or each "R^{C0} and R^{D0}" together with the carbon atom(s) to which they are attached form a 3- to 12-membered ring containing 0, 1 or 2 heteroatoms independently selected from oxygen, sulfur and nitrogen, and optionally substituted with 1, 2 or 3 R^{X} groups;
each R^{E1}, R^{E4}, R^{E5}, R^{E6}, R^{E7}, R^{E8}, R^{E9}, R^{E10}, R^{E11} and R^{E12} are independently selected from hydrogen, C₁₋₁₀ alkyl, CN, NO₂, -OR^{a1}, -SR^{a1}, -S(O)ᵣR^{a1}, _C(O)R^{a1}, -C(O)OR^{a1}, -C(O)NR^{a1}R^{b1} and -S(O)ᵣNR^{a1}R^{b1}, wherein alkyl is unsubstituted or substituted with at least one substituent, independently selected from R^{X};
each R^{X} is independently selected from hydrogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, halogen, CN, NO₂, -(CR^{c1}R^{d1})ₜNR^{a1}R^{b1}, -(CR^{c1}R^{d1})ₜOR^{b1}, -(CR^{c1}R^{d1})ₜC(O)R^{a1}, -(CR^{c1}R^{d1})ₜC(=NR^{e1})R^{a1}, -(CR^{c1}R^{d1})ₜC(=N-OR^{b1})R^{a1}, -(CR^{c1}R^{d1})ₜC(O)OR^{b1}, -(CR^{c1}R^{d1})ₜOC(O)R^{b1}, -(CR^{c1}R^{d1})ₜC(O)NR^{a1}R^{b1}, -(CR^{c1}R^{d1})ₜNR^{a1}C(O)R^{b1}, -(CR^{c1}R^{d1})ₜC(=NR^{e1})NR^{a1}R^{b1}, -(CR^{c1}R^{d1})ₜNR^{a1}C(=NR^{e1})R^{b1}, -(CR^{c1}R^{d1})ₜOC(O)NR^{a1}R^{b1}, -(CR^{c1}R^{d1})ₜNR^{a1}C(O)OR^{b1}, -(CR^{c1}R^{d1})ₜNR^{a1}C(O)NR^{a1}R^{b1}, -(CR^{c1}R^{d1})NR^{a1}C(S)NR^{a1}R^{b1}, -(CR^{c1}R^{d1})ₜNR^{a1}C(=NR^{e1})NR^{a1}R^{b1}, -(CR^{c1}R^{d1})ₜS(O)ᵣR^{b1}, -(CR^{c1}R^{d1})ₜS(O)(=NR^{e1})R^{b1}, -(CR^{c1}R^{d1})ₜN=S(O)R^{a1}R^{b1}, -(CR^{c1}R^{d1})ₜS(O)₂OR^{b1}, -(CR^{c1}R^{d1})ₜOS(O)₂R^{b1}, -(CR^{c1}R^{d1})ₜNR^{a1}S(O)ᵣR^{b1}, -(CR^{c1}R^{a1})ₜNR^{a1}S(O)(=NR^{e1})R^{b1}, -(CR^{c1}R^{d1})tS(O)ᵣNR^{a1}R^{b1}, -(CR^{c1}R^{d1})ₜS(O)(=NR^{e1})NR^{a1}R^{b1}, -(CR^{c1}R^{d1})ₜNR^{a1}S(O)₂NR^{a1}R^{b1}, -(CR^{c1}R^{d1})ₜNR^{a1}S(O)(=NR^{e1})NR^{a1}R^{b1}, -(CR^{c1}R^{d1})ₜP(O)R^{a1}R^{b1} and -(CR^{c1}R^{d1})ₜP(O)(OR^{a1})(OR^{b1}), wherein alkyl, alkenyl, alkynyl, cycloalkyl, heterocyclyl, aryl and heteroaryl are each unsubstituted or substituted with at least one substituent, independently selected from R^{Y};
wherein
when X¹ is CR⁸, X² is CR⁹, X³ is CR¹⁰, R¹ and R⁸ together with the atoms to which they are attached form a C₅₋₁₀ cycloalkyl or heterocyclic ring of 5 to 12 members containing 1, 2 or 3 heteroatoms independently selected from oxygen, sulfur, nitrogen and phosphorus, and optionally substituted with 1, 2 or 3 R^{X} groups and L¹ is selected from -(CR^{C0}R^{D0})ᵤNR^{A0}(CR^{C0}R^{D0})ₜ-, L² is selected from a bond, -(CR^{C0}R^{D0})ᵤO(CR^{C0}R^{D0})ₜ-, -(CR^{C0}R^{D0})ᵤS(O)ᵣ(CR^{C0}R^{D0})ₜ-, wherein when L² is -O- and R² is 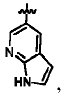 the substituent R^{X} of R² is selected from C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, halogen, CN, NO2, -(CR^{c1}R^{d1})ₜNR^{a1}R^{b1},-(CR^{c1}R^{d1})ₜOR^{b1}, -(CR^{c1}R^{a1})ₜC(O)R^{a1}, -(CR^{c1}R^{d1})ₜC(=NR^{e1})R^{a1}, -(CR^{c1}R^{d1})ₜC(=N-OR^{b1})R^{a1}, -(CR^{c1}R^{d1})ₜC(O)OR^{b1}, -(CR^{c1}R^{a1})ₜOC(O)R^{b1}, -(CR^{c1}R^{d1})ₜC(O)NR^{a1}R^{b1}, -(CR^{c1}R^{d1})NR^{a1}C(O)R^{b1}, -(CR^{c1}R^{d1})ₜC(=NR^{e1})NR^{a1}R^{b1}, -(CR^{c1}R^{d1})ₜNR^{a1}C(=NR^{e1})R^{b1}, -(CR^{c1}R^{d1})ₜOC(O)NR^{a1}R^{b1}, -(CR^{c1}R^{d1})ₜNR^{a1}C(O)OR^{b1}, -(CR^{c1}R^{d1})ₜNR^{a1}C(O)NR^{a1}R^{b1} -(CR^{c1}R^{d1})ₜNR^{a1}C(S)NR^{a1}R^{b1}, -(CR^{c1}R^{d1})ₜNR^{a1}C(=NR^{e1})NR^{a1}R^{b1}, -(CR^{c1}R^{d1})ₜS(O)ᵣR^{b1}, -(CR^{c1}R^{d1})ₜS(O)(=NR^{e1})R^{b1}, -(CR^{c1}R^{a1})ₜN=S(O)R^{a1}R^{b1}, -(CR^{c1}R^{a1})ₜS(O)₂OR^{b1}, -(CR^{c1}R^{d1})ₜOS(O)₂R^{b1}, -(CR^{c1}R^{d1})ₜNR^{a1}S(O)ᵣR^{b1}, -(CR^{c1}R^{d1})ₜNR^{a1}S(O)(=NR^{e1})R^{b1}, -(CR^{c1}R^{d1})ₜS(O)ᵣNR^{a1}R^{b1}, -(CR^{c1}R^{d1})ₜS(O)(=NR^{e1})NR^{a1}R^{b1}, -(CR^{c1}R^{a1})ₜNR^{a1}S(O)₂NR^{a1}R^{b1}, -(CR^{c1}R^{d1})ₜNR^{a1}S(O)(=NR^{e1})NR^{a1}R^{b1}, -(CR^{c1}R^{a1})ₜP(O)R^{a1}R^{b1} and -(CR^{c1}R^{d1})ₜP(O)(OR^{a1})(OR^{b1}), wherein alkyl, alkenyl, alkynyl, cycloalkyl, heterocyclyl, aryl and heteroaryl are each unsubstituted or substituted with at least one substituent, independently selected from R^{Y};
when X¹ is CR³, X² is CR⁹, X³ is CR¹⁰ and R¹ and R⁸ together with the atoms to which they are attached do not form a ring, A is CH. W is selected from -CR¹¹-, and L² is selected from -(CR^{C0}R^{D0})ᵤO(CR^{C0}R^{D0})ₜ- and -(CR^{C0}R^{D0})ᵤS(O)ᵣ(CR^{C0}R^{D0})ₜ-;
each R^{a1} and each R^{b1} are independently selected from hydrogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl and heteroaryl-C₁₋₄ alkyl, wherein alkyl, alkenyl, alkynyl, cycloalkyl, heterocyclyl, aryl and heteroaryl are each unsubstituted or substituted with at least one substituent, independently selected from R^{Y};
or R^{a1} and R^{b1} together with the atom(s) to which they are attached form a heterocyclic ring of 4 to 12 members containing 0, 1 or 2 additional heteroatoms independently selected from oxygen, sulfur, nitrogen and phosphorus, and optionally substituted with 1, 2 or 3 R^{Y} groups;
each R^{c1} and each R^{d1} are independently selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl and heteroaryl-C₁₋₄ alkyl, wherein alkyl, alkenyl, alkynyl, cycloalkyl, heterocyclyl, aryl and heteroaryl are each unsubstituted or substituted with at least one substituent, independently selected from R^{Y};
or R^{c1} and R^{d1} together with the carbon atom(s) to which they are attached form a ring of 3 to 12 members containing 0, 1 or 2 heteroatoms independently selected from oxygen, sulfur and nitrogen, and optionally substituted with 1, 2 or 3 R^{Y} groups;
each R^{e1} is independently selected from hydrogen, C₁₋₁₀ alkyl, C₃₋₁₀ cycloalkyl, C₃₋₁₀ cycloalkyl-C₁₋₄ alkyl, CN, NO2, -OR^{a2}, -SR^{a2}, -S(O)rR^{a2}, -C(O)R^{a2}, -C(O)OR^{a2}, -S(O)ᵣNR^{a2}R^{b2} and -C(O)NR^{a2}R^{b2};
each R^{Y} is independently selected from C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, halogen, CN, NO₂, -(CR^{c2}R^{d2})ₜNR^{a2}R^{b2}, -(CR^{c2}R^{d2})ₜOR^{b2}, -(CR^{c2}R^{d2})ₜC(O)R^{a2}, -(CR^{c2}R^{d2})ₜC(=NR^{e2})R^{a2}, -(CR^{c2}R^{d2})ₜC(=N-OR^{b2})R^{a2}, -(CR^{c2}R^{d2})ₜC(O)OR^{b2}, -(CR^{c2}R^{d2})ₜOC(O)R^{b2}, -(CR^{c2}R^{d2})ₜC(O)NR^{a2}R^{b2}, -(CR^{c2}R^{d2})NR^{a2}C(O)R^{b2}, -(CR^{c2}R^{d2})ₜC(=NR^{e2})NR^{a2}R^{b2}, -(CR^{c2}R^{d2})ₜNR^{a2}C(=NR^{e2})R^{b2}, -(CR^{c2}R^{d2})ₜOC(O)NR^{a2}R^{b2}, -(CR^{c2}R^{d2})ₜNR^{a2}C(O)OR^{b2}, -(CR^{c2}R^{d2})ₜNR^{a2}C(O)NR^{a2}R^{b2}, -(CR^{c2}R^{d2})ₜNR^{a2}C(S)NR^{a2}R^{b2}, -(CR^{c2}R^{d2})ₜNR^{a2}C(=NR^{e2})NR^{a2}R^{b2}, -(CR^{c2}R^{d2})ₜS(O)ᵣR^{b2}, -(CR^{c2}R^{d2})ₜS(O)(=NR^{e2})R^{b2}, -(CR^{c2}R^{d2})ₜN=S(O)R^{a2}R^{b2}, -(CR^{c2}R^{d2})ₜS(O)₂OR^{b2}, -(CR^{c2}R^{d2})ₜOS(O)2R^{b2}, -(CR^{c2}R^{d2})ₜNR^{a2}S(O)ᵣR^{b2}, -(CR^{c2}R^{d2})ₜNR^{a2}S(O)(=NR^{e2})R^{b2}, -(CR^{c2}R^{d2})ₜS(O)ᵣNR^{a2}R^{b2}, -(CR^{c2}R^{d2})ₜS(O)(=NR^{e2})NR^{a2}R^{b2}, -(CR^{c2}R^{d2})ₜNR^{a2}S(O)₂NR^{a2}R^{b2}, -(CR^{c2}R^{d2})ₜNR^{a2}S(O)(=NR^{e2})NR^{a2}R^{b2}, -(CR^{c2}R^{d2})ₜP(O)R^{a2}R^{b2} and -(CR^{c2}R^{d2})ₜP(O)(OR^{a2})(OR^{b2}), wherein alkyl, alkenyl, alkynyl, cycloalkyl, heterocyclyl, aryl and heteroaryl are each unsubstituted or substituted with at least one substituent, independently selected from OH, CN, amino, halogen, C₁-₁₀ alkyl, C₂₋₁₀ alkenyl, C₂₋₁₀ alkynyl, C₃₋₁₀ cycloalkyl, C₁₋₁₀ alkoxy, C₃₋₁₀ cycloalkoxy, C₁-₁₀ alkylthio, C₃₋₁₀ cycloalkylthio, C₁₋₁₀ alkylamino, C₃₋₁₀ cycloalkylamino and di(C₁₋₁₀ alkyl)amino;
each R^{a2} and each R^{b2} are independently selected from hydrogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, C₁₋₁₀ alkoxy, C₃₋₁₀ cycloalkoxy, C₁₋₁₀ alkylthio, C₃₋₁₀ cycloalkylthio, C₁₋₁₀ alkylamino, C₃₋₁₀ cycloalkylamino, di(C₁₋₁₀ alkyl)amino, heterocyclyl, heterocyclyl-C₁₋₄ alkyl, aryl, aryl-C₁₋₄ alkyl, heteroaryl and heteroaryl-C₁₋₄ alkyl, wherein alkyl, alkenyl, alkynyl, cycloalkyl, alkoxy, cycloalkoxy, alkylthio, cycloalkylthio, alkylamino, cycloalkylamino, heterocyclyl, aryl and heteroaryl are each unsubstituted or substituted with at least one substituent, independently selected from halogen, CN, C₁₋₁₀ alkyl, C₂₋₁₀ alkenyl, C₂₋₁₀ alkynyl, C₃₋₁₀ cycloalkyl, OH, C₁₋₁₀ alkoxy, C₃₋₁₀ cycloalkoxy, C₁₋₁₀ alkylthio, C₃₋₁₀ cycloalkylthio, amino, C₁₋₁₀ alkylamino, C₃₋₁₀ cycloalkylamino and di(C₁₋₁₀ alkyl)amino;
or R^{a2} and R^{b2} together with the atom(s) to which they are attached form a heterocyclic ring of 4 to 12 members containing 0, 1 or 2 additional heteroatoms independently selected from oxygen, sulfur, nitrogen and phosphorus, and optionally substituted with 1 or 2 substituents, independently selected from halogen, CN, C₁₋₁₀ alkyl, C₂₋₁₀ alkenyl, C₂₋₁₀ alkynyl, C₃₋₁₀ cycloalkyl, OH, C₁₋₁₀ alkoxy, C₃₋₁₀ cycloalkoxy, C₁₋₁₀ alkylthio, C₃₋₁₀ cycloalkylthio, amino, C₁₋₁₀ alkylamino, C₃₋₁₀ cycloalkylamino and di(C₁₋₁₀ alkyl)amino;
each R^{c2} and each R^{d2} are independently selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, C₁₋₁₀ alkoxy, C₃₋₁₀ cycloalkoxy, C₁₋₁₀ alkylthio, C₃₋₁₀ cycloalkylthio, C₁₋₁₀ alkylamino, C₃₋₁₀ cycloalkylamino, di(C₁₋₁₀ alkyl)amino, heterocyclyl, heterocyclyl-C₁₋₄ alkyl, aryl, aryl-C₁₋₄ alkyl, heteroaryl and heteroaryl-C₁₋₄ alkyl, wherein alkyl, alkenyl, alkynyl, cycloalkyl, alkoxy, cycloalkoxy, alkylthio, cycloalkylthio, alkylamino, cycloalkylamino, heterocyclyl, aryl and heteroaryl are each unsubstituted or substituted with at least one substituent, independently selected from halogen, CN, C₁₋₁₀ alkyl, C₂₋₁₀ alkenyl, C₂₋₁₀ alkynyl, C₃₋₁₀ cycloalkyl, OH, C₁₋₁₀ alkoxy, C₃₋₁₀ cycloalkoxy, C₁₋₁₀ alkylthio, C₃₋₁₀ cycloalkylthio, amino, C₁₋₁₀ alkylamino, C₃₋₁₀ cycloalkylamino and di(C₁₋₁₀ alkyl)amino;
or R^{c2} and R^{d2} together with the carbon atom(s) to which they are attached form a ring of 3 to 12 members containing 0, 1 or 2 heteroatoms independently selected from oxygen, sulfur and nitrogen, and optionally substituted with 1 or 2 substituents, independently selected from halogen, CN, C₁₋₁₀ alkyl, C₂₋₁₀ alkenyl, C₂₋₁₀ alkynyl, C₃₋₁₀ cycloalkyl, OH, C₁₋₁₀ alkoxy, C₃₋₁₀ cycloalkoxy, C₁₋₁₀ alkylthio, C₃₋₁₀ cycloalkylthio, amino, C₁₋₁₀ alkylamino, C₃₋₁₀ cycloalkylamino and di(C₁₋₁₀ alkyl)amino;
each R^{e2} is independently selected from hydrogen, CN, NO₂, C₁₋₁₀ alkyl, C₃₋₁₀ cycloalkyl, C₃₋₁₀ cycloalkyl-C₁₋₄ alkyl, C₁₋₁₀ alkoxy, C₃₋₁₀ cycloalkoxy, -C(O)C₁₋₄ alkyl, -C(O)C₃₋₁₀ cycloalkyl, -C(O)OC₁₋₄ alkyl, -C(O)OC₃₋₁₀ cycloalkyl, -C(O)N(C₁₋₄ alkyl)₂, -C(O)N(C₃₋₁₀ cycloalkyl)₂, -S(O)₂C₁₋₄ alkyl, -S(O)₂C₃₋₁₀ cycloalkyl, -S(O)₂N(C₁₋₄ alkyl)₂ and -S(O)₂N(C₃₋₁₀ cycloalkyl)₂;
m, m1, m2, n, n1, n2, p, p1, p2 and q are independently selected from 0, 1, 2 and 3;
each r is independently selected from 0, 1 and 2;
each t is independently selected from 0, 1, 2, 3 and 4;
each u is independently selected from 0, 1, 2, 3 and 4.

In another embodiment, the invention provides a compound of formula (I'') or a pharmaceutically acceptable salt thereof,
wherein X³ is CH, A is N, B is CH, Z is N, Y¹, Y² and Y³ are CH, shown as formula (II"), 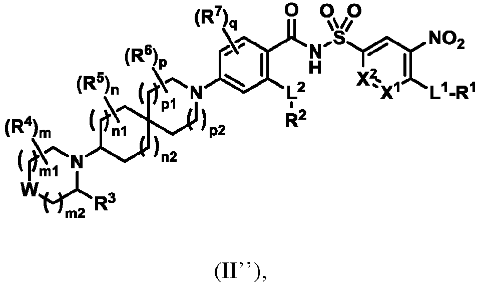
wherein
X¹ is selected from N and CR⁸, X² is selected from N and CR⁹, wherein at least one of X¹ and X² is N; W is selected from -CR¹⁰-, -NR¹¹-, -O- and -S(O)ᵣ-; L¹, L², W, R¹, R², R³, R⁴, R⁵, R⁶, R⁷, m, ml, m2, n, nl, n2, p, p1, p2 and q are as defined in formula (I").

In another Embodiment (77), the invention provides a compound selected from 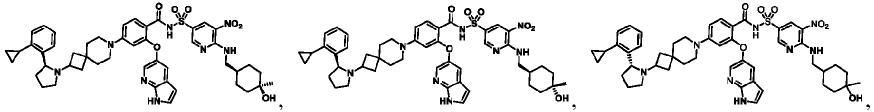 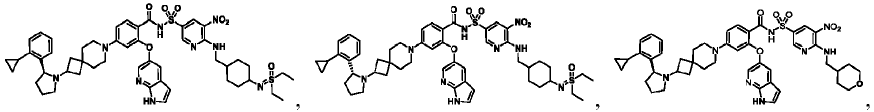 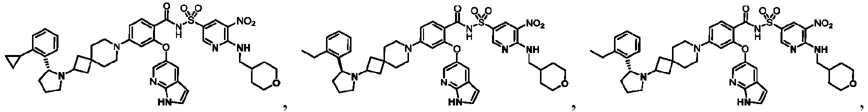 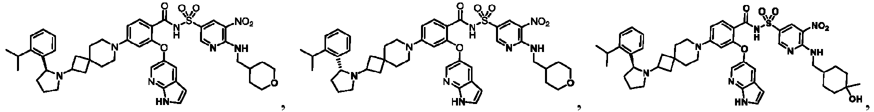 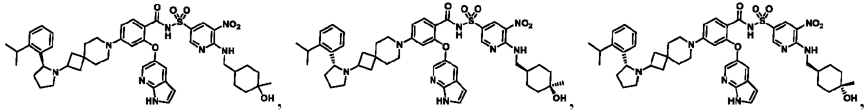 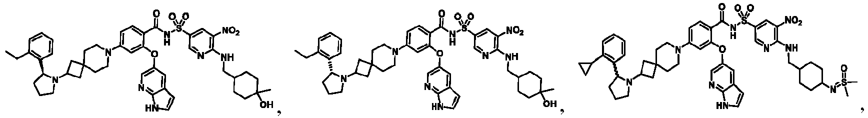 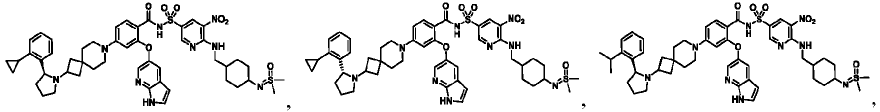 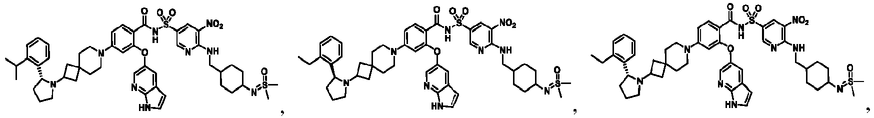 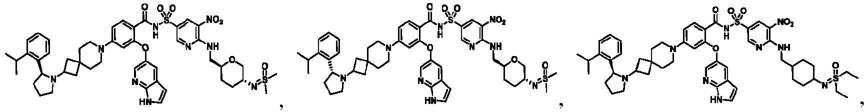 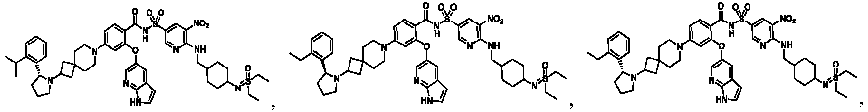 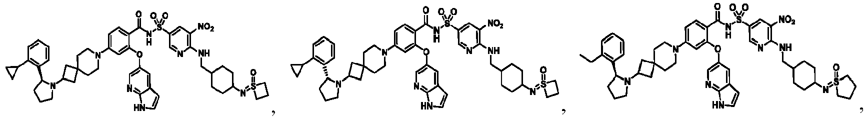 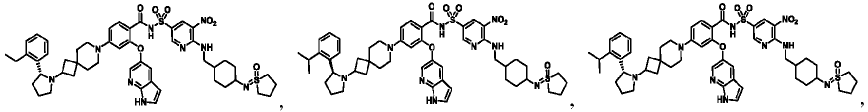 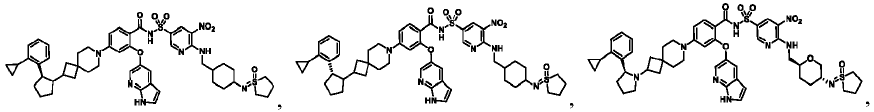 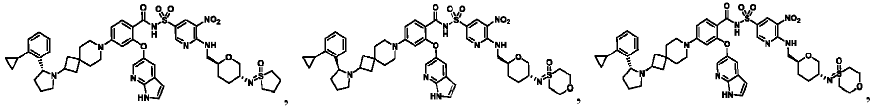 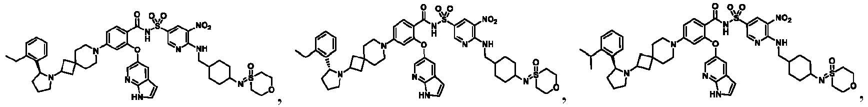 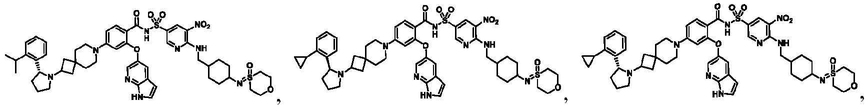 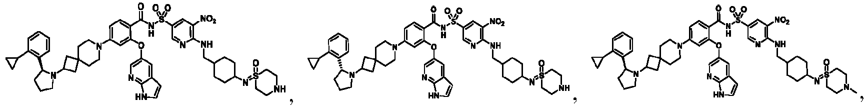 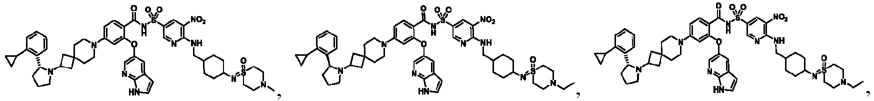 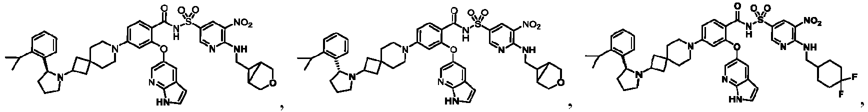 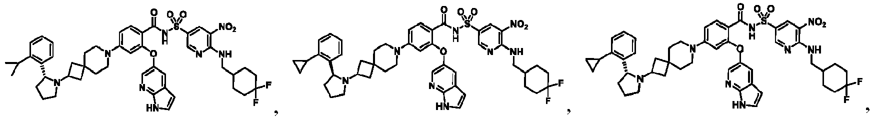 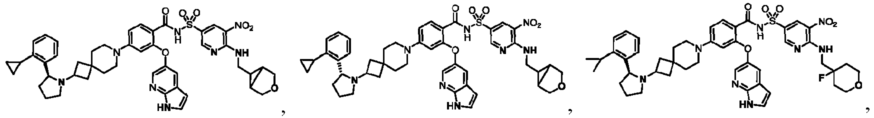 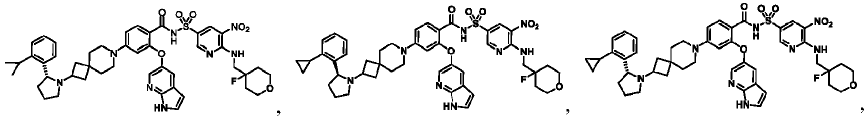 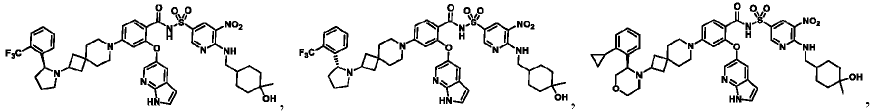 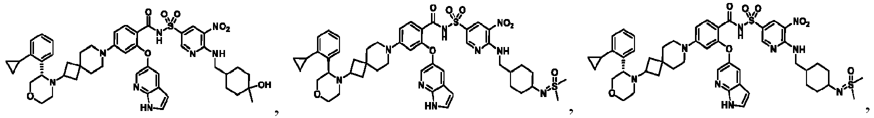 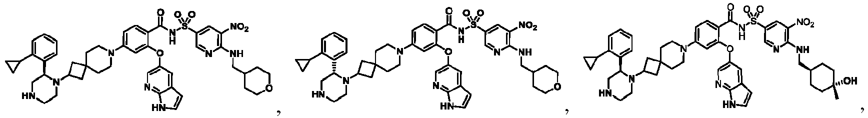 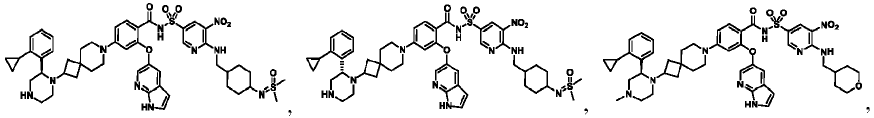 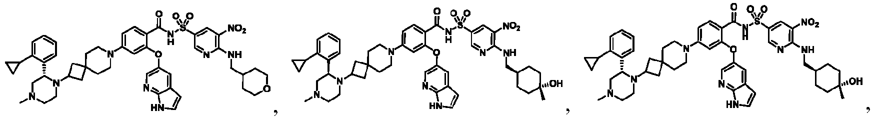 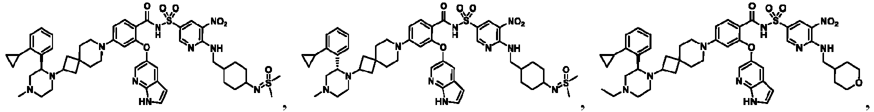 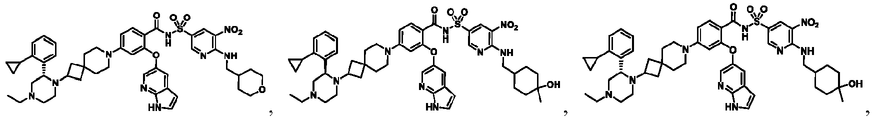 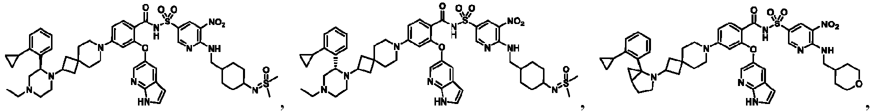 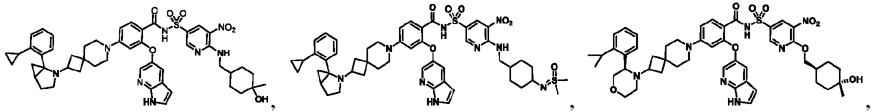 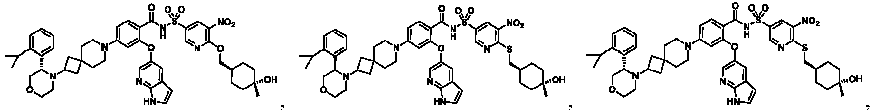 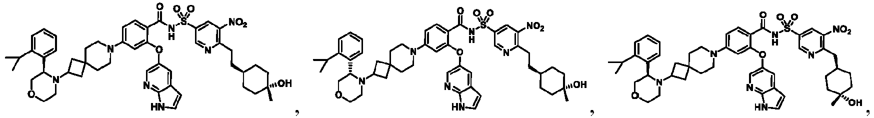 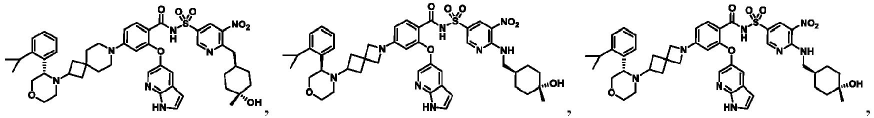 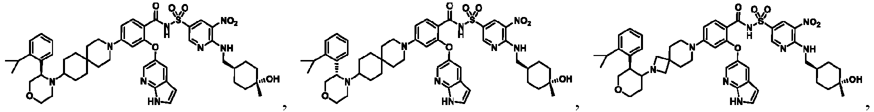 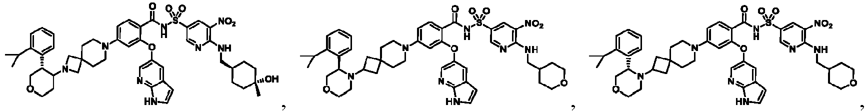 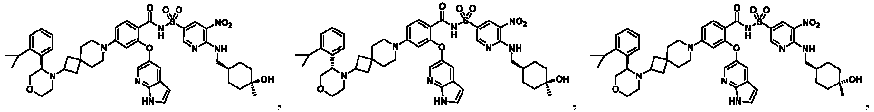 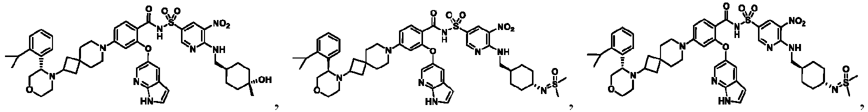 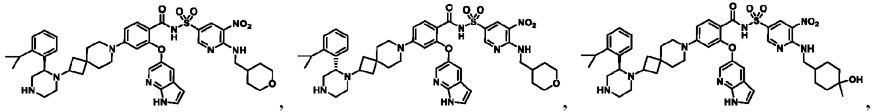 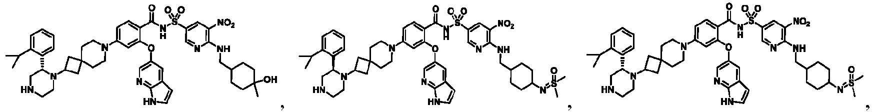 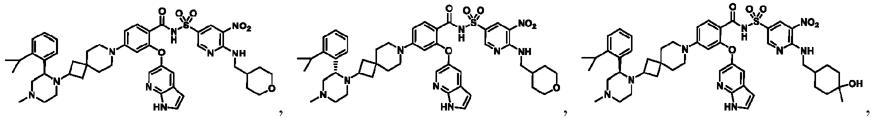 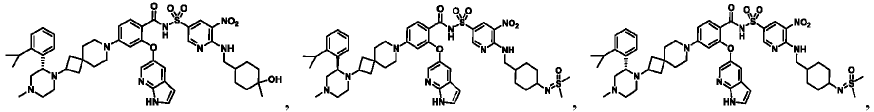 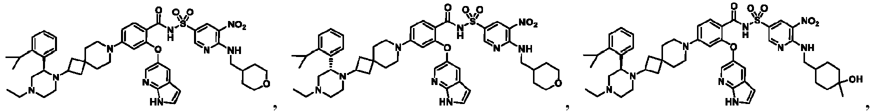 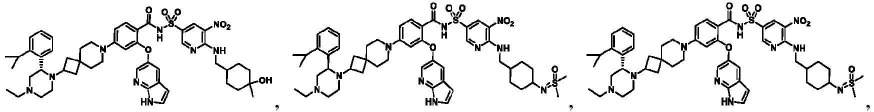 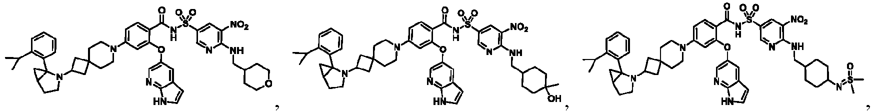 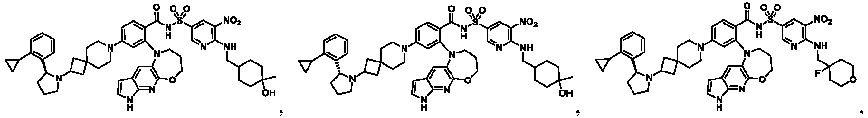 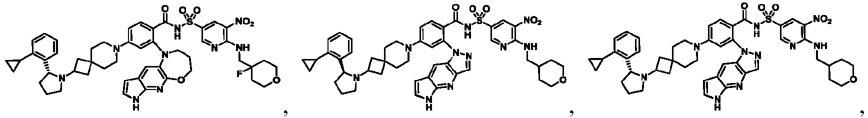 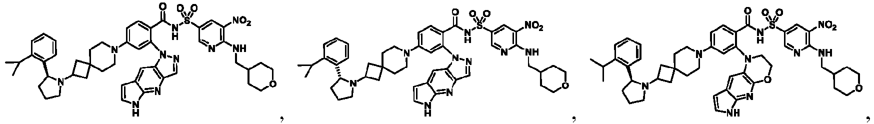 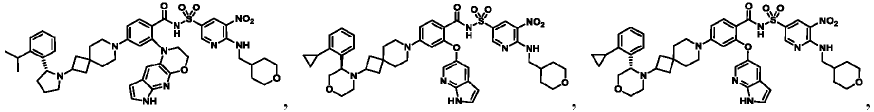 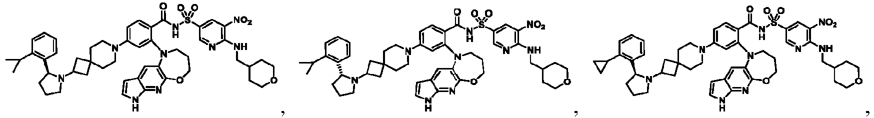 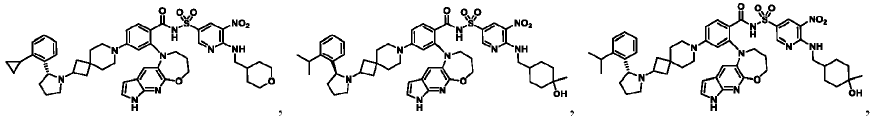 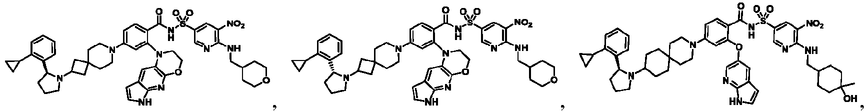 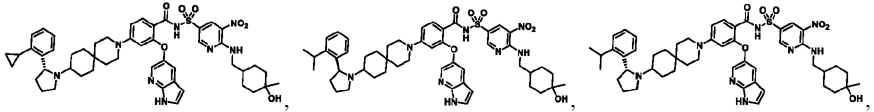 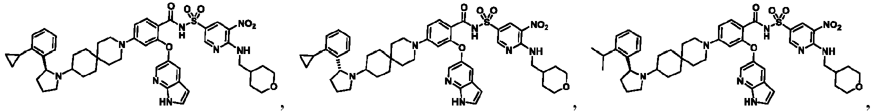 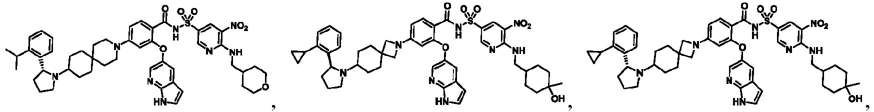 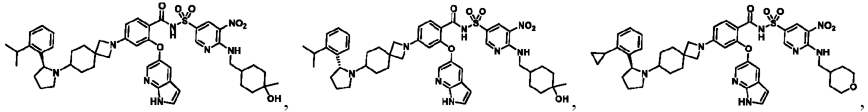 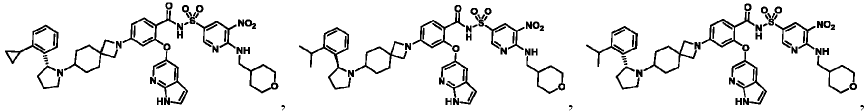 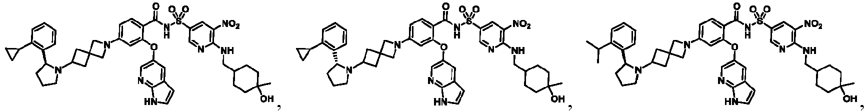 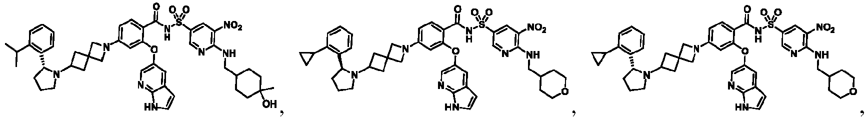 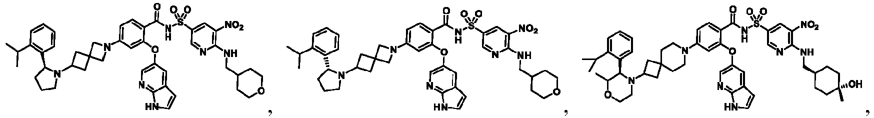 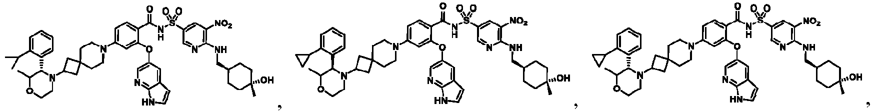 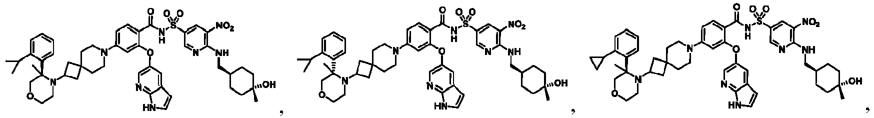 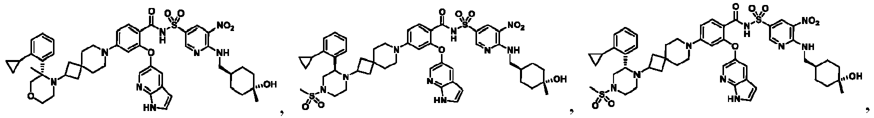 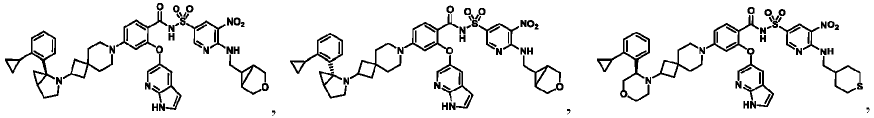 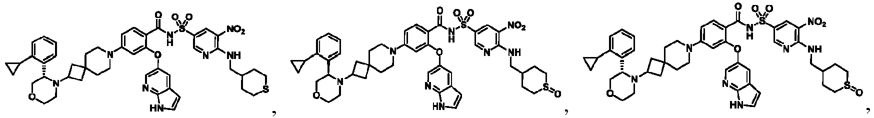 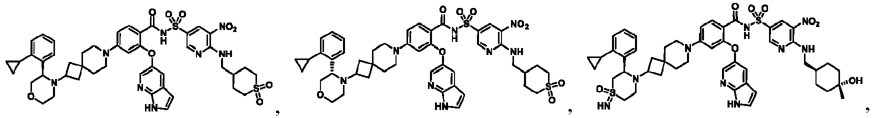 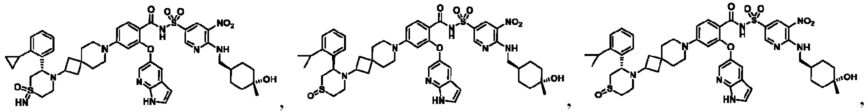 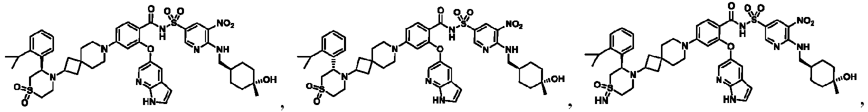 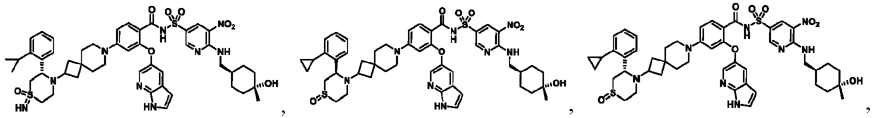 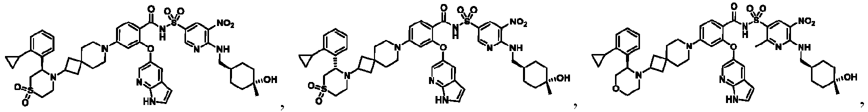 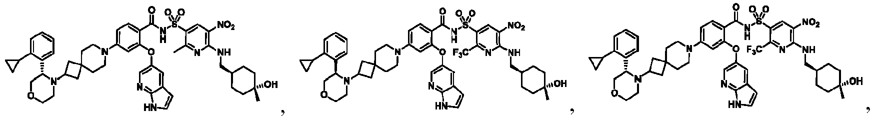 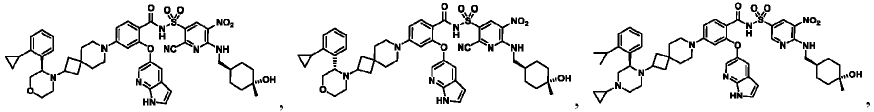 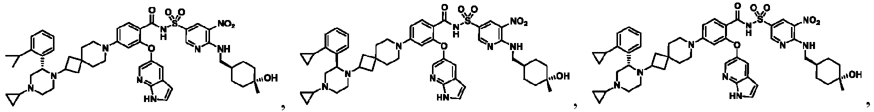 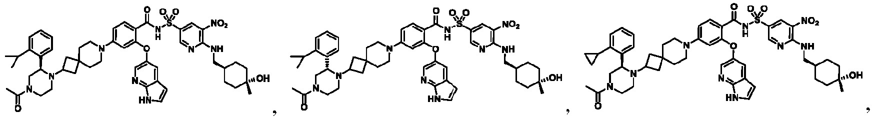 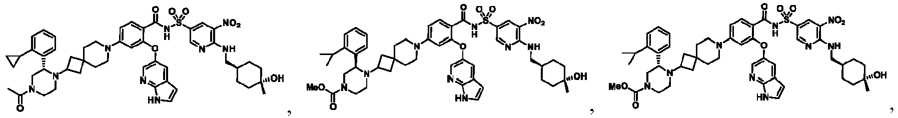 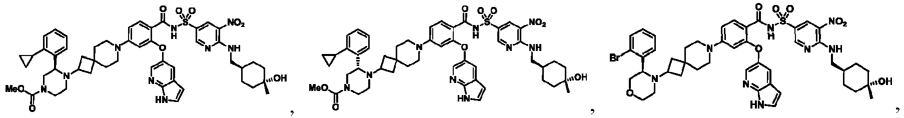 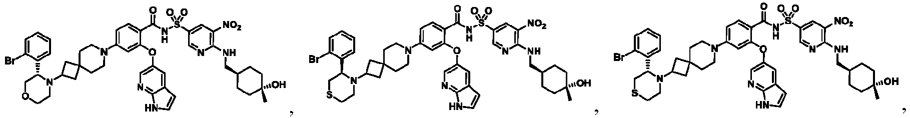 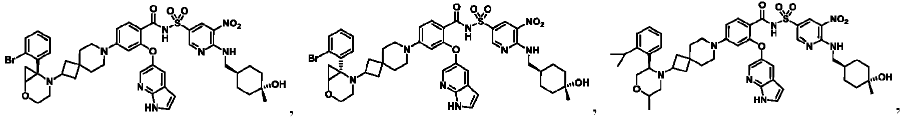 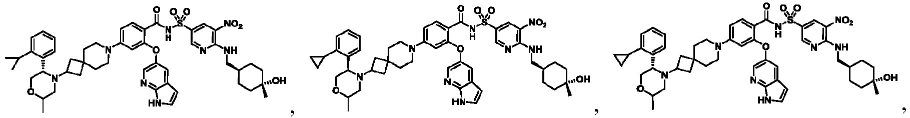 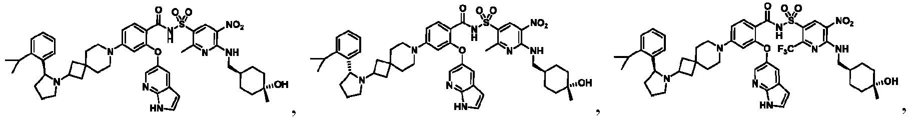 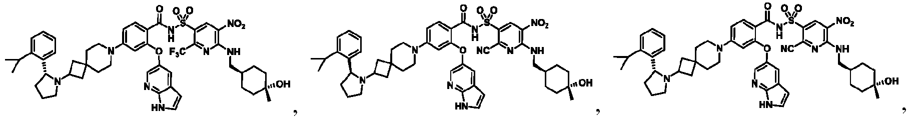 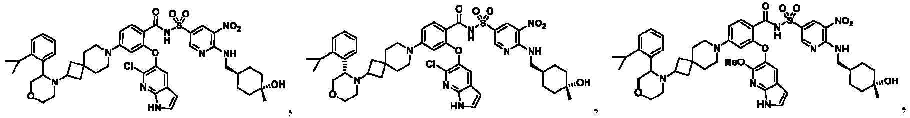 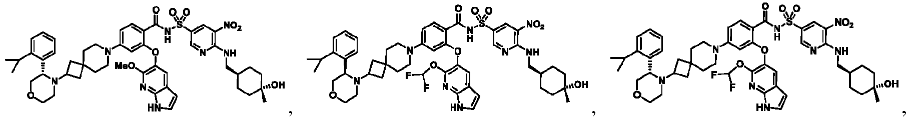 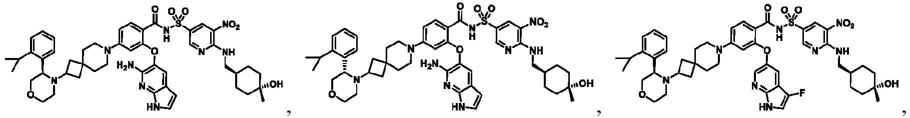 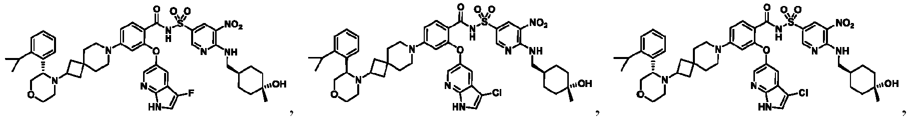 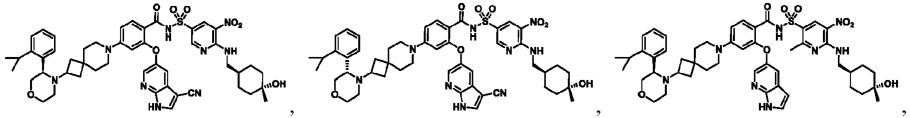 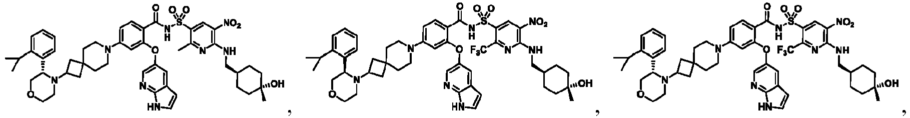 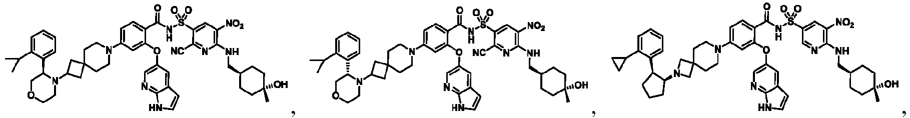 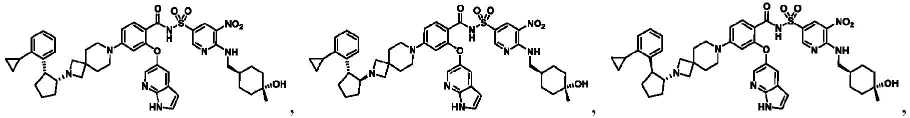 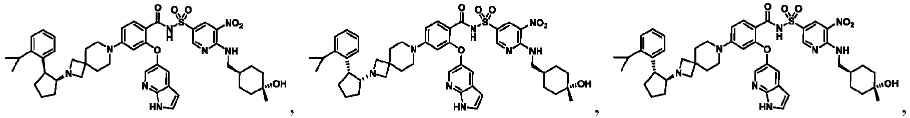 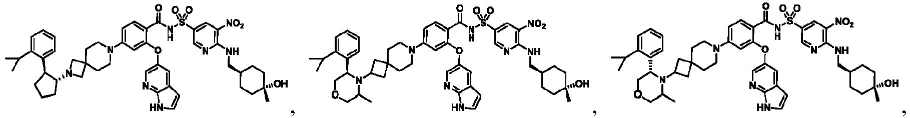 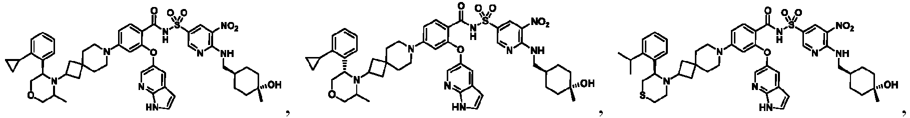 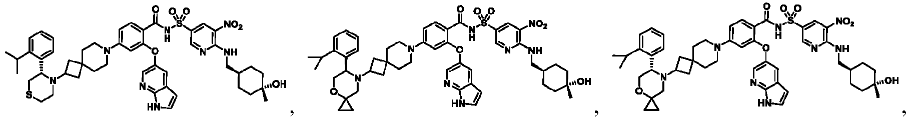 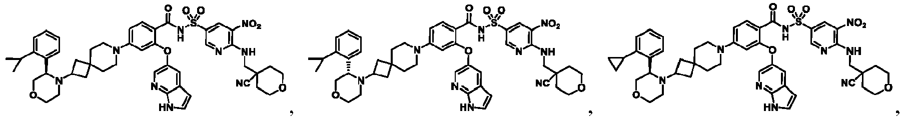 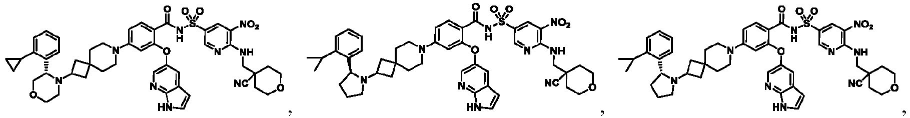 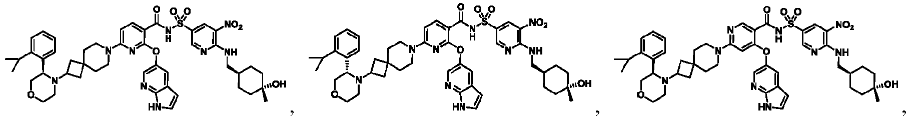 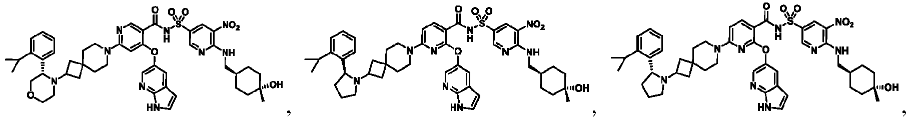 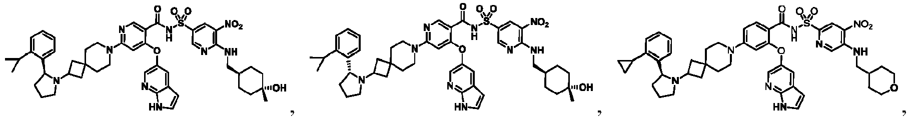 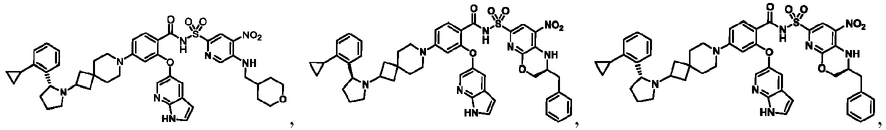 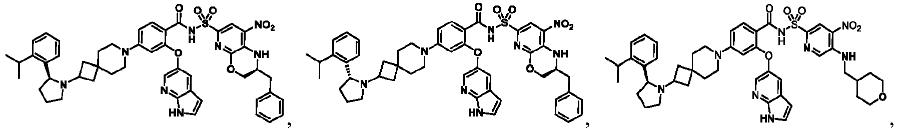 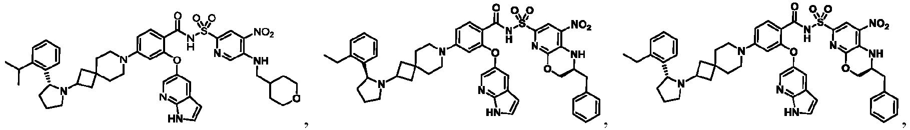 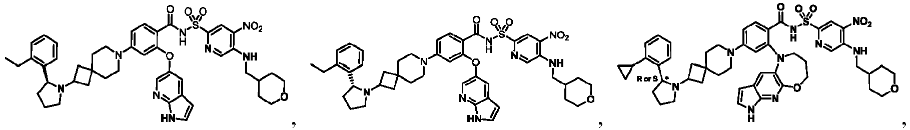 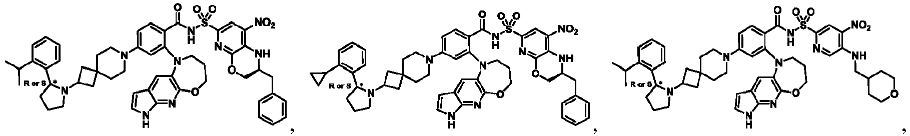 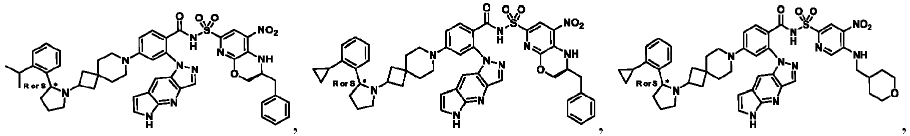 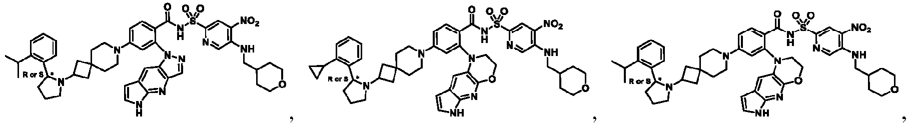 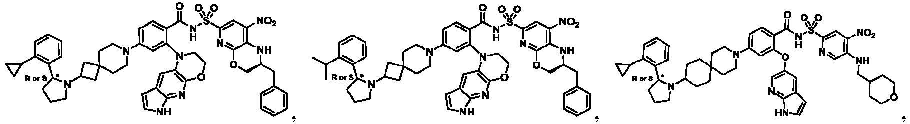 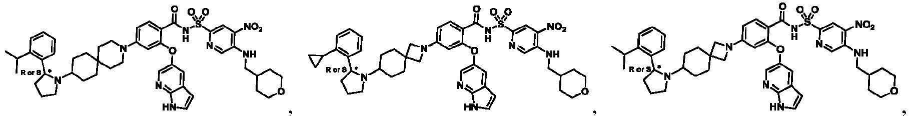 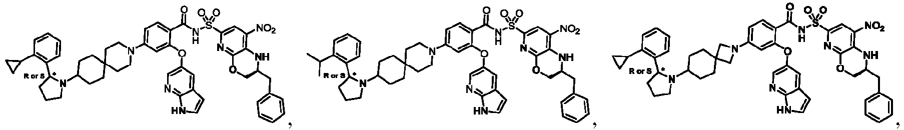 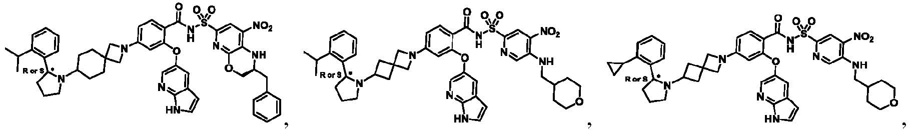 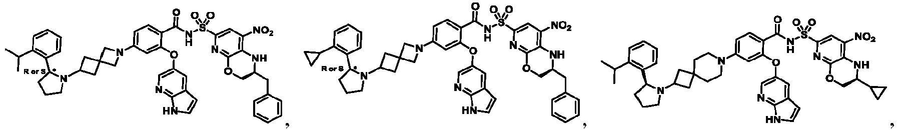 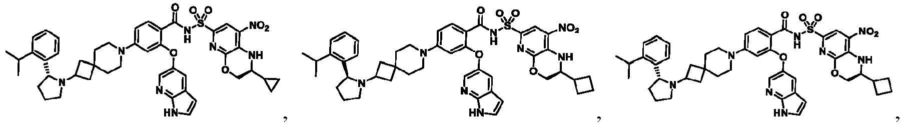 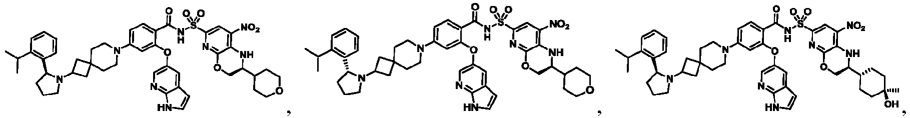 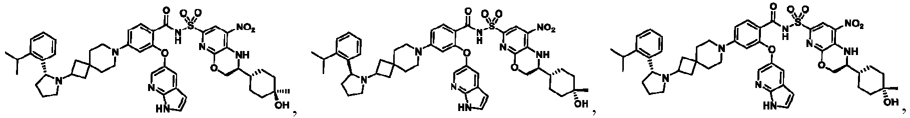 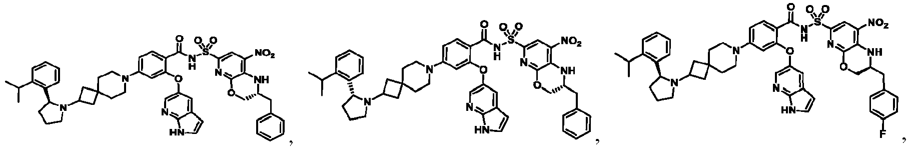 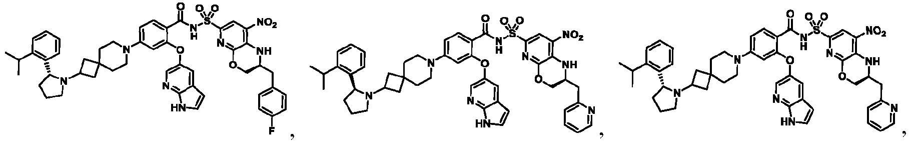 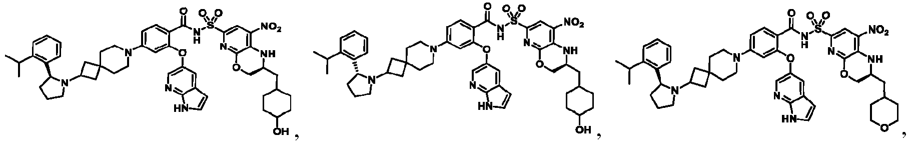 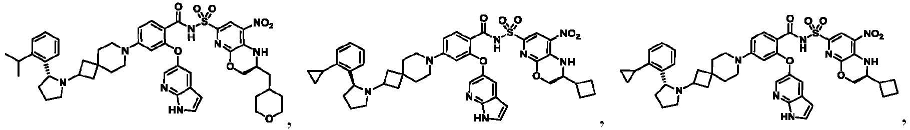 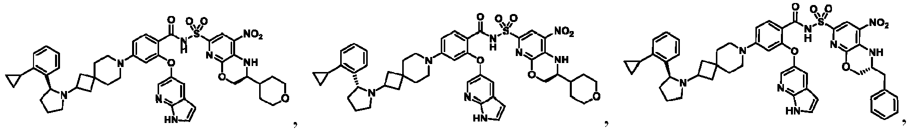 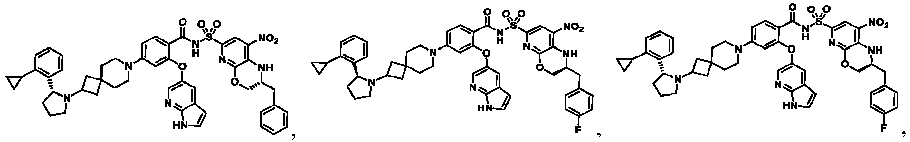 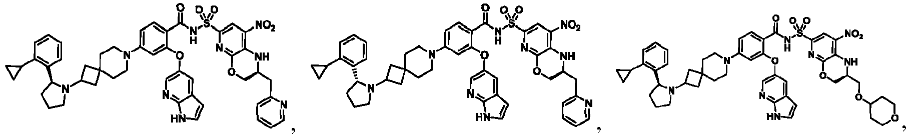 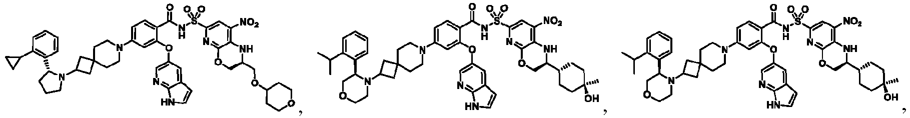 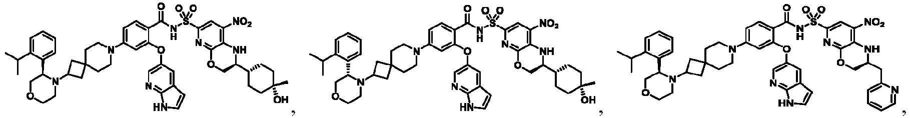 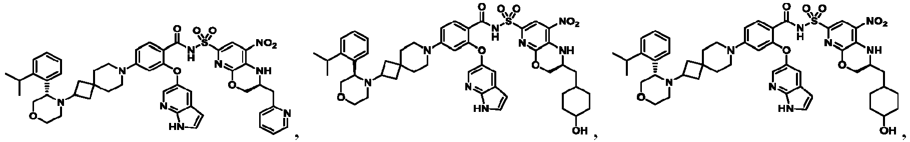 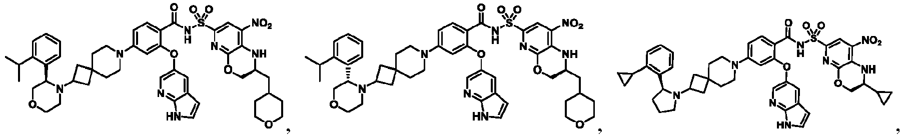 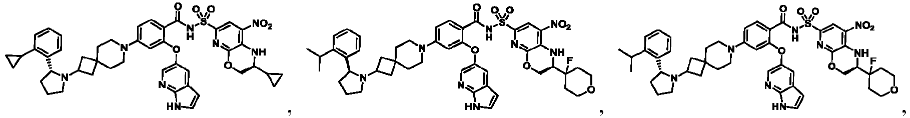 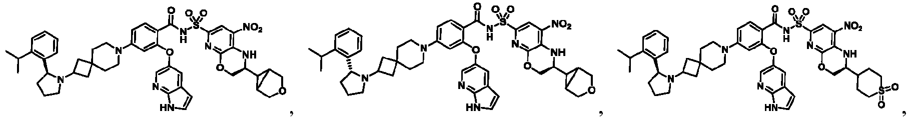 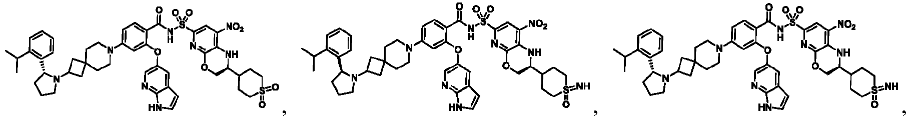 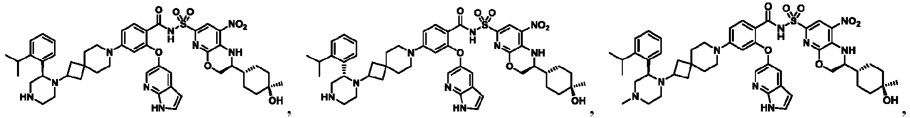 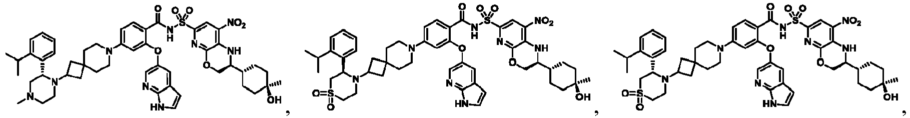 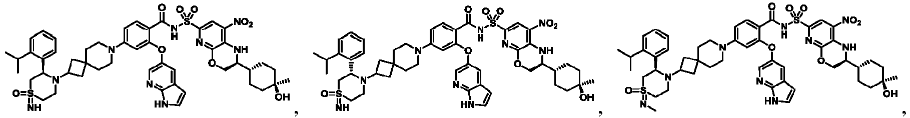 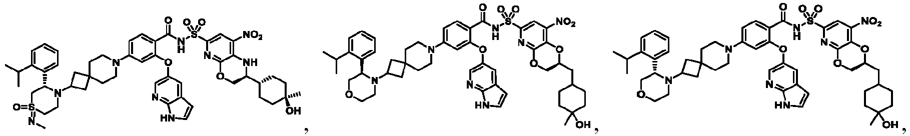 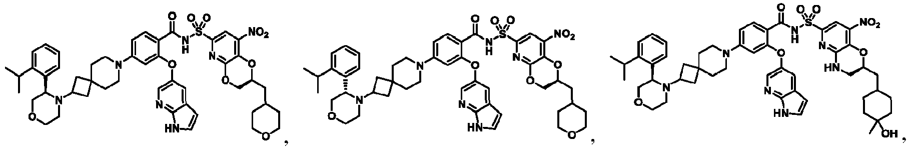 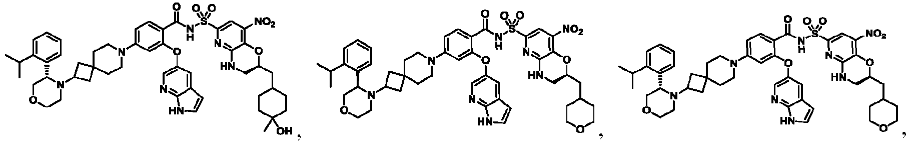 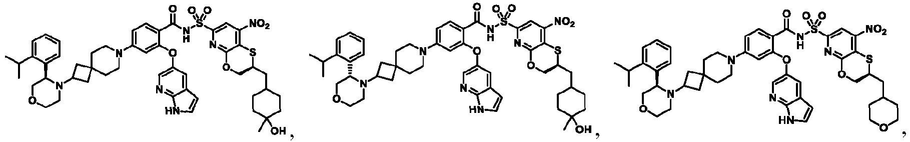 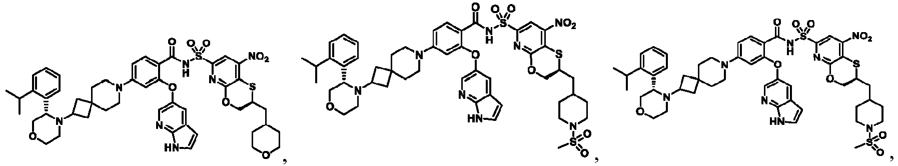 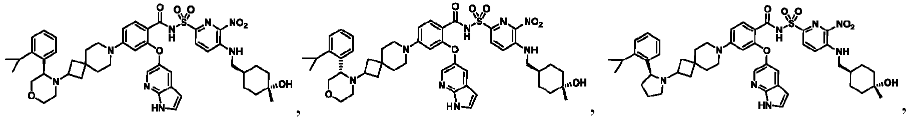 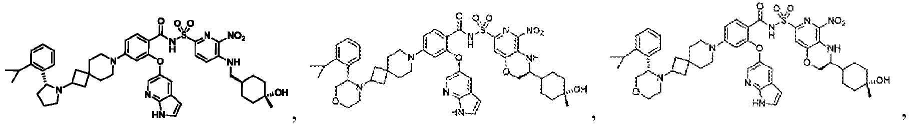 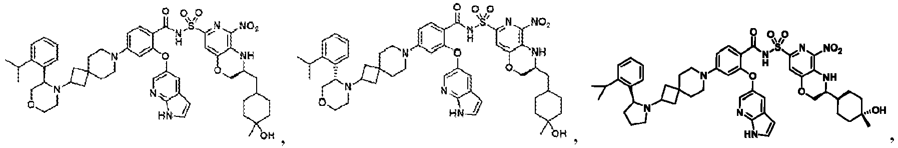 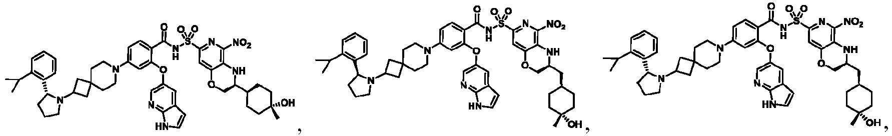 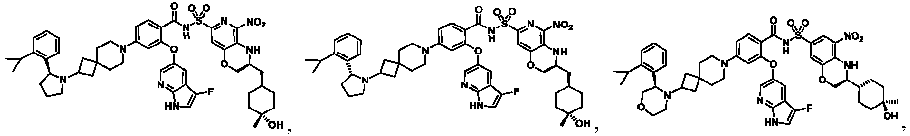 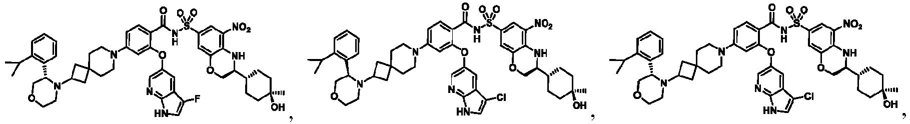 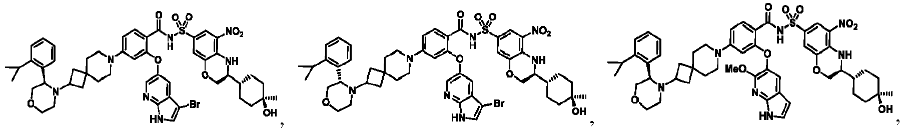 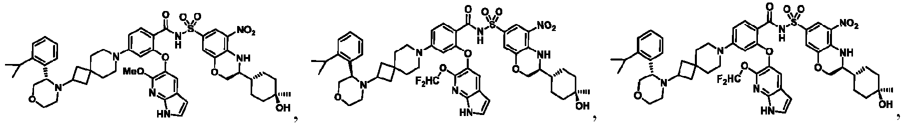 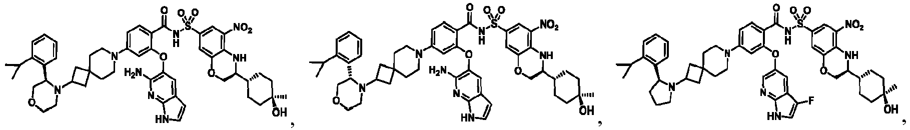 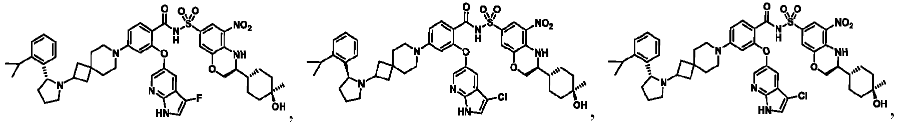 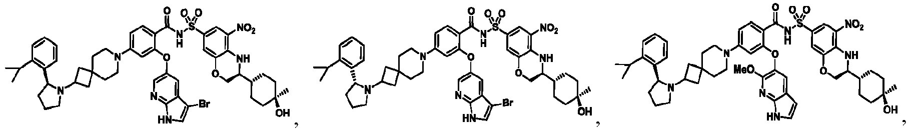 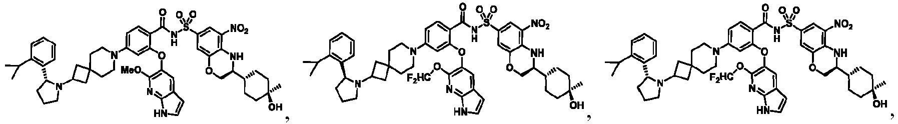 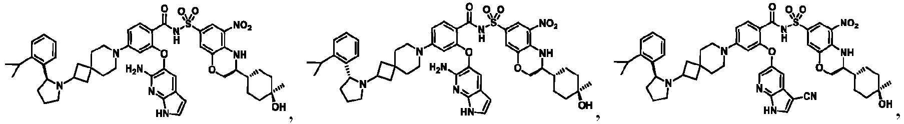 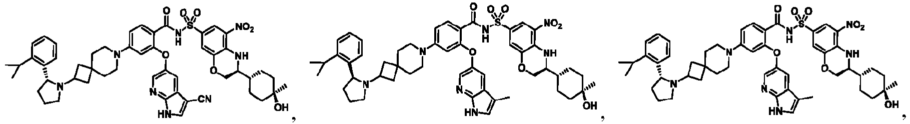 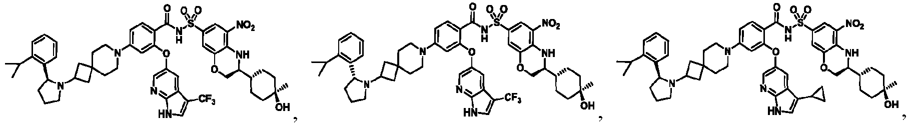 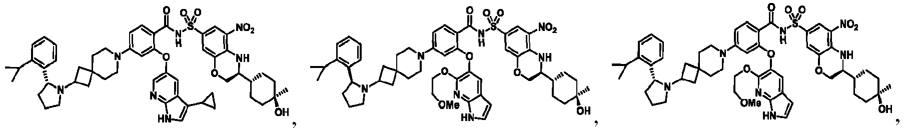 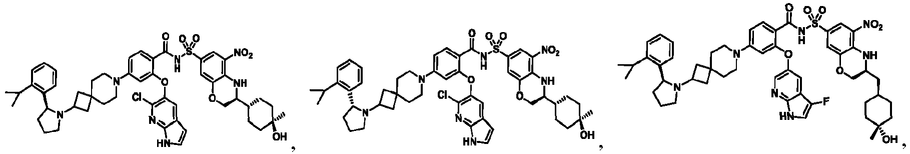 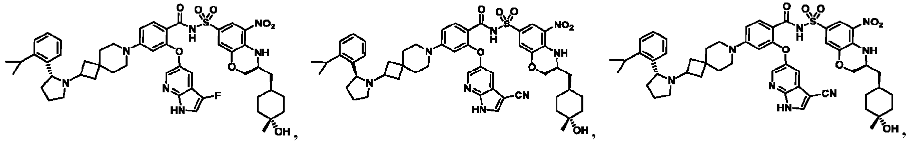 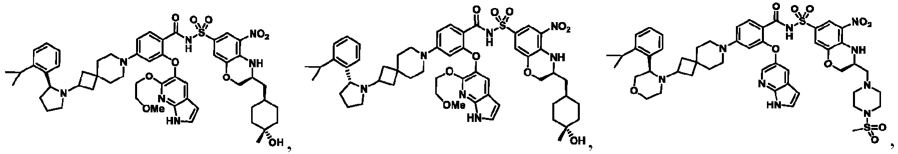 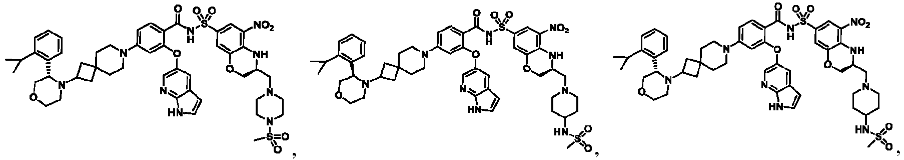 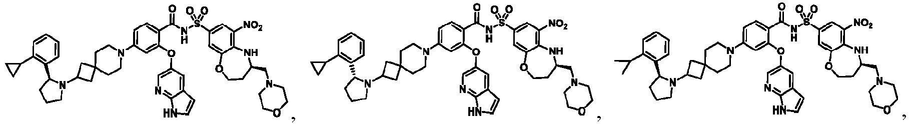 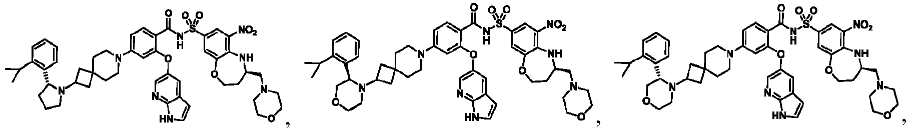 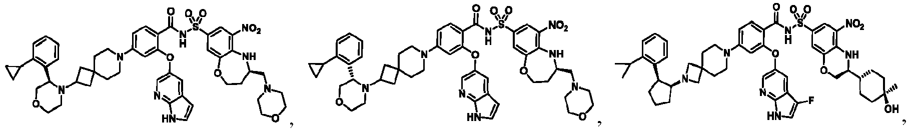 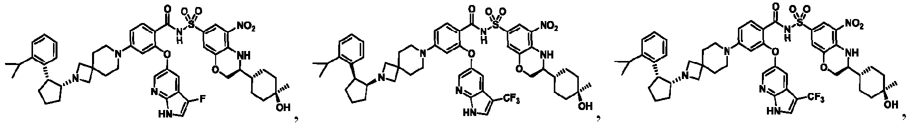 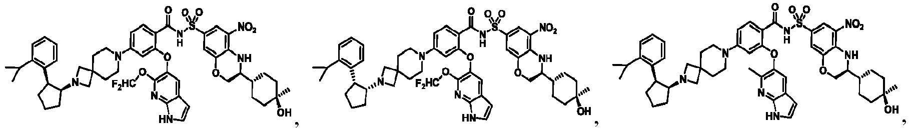 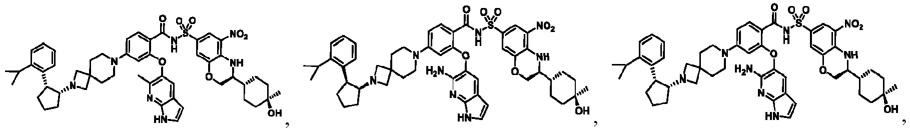 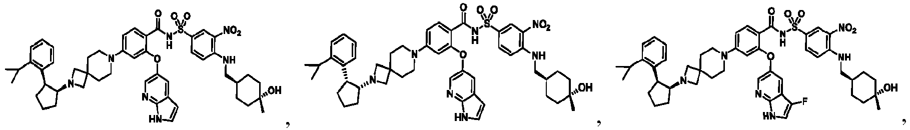 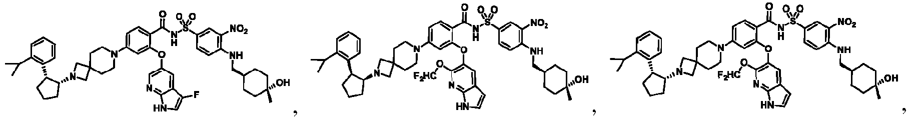 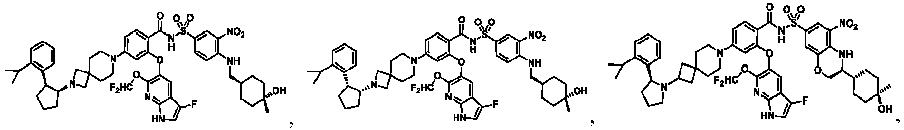 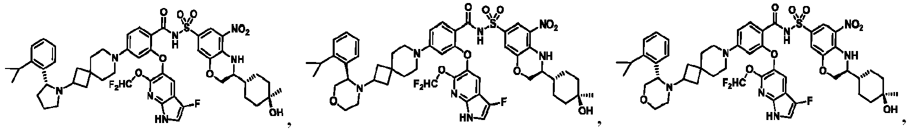 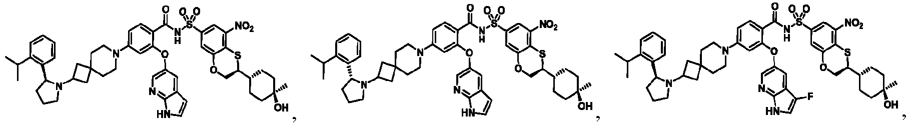 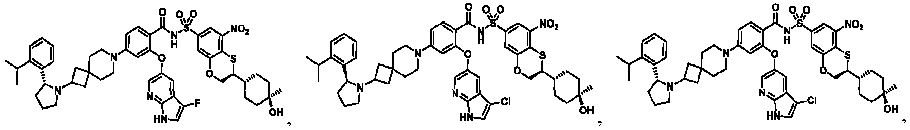 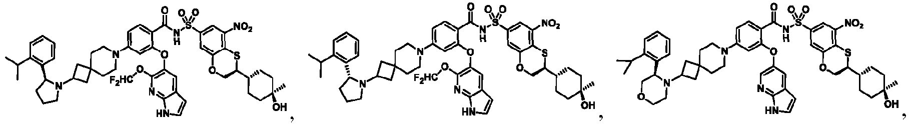 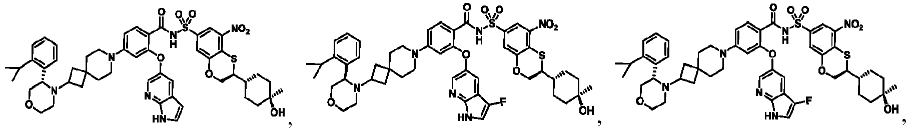 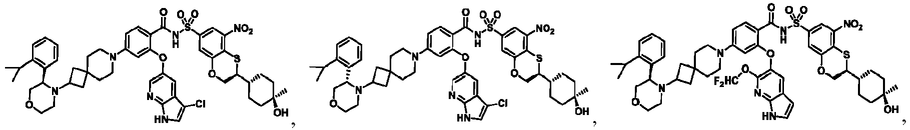 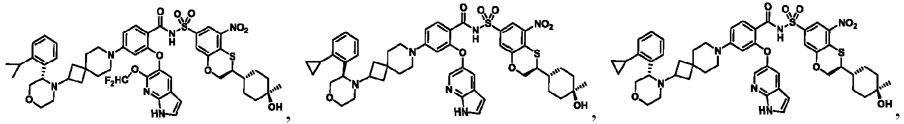 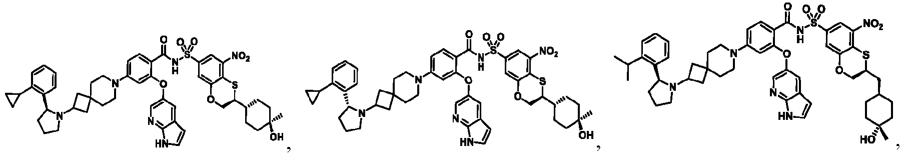 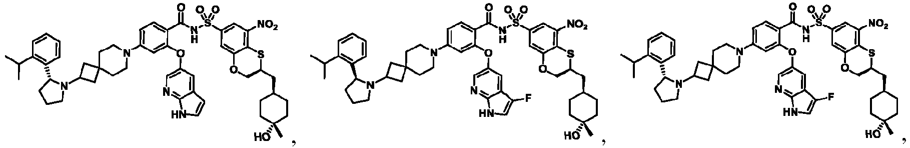 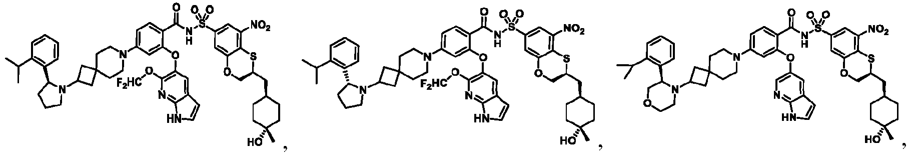 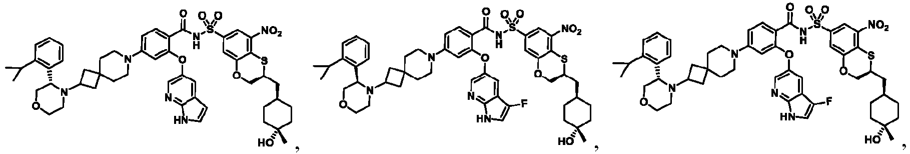 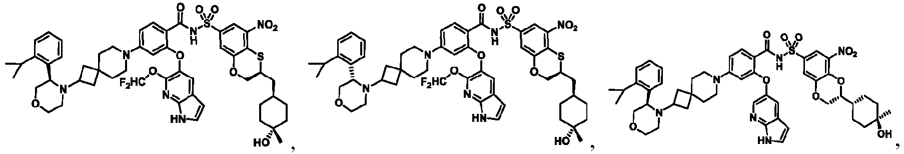 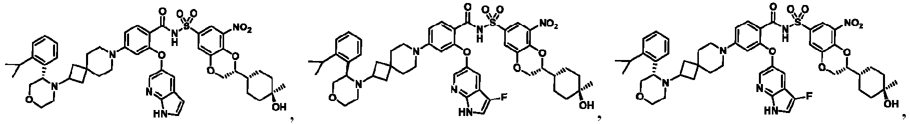 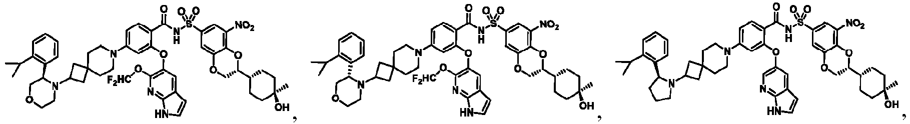 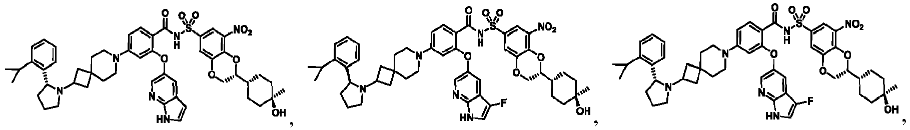 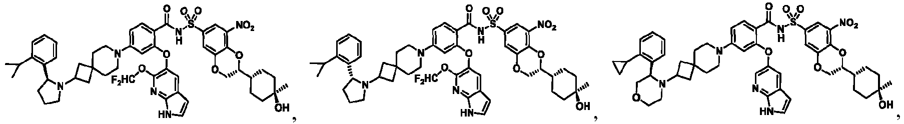 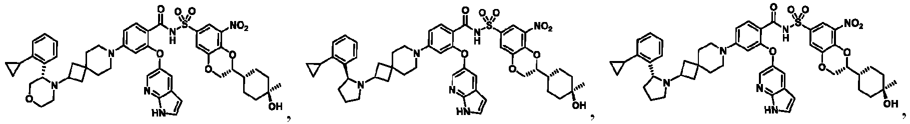 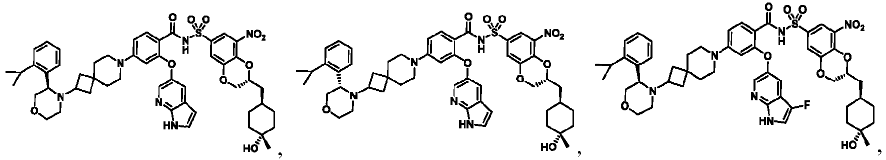 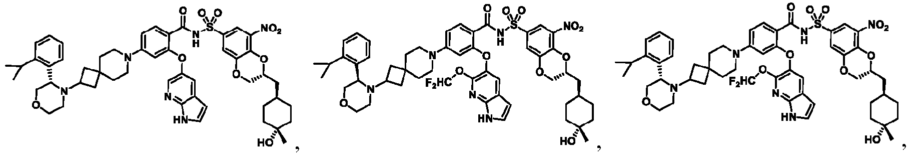 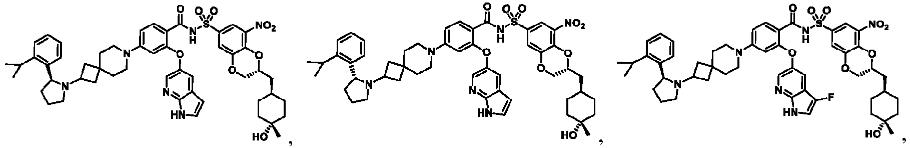 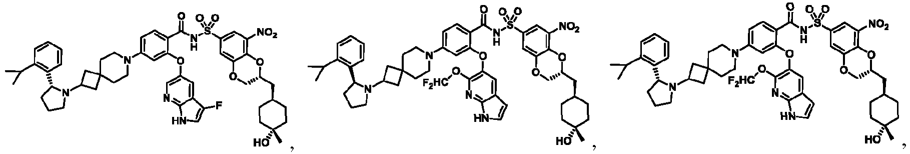 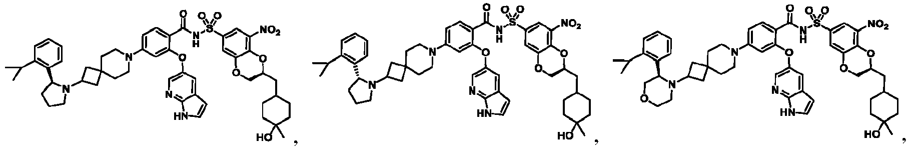 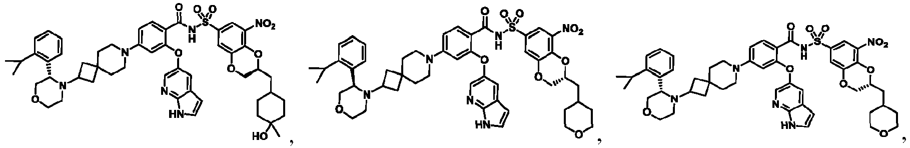 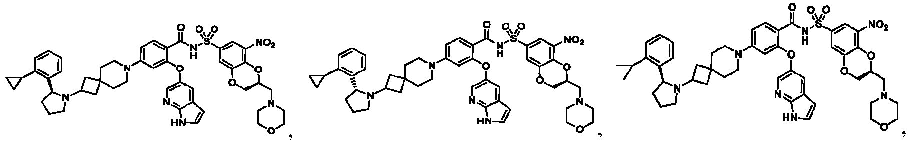 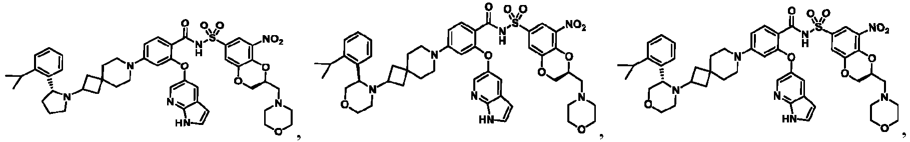 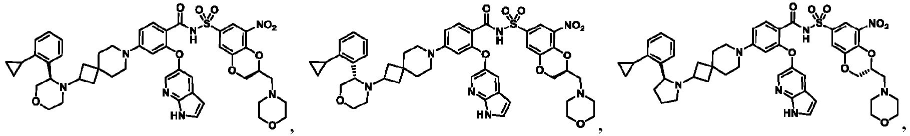 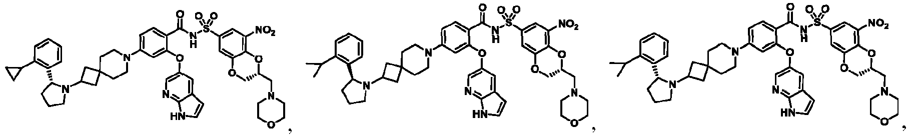 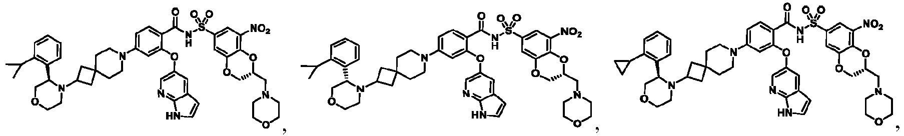 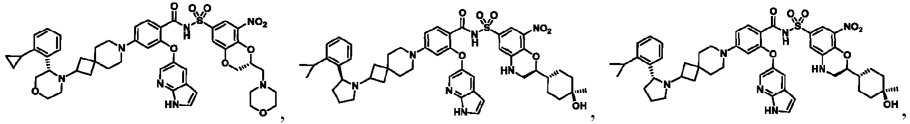 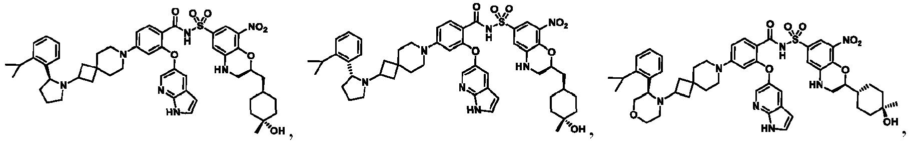 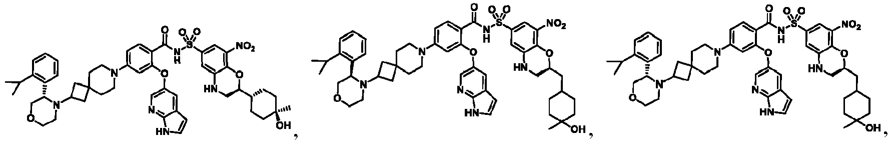 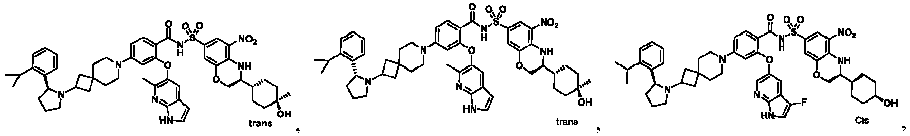 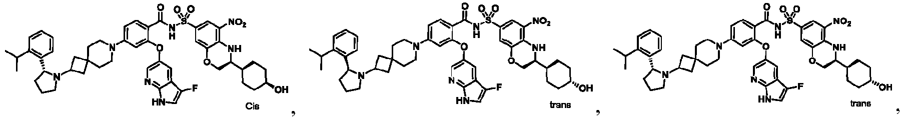 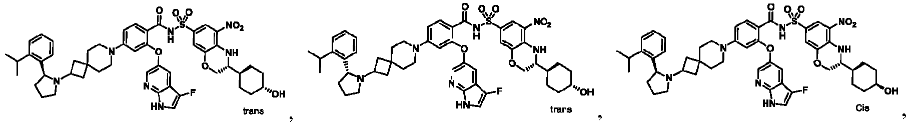 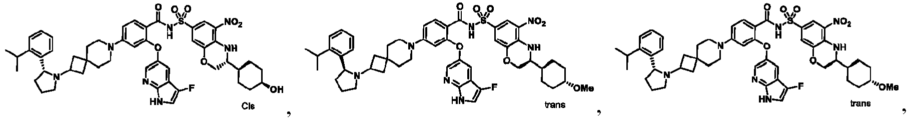 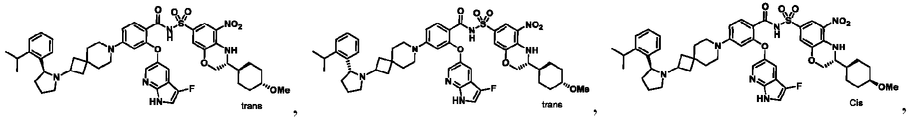 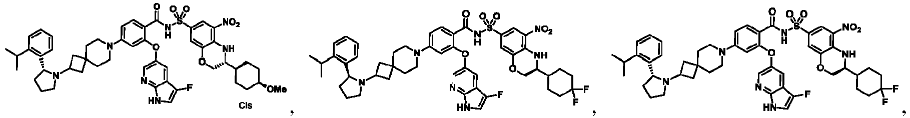 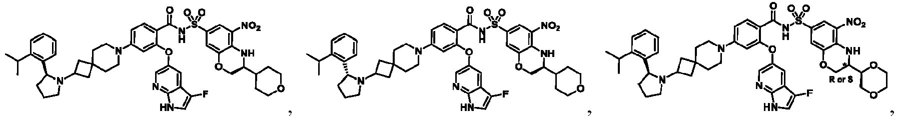 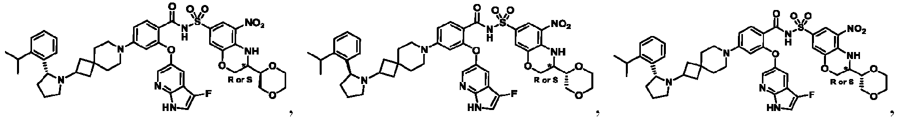 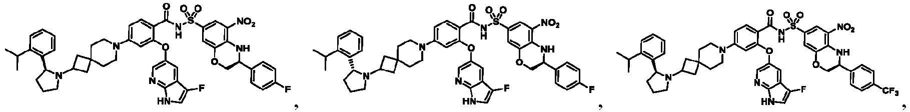 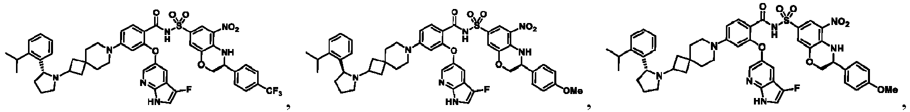 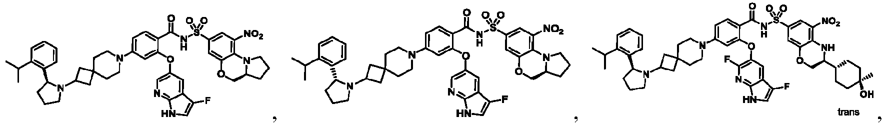 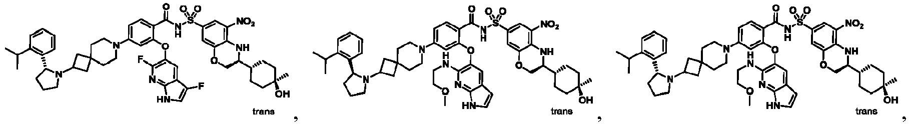 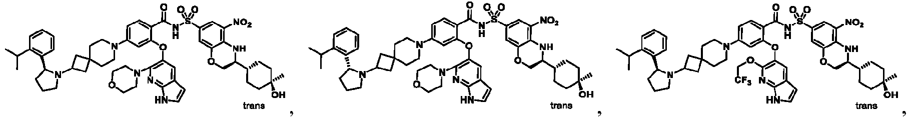 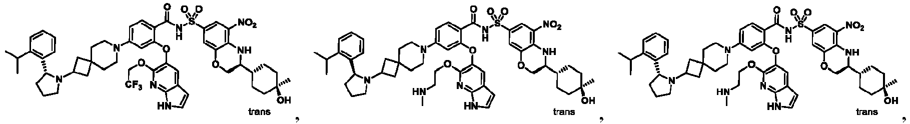 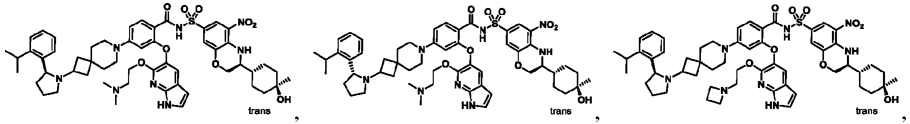 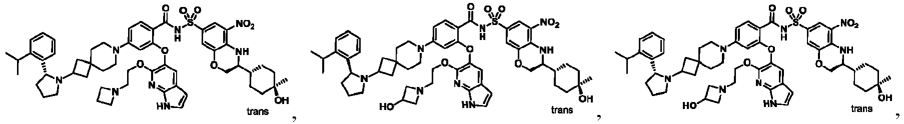 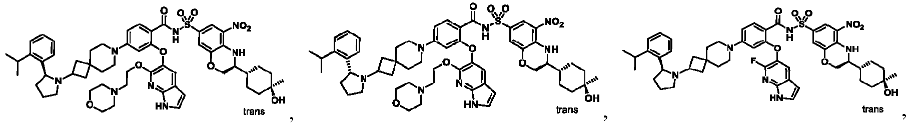 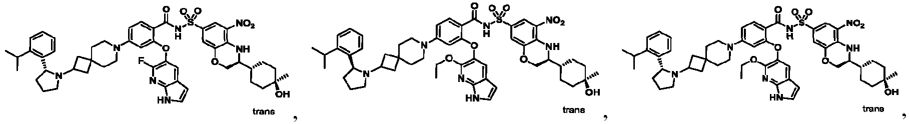 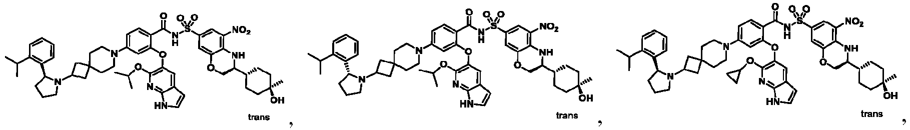 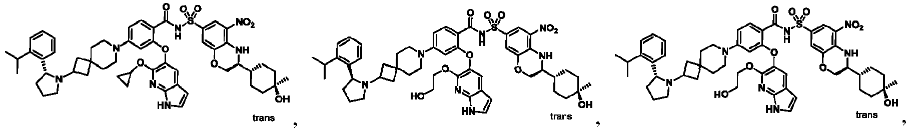 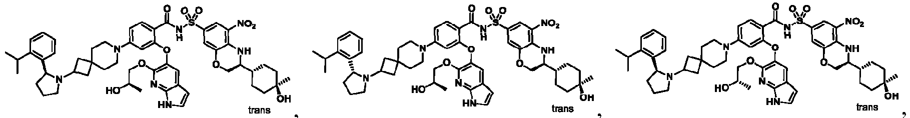 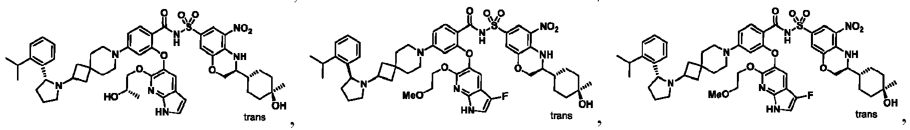 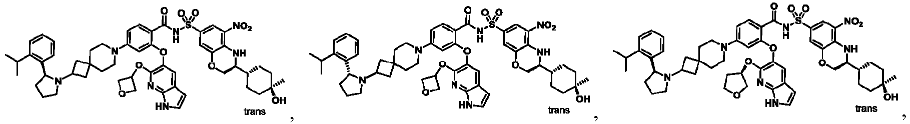 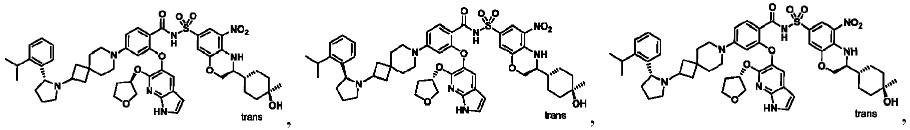 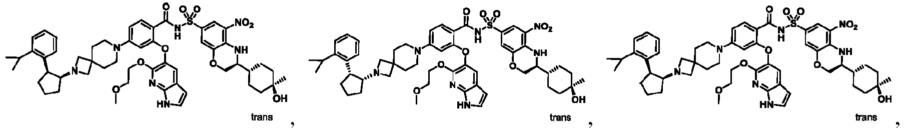 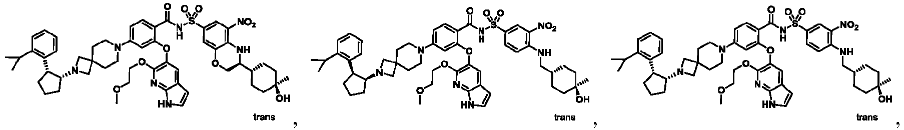 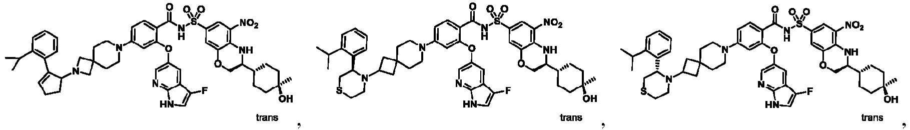 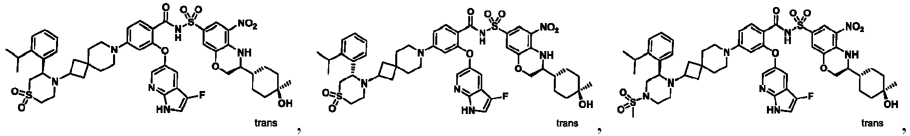 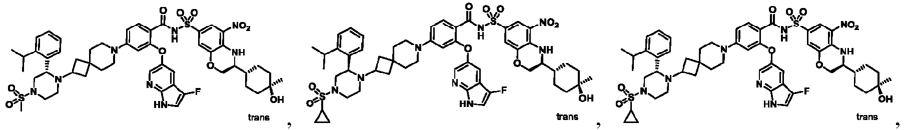 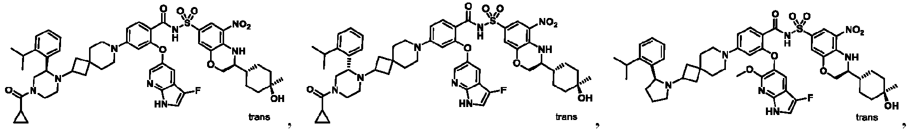 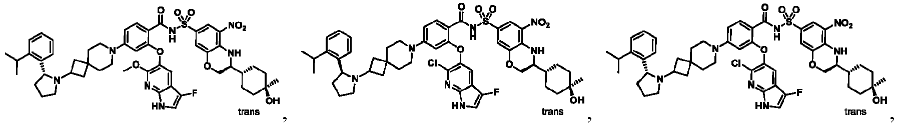 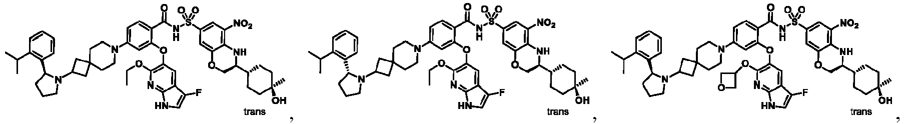 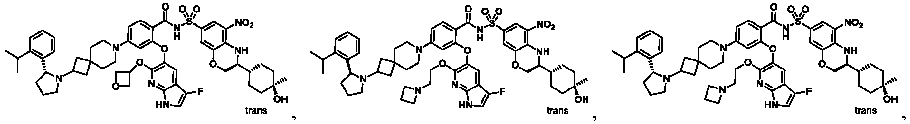 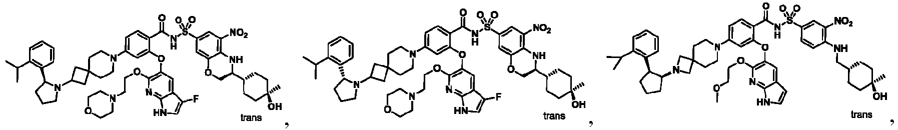 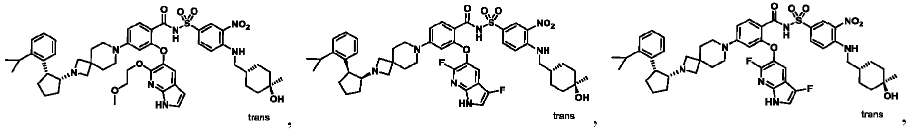 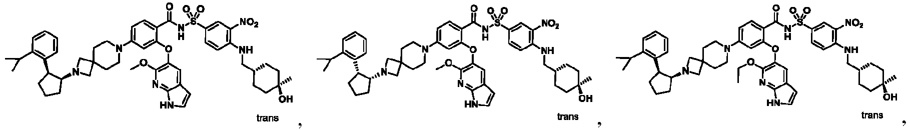 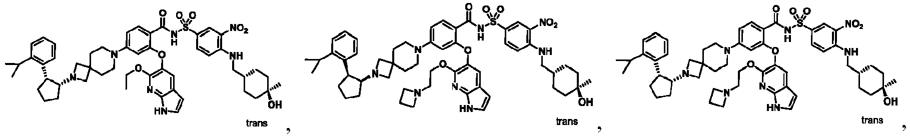 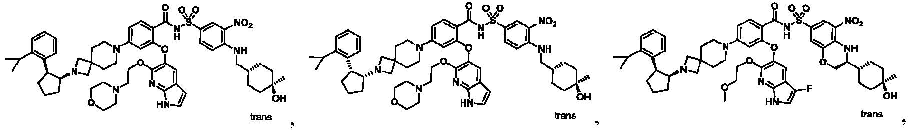 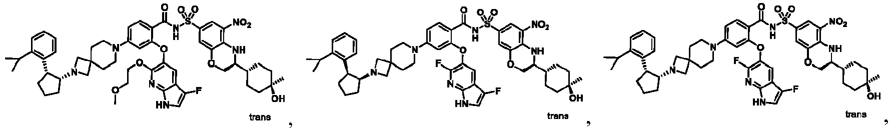 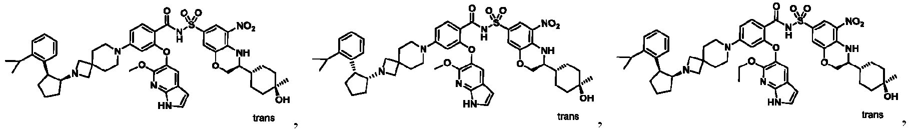 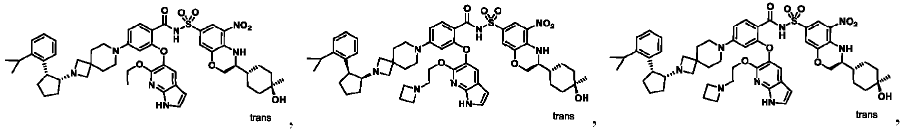 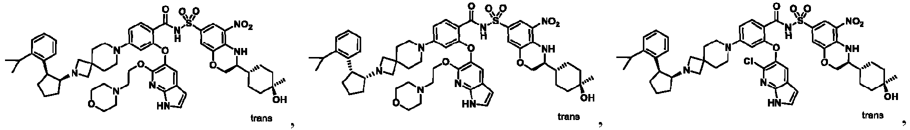 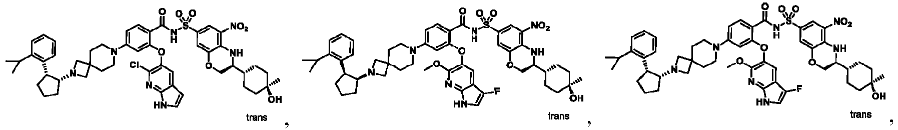 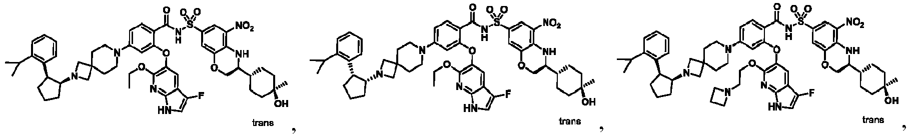 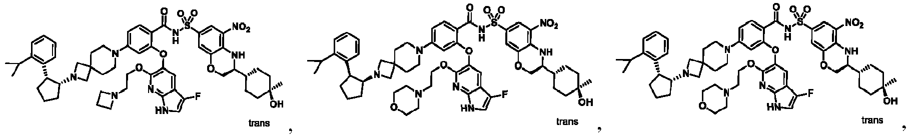 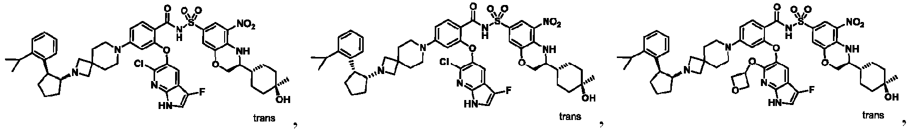 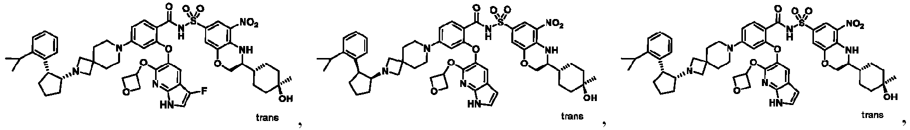 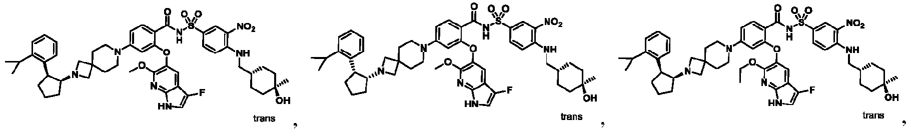 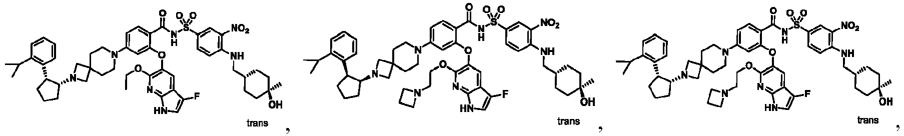 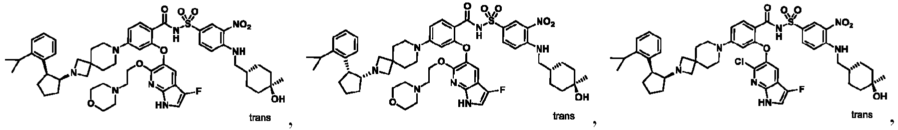 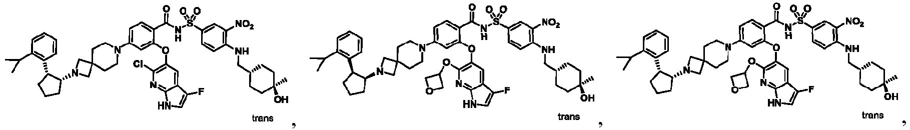 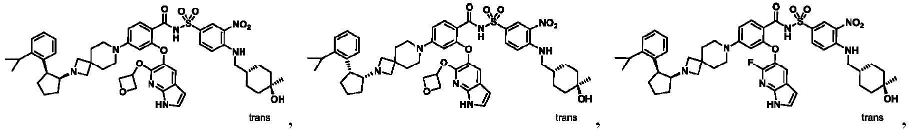 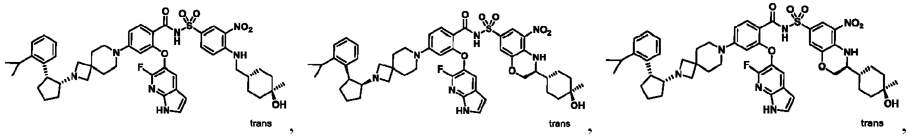 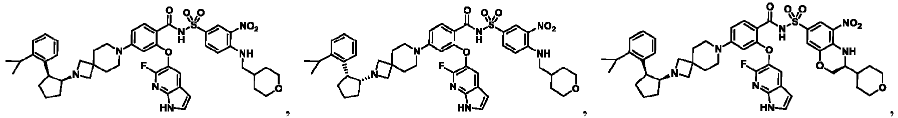 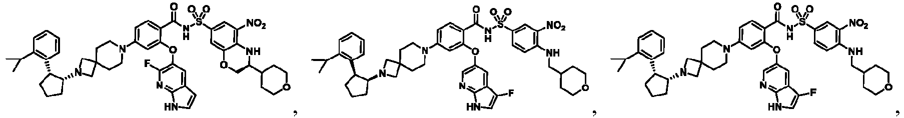 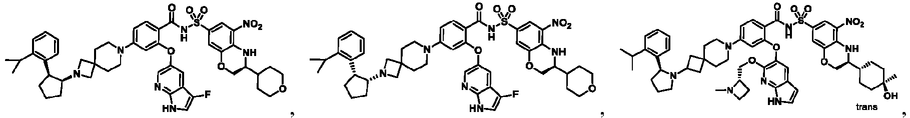 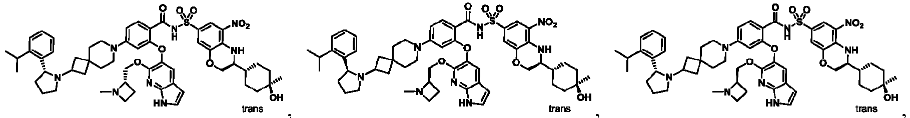 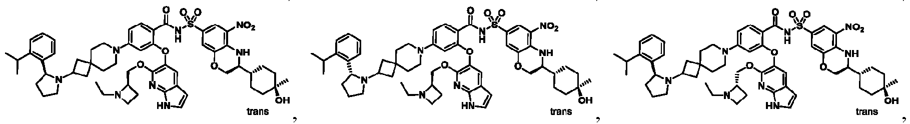 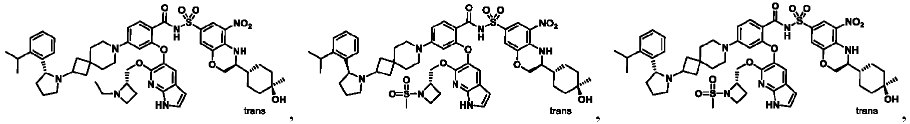 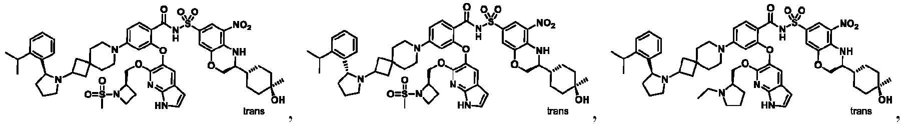 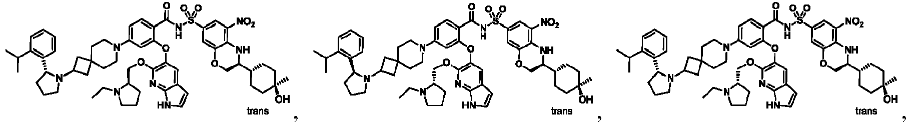 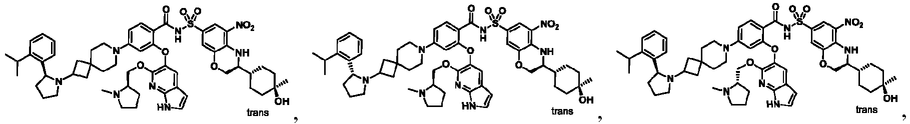 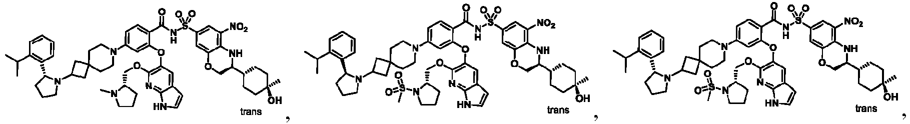 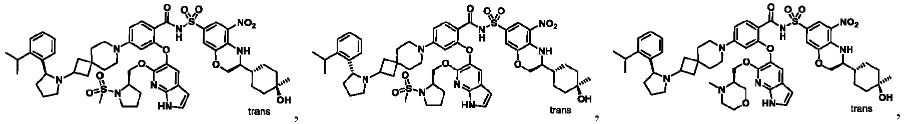 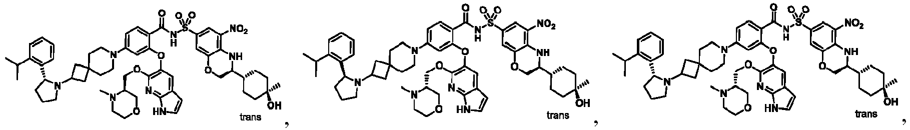 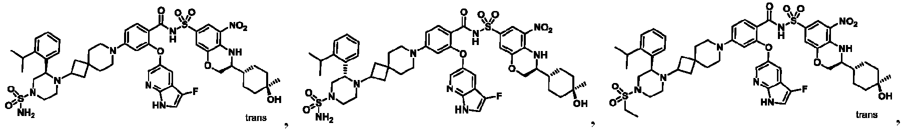 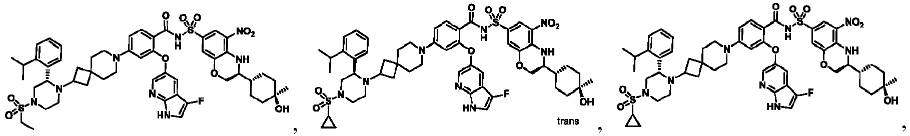 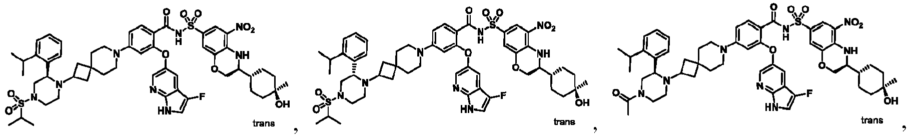 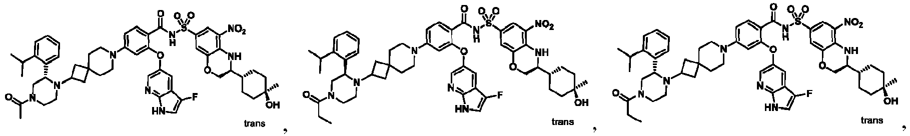 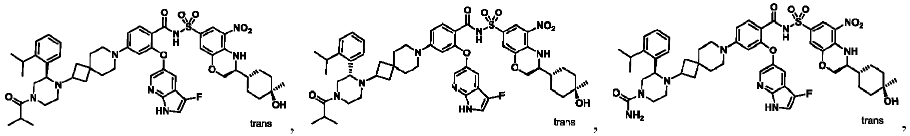 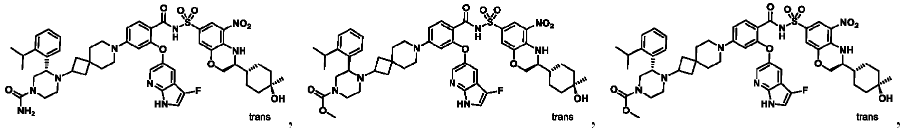 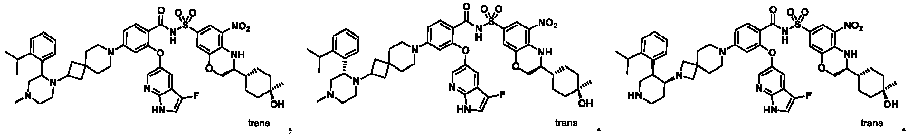 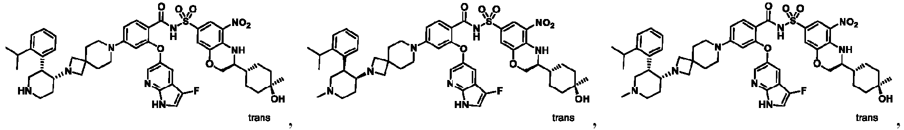 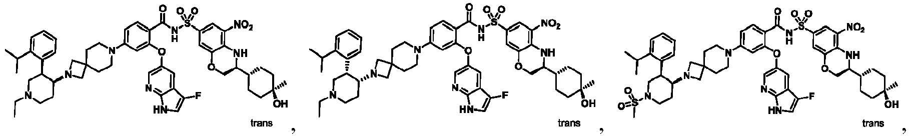 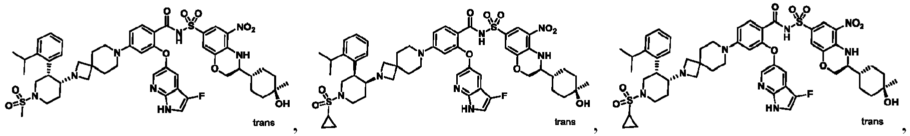 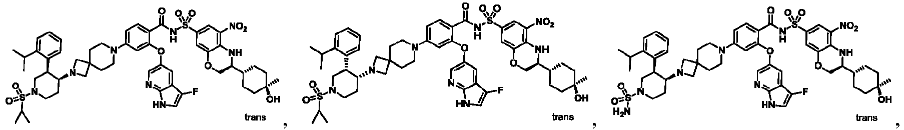 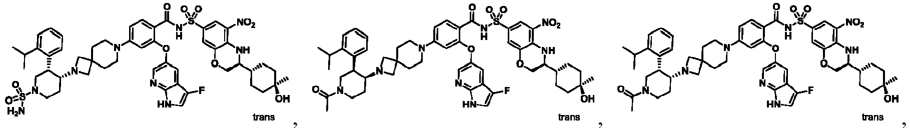 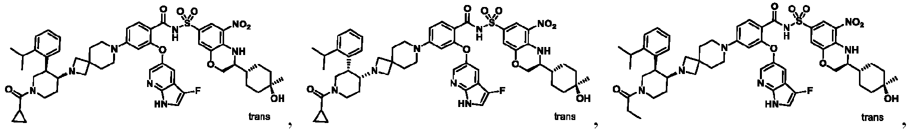 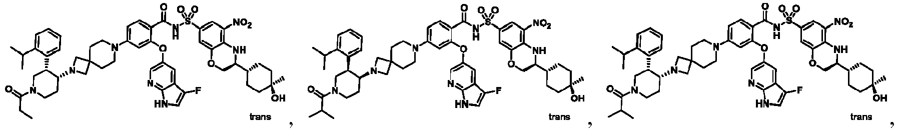 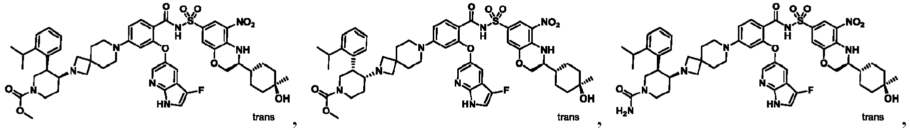 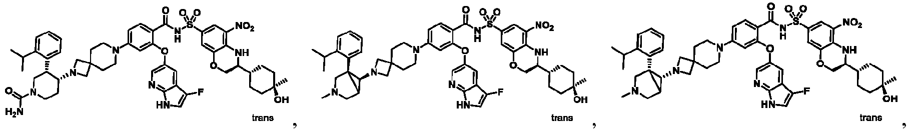 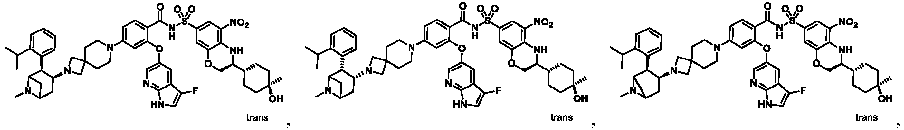 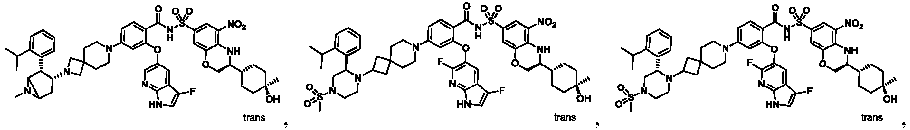 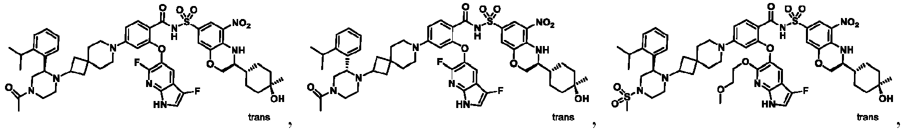 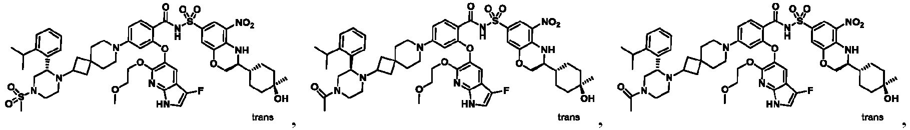 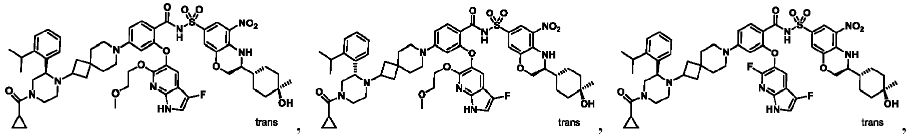 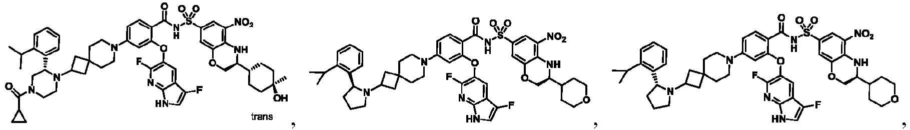 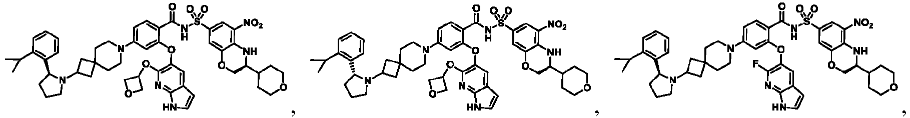 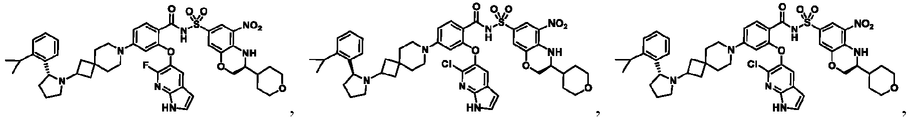 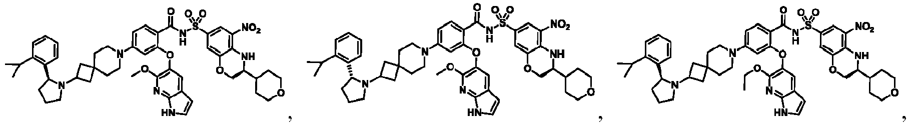 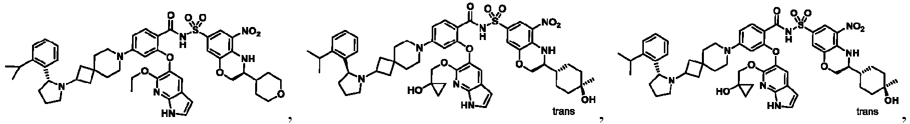 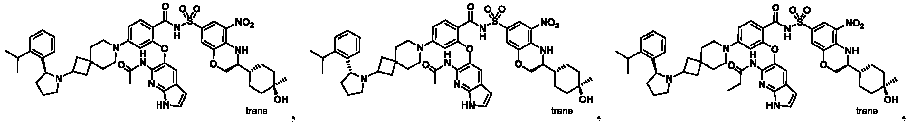 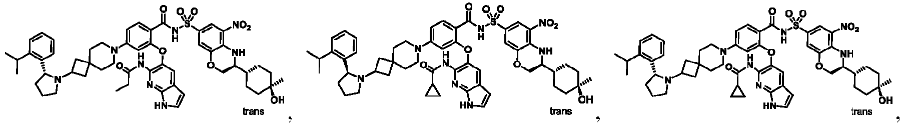 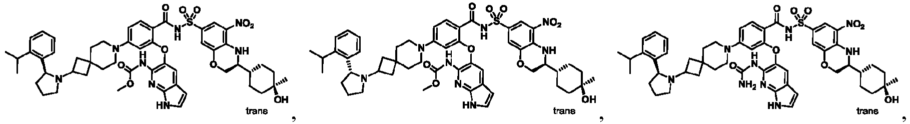 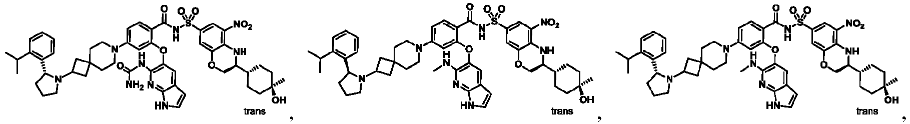 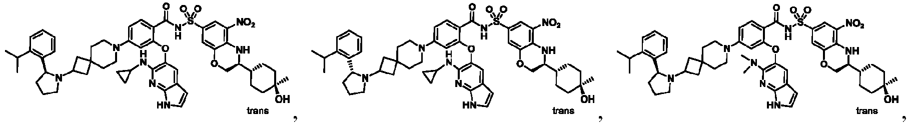 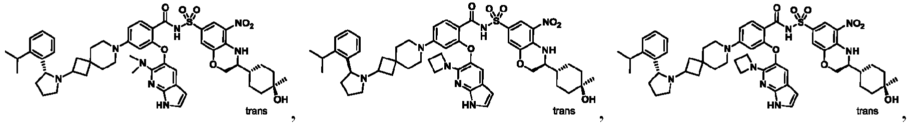 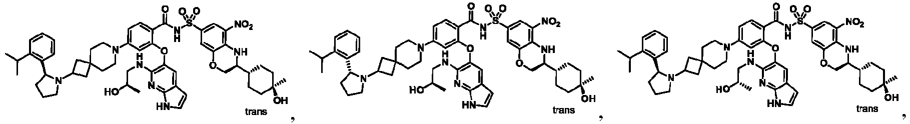 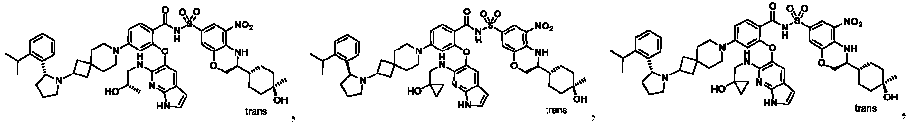 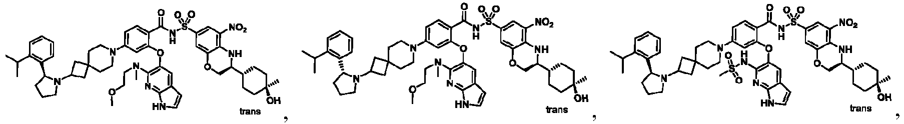 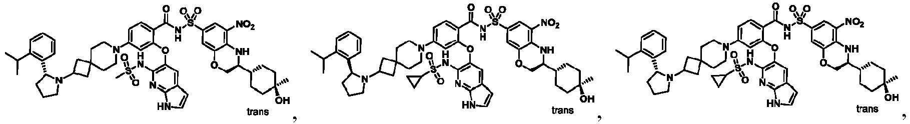 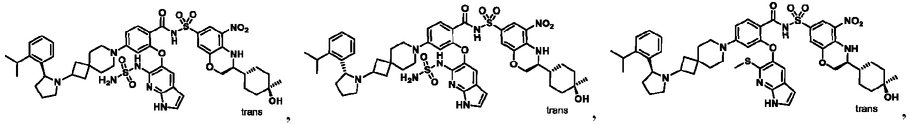 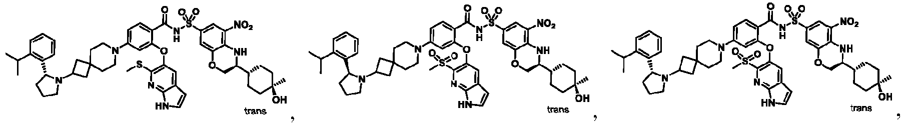 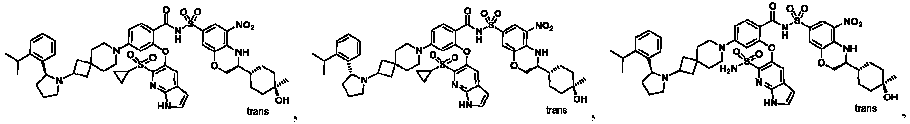 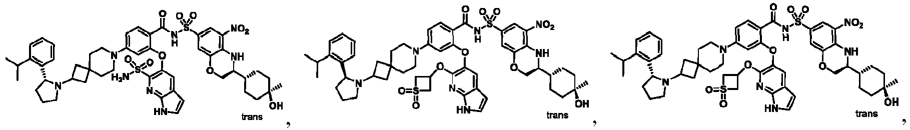 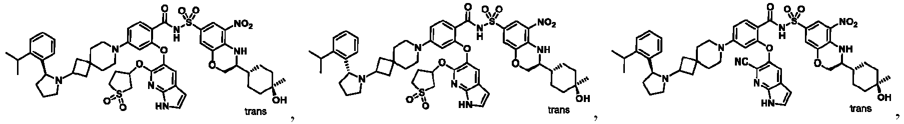 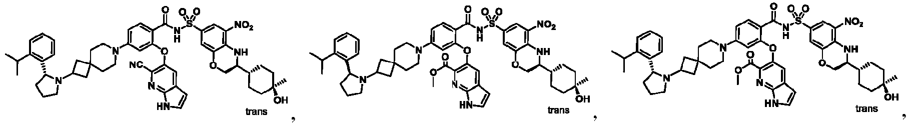 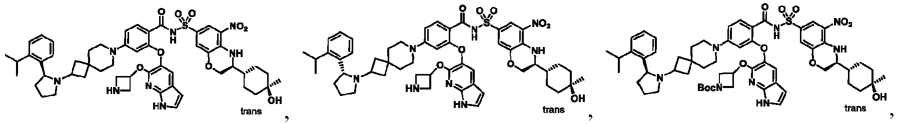 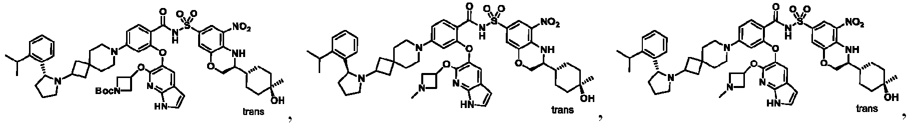 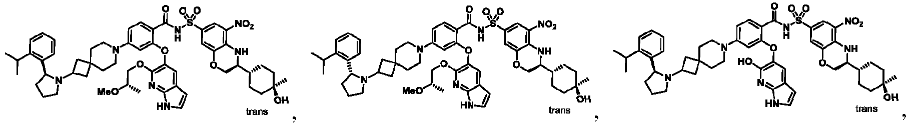 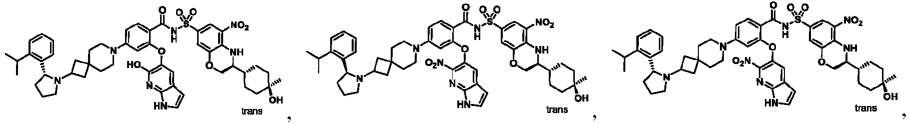 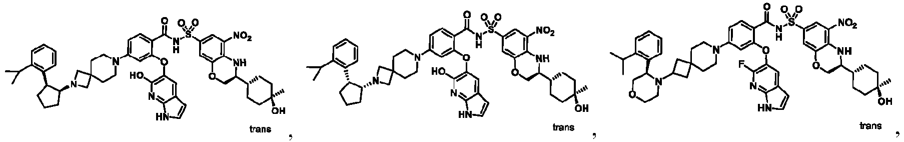 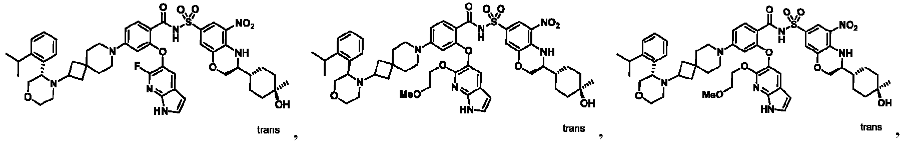 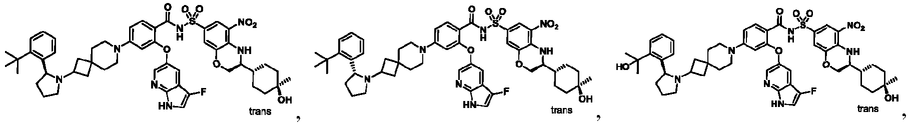 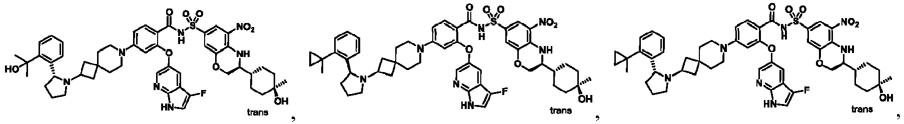 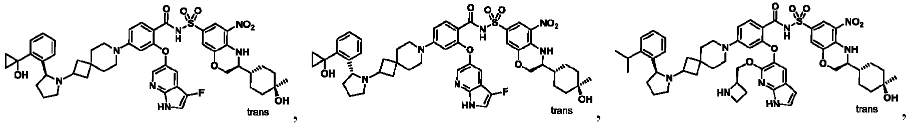 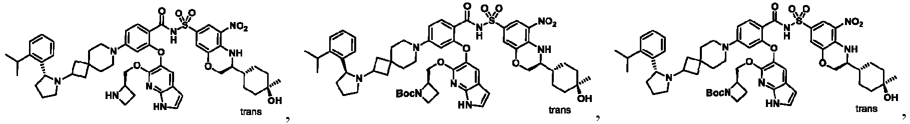 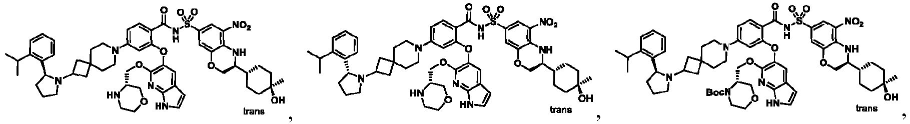 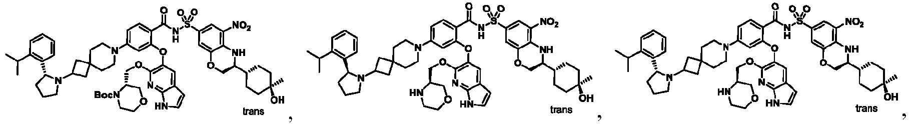 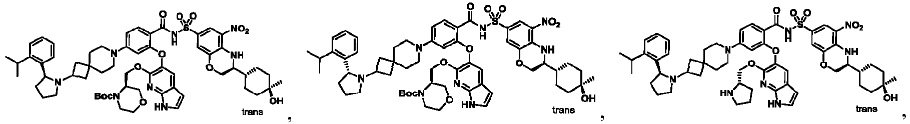 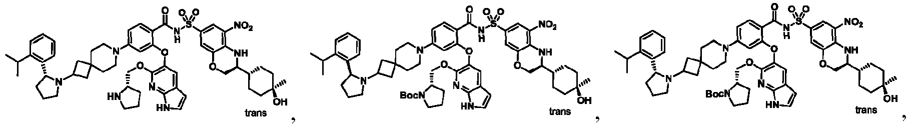 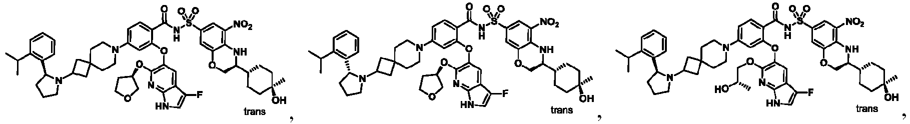 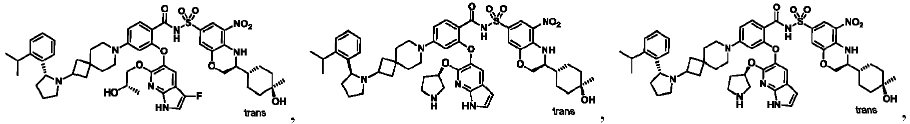 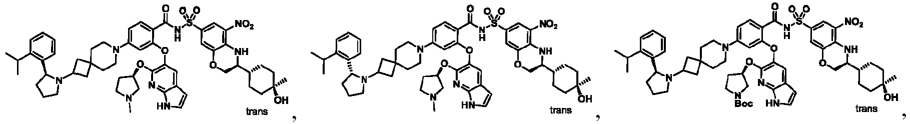 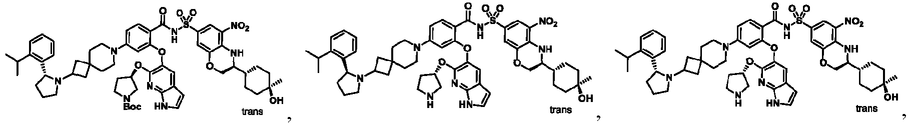 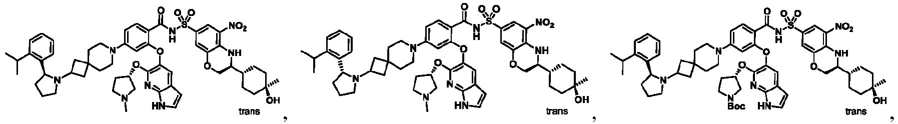 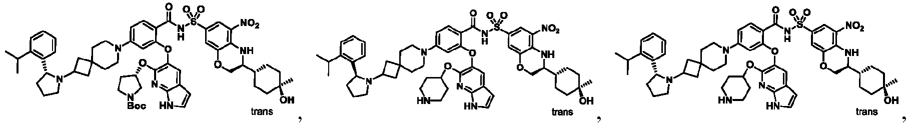 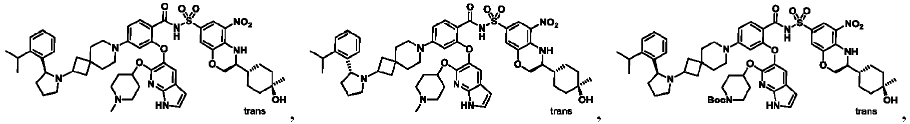 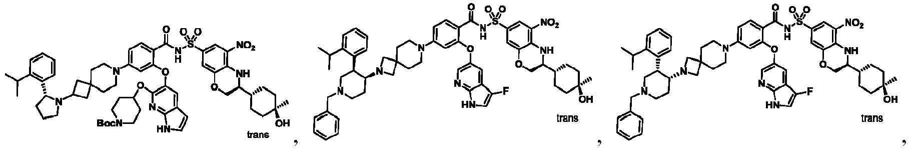 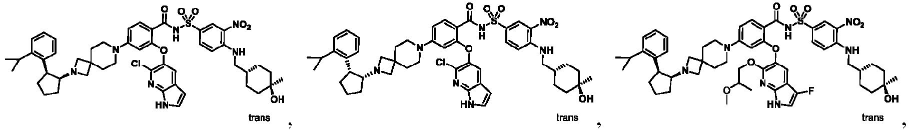 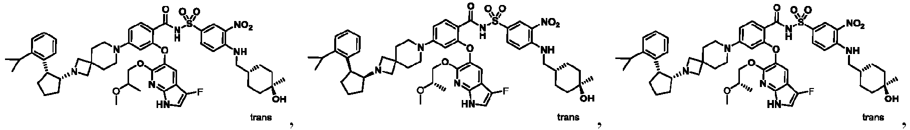 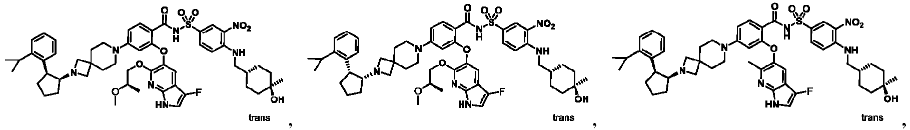 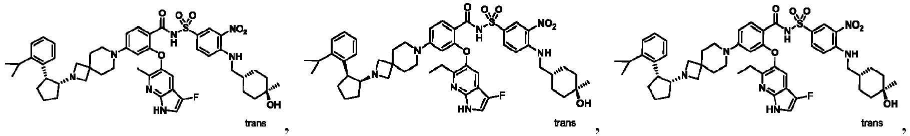 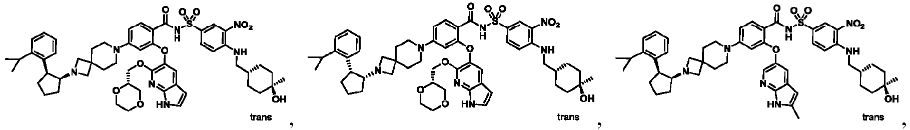 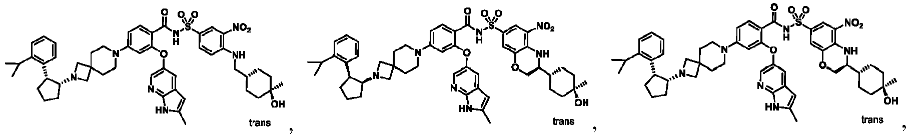 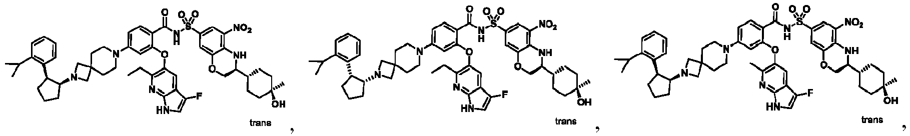 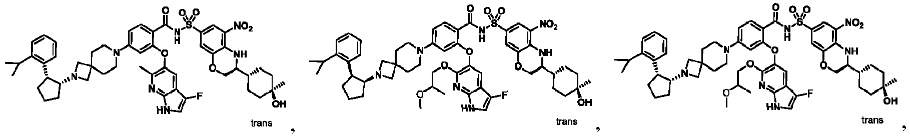 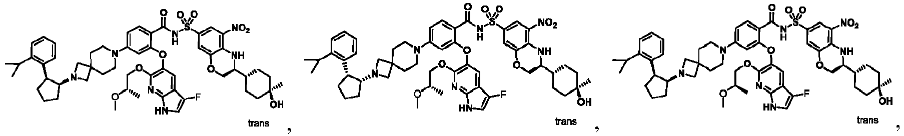 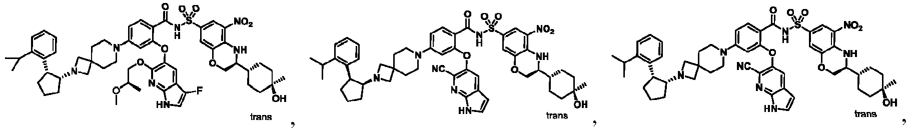 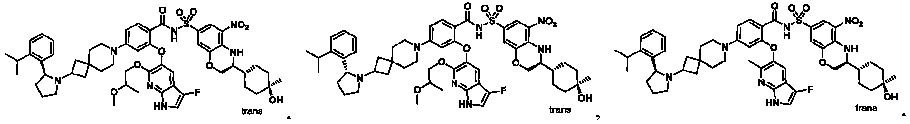 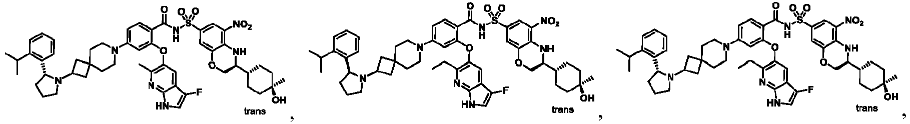 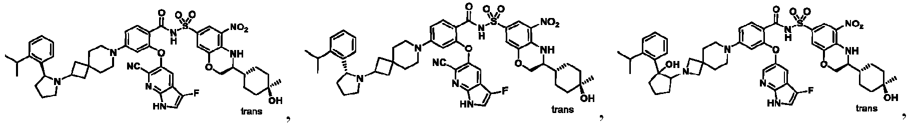 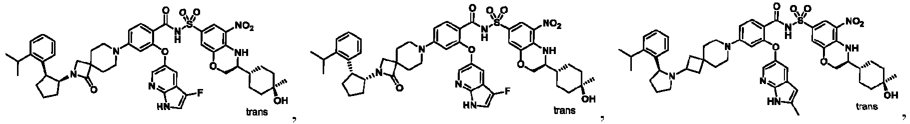 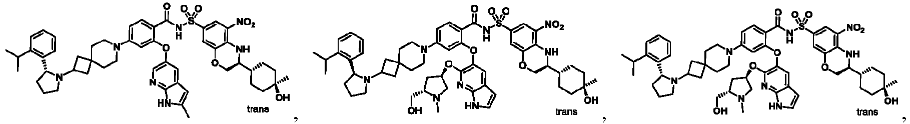 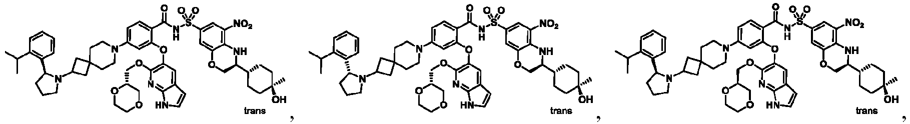 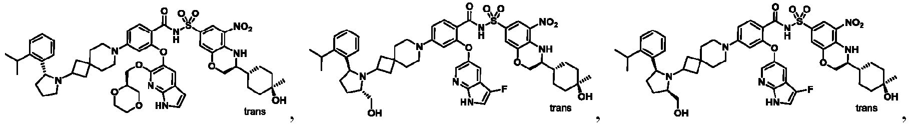 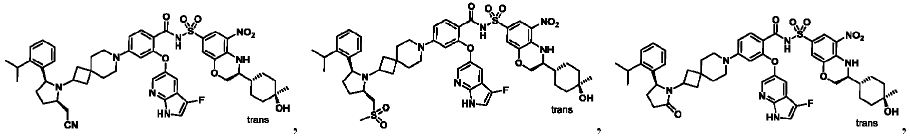 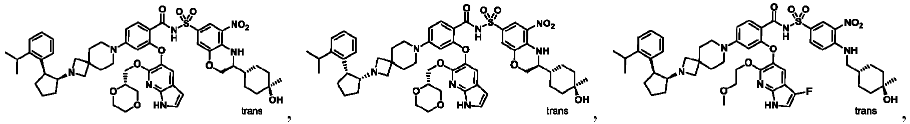 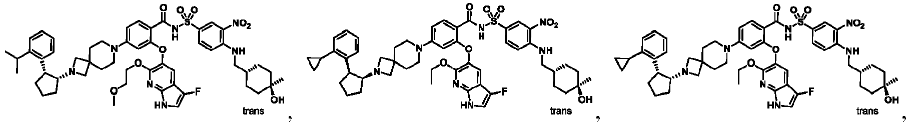 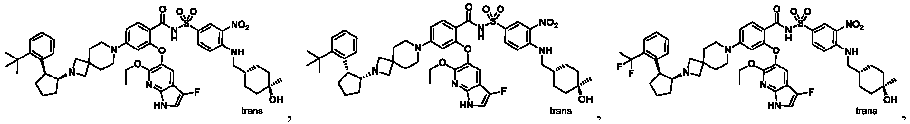 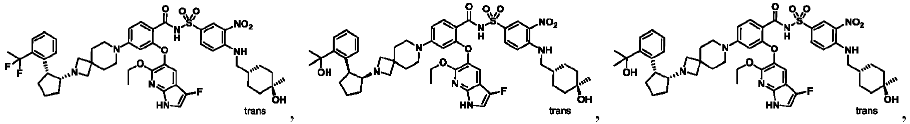 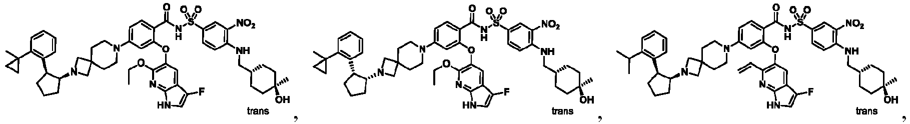 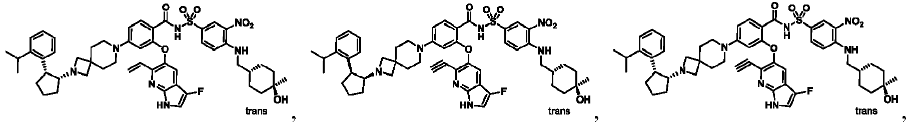 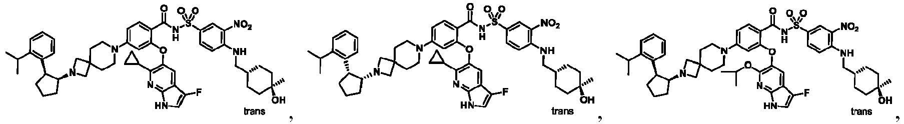 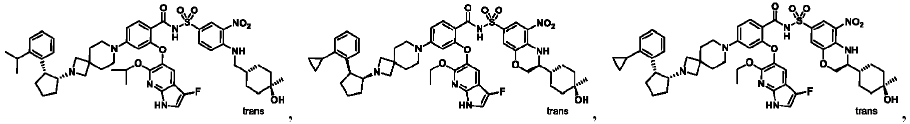 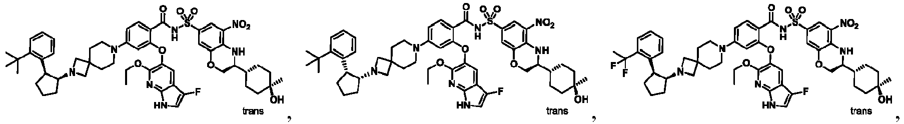 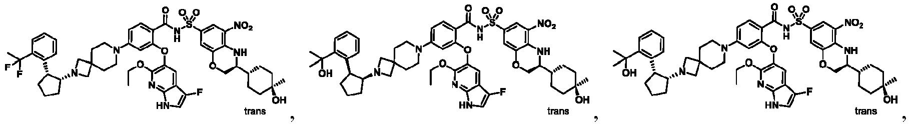 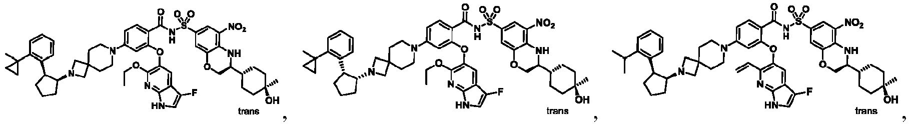 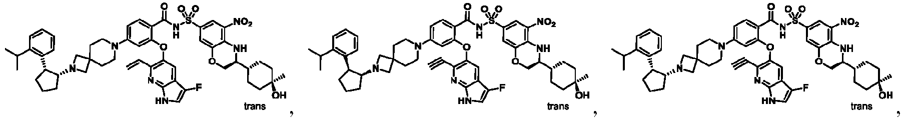 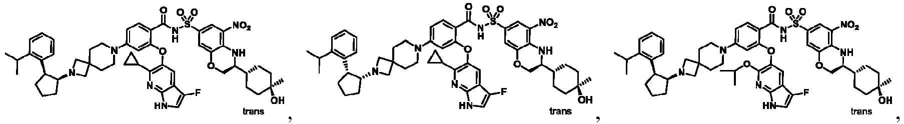 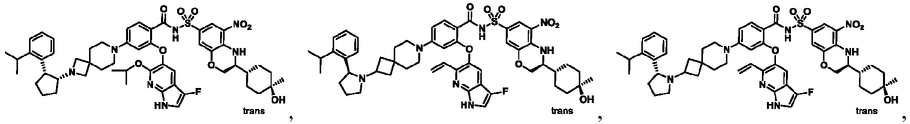 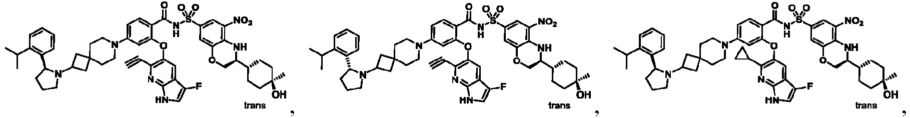 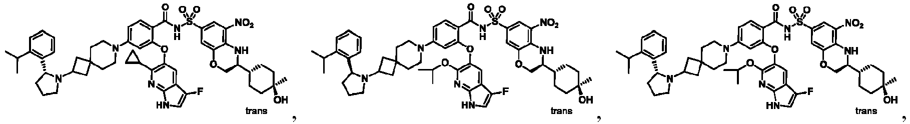 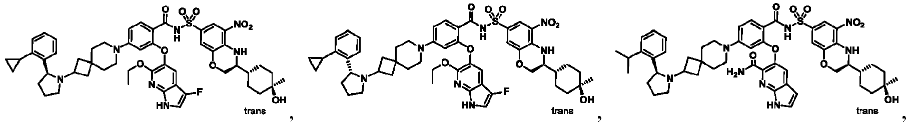 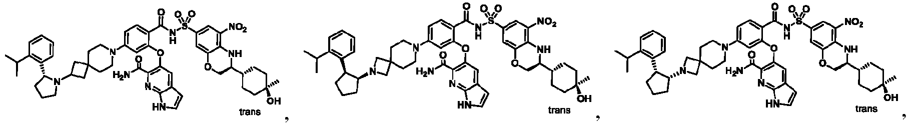 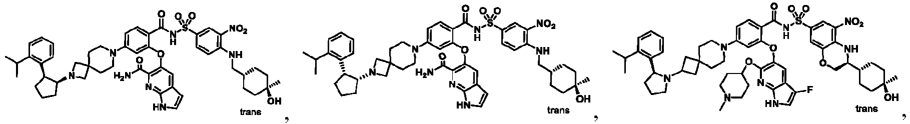 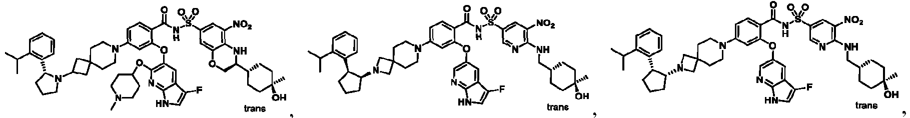 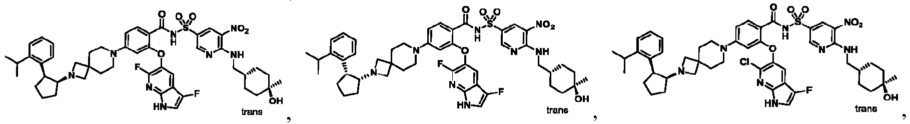 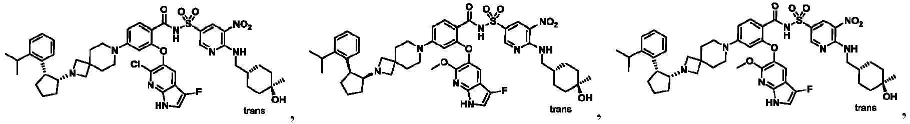 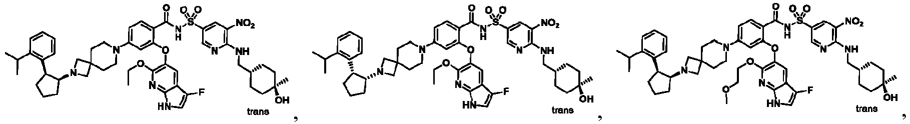 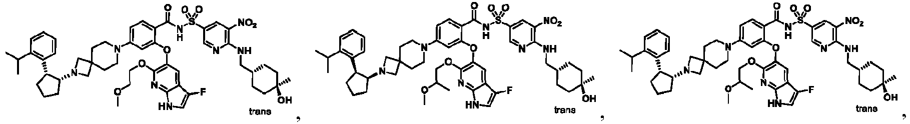 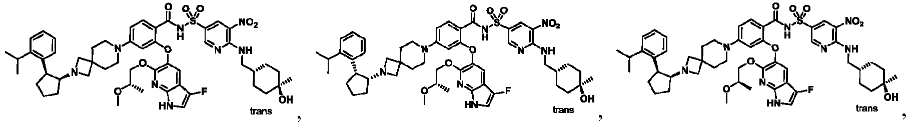 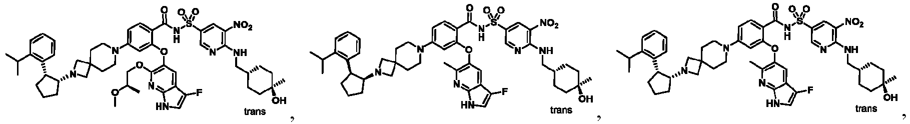 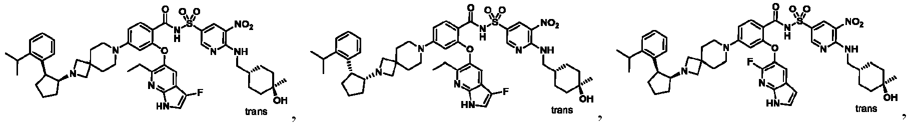 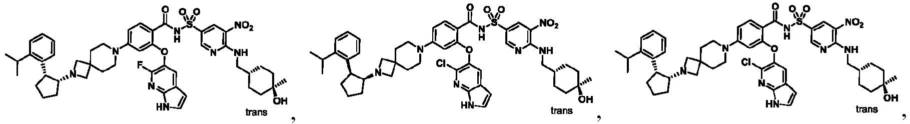 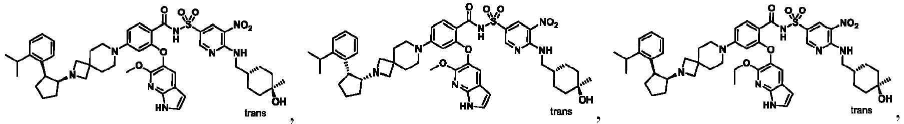 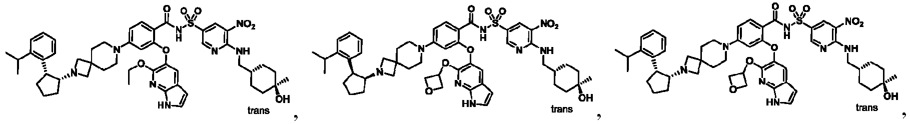 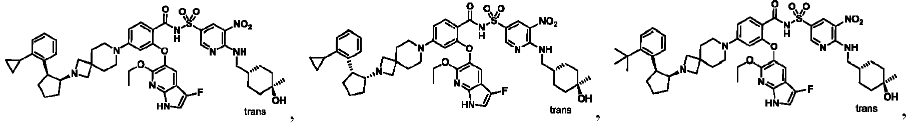 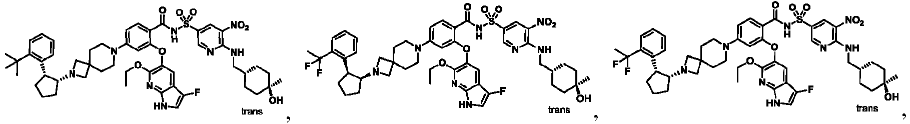 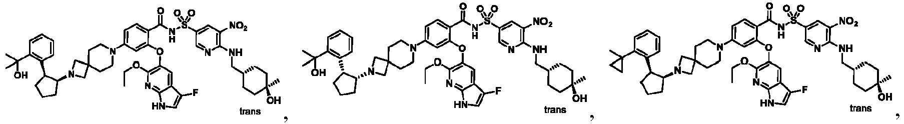 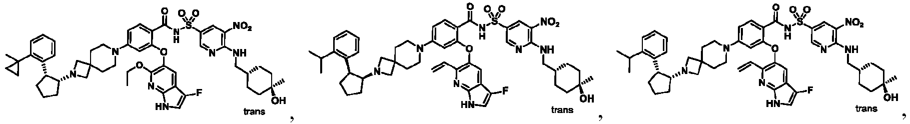 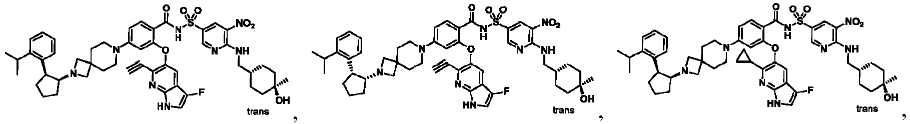 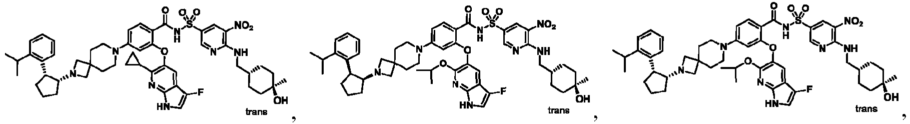 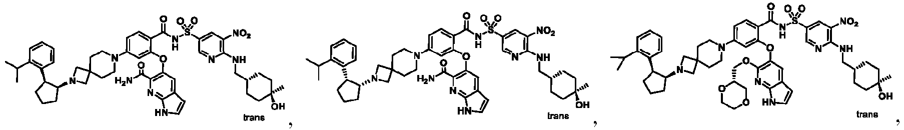 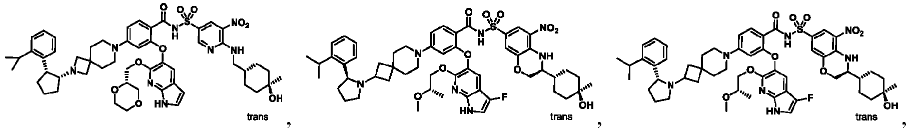 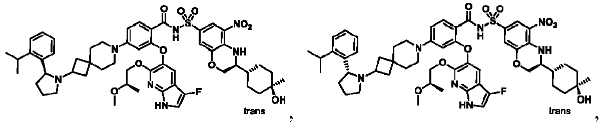 and pharmaceutically acceptable salts thereof.

In another Embodiment (78), the invention provides a compound selected from 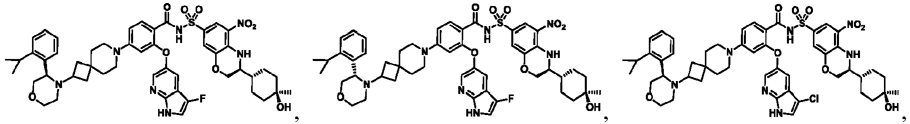 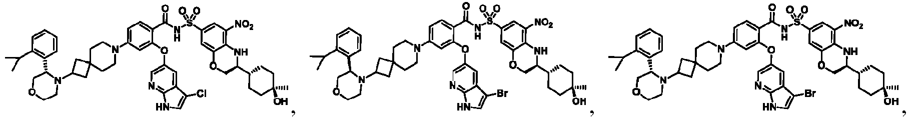 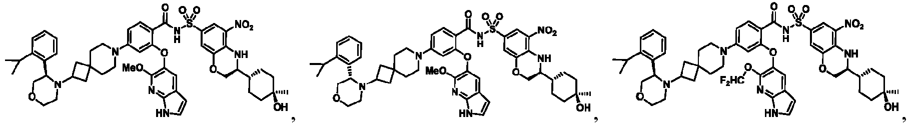 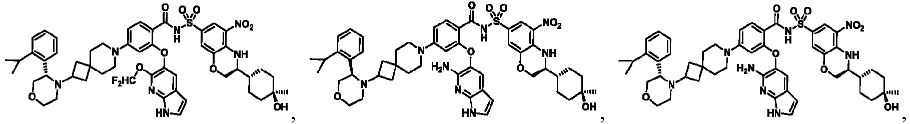 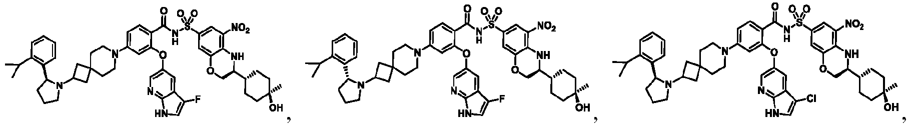 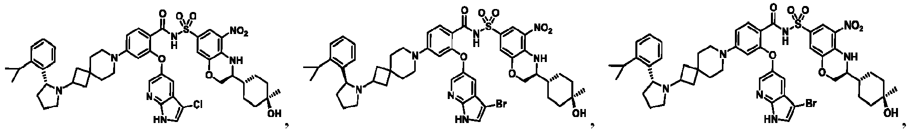 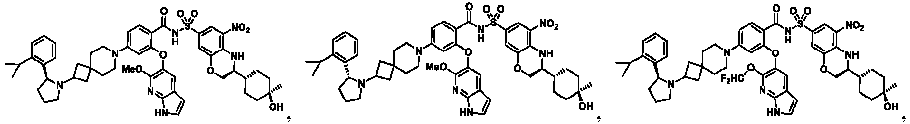 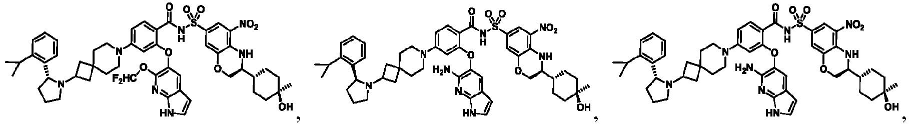 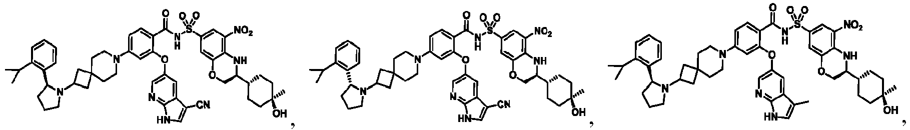 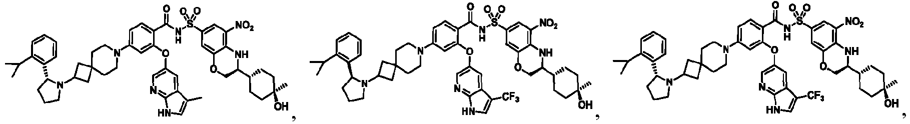 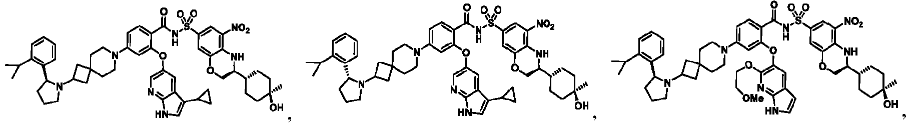 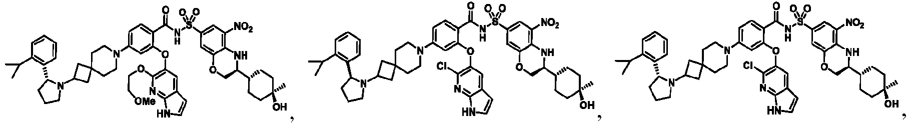 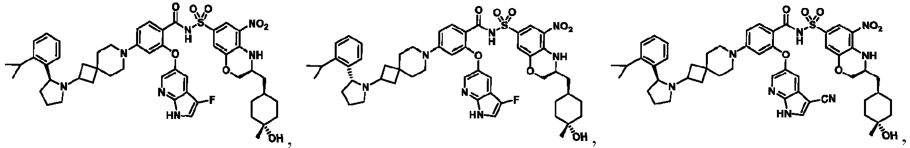 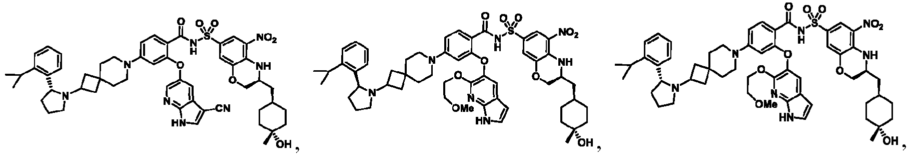 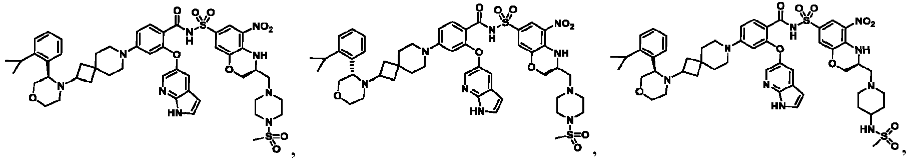 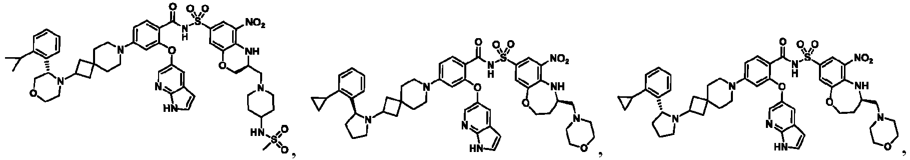 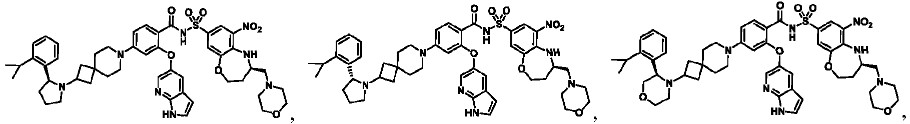 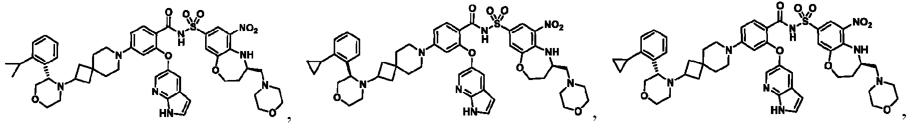 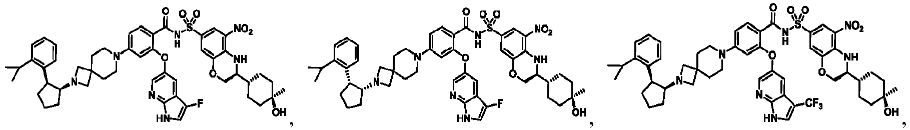 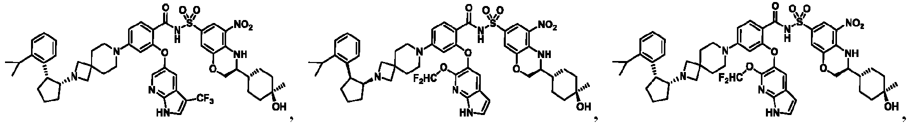 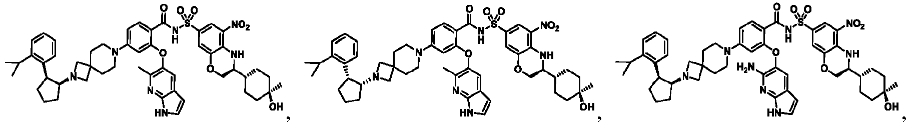 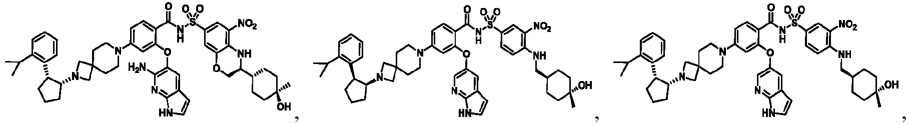 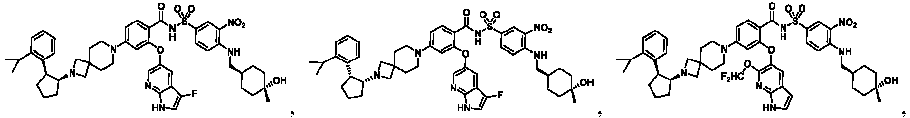 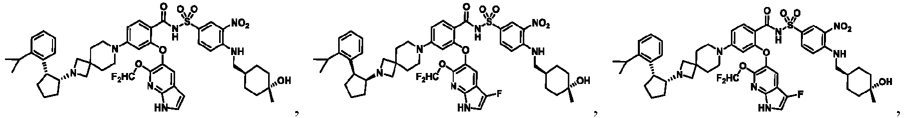 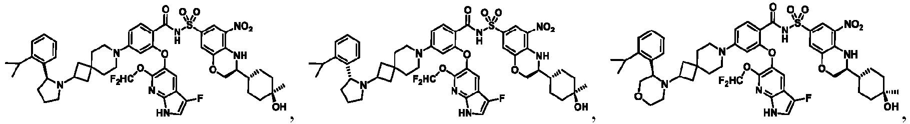 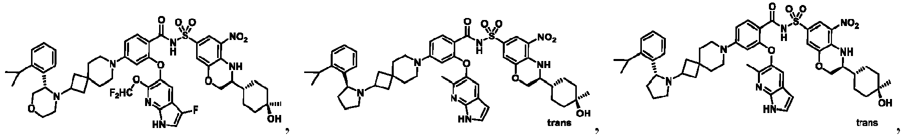 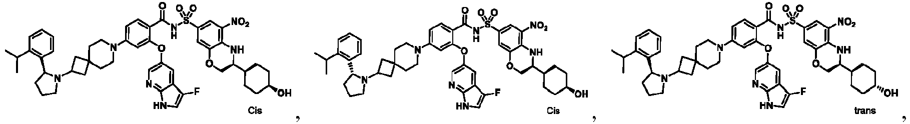 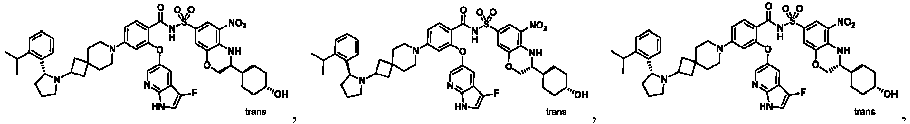 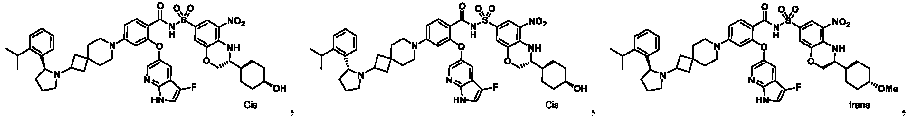 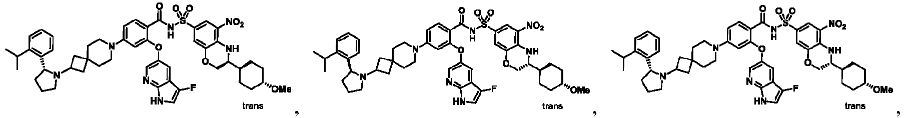 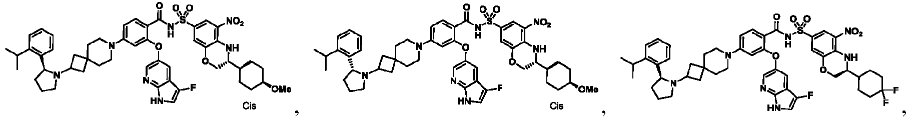 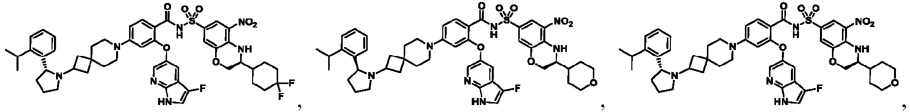 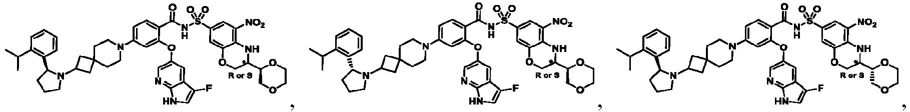 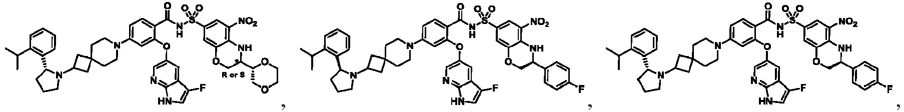 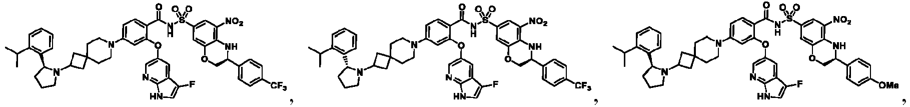 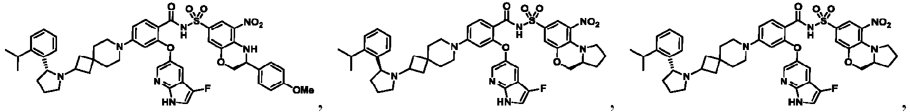 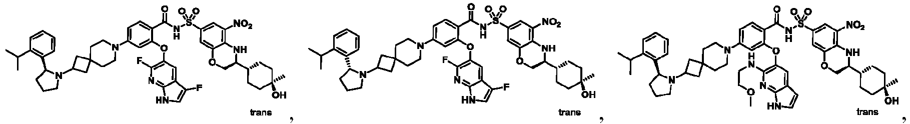 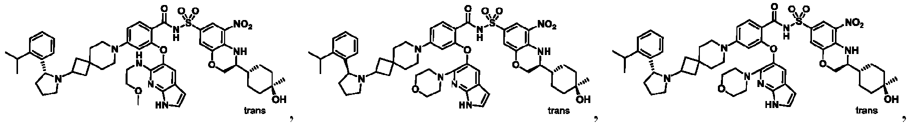 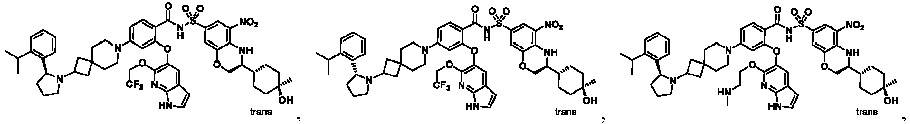 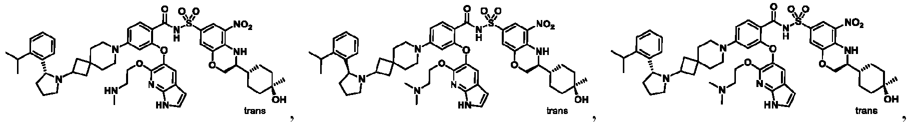 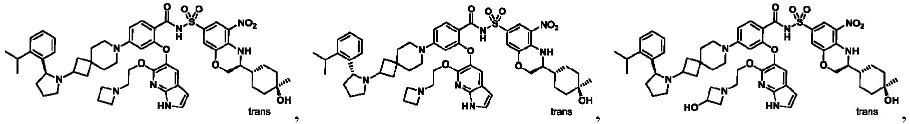 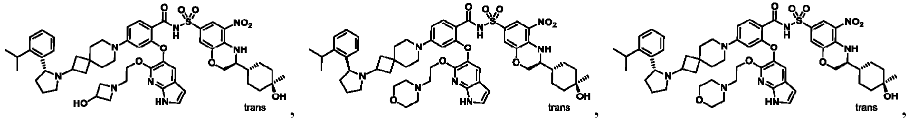 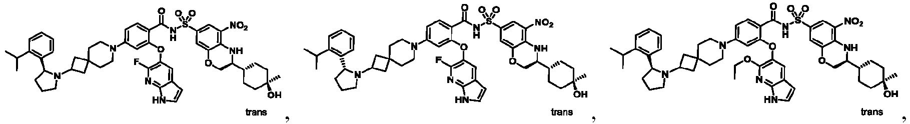 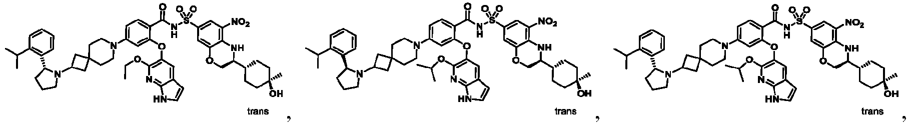 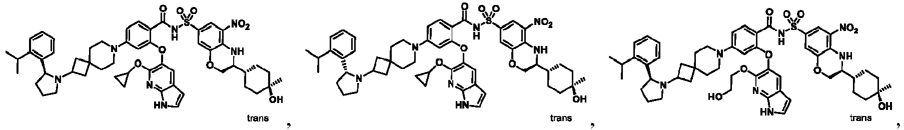 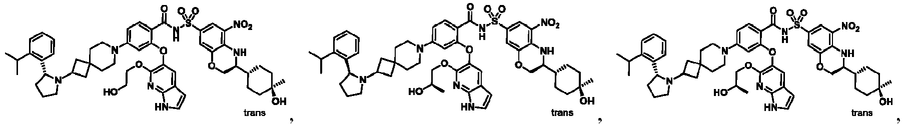 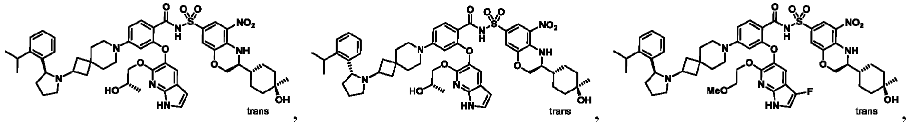 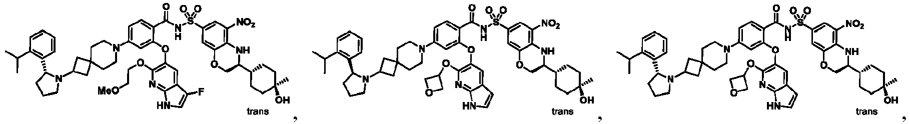 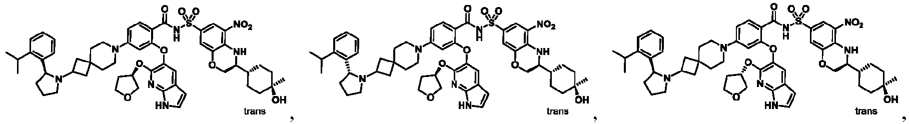 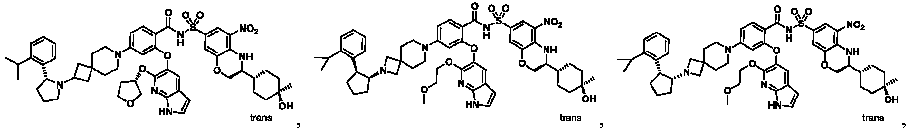 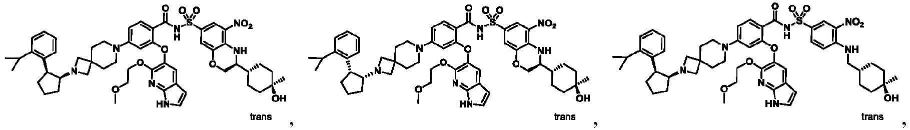 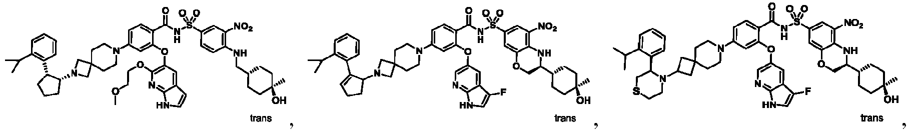 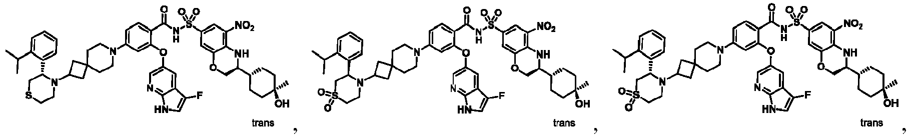 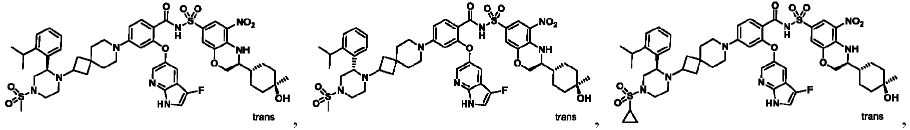 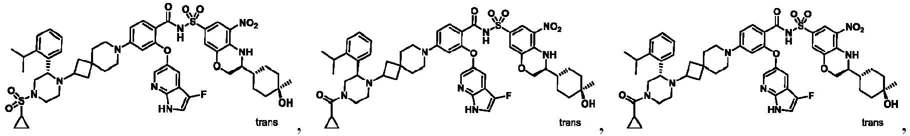 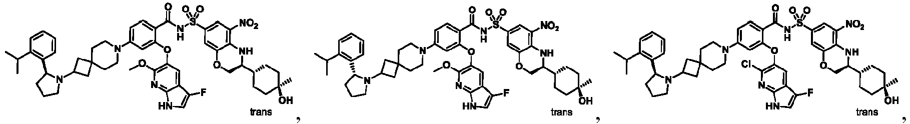 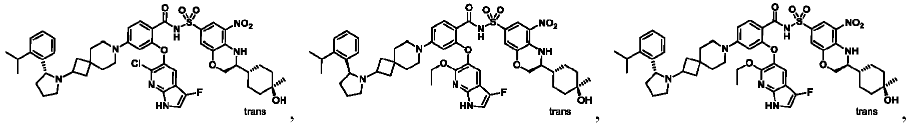 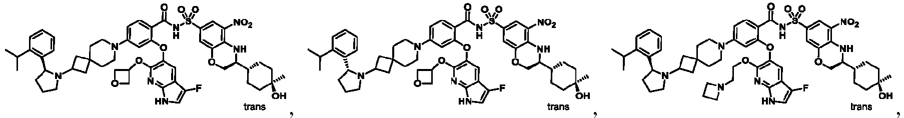 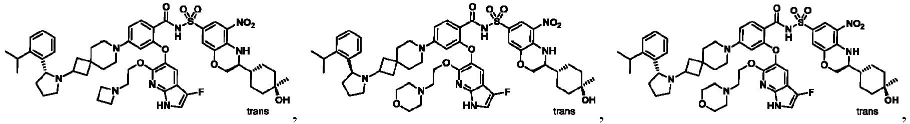 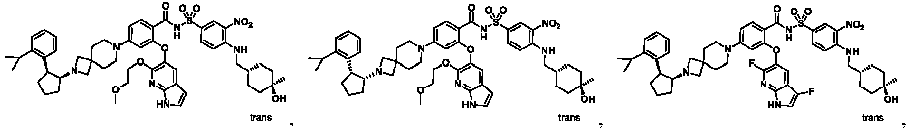 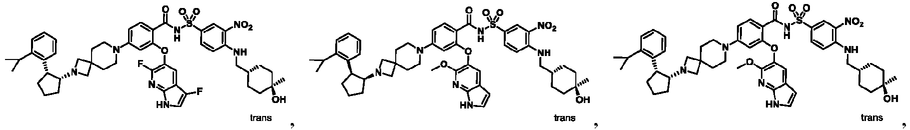 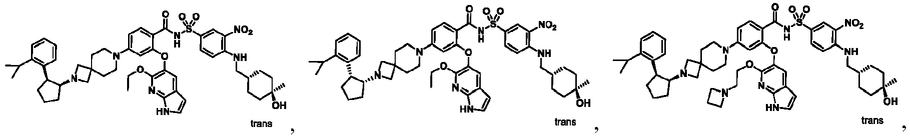 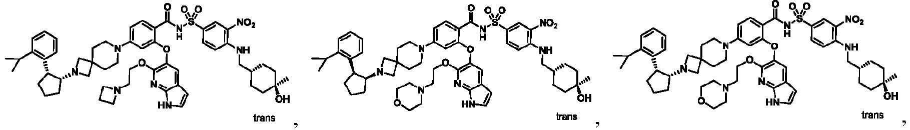 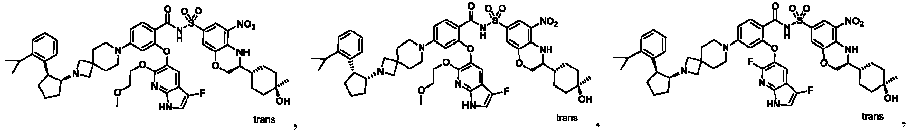 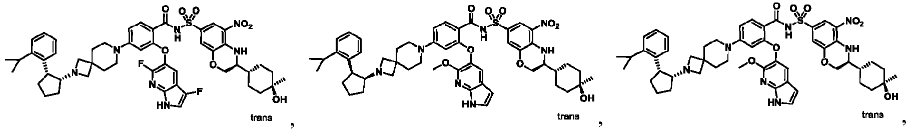 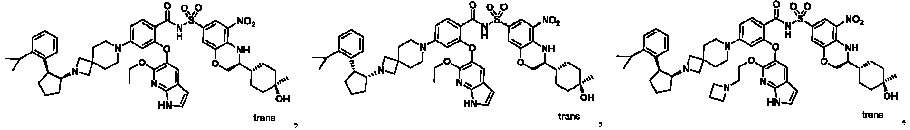 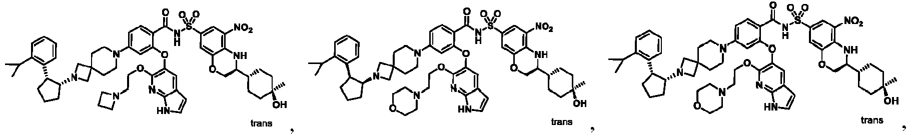 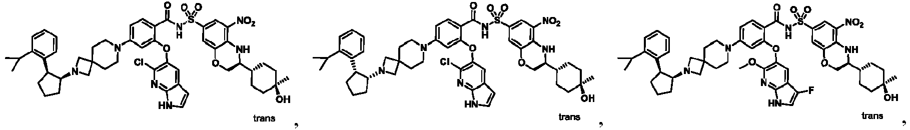 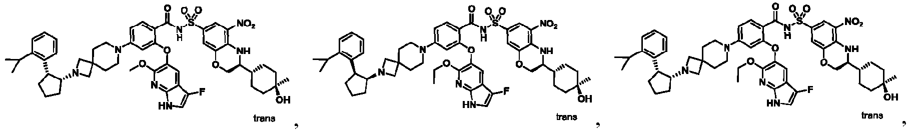 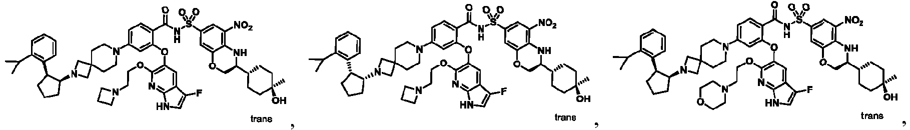 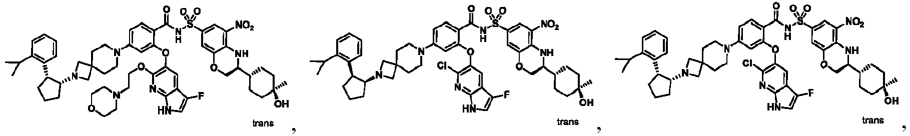 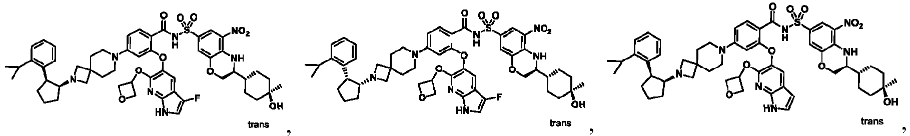 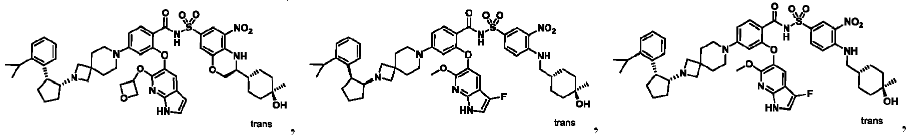 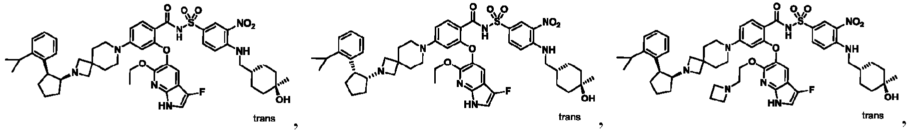 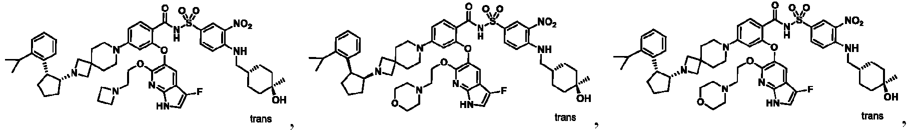 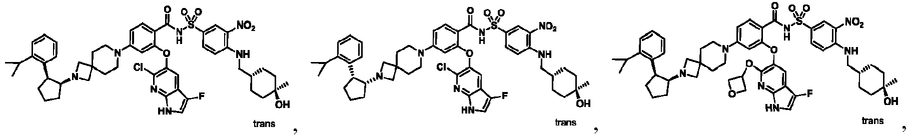 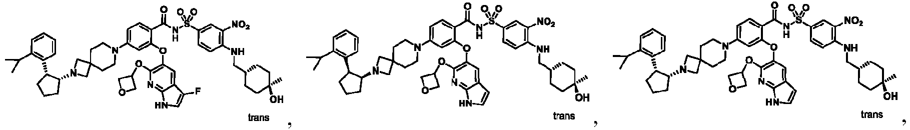 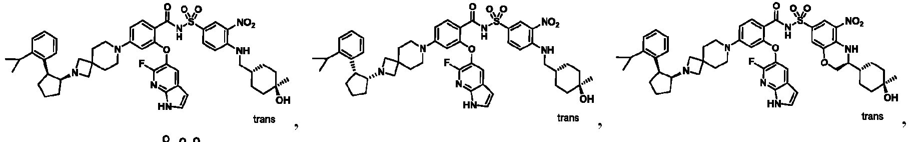 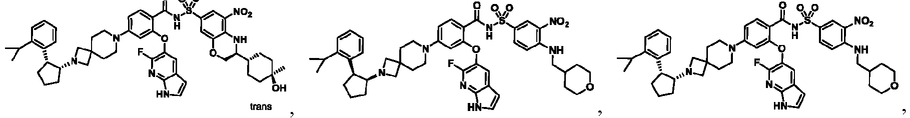 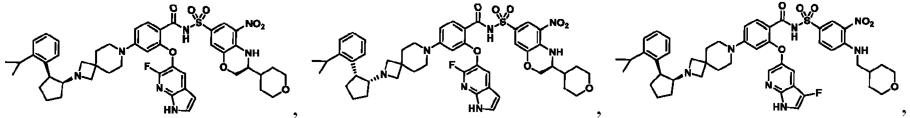 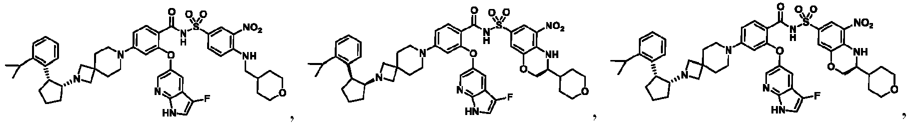 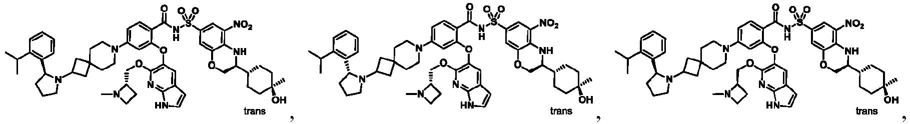 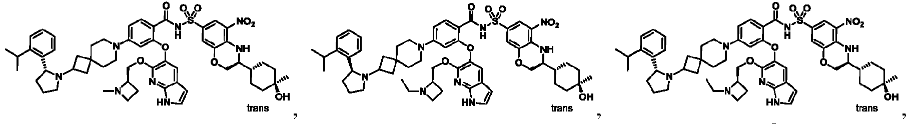 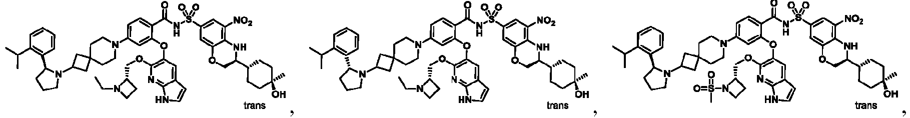 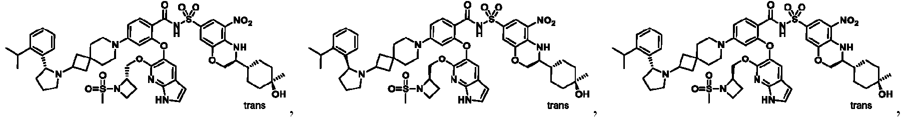 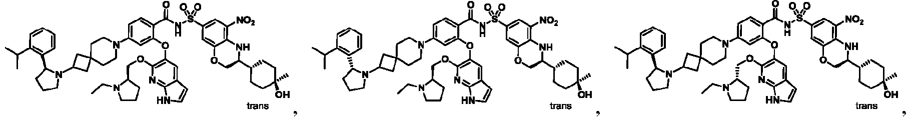 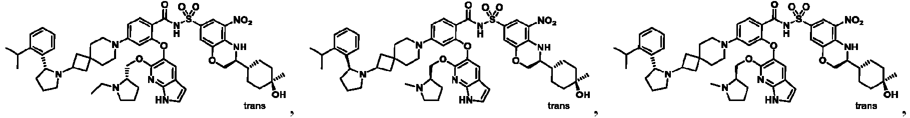 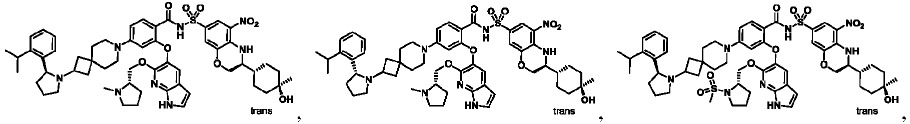 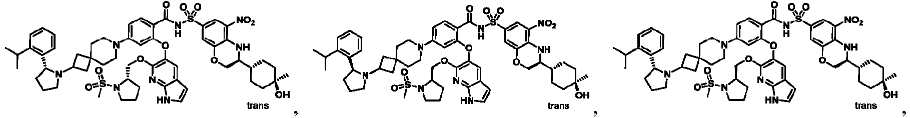 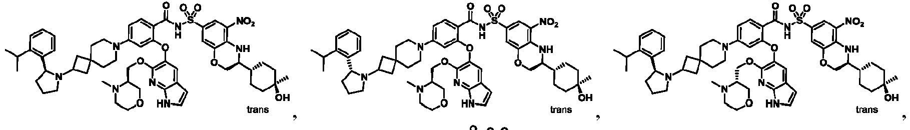 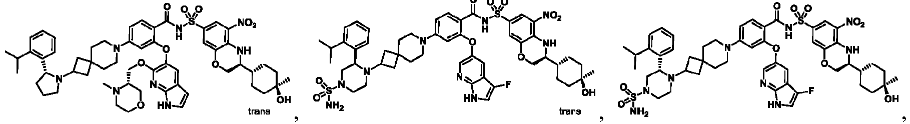 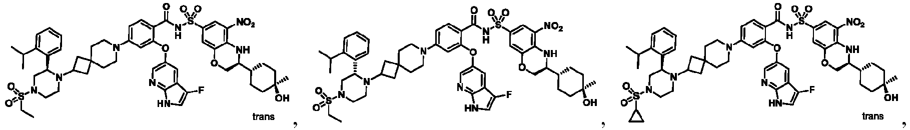 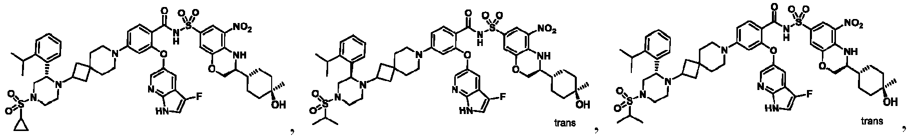 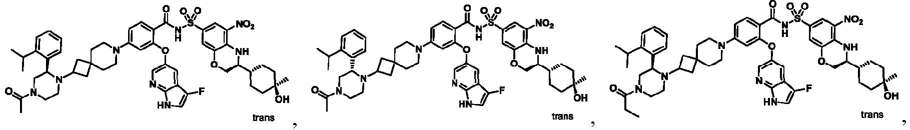 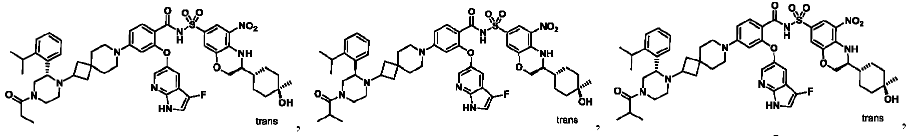 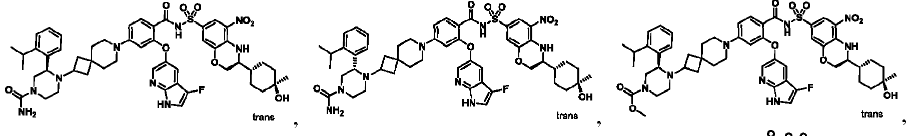 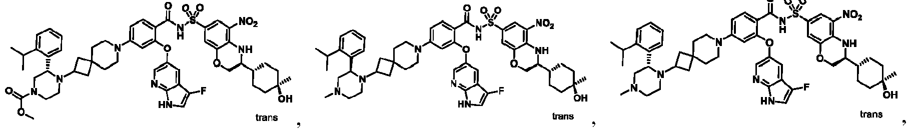 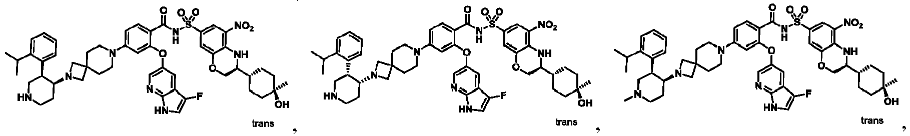 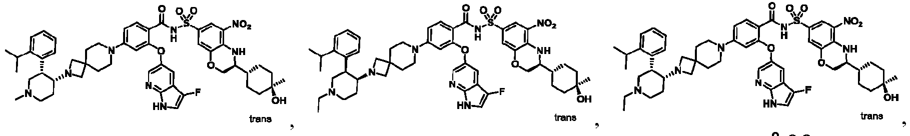 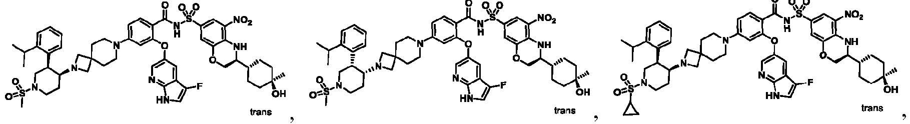 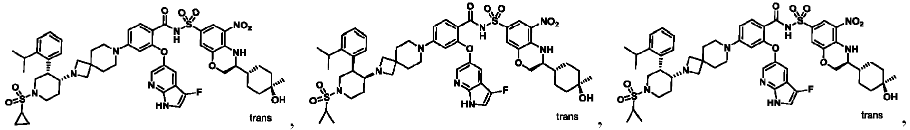 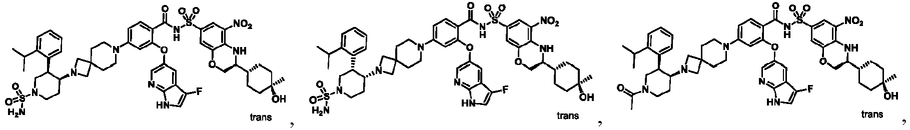 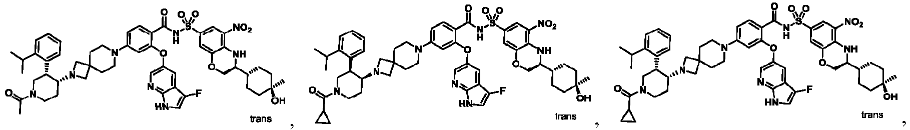 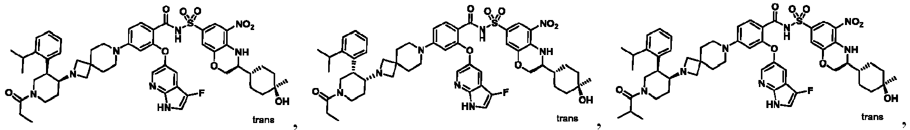 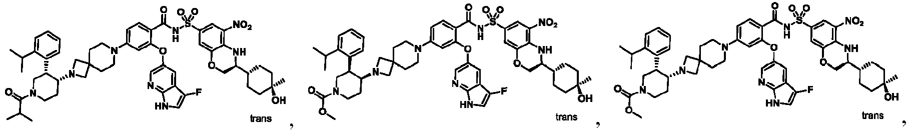 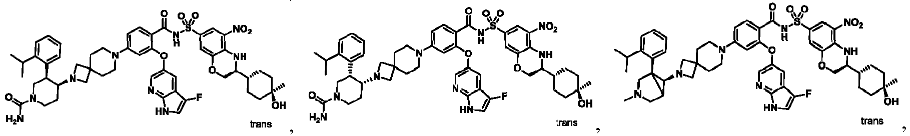 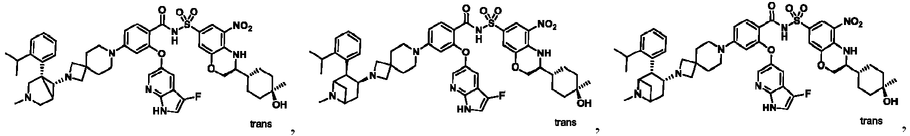 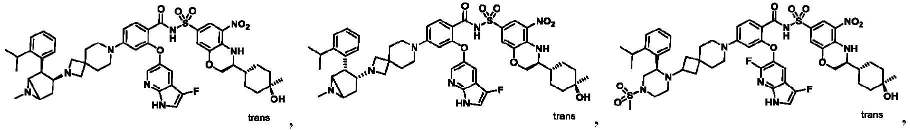 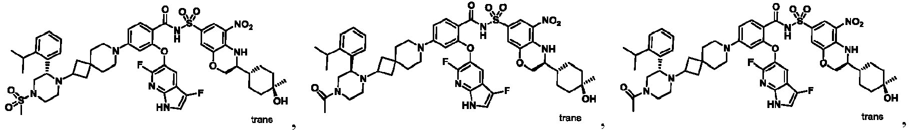 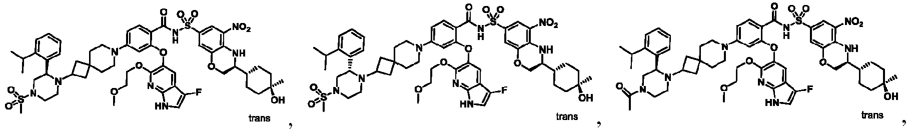 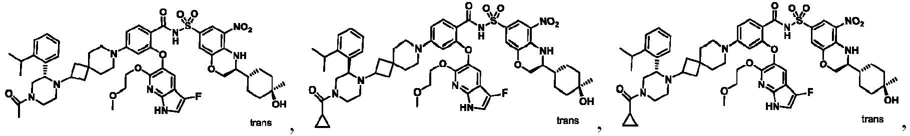 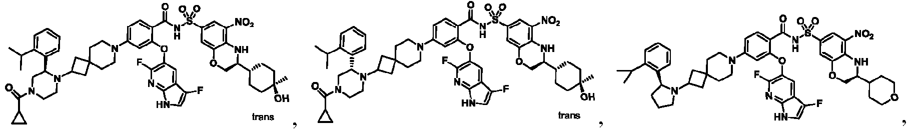 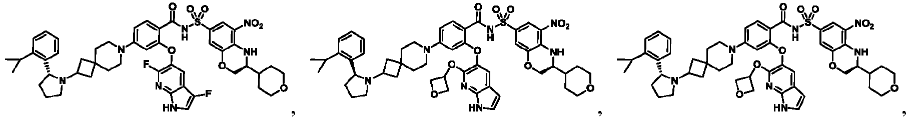 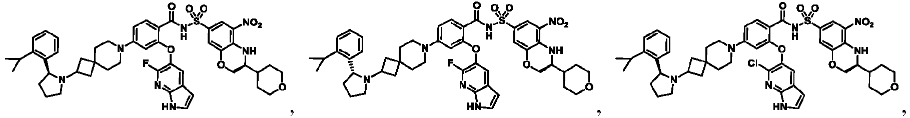 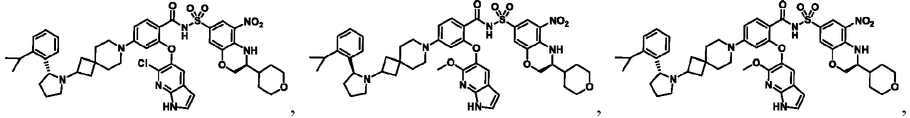 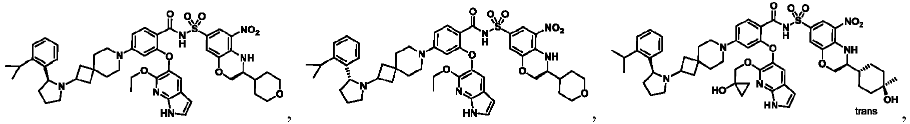 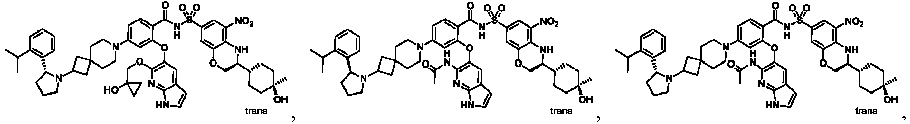 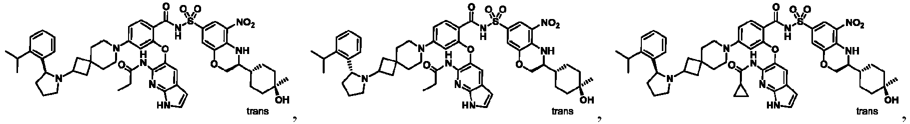 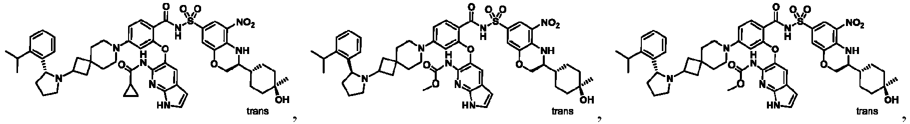 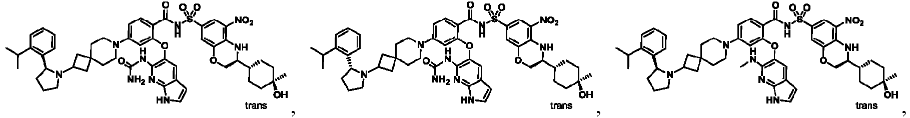 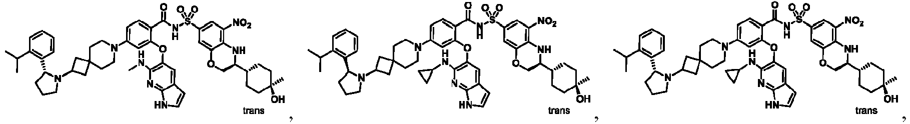 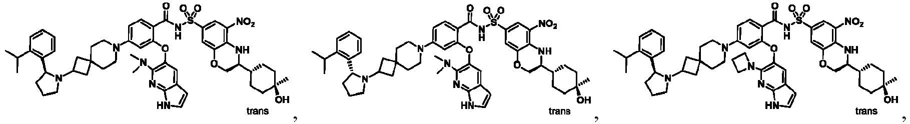 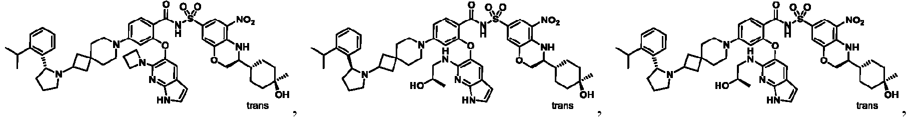 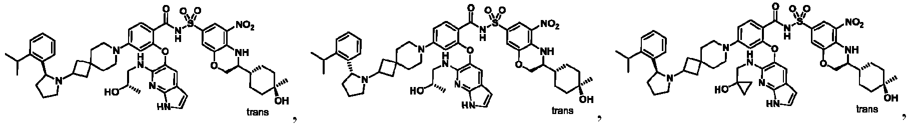 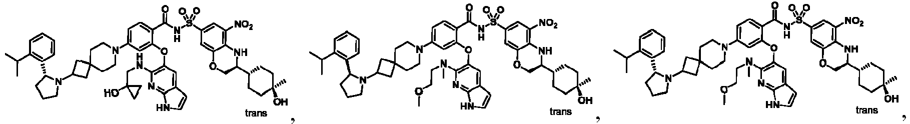 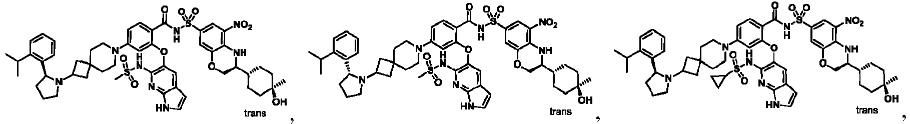 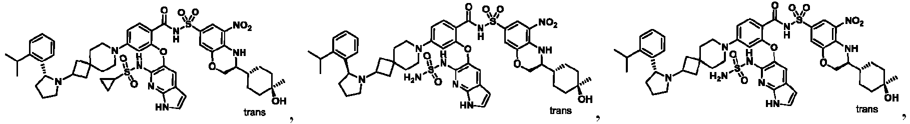 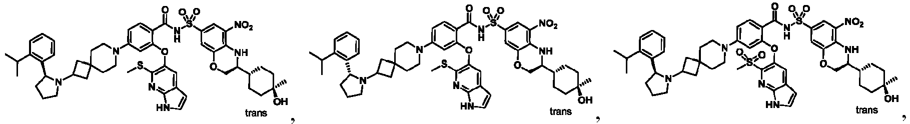 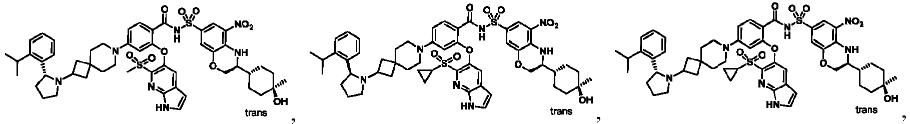 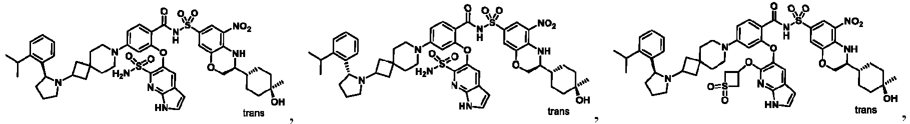 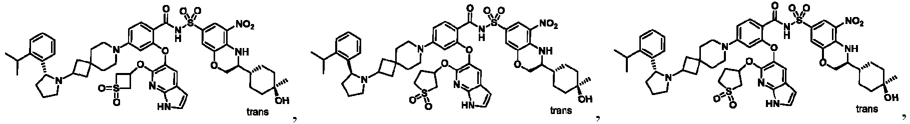 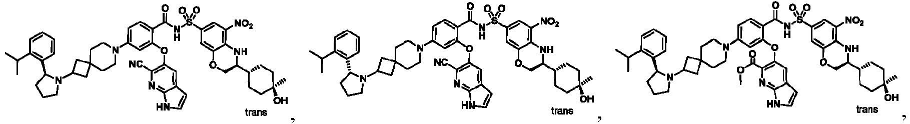 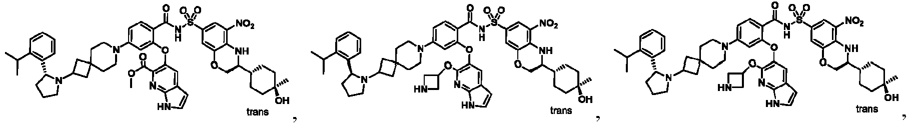 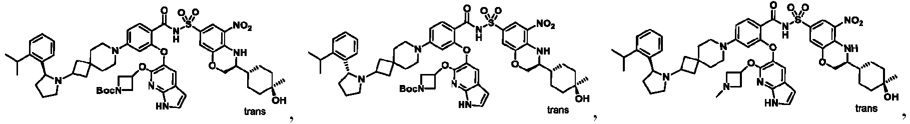 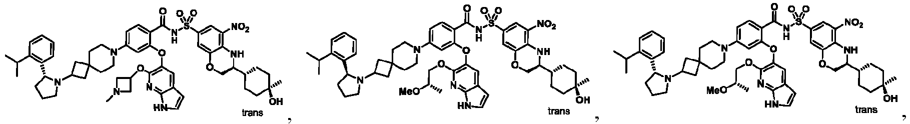 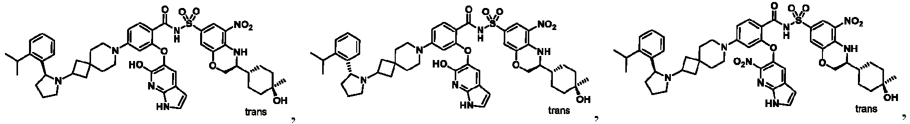 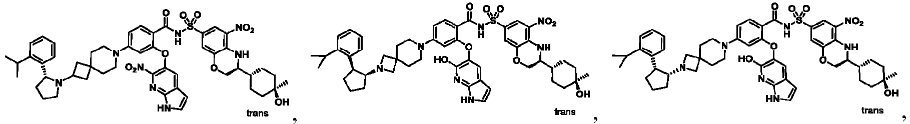 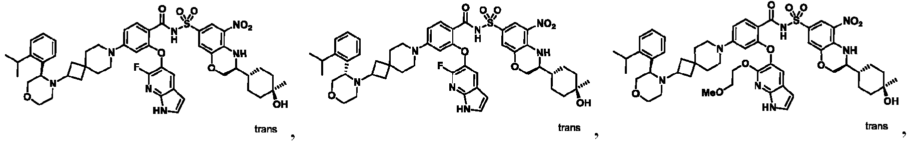 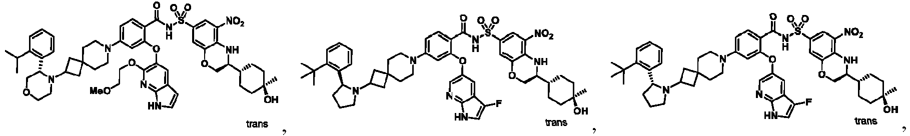 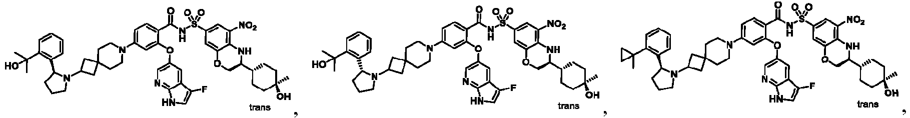 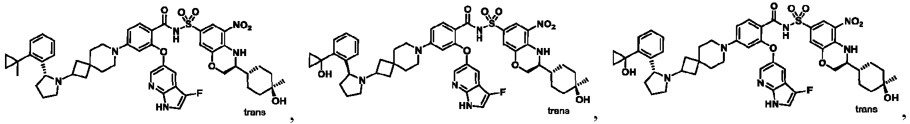 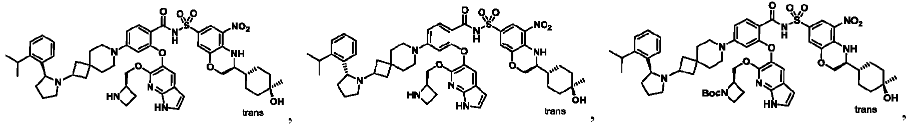 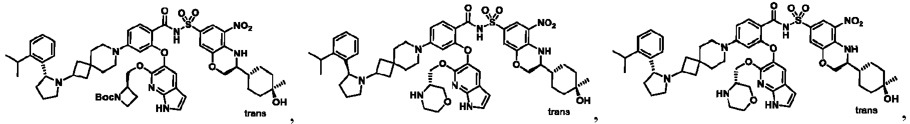 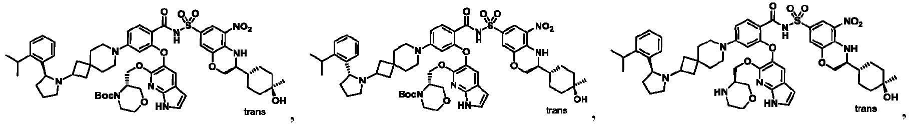 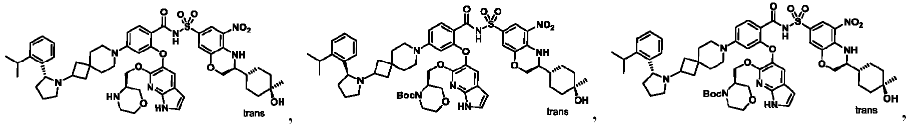 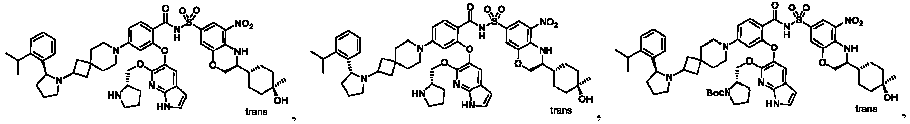 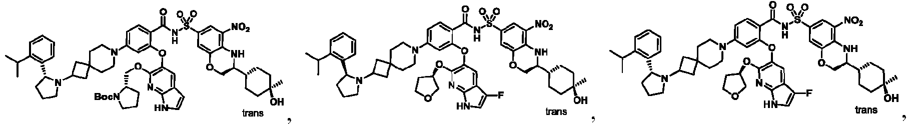 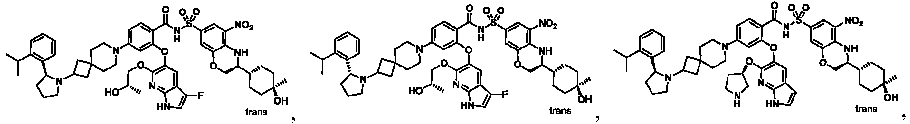 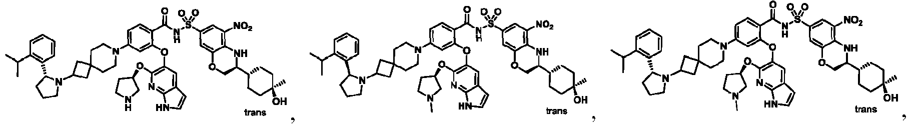 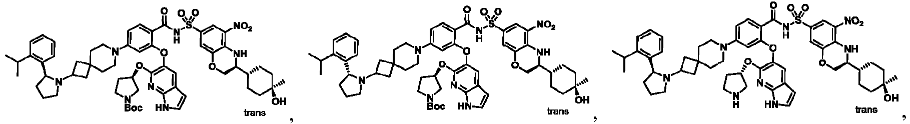 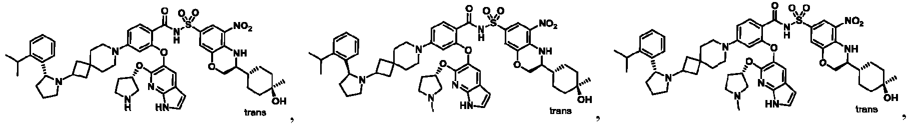 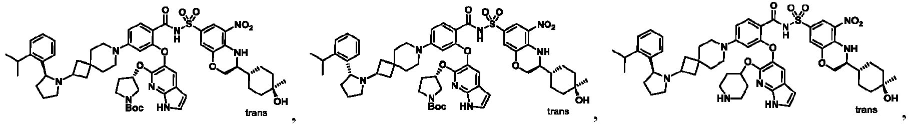 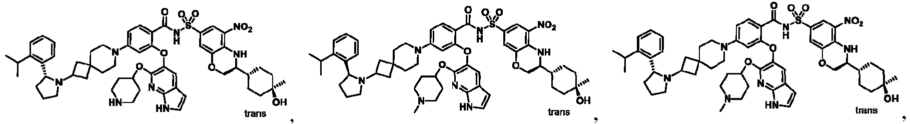 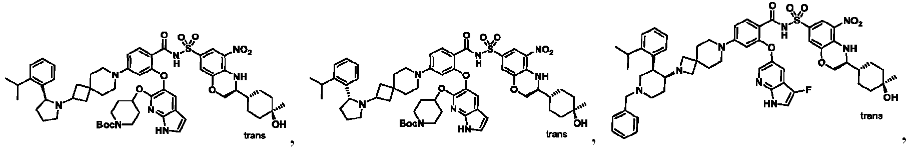 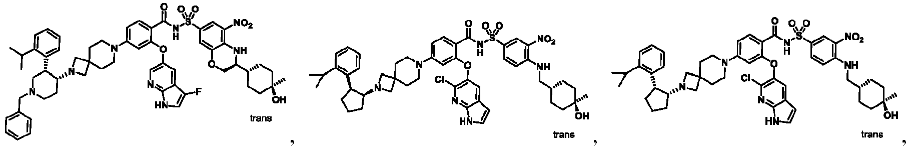 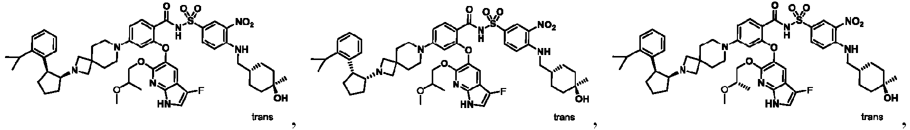 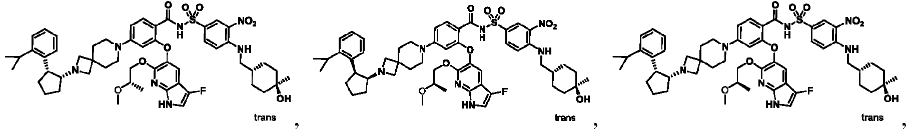 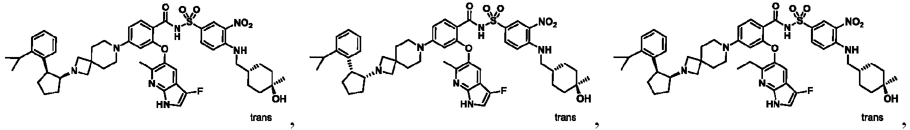 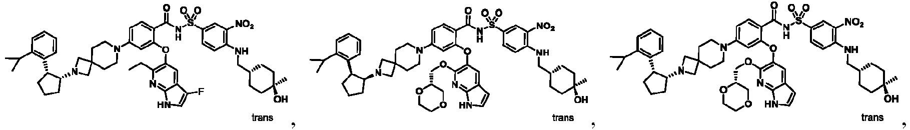 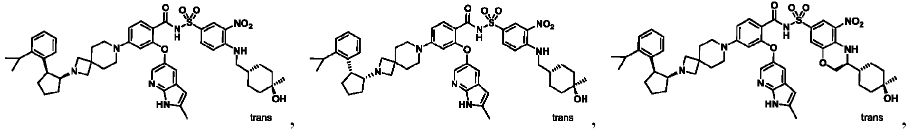 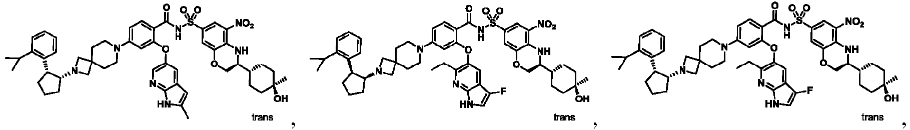 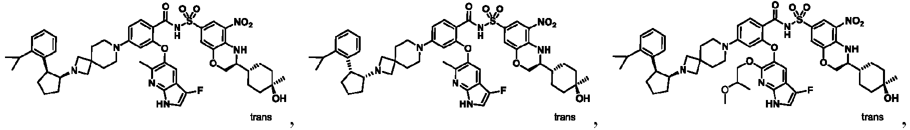 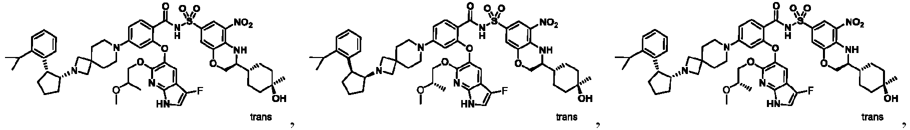 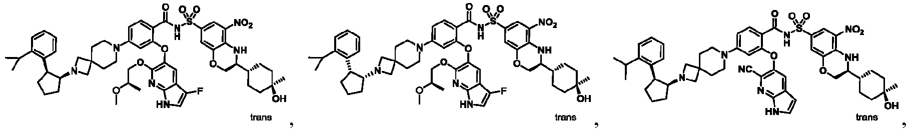 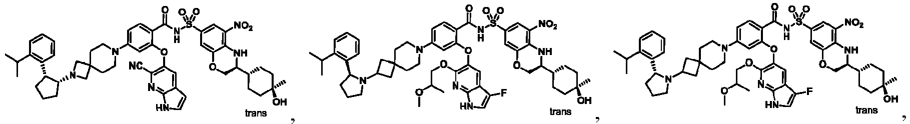 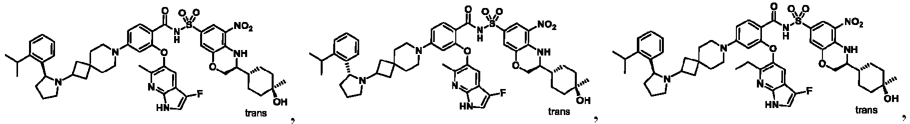 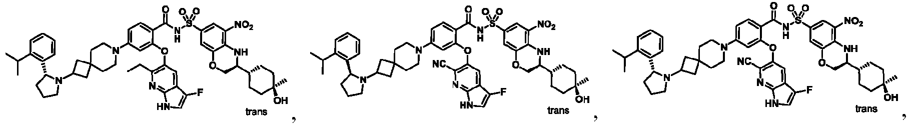 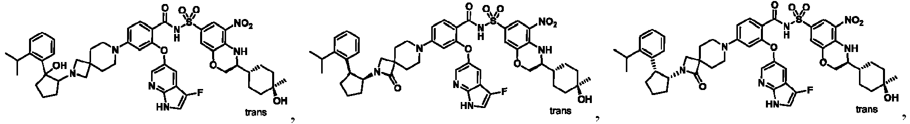 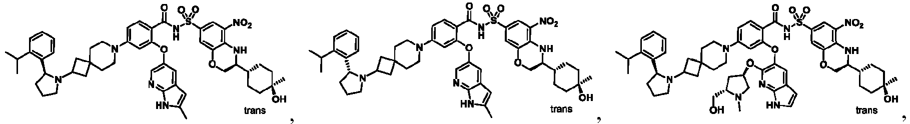 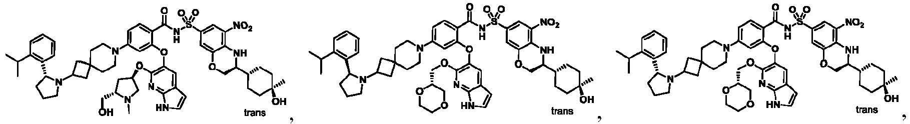 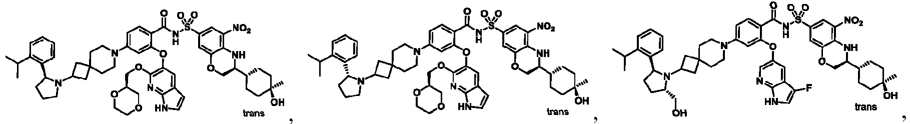 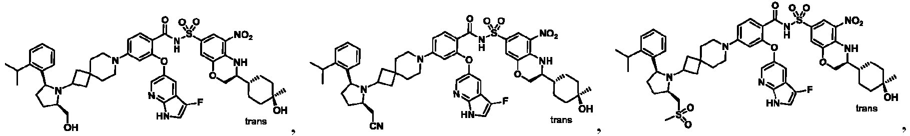 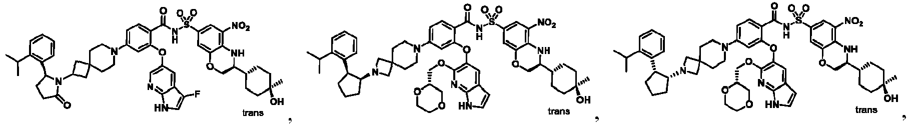 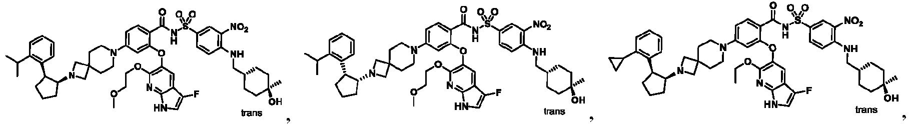 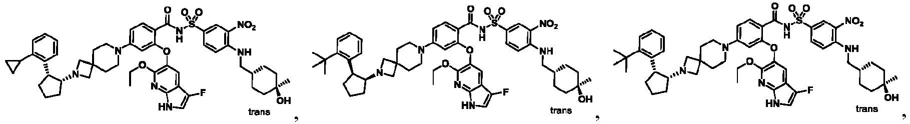 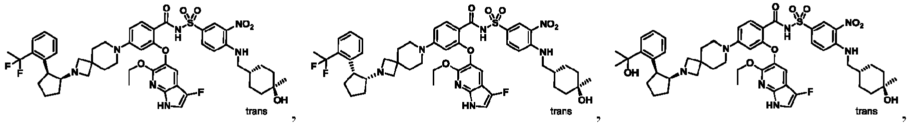 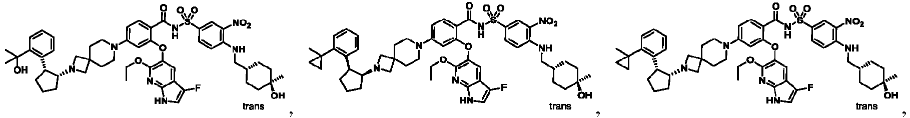 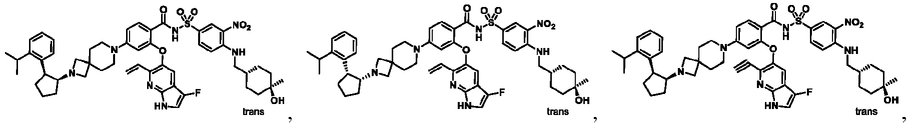 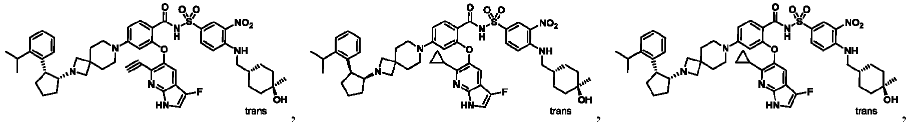 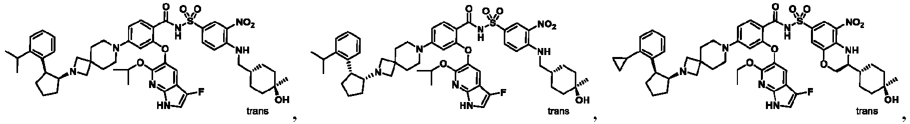 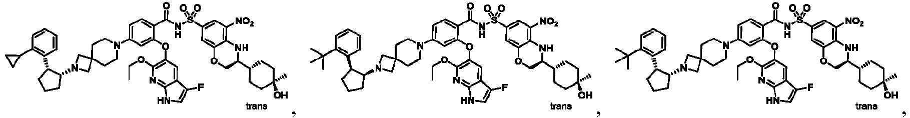 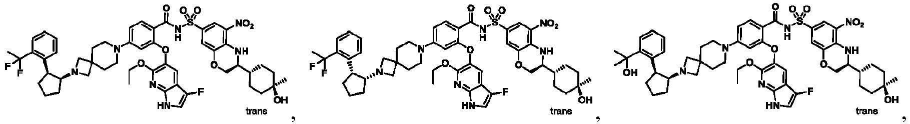 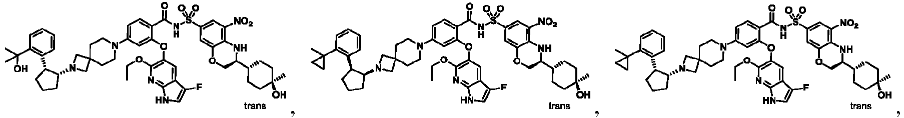 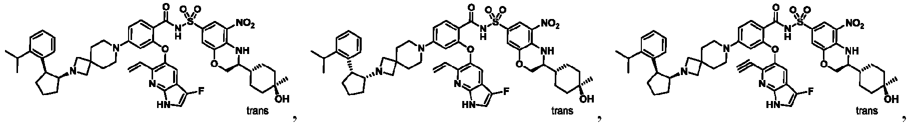 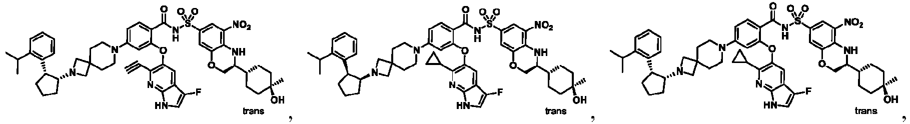 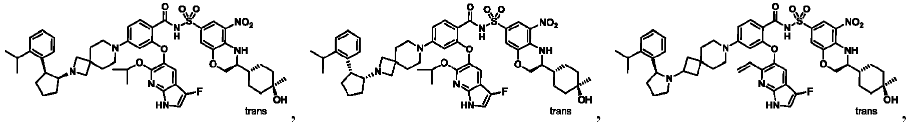 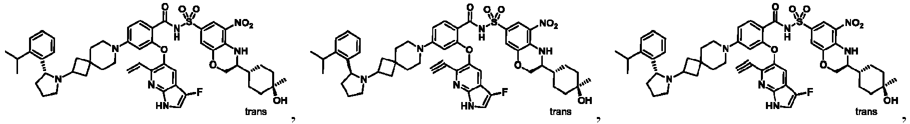 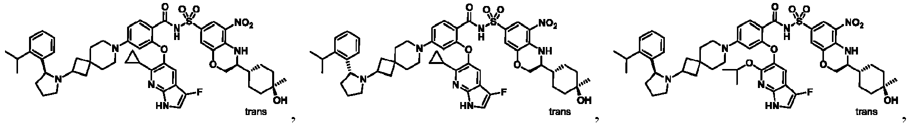 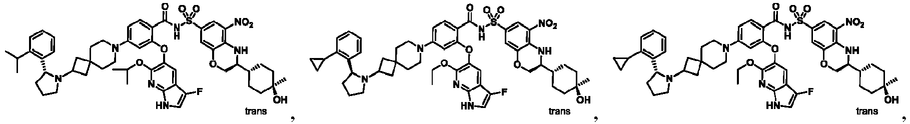 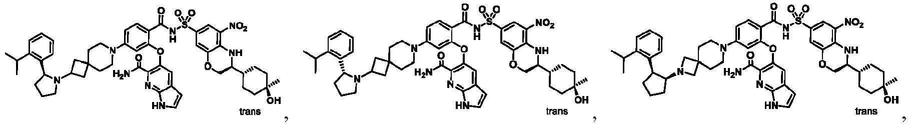 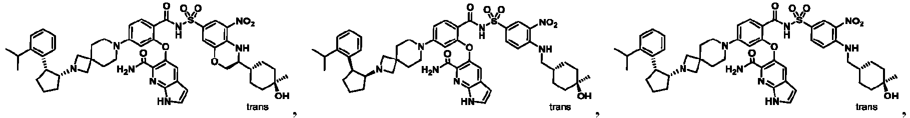 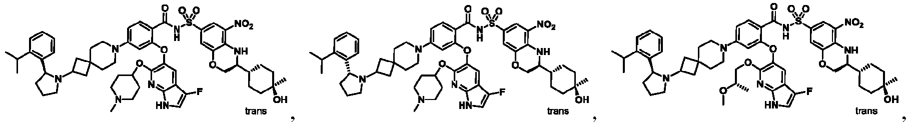 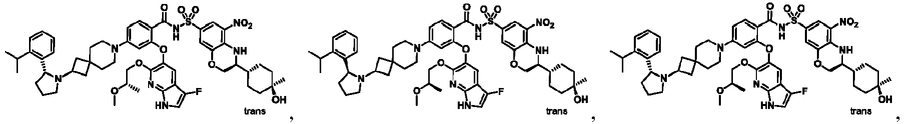 and pharmaceutically acceptable salts thereof.

In another Embodiment (79), the invention provides a pharmaceutical composition comprising a compound of any one of Embodiments (1)-(78) or a pharmaceutically acceptable salt thereof and at least one pharmaceutically acceptable carrier.

In another Embodiment (80), the invention provides a method of treating, ameliorating or preventing a condition, which responds to inhibition of Bcl-2, comprising administering to a subject in need of such treatment an effective amount of a compound of any one of Embodiments (1)-(78), or a pharmaceutically acceptable salt thereof, or a pharmaceutical composition thereof, and optionally in combination with a second therapeutic agent.

In another Embodiment (81), the invention provides a use of a compound of any one of Embodiments (1)-(78) or a pharmaceutically acceptable salt thereof in the preparation of a medicament for treating a cell-proliferative disorder or autoimmune disease.

In an In another Embodiment (82), the invention provides the use of Embodiment (81), wherein the cell-proliferative disorder is includes but not limited to, breast cancer, ovarian cancer, bladder cancer, uterine cancer, prostate cancer, testicular cancer, lung cancer (including NSCLC, SCLC, squamous cell carcinoma or adenocarcinoma), esophageal cancer, head and neck cancer, colorectal cancer, kidney cancer (including RCC), liver cancer (including HCC), pancreatic cancer, stomach (i.e., gastric) cancer, thyroid cancer, chronic lymphocytic leukemia (CLL), lymphoblastic leukemia, follicular lymphoma, lymphoid malignancies of T-cell or B-cell origin, melanoma, myelogenous leukemia and myeloma.

In another Embodiment (83), the invention provides the use of Embodiment (81), wherein the autoimmune disease is includes but not limited to, allergy, Alzheimer's disease, acute disseminated encephalomyelitis, Addison's disease, ankylosing spondylitis, antiphospholipid antibody syndrome, asthma, atherosclerosis, autoimmune hemolytic anemia, autoimmune hemolytic and thrombocytopenic states, autoimmune hepatitis, autoimmune inner ear disease, bullous pemphigoid, coeliac disease, chagas disease, chronic obstructive pulmonary disease, chronic Idiopathic thrombocytopenic purpura (ITP), churg-strauss syndrome, Crohn's disease, dermatomyositis, diabetes mellitus type 1, endometriosis, Goodpasture's syndrome(and associated glomerulonephritis and pulmonary hemorrhage), graves' disease, guillainbarre syndrome, hashimoto's disease, hidradenitis suppurativa, idiopathic thrombocytopenic purpura, interstitial cystitis, irritable bowel syndrome, lupus erythematosus, morphea, multiple sclerosis, myasthenia gravis, narcolepsy, neuromyotonia, Parkinson's disease, pemphigus vulgaris, pernicious anaemia, polymyositis, primary biliary cirrhosis, psoriasis, psoriatic arthritis, rheumatoid arthritis, schizophrenia, septic shock, scleroderma, Sjogren's disease, systemic lupus erythematosus (and associated glomerulonephritis), temporal arteritis, tissue graft rejection and hyperacute rejection of transplanted organs, vasculitis (ANCA-associated and other vasculitides), vitiligo, and wegener's granulomatosis.

Some embodiments can also be described as follows:

In another Embodiment <1>, the invention provides a compound of formula <I'> 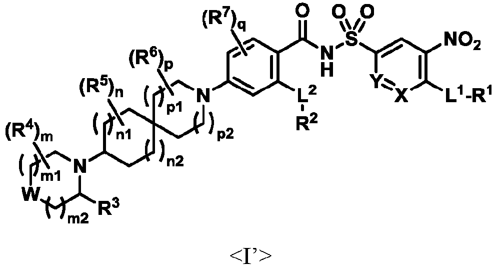 or a pharmaceutically acceptable salt thereof, wherein:
X is selected from N and CR⁸, Y is selected from N and CR⁹, wherein at least one of X and Y is N;
W is selected from -CR¹⁰-, -NR¹¹-, -O- and -S(O)ᵣ-;
L¹ and L² are independently selected from a bond, -(CR^{C0}R ^{D0}))ᵤ-, -(CR^{C0}R^{D0})ᵤO(CR^{C0}R^{D0})ₜ-, -(CR^{C0}R^{D0})ᵤNR^{A0}(CR^{C0}R^{D0})ₜ- and -(CR^{C0}R^{D0})ᵤS(O)ᵣ(CR^{C0}R^{D0})ₜ-;
R¹ is selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A1}R^{B1} -OR^{A1}, -C(O)R^{A1}, -C(=NR^{E1})R^{A1} -C(=N-OR^{B1})R^{A1}, -C(O)OR^{A1}, -OC(O)R^{A1}, -C(O)NR^{A1}R^{B1}, -NR^{A1}C(O)R^{B1}, -C(=NR^{E1})NR^{A1}R^{B1}, -NR^{A1}C(=NR^{E1})R^{B1}, -OC(O)NR^{A1}R^{B1}, -NR^{A1}C(O)OR^{B1}, -NR^{A1}C(O)NR^{A1}R^{B1}, -NR^{A1}C(S)NR^{A1}R^{B1}, -NR^{A1}C(=NR^{E1})NR^{A1}R^{B1}, -S(O)ᵣR^{A1}, -S(O)(=NR^{E1})R^{B1}, -N=S(O)R^{A1}R^{B1}, -S(O)₂OR^{A1}, -OS(O)₂R^{A1}, -NR^{A1}S(O)ᵣR^{B1}, -NR^{A1}S(O)(=NR^{E1})R^{B1}, -S(O)ᵣNR^{A1}R^{B1}, -S(O)(=NR^{E1})NR^{A1}R^{B1}, -NR^{A1}S(O)₂NR^{A1}R^{B1}, -NR^{A1}S(O)(=NR^{E1})NR^{A1}R^{B1}, -P(O)R^{A1}R^{B1} and -P(O)(OR^{A1})(OR^{B1}), wherein alkyl, alkenyl, alkynyl, cycloalkyl, heterocyclyl, aryl and heteroaryl are each unsubstituted or substituted with at least one substituent, independently selected from R^{X};
R² and R³ are independently selected from aryl, heteroaryl and heterocyclyl, wherein aryl, heteroaryl and heterocyclyl are each unsubstituted or substituted with at least one substituent, independently selected from R^{X};
each R⁴ is independently selected from hydrogen, halogen, C₁-₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A4}R^{B4}, -OR^{A4} -C(O)R^{A4}, -C(=NR^{E4})R^{A4} -C(=N-OR^{B4})R^{A4}, -C(O)OR^{A4}, -OC(O)R^{A4}, -C(O)NR^{A4}R^{B4} -NR^{A4}C(O)R^{B4}, -C(=NR^{E4})NR^{A4}R^{B4}, -NR^{A4}C(=NR^{E4})R^{B4}, -OC(O)NR^{A4}R^{B4}, -NR^{A4}C(O)OR^{B4}, -NR^{A4}C(O)NR^{A4} R^{B4}, -NR^{A4}C(S)NR^{A4}R^{B4}, -NR^{A4}C(=NR^{E4})NR^{A4}R^{B4}, -S(O)ᵣR^{A4}, -S(O)(=NR^{E4})R^{B4}, -N=S(O)R^{A4}R^{B4}, -S(O)₂OR^{A4} -OS(O)₂R^{A4}, -NR^{A4}S(O)ᵣR^{B4}, -NR^{A4}S(O)(=NR^{E4})R^{B4}, -S(O)ᵣNR^{A4}R^{B4}, -S(O)(=NR^{E4})NR^{A4}R^{B4}, -NR^{A4}S(O)NR^{A4}R^{B4}, -NR^{A4}S(O)(=NR^{E4})NR^{A4}R^{B4}, -P(O)R^{A4}R^{B4} and -P(O)(OR^{A4})(OR^{B4}), wherein alkyl, alkenyl, alkynyl, cycloalkyl, heterocyclyl, aryl and heteroaryl are each unsubstituted or substituted with at least one substituent, independently selected from R^{X};
or any two of R⁴ or "R⁴ and R¹⁰" or "R⁴ and R¹¹" together with the atoms to which they are attached form a C₃₋₁₀ cycloalkyl or heterocyclic ring of 4 to 12 members containing 1, 2 or 3 heteroatoms independently selected from oxygen, sulfur, nitrogen and phosphorus, and optionally substituted with 1, 2 or 3 R^{X} groups;
each R⁵ is independently selected y from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A5}R^{B5} , -OR^{A5} , -C(O)R ^{A5} , -C(=NR^{E5})R^{A5}, -C(=N-OR^{B5})R^{A5}, -C(O)OR^{A5}, -OC(O)R^{A5}, -C(O)NR^{A5}R^{B5}, -NR^{A5}C(O)R^{B5}, -C(=NR^{E5})NR^{A5}R^{B5}, -NR^{A5}C(=NR^{E5})R^{B5}, -OC(O)NR^{A5}R^{B5}, -NR^{A5}C(O)OR^{B5}, -NR^{A5}C(O)NR^{A5}R^{B5}, -NR^{A5}C(S)NR^{A5}R^{B5}, -NR^{A5}C(=NR^{E5})NR^{A5}R^{B5}, -S(O)ᵣR^{A5}, -S(0)(=NR^{E5})R^{B5}, -N=S(O)R^{A5}R^{B5}, -S(O)₂OR^{A5}, -OS(O)₂R^{A5}, -NR^{A5}S(O)ᵣR^{B5}, -NR^{A5}S(O)(=NR^{E5})R^{B5}, -S(O)ᵣNR^{A5}R^{B5}, -S(O)(=NR^{E5})NR^{A5}R^{B5}, - NR^{A5}S(O)₂NR^{A5}R^{B5}, -NR^{A5}S(O)(=NR^{E5})NR^{A5}R^{B5}, -P(O)R^{A5}R^{B5} and -P(O)(OR^{A5})(OR^{B5}), wherein alkyl, alkenyl, alkynyl, cycloalkyl, heterocyclyl, aryl and heteroaryl are each unsubstituted or substituted with at least one substituent, independently selected from R^{X};
or any two of R⁵ together with the carbon atoms to which they are attached form a C₃₋₁₀ cycloalkyl or heterocyclic ring of 4 to 12 members containing 1, 2 or 3 heteroatoms independently selected from oxygen, sulfur, nitrogen and phosphorus, and optionally substituted with 1, 2 or 3 R^{X} groups;
each R⁶ is independently selected from hydrogen, halogen, C₁-₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A6}R^{B6}, -OR^{A6}, -C(O)R^{A6}, -C(=NR^{E6})R^{A6}, -C(=N-OR^{B6})R^{A6}, -C(O)OR^{A6}, -OC(O)R^{A6}, -C(O)NR^{A6}R^{B6}, -NR^{A6}C(O)R^{B6}, -C(=NR^{E6})NR^{A6}R^{B6}, -NR^{A6}C(=NR^{E6})R^{B6}, -OC(O)NR^{A6}R^{B6}, -NR^{A6}C(O)OR^{B6}, -NR^{A6}C(O)NR^{A6}R^{B6}, -NR^{A6}C(S)NR^{A6}R^{B6}, -NR^{A6}C(=NR^{E6})NR^{A6}R^{B6}, -S(O)ᵣR^{A6}, -S(O)(=NR^{E6})R^{B6}, -N=S(O)R^{A6}R^{B6}, -S(O)₂OR^{A6}, -OS(O)₂R^{A6}, -NR^{A6}S(O)ᵣR^{B6}, -NR^{A6}S(O)(=NR^{E6})R^{B6}, -S(O)ᵣNR^{A6}R^{B6}, -S(O)(=NR^{E6})NR^{A6}R^{B6}, -NR^{A6}S(O)2NR^{A6}R^{B6}, -NR^{A6}S(O)(=NR^{E6})NR^{A6}R^{B6}, -P(O)R^{A6}R^{B6} and -P(O)(OR^{A6})(OR^{B6}), wherein alkyl, alkenyl, alkynyl, cycloalkyl, heterocyclyl, aryl and heteroaryl are each unsubstituted or substituted with at least one substituent, independently selected from R^{X};
or any two of R⁶ together with the carbon atoms to which they are attached form a C₃₋₁₀ cycloalkyl or heterocyclic ring of 4 to 12 members containing 1, 2 or 3 heteroatoms independently selected from oxygen, sulfur, nitrogen and phosphorus, and optionally substituted with 1, 2 or 3 R^{X} groups;
each R⁷ is independently selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A7}R^{B7}, -OR^{A7}, -C(O)R^{A7}, -C(=NR^{E7})R^{A7}, -C(=N-OR^{B7})R^{A7}, -C(O)OR^{A7}, -OC(O)R^{A7}, -C(O)NR^{A7}R^{B7}, -NR^{A7}C(O)R^{B7}, -C(=NR^{E7})NR^{A7}R^{B7}, -NR^{A7}C(=NR^{E7})R^{B7}, -OC(O)NR^{A7}R^{B7}, -NR^{A7}C(O)OR^{B7}, -NR^{A7}C(O)NR^{A7}R^{B7}, -NR^{A7}C(S)NR^{A7}R^{B7}, -NR^{A7}C(=NR^{E7})NR^{A7}R^{B7}, -S(O)ᵣR^{A7}, -S(O)(=NR^{E7})R^{B7}, -N=S(O)R^{A7}R^{B7}, -S(O)₂OR^{A7}, -OS(O)₂R^{A7}, -NR^{A7}S(O)ᵣR^{B7}, -NR^{A7}S(O)(=NR^{E7})R^{B7}, -S(O)ᵣNR^{A7}R^{B7}, -S(O)(=NR^{E7})NR^{A7}R^{B7}, -NR^{A7}S(O)2NR^{A7}R^{B7}, -NR^{A7}S(O)(=NR^{E7})NR^{A7}R^{B7}, -P(O)R^{A7}R^{B7} and -P(O)(OR^{A7})(OR^{B7}), wherein alkyl, alkenyl, alkynyl, cycloalkyl, heterocyclyl, aryl and heteroaryl are each unsubstituted or substituted with at least one substituent, independently selected from R^{X};
R⁸ is selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A8}R^{B8}, -OR^{A8}, -C(O)R^{A8}, -C(=NR^{E8})R^{A8}, -C(=N-OR^{B8})R^{A8}, -C(O)OR^{A8}, -OC(O)R^{A8}, -C(O)NR^{A8}R^{B8}, -NR^{AB}C(O)R^{B8}, -C(=NR^{E8})NR^{A8}R^{B8}, -NR^{AB}C(=NR^{E8})R^{B8}, -OC(O)NR^{A8}R^{B8}, -NR^{A8}C(O)OR^{B8}, -NR^{A8}C(O)NR^{A8}R^{B8}, -NR^{A8}C(S)NR^{A8}R^{B8}, -NR^{A8}C(=NR^{E8})NR^{A8}R^{B8}, -S(O)ᵣR^{A8}, -S(O)(=NR^{E8})R^{B8}, -N=S(O)R^{A8}R^{B8}, -S(O)₂OR^{A8}, -OS(O)₂R^{A8} -NR^{A8}S(O)ᵣR^{B8}, -NR^{A8}S(O)(=NR^{E8})R^{B8}, -S(O)ᵣNR^{A8}R^{B8}, -S(O)(=NR^{E8})NR^{A8}R^{B8}, -NR^{A8}S(O)₂NR^{A8}R^{B8}, -NR^{A8}S(O)(=NR^{E8})NR^{A8}R^{B8}, -P(O)R^{A8}R^{B8} and -P(O)(OR^{A8})(OR^{B8}), wherein alkyl, alkenyl, alkynyl, cycloalkyl, heterocyclyl, aryl and heteroaryl are each unsubstituted or substituted with at least one substituent, independently selected from R^{X};
or R¹ and R⁸ together with the atoms to which they are attached form a C₅₋₁₀ cycloalkyl or heterocyclic ring of 5 to 12 members containing 1, 2 or 3 heteroatoms independently selected from oxygen, sulfur, nitrogen and phosphorus, and optionally substituted with 1, 2 or 3 R^{X} groups;
R⁹ is selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A9}R^{B9}, -OR^{A9}, -C(O)R^{A9}, -C(=NR^{E9})R^{A9}, -C(=N-OR^{B9})R^{A9}, -C(O)OR^{A9}, -OC(O)R^{A9}, -C(O)NR^{A9}R^{B9}, -NR^{A9}C(O)R^{B9}, -C(=NR^{E9})NR^{A9}R^{B9}, -NR^{A9}C(=NR^{E9})R^{B9}, -OC(O)NR^{A9}R^{A9}, -NR^{A9}C(O)OR^{B9}, -NR^{A9}C(O)NR^{A9}R^{B9}, -NR^{A9}C(S)NR^{A9}R^{B9}, -NR^{A9}C(=NR^{E9})NR^{A9}R^{B9}, -S(O)ᵣR^{A9}, -S(O)(=NR^{E9})R^{B9}, -N=S(O)R^{A9}R^{B9}, -S(O)₂OR^{A9}, -OS(O)₂R^{A9}, -NR^{A9}S(O)ᵣR^{B9}, -NR^{A9}S(O)(=NR^{E9})R^{B9}, -S(O)ᵣNR^{A9}R^{B9}, -S(O)(=NR^{E9})NR^{A9}R^{B9}, -NR^{A9}S(O)₂NR^{A9}R^{B9}, _NR^{A9}S(O)(=NR^{E9})NR^{A9}R^{B9}, -P(O)R^{A9}R^{B9} and -P(O)(OR^{A9})(OR^{B9}), wherein alkyl, alkenyl, alkynyl, cycloalkyl, heterocyclyl, aryl and heteroaryl are each unsubstituted or substituted with at least one substituent, independently selected from R^{X};
R¹⁰ is selected from hydrogen, halogen, C₁-₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A10}R^{B10} -OR^{A10}, -C(O)R^{A10}, -C(=NR^{E10})R^{A10}, -C(=N-OR^{B10})R^{A10}, -C(O)OR^{A10}, -OC(O)R^{A10}, -C(O)NR^{A10}R^{B10}, -NR^{A10}C(O)R^{B10}, -C(=NR^{E10})NR^{A10}R^{B10}, -NR^{A10}C(=NR^{E10})R^{B10}, -OC(O)NR^{A10}R^{B10}, -NR^{A10}C(O)OR^{B10}, -NR^{A10}C(O)NR^{A10}R^{B10}, -NR^{A10}C(S)NR^{A10}R^{B10}, -NR^{A10}C(=NR^{E10})NR^{A10}R^{B10}, -S(O)ᵣR^{A10}, -S(O)(=NR^{E10})R^{B10}, -N=S(O)R^{A10}R^{B10}, -S(O)₂OR^{A10}, -OS(O)₂R^{A10}, -NR^{A10}S(O)ᵣR^{B10}, -NR^{A10}S(O)(=NR^{E10})R^{B10}, -S(O)ᵣNR^{A10}R^{B10}, -S(O)(=NR^{E10})NR^{A10}R^{B10}, -NR^{A10}S(O)₂NR^{A10}R^{B10}, -NR^{A10}S(O)(=NR^{E10})NR^{A10}R^{B10}, -P(O)R^{A10}R^{B10} and -P(O)(OR^{A10})(OR^{B10}), wherein alkyl, alkenyl, alkynyl, cycloalkyl, heterocyclyl, aryl and heteroaryl are each unsubstituted or substituted with at least one substituent, independently selected from R^{X};
R¹¹ is selected from hydrogen, C₁-₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, -C(O)R^{A11}, -C(=NR^{E11})R^{A11}, -C(=N-OR^{B11})R^{A11}, -C(O)OR^{A11}, -C(O)NR^{A11}R^{B11}, -C(=NR^{E11})NR^{A11}R^{B11}, -S(O)ᵣR^{A11}, -S(O)(=NR^{E11})R^{B11}, -S(O)₂OR^{A11}, -S(O)ᵣNR^{A11}R^{B11}, -S(O)(=NR^{E11})NR^{A11}R^{B11}, -P(O)R^{A11}R^{B11} and -P(O)(OR^{A11})(OR^{B11}), wherein alkyl, alkenyl, alkynyl, cycloalkyl, heterocyclyl, aryl and heteroaryl are each unsubstituted or substituted with at least one substituent, independently selected from R^{X};

each R^{A0}, R^{A1}, R^{A4}, R^{A5}, R^{A6}, R^{A7}, R^{A8}, R^{A9}, R^{A10}, R^{A11}, R^{B1}, R^{B4}, R^{B5}, R^{B6}, R^{B7}, R^{B8}, R^{B9}, R^{B10} and R^{B11} are independently selected from hydrogen, C₁-₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl and heteroaryl-C₁₋₄ alkyl, wherein alkyl, alkenyl, alkynyl, cycloalkyl, heterocyclyl, aryl and heteroaryl are each unsubstituted or substituted with at least one substituent, independently selected from R^{X};
or "R^{A1} and R^{B1}" or "R^{A4} and R^{B4}" or "R^{A5} and R^{B5}" or "R^{A6} and R^{B6}" or "R^{A7} and R^{B7}" or "R^{A8} and R^{B8}" or "R^{A9} and R^{B9}" or "R^{A10} and R^{B10}" or "R^{A11} and R^{B11}" together with the atom(s) to which they are attached form a heterocyclic ring of 4 to 12 members containing 0, 1 or 2 additional heteroatoms independently selected from oxygen, sulfur, nitrogen and phosphorus and optionally substituted with 1, 2 or 3 R^{X} groups;
   each R^{C0} and R^{D0} are independently selected from hydrogen, halogen, C₁-₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl and heteroaryl-C₁₋₄ alkyl, wherein alkyl, alkenyl, alkynyl, cycloalkyl, heterocyclyl, aryl and heteroaryl are each unsubstituted or substituted with at least one substituent, independently selected from R^{X};
   or each "R^{C0} and R^{D0}" together with the carbon atom(s) to which they are attached form a 3- to 12-membered ring containing 0, 1 or 2 heteroatoms independently selected from oxygen, sulfur and nitrogen, and optionally substituted with 1, 2 or 3 R^{X} groups;
   each R^{E1}, R^{E4}, R^{E5}, R^{E6}, R^{E7}, R^{E8}, R^{E9}, R^{E10} and R^{E11} are independently selected from hydrogen, C₁₋₁₀ alkyl, CN, NO₂, -OR^{a1}, -SR^{a1}, -S(O)ᵣR^{a1}, -C(O)R^{a1}, -C(O)OR^{a1}, -C(O)NR^{a1}R^{b1} and -S(O)ᵣNR^{a1}R^{b1}, wherein alkyl is unsubstituted or substituted with at least one substituent, independently selected from R^{X};
   each R^{X} is independently selected from hydrogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, halogen, CN, NO₂, -(CR^{c1}R^{d1})ₜNR^{a1}R^{b1},-(CR^{c1}R^{d1})ₜOR^{b1}, -(CR^{c1}R^{d1})ₜC(O)R^{a1}, -(CR^{c1}R^{d1})ₜC(=NR^{e1})R^{a1}, -(CR^{c1}R^{d1})ₜC(=N-OR^{b1})R^{a1}, -(CR^{c1}R^{d1})ₜC(O)OR^{b1}, -(CR^{c1}R^{a1})ₜOC(O)R^{b1}, -(CR^{c1}R^{d1})ₜC(O)NR^{a1}R^{b1}, -(CR^{c1}R^{d1})ₜNR^{a1}C(O)R^{b1}, -(CR^{c1}R^{d1})ₜC(=NR^{e1})NR^{a1}R^{b1}, -(CR^{c1}R^{d1})ₜNR^{a1}C(=NR^{e1})R¹, -(CR^{c1}R^{d1})ₜOC(O)NR^{a1}R^{b1}, -(CR^{c1}R^{d1})ₜNR^{a1}C(O)OR^{b1}, -(CR^{c1}R^{d1})ₜNR^{a1}C(O)NR^{a1}R^{b1}, -(CR^{c1}R^{d1})ₜNR^{a1}C(S)NR^{a1}R^{b1}, -(CR^{c1}R^{d1})tNR^{a1}C(=NR^{e1})NR^{a1}R^{b1}, -(CR^{c1}R^{d1})ₜS(O)ᵣR^{b1}, -(CR^{c1}R^{d1})ₜS(O)(=NR^{e1})R^{b1}, -(CR^{c1}R^{d1})ₜN=S(O)R^{a1}R^{b1}, -(CR^{c1}R^{d1})ₜS(O)₂OR^{b1}, -(CR^{c1}R^{a1})ₜOS(O)₂R^{b1}, -(CR^{c1}R^{d1})ₜNR^{a1}S(O)ᵣR^{b1}, -(CR^{c1}R^{d1})ₜNR^{a1}S(O)(=NR^{e1})R^{b1}, -(CR^{c1}R^{d1})ₜS(O)ᵣNR^{a1}R^{b1}, -(CR^{c1}R^{d1})ₜS(O)(=NR^{e1})NR^{a1}R^{b1}, -(CR^{c1}R^{d1})ₜNR^{a1}S(O)₂NR^{a1}R^{b1}, -(CR^{c1}R^{d1})ₜNR^{a1}S(O)(=NR^{e1})NR^{a1}R^{b1}, -(CR^{c1}R^{d1})ₜP(O)R^{a1}R^{b1} and -(CR^{c1}R^{d1})ₜP(O)(OR^{a1})(OR^{b1}), wherein alkyl, alkenyl, alkynyl, cycloalkyl, heterocyclyl, aryl and heteroaryl are each unsubstituted or substituted with at least one substituent, independently selected from R^{Y};
   each R^{a1} and each R^{b1} are independently selected from hydrogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl and heteroaryl-C₁₋₄ alkyl, wherein alkyl, alkenyl, alkynyl, cycloalkyl, heterocyclyl, aryl and heteroaryl are each unsubstituted or substituted with at least one substituent, independently selected from R^{Y};
   or R^{a1} and R^{b1} together with the atom(s) to which they are attached form a heterocyclic ring of 4 to 12 members containing 0, 1 or 2 additional heteroatoms independently selected from oxygen, sulfur, nitrogen and phosphorus, and optionally substituted with 1, 2 or 3 R^{Y} groups;
   each R^{c1} and each R^{d1} are independently selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl and heteroaryl-C₁₋₄ alkyl, wherein alkyl, alkenyl, alkynyl, cycloalkyl, heterocyclyl, aryl and heteroaryl are each unsubstituted or substituted with at least one substituent, independently selected from R^{Y};
   or R^{c1} and R^{d1} together with the carbon atom(s) to which they are attached form a ring of 3 to 12 members containing 0, 1 or 2 heteroatoms independently selected from oxygen, sulfur and nitrogen, and optionally substituted with 1, 2 or 3 R^{Y} groups;
   each R^{e1} is independently selected from hydrogen, C₁₋₁₀ alkyl, C₃₋₁₀ cycloalkyl, C₃₋₁₀ cycloalkyl-C₁₋₄ alkyl, CN, NO₂, -OR^{a2}, -SR^{a2}, -S(O)ᵣR^{a2}, -C(O)R^{a2}, -C(O)OR^{a2}, -S(O)ᵣNR^{a2}R^{b2} and -C(O)NR^{a2}R^{b2};
   each R^{Y} is independently selected from C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, halogen, CN, NO₂, -(CR^{c2}R^{d2})ₜNR^{a2}R^{b2}, -(CR^{c2}R^{d2})ₜOR^{b2}, -(CR^{c2}R^{d2})ₜC(O)R^{a2}, -(CR^{c2}R^{d2})ₜC(=NR^{e2})R^{a2}, -(CR^{c2}R^{d2})ₜC(=N-OR^{b2})R^{a2}, -(CR^{c2}R^{d2})ₜC(O)OR^{b2}, -(CR^{c2}R^{d2})ₜOC(O)R^{b2}, -(CR^{c2}R^{d2})ₜC(O)NR^{a2}R^{b2}, -(CR^{c2}R^{d2})ₜNR^{a2}C(O)R^{b2}, -(CR^{c2}R^{d2})ₜC(=NR^{e2})NR^{a2}R^{b2}, -(CR^{c2}R^{d2})ₜNR^{a2}C(=NR^{e2})R^{b2}, -(CR^{c2}R^{d2})ₜOC(O)NR^{a2}R^{b2}, -(CR^{c2}R^{d2})ₜNR^{a2}C(O)OR^{b1}, -(CR^{c2}R^{d2})ₜNR^{a2}C(O)NR^{a2}R^{b2}, -(CR^{c2}R^{d2})ₜNR^{a2}C(S)NR^{a2}R^{b2}, -(CR^{c2}R^{d2})ₜNR^{a2}C(=NR^{e2})NR^{a2}R^{b2}, -(CR^{c2}R^{d2})ₜS(O)ᵣR^{b2}, -(CR^{c2}R^{d2})ₜS(O)(=NR^{e2})R^{b2}, -(CR^{c2}R^{d2})ₜN=S(O)R^{a2}R^{b2}, -(CR^{c2}R^{d2})ₜS(O)₂OR^{b2}, -(CR^{c2}R^{d2})ₜOS(O)2R^{b2}, -(CR^{c2}R^{d2})ₜNR^{a2}S(O)ᵣR^{b2}, -(CR^{c2}R^{d2})ₜNR^{a2}S(O)(=NR^{c2})R^{b2}, -(CR^{c2}R^{d2})ₜS(O)ᵣNR^{a2}R^{b2}, -(CR^{c2} R^{d2})ₜS(O)(=NR^{e2})NR^{a2}R^{b2}, -(CR^{c2}R^{d2})ₜNR^{a2}S(O)₂NR^{a2}R^{b2}, -(CR^{c2}R^{d2})ₜNR^{a2}S(O)(=NR^{e2})NR^{a2}R^{b2}, -(CR^{c2}R^{d2})ₜP(O)R^{a2}R^{b2} and -(CR^{c2}R^{d2})ₜP(O)(OR^{a2})(OR^{b2}), wherein alkyl, alkenyl, alkynyl, cycloalkyl, heterocyclyl, aryl and heteroaryl are each unsubstituted or substituted with at least one substituent, independently selected from OH, CN, amino, halogen, C₁₋₁₀ alkyl, C₂₋₁₀ alkenyl, C₂₋₁₀ alkynyl, C₃₋₁₀ cycloalkyl, C₁₋₁₀ alkoxy, C₃₋₁₀ cycloalkoxy, C₁₋₁₀ alkylthio, C₃₋₁₀ cycloalkylthio, C₁₋₁₀ alkylamino, C₃₋₁₀ cycloalkylamino and di(C₁₋₁₀ alkyl)amino;
   each R^{a2} and each R^{b2} are independently selected from hydrogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, C₁₋₁₀ alkoxy, C₃₋₁₀ cycloalkoxy, C₁₋₁₀ alkylthio, C₃₋₁₀ cycloalkylthio, C₁-₁₀ alkylamino, C₃₋₁₀ cycloalkylamino, di(C₁₋₁₀ alkyl)amino, heterocyclyl, heterocyclyl-C₁₋₄ alkyl, aryl, aryl-C₁₋₄ alkyl, heteroaryl and heteroaryl-C₁₋₄ alkyl, wherein alkyl, alkenyl, alkynyl, cycloalkyl, alkoxy, cycloalkoxy, alkylthio, cycloalkylthio, alkylamino, cycloalkylamino, heterocyclyl, aryl and heteroaryl are each unsubstituted or substituted with at least one substituent, independently selected from halogen, CN, C₁₋₁₀ alkyl, C₂₋₁₀ alkenyl, C₂₋₁₀ alkynyl, C₃₋₁₀ cycloalkyl, OH, C₁₋₁₀ alkoxy, C₃₋₁₀ cycloalkoxy, C₁₋₁₀ alkylthio, C₃₋₁₀ cycloalkylthio, amino, C₁₋₁₀ alkylamino, C₃₋₁₀ cycloalkylamino and di(C₁₋₁₀ alkyl)amino;
   or R^{a2} and R^{b2} together with the atom(s) to which they are attached form a heterocyclic ring of 4 to 12 members containing 0, 1 or 2 additional heteroatoms independently selected from oxygen, sulfur, nitrogen and phosphorus, and optionally substituted with 1 or 2 substituents, independently selected from halogen, CN, C₁₋₁₀ alkyl, C₂₋₁₀ alkenyl, C₂₋₁₀ alkynyl, C₃₋₁₀ cycloalkyl, OH, C₁₋₁₀ alkoxy, C₃₋₁₀ cycloalkoxy, C₁₋₁₀ alkylthio, C₃₋₁₀ cycloalkylthio, amino, C₁₋₁₀ alkylamino, C₃₋₁₀ cycloalkylamino and di(C₁₋₁₀ alkyl)amino;
   each R^{c2} and each R^{d2} are independently selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, C₁₋₁₀ alkoxy, C₃₋₁₀ cycloalkoxy, C₁₋₁₀ alkylthio, C₃₋₁₀ cycloalkylthio, C₁₋₁₀ alkylamino, C₃₋₁₀ cycloalkylamino, di(C₁₋₁₀ alkyl)amino, heterocyclyl, heterocyclyl-C₁₋₄ alkyl, aryl, aryl-C₁₋₄ alkyl, heteroaryl and heteroaryl-C₁₋₄ alkyl, wherein alkyl, alkenyl, alkynyl, cycloalkyl, alkoxy, cycloalkoxy, alkylthio, cycloalkylthio, alkylamino, cycloalkylamino, heterocyclyl, aryl and heteroaryl are each unsubstituted or substituted with at least one substituent, independently selected from halogen, CN, C₁₋₁₀ alkyl, C₂₋₁₀ alkenyl, C₂₋₁₀ alkynyl, C₃₋₁₀ cycloalkyl, OH, C₁₋₁₀ alkoxy, C₃₋₁₀ cycloalkoxy, C₁₋₁₀ alkylthio, C₃₋₁₀ cycloalkylthio, amino, C₁₋₁₀ alkylamino, C₃₋₁₀ cycloalkylamino and di(C₁₋₁₀ alkyl)amino;
   or R^{c2} and R^{d2} together with the carbon atom(s) to which they are attached form a ring of 3 to 12 members containing 0, 1 or 2 heteroatoms independently selected from oxygen, sulfur and nitrogen, and optionally substituted with 1 or 2 substituents, independently selected from halogen, CN, C₁₋₁₀ alkyl, C₂₋₁₀ alkenyl, C₂₋₁₀ alkynyl, C₃₋₁₀ cycloalkyl, OH, C₁₋₁₀ alkoxy, C₃₋₁₀ cycloalkoxy, C₁₋₁₀ alkylthio, C₃₋₁₀ cycloalkylthio, amino, C₁₋₁₀ alkylamino, C₃₋₁₀ cycloalkylamino and di(C₁₋₁₀ alkyl)amino;
   each R^{e2} is independently selected from hydrogen, CN, NO₂, C₁₋₁₀ alkyl, C₃₋₁₀ cycloalkyl, C₃₋₁₀ cycloalkyl-C₁₋₄ alkyl, C₁₋₁₀ alkoxy, C₃₋₁₀ cycloalkoxy, -C(O)C₁₋₄ alkyl, -C(O)C₃₋₁₀ cycloalkyl, -C(O)OC₁₋₄ alkyl, -C(O)OC₃₋₁₀ cycloalkyl, -C(O)N(C₁₋₄ alkyl)₂, -C(O)N(C₃₋₁₀ cycloalkyl)₂, -S(O)₂C₁₋₄ alkyl, -S(O)₂C₃₋₁₀ cycloalkyl, -S(O)₂N(C₁₋₄ alkyl)₂ and -S(O)₂N(C₃₋₁₀ cycloalkyl)₂;
   m, m1, m2, n, n1, n2, p, p1, p2 and q are independently selected from 0, 1, 2 and 3;
   each r is independently selected from 0, 1 and 2;
   each t is independently selected from 0, 1, 2, 3 and 4;
   each u is independently selected from 0, 1, 2, 3 and 4.

In another Embodiment <2>, the invention provides a compound of Embodiment <1> or a pharmaceutically acceptable salt thereof, wherein X is N and Y is CR⁹.

In another Embodiment <3>, the invention provides a compound of Embodiment <2> or a pharmaceutically acceptable salt thereof, wherein X is N and Y is CH.

In another Embodiment <4>, the invention provides a compound of Embodiment <1> or a pharmaceutically acceptable salt thereof, wherein X is N and Y is N.

In another Embodiment <5>, the invention provides a compound of Embodiment <1> or a pharmaceutically acceptable salt thereof, wherein X is CR⁸ and Y is N.

In another Embodiment <6>, the invention provides a compound of Embodiment <5> or a pharmaceutically acceptable salt thereof, wherein R⁸ is selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A8}R^{B8}, -OR^{A8}, -C(O)R^{AB}, -C(=NR^{E8})R^{A8}, -C(=N-OR^{B8})R^{A8}, -C(O)OR^{A8}, -OC(O)R^{A8}, -C(O)NR^{A8}R^{B8}, -NR^{A8}C(O)R^{B8}, -C(=NR^{E8})NR^{A8}R^{B8}, -NR^{A8}C(=NR^{E8})R^{B8}, -OC(O)NR^{A8}R^{B8}, -NR^{A8}C(O)OR^{B8}, -NR^{A8}C(O)NR^{A8}R^{B8}, -NR^{A8}C(S)NR^{A8}R^{B8}, -NR^{A8}C(=NR^{E8})NR^{A8}R^{B8}, -S(O)ᵣR^{A8}, -S(O)(=NR^{E8})R^{B8}, -N=S(O)R^{A8}R^{B8}, -S(O)₂OR^{A8}, -OS(O)₂R^{A8}, -NR^{A8}S(O)ᵣR^{B8}, -NR^{A8}S(O)(=NR^{E8})R^{B8} and -S(O)ᵣNR^{A8}R^{B8}, wherein alkyl, alkenyl, alkynyl, cycloalkyl, heterocyclyl, aryl and heteroaryl are each unsubstituted or substituted with at least one substituent, independently selected from R^{X}.

In another Embodiment <7>, the invention provides a compound of Embodiment <6> or a pharmaceutically acceptable salt thereof, wherein R⁸ is selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, CN, NO₂, -NR^{A8}R^{B8}, -OR^{A8}, -C(O)R^{A8}, -C(O)OR^{A8}, -OC(O)R^{A8}, -C(O)NR^{A8}R^{B8}, -NR^{A8}C(O)R^{B8}, -OC(O)NR^{A8}R^{B8}, -NR^{A8}C(O)OR^{B8}, -NR^{A8}C(O)NR^{A8}R^{B8}, -NR^{A8}C(S)NR^{A8}R^{B8}, -S(O)ᵣR^{A8}, -S(O)₂OR^{A8}, -OS(O)₂R^{A8} and -NR^{A8}S(O)ᵣR^{B8}, wherein alkyl, alkenyl, alkynyl, cycloalkyl, heterocyclyl, aryl and heteroaryl are each unsubstituted or substituted with at least one substituent, independently selected from R^{X}.

In another Embodiment <8>, the invention provides a compound of any one of Embodiments <1>- <7> or a pharmaceutically acceptable salt thereof, wherein L₁ is selected from -(CR^{C0}R^{D0})ᵤ-, -(CR^{C0}R^{D0})ᵤO(CR^{C0}R^{D0})ₜ-, -(CR^{C0}R^{D0})ᵤNR^{A0}(CR^{C0}R^{D0})ₜ- and -(CR^{C0}R^{D0})ᵤS(O)ᵣ(CR^{C0}R^{D0})ₜ-.

In another Embodiment <9>, the invention provides a compound of Embodiment <8> or a pharmaceutically acceptable salt thereof, wherein L₁ is selected from -O-, -NH- and-S(O)_{r-.}.

In another Embodiment <10>, the invention provides a compound of any one of Embodiments <1>- <9> or a pharmaceutically acceptable salt thereof, wherein R¹ is selected from C₁₋₁₀ alkyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A1}R^{B1}, -OR^{A1}, -C(O)R^{A1}, -C(O)OR^{A1}, -OC(O)R^{A1}, -C(O)NR^{A1}R^{B1}, -NR^{A1}C(O)R^{B1}, -OC(O)NR^{A1}R^{B1}, -NR^{A1}C(O)OR^{B1}, -NR^{A1}C(O)NR^{A1}R^{B1} and -S(O)ᵣR^{A1}, wherein alkyl, cycloalkyl, heterocyclyl, aryl and heteroaryl are each unsubstituted or substituted with at least one substituent, independently selected from R^{X}.

In another Embodiment <11>, the invention provides a compound of Embodiment <10> or a pharmaceutically acceptable salt thereof, wherein R¹ is selected from C₁₋₁₀ alkyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl,, wherein alkyl, cycloalkyl, heterocyclyl, aryl and heteroaryl are each unsubstituted or substituted with at least one substituent, independently selected from R^{X}.

In another Embodiment <12>, the invention provides a compound of Embodiment <11> or a pharmaceutically acceptable salt thereof, wherein R¹ is selected from 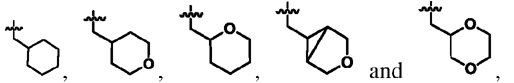 which are unsubstituted or substituted with at least one substituent, independently selected from R^{X}.

In another Embodiment <13>, the invention provides a compound of any one of Embodiments <1>- <12> or a pharmaceutically acceptable salt thereof, wherein the substituent R^{X} of R¹ is independently selected from C₁₋₁₀ alkyl, C₃₋₁₀ cycloalkyl, halogen, CN, NO2, -(CR^{c1}R^{d1})ₜNR^{a1}R^{b1}, -(CR^{c1}R^{d1})ₜOR^{b1}, -(CR^{c1}R^{d1})ₜC(O)R^{a1}, -(CR^{c1}R^{d1})ₜN=S(O)R^{a1}R^{b1}, wherein alkyl, cycloalkyl are each unsubstituted or substituted with at least one substituent, independently selected from R^{Y}.

In another Embodiment <14>, the invention provides a compound of Embodiment <13> or a pharmaceutically acceptable salt thereof, wherein each R^{a1} and each R^{b1} are independently selected from hydrogen, C₁₋₁₀ alkyl, C₂₋₁₀ alkenyl and C₂₋₁₀ alkynyl, wherein alkyl, alkenyl and alkynyl are each unsubstituted or substituted with at least one substituent, independently selected from R^{Y};
or R^{a1} and R^{b1} together with the atom(s) to which they are attached form a heterocyclic ring of 4 to 8 members containing 0, 1 or 2 additional heteroatoms independently selected from oxygen, sulfur, nitrogen and phosphorus, and optionally substituted with 1, 2 or 3 R^{Y} groups.

In another Embodiment <15>, the invention provides a compound of any one of Embodiments <1>- <14> or a pharmaceutically acceptable salt thereof, wherein the substituent R^{X} of R¹ is independently selected from OH, halogen, C₁₋₁₀ alkyl,

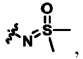

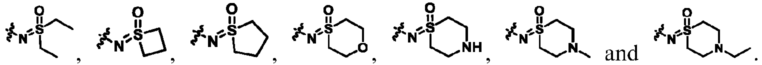

In another Embodiment <16>, the invention provides a compound of any one of Embodiments <1>-<15> or a pharmaceutically acceptable salt thereof, wherein R¹ is selected from 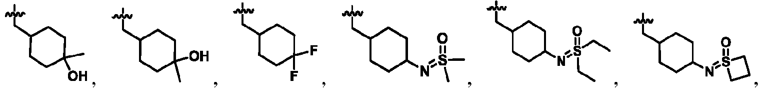 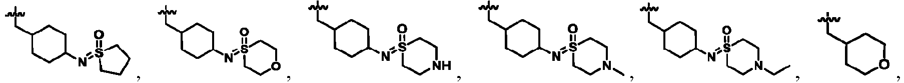 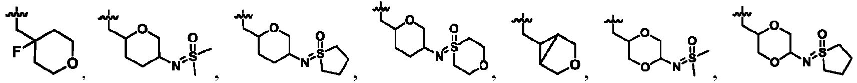 and

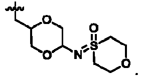

In another Embodiment <17>, the invention provides a compound of any one of Embodiments <1>- <10> or a pharmaceutically acceptable salt thereof, wherein R¹ and R⁸ together with the atoms to which they are attached form a cycloalkyl or heterocyclic ring of 5 to 8 members containing 1, 2 or 3 heteroatoms independently selected from oxygen, sulfur, nitrogen and phosphorus, and optionally substituted with 1, 2 or 3 R^{X} groups.

In another Embodiment <18>, the invention provides a compound of Embodiment <17> or a pharmaceutically acceptable salt thereof, wherein R¹ and R⁸ together with the atoms to which they are attached form heterocyclic ring of 5 to 6 members containing 1, 2 or 3 heteroatoms independently selected from oxygen, sulfur, nitrogen and phosphorus, and optionally substituted with 1, 2 or 3 R^{X} groups.

In another Embodiment <19>, the invention provides a compound of any one of Embodiments <1>- <18> or a pharmaceutically acceptable salt thereof, wherein the substructure 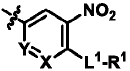 in Formula <I'> is 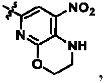 wherein the heterocyclic ring formed by R¹ and R⁸ is optionally substituted with 1, 2 or 3 R^{X} groups, wherein the 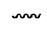 symbol indicates the point of attachment to the rest of the molecule.

In another Embodiment <20>, the invention provides a compound of Embodiment <19> or a pharmaceutically acceptable salt thereof, wherein the substituent R^{X} of 5 to 6 members heterocyclic ring is independently selected from C₁₋₁₀ alkyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, halogen, CN, NO₂, -(CR^{c1}R^{d1})ₜNR^{a1}R^{b1} and -(CR^{c1}R^{d1})ₜOR^{b1}, wherein alkyl, cycloalkyl, heterocyclyl, aryl and heteroaryl are each unsubstituted or substituted with at least one substituent, independently selected from R^{Y}.

In another Embodiment <21>, the invention provides a compound of Embodiment (20) or a pharmaceutically acceptable salt thereof, wherein the substituent R^{X} of 5 to 6 members heterocyclic ring is selected from halogen, CN, NO₂, OH, C₁₋₈ alkyl, C₃₋₈ cycloalkyl and benzyl.

In another Embodiment <22>, the invention provides a compound of any one of Embodiments <1>-<21> or a pharmaceutically acceptable salt thereof, wherein L₂ is selected from -(CR^{C0}R^{D0})ᵤ-, -(CR^{C0}R^{D0})ᵤO(CR^{C0}R^{D0})ₜ- and -(CR^{C0}R^{D0})ᵤNR^{A0}(CR^{C0}R^{D0})ₜ-.

In another Embodiment <23>, the invention provides a compound of Embodiment (18) or a pharmaceutically acceptable salt thereof, wherein L₂ is selected from a bond and -O-.

In another Embodiment <24>, the invention provides a compound of any one of Embodiments <1>- <23> or a pharmaceutically acceptable salt thereof, wherein R² is selected from heterocyclyl and heteroaryl, wherein heterocyclyl and heteroaryl are each unsubstituted or substituted with at least one substituent, independently selected from R^{X}.

In another Embodiment <25>, the invention provides a compound of Embodiment <24> or a pharmaceutically acceptable salt thereof, wherein R² is selected from

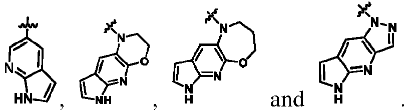

In another Embodiment <26>, the invention provides a compound of any one of Embodiments <1>- <25> or a pharmaceutically acceptable salt thereof, wherein W is selected from -CR¹⁰-, -NR¹¹- and -O-.

In another Embodiment <27>, the invention provides a compound of Embodiment <26> or a pharmaceutically acceptable salt thereof, wherein R¹⁰ and R¹¹ are independently selected from hydrogen, C₁₋₁₀ alkyl and C₃₋₁₀ cycloalkyl, wherein alkyl and cycloalkyl are each unsubstituted or substituted with at least one substituent, independently selected from R^{X}.

In another Embodiment <28>, the invention provides a compound of Embodiment <27> or a pharmaceutically acceptable salt thereof, wherein R¹⁰ is H.

In another Embodiment <29>, the invention provides a compound of Embodiment <27> or a pharmaceutically acceptable salt thereof, wherein R¹¹ is selected from H, methyl and ethyl.

In another Embodiment <30>, the invention provides a compound of any one of Embodiments <1>- <26> or a pharmaceutically acceptable salt thereof, wherein "R⁴ and R¹⁰" or "R⁴ and R¹¹" together with the atoms to which they are attached form a C₃₋₁₀ cycloalkyl or heterocyclic ring of 4 to 12 members containing 1, 2 or 3 heteroatoms independently selected from oxygen, sulfur, nitrogen and phosphorus, and optionally substituted with 1, 2 or 3 R^{X} groups.

In another Embodiment <31>, the invention provides a compound of Embodiment <30> or a pharmaceutically acceptable salt thereof, wherein "R⁴ and R¹⁰" or "R⁴ and R¹¹" together with the atoms to which they are attached form a C₃₋₈ cycloalkyl or heterocyclic ring of 4 to 10 members containing 1, 2 or 3 heteroatoms independently selected from oxygen, sulfur, nitrogen and phosphorus, and optionally substituted with 1, 2 or 3 R^{X} groups.

In another Embodiment <32>, the invention provides a compound of any one of Embodiments <1>- <31> or a pharmaceutically acceptable salt thereof, wherein m1 and m2 are independently selected from 0 and 1.

In another Embodiment <33>, the invention provides a compound of any one of Embodiments <1>- <31> or a pharmaceutically acceptable salt thereof, wherein R³ is aryl, wherein aryl is unsubstituted or substituted with at least one substituent, independently selected from R^{X}.

In another Embodiment <34>, the invention provides a compound of Embodiment <33> or a pharmaceutically acceptable salt thereof, wherein R³ is phenyl, wherein phenyl is unsubstituted or substituted with at least one substituent, independently selected from R^{X}.

In another Embodiment <35>, the invention provides a compound of Embodiment <34> or a pharmaceutically acceptable salt thereof, the substituent R^{X} of R³ is selected from C₁₋₁₀ alkyl, C₃₋₁₀ cycloalkyl, wherein alkyl and cycloalkyl are each unsubstituted or substituted with at least one substituent, independently selected from R^{Y}.

In another Embodiment <36>, the invention provides a compound of Embodiment <35> or a pharmaceutically acceptable salt thereof, the substituent R^{X} of R³ is selected from methyl, ethyl, isopropyl and cyclopropyl, wherein methyl, ethyl, isopropyl and cyclopropyl are each unsubstituted or substituted with at least one substituent, independently selected from halogen.

In another Embodiment <37>, the invention provides a compound of any one of Embodiments <1>-<36> or a pharmaceutically acceptable salt thereof, wherein m is selected from 0, 1 and 2.

In another Embodiment <38>, the invention provides a compound of Embodiment <37> or a pharmaceutically acceptable salt thereof, wherein each R⁴ is independently selected from hydrogen, halogen, C₁₋₁₀ alkyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, CN, NO₂, -NR^{A4}R^{B4}, -OR^{A4}, -C(O)R^{A4}, -C(O)OR^{A4}, -OC(O)R^{A4} -C(O)NR^{A4}R^{B4} and -S(O)ᵣR^{A4}, wherein alkyl, cycloalkyl, heterocyclyl, aryl and heteroaryl are each unsubstituted or substituted with at least one substituent, independently selected from R^{X}.

In another Embodiment <39>, the invention provides a compound of Embodiment <38> or a pharmaceutically acceptable salt thereof, wherein any two of R⁴ together with the atoms to which they are attached form a C₃₋₈ cycloalkyl or heterocyclic ring of 4 to 8 members containing 1, 2 or 3 heteroatoms independently selected from oxygen, sulfur, nitrogen and phosphorus, and optionally substituted with 1, 2 or 3 R^{X} groups.

In another Embodiment <40>, the invention provides a compound of Embodiment <39> or a pharmaceutically acceptable salt thereof, wherein any two of R⁴ together with the atoms to which they are attached form cyclopropyl, wherein cyclopropyl is unsubstituted or substituted with 1, 2 or 3 R^{X} groups.

In another Embodiment <41>, the invention provides a compound of any one of Embodiments <1>- <40> or a pharmaceutically acceptable salt thereof, wherein the substructure of Formula <II'> 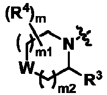 in Formula <I'> is selected from 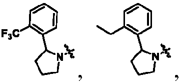 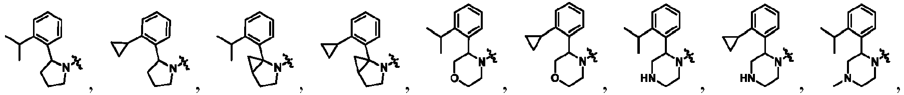 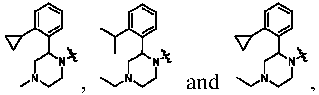 wherein the 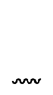 symbol indicates the point of attachment to the rest of the molecule.

In another Embodiment <42>, the invention provides a compound of any one of Embodiments <1>- <41> or a pharmaceutically acceptable salt thereof, wherein each R⁵ and R⁶ are independently selected from hydrogen, halogen, C₁₋₁₀ alkyl, C₃₋₁₀ cycloalkyl, CN, NO₂, -NR^{A7}R^{B7}, -OR^{A7} and -C(O)R^{A7}, wherein alkyl and cycloalkyl are each unsubstituted or substituted with at least one substituent, independently selected from R^{X}.

In another Embodiment <43>, the invention provides a compound of Embodiment <41> or a pharmaceutically acceptable salt thereof, wherein R⁵ and R⁶ are H.

In another Embodiment <44>, the invention provides a compound of any one of Embodiments <1>-<43> or a pharmaceutically acceptable salt thereof, wherein the substructure of Formula <III'> 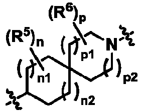 in Formula <I'> is selected from 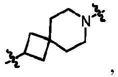 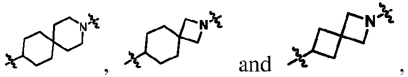 wherein the 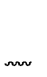 symbol indicates the point of attachment to the rest of the molecule.

In another Embodiment <45>, the invention provides a compound of any one of Embodiments <1>-<44> or a pharmaceutically acceptable salt thereof, wherein each R⁷ is independently selected from hydrogen, halogen, C₁₋₁₀ alkyl, C₃₋₁₀ cycloalkyl, CN, NO₂, -NR^{A7}R^{B7}, -OR^{A7} and -C(O)R^{A7}, wherein alkyl and cycloalkyl are each unsubstituted or substituted with at least one substituent, independently selected from R^{X}.

In another Embodiment <46>, the invention provides a compound of Embodiment <45> or a pharmaceutically acceptable salt thereof, wherein R⁷ is H.

In another Embodiment <48>, the invention provides a pharmaceutical composition comprising a compound of any one of Embodiments <1>- <47> or a pharmaceutically acceptable salt thereof and at least one pharmaceutically acceptable carrier.

In another Embodiment <49>, the invention provides a method of treating, ameliorating or preventing a condition, which responds to inhibition of Bcl-2, comprising administering to a subject in need of such treatment an effective amount of a compound of any one of Embodiments <1>- <47>, or a pharmaceutically acceptable salt thereof, or a pharmaceutical composition thereof, and optionally in combination with a second therapeutic agent.

In another Embodiment <50>, the invention provides a use of a compound of any one of Embodiments<1>- <47> or a pharmaceutically acceptable salt thereof in the preparation of a medicament for treating a cell-proliferative disorder.

In yet another of its aspects, there is provided a kit comprising a compound disclosed herein, or a pharmaceutically acceptable salt thereof; and instructions which comprise one or more forms of information selected from the group consisting of indicating a disease state for which the composition is to be administered, storage information for the composition, dosing information and instructions regarding how to administer the composition. In one particular variation, the kit comprises the compound in a multiple dose form.

In still another of its aspects, there is provided an article of manufacture comprising a compound disclosed herein, or a pharmaceutically acceptable salt thereof; and packaging materials. In one variation, the packaging material comprises a container for housing the compound. In one particular variation, the container comprises a label indicating one or more members of the group consisting of a disease state for which the compound is to be administered, storage information, dosing information and/or instructions regarding how to administer the compound. In another variation, the article of manufacture comprises the compound in a multiple dose form.

In a further of its aspects, there is provided a therapeutic method comprising administering a compound disclosed herein, or a pharmaceutically acceptable salt thereof.

In another of its aspects, there is provided a method of inhibiting a Bcl-2 comprising contacting the Bcl-2 with a compound disclosed herein, or a pharmaceutically acceptable salt thereof.

In yet another of its aspects, there is provided a method of inhibiting a Bcl-2 comprising causing a compound disclosed herein, or a pharmaceutically acceptable salt thereof to be present in a subject in order to inhibit the Bcl-2 *in vivo.*

In a further of its aspects, there is provided a method of inhibiting Bcl-2 comprising administering a first compound to a subject that is converted *in vivo* to a second compound wherein the second compound inhibits the Bcl-2 *in vivo,* the second compound being a compound according to any one of the above embodiments and variations.

In another of its aspects, there is provided a method of treating a disease state for which a Bcl-2 possesses activity that contributes to the pathology and/or symptomology of the disease state, the method comprising causing a compound disclosed herein, or a pharmaceutically acceptable salt thereof to be present in a subject in a therapeutically effective amount for the disease state.

In a further of its aspects, there is provided a method of treating a disease state for which a Bcl-2 possesses activity that contributes to the pathology and/or symptomology of the disease state, the method comprising administering a first compound to a subject that is converted in vivo to a second compound wherein the second compound inhibits the Bcl-2 *in vivo.* It is noted that the compounds of the present invention may be the first or second compounds.

In one variation of each of the above methods the disease state is selected from the group consisting of cancerous hyperproliferative disorders (e.g., brain, lung, squamous cell, bladder, gastric, pancreatic, breast, head, neck, renal, kidney, ovarian, prostate, colorectal, epidermoid, esophageal, testicular, gynecological or thyroid cancer); non-cancerous hyperproliferative disorders (e.g., benign hyperplasia of the skin (e.g., psoriasis), restenosis, and benign prostatic hypertrophy (BPH)); pancreatitis; kidney disease; pain; preventing blastocyte implantation; treating diseases related to vasculogenesis or angiogenesis (e.g., tumor angiogenesis, acute and chronic inflammatory disease such as rheumatoid arthritis, atherosclerosis, inflammatory bowel disease, skin diseases such as psoriasis, eczema, and scleroderma, diabetes, diabetic retinopathy, retinopathy of prematurity, age-related macular degeneration, hemangioma, glioma, melanoma, Kaposi's sarcoma and ovarian, breast, lung, pancreatic, prostate, colon and epidermoid cancer); asthma; neutrophil chemotaxis (e.g., reperfusion injury in myocardial infarction and stroke and inflammatory arthritis); septic shock; T-cell mediated diseases where immune suppression would be of value (e.g., the prevention of organ transplant rejection, graft versus host disease, lupus erythematosus, multiple sclerosis, and rheumatoid arthritis); atherosclerosis; inhibition of keratinocyte responses to growth factor cocktails; chronic obstructive pulmonary disease (COPD) and other diseases.

In another of its aspects, there is provided a method of treating a disease state for which a mutation in the *Bcl-2* gene contributes to the pathology and/or symptomology of the disease state including, for example, melanomas, lung cancer, colon cancer and other tumor types.

In still another of its aspects, the present invention relates to the use of a compound of any of the above embodiments and variations as a medicament. In yet another of its aspects, the present invention relates to the use of a compound according to any one of the above embodiments and variations in the manufacture of a medicament for inhibiting a Bcl-2.

In a further of its aspects, the present invention relates to the use of a compound according to any one of the above embodiments and variations in the manufacture of a medicament for treating a disease state for which a Bcl-2 possesses activity that contributes to the pathology and/or symptomology of the disease state.

### Administration and Pharmaceutical Compositions

In general, compounds of the disclosure will be administered in therapeutically effective amounts via any of the usual and acceptable modes known in the art, either singly or in combination with one or more therapeutic agents. A therapeutically effective amount may vary widely depending on the severity of the disease, the age and relative health of the subject, the potency of the compound used and other factors known to those of ordinary skill in the art. For example, for the treatment of neoplastic diseases and immune system disorders, the required dosage will also vary depending on the mode of administration, the particular condition to be treated and the effect desired.

In general, satisfactory results are indicated to be obtained systemically at daily dosages of from about 0.001 to about 100 mg/kg per body weight, or particularly, from about 0.03 to 2.5 mg/kg per body weight. An indicated daily dosage in the larger mammal, e.g. humans, may be in the range from about 0.5 mg to about 2000 mg, or more particularly, from about 0.5 mg to about 1000 mg, conveniently administered, for example, in divided doses up to four times a day or in retard form. Suitable unit dosage forms for oral administration comprise from ca. 1 to 50 mg active ingredient.

Compounds of the disclosure may be administered as pharmaceutical compositions by any conventional route; for example, enterally, e.g., orally, e.g., in the form of tablets or capsules; parenterally, e.g., in the form of injectable solutions or suspensions; or topically, e.g., in the form of lotions, gels, ointments or creams, or in a nasal or suppository form.

Pharmaceutical compositions comprising a compound of the present disclosure in free form or in a pharmaceutically acceptable salt form in association with at least one pharmaceutically acceptable carrier or diluent may be manufactured in a conventional manner by mixing, granulating, coating, dissolving or lyophilizing processes. For example, pharmaceutical compositions comprising a compound of the disclosure in association with at least one pharmaceutical acceptable carrier or diluent may be manufactured in conventional manner by mixing with a pharmaceutically acceptable carrier or diluent. Unit dosage forms for oral administration contain, for example, from about 0.1 mg to about 500 mg of active substance.

In one embodiment, the pharmaceutical compositions are solutions of the active ingredient, including suspensions or dispersions, such as isotonic aqueous solutions. In the case of lyophilized compositions comprising the active ingredient alone or together with a carrier such as mannitol, dispersions or suspensions can be made up before use. The pharmaceutical compositions may be sterilized and/or contain adjuvants, such as preserving, stabilizing, wetting or emulsifying agents, solution promoters, salts for regulating the osmotic pressure and/or buffers. Suitable preservatives include but are not limited to antioxidants such as ascorbic acid, or microbicides, such as sorbic acid or benzoic acid. The solutions or suspensions may further comprise viscosity-increasing agents, including but not limited to, sodium carboxymethylcellulose, carboxymethylcellulose, dextran, polyvinylpyrrolidone, gelatins, or solubilizers, e.g. Tween 80 (polyoxyethylene (20) sorbitan monooleate).

Suspensions in oil may comprise as the oil component the vegetable, synthetic, or semi-synthetic oils customary for injection purposes. Examples include but are not limited to liquid fatty acid esters that contain as the acid component a long-chained fatty acid having 8-22 carbon atoms, or in some embodiments, 12-22 carbon atoms. Suitable liquid fatty acid esters include but are not limited to lauric acid, tridecylic acid, myristic acid, pentadecylic acid, palmitic acid, margaric acid, stearic acid, arachidic acid, behenic acid or corresponding unsaturated acids, for example oleic acid, elaidic acid, erucic acid, brassidic acid and linoleic acid, and if desired, may contain antioxidants, for example vitamin E, 3-carotene or 3,5-di-tert-butyl-hydroxytoluene. The alcohol component of these fatty acid esters may have six carbon atoms and may be monovalent or polyvalent, for example a mono-, di- or trivalent, alcohol. Suitable alcohol components include but are not limited to methanol, ethanol, propanol, butanol or pentanol or isomers thereof; glycol and glycerol.

Other suitable fatty acid esters include but are not limited ethyl-oleate, isopropyl myristate, isopropyl palmitate, LABRAFIL^{®} M 2375, (polyoxyethylene glycerol), LABRAFIL^{®} M 1944 CS (unsaturated polyglycolized glycerides prepared by alcoholysis of apricot kernel oil and comprising glycerides and polyethylene glycol ester), LABRASOL^{™} (saturated polyglycolized glycerides prepared by alcoholysis of TCM and comprising glycerides and polyethylene glycol ester; all available from GaKefosse, France), and/or MIGLYOL^{®} 812 (triglyceride of saturated fatty acids of chain length C8 to C12 from Hüls AG, Germany), and vegetable oils such as cottonseed oil, almond oil, olive oil, castor oil, sesame oil, soybean oil, or groundnut oil.

Pharmaceutical compositions for oral administration may be obtained, for example, by combining the active ingredient with one or more solid carriers, and if desired, granulating a resulting mixture, and processing the mixture or granules by the inclusion of additional excipients, to form tablets or tablet cores.

Suitable carriers include but are not limited to fillers, such as sugars, for example lactose, saccharose, mannitol or sorbitol, cellulose preparations and/or calcium phosphates, for example tricalcium phosphate or calcium hydrogen phosphate, and also binders, such as starches, for example corn, wheat, rice or potato starch, methylcellulose, hydroxypropyl methylcellulose, sodium carboxymethylcellulose, and/or polyvinylpyrrolidone, and/or, if desired, disintegrators, such as the above-mentioned starches, carboxymethyl starch, crosslinked polyvinylpyrrolidone, alginic acid or a salt thereof, such as sodium alginate. Additional excipients include but are not limited to flow conditioners and lubricants, for example silicic acid, talc, stearic acid or salts thereof, such as magnesium or calcium stearate, and/or polyethylene glycol, or derivatives thereof.

Tablet cores may be provided with suitable, optionally enteric, coatings through the use of, inter alia, concentrated sugar solutions which may comprise gum arable, talc, polyvinylpyrrolidone, polyethylene glycol and/or titanium dioxide, or coating solutions in suitable organic solvents or solvent mixtures, or, for the preparation of enteric coatings, solutions of suitable cellulose preparations, such as cellulose acetate phthalate or hydroxypropyl methylcellulose phthalate. Dyes or pigments may be added to the tablets or tablet coatings, for example for identification purposes or to indicate different doses of active ingredient.

Pharmaceutical compositions for oral administration may also include hard capsules comprising gelatin or soft-sealed capsules comprising gelatin and a plasticizer, such as glycerol or sorbitol. The hard capsules may contain the active ingredient in the form of granules, for example in admixture with fillers, such as corn starch, binders, and/or glidants, such as talc or magnesium stearate, and optionally stabilizers. In soft capsules, the active ingredient may be dissolved or suspended in suitable liquid excipients, such as fatty oils, paraffin oil or liquid polyethylene glycols or fatty acid esters of ethylene or propylene glycol, to which stabilizers and detergents, for example of the polyoxyethylene sorbitan fatty acid ester type, may also be added.

Pharmaceutical compositions suitable for rectal administration are, for example, suppositories comprising a combination of the active ingredient and a suppository base. Suitable suppository bases are, for example, natural or synthetic triglycerides, paraffin hydrocarbons, polyethylene glycols or higher alkanols.

Pharmaceutical compositions suitable for parenteral administration may comprise aqueous solutions of an active ingredient in water-soluble form, for example of a water-soluble salt, or aqueous injection suspensions that contain viscosity-increasing substances, for example sodium carboxymethylcellulose, sorbitol and/or dextran, and, if desired, stabilizers. The active ingredient, optionally together with excipients, can also be in the form of a lyophilizate and can be made into a solution before parenteral administration by the addition of suitable solvents. Solutions such as are used, for example, for parenteral administration can also be employed as infusion solutions. The manufacture of injectable preparations is usually carried out under sterile conditions, as is the filling, for example, into ampoules or vials, and the sealing of the containers.

The disclosure also provides for a pharmaceutical combination, e.g. a kit, comprising a) a first agent which is a compound of the disclosure as disclosed herein, in free form or in pharmaceutically acceptable salt form, and b) at least one co-agent. The kit can comprise instructions for its administration.

### Combination therapies

The compounds or pharmaceutical acceptable salts of the disclosure may be administered as the sole therapy, or together with other therapeutic agent or agents.

For example, the therapeutic effectiveness of one of the compounds described herein may be enhanced by administration of an adjuvant (i.e. by itself the adjuvant may only have minimal therapeutic benefit, but in combination with another therapeutic agent, the overall therapeutic benefit to the individual is enhanced). Or, by way of example only, the benefit experienced by an individual may be increased by administering one of the compounds described herein with another therapeutic agent that also has therapeutic benefit. By way of example only, in a treatment for gout involving administration of one of the compounds described herein, increased therapeutic benefit may result by also providing the individual with another therapeutic agent for gout. Or, by way of example only, if one of the side effects experienced by an individual upon receiving one of the compounds described herein is nausea, then it may be appropriate to administer an anti-nausea agent in combination with the compound. Or, the additional therapy or therapies include, but are not limited to physiotherapy, psychotherapy, radiation therapy, application of compresses to a diseased area, rest, altered diet, and the like. Regardless of the disease, disorder or condition being treated, the overall benefit experienced by the individual may be additive of the two therapies or the individual may experience a synergistic benefit.

In the instances where the compounds described herein are administered in combination with other therapeutic agents, the compounds described herein may be administered in the same pharmaceutical composition as other therapeutic agents, or because of different physical and chemical characteristics, be administered by a different route. For example, the compounds described herein may be administered orally to generate and maintain good blood levels thereof, while the other therapeutic agent may be administered intravenously. Thus the compounds described herein may be administered concurrently, sequentially or dosed separately to other therapeutic agents.

### EXAMPLES

Various methods may be developed for synthesizing a compound of formula (I) or a pharmaceutically acceptable salt thereof. Representative methods for synthesizing a compound of formula (I) or a pharmaceutically acceptable salt thereof are provided in the Examples. It is noted, however, that a compound of formula (I) or a pharmaceutically acceptable salt thereof may also be synthesized by other synthetic routes that others may devise.

It will be readily recognized that certain compounds of formula (I) have atoms with linkages to other atoms that confer a particular stereochemistry to the compound (e.g., chiral centers). It is recognized that synthesis of a compound of formula (I) or a pharmaceutically acceptable salt thereof may result in the creation of mixtures of different stereoisomers (enantiomers, diastereomers). Unless a particular stereochemistry is specified, recitation of a compound is intended to encompass all of the different possible stereoisomers.

A compound of formula (I) can also be prepared as a pharmaceutically acceptable acid addition salt by, for example, reacting the free base form of the at least one compound with a pharmaceutically acceptable inorganic or organic acid. Alternatively, a pharmaceutically acceptable base addition salt of the at least one compound of formula (I) can be prepared by, for example, reacting the free acid form of the at least one compound with a pharmaceutically acceptable inorganic or organic base. Inorganic and organic acids and bases suitable for the preparation of the pharmaceutically acceptable salts of compounds of formula (I) are set forth in the definitions section of this Application. Alternatively, the salt forms of the compounds of formula (I) can be prepared using salts of the starting materials or intermediates.

The free acid or free base forms of the compounds of formula (I) can be prepared from the corresponding base addition salt or acid addition salt form. For example, a compound of formula (I) in an acid addition salt form can be converted to the corresponding free base thereof by treating with a suitable base (e.g., ammonium hydroxide solution, sodium hydroxide, and the like). A compound of formula (I) in a base addition salt form can be converted to the corresponding free acid thereof by, for example, treating with a suitable acid (e.g., hydrochloric acid, etc).

The N-oxides of a compound of formula (I) or a pharmaceutically acceptable salt thereof can be prepared by methods known to those of ordinary skill in the art. For example, N-oxides can be prepared by treating an unoxidized form of the compound of formula (I) with an oxidizing agent (e.g., trifluoroperacetic acid, permaleic acid, perbenzoic acid, peracetic acid, meta-chloroperoxybenzoic acid, or the like) in a suitable inert organic solvent (e.g., a halogenated hydrocarbon such as dichloromethane) at approximately 0 to 80°C. Alternatively, the N-oxides of the compounds of formula (I) can be prepared from the N-oxide of an appropriate starting material.

Compounds of formula (I) in an unoxidized form can be prepared from N-oxides of compounds of formula (I) by, for example, treating with a reducing agent (e.g., sulfur, sulfur dioxide, triphenyl phosphine, lithium borohydride, sodium borohydride, phosphorus trichloride, tribromide, and the like) in an suitable inert organic solvent (e.g., acetonitrile, ethanol, aqueous dioxane, and the like) at 0 to 80°C.

Protected derivatives of the compounds of formula (I) can be made by methods known to those of ordinary skill in the art. A detailed description of the techniques applicable to the creation of protecting groups and their removal can be found in T.W. Greene, Protecting Groups in Organic Synthesis, 3rd edition, John Wiley & Sons, Inc. 1999.

As used herein the symbols and conventions used in these processes, schemes and examples are consistent with those used in the contemporary scientific literature, for example, the Journal of the American Chemical Society or the Journal of Biological Chemistry. Standard single-letter or three-letter abbreviations are generally used to designate amino acid residues, which are assumed to be in the L-configuration unless otherwise noted. Unless otherwise noted, all starting materials were obtained from commercial suppliers and used without further purification. For example, the following abbreviations may be used in the examples and throughout the specification: g (grams); mg (milligrams); L (liters); mL (milliliters); µL (microliters); psi (pounds per square inch); M (molar); mM (millimolar); i.v. (intravenous); Hz (Hertz); MHz (megahertz); mol (moles); mmol (millimoles); RT (room temperature); min (minutes); h (hours); mp (melting point); TLC (thin layer chromatography); Rt (retention time); RP (reverse phase); MeOH (methanol); i-PrOH (isopropanol); TEA (triethylamine); TFA (trifluoroacetic acid); TFAA (trifluoroacetic anhydride); THF (tetrahydrofuran); DMSO (dimethyl sulfoxide); EtOAc (ethyl acetate); DME (1,2-dimethoxyethane); DCM (dichloromethane); DCE (dichloroethane); DMF (N,N-dimethylformamide); DMPU (N,N'-dimethylpropyleneurea); CDI (1,1-carbonyldiimidazole); IBCF (isobutyl chloroformate); HOAc (acetic acid); HOSu (N-hydroxysuccinimide); HOBT (1-hydroxybenzotriazole); Et₂O (diethyl ether); EDCI (1-(3-dimethylaminopropyl)-3-ethylcarbodiimide hydrochloride); BOC (tert-butyloxycarbonyl); FMOC (9-fluorenylmethoxycarbonyl); DCC (dicyclohexylcarbodiimide); CBZ (benzyloxycarbonyl); Ac (acetyl); atm (atmosphere); TMSE (2-(trimethylsilyl)ethyl); TMS (trimethylsilyl); TIPS (triisopropylsilyl); TBS (t-butyldimethylsilyl); DMAP (4-dimethylaminopyridine); Me (methyl); OMe (methoxy); Et (ethyl); tBu (tert-butyl); HPLC (high pressure liquid chromatography); BOP (bis(2-oxo-3-oxazolidinyl)phosphinic chloride); TBAF (tetra-n-butylammonium fluoride); m-CPBA (meta-chloroperbenzoic acid). For example, the following abbreviations in table 1 may be used in the examples and throughout the specification.

References to ether or Et₂O are to diethyl ether; brine refers to a saturated aqueous solution of NaCl. Unless otherwise indicated, all temperatures are expressed in °C (degrees Centigrade). All reactions were conducted under an inert atmosphere at RT unless otherwise noted.

¹H NMR spectra were recorded on a Varian Mercury Plus 400. Chemical shifts are expressed in parts per million (ppm). Coupling constants are in units of hertz (Hz). Splitting patterns describe apparent multiplicities and are designated as s (singlet), d (doublet), t (triplet), q (quartet), m (multiplet) and br (broad).

Low-resolution mass spectra (MS) and compound purity data were acquired on a Shimadzu LC/MS single quadrupole system equipped with electrospray ionization (ESI) source, UV detector (220 and 254 nm), and evaporative light scattering detector (ELSD). Thin-layer chromatography was performed on 0.25 mm Superchemgroup silica gel plates (60F-254), visualized with UV light, 5% ethanolic phosphomolybdic acid, ninhydrin, or p-anisaldehyde solution. Flash column chromatography was performed on silica gel (200-300 mesh, Branch of Qingdao Haiyang Chemical Co., Ltd).

### Synthetic Schemes

A compound of formula I or a pharmaceutically acceptable salt thereof may be synthesized according to a variety of reaction schemes. Some illustrative schemes are provided below and in the examples. Other reaction schemes could be readily devised by those skilled in the art in view of the present disclosure.

In the reactions described hereinafter it may be necessary to protect reactive functional groups, for example hydroxyl, amino, imino, thio or carboxyl groups, where these are desired in the final product, to avoid their unwanted participation in the reactions. Conventional protecting groups may be used in accordance with standard practice, for examples see T.W. Greene and P. G. M. Wuts in "Protective Groups in Organic Chemistry" John Wiley and Sons, 1991.

Synthetic methods for preparing the compounds of the present invention are illustrated in the following Schemes and Examples. Starting materials are commercially available or may be made according to procedures known in the art or as illustrated herein.

The intermediates shown in the following schemes are either known in the literature or may be prepared by a variety of methods familiar to those skilled in the art.

As an illustration, one synthetic approach of compounds of formula I of the present disclosure is shown in Scheme 1. As show in the scheme, the compounds of formula I can be disassembled into intermediates II to V, which are either commercially available or known in the literature. Intermediates of formula III can be prepared by the coupling of IV with the intermediates V using nucleophilic substitution reactions or transitional metal catalyzed cross coupling reactions known in the literature. Coupling of Intermediates of formula III with Intermediates of formula II provides compounds of formula I through condensation reaction.

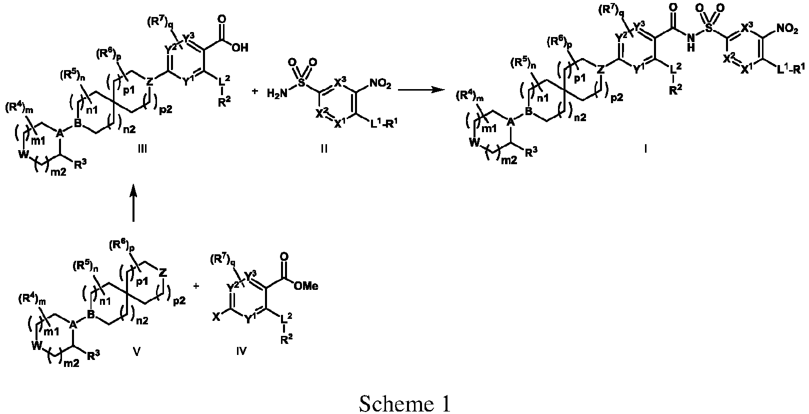

As an illustration of the preparation of intermediates of formula II, a route of intermediates IIa & IIb are shown in Scheme 2. Starting from the commercially available IIa-A, IIa can be obtained by reaction with IIa-B through nucleophilic substitution reactions. Similarly, intermediates of formula IIb can be prepared from IIb-A via a sequence of nucleophilic cross coupling reaction followed by intramolecular cyclization using metal catalyzed coupling reactions such as Buchwald reaction or other conditions known in the literature.

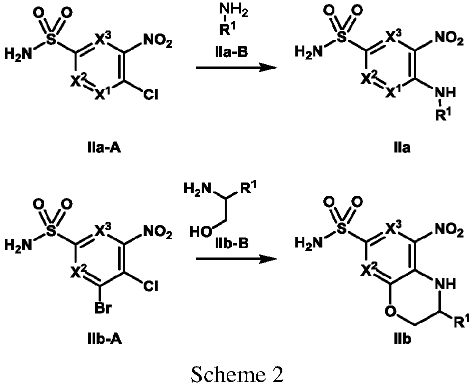

As an illustration of the synthesis of intermediates of formula IV, one of the synthetic approach of the compounds of formula IVa is outlined in Scheme 3. Starting from the IVa-A, which is either commercially available or known in the literature, IVa-D can be prepared by converting the halogen group in IVa-A into a hydroxyl group. Cross-coupling of IVa-D with IVa-E through nucleophilic displacement reaction leads to intermediates of formula IVa.

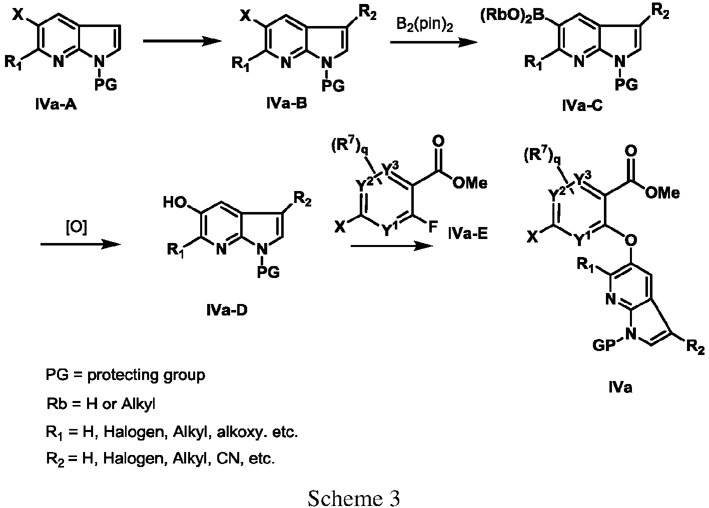

As an illustration of the synthesis of compounds of formula V, one synthetic approach to the compounds of formula Va is outlined in Scheme 4. Intermediate Va can be prepared from Va-A, which is either commercially available or known in the literature, via a sequence of reduction-amination and deprotection reactions.

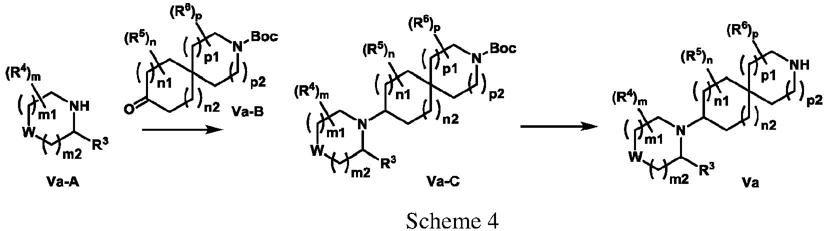

As a further illustration of the synthesis of compounds of formula V, one synthetic approach to intermediate Vb is outlined in Scheme 5. Starting from the Vb-A, which is either commercially available or known in the literature, intermediates of formula Vb can be prepared via a three-step sequence of reduction-amination intramolecular, SN₂ nucleophilic displacement and deprotection reactions.

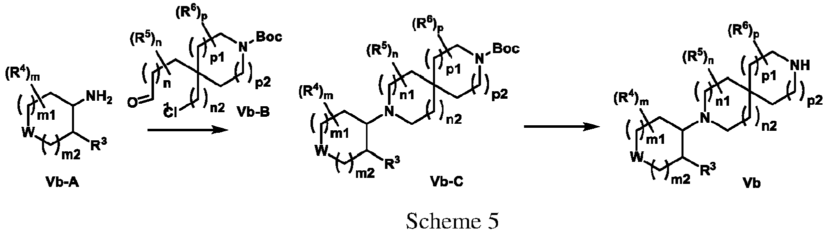

In some cases, the order of carrying out the foregoing reaction schemes may be varied to facilitate the reaction or to avoid unwanted reaction products. The following examples are provided so that the invention might be more fully understood. These examples are illustrative only and should not be construed as limiting the invention in any way.

### Preparation of Intermediates

### Intermediate A

### (S)-2-((1H-pyrrolo[2,3-b]pyridin-5-yl)oxy)-4-(2-(2-(2-cyclopropylphenyl)pyr rolidin-1-yl)-7-azaspiro[3.5]nonan-7-yl)benzoic acid (Intermediate A)

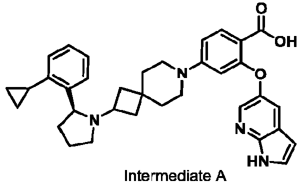

### (R,E)-N-(2-bromobenzylidene)-2-methylpropane-2-sulfinamide (A-1)

A mixture of 2-bromobenzaldehyde (1.0 g, 5.0 mmol), (*R*)-2-methylpropane-2-sulfinamide (0.69 g, 5.6 mmol) and Cs₂CO₃ (1.1 g, 3.5 mmol) in DCM (10 mL) was stirred at 25°C for 12 h. The mixture was concentrated and extracted by EtOAc, the extracts were washed with brine, dried over Na₂SO₄ and concentrated, the residue was purified by column chromatography on silica gel eluting with PE / EtOAc (30:1~ 20:1) to give the title compound (*R,E*)-*N*-(2-bromobenzylidene)-2-methylpropane-2-sulfinamide **(A-1).** MS-ESI (m/z): 288[M + 1]⁺.

### (R)-N-((S)-1-(2-bromophenyl)-3-(1,3-dioxan-2-yl)propyl)-2-methylpropane-2 -sulfinamide (A-2)

To a solution of (*R,E*)-*N*-(2-bromobenzylidene)-2-methylpropane-2-sulfinamide (**A-1**) (1.3 g, 4.5 mmol) in THF (30 mL) was added (2-(1,3-dioxan-2-yl)ethyl)magnesium bromide (0.5 M, 27 mL, 13.0 mmol) at -78°C. The mixture was stirred at -78°C for 2 h. Then the mixture was poured into NH₄Cl solution (50 mL). The mixture was extracted by EtOAc, the extracts were washed with brine, dried over Na₂SO₄ and concentrated, the residue was purified by column chromatography on silica gel eluting with DCM / MeOH(30:1) to give the title compound (*R*)-*N*-((*S*)-1-(2-bromophenyl)-3-(1,3-dioxan-2-yl)propyl)-2-methylpropane-2- sulfinamide **(A-2).** MS-ESI (m/z): 404 [M + 1]⁺.

### (S)-2-(2-bromophenyl)pyrrolidine (A-3)

To a solution of (*R*)-*N*-((*S*)-1-(2-bromophenyl)-3-(1,3-dioxan-2-yl)propyl)-2-methylpropane-2-sulfinamide **(A-2)** (9.5 g, 2.3 mmol) in HCl (4 M, in dioxane) (100 mL) was added HCl (Con, 15 mL) at 25°C. The mixture was stirred at 50°C for 2.5 h. Then added NaBH4 (1.8 g, 4.6 mmol) in portions. Then added MeOH (50 mL).The mixture was stirred at 25°C for 1 h. Then the mixture was poured into ice (200 g). The mixture was extracted by EtOAc, the extracts were washed with brine, dried over Na₂SO₄ and concentrated to give the title compound (S)-2-(2-bromophenyl)pyrrolidine **(A-3).** MS-ESI (m/z): 226 [M + 1]⁺.

### (S)-2-((1H-pyrrolo[2,3-b]pyridin-5-yl)oxy)-4-(2-(2-(2-cyclopropylphenyl)pyr rolidin-1-yl)-7-azaspiro[3.5]nonan-7-yl)benzoic acid (Intermediate A)

(*S*)-2-((1*H*-pyrrolo[2,3-*b*]pyridin-5-yl)oxy)-4-(2-(2-(2-cyclopropylphenyl)pyr rolidin-1-yl)-7-azaspiro[3.5]nonan-7-yl)benzoic acid **(A)** was prepared according to the method described in WO2019210828*.*

### Intermediate B

### (S)-2-((1H-pyrrolo[2,3-b]pyridin-5-yl)oxy)-4-(2-(2-(2-isopropylphenyl)pyrrol idin-1-yl)-7-azaspiro[3.5]nonan-7-yl)benzoic acid (Intermediate B)

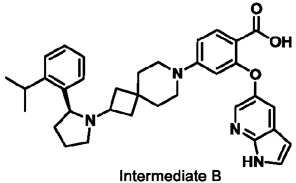

(*S*)-2-((1*H*-pyrrolo[2,3-*b*]pyridin-5-yl)oxy)-4-(2-(2-(2-isopropylphenyl)pyrrol idin-1-yl)-7-azaspiro[3.5]nonan-7-yl)benzoic acid **(B)** was prepared according to the method described in WO2019210828*.*

### Intermediate C

### 6-(((4-hydroxy-4-methylcyclohexyl)methyl)amino)-5-nitropyridine-3-sulfona mide (Intermediate C-a and C-b)

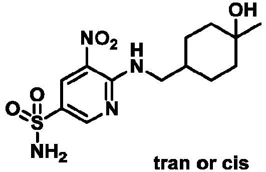

### 6-amino-5-nitropyridine-3-sulfonic acid (C-1)

A solution of 6-aminopyridine-3-sulfonic acid (3.5 g, 20 mmol) in con. H₂SO₄ (14 mL) was heated to 50°C. Then fuming HNO₃ was dropwised slowly. The mixture was stirred at 50°C for 1.5 h. Then the mixture was poured into ice water (50 mL) and filtered to give the title compound 6-amino-5-nitropyridine-3-sulfonic acid **(C-1).** MS-ESI (m/z): 220 [M + 1]⁺.

### 6-hydroxy-5-nitropyridine-3-sulfonic acid (C-2)

To a suspension of 6-amino-5-nitropyridine-3-sulfonic acid **(C-1)** (2.00 g, 9.13 mmol) in 3*N* HCl (35 mL) was added NaNO₂ solution (0.945 mg, 13.7 mmol) at 0°C. The mixture was stirred at 0°C for 1 h. Then warmed to 100°C for 1 h. The mixture was concentrated to give the title compound 6-hydroxy-5-nitropyridine-3-sulfonic acid **(C-2).** MS-ESI (m/z): 221 [M + 1]⁺.

### 6-chloro-5-nitropyridine-3-sulfonyl chloride (C-3)

To a solution of 6-hydroxy-5-nitropyridine-3-sulfonic acid **(C-2)** (2.00 g, 9.13 mmol) in sulfolane (20 mL) was added POCl₃ (10 mL) at 25°C. The mixture was stirred at 110°C for 18 h. Then the mixture was poured into ice (200 g). The mixture was extracted by DCM, the extracts were washed with brine, dried over Na₂SO₄ and concentrated to give the title compound 6-chloro-5-nitropyridine-3-sulfonyl chloride **(C-3).**

### 6-chloro-5-nitropyridine-3-sulfonamide (C-4)

A solution of 6-chloro-5-nitropyridine-3-sulfonyl chloride **(C-3)** (0.701 g, 2.74 mmol) in THF (14 mL) was added 37% ammonium hydroxide at -10°C. The mixture was stirred at -10°C for 0.5 h. Then the mixture was extracted by DCM, the extracts were washed with brine, dried over Na₂SO₄ and concentrated to give the title compound 6-chloro-5-nitropyridine-3-sulfonamide **(C-4).** MS-ESI (m/z): 238[M + 1]⁺.

### benzyl ((1,4-dioxaspiro[4.5]decan-8-yl)methyl)carbamate (C-5)

To a solution of (1,4-dioxaspiro[4.5]decan-8-yl)methanamine (0.092 g, 0.54 mmol) and TEA (0.082 g, 0.81 mmol) in DCM (3 mL) was added benzyl chloroformate (0.111 g, 0.65 mmol) at 0°C. The mixture was stirred at 0°C for 0.5 h. Then the mixture was extracted by DCM, the extracts were washed with brine, dried over Na₂SO₄ and concentrated to give the title compound benzyl ((1,4-dioxaspiro[4.5]decan-8-yl)methyl)carbamate **(C-5).** MS-ESI (m/z): 306 [M + 1]⁺.

### benzyl ((4-oxocyclohexyl)methyl)carbamate (C-6)

To a solution of benzyl ((1.4-dioxaspiro[4.5]decan-8-yl)methyl)carbamate **(C-5)** (0.10 g, 0.33 mmol) in THF (1 mL) was added 2 N HCl (1 mL). The mixture was stirred at 40°C for 2.5 h. Then the mixture was extracted by EA, the extracts were washed with brine, dried over Na₂SO₄ and concentrated to give the title compound benzyl ((4-oxocyclohexyl)methyl)carbamate (**C-6**). MS-ESI (m/z): 262 [M + 1]⁺.

### benzyl ((4-hydroxy-4-methylcyclohexyl)methyl)carbamate (C-7)

To a solution of benzyl ((4-oxocyclohexyl)methyl)carbamate **(C-6)** (0.26 g, 1.0 mmol) in THF (4 mL) was added methyl magnesium bromide (3 M, 1 mL) at -70°C. The mixture was stirred at -70°C for 2.5 h. Then the mixture was quenched with NH₄Cl solution, extracted by EA, the extracts were washed with brine, dried over Na₂SO₄ and concentrated to give the title compound benzyl ((4-hydroxy-4-methylcyclohexyl)methyl)carbamate **(C-7).** MS-ESI (m/z): 278 [M + 1]⁺.

### 4-(aminomethyl)-1-methylcyclohexan-1-ol (C-8)

To a solution of benzyl ((4-hydroxy-4-methylcyclohexyl)methyl)carbamate **(C-7)** (0.223 g, 0.805 mmol) in CH₃OH (8 mL) was added 10% Pd/C (67 mg) at RT. The mixture was stirred at RT for 2 h under H₂ atmosphere. The mixture was filtered and concentrated to give the title compound 4-(aminomethyl)-1-methylcyclohexan-1-ol **(C-8).** MS-ESI (m/z): 144 [M + 1]⁺.

### 6-(((4-hydroxy-4-methylcyclohexyl)methyl)amino)-5-nitropyridine-3-sulfona mide (C-a)

To a solution of 4-(aminomethyl)-1-methylcyclohexan-1-ol **(C-8)** (0.070 g, 0.49 mmol) and 6-chloro-5-nitropyridine-3-sulfonamide **(C-4)** (0.116 g, 0.49 mmol) in CH₃CN (1 mL) was added DIPEA (0.095 g, 0.74 mmol) at RT. The mixture was stirred at RT for 1 h. Then the mixture was extracted by EA, the extracts were washed with brine, dried over Na₂SO₄ and concentrated. The residue was purified by preparative TLC up spot to give title compound 6-(((4-hydroxy-4-methylcyclohexyl)methyl)amino)-5-nitropyridine-3-sulfonamide **(C-a).** MS-ESI (m/z): 345 [M + 1]⁺.

### 6-(((4-hydroxy-4-methylcyclohexyl)methyl)amino)-5-nitropyridine-3-sulfona mide (C-b)

To a solution of 4-(aminomethyl)-1-methylcyclohexan-1-ol **(C-8)** (0.070 g, 0.49 mmol) and 6-chloro-5-nitropyridine-3-sulfonamide **(C-4)** (0.116 g, 0.49 mmol) in CH₃CN (1 mL) was added DIPEA (0.095 g, 0.74 mmol) at RT. The mixture was stirred at RT for 1 h. Then the mixture was extracted by EA, the extracts were washed with brine, dried over Na₂SO₄ and concentrated. The residue was purified by preparative TLC down spot to give title compound 6-(((4-hydroxy-4-methylcyclohexyl)methyl)amino)-5-nitropyridine-3-sulfonamide (**C-b**). MS-ESI (m/z): 345 [M + 1]⁺.

### Intermediate D

### 6-(((4-((diethyl(oxo)-16-sulfanylidene)amino)cyclohexyl)methyl)amino)-5-nit ropyridine-3-sulfonamide (D)

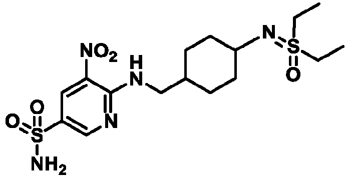

### diethyl(imino)-λ⁶-sulfanone (D-1)

The title compound diethyl(imino)-λ⁶-sulfanone (**D-1**) was prepared according to the method described in WO 2008/141843 (A1)*.*

### ethyl 4-((diethyl(oxo)-λ⁶-sulfanylidene)amino)cyclohexane-1-carboxylate (D-2)

A mixture of diethyl(imino)-λ⁶-sulfanone (**D-1**) (310 mg, 2.56 mmol) and ethyl 4-oxocyclohexane-1-carboxylate (1.30 g, 7.68 mmol) in DCE (10 mL) was stirred at RT for 15 min, and NaBH(OAc)₃ (1.82 g, 8.60 mmol) was added. The resulted mixture was stirred at 0°C overnight. The reaction was quenched by saturated NaHCO₃ aqueous solution (15 mL) and the mixture was extracted with DCM (3 × 30 mL). The extracts were washed with brine (30 mL), dried over Na₂SO₄ and concentrated. The residue was purified by column chromatography on silica gel eluting with DCM/MeOH (15:1) to give title compound ethyl 4-((diethyl(oxo)-λ⁶-sulfanylidene)amino)cyclohexane-1-carboxylate **(D-2).** MS-ESI (m/z): 276 [M + 1]⁺.

### diethyl((4-(hydroxymethyl)cyclohexyl)imino)-λ⁶-sulfanone (D-3)

A mixture of ethyl 4-((diethyl(oxo)-λ⁶-sulfanylidene)amino)cyclohexane-1-carboxylate **(D-2)** (205 mg, 0.745 mmol) and LiAlH₄ (142 mg, 3.73 mmol) in THF (5 mL) was stirred at 50°C for 1 h. The reaction was quenched by 10% NaOH (165 µL) and water (165 µL). The mixture was filtered, and filtrate was concentrated. The residue was purified by column chromatography on silica gel eluting with DCM/MeOH (50:1~12:1) to give title compound diethyl((4-(hydroxymethyl)cyclohexyl)imino)-λ⁶-sulfanone (**D-3**). MS-ESI (m/z): 234 [M + 1]⁺.

### (4-((diethyl(oxo)-λ⁶-sulfanylidene)amino)cyclohexyl)methyl methanesulfonate (D-4)

A mixture of diethyl((4-(hydroxymethyl)cyclohexyl)imino)λ⁶-sulfanone **(D-3)** (46 mg, 0.197 mmol), MsCl (18 µL, 0.237 mmol) and TEA (33 µL, 0.237 mmol) in DCM (3 mL) was stirred at RT for 15 min, The reaction was quenched by saturated NaHCO₃ aqueous solution (15 mL) and the mixture was extracted with DCM (3 × 30 mL). The extracts were dried over Na₂SO₄ and concentrated to give title compound (4-((diethyl(oxo)-λ⁶-sulfanylidene)amino)- cyclohexyl)methyl methanesulfonate **(D-4).** MS-ESI (m/z): 312 [M + 1]⁺.

### ((4-(azidomethyl)cyclohexyl)imino)diethyl-λ⁶-sulfanone (D-5)

A mixture of (4-((diethyl(oxo)-λ⁶-sulfanylidene)amino)cyclohexyl)methyl methanesulfonate **(D-4)** (106 mg, 0.341 mmol) and NaN₃ (44 mg, 0.682 mmol) in DMF (2 mL) was stirred at 70°C for 12 h. The reaction was quenched by water and the mixture was extracted with EtOAc (3 × 30 mL). The extracts were washed with brine (2 × 30 mL), dried over Na₂SO₄ and concentrated. The residue was purified by column chromatography on silica gel eluting with DCM/MeOH (10:1) to give title compound ((4-(azidomethyl)cyclohexyl)imino)diethyl-λ⁶ -sulfanone **(D-5).** MS-ESI (m/z): 259 [M + 1]⁺.

### ((4-(aminomethyl)cyclohexyl)imino)diethyl-λ⁶-sulfanone (D-6)

A mixture of ((4-(azidomethyl)cyclohexyl)imino)diethyl-λ⁶ -sulfanone **(D-5)** (33 mg, 0.128 mmol) and PPh₃ (44 mg, 0.166 mmol) in THF/ H₂O (2/0.4 mL) was stirred at 30°C overnight. The reaction mixture was acidified with HCL (PH= 2) and then extracted with DCM (2 × 30 mL). The extracts were washed with saturated NaHCO₃ aqueous solution (30 mL) and concentrated. The residue was dissolved in DCM/MeOH (5:1), dried over Na₂SO₄, and concentrated. The residue was purified by column chromatography on silica gel eluting with DCM/MeOH (10:1~5:1) to give title compound ((4-(aminomethyl)cyclohexyl)imino)diethyl-λ⁶ -sulfanone **(D-6).** MS-ESI (m/z): 233 [M + 1]⁺.

### 6-(((4-((diethyl(oxo)-l6-sulfanylidene)amino)cyclohexyl)methyl)amino)-5-nitr opyridine-3-sulfonamide (D)

A mixture of ((4-(aminomethyl)cyclohexyl)imino)diethyl-λ⁶-sulfanone (**D-6**) (10.0 mg, 0.043 mmol), 6-chloro-5-nitropyridine-3-sulfonamide **(C-4)** (12.0 mg, 0.052 mmol) and DIPEA (28.0 mg, 0.215 mmol) in acetonitrile (1 mL) was stirred at 80°C overnight. The reaction mixture was cooled to RT and concentrated. The residue was purified by preparative TLC up spot to give title compound 6-(((4-((diethyl(oxo)-λ⁶-sulfanylidene)amino)cyclohexyl)methyl)amino)-5-nitropyridine-3-sulfonamide **(D).** MS-ESI (m/z): 433 [M + 1]⁺.

### Intermediate E

### (R)-2-((1H-pyrrolo[2,3-b]pyridin-5-yl)oxy)-4-(2-(2-(2-cyclopropylphenyl)pyr rolidin-1-yl)-7-azaspiro[3.5]nonan-7-yl)benzoic acid (Intermediate E)

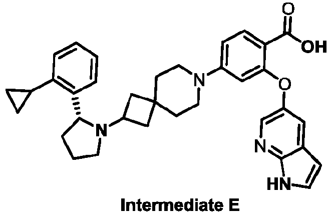

(*R*)-2-((1*H*-pyrrolo[2,3-*b*]pyridin-5-yl)oxy)-4-(2-(2-(2-cyclopropylphenyl)pyr rolidin-1-yl)-7-azaspiro[3.5]nonan-7-yl)benzoic acid **(Intermediate E)** was prepared according to the synthetic method of **Intermediate A** by replacing (*R*)-2-methylpropane-2-sulfinamide with (*S*)-2-methylpropane-2-sulfinamide. MS-ESI (m/z): 563 [M + 1]⁺.

### Intermediate F

### (S)-3-(4-hydroxy-4-methylcyclohexyl)-5-nitro-2,3-dihydrobenzo[b][1,4]oxath iine-7-sulfonamide (Intermediate F)

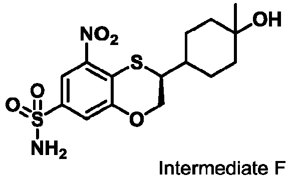

### (R)-2-((tert-butoxycarbonyl)amino)-2-(4-hydroxycyclohexyl)acetic acid (F-1)

(*R*)-2-((*tert*-butoxycarbonyl)amino)-2-(4-hydroxycyclohexyl)acetic acid **(F-1)** was prepared according to the method described in US2005/234065*.*

### (R)-2-bromo-2-(4-hydroxycyclohexyl)acetic acid (F-2)

A mixture of (*R*)-2-((*tert*-butoxycarbonyl)amino)-2-(4-hydroxycyclohexyl)acetic acid (**F-1**) (7.0 g, 25.6 mmol) in 48% HBr aqueous solution (56 mL) was stirred at room temperature for 0.5 hours. The mixture was cooled to 0°C, and NaNO₂ (2.82 g, 40.9 mmol) in water (28 mL) was added, and then the reaction was slowly raised to room temperature and stirred overnight. The reaction mixture was diluted with saturated brine (50 mL) and extracted with EtOAc (2 × 70 mL). The extracts were washed with saturated aqueous NaHSO₃ (50 mL) and 15% saturated brine (50 mL), dried over Na₂SO₄ and concentrate. The residue was purified with dichloromethane (10 mL), filtered and dried to give title compound (*R*)-2-bromo-2-(4-hydroxycyclohexyl)acetic acid **(F-2).** MS-ESI (m/z): 235, 237 [M - 1]⁻.

### (R)-2-bromo-2-(4-oxocyclohexyl)acetic acid (F-3)

To a solution of (R)-2-bromo-2-(4-hydroxycyclohexyl)acetic acid (**F-2**) (1.9 g, 8.0 mmol) in tetrahydrofuran (6 mL) and dichloromethane (80 mL) was added Dess-Martin oxidant (4.1 g, 9.7 mmol). The reaction mixture was stirred at room temperature for overnight. The mixture was filtered, filter cake was washed the with dichloromethane (30 mL), and filtrate was concentrated to give the crude product of title compound (*R*)-2-bromo-2-(4-oxocyclohexyl)acetic acid (**F-3**) which was used directly for next step.

### (R)-2-bromo-2-(1,4-dioxaspiro[4.5]decan-8-yl)acetic acid (F-4)

To a solution of (R)-2-bromo-2-(4-oxocyclohexyl)acetic acid (**F-3**) (6.0 g, 10.1 mmol) in toluene (90 mL) was added ethylene glycol (0.038 g, 0.36 mmol), p-toluene sulfonic acid (150 mg) and 4A molecular sieve (6 g). The mixture was stirred at 100°C for 3-4 hours. The mixture was cooled to room temperature and concentrate. The residue was purified by column chromatography on silica gel eluting with EtOAc / PE (1:5 ~ 1:1) to give title compound (*R*)-2-bromo-2-(1,4-dioxaspiro[4.5]decan-8-yl)acetic acid **(F-4).** MS-ESI (m/z): 277, 279 [M - 1]⁻.

### methyl (R)-2-bromo-2-(1,4-dioxaspiro[4.5]decan-8-yl)acetate (F-5)

To a solution of (*R*)-2-bromo-2-(1,4-dioxaspiro[4.5]decan-8-yl)acetic acid **(F-4)** (250 mg, 0.896 mmol) in *N,N*-dimethylacetamide (2 mL) was added K₂CO₃ (150 mg, 1.08 mmol) and CH₃I (191 mg, 1.34 mmol). The mixture was stirred at RT for 1-2 h. The reaction was quenched with water (20 mL) and extracted with EtOAc (3× 20 mL). The extracts were washed with water (10 mL) and brine (10 mL), dried with Na₂SO₄ and concentrated to give the crude product of methyl (*R*)-2-bromo-2-(1,4-dioxaspiro[4.5]decan-8-yl)acetate **(F-5),** which was used for next step directly. MS-ESI (m/z): 293, 295 [M + 1]⁺.

### 3-bromo-4-chloro-5-nitrobenzenesulfonamide (F-6)

3-bromo-4-chloro-5-nitrobenzenesulfonamide **(F-6)** was prepared according to the method described in WO2018/192462*.*

### methyl (S)-2-((2-bromo-6-nitro-4-sulfamoylphenyl)thio)-2-(1,4-dioxaspiro[4.5]decan-8-yl)acetate (F-7)

To a solution of 3-bromo-4-chloro-5-nitrobenzenesulfonamide **(F-6)** (700 mg, 2.22 mmol) in DMF (7 mL) was added Na₂S (208 mg, 2.67 mmol) and 18-crown-6 (70 mg). The mixture was stirred at RT for overnight. And then methyl (*R*)-2-bromo-2-(1,4-dioxaspiro[4.5]decan-8-yl)acetate **(F-5)** (220 mg, 0.75 mmol) was added. The mixture was stirred at 40°C overnight. The reaction was quenched with water (40 mL) and the mixture was extracted with EtOAc (3 × 20 mL). The organic phase was washed with water (2 × 10 mL) and brine (10 mL), dried over Na₂SO₄, and concentrated. The residue was purified by column chromatography on silica gel eluting with dichloromethane/acetonitrile (20:1~10:1) to give title compound methyl (*S*)-2-((2-bromo-6-nitro-4-sulfamoylphenyl)thio)-2-(1,4- dioxaspiro[4.5]decan-8-yl)acetate **(F-7).** MS-ESI (m/z): 525, 527 [M + 1]⁺.

### (S)-3-bromo-4-((2-hydroxy-1-(1,4-dioxaspiro[4.5]decan-8-yl)ethyl)thio)-5-ni trobenzenesulfonamide (F-8)

To a solution of methyl (*S*)-2-((2-bromo-6-nitro-4-sulfamoylphenyl)thio)-2-(1,4- dioxaspiro[4.5]decan-8-yl)acetate **(F-7)** (90 mg, 0.086 mmol) in THF (9 mL) was added DIBAL-H in hexane solution (4 mL) dropwise at -60°C under N₂ atmosphere. The mixture was stirred at -60 ~ -10°C for 2-3 h. The reaction was quenched with methanol (2 mL) and the mixture was diluted with DCM (10 mL) at -60°C. The reaction was slowly raised to room temperature and concentrated. The residue was purified by column chromatography on silica gel eluting with dichloromethane/acetonitrile (3:1) to give title compound (*S*)-3-bromo-4-((2-hydroxy-1-(1,4-dioxaspiro[4.5]decan-8-yl)ethyl)thio)-5-nitrobenzenesulfo namide **(F-8).** MS-ESI (m/z): 495, 497 [M - 1]⁻.

### (S)-5-nitro-3-(1,4-dioxaspiro[4.5]decan-8-yl)-2,3-dihydrobenzo[b][1,4]oxath iine-7-sulfonamide (F-9)

To a solution of (*S*)-3-bromo-4-((2-hydroxy-1-(1,4-dioxaspiro[4.5]decan-8-yl)ethyl)thio)-5-nitrobenzenesulfonamide **(F-8)** (35 mg, 0.0704 mmol) in dioxane and toluene (1.1 mL/ 3.3 mL) was added Pd(OAc)₂ (3.2 mg, 0.014 mmol), Xantphos (0.028 mg, 0.0704 mmol) and K₂CO₃ (19.5 mg, 0.14 mmol) under N₂ atmosphere. The mixture was stirred at 90°C for 0.5-1 h. The mixture was cooled to RT and filtered through Celite, filtrate was concentrated. The residue was purified by preparative TLC (DCM/acetonitrile = 3:1) to give title compound (*S*)-5-nitro-3-(1,4-dioxaspiro[4.5]decan-8-yl)-2,3-dihydrobenzo[*b*][1,4]oxathiine-7-sulfonamide **(F-9).** MS-ESI (m/z): 415 [M - 1]⁻.

### (S)-5-nitro-3-(4-oxocyclohexyl)-2,3-dihydrobenzo[b][1,4]oxathiine-7-sulfona mide (F-10)

To a solution of (S)-5-nitro-3-(1,4-dioxaspiro[4.5]decan-8-yl)-2,3-dihydrobenzo[*b*][1,4]oxathiine-7-sulfonamide **(F-9)** (35 mg, 0.0704 mmol) in THF (2.5 mL) was added HCl (3 N, 2 mL). The mixture was stirred at RT for 1-2 h. The mixture was adjusted with saturated aqueous Na₂CO₃ to pH = 7. The mixture was extracted with EtOAc (3 × 10 mL). The extracts were washed with water (5 mL) and brine (5 mL), dried with Na₂SO₄ and concentrated. The residue was purified by preparative TLC (PE/ EtOAc = 1:1) to give title compound (*S*)-5-nitro-3-(4-oxocyclohexyl)-2,3-dihydrobenzo[b][1,4]oxathiine-7-sulfonamide (**F-10**). MS-ESI (m/z): 371 [M - 1]⁻.

### (S)-3-(4-hydroxy-4-methylcyclohexyl)-5-nitro-2,3-dihydrobenzo[b][1,4]oxath iine-7-sulfonamide (F-a and F-b)

To a solution of (*S*)-5-nitro-3-(4-oxocyclohexyl)-2,3-dihydrobenzo[*b*][1,4]-oxathiine-7-sulfonamide (**F-10**) (12.5 mg, 0.086 mmol) in THF (4 mL) was slowly added dropwise methyl lithium ether solution (0.1 mL) at -78°C under N₂ atmosphere. The reaction was stirred at -78°C for 0.5 hour. The reaction was quenched with saturated ammonium chloride aqueous solution (2 mL) at -78°C and extracted with EtOAc (3 × 10 mL). The extracts were washed with water (5 mL) and brine (5 mL), dried with Na₂SO₄ and concentrated. The residue was purified by preparative TLC (DCM/acetonitrile = 2:1) upper or lower spot to give title compound (*S*)-3-(4-hydroxy-4-methylcyclohexyl)-5-nitro-2,3-dihydrobenzo[*b*][1,4]oxathiine-7-sulfonamide **(F-a and F-b).** MS-ESI (m/z): 387 [M - 1]⁻.

### Example A-1a

### (S)-2-((1H-pyrrolo[2,3-b]pyridin-5-yl)oxy)-4-(2-(2-(2-cyclopropylphenyl)pyr rolidin-1-yl)-7-azaspiro[3.5]nonan-7-yl)-N-((6-(((4-hydroxy-4-methylcyclohexyl)methyl)amin o)-5-nitropyridin-3-yl)sulfonyl)benzamide (A-1a)

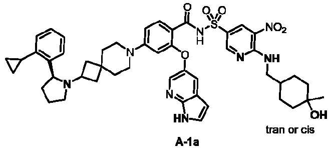

A mixture of 6-(((4-hydroxy-4-methylcyclohexyl)methyl)amino)-5-nitropyridine-3-sulfonamide **(C-a)** (4 mg, 0.012 mmol), EDCI (57 mg, 0.030 mmol) and DMAP (36 mg, 0.030 mmol) in DCM (1.5 mL) was stirred at RT for 15 min, and then a mixture of (*S*)-2-((1*H*-pyrrolo[2,3-*b*]pyridin-5-yl)oxy)-4-(2-(2-(2-cyclopropylphenyl)pyrrolidin-1-yl)-7-a zaspiro[3.5]nonan-7-yl)benzoic acid **(Intermediate A)** (5 mg, 0.009 mmol) and TEA (4 mg, 0.04 mmol) in DCM (0.5 mL) was added. The resulted mixture was stirred at RT for overnight and concentrated. The residue was purified by preparative TLC to give title compound (*S*)-2-((1*H*-pyrrolo[2,3-*b*]pyridin-5-yl)oxy)-4-(2-(2-(2-cyclopropylphenyl)pyrrolidin-1-yl)-7-a zaspiro[3.5]nonan-7-yl)-*N*-((6-(((4-hydroxy-4-methylcyclohexyl)methyl)amino)-5-nitropyridi n-3-yl)sulfonyl)benzamide (**A-1a**). MS-ESI (m/z): 889 [M + 1]⁺.

### Example A-1b

### (S)-2-((1H-pyrrolo[2,3-b]pyridin-5-yl)oxy)-4-(2-(2-(2-cyclopropylphenyl)pyr rolidin-1-yl)-7-azaspiro[3.5]nonan-7-yl)-N-((6-(((4-hydroxy-4-methylcyclohexyl)methyl)amin o)-5-nitropyridin-3-yl)sulfonyl)benzamide (A-1b)

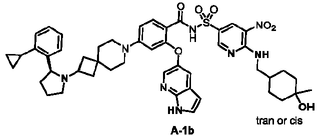

A mixture of 6-(((4-hydroxy-4-methylcyclohexyl)methyl)amino)-5-nitropyridine-3-sulfonamide (**C-b**) (4 mg, 0.012 mmol), EDCI (57 mg, 0.030 mmol) and DMAP (36 mg, 0.030 mmol) in DCM (1.5 mL) was stirred at RT for 15 min, and then a mixture of (*S*)-2-((1*H*-pyrrolo[2,3-*b*]pyridin-5-yl)oxy)-4-(2-(2-(2-cyclopropylphenyl)pyrrolidin-1-yl)-7-a zaspiro[3.5]nonan-7-yl)benzoic acid (**Intermediate A**) (5 mg, 0.009 mmol) and TEA (4 mg, 0.04 mmol) in DCM (0.5 mL) was added. The resulted mixture was stirred at RT for overnight and concentrated. The residue was purified by preparative TLC to give title compound (*S*)-2-((1*H*-pyrrolo[2,3-*b*]pyridin-5-yl)oxy)-4-(2-(2-(2-cyclopropylphenyl)pyrrolidin-1-yl)-7-a zaspiro[3.5]nonan-7-yl)-*N*-((6-(((4-hydroxy-4-methylcyclohexyl)methyl)amino)-5-nitropyridi n-3-yl)sulfonyl)benzamide **(A-1b).** MS-ESI (m/z): 889 [M + 1]⁺.

### Example A-1c

### (R)-2-((1H-pyrrolo[2,3-b]pyridin-5-yl)oxy)-4-(2-(2-(2-cyclopropylphenyl)pyr rolidin-1-yl)-7-azaspiro[3.5]nonan-7-yl)-N-((6-(((4-hydroxy-4-methylcyclohexyl)methyl)amin o)-5-nitropyridin-3-yl)sulfonyl)benzamide (A-1c)

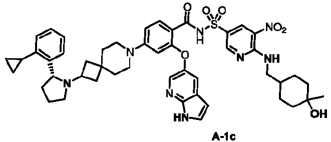

(*R*)-2-((1*H*-pyrrolo[2,3-*b*]pyridin-5-yl)oxy)-4-(2-(2-(2-cyclopropylphenyl)pyr rolidin-1-yl)-7-azaspiro[3.5]nonan-7-yl)-*N*-((6-(((4-hydroxy-4-methylcyclohexyl)methyl)ami no)-5-nitropyridin-3-yl)sulfonyl)benzamide **(A-1c)** was prepared according to the synthetic method of **A-1a** by replacing (*S*)-2-((1*H*-pyrrolo[2,3-*b*]pyridin-5-yl)oxy)-4-(2-(2-(2-cyclopropylphenyl)-pyrrolidin-1-yl)-7-azaspiro[3.5]nonan-7-yl)benzoic acid **(Intermediate A)** with (*R*)-2-((1*H*-pyrrolo[2,3-b]pyridin-5-yl)oxy)-4-(2-(2-(2-cyclopropylphenyl)pyrrolidin-1-yl)-7-a zaspiro[3.5]nonan-7-yl)benzoic acid **(Intermediate E).** MS-ESI (m/z): 978 [M + 1]⁺.

### Example A-2a

### (S)-2-((1H-pyrrolo[2,3-b]pyridin-5-yl)oxy)-4-(2-(2-(2-cyclopropylphenyl)pyr rolidin-1-yl)-7-azaspiro[3.5]nonan-7-yl)-N-((6-(((4-((diethyl(oxo)-λ⁶-sulfanylidene)amino)cyc lohexyl)methyl)amino)-5-nitropyridin-3-yl)sulfonyl)benzamide (A-2a)

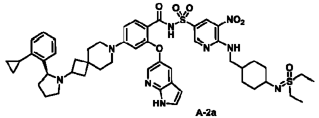

(*S*)-2-((1*H*-pyrrolo[2,3-*b*]pyridin-5-yl)oxy)-4-(2-(2-(2-cyclopropylphenyl)pyr rolidin-1-yl)-7-azaspiro[3.5]nonan-7-yl)-*N*-((6-(((4-((diethyl(oxo)-λ⁶-sulfanylidene)amino)cyc lohexyl)methyl)amino)-5-nitropyridin-3-yl)sulfonyl)benzamide (**A-2a**) was prepared according to the synthetic method of **A-1a** by replacing 6-(((4-hydroxy-4-methylcyclohexyl)methyl)amino)-5- nitropyridine-3-sulfonamide **(C-a)** with 6-(((4-((diethyl(oxo)-λ⁶-sulfanylidene)amino)cyclohexyl)methyl)amino)-5-nitropyridine-3-sulfonamide **(Intermediate D).** MS-ESI (m/z): 978 [M + 1]⁺.

### Example A-2b

### (R)-2-((1H-pyrrolo[2,3-b]pyridin-5-yl)oxy)-4-(2-(2-(2-cyclopropylphenyl)pyr rolidin-1-yl)-7-azaspiro[ 3.5]nonan-7-yl)-N-((6-(((4-((diethyl(oxo)-λ⁶-sulfanylidene)amino)cyc lohexyl)methyl)amino)-5-nitropyridin-3-yl)sulfonyl)benzamide (A-2b)

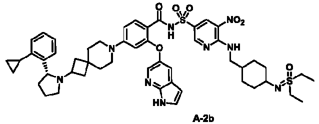

(*R*)-2-((1*H*-pyrrolo[2,3-*b*]pyridin-5-yl)oxy)-4-(2-(2-(2-cyclopropylphenyl)pyr rolidin-1-yl)-7-azaspiro[3.5]nonan-7-yl)-*N*-((6-(((4-((diethyl(oxo)-λ⁶-sulfanylidene)amino)cyc lohexyl)methyl)amino)-5-nitropyridin-3-yl)sulfonyl)benzamide (**A-2b**) was prepared according to the synthetic method of **A-1a** by replacing 6-(((4-hydroxy-4-methylcyclohexyl)methyl)amino)-5- nitropyridine-3-sulfonamide **(C-a)** and (*S*)-2-((1*H*-pyrrolo[2,3-*b*]pyridin-5-yl)oxy)-4-(2-(2-(2-cyclopropylphenyl)pyrrolidin-1-yl)-7-a zaspiro[3.5]nonan-7-yl)benzoic acid **(Intermediate A)** with 6-(((4-((diethyl(oxo)-λ⁶-sulfanylidene)amino)cyclohexyl)methyl)amino)-5-nitropyridine-3-sulfonamide **(Intermediate D)** and (*R*)-2-((1*H*-pyrrolo[2,3-b]pyridin-5-yl)oxy)-4-(2-(2-(2-cyclopropylphenyl)pyrrolidin-1-yl)-7-azaspiro[3.5]nonan-7-yl)benzoic acid **(Intermediate E).** MS-ESI (m/z): 978 [M + 1]⁺.

Following essentially the same procedures described for Examples A-1 ~ A-2 or using similar synthetic strategies or methods. Examples A-3 ~ A-33 listed in Table 1 were prepared using appropriate intermediates, which can be readily synthesized by methods known in the art, and sequential modifications as necessary. The structures and names of Examples A-3 ~ A-33 are given in table 1.

**Table 1**

| EXAMPLE | STRUCTURE | NAME | DATA |
|---|---|---|---|
| A-3a | 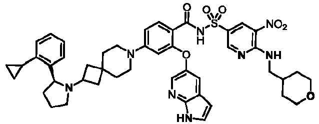 | (*S*)-2-((1*H*-pyrrolo[2,3-*b*]pyridin-5-yl)oxy)-4-(2-(2-(2-cyclopropyl phenyl)pyrrolidin-1-yl)-7-azaspir o[3.5]nonan-7-yl)-*N*-((5-nitro-6-( ((tetrahydro-2*H*-pyran-4-yl)meth yl)amino)pyridin-3-yl)sulfonyl)b enzamide | MS-ESI (m/z): 861 [M + 1]⁺ |
| A-3b | 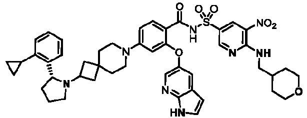 | (*R*)-2-((1*H*-pyrrolo[2,3-*b*]pyridin-5-yl)oxy)-4-(2-(2-(2-cyclopropyl phenyl)pyrrolidin-1-yl)-7-azaspiro [3.5]nonan-7-yl)-*N*-((5-nitro-6-( ((tetrahydro-2*H*-pyran-4-yl)meth yl)amino)pyridin-3-yl)sulfonyl) benzamide | MS-ESI (m/z): 861 [M + 1]⁺ |
| A-4a | 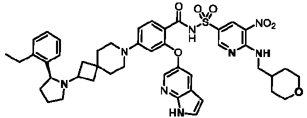 | (*S*)-2-((1*H*-pyrrolo[2,3-*b*]pyridin-5-yl)oxy)-4-(2-(2-(2-ethylphenyl) pyrrolidin-1-yl)-7-azaspiro[3.5]n onan-7-yl)-*N*-((5-nitro-6-(((tetrah ydro-2*H*-pyran-4-yl)methyl)amin o)pyridin-3-yl)sulfonyl)benzamide | MS-ESI (m/z): 849 [M + 1]⁺ |
| A-4b | 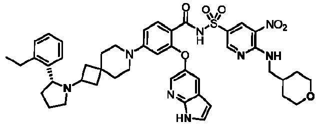 | (*R*)-2-((1*H*-pyrrolo[2,3-*b*]pyridin-5-yl)oxy)-4-(2-(2-(2-ethylphenyl) pyrrolidin-1-yl)-7-azaspiro[3.5]n onan-7-yl)-*N*-((5-nitro-6-(((tetrah ydro-2*H*-pyran-4-yl)methyl)amin o)pyridin-3-yl)sulfonyl)benzamide | MS-ESI (m/z): 849 [M + 1]⁺ |
| A-5a | 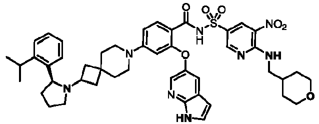 | (*S*)-2-((1*H*-pyrrolo[2,3-*b*]pyridin-5-yl)oxy)-4-(2-(2-(2-isopropylph enyl)pyrrolidin-1-yl)-7-azaspiro[ 3.5]nonan-7-yl)-*N*-((5-nitro-6-((( tetrahydro-2*H*-pyran-4-yl)methyl) amino)pyridin-3-yl)sulfonyl)benz amide | MS-ESI (m/z): 863 [M + 1]⁺ |
| A-5b | 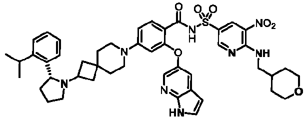 | (R)-2-((1H-pyrrolo[2,3-b]pyridin-5-yl)oxy)-4-(2-(2-(2-isopropylph enyl)pyrrolidin-1-yl)-7-azaspiro[ 3.5]nonan-7-yl)-N-((5-nitro-6-((( tetrahydro-2H-pyran-4-yl)methyl) amino)pyridin-3-yl)sulfonyl)benz amide | MS-ESI (m/z): 863 [M + 1]⁺ |
| A-6a | 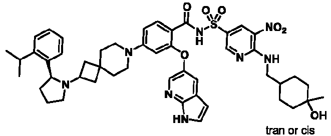 | (*S*)-2-((1*H*-pyrrolo[2,3-*b*]pyridin-5-yl)oxy)-*N*-((6-(((4-hydroxy-4-methylcyclohexyl)methyl)amino) -5-nitropyridin-3-yl)sulfonyl)-4-( 2-(2-(2-isopropylphenyl)pyrrolidin -1-yl)-7-azaspiro[3.5]nonan-7-yl )benzamide | MS-ESI (m/z): 891 [M + 1]⁺ |
| A-6b | 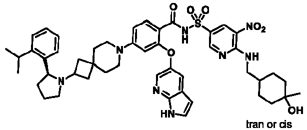 | (*S*)-2-((1*H*-pyrrolo[2,3-*b*]pyridin-5-yl)oxy)-*N*-((6-(((4-hydroxy-4-methylcyclohexyl)methyl)amino) -5-nitropyridin-3-yl)sulfonyl)-4-( 2-(2-(2-isopropylphenyl)pyrrolidin -1-yl)-7-azaspiro[3.5]nonan-7-yl )benzamide | MS-ESI (m/z): 891 [M + 1]⁺ |
| A-7a | 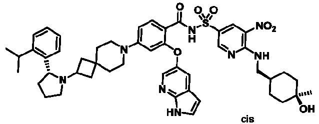 | 2-((1*H*-pyrrolo[2,3-*b*]pyridin-5-yl )oxy)-*N*-((6-((((1*s,*4*s*)-4-hydroxy-4-methylcyclohexyl)methyl)amin o)-5-nitropyridin-3-yl)sulfonyl)-4 -(2-((*R*)-2-(2-isopropylphenyl)pyr rolidin-1-yl)-7-azaspiro[3.5]nonan -7-yl)benzamide | MS-ESI (m/z): 891 [M + 1]⁺ |
| A-7b | 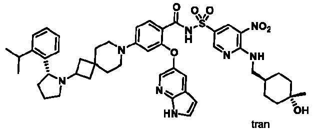 | 2-((1*H*-pyrrolo[2,3-*b*]pyridin-5-yl )oxy)-*N*-((6-((((1*r*,4*r*)-4-hydroxy-4-methylcyclohexyl)methyl)amin o)-5-nitropyridin-3-yl)sulfonyl)-4 -(2-((*R*)-2-(2-isopropylphenyl)pyr rolidin-1-yl)-7-azaspiro[3.5]nona n-7-vl)benzamide | MS-ESI (m/z): 891 [M + 1]⁺ |
| A-8a | 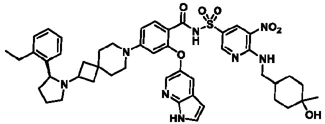 | (*S*)-2-((1*H*-pyrrolo[2,3-*b*]pyridin-5-yl)oxy)-4-(2-(2-(2-ethylphenyl) pyrrolidin-1-yl)-7-azaspiro[3.5]n onan-7-yl)-*N*-((6-(((4-hydroxy-4-methylcyclohexyl)methyl)amino) -5-nitropyridin-3-yl)sulfonyl)ben zamide | MS-ESI (m/z): 877 [M + 1]⁺ |
| A-8b | 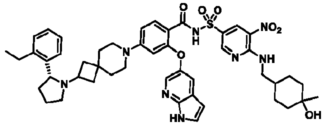 | (*R*)-2-((1*H*-pyrrolo[2,3-*b*]pyridin-5-yl)oxy)-4-(2-(2-(2-ethylphenyl) pyrrolidin-1-yl)-7-azaspiro[3.5]n onan-7-yl)-*N*-((6-(((4-hydroxy-4-methylcyclohexyl)methyl)amino) -5-nitropyridin-3-yl)sulfonyl)ben zamide | MS-ESI (m/z): 877 [M + 1]⁺ |
| A-9 | 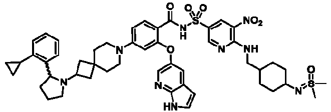 | 2-((1*H*-pyrrolo[2,3-*b*]pyridin-5-yl )oxy)-4-(2-(2-(2-cyclopropylphen yl)pyrrolidin-1-yl)-7-azaspiro[3.5 ]nonan-7-yl)-*N*-((6-(((4-((dimeth yl(oxo)-λ⁶-sulfanylidene)amino)c yclohexyl)methyl)amino)-5-nitro pyridin-3-yl)sulfonyl)benzamide | MS-ESI (m/z): 950 [M + 1]⁺ |
| A-9a | 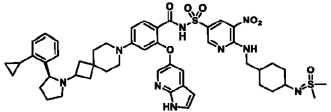 or 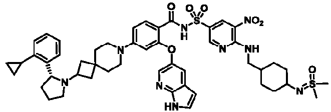 | (R or S)-2-((1H-pyrrolo[2,3-b]pyridin-5-yl)oxy)-4-(2-(2-(2-cyclopropyl phenyl)pyrrolidin-1-yl)-7-azaspir o[3.5]nonan-7-yl)-N-((6-(((4-((di methyl(oxo)-λ⁶-sulfanylidene)amino)cyclohex yl)methyl)amino)-5-nitropyridin-3-yl)sulfonyl)benzamide | MS-ESI (m/z): 950 [M + 1]⁺ |
| A-9b | | | |
| A-10a | 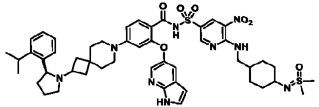 | (*S*)-2-((1*H*-pyrrolo[2,3-*b*]pyridin-5-yl)oxy)-*N*-((6-(((4-((dimethyl(o xo)-λ⁶-sulfanylidene)amino)cyclo hexyl)methyl)amino)-5-nitropyri din-3-yl)sulfonyl)-4-(2-(2-(2-isop ropylphenyl)pyrrolidin-1-yl)-7-az aspiro[3.5]nonan-7-yl)benzamide | MS-ESI (m/z): 952 [M + 1]⁺ |
| A-10b | 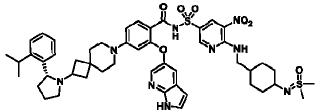 | (*R*)-2-((1*H*-pyrrolo[2,3-*b*]pyridin-5-yl)oxy)-*N*-((6-(((4-((dimethyl( oxo)-λ⁶-sulfanylidene)amino)cyclo hexyl)methyl)amino)-5-nitropyri din-3-yl)sulfonyl)-4-(2-(2-(2-isop ropylphenyl)pyrrolidin-1-yl)-7-az aspiro[3.5]nonan-7-vl)benzamide | MS-ESI (m/z): 952 [M + 1]⁺ |
| A-11 | 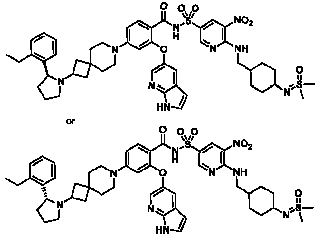 | (*R* or *S*)-2-((1*H*-pyrrolo[2,3-*b*]pyridin-5 -yl)oxy)-*N*-((6-(((4-((dimethyl(oxo )-λ⁶-sulfanylidene)amino)cycloh exyl)methyl)amino)-5-nitropyridi n-3-yl)sulfonyl)-4-(2-(2-(2-ethylp henyl)pyrrolidin-1-yl)-7-azaspiro [3.5]nonan-7-yl)benzamide | MS-ESI (m/z): 938 [M + 1]⁺ |
| A-12 | 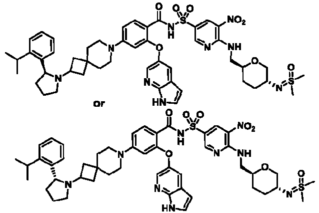 | (*R* or *S*)-2-((1*H*-pyrrolo[2,3-*b*]pyridin-5 -yl)oxy)-*N*-((6-((((2*S*,5*R*)-5-((dim ethyl(oxo)-λ⁶-sulfanylidene)amin o)tetrahydro-2H-pyran-2-yl)meth yl)amino)-5-nitropyridin-3-yl)sul fonyl)-4-(2-(2-(2-isopropylphenyl )pyrrolidin-1-yl)-7-azaspiro[3.5]n onan-7-yl)benzamide | MS-ESI (m/z): 954 [M + 1]⁺ |
| A-13a | 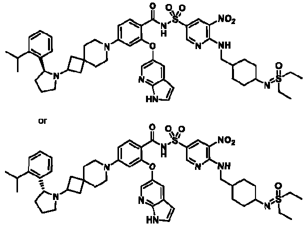 | (*R* or *S*)-2-((1*H*-pyrrolo[2,3-*b*]pyridin-5 -yl)oxy)-*N*-((6-(((4-((diethyl(oxo) -λ⁶-sulfanylidene)amino)cyclohex yl)methyl)amino)-5-nitropyridin-3-yl)sulfonyl)-4-(2-(2-(2-isoprop ylphenyl)pyrrolidin-1-yl)-7-azasp iro[3.5]nonan-7-yl)benzamide | MS-ESI (m/z): 980 [M + 1]⁺ |
| A-13b | | | |
| A-14 | 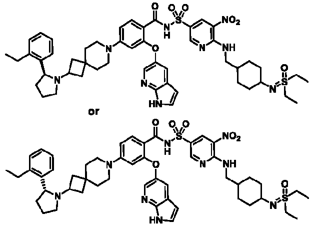 | (*R* or *S*)-2-((1*H*-pyrrolo[2,3-*b*]pyridin-5 -yl)oxy)-*N*-((6-(((4-((diethyl(oxo) -λ⁶-sulfanylidene)amino)cyclohex yl)methyl)amino)-5-nitropyridin-3-yl)sulfonyl)-4-(2-(2-(2-ethylph enyl)pyrrolidin-1-yl)-7-azaspiro[ 3.5]nonan-7-yl)benzamide | MS-ESI (m/z): 966 [M + 1]⁺ |
| A-15a | 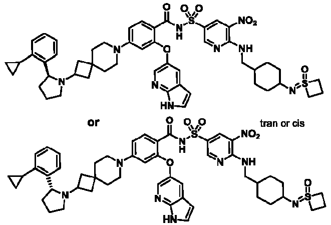 | (*R* or *S*)-2-((1*H*-pyrrolo[2,3-*b*]pyridin-5 -yl)oxy)-4-(2-(2-(2-cyclopropylp henyl)pyrrolidin-1-yl)-7-azaspiro [3.5]nonan-7-yl)-*N*-((5-nitro-6-((( 4-((1-oxido-1λ⁶-thietan-1-ylidene )amino)cyclohexyl)methyl)amino )pyridin-3-yl)sulfonyl)benzamide | MS-ESI (m/z): 962 [M + 1]⁺ |
| A-15b | | | |
| A-16 | 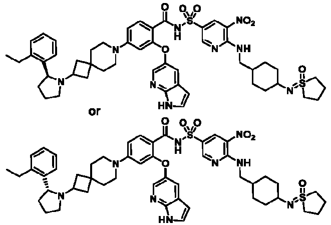 | (*R* or *S*)-2-((1*H*-pyrrolo[2,3-*b*]pyridin-5 -yl)oxy)-4-(2-(2-(2-ethylphenyl)p yrrolidin-1-yl)-7-azaspiro[3.5]no nan-7-yl)-*N*-((5-nitro-6-(((4-((1-o xidotetrahydro-1λ⁶-thiophen-1-yli dene)amino)cyclohexyl)methyl) amino)pyridin-3-yl)sulfonyl)benza mide | MS-ESI (m/z): 964 [M + 1]⁺ |
| A-17a | 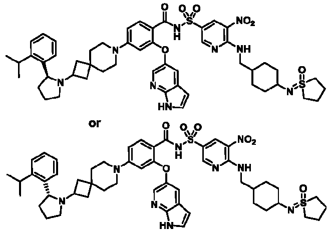 | (*R* or *S*)-2-((1*H*-pyrrolo[2,3-*b*]pyridin-5 -yl)oxy)-4-(2-(2-(2-isopropylphe nyl)pyrrolidin-1-yl)-7-azaspiro[3. 5]nonan-7-yl)-*N*-((5-nitro-6-(((4-( (1-oxidotetrahydro- 1λ⁶-thiophen-1-ylidene)amino)cyclohexyl)met hyl)amino)pyridin-3-yl)sulfonyl) benzamide | MS-ESI (m/z): 978 [M + 1]⁺ |
| A-17b | | | |
| A-18a | 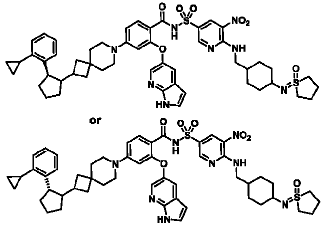 | 2-((1*H*-pyrrolo[2,3-*b*]pyridin-5-yl )oxy)-4-(2-((2 *R* or *S*)-2-(2-cyclopropylphenyl)pyrrol idin-1-yl)-7-azaspiro[3.5]nonan-7 -yl)*-N*-((5-nitro-6-(((4-((1-oxidot etrahydro-1λ⁶-thiophen-1-ylidene )amino)cyclohexyl)methyl)amino )pyridin-3-yl)sulfonyl)benzamide | MS-ESI (m/z): 976 [M + 1]⁺ |
| A-18b | | | |
| A-19 | 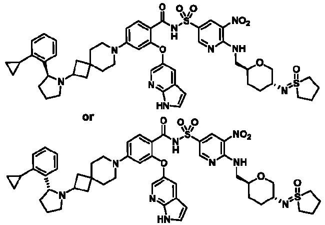 | 2-((1*H*-pyrrolo[2,3-*b*]pyridin-5-yl )oxy)-4-(2-((*R* or *S*)-2-(2-cyclopropylphenyl)pyrrol idin-1-yl)-7-azaspiro[3.5]nonan-7 -yl)-*N*-((5-nitro-6-((((2*S*,5*R*)-5-(( 1-oxidotetrahydro-1λ⁶-thiophen-1 -ylidene)amino)tetrahydro-2*H*-py ran-2-yl)methyl)amino)pyridin-3-yl)sulfonyl)benzamide | MS-ESI (m/z): 978 [M + 1]⁺ |
| A-20 | 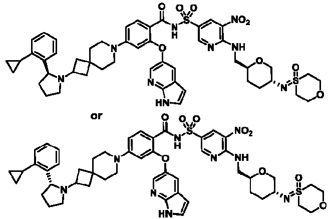 | 2-((1*H*-pyrrolo[2,3-*b*]pyridin-5-yl )oxy)-4-(2-((*R* or *S*)-2-(2-cyclopropylphenyl)pyrrol idin-1-yl)-7-azaspiro[3.5]nonan-7 -yl)-*N*-((5-nitro-6-((((2*S*,5*R*)-5-(( 4-oxido-1,4λ⁶-oxathian-4-ylidene )amino)tetrahydro-2*H*-pyran-2-yl )methyl)amino)pyridin-3-yl) sulfonyl)benzamide | MS-ESI (m/z): 994 [M + 1]⁺ |
| A-21a | 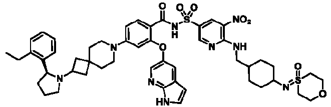 | (*S*)-2-((1*H*-pyrrolo[2,3-*b*]pyridin-5-yl)oxy)-4-(2-(2-(2-ethylphenyl) pyrrolidin-1-yl)-7-azaspiro[3.5] nonan-7-yl)-*N*-((5-nitro-6-(((4-((4-oxido-1,4λ⁶-oxathian-4-ylidene) amino)cyclohexyl)methyl)amino) pyridin-3-yl)sulfonyl)benzamide | MS-ESI (m/z): 980 [M + 1]⁺ |
| A-21b | 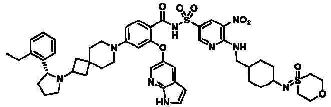 | (*R*)-2-((1*H*-pyrrolo[2,3-*b*]pyridin-5-yl)oxy)-4-(2-(2-(2-ethylphenyl) pyrrolidin-1-yl)-7-azaspiro[3.5]n onan-7-yl)-*N*-((5-nitro-6-(((4-((4-oxido-1,4λ⁶-oxathian-4-ylidene)a mino)cyclohexyl)methyl)amino)p yridin-3-yl)sulfonyl)benzamide | MS-ESI (m/z): 980 [M + 1]⁺ |
| A-22a | 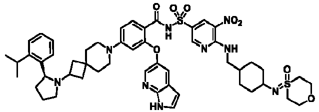 | (*S*)-2-((1*H*-pyrrolo[2,3-*b*]pyridin-5-yl)oxy)-4-(2-(2-(2-isopropylph enyl)pyrrolidin-1-yl)-7-azaspiro[ 3.5]nonan-7-yl)-*N*-((5-nitro-6-((( 4-((4-oxido-1,4λ⁶-oxathian-4-ylid ene)amino)cyclohexyl)methyl) amino)pyridin-3-yl)sulfonyl)benzam ide | MS-ESI (m/z): 994 [M + 1]⁺ |
| A-22b | 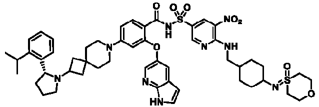 | (*R*)-2-((1*H*-pyrrolo[2,3-*b*]pyridin-5-yl)oxy)-4-(2-(2-(2-isopropylph enyl)pyrrolidin-1-yl)-7-azaspiro[ 3.5]nonan-7-yl)-*N*-((5-nitro-6-((( 4-((4-oxido-1,4λ⁶-oxathian-4-ylid ene)amino)cyclohexyl)methyl)am ino)pyridin-3-yl)sulfonyl)benzam ide | MS-ESI (m/z): 994 [M + 1]⁺ |
| A-23a | 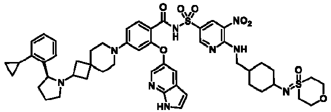 | (*S*)-2-((1*H*-pyrrolo[2,3-*b*]pyridin-5-yl)oxy)-4-(2-(2-(2-cyclopropyl phenyl)pyrrolidin-1-yl)-7-azaspir o[3.5]nonan-7-yl)-*N*-((5-nitro-6-( ((4-((4-oxido-1,4λ⁶-oxathian-4-yl idene)amino)cyclohexyl)methyl) amino)pyridin-3-yl)sulfonyl)benza mide | MS-ESI (m/z): 992 [M + 1]⁺ |
| A-23b | 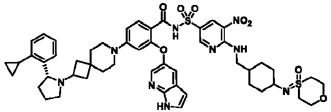 | (*R*)-2-((1*H*-pyrrolo[2,3-*b*]pyridin-5-yl)oxy)-4-(2-(2-(2-cyclopropyl phenyl)pyrrolidin-1-yl)-7-azaspiro [3.5]nonan-7-yl)-*N*-((5-nitro-6-( ((4-((4-oxido-1,4λ⁶-oxathian-4-yl idene)amino)cyclohexyl)methyl) amino)pyridin-3-yl)sulfonyl)benza mide | MS-ESI (m/z): 992 [M + 1]⁺ |
| A-24 | 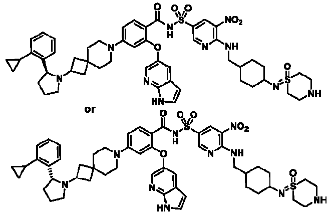 | (*R* or *S*)-2-((1*H*-pyrrolo[2,3-*b*]pyridin-5 -yl)oxy)-4-(2-(2-(2-cyclopropylp henyl)pyrrolidin-1-yl)-7-azaspiro [3.5]nonan-7-yl)-*N*-((5-nitro-6-((( 4-((1-oxido-1λ⁶-thiomorpholin-1-ylidene)amino)cyclohexyl)methyl )amino)pyridin-3-yl)sulfonyl)ben zamide | MS-ESI (m/z): 991 [M + 1]⁺ |
| A-25 | 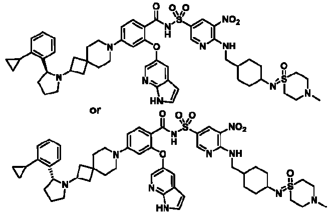 | (*R* or *S*)-2-((1*H*-pyrrolo[2,3-*b*]pyridin-5 -yl)oxy)-4-(2-(2-(2-cyclopropylp henyl)pyrrolidin-1-yl)-7-azaspiro [3.5]nonan-7-yl)-*N*-((6-(((4-((4-methyl-1-oxido-1λ⁶ -thiomorpholin-1-ylidene)amino)cyclohexyl)met hyl)amino)-5-nitropyridin-3-yl) sulfonyl)benzamide | MS-ESI (m/z): 1005 [M + 1]⁺ |
| A-26 | 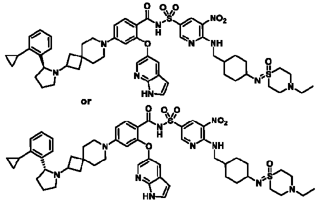 | (*R* or *S*)-2-((1*H*-pyrrolo[2,3-*b*]pyridin-5 -yl)oxy)-4-(2-(2-(2-cyclopropylp henyl)pyrrolidin-1-yl)-7-azaspiro [3.5]nonan-7-yl)-*N*-((6-(((4-((4-et hyl-1-oxido-1λ⁶-thiomorpholin-1-ylidene)amino)cyclohexyl)methyl )amino)-5-nitropyridin-3-yl)sulfo nvl)benzamide | MS-ESI (m/z): 1019 [M + 1]⁺ |
| A-27a | 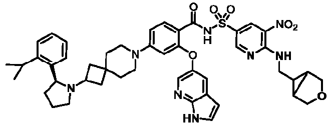 | 2-((1*H*-pyrrolo[2,3-*b*]pyridin-5-yl )oxy)-*N*-((6-(((3-oxabicyclo[3.1.0 ]hexan-6-yl)methyl)amino)-5-nitr opyridin-3-yl)sulfonyl)-4-(2-((S)-2-(2-isopropylphenyl)pyrrolidin-1-yl)-7-azaspiro[3.5]nonan-7-yl)b enzamide | MS-ESI (m/z): 861 [M + 1]⁺ |
| A-27b | 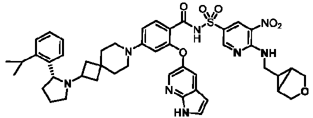 | 2-((1*H*-pyrrolo[2,3-*b*]pyridin-5-yl )oxy)-*N*-((6-(((3-oxabicyclo[3.1.0 ]hexan-6-yl)methyl)amino)-5-nitr opyridin-3-yl)sulfonyl)-4-(2-((*R*)-2-(2-isopropylphenyl)pyrrolidin-1-yl)-7-azaspiro[3.5]nonan-7-yl) benzamide | MS-ESI (m/z): 861 [M + 1]⁺ |
| A-28 | 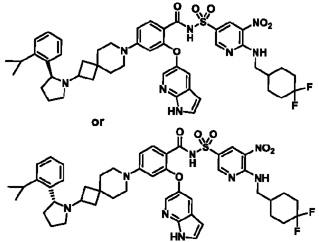 | (*R* or *S*)-2-((1*H*-pyrrolo[2,3-*b*]pyridin-5 -yl)oxy)-*N*-((6-(((4,4-difluorocycl ohexyl)methyl)amino)-5-nitropyr idin-3-yl)sulfonyl)-4-(2-(2-(2-iso propylphenyl)pyrrolidin-1-yl)-7-azaspiro[3.5]nonan-7-yl)benzamide | MS-ESI (m/z): 897 [M + 1]⁺ |
| A-29 | 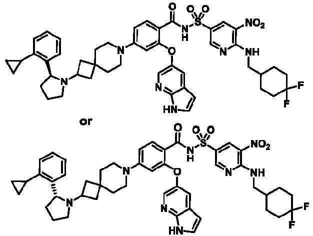 | (*R* or *S*)-2-((1*H*-pyrrolo[2,3-*b*]pyridin-5 -yl)oxy)-4-(2-(2-(2-cyclopropylp henyl)pyrrolidin-1-yl)-7-azaspiro [3.5]nonan-7-yl)-*N*-((6-(((4,4-difl uorocyclohexyl)methyl)amino)-5 -nitropyridin-3-yl)sulfonyl)benzamide | MS-ESI (m/z): 895 [M + 1]⁺ |
| A-30a | 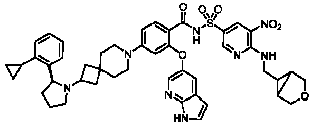 | 2-((1*H*-pyrrolo[2,3-*b*]pyridin-5-yl )oxy)-*N*-((6-(((3-oxabicyclo[3.1.0 ]hexan-6-yl)methyl)amino)-5-nitr opyridin-3-yl)sulfonyl)-4-(2-((S)-2-(2-cyclopropylphenyl)pyrrolidin -1-yl)-7-azaspiro[3.5]nonan-7-yl )benzamide | MS-ESI (m/z): 859 [M + 1]⁺ |
| A-30b | 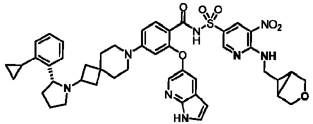 | 2-((1*H*-pyrrolo[2,3-*b*]pyridin-5-yl )oxy)-*N*-((6-(((3-oxabicyclo[3.1.0 ]hexan-6-yl)methyl)amino)-5-nitr opyridin-3-yl)sulfonyl)-4-(2-((*R*)-2-(2-cyclopropylphenyl)pyrrolidi ne-1-yl)-7-azaspiro[3.5]nonan-7-yl)benzamide | MS-ESI (m/z): 859 [M + 1]⁺ |
| A-31a | 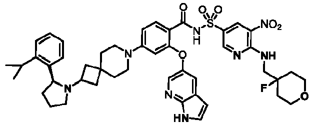 | (*S*)-2-((1*H*-pyrrolo[2,3-*b*]pyridin-5-yl)oxy)-*N*-((6-(((4-fluorotetrah ydro-2*H*-pyran-4-yl)methyl)amin o)-5-nitropyridin-3-yl)sulfonyl)-4 -(2-(2-(2-isopropylphenyl)pyrroli din-1-yl)-7-azaspiro[3.5]nonan-7-yl)benzamide | MS-ESI (m/z): 881 [M + 1]⁺ |
| A-31b | 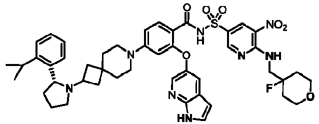 | (*R*)-2-((1*H-*pyrrolo[2,3-*b*]pyridin-5-yl)oxy)-*N*-((6-(((4-fluorotetrah ydro-2*H*-pyran-4-yl)methyl)amin o)-5-nitropyridin-3-yl)sulfonyl)-4 -(2-(2-(2-isopropylphenyl)pyrroli din-1-yl)-7-azaspiro[3.5]nonan-7-yl)benzamide | MS-ESI (m/z): 881 [M + 1]⁺ |
| A-32a | 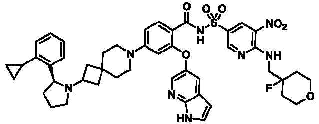 | (*S*)-2-((1*H*-pyrrolo[2,3-*b*]pyridin-5-yl)oxy)-4-(2-(2-(2-cyclopropyl phenyl)pyrrolidin-1-yl)-7-azaspir o[3.5]nonan-7-yl)-*N*-((6-(((4-fluo rotetrahydro-2*H*-pyran-4-yl)meth yl)amino)-5-nitropyridin-3-yl)sul fonyl)benzamide | MS-ESI (m/z): 879 [M + 1]⁺ |
| A-32b | 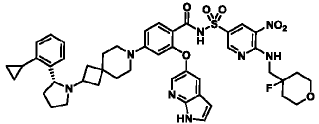 | (*R*)-2-((1*H*-pyrrolo[2,3-*b*]pyridin-5-yl)oxy)-4-(2-(2-(2-cyclopropyl phenyl)pyrrolidin-1-yl)-7-azaspir o[3.5]nonan-7-yl)-*N*-((6-(((4-fluo rotetrahydro-2*H*-pyran-4-yl)meth yl)amino)-5-nitropyridin-3-yl)sul fonyl)benzamide | MS-ESI (m/z): 879 [M + 1]⁺ |
| A-33 | 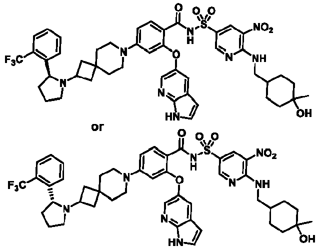 | (*R* or *S*)-2-((1*H*-pyrrolo[2,3-*b*]pyridin-5 -yl)oxy)-*N*-((6-(((4-hydroxy-4-methylcyclohexyl)methyl)amino)-5 -nitropyridin-3-yl)sulfonyl)-4-(2-(2-(2-(trifluoromethyl)phenyl)pyr rolidin-1-yl)-7-azaspiro[3.5]nonan -7-yl)benzamide | MS-ESI (m/z): 917 [M + 1]⁺ |

Examples A-34 ~ A-173 listed in Table 2 were prepared using appropriate intermediates, which can be readily synthesized by methods known in the literature or may be prepared by a variety of methods familiar to those skilled in the art. The structures and names of Examples A-34 ~ A-173 are given in table 2.

**Table 2**

| EXAMPLE | STRUCTURE | NAME | DATA |
|---|---|---|---|
| A-34 | 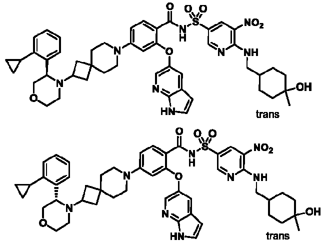 | (*R* or *S*)-2-((1*H*-pyrrolo[2,3-*b*]pyridin-5 -yl)oxy)-4-(2-(3-(2-cyclopropylp henyl)morpholino)-7-azaspiro[3.5 ]nonan-7-yl)-*N*-((6-(((4-hydroxy-4-methylcyclohexyl)methyl)amin o)-5-nitropyridin-3-yl)sulfonyl)be nzamide | MS-ESI (m/z): 905 [M + 1]⁺ |
| A-35 | 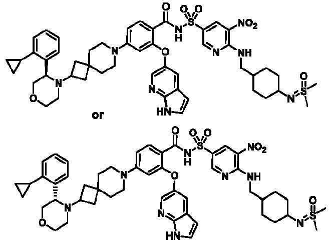 | (*R* or *S*)-2-((1*H*-pyrrolo[2,3-*b*]pyridin-5 -yl)oxy)-4-(2-(3-(2-cyclopropylp henyl)morpholino)-7-azaspiro[3.5 ]nonan-7-yl)-*N*-((6-(((4-((dimeth yl(oxo)-λ⁶-sulfanylidene)amino) cyclohexyl)methyl)amino)-5-nitro pyridin-3-yl)sulfonyl)benzamide | MS-ESI (m/z): 966 [M + 1]⁺ |
| A-36a | 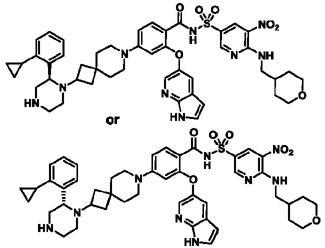 | (*R* or *S*)-2-((1*H*-pyrrolo[2,3-*b*]pyridin-5 -yl)oxy)-4-(2-(2-(2-cyclopropylp henyl)piperazin-1-yl)-7-azaspiro[ 3.5]nonan-7-yl)-*N*-((5-nitro-6-((( tetrahydro-2*H*-pyran-4-yl)methyl) amino)pyridin-3-yl)sulfonyl)benz amide | MS-ESI (m/z): 876 [M + 1]⁺ |
| A-36b | | | |
| A-37 | 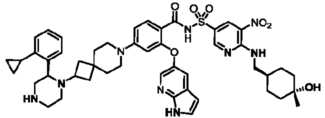 | 2-((1H-pyrrolo[2,3-b]pyridin-5-yl )oxy)-4-(2-((R)-2-(2-cyclopropyl phenyl)piperazin-1-yl)-7-azaspiro [3.5]nonan-7-yl)-N-((6-((((1r,4r)-4-hydroxy-4-methylcyclohexyl) methyl)amino)-5-nitropyridin-3-yl) sulfonyl)benzamide | MS-ESI (m/z): 904 [M + 1]⁺ |
| A-38 | 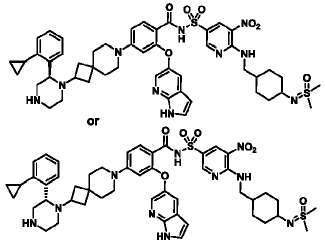 | (*R* or *S*)-2-((1*H*-pyrrolo[2,3-*b*]pyridin-5 -yl)oxy)-4-(2-(2-(2-cyclopropylp henyl)piperazin-1-yl)-7-azaspiro[ 3.5]nonan-7-yl)-*N*-((6-(((4-((dime thyl(oxo)-λ⁶- sulfanylidene)amino )cyclohexyl)methyl)amino)-5-nitr opyridin-3-yl)sulfonyl)benzamide | MS-ESI (m/z): 965 [M + 1]⁺ |
| A-39a | 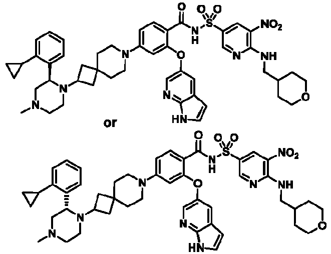 | (*R* or *S*)-2-((1*H*-pyrrolo[2,3-*b*]pyridin-5 -yl)oxy)-4-(2-(2-(2-cyclopropylp henyl)-4-methylpiperazin-1-yl)-7 -azaspiro[3.5]nonan-7-yl)-*N*-((5-nitro-6-(((tetrahydro-2*H*-pyran-4-yl)methyl)amino)pyridin-3-yl)sulf onyl)benzamide | MS-ESI (m/z): 890 [M + 1]⁺ |
| A-39b | | | |
| A-40 | 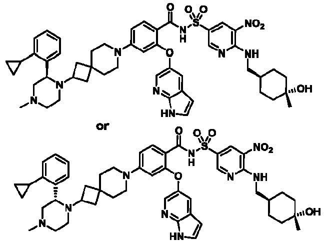 | 2-((1H-pyrrolo[2,3-b]pyridin-5-yl )oxy)-4-(2-((R or S)-2-(2-cyclopropylphenyl)-4-methylpiperazin-1-yl)-7-azaspiro[3.5 ]nonan-7-yl)-N-((6-((((1r,4r)-4-hy droxy-4-methylcyclohexyl)methyl )amino)-5-nitropyridin-3-yl)sulf onyl)benzamide | MS-ESI (m/z): 918 [M + 1]⁺ |
| A-41 | 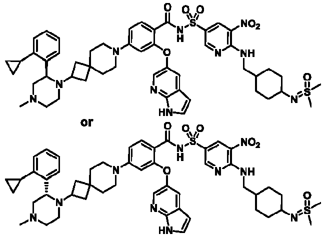 | (R or S)-2-((1*H*-pyrrolo[2,3-*b*]pyridin-5-yl)oxy)-4-(2-(2-(2-cyclopropyl phenyl)-4-methylpiperazin-1-yl)-7-azaspiro[3.5]nonan-7-yl)-*N*-((6-(((4-((dimethyl(oxo)-λ⁶-sulfanyli dene)amino)cyclohexyl)methyl) amino)-5-nitropyridin-3-yl)sulfony l)benzamide | MS-ESI (m/z): 979 [M + 1]⁺ |
| A-42 | 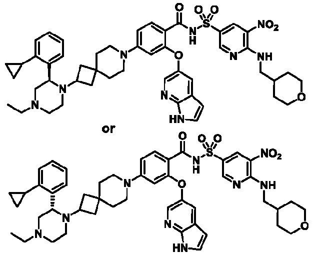 | (R or S)-2-((1*H*-pyrrolo[2,3-*b*]pyridin-5-yl)oxy)-4-(2-(2-(2-cyclopropyl phenyl)-4-ethylpiperazin-1-yl)-7-azaspiro[3.5]nonan-7-yl)-*N*-((5-ni tro-6-(((tetrahydro-2*H*-pyran-4-yl )methyl)amino)pyridin-3-yl)sulfo nyl)benzamide | MS-ESI (m/z): 904 [M + 1]⁺ |
| A-43 | 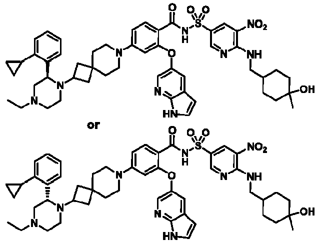 | (R or S)-2-((1*H*-pyrrolo[2,3-*b*]pyridin-5-yl)oxy)-4-(2-(2-(2-cyclopropyl phenyl)-4-ethylpiperazin-1-yl)-7-azaspiro[3.5]nonan-7-yl)-*N*-((6-(( (4-hydroxy-4-methylcyclohexyl) methyl)amino)-5-nitropyridin-3-y l)sulfonyl)benzamide | MS-ESI (m/z): 932 [M + 1]⁺ |
| A-44 | 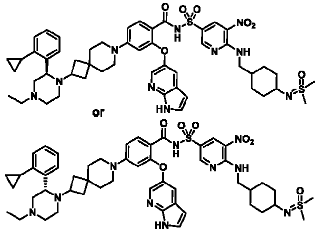 | (R or S)-2-((1*H*-pyrrolo[2,3-*b*]pyridin-5-yl)oxy)-4-(2-(2-(2-cyclopropyl phenyl)-4-ethylpiperazin-1-yl)-7-azaspiro[3.5]nonan-7-yl)-*N*-((6-(( (4-((dimethyl(oxo)-λ⁶-sulfanylide ne)amino)cyclohexyl)methyl)ami no)-5-nitropyridin-3-yl)sulfonyl) benzamide | MS-ESI (m/z): 993 [M + 1]⁺ |
| A-45 | 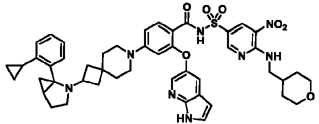 | 2-((1*H*-pyrrolo[2,3-*b*]pyridin-5-yl )oxy)-4-(2-(1-(2-cyclopropylphen yl)-2-azabicyclo[3.1.0]hexan-2-yl )-7-azaspiro[3.5]nonan-7-yl)-*N*-(( 5-nitro-6-(((tetrahydro-2H-pyran-4-yl)methyl)amino)pyridin-3-yl)s ulfonyl)benzamide | MS-ESI (m/z): 873 [M + 1]⁺ |
| A-46 | 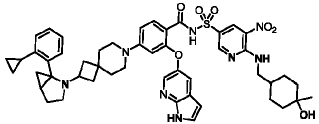 | 2-((1*H*-pyrrolo[2,3-b]pyridin-5-yl )oxy)-4-(2-(1-(2-cyclopropylphen yl)-2-azabicyclo[3.1.0]hexan-2-yl )-7-azaspiro[3.5]nonan-7-yl)-*N*-(( 6-(((4-hydroxy-4-methylcyclohex yl)methyl)amino)-5-nitropyridin-3-yl)sulfonyl)benzamide | MS-ESI (m/z): 901 [M + 1]⁺ |
| A-47 | 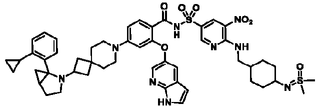 | 2-((1*H*-pyrrolo[2,3-*b*]pyridin-5-yl )oxy)-4-(2-(1-(2-cyclopropylphen yl)-2-azabicyclo[3.1.0]hexan-2-yl )-7-azaspiro[3.5]nonan-7-yl)-*N*-(( 6-(((4-((dimethyl(oxo)-λ⁶-sulfany lidene)amino)cyclohexyl)methyl) amino)-5-nitropyridin-3-yl)sulfon yl)benzamide | MS-ESI (m/z): 962 [M + 1]⁺ |
| A-48 | 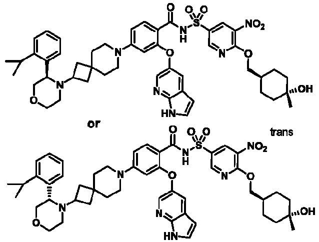 | 2-((1H-pyrrolo[2,3-b]pyridin-5-yl )oxy)-N-((6-(((1r,4r)-4-hydroxy-4 -methylcyclohexyl)methoxy)-5-ni tropyridin-3-yl)sulfonyl)-4-(2-((R or S)-3-(2-isopropylphenyl)morphol ino)-7-azaspiro[3.5]nonan-7-yl) benzamide | MS-ESI (m/z): 908 [M + 1]⁺ |
| A-49 | 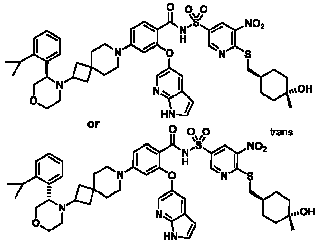 | 2-((1H-pyrrolo[2,3-b]pyridin-5-yl )oxy)-N-((6-((((1r,4r)-4-hydroxy-4-methylcyclohexyl)methyl)thio) -5-nitropyridin-3-yl)sulfonyl)-4-( 2-((R or S)-3-(2-isopropylphenyl)morphol ino)-7-azaspiro[3.5]nonan-7-yl) benzamide | MS-ESI (m/z): 924 [M + 1]⁺ |
| A-50 | 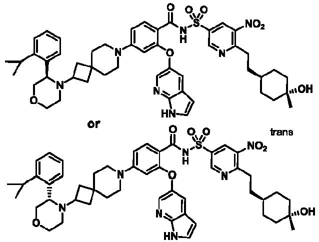 | 2-((1H-pyrrolo[2,3-b]pyridin-5-yl )oxy)-N-((6-(2-((1r,4s)-4-hydroxy -4-methylcyclohexyl)ethyl)-5-nitr opyridin-3-yl)sulfonyl)-4-(2-((R or S)-3-(2-isopropylphenyl)morphol ino)-7-azaspiro[3.5]nonan-7-yl) benzamide | MS-ESI (m/z): 906 [M + 1]⁺ |
| A-51 | 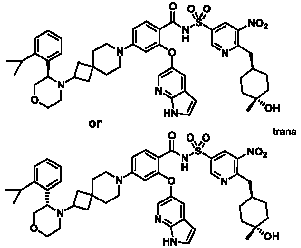 | 2-((1H-pyrrolo[2,3-b]pyridin-5-yl )oxy)-N-((6-(((1r,4r)-4-hydroxy-4 -methylcyclohexyl)methyl)-5-nitr opyridin-3-yl)sulfonyl)-4-(2-((R or S)-3-(2-isopropylphenyl)morphol ino)-7-azaspiro[3.5]nonan-7-yl) benzamide | MS-ESI (m/z): 892 [M + 1]⁺ |
| A-52 | 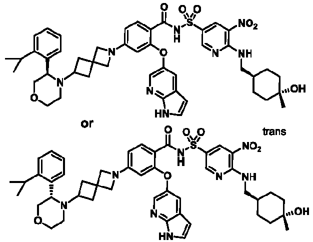 | 2-((1H-pyrrolo[2,3-b]pyridin-5-yl )oxy)-N-((6-((((1r,4r)-4-hydroxy-4-methylcyclohexyl)methyl)amin o)-5-nitropyridin-3-yl)sulfonyl)-4 -(6-((R or S)-3-(2-isopropylphenyl)morphol ino)-2-azaspiro[3.3]heptan-2-yl) benzamide | MS-ESI (m/z): 879 [M + 1]⁺ |
| A-53 | 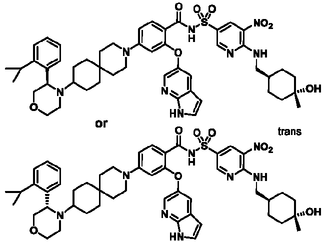 | 2-((1H-pyrrolo[2,3-b]pyridin-5-yl )oxy)-N-((6-((((1r,4r)-4-hydroxy-4-methylcyclohexyl)methyl)amin o)-5-nitropyridin-3-yl)sulfonyl)-4 -(9-((R or S)-3-(2-isopropylphenyl)morphol ino)-3-azaspiro[5.5]undecan-3-yl) benzamide | MS-ESI (m/z): 953 [M + 1]⁺ |
| A-54 | 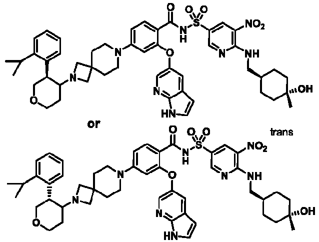 | 2-((1H-pyrrolo[2,3-b]pyridin-5-yl )oxy)-N-((6-((((1r,4r)-4-hydroxy-4-methylcyclohexyl)methyl)amin o)-5-nitropyridin-3-yl)sulfonyl)-4 -(2-((3R or 3S)-3-(2-isopropylphenyl)tetrahy dro-2H-pyran-4-yl)-2,7-diazaspir o[3.5]nonan-7-yl)benzamide | MS-ESI (m/z): 907 [M + 1]⁺ |
| A-55a | 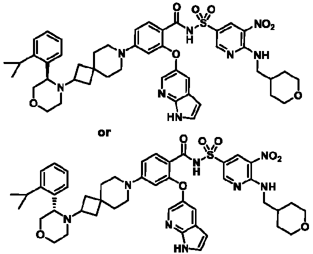 | (*R* or S)-2-((1*H*-pyrrolo[2,3-*b*]pyridin-5-yl)oxy)-4-(2-(3-(2-isopropylph enyl)morpholino)-7-azaspiro[3.5] nonan-7-yl)-*N*-((5-nitro-6-(((tetra hydro-2*H*-pyran-4-yl)methyl)ami no)pyridin-3-yl)sulfonyl)benzamide | MS-ESI (m/z): 879 [M + 1]⁺ |
| A-55b | | | |
| A-56a | 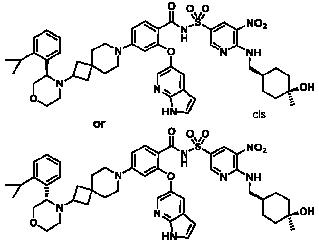 | 2-((1*H*-pyrrolo[2,3-*b*]pyridin-5-yl )oxy)-*N*-((6-(((1*s*,4*s*)-4-hydroxy-4-methylcyclohexyl)methyl)amin o)-5-nitropyridin-3-yl)sulfonyl)-4 -(2-((*R* or S)-3-(2-isopropylphenyl)morphol ino)-7-azaspiro[3.5]nonan-7-yl) benzamide | MS-ESI (m/z): 907 [M + 1]⁺ |
| A-56b | 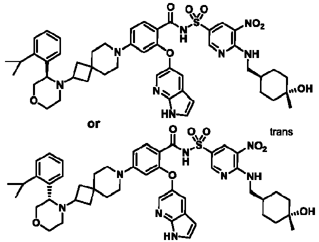 | 2-((1*H*-pyrrolo[2,3-*b*]pyridin-5-yl )oxy)-*N*-((6-((((1*r*,4*r*)-4-hydroxy-4-methylcyclohexyl)methyl)amino )-5-nitropyridin-3-yl)sulfonyl)-4 -(2-((*R* or S)-3-(2-isopropylphenyl)morphol ino)-7-azaspiro[3.5]nonan-7-yl) benzamide | MS-ESI (m/z): 907 [M + 1]⁺ |
| A-57 | 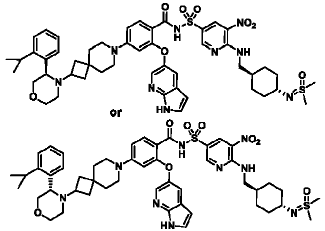 | 2-((1H-pyrrolo[2,3-b]pyridin-5-yl )oxy)-N-((6-((((1r,4r)-4-((dimeth yl(oxo)-λ⁶-sulfanylidene)amino)c yclohexyl)methyl)amino)-5-nitro pyridin-3-yl)sulfonyl)-4-(2-((R or S)-3-(2-isopropylphenyl)morphol ino)-7-azaspiro[3.5]nonan-7-yl)b enzamide | MS-ESI (m/z): 968 [M + 1]⁺ |
| A-58 | 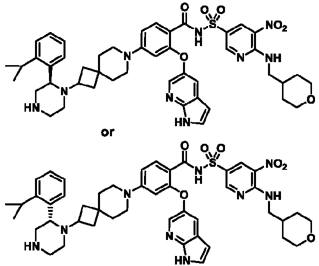 | (R or S)-2-((1*H*-pyrrolo[2,3-*b*]pyridin-5-yl)oxy)-4-(2-(2-(2-isopropylph enyl)piperazin-1-yl)-7-azaspiro[3. 5]nonan-7-yl)-*N*-((5-nitro-6-(((tet rahydro-2*H*-pyran-4-yl)methyl)a mino)pyridin-3-yl)sulfonyl)benza mide | MS-ESI (m/z): 878 [M + 1]⁺ |
| A-59 | 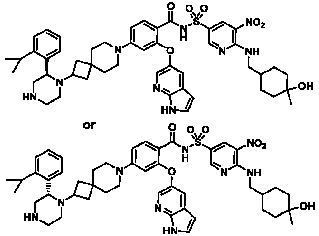 | (R or S)-2-((1*H*-pyrrolo[2,3-*b*]pyridin-5-yl)oxy)-*N*-((6-(((4-hydroxy-4-methylcyclohexyl)methyl)amino) -5-nitropyridin-3-yl)sulfonyl)-4-( 2-(2-(2-isopropylphenyl)piperazin -1-yl)-7-azaspiro[3.5]nonan-7-yl )benzamide | MS-ESI (m/z): 906 [M + 1]⁺ |
| A-60 | 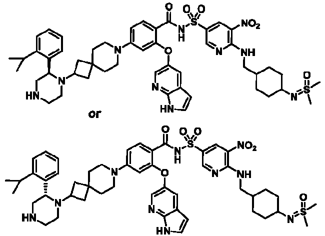 | (R or S)-2-((1*H*-pyrrolo[2,3*-b*]pyridin-5-yl)oxy)-*N*-((6-(((4-((dimethyl(o xo)-λ⁶-sulfanylidene)amino)cyclo hexyl)methyl)amino)-5-nitropyri din-3-yl)sulfonyl)-4-(2-(2-(2-isop ropylphenyl)piperazin-1-yl)-7-az aspiro[3.5]nonan-7-yl)benzamide | MS-ESI (m/z): 967 [M + 1]⁺ |
| A-61 | 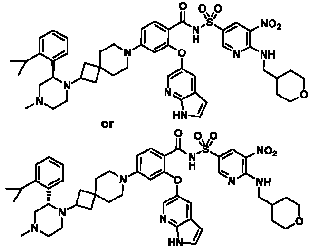 | (R or S)-2-((1*H*-pyrrolo[2,3-b]pyridin-5-yl)oxy)-4-(2-(2-(2-isopropylph enyl)-4-methylpiperazin-1-yl)-7-a zaspiro[3.5]nonan-7-yl)-*N*-((5-nit ro-6-(((tetrahydro-2*H*-pyran-4-yl) methyl)amino)pyridin-3-yl)sulfon yl)benzamide | MS-ESI (m/z): 892 [M + 1]⁺ |
| A-62 | 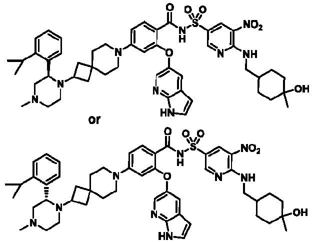 | (R or S)-2-((1*H*-pyrrolo[2,3-b]pyridin-5-yl)oxy)-*N*-((6-(((4-hydroxy-4-methylcyclohexyl)methyl)amino) -5-nitropyridin-3-yl)sulfonyl)-4-( 2-(2-(2-isopropylphenyl)-4-meth ylpiperazin-1-yl)-7-azaspiro[3.5] nonan-7-yl)benzamide | MS-ESI (m/z): 920 [M + 1]⁺ |
| A-63 | 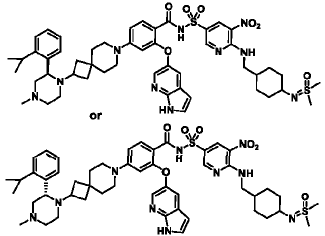 | (R or S)-2-((1*H*-pyrrolo[2,3-b]pyridin-5-yl)oxy)-*N*-((6-(((4-((dimethyl(o xo)-λ⁶-sulfanylidene)amino)cyclo hexyl)methyl)amino)-5-nitropyri din-3-yl)sulfonyl)-4-(2-(2-(2-isop ropylphenyl)-4-methylpiperazin-1-yl)-7-azaspiro[3.5]nonan-7-yl)b enzamide | MS-ESI (m/z): 981 [M + 1]⁺ |
| A-64 | 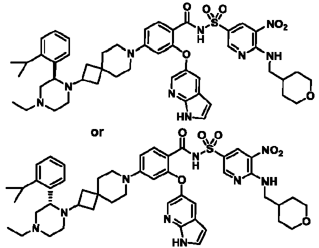 | (R or S)-2-((1*H*-pyrrolo[2,3-*b*]pyridin-5-yl)oxy)-4-(2-(4-ethyl-2-(2-isopr opylphenyl)piperazin-1-yl)-7-aza *s*piro[3.5]nonan-7-yl)-*N-*((5-nitro -6-(((tetrahydro-2*H*-pyran-4-yl)m ethyl)amino)pyridin-3-yl)sulfonyl )benzamide | MS-ESI (m/z): 906 [M + 1]⁺ |
| A-65 | 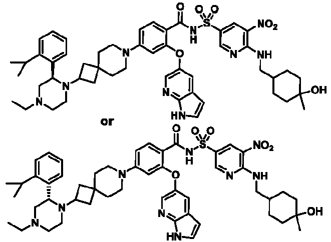 | (R or S)-2-((1H-pyrrolo[2,3-b]pyridin-5-yl)oxy)-4-(2-(4-ethyl-2-(2-isopr opylphenyl)piperazin-1-yl)-7-aza spiro[3.5]nonan-7-yl)-N-((6-((((1r ,4r)-4-hydroxy-4-methylcyclohex yl)methyl)amino)-5-nitropyridin-3-yl)sulfonyl)benzamide | MS-ESI (m/z): 934 [M + 1]⁺ |
| A-66 | 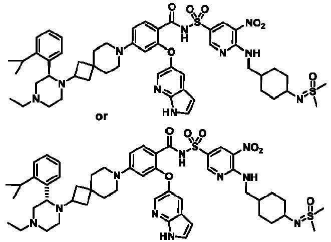 | (R or S)-2-((1*H*-pyrrolo[2,3-b]pyridin-5-yl)oxy)-*N*-((6-(((4-((dimethyl(o xo)-λ⁶-sulfanylidene)amino)cyclo hexyl)methyl)amino)-5-nitropyri din-3-yl)sulfonyl)-4-(2-(4-ethyl-2 -(2-isopropylphenyl)piperazin-1-yl)-7-azaspiro[3.5]nonan-7-yl)be nzamide | MS-ESI (m/z): 995 [M + 1]⁺ |
| A-67 | 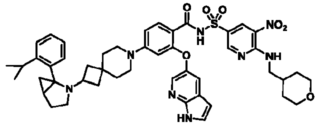 | 2-((1*H*-pyrrolo[2,3-b]pyridin-5-yl )oxy)-4-(2-(1-(2-isopropylphenyl )-2-azabicyclo[3.1.0]hexan-2-yl)-7-azaspiro[3.5]nonan-7-yl)-*N*-((5-nitro-6-(((tetrahydro-2*H*-pyran-4-yl)methyl)amino)pyridin-3-yl)sul fonyl)benzamide | MS-ESI (m/z): 875 [M + 1]⁺ |
| A-68 | 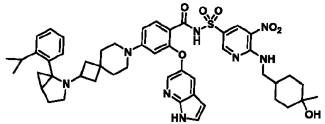 | 2-((1*H*-pyrrolo[2,3-*b*]pyridin-5-yl )oxy)-*N*-((6-(((4-hydroxy-4-meth ylcyclohexyl)methyl)amino)-5-ni tropyridin-3-yl)sulfonyl)-4-(2-(1-(2-isopropylphenyl)-2-azabicyclo [3.1.0]hexan-2-yl)-7-azaspiro[3.5 ]nonan-7-vl)benzamide | MS-ESI (m/z): 903 [M + 1]⁺ |
| A-69 | 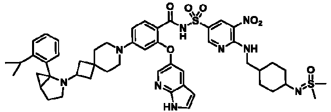 | 2-((1*H*-pyrrolo[2,3-*b*]pyridin-5-yl )oxy)-N-((6-(((4-((dimethyl(oxo)-λ⁶-sulfanylidene)amino)cyclohex yl)methyl)amino)-5-nitropyridin-3-yl)sulfonyl)-4-(2-(1-(2-isoprop ylphenyl)-2-azabicyclo[3.1.0]hex an-2-yl)-7-azaspiro[3.5]nonan-7-yl)benzamide | MS-ESI (m/z): 964 [M + 1]⁺ |
| A-70 | 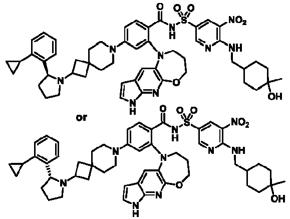 | (R or S)-4-(2-(2-(2-cyclopropylphenyl) pyrrolidin-1-yl)-7-azaspiro[3.5]n onan-7-yl)-2-(3,4-dihydro-2*H*-pyr rolo[3',2':5,6]pyrido[2,3-b] [1,4]o xazepin-1(7H)-yl)-N-((6-(((4-hyd roxy-4-methylcyclohexyl)methyl) amino)-5-nitropyridin-3-yl)sulfon yl)benzamide | MS-ESI (m/z): 944 [M + 1]⁺ |
| A-71 | 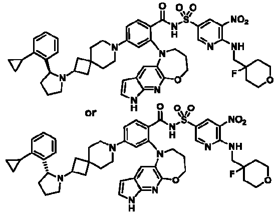 | (R or S)-4-(2-(2-(2-cyclopropylphenyl) pyrrolidin-1-yl)-7-azaspiro[3.5]n onan-7-yl)-2-(3,4-dihydro-2*H*-pyr rolo[3',2':5,6]pyrido[2,3-*b*][1,4]o xazepin-1(7*H*)-yl)-N-((6-(((4-fluo rotetrahydro-2*H*-pyran-4-yl)meth yl)amino)-5-nitropyridin-3-yl)sul fonyl)benzamide | MS-ESI (m/z): 934 [M + 1]⁺ |
| A-72 | 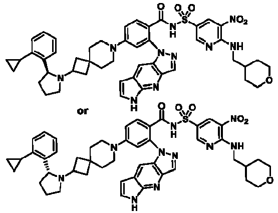 | (R or S)-4-(2-(2-(2-cyclopropylphenyl) pyrrolidin-1-yl)-7-azaspiro[3.5]n onan-7-yl)-*N*-((5-nitro-6-(((tetrah ydro-2H-pyran-4-yl)methyl)amin o)pyridin-3-yl)sulfonyl)-2-(pyraz olo[4,3-*b*]pyrrolo[3,2-e]pyridin-1 (5*H*)-yl)benzamide | MS-ESI (m/z): 885 [M + 1]⁺ |
| A-73 | 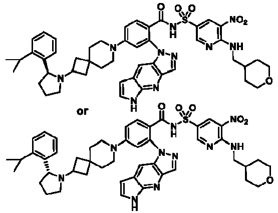 | (R or S)-4-(2-(2-(2-isopropylphenyl)py rrolidin-1-yl)-7-azaspiro[3.5]nona n-7-yl)-*N*-((5-nitro-6-(((tetrahydr o-2*H*-pyran-4-yl)methyl)amino)p yridin-3-yl)sulfonyl)-2-(pyrazolo[ 4,3-*b*]pyrrolo[3,2-e]pyridin-1(5*H* )-vl)benzamide | MS-ESI (m/z): 887 [M + 1]⁺ |
| A-74 | 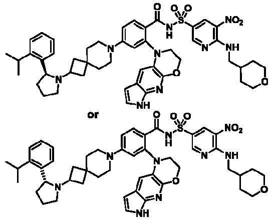 | (R or S)-2-(2,3-dihydropyrrolo[3',2':5,6 ]pyrido[2,3-*b*][1,4]oxazin-1(6*H*)-yl)-4-(2-(2-(2-isopropylphenyl)py rrolidin-1-yl)-7-azaspiro[3.5]nona n-7-yl)-*N*-((5-nitro-6-(((tetrahydr o-2*H*-pyran-4-yl)methyl)amino)p yridin-3-yl)sulfonyl)benzamide | MS-ESI (m/z): 904 [M + 1]⁺ |
| A-75 | 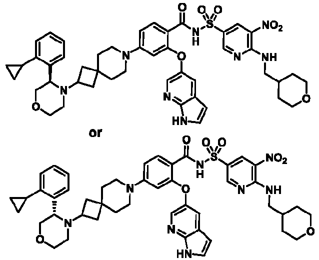 | (R or S)-2-((1H-pyrrolo[2,3-b]pyridin-5-yl)oxy)-4-(2-(3-(2-cyclopropyl phenyl)morpholino)-7-azaspiro[3. 5]nonan-7-yl)-N-((5-nitro-6-(((tet rahydro-2H-pyran-4-yl)methyl)a mino)pyridin-3-yl)sulfonyl)benza mide | MS-ESI (m/z): 877 [M + 1]⁺ |
| A-76 | 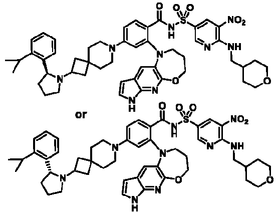 | (R or S)-2-(3,4-dihydro-2*H*-pyrrolo[3',2':5 ,6]pyrido[2,3-b][1,4]oxazepin-1(7*H*) -yl)-4-(2-(2-(2-isopropylphenyl)pyrr olidin-1-yl)-7-azaspiro[3.5]nonan-7-*yl)-N-((5-nitro-6-(((tetrahydro-2H-p* yran-4-yl)methyl)amino)pyridin-3-y l)sulfonyl)benzamide | MS-ESI (m/z): 918 [M + 1]⁺ |
| A-77 | 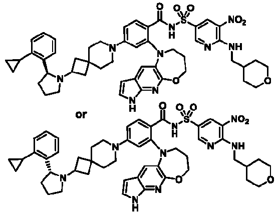 | (R or S)-4-(2-(2-(2-cyclopropylphenyl)py rrolidin-1-yl)-7-azaspiro[3.5]nonan-7-yl)-2-(3,4-dihydro-2*H*-pyrrolo[3', 2':5,6]pyrido[2,3-*b*][1,4]oxazepin-1 ( 7H)-yl)-*N*-((5-nitro-6-(((tetrahydro-2*H*-pyran-4-yl)methyl)amino)pyridi n-3-yl)sulfonyl)benzamide | MS-ESI (m/z): 916 [M + 1]⁺ |
| A-78a | 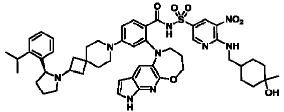 | (*S*)-2-(3,4-dihydro-2*H*-pyrrolo[3',2': 5,6]pyrido[2,3-b][1,4]oxazepin-1(7 *H*)-yl)-*N*-((6-(((4-hydroxy-4-methyl cyclohexyl)methyl)amino)-5-nitrop yridin-3-yl)sulfonyl)-4-(2-(2-(2-isor ropylphenyl)pyrrolidin-1-yl)-7-azas piro[3.5]nonan-7-yl)benzamide | MS-ESI (m/z): 946 [M + 1]⁺ |
| A-78b | 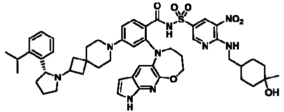 | (*R*)-2-(3,4-dihydro-2H-pyrrolo[3',2': 5,6]pyrido[2,3-b][1,4]oxazepin-1(7 H)-yl)-N-((6-(((4-hydroxy-4-methyl cyclohexyl)methyl)amino)-5-nitrop yridin-3-yl)sulfonyl)-4-(2-(2-(2-isop ropylphenyl)pyrrolidin-1-yl)-7-azas piro[3.5]nonan-7-yl)benzamide | MS-ESI (m/z): 946 [M + 1]⁺ |
| A-79 | 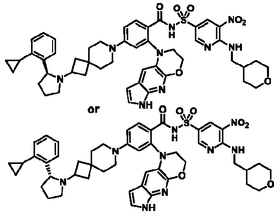 | (R or S)-4-(2-(2-(2-cyclopropylphenyl)py rrolidin-1-yl)-7-azaspiro[3.5]nonan-7-yl)-2-(2,3-dihydropyrrolo[3',2':5,6 ]pyrido[2,3-*b*][1,4]oxazin-1(6*H*)-yl) *-N*-((5-nitro-6-(((tetrahydro-2*H*-pyra n-4-yl)methyl)amino)pyridin-3-yl) sulfonyl)benzamide | MS-ESI (m/z): 902 [M + 1]⁺ |
| A-80 | 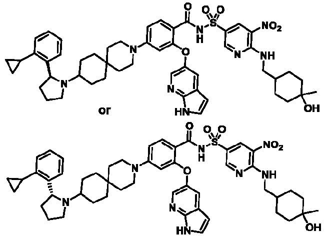 | (R or S)-2-((1*H*-pyrrolo[2,3-b]pyridin-5-yl )oxy)-4-(9-(2-(2-cyclopropylphenyl )pyrrolidin-1-yl)-3-azaspiro[5.5]und ecan-3-yl)-*N*-((6-(((4-hydroxy-4-me thylcyclohexyl)methyl)amino)-5-nit ropyridin-3-yl)sulfonyl)benzamide | MS-ESI (m/z): 917 [M + 1]+ |
| A-81 | 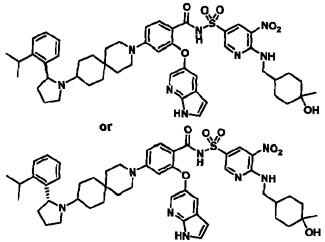 | (R or S)-2-((1*H*-pyrrolo[2,3-b]pyridin-5-yl )oxy)-*N*-((6-(((4-hydroxy-4-methyl cyclohexyl)methyl)amino)-5-nitrop yridin-3-yl)sulfonyl)-4-(9-(2-(2-isop ropylphenyl)pyrrolidin-1-yl)-3-azas piro[5.5]undecan-3-yl)benzamide | MS-ESI (m/z): 919 [M + 1]⁺ |
| A-82 | 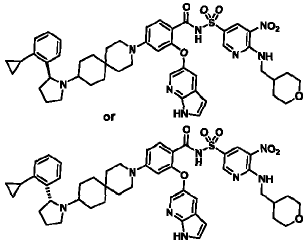 | (R or S)-2-((1*H*-pyrrolo[2,3-b]pyridin-5-yl )oxy)-4-(9-(2-(2-cyclopropylphenyl )pyrrolidin-1-yl)-3-azaspiro[5.5]und ecan-3-yl)-*N*-((5-nitro-6-(((tetrahydr o-2*H*-pyran-4-yl)methyl)amino)pyri din-3-yl)sulfonyl)benzamide | MS-ESI (m/z): 889 [M + 1]⁺ |
| A-83 | 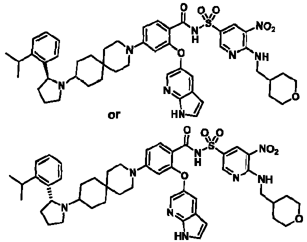 | (R or S)-2-((1*H*-pyrrolo[2,3-b]pyridin-5-yl )oxy)-4-(9-(2-(2-isopropylphenyl) pyrrolidin-1-yl)-3-azaspiro[5.5]under an-3-yl)-*N*-((5-nitro-6-(((tetrahydro-2*H*-pyran-4-yl)methyl)amino)pyridi n-3-yl)sulfonyl)benzamide | MS-ESI (m/z): 890 [M + 1]⁺ |
| A-84 | 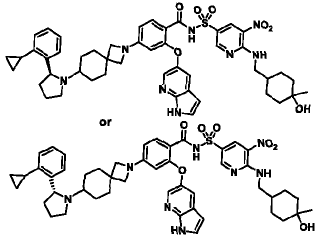 | (R or S)-2-((1*H*-pyrrolo[2,3-*b*]pyridin-5-y l)oxy)-4-(7-(2-(2-cyclopropylpheny )pyrrolidin-1-yl)-2-azaspiro[3.5]non an-2-yl)-*N*-((6-(((4-hydroxy-4-meth ylcyclohexyl)methyl)amino)-5-nitro pyridin-3-yl)sulfonyl)benzamide | MS-ESI (m/z): 889 [M + 1]⁺ |
| A-85 | 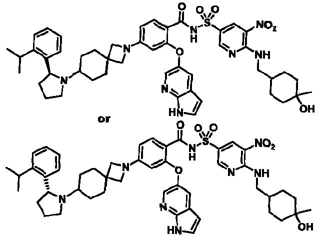 | (R or S)-2-((1*H*-pyrrolo[2,3-*b*]pyridin-5-yl )oxy)-*N*-((6-(((4-hydroxy-4-methyl cyclohexyl)methyl)amino)-5-nitrop yridin-3-yl)sulfonyl)-4-(7-(2-(2-isop ropylphenyl)pyrrolidin-1-yl)-2-azas piro[3.5]nonan-2-yl)benzamide | MS-ESI (m/z): 891[M + 1]⁺ |
| A-86 | 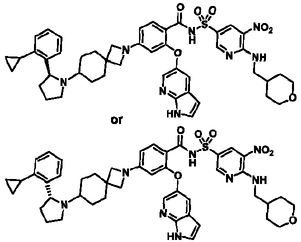 | (R or S)-2-((1*H*-pyrrolo[2,3-*b*]pyridin-5-yl )oxy)-4-(7-(2-(2-cyclopropylphenyl )pyrrolidin-1-yl)-2-azaspiro[3.5]non an-2-yl)-*N*-((5-nitro-6-(((tetrahydro-2*H*-pyran-4-yl)methyl)amino)pyridi n-3-yl)sulfonyl)benzamide | MS-ESI *(m*/*z):* 861 [M + 1]⁺ |
| A-87 | 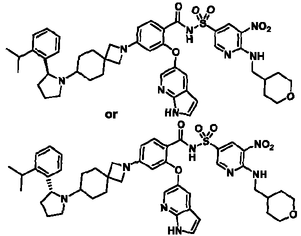 | (R or S)-2-((1*H*-pyrrolo[2,3-*b*]pyridin-5-yl )oxy)-4-(7-(2-(2-isopropylphenyl)p yrrolidin-1-yl)-2-azaspiro[3.5]nonar -2-yl)-*N*-((5-nitro-6-(((tetrahydro-2 *H*-pyran-4-yl)methyl)amino)pyridin -3-yl)sulfonyl)benzamide | MS-ESI (m/z): 863 [M + 1]⁺ |
| A-88 | 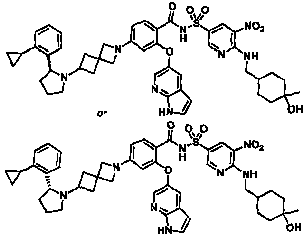 | (R or S)-2-((1*H*-pyrrolo[2,3-*b*]pyridin-5-yl )oxy)-4-(6-(2-(2-cyclopropylphenyl )pyrrolidin-1-yl)-2-azaspiro[3.3]hep tan-2-yl)-*N*-((6-(((4-hydroxy-4-meth ylcyclohexyl)methyl)amino)-5-nitro pyridin-3-yl)sulfonyl)benzamide | MS-ESI (m/z): 861 [M + 1]⁺ |
| A-89 | 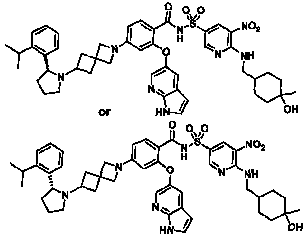 | (R or S)-2-((1*H*-pyrrolo[2,3-*b*]pyridin-5-yl )oxy)-*N*-((6-(((4-hydroxy-4-methyl cyclohexyl)methyl)amino)-5-nitrop yridin-3-yl)sulfonyl)-4-(6-(2-(2-isop ropylphenyl)pyrrolidin-1-yl)-2-azas piro[3.3]heptan-2-yl)benzamide | MS-ESI (m/z): 863 [M + 1]⁺ |
| A-90 | 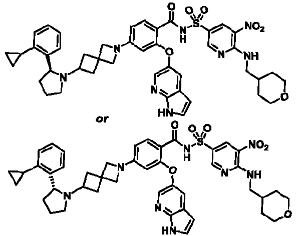 | (R or S)-2-((1*H*-pyrrolo[2,3-*b*]pyridin-5-y l)oxy)-4-(6-(2-(2-cyclopropylphenyl )pyrrolidin-1-yl)-2-azaspiro[3.3]hep tan-2-yl)-*N*-((5-nitro-6-(((tetrahydro -2*H*-pyran-4-yl)methyl)amino)pyrid in-3-yl)sulfonyl)benzamide | MS-ESI (m/z): 833 [M + 1]⁺ |
| A-91 | 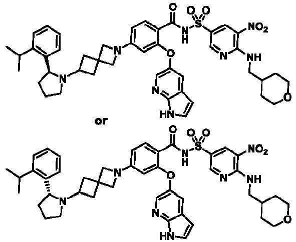 | (R or S)-2-((1*H*-pyrrolo[2,3-*b*]pyridin-5-y l)oxy)-4-(6-(2-(2-isopropylphenyl)p yrrolidin-1-yl)-2-azaspiro[3.3]hepta n-2-yl)-*N*-((5-nitro-6-(((tetrahydro-2 *H*-pyran-4-yl)methyl)amino)pyridin -3-yl)sulfonyl)benzamide | MS-ESI (m/z): 835 [M + 1]⁺ |
| A-92 | 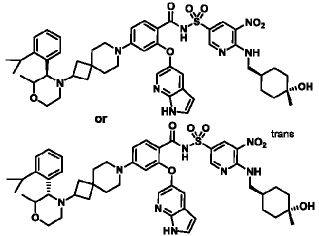 | 2-((1H-pyrrolo[2,3-b]pyridin-5-yl)o xy)-N-((6-((((1r,4r)-4-hydroxy-4-me thylcyclohexyl)methyl)amino)-5-nit ropyridin-3-yl)sulfonyl)-4-(2-((3R or 3S)-3-(2-isopropylphenyl)-2-methyl norpholino)-7-azaspiro[3.5]nonan-7 -yl)benzamide | MS-ESI (m/z): 921 [M + 1]⁺ |
| A-93 | 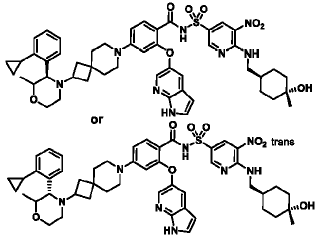 | 2-((1H-pyrrolo[2,3-b]pyridin-5-yl)o xy)-4-(2-((3R or 3S)-3-(2-cyclopropylphenyl)-2-met hylmorpholino)-7-azaspiro[3.5]nona n-7-yl)-N-((6-((((1r,4r)-4-hydroxy-4 -methylcyclohexyl)methyl)amino)-5 -nitropyridin-3-yl)sulfonyl)benzami de | MS-ESI (m/z): 919 [M + 1]⁺ |
| A-94 | 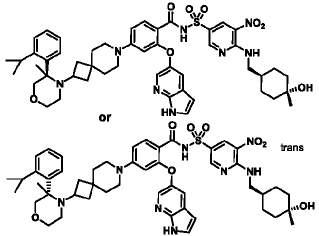 | 2-((1H-pyrrolo[2,3-b]pyridin-5-yl)o xy)-N-((6-((((1r,4r)-4-hydroxy-4-me thylcyclohexyl)methyl)amino)-5-nit ropyridin-3-yl)sulfonyl)-4-(2-((R or S)-3-(2-isopropylphenyl)-3-methyl norpholino)-7-azaspiro[3.5]nonan-7 -yl)benzamide | MS-ESI (m/z): 921 [M + 1]⁺ |
| A-95 | 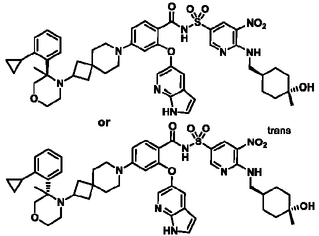 | 2-((1H-pyrrolo[2,3-b]pyridin-5-yl)o xy)-4-(2-((R or S)-3-(2-cyclopropylphenyl)-3-meth ylmorpholino)-7-azaspiro[3.5]nonan -7-yl)-N-((6-((((1r,4r)-4-hydroxy-4-methylcyclohexyl)methyl)amino)-5-nitropyridin-3-yl)sulfonyl)benzamide | MS-ESI (m/z): 919 [M + 1]⁺ |
| A-96 | 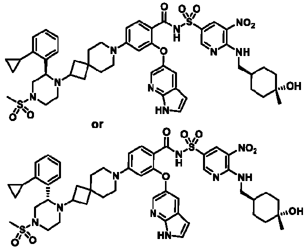 | 2-((1H-pyrrolo[2,3-b]pyridin-5-yl)o xy)-4-(2-((R or S)-2-(2-cyclopropylphenyl)-4-(meth ylsulfonyl)piperazin-1-yl)-7-azaspir o[3.5]nonan-7-yl)-N-((6-((((1r,4r)-4 -hydroxy-4-methylcyclohexyl)meth yl)amino)-5-nitropyridin-3-yl)sulfon yl)benzamide | MS-ESI (m/z): 982 [M + 1]⁺ |
| A-97a | 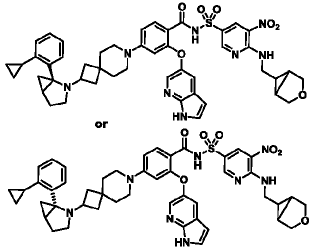 | 2-((1H-pyrrolo[2,3-b]pyridin-5-yl )oxy)-N-((6-(((3-oxabicyclo[3.1.0 ]hexan-6-yl)methyl)amino)-5-nitr opyridin-3-yl)sulfonyl)-4-(2-((1R or 1S)-1-(2-cyclopropylphenyl)-2-az abicyclo[3.1.0]hexan-2-yl)-7-azas piro[3.5]nonan-7-yl)benzamide | MS-ESI (m/z): 871 [M + 1]⁺ |
| A-97b | | | |
| A-98 | 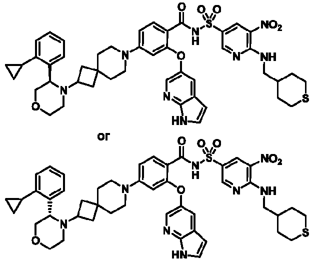 | (R or S)-2-((1H-pyrrolo[2,3-b]pyridin-5-yl)oxy)-4-(2-(3-(2-cyclopropyl phenyl)morpholino)-7-azaspiro[3. 5]nonan-7-yl)-N-((5-nitro-6-(((tet rahydro-2H-thiopyran-4-yl)methy l)amino)pyridin-3-yl)sulfonyl)be nzamide | MS-ESI (m/z): 893 [M + 1]⁺ |
| A-99 | 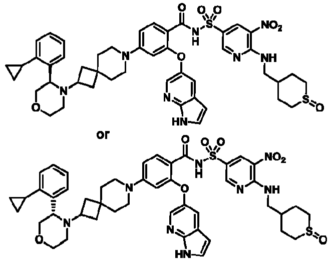 | (R or S)-2-((1H-pyrrolo[2,3-b]pyridin-5-yl)oxy)-4-(2-(3-(2-cyclopropyl phenyl)morpholino)-7-azaspiro[3. 5]nonan-7-yl)-N-((5-nitro-6-(((1-oxidotetrahydro-2H-thiopyran-4-yl)methyl)amino)pyridin-3-yl)sul fonyl)benzamide | MS-ESI (m/z): 909 [M + 1]⁺ |
| A-100 | 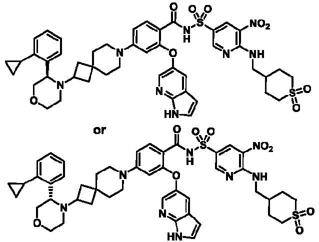 | (R or S)-2-((1H-pyrrolo[2,3-b]pyridin-5-yl)oxy)-4-(2-(3-(2-cyclopropyl phenyl)morpholino)-7-azaspiro[3. 5]nonan-7-yl)-N-((6-(((1,1-dioxid otetrahydro-2H-thiopyran-4-yl)m ethyl)amino)-5-nitropyridin-3-yl) sulfonyl)benzamide | MS-ESI (m/z): 925 [M + 1]⁺ |
| A-101 | 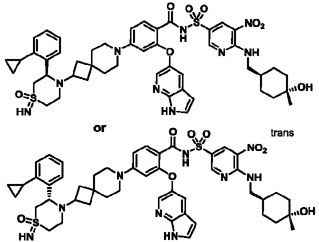 | 2-((1H-pyrrolo[2,3-b]pyridin-5-yl )oxy)-N-((6-((((1r,4r)-4-hydroxy-4-methylcyclohexyl)methyl)amin o)-5-nitropyridin-3-yl)sulfonyl)-4 -(2-((3R or 3S)-1-imino-3-(2-cyclopropylphenyl)-1-oxido-1λ⁶-thiomorpholino)-7-azaspiro[3.5]n onan-7-yl)benzamide | MS-ESI (m/z): 954 [M + 1]⁺ |
| A-102 | 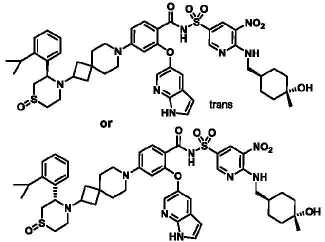 | 2-((1H-pyrrolo[2,3-b]pyridin-5-yl )oxy)-N-((6-((((1r,4r)-4-hydroxy-4-methylcyclohexyl)methyl)amin o)-5-nitropyridin-3-yl)sulfonyl)-4 -(2-((3R or 3S)-3-(2-isopropylphenyl)-1-oxid othiomorpholino)-7-azaspiro[3.5] nonan-7-yl)benzamide | MS-ESI (m/z): 939 [M + 1]⁺ |
| A-103 | 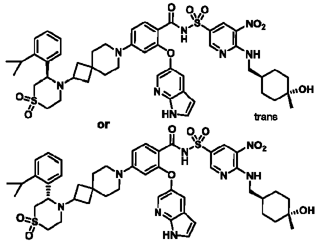 | 2-((1H-pyrrolo[2,3-b]pyridin-5-yl )oxy)-N-((6-((((1r,4r)-4-hydroxy-4-methylcyclohexyl)methyl)amin o)-5-nitropyridin-3-yl)sulfonyl)-4 -(2-((R or S)-3-(2-isopropylphenyl)-1,1-dio xidothiomorpholino)-7-azaspiro[ 3.5]nonan-7-yl)benzamide | MS-ESI (m/z): 955 [M + 1]⁺ |
| A-104 | 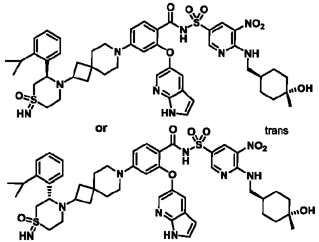 | 2-((1H-pyrrolo[2,3-b]pyridin-5-yl )oxy)-N-((6-((((1r,4r)-4-hydroxy-4-methylcyclohexyl)methyl)amin o)-5-nitropyridin-3-yl)sulfonyl)-4 -(2-((3R or 3S)-1-imino-3-(2-isopropylpheny l)-1-oxido-1λ⁶-thiomorpholino)-7 -azaspiro[3.5]nonan-7-yl)benzam ide | MS-ESI (m/z): 954 [M + 1]⁺ |
| A-105 | 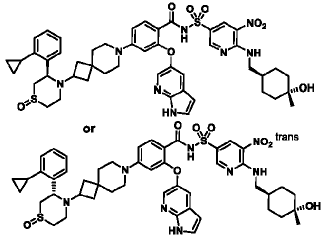 | 2-((1H-pyrrolo[2,3-b]pyridin-5-yl )oxy)-4-(2-((3R or 3S)-3-(2-cyclopropylphenyl)-1-o xidothiomorpholino)-7-azaspiro[ 3.5]nonan-7-yl)-N-((6-((((lr,4r)-4 -hydroxy-4-methylcyclohexyl)me thyl)amino)-5-nitropyridin-3-yl)s ulfonyl)benzamide | MS-ESI (m/z): 937 [M + 1]⁺ |
| A-106 | 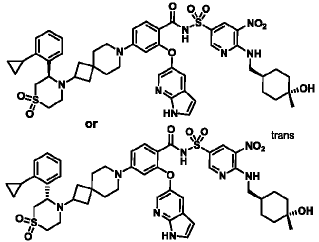 | 2-((1H-pyrrolo[2,3-b]pyridin-5-yl )oxy)-4-(2-((R or S)-3-(2-cyclopropylphenyl)-1,1-dioxidothiomorpholino)-7-azaspiro [3.5]nonan-7-yl)-N-((6-((((1r,4r)-4-hydroxy-4-methylcyclohexyl)m ethyl)amino)-5-nitropyridin-3-yl) sulfonyl)benzamide | MS-ESI (m/z): 953 [M + 1]⁺ |
| A-107 | 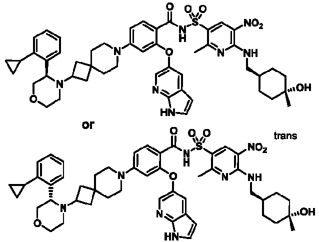 | 2-((1H-pyrrolo[2,3-b]pyridin-5-yl )oxy)-4-(2-((R or S)-3-(2-cyclopropylphenyl)morp holino)-7-azaspiro[3.5]nonan-7-y l)-N-((6-((((1r,4r)-4-hydroxy-4-m ethylcyclohexyl)methyl)amino)-2 -methyl-5-nitropyridin-3-yl)sulfo nyl)benzamide | MS-ESI (m/z): 919 [M + 1]⁺ |
| A-108 | 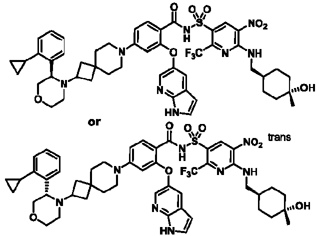 | 2-((1H-pyrrolo[2,3-b]pyridin-5-yl )oxy)-4-(2-((R or S)-3-(2-cyclopropylphenyl)morp holino)-7-azaspiro[3.5]nonan-7-yl )-N-((6-((((1r,4r)-4-hydroxy-4-m ethylcyclohexyl)methyl)amino)-5 -nitro-2-(trifluoromethyl)pyridin-3-yl)sulfonyl)benzamide | MS-ESI (m/z): 973 [M + 1]⁺ |
| A-109 | 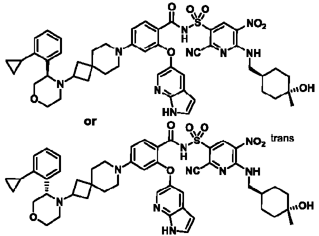 | 2-((1H-pyrrolo[2,3-b]pyridin-5-yl )oxy)-N-((2-cyano-6-((((1r,4r)-4-hydroxy-4-methylcyclohexyl)met hyl)amino)-5-nitropyridin-3-yl)su lfonyl)-4-(2-((R or S)-3-(2-cyclopropylphenyl)morp holino)-7-azaspiro[3.5]nonan-7-y l)benzamide | MS-ESI (m/z): 930 [M + 1]⁺ |
| A-110 | 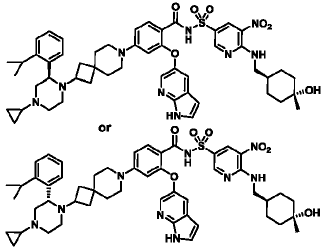 | 2-((1H-pyrrolo[2,3-b]pyridin-5-yl )oxy)-4-(2-((R or S)-4-cyclopropyl-2-(2-isopropylp henyl)piperazin-1-yl)-7-azaspiro[ 3.5]nonan-7-yl)-N-((6-((((1r,4r)-4 -hydroxy-4-methylcyclohexyl)me thyl)amino)-5-nitropyridin-3-yl) sulfonyl)benzamide | MS-ESI (m/z): 946 [M + 1]⁺ |
| A-111 | 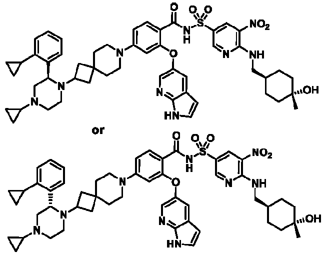 | 2-((1H-pyrrolo[2,3-b]pyridin-5-yl )oxy)-4-(2-((R or S)-4-cyclopropyl-2-(2-cycloprop ylphenyl)piperazin-1-yl)-7-azaspi ro[3.5]nonan-7-yl)-N-((6-((((1r,4r )-4-hydroxy-4-methylcyclohexyl) methyl)amino)-5-nitropyridin-3-yl)sulfonyl)benzamide | MS-ESI (m/z): 944 [M + 1]⁺ |
| A-112 | 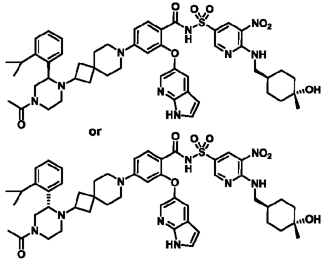 | 2-((1H-pyrrolo[2,3-b]pyridin-5-yl )oxy)-4-(2-((R or S)-4-acetyl-2-(2-isopropylphenyl) piperazin-1-yl)-7-azaspiro[3.5]no nan-7-yl)-N-((6-((((1r,4r)-4-hydro xy-4-methylcyclohexyl)methyl)a mino)-5-nitropyridin-3-yl)sulfony l)benzamide | MS-ESI (m/z): 948 [M + 1]⁺ |
| A-113 | 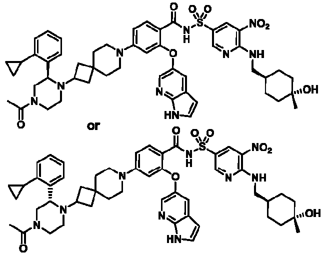 | 2-((1H-pyrrolo[2,3-b]pyridin-5-yl )oxy)-4-(2-((R or S)-4-acetyl-2-(2-cyclopropylphen yl)piperazin-1-yl)-7-azaspiro[3.5] nonan-7-yl)-N-((6-((((1r,4r)-4-hy droxy-4-methylcyclohexyl)methy l)amino)-5-nitropyridin-3-yl)sulf onyl)benzamide | MS-ESI (m/z): 946 [M + 1]⁺ |
| A-114 | 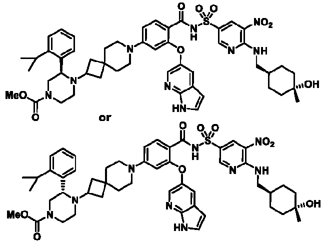 | methyl (R or S)-4-(7-(3-((1H-pyrrolo[2,3-b]pyr idin-5-yl)oxy)-4-(((6-((((1r,4r)-4-hydroxy-4-methylcyclohexyl)met hyl)amino)-5-nitropyridin-3-yl)su lfonyl)carbamoyl)phenyl)-7-azas piro[3.5]nonan-2-yl)-3-(2-isoprop ylphenyl)piperazine-1-carboxylat e | MS-ESI (m/z): 964 [M + 1]⁺ |
| A-115 | 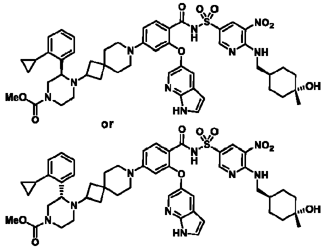 | methyl (R or S)-4-(7-(3-((1H-pyrrolo[2,3-b]pyr idin-5-yl)oxy)-4-(((6-((((1r,4r)-4-hydroxy-4-methylcyclohexyl)met hyl)amino)-5-nitropyridin-3-yl)su lfonyl)carbamoyl)phenyl)-7-azas piro[3.5]nonan-2-yl)-3-(2-cyclopr opylphenyl)piperazine-1-carboxy late | MS-ESI (m/z): 962 [M + 1]⁺ |
| A-116 | 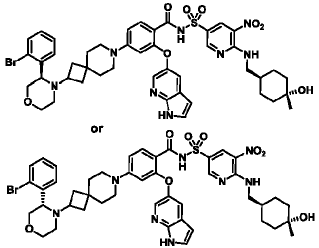 | 2-((1H-pyrrolo[2,3-b]pyridin-5-yl )oxy)-4-(2-((R or S)-3-(2-bromophenyl)morpholino )-7-azaspiro[3.5]nonan-7-yl)-N-(( 6-((((1r,4r)-4-hydroxy-4-methylc yclohexyl)methyl)amino)-5-nitro pyridin-3-yl)sulfonyl)benzamide | MS-ESI (m/z): 943, 945 [M + 1]⁺ |
| A-117 | 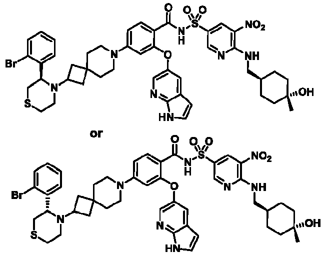 | 2-((1H-pyrrolo[2,3-b]pyridin-5-yl )oxy)-4-(2-((R or S)-3-(2-bromophenyl)thiomorpho lino)-7-azaspiro[3.5]nonan-7-yl)-N-((6-((((1r,4r)-4-hydroxy-4-met hylcyclohexyl)methyl)amino)-5-nitropyridin-3-yl)sulfonyl)benzami de | MS-ESI (m/z): 959, 961 [M + 1]⁺ |
| A-118 | 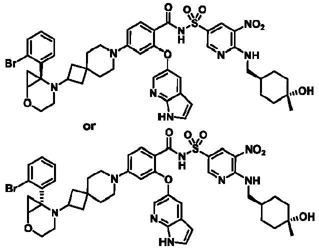 | 2-((1H-pyrrolo[2,3-b]pyridin-5-yl )oxy)-4-(2-((6R or S)-6-(2-bromophenyl)-2-oxa-5-az abicyclo[4.1.0]heptan-5-yl)-7-aza spiro[3.5]nonan-7-yl)-N-((6-((((1r ,4r)-4-hydroxy-4-methylcyclohex yl)methyl)amino)-5-nitropyridin-3-yl)sulfonyl)benzamide | MS-ESI (m/z): 955, 957 [M + 1]⁺ |
| A-119 | 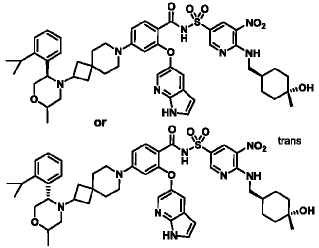 | 2-((1H-pyrrolo[2,3-b]pyridin-5-yl )oxy)-N-((6-((((1r,4r)-4-hydroxy-4-methylcyclohexyl)methyl)amin o)-5-nitropyridin-3-yl)sulfonyl)-4 -(2-((5R or 5S)-5-(2-isopropylphenyl)-2-met hylmorpholino)-7-azaspiro[3.5]n onan-7-yl)benzamide | MS-ESI (m/z): 921 [M + 1]⁺ |
| A-120 | 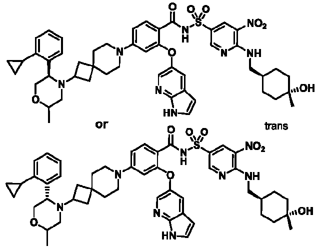 | 2-((1H-pyrrolo[2,3-b]pyridin-5-yl )oxy)-4-(2-((5R or 5S)-5-(2-cyclopropylphenyl)-2-m ethylmorpholino)-7-azaspiro[3.5] nonan-7-yl)-N-((6-((((1r,4r)-4-hy droxy-4-methylcyclohexyl)methy l)amino)-5-nitropyridin-3-yl)sulf onyl)benzamide | MS-ESI (m/z): 919 [M + 1]⁺ |
| A-121 | 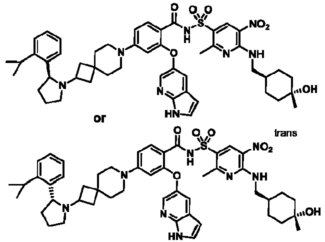 | 2-((1H-pyrrolo[2,3-b]pyridin-5-yl )oxy)-N-((6-((((1r,4r)-4-hydroxy-4-methylcyclohexyl)methyl)amin o)-2-methyl-5-nitropyridin-3-yl)s ulfonyl)-4-(2-((R or S)-2-(2-isopropylphenyl)pyrrolidi n-1-yl)-7-azaspiro[3.5]nonan-7-yl )benzamide | MS-ESI (m/z): 905 [M + 1]⁺ |
| A-122 | 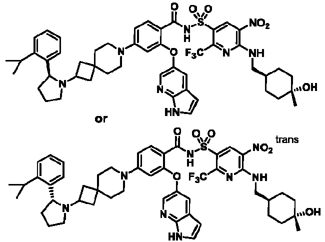 | 2-((1H-pyrrolo[2,3-b]pyridin-5-yl )oxy)-N-((6-((((1r,4r)-4-hydroxy-4-methylcyclohexyl)methyl)amin o)-5-nitro-2-(trifluoromethyl)pyri din-3-yl)sulfonyl)-4-(2-((R or S)-2-(2-isopropylphenyl)pyrrolidi n-1-yl)-7-azaspiro[3.5]nonan-7-yl )benzamide | MS-ESI (m/z): 959 [M + 1]⁺ |
| A-123 | 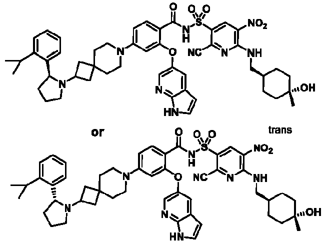 | 2-((1H-pyrrolo[2,3-b]pyridin-5-yl )oxy)-N-((2-cyano-6-((((1r4dr)-4-hydroxy-4-methylcyclohexyl)met hyl)amino)-5-nitropyridin-3-yl)su lfonyl)-4-(2-((R or S)-2-(2-isopropylphenyl)pyrrolidi n-1-yl)-7-azaspiro[3.5]nonan-7-yl )benzamide | MS-ESI (m/z): 916 [M + 1]⁺ |
| A-124 | 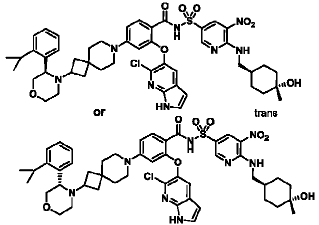 | 2-((6-chloro-1H-pyrrolo[2,3-b]py ridin-5-yl)oxy)-N-((6-((((1r,4r)-4-hydroxy-4-methylcyclohexyl)met hyl)amino)-5-nitropyridin-3-yl)su lfonyl)-4-(2-((R or S)-3-(2-isopropylphenyl)morphol ino)-7-azaspiro[3.5]nonan-7-yl)b enzamide | MS-ESI (m/z): 941 [M + 1]⁺ |
| A-125 | 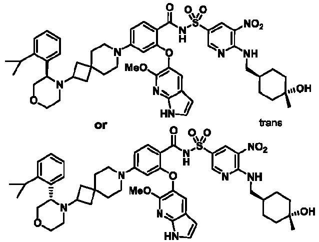 | N-((6-((((1r,4r)-4-hydroxy-4-met hylcyclohexyl)methyl)amino)-5-n itropyridin-3-yl)sulfonyl)-4-(2-(( R or S)-3-(2-isopropylphenyl)morphol ino)-7-azaspiro[3.5]nonan-7-yl)-2 -((6-methoxy-1H-pyrrolo[2,3-b]p yridin-5-yl)oxy)benzamide | MS-ESI (m/z): 937 [M + 1]⁺ |
| A-126 | 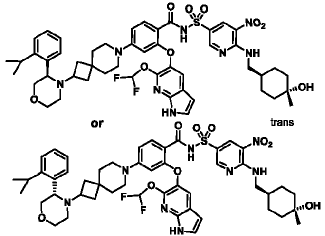 | 2-((6-(difluoromethoxy)-1H-pyrr olo[2,3-b]pyridin-5-yl)oxy)-N-((6 -((((1r,4r)-4-hydroxy-4-methylcy clohexyl)methyl)amino)-5-nitrop yridin-3-yl)sulfonyl)-4-(2-((R or S)-3-(2-isopropylphenyl)morphol ino)-7-azaspiro[3.5]nonan-7-yl)b enzamide | MS-ESI (m/z): 973 [M + 1]⁺ |
| A-127 | 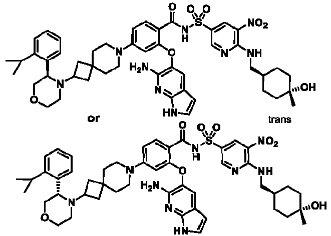 | 2-((6-amino- 1H-pyrrolo[2,3-b]py ridin-5-yl)oxy)-N-((6-((((1r,4r)-4-hydroxy-4-methylcyclohexyl)met hyl)amino)-5-nitropyridin-3-yl)su lfonyl)-4-(2-((R or S)-3-(2-isopropylphenyl)morphol ino)-7-azaspiro[3.5]nonan-7-yl)b enzamide | MS-ESI (m/z): 922 [M + 1]⁺ |
| A-128 | 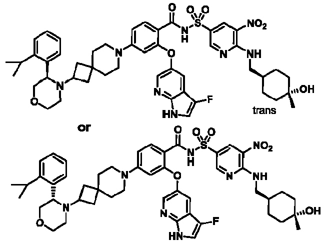 | 2-((3-fluoro-1H-pyrrolo[2,3-b]py ridin-5-yl)oxy)-N-((6-((((1r,4r)-4-hydroxy-4-methylcyclohexyl)met hyl)amino)-5-nitropyridin-3-yl)su lfonyl)-4-(2-((R or S)-3-(2-isopropylphenyl)morphol ino)-7-azaspiro[3.5]nonan-7-yl)b enzamide | MS-ESI (m/z): 925 [M + 1]⁺ |
| A-129 | 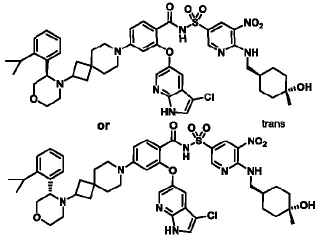 | 2-((3-chloro-1H-pyrrolo[2,3-b]py ridin-5-yl)oxy)-N-((6-((((1r,4r)-4-hydroxy-4-methylcyclohexyl)met hyl)amino)-5-nitropyridin-3-yl)su lfonyl)-4-(2-((R or S)-3-(2-isopropylphenyl)morphol ino)-7-azaspiro[3.5]nonan-7-yl)b enzamide | MS-ESI (m/z): 941 [M + 1]⁺ |
| A-130 | 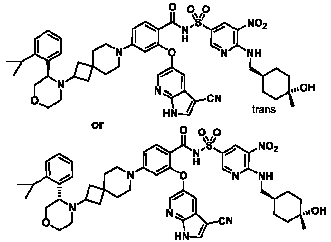 | 2-((3-cyano-1H-pyrrolo[2,3-b]pyr idin-5-yl)oxy)-N-((6-((((1r,4r)-4-hydroxy-4-methylcyclohexyl)met hyl)amino)-5-nitropyridin-3-yl)su lfonyl)-4-(2-((R or S)-3-(2-isopropylphenyl)morphol ino)-7-azaspiro[3.5]nonan-7-yl)b enzamide | MS-ESI (m/z): 932 [M + 1]⁺ |
| A-131 | 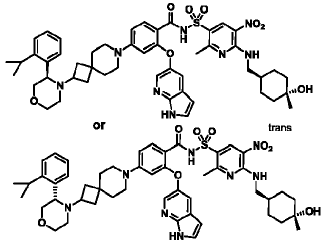 | 2-((1H-pyrrolo[2,3-b]pyridin-5-yl )oxy)-N-((6-((((1r,4r)-4-hydroxy-4-methylcyclohexyl)methyl)amin o)-2-methyl-5-nitropyridin-3-yl) sulfonyl)-4-(2-((R or S)-3-(2-isopropylphenyl)morphol ino)-7-azaspiro[3.5]nonan-7-yl)b enzamide | MS-ESI (m/z): 921 [M + 1]⁺ |
| A-132 | 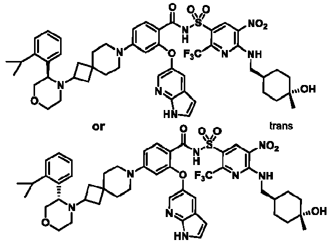 | 2-((1H-pyrrolo[2,3-b]pyridin-5-yl )oxy)-N-((6-((((1r,4r)-4-hydroxy-4-methylcyclohexyl)methyl)amin o)-5-nitro-2-(trifluoromethyl)pyri din-3-yl)sulfonyl)-4-(2-((R or S)-3-(2-isopropylphenyl)morphol ino)-7-azaspiro[3.5]nonan-7-yl)b enzamide | MS-ESI (m/z): 975 [M + 1]⁺ |
| A-133 | 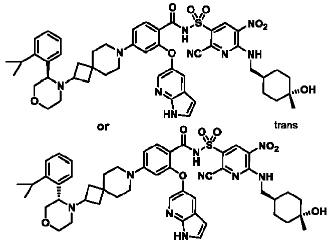 | 2-((1H-pyrrolo[2,3-b]pyridin-5-yl )oxy)-N-((2-cyano-6-((((1r4dr)-4-hydroxy-4-methylcyclohexyl)met hyl)amino)-5-nitropyridin-3-yl)su lfonyl)-4-(2-((R or S)-3-(2-isopropylphenyl)morphol ino)-7-azaspiro[3.5]nonan-7-yl) benzamide | MS-ESI (m/z): 932 [M + 1]⁺ |
| A-134 | 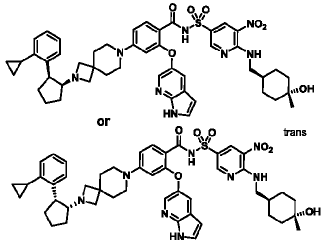 | 2-((1H-pyrrolo[2,3-b]pyridin-5-yl )oxy)-4-(2-((1R, 2R or 1S, 2S)-2-(2-cyclopropylphenyl)cycl opentyl)-2,7-diazaspiro[3.5]nona n-7-yl)-N-((6-((((1r,4r)-4-hydrox y-4-methylcyclohexyl)methyl)am ino)-5-nitropyridin-3-yl)sulfonyl) benzamide | MS-ESI (m/z): 889 [M + 1]⁺ |
| A-135 | 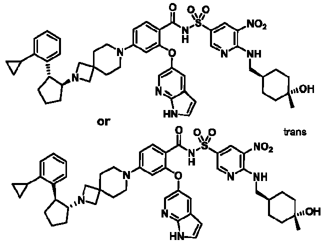 | 2-((1H-pyrrolo[2,3-b]pyridin-5-yl )oxy)-4-(2-((1R, 2S or 1S, 2R)-2-(2-cyclopropylphenyl)cycl opentyl)-2,7-diazaspiro[3.5]nona n-7-yl)-N-((6-((((1r,4r)-4-hydrox y-4-methylcyclohexyl)methyl)am ino)-5-nitropyridin-3-yl)sulfonyl) benzamide | MS-ESI (m/z): 889 [M + 1]⁺ |
| A-136 | 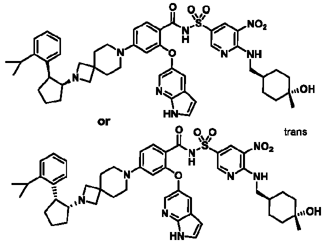 | 2-((1H-pyrrolo[2,3-b]pyridin-5-yl )oxy)-N-((6-((((1r,4r)-4-hydroxy-4-methylcyclohexyl)methyl)amin o)-5-nitropyridin-3-yl)sulfonyl)-4 -(2-((1R, 2R or 1S, 2S)-2-(2-isopropylphenyl)cyclop entyl)-2,7-diazaspiro[3.5]nonan-7 -yl)benzamide | MS-ESI (m/z): 891 [M + 1]⁺ |
| A-137 | 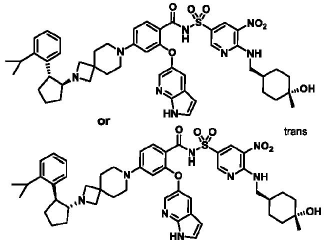 | 2-((1H-pyrrolo[2,3-b]pyridin-5-yl )oxy)-N-((6-((((1r,4r)-4-hydroxy-4-methylcyclohexyl)methyl)amin o)-5-nitropyridin-3-yl)sulfonyl)-4 -(2-((1R, 2S or 1S, 2R)-2-(2-isopropylphenyl)cyclop entyl)-2,7-diazaspiro[3.5]nonan-7 -yl)benzamide | MS-ESI (m/z): 891 [M + 1]⁺ |
| A-138 | 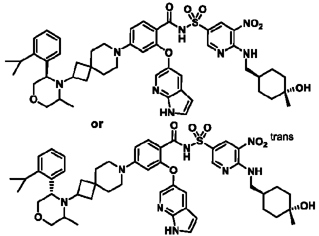 | 2-((1H-pyrrolo[2,3-b]pyridin-5-yl)o xy)-N-((6-((((1r,4r)-4-hydroxy-4-me thylcyclohexyl)methyl)amino)-5-nit ropyridin-3-yl)sulfonyl)-4-(2-((3R or 3S)-3-(2-isopropylphenyl)-5-methyl morpholino)-7-azaspiro[3.5]nonan-7 -yl)benzamide | MS-ESI (m/z): 921 [M + 1]⁺ |
| A-139 | 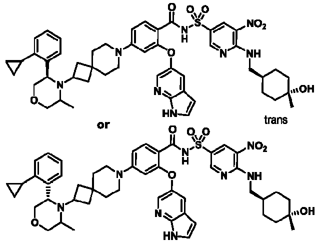 | 2-((1H-pyrrolo[2,3-b]pyridin-5-yl)o xy)-4-(2-((3R or 3S)-3-(2-cyclopropylphenyl)-5-met hylmorpholino)-7-azaspiro[3.5]nona n-7-yl)-N-((6-((((1r,4r)-4-hydroxy-4 -methylcyclohexyl)methyl)amino)-5 -nitropyridin-3-yl)sulfonyl)benzami de | MS-ESI (m/z): 919 [M + 1]⁺ |
| A-140 | 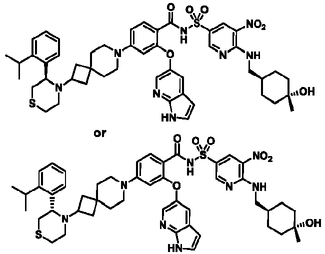 | 2-((1H-pyrrolo[2,3-b]pyridin-5-yl) oxy)-N-((6-((((1r,4r)-4-hydroxy-4-me thylcyclohexyl)methyl)amino)-5-nit ropyridin-3-yl)sulfonyl)-4-(2-((R or S)-3-(2-isopropylphenyl)thiomorph olino)-7-azaspiro[3.5]nonan-7-yl)be nzamide | MS-ESI (m/z): 923 [M + 1]⁺ |
| A-141 | 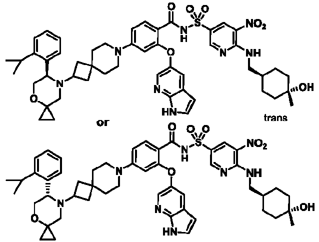 | 2-((1H-pyrrolo[2,3-b]pyridin-5-yl)o xy)-N-((6-((((1r,4r)-4-hydroxy-4-me thylcyclohexyl)methyl)amino)-5-nit ropyridin-3-yl)sulfonyl)-4-(2-((R or S)-6-(2-isopropylphenyl)-4-oxa-7-a zaspiro[2.5]octan-7-yl)-7-azaspiro[3 .5]nonan-7-yl)benzamide | MS-ESI (m/z): 933 [M + 1]⁺ |
| A-142 | 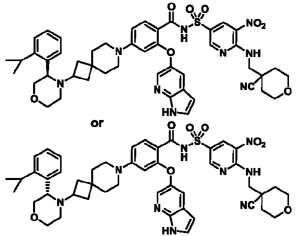 | (R or S)-2-((1H-pyrrolo[2,3-b]pyridin-5-y l)oxy)-N-((6-(((4-cyanotetrahydro-2 H-pyran-4-yl)methyl)amino)-5-nitro pyridin-3-yl)sulfonyl)-4-(2-(3-(2-iso propylphenyl)morpholino)-7-azaspin o[3.5]nonan-7-yl)benzamide | MS-ESI (m/z): 904 [M + 1]⁺ |
| A-143 | 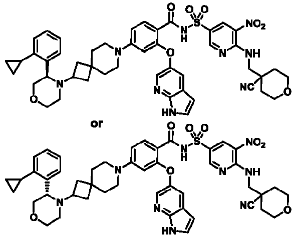 | (R or S)--2-((1H-pyrrolo[2,3-b]pyridin-5-yl) oxy)-N-((6-(((4-cyanotetrahydro-2H -pyran-4-yl)methyl)amino)-5-nitrop yridin-3-yl)sulfonyl)-4-(2-(3-(2-cycl opropylphenyl)morpholino)-7-azasp iro[3.5]nonan-7-yl)benzamide | MS-ESI (m/z): 902 [M + 1]⁺ |
| A-144 | 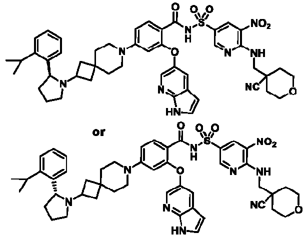 | (R or S)-2-((1H-pyrrolo[2,3-b]pyridin-5-y l)oxy)-N-((6-(((4-cyanotetrahydro-2 H-pyran-4-yl)methyl)amino)-5-nitro pyridin-3-yl)sulfonyl)-4-(2-(2-(2-iso propylphenyl)pyrrolidin-1-yl)-7-aza spiro[3.5]nonan-7-yl)benzamide | MS-ESI (m/z): 888 [M + 1]⁺ |
| A-145 | 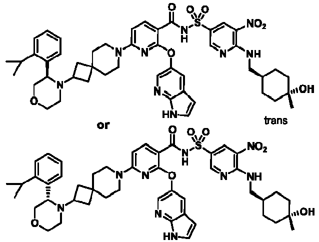 | 2-((1H-pyrrolo[2,3-b]pyridin-5-yl) oxy)-N-((6-((((1r,4r)-4-hydroxy-4-me thylcyclohexyl)methyl)amino)-5-nit ropyridin-3-yl)sulfonyl)-6-(2-((R or S)-3-(2-isopropylphenyl)morpholino )-7-azaspiro[3.5]nonan-7-yl)nicoti namide | MS-ESI (m/z): 908 [M + 1]⁺ |
| A-146 | 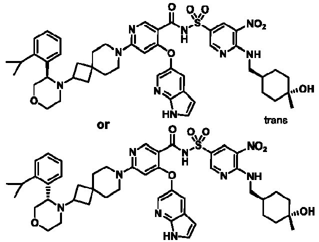 | 4-((1H-pyrrolo[2,3-b]pyridin-5-yl)o xy)-N-((6-((((1r,4r)-4-hydroxy-4-me thylcyclohexyl)methyl)amino)-5-nit ropyridin-3-yl)sulfonyl)-6-(2-((R or S)-3-(2-isopropylphenyl)morpholin o)-7-azaspiro[3.5]nonan-7-yl)nicoti namide | MS-ESI (m/z): 908 [M + 1]⁺ |
| A-147 | 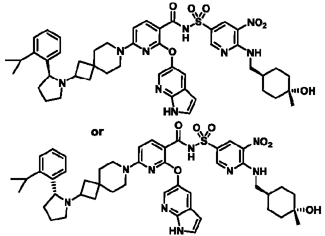 | 2-((1H-pyrrolo[2,3-b]pyridin-5-yl)o xy)-N-((6-((((1r,4r)-4-hydroxy-4-me thylcyclohexyl)methyl)amino)-5-nit ropyridin-3-yl)sulfonyl)-6-(2-((R or S)-2-(2-isopropylphenyl)pyrrolidin-1-yl)-7-azaspiro[3.5]nonan-7-yl)nic otinamide | MS-ESI 892 (m/z):[M + 1]⁺ |
| A-148 | 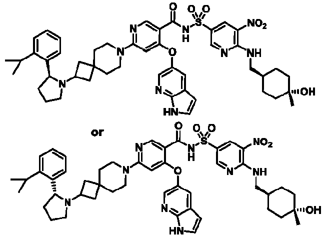 | 4-((1H-pyrrolo[2,3-b]pyridin-5-yl)o xy)-N-((6-((((1r,4r)-4-hydroxy-4-me thylcyclohexyl)methyl)amino)-5-nit ropyridin-3-yl)sulfonyl)-6-(2-((R or S)-2-(2-isopropylphenyl)pyrrolidin-1-yl)-7-azaspiro[3.5]nonan-7-yl)nic otinamide | MS-ESI (m/z): [M + 1]⁺ |
| A-149 | 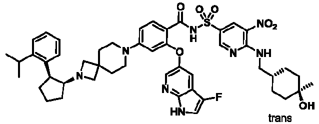 | 2-((3-fluoro-1*H*-pyrrolo[2,3-b]pyrid in-5-yl)oxy)-*N*-((6-((((1*r*,4*r*)-4-hydr oxy-4-methylcyclohexyl)methyl)am ino)-5-nitropyridin-3-yl)sulfonyl)-4-(2-((1*S*,2*S* or 1*R*,2*R*)-2-(2-isopropylphenyl)cyclop entyl)-2,7-diazaspiro[3.5]nonan-7-yl )benzamide | MS-ESI (m/z): 909 [M + 1]⁺ |
| | or 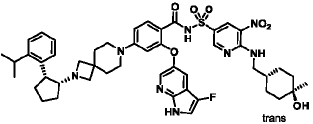 | | |
| A-150 | 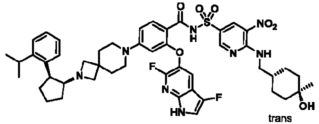 | 2-((3,6-difluoro-1H-pyrrolo[2,3-b]p yridin-5-yl)oxy)-*N*-((6-((((1*r*,4*r*)-4-h ydroxy-4-methylcyclohexyl)methyl) amino)-5-nitropyridin-3-yl)sulfonyl) -4-(2-((1*S*,2*S* or 1*R*,2*R*)-2-(2-isopropylphenyl)cyclop entyl)-2,7-diazaspiro[3.5]nonan-7-yl )benzamide | MS-ESI (m/z): 927 [M + 1]⁺ |
| | or 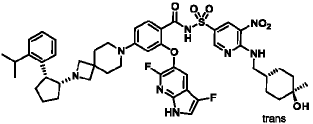 | | |
| A-151 | 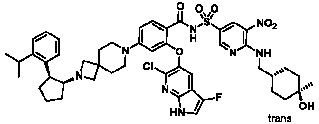 | 2-((6-chloro-3-fluoro-1*H*-pyrrolo[2, 3-*b*]pyridin-5-yl)oxy)-*N-*((6-((((1*r*,4 *r*)-4-hydroxy-4-methylcyclohexyl)m ethyl)amino)-5-nitropyridin-3-yl)sul fonyl)-4-(2-((1*S*,2*S* or 1*R*,2*R*)-2-(2-isopropylphenyl)cyclop entyl)-2,7-diazaspiro[3.5]nonan-7-yl )benzamide | MS-ESI (m/z): 944 [M + 1]⁺ |
| | or 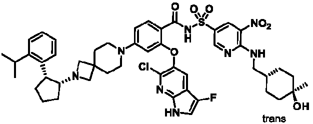 | | |
| A-152 | 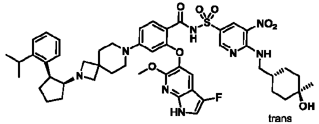 | 2-((3-fluoro-6-methoxy-1*H*-pyrrolo[ 2,3-*b*]pyridin-5-yl)oxy)-*N*-((6-((((1*r* ,4*r*)-4-hydroxy-4-methylcyclohexyl) methyl)amino)-5-nitropyridin-3-yl)s ulfonyl)-4-(2-((1S,2S or 1*R*,2*R*)-2-(2-isopropylphenyl)cyclop entyl)-2,7-diazaspiro[3.5]nonan-7-yl )benzamide | MS-ESI (m/z): 939 [M + 1]⁺ |
| | or 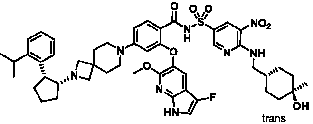 | | |
| A-153 | 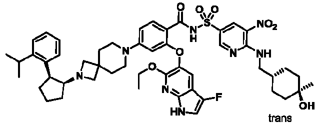 | 2-((6-ethoxy-3-fluoro-1*H*-pyrrolo[2, 3-*b*]pyridin-5-yl)oxy)-*N*-((6-((((1*r*,4 *r*)-4-hydroxy-4-methylcyclohexyl)m ethyl)amino)-5-nitropyridin-3-yl)sul fonyl)-4-(2-((1*S*,2*S* or 1*R*,2*R*)-2-(2-isopropylphenyl)cyclop entyl)-2,7-diazaspiro[3.5]nonan-7-yl )benzamide | MS-ESI (m/z): 953 [M + 1]⁺ |
| | or 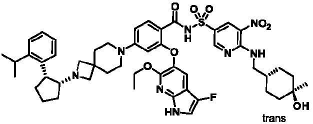 | | |
| A-154 | 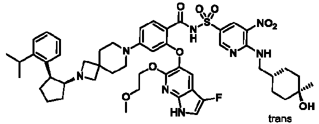 | 2-((3-fluoro-6-(2-methoxyethoxy)-1 *H*-pyrrolo[2,3-b]pyridin-5-yl)oxy)-*N*-((6-((((1*r*,4*r*)-4-hydroxy-4-methy cyclohexyl)methyl)amino)-5-nitrop yridin-3-yl)sulfonyl)-4-(2-((1*S*,2*S* or 1*R*,2*R*)-2-(2-isopropylphenyl)cyclop entyl)-2,7-diazaspiro[3.5]nonan-7-yl )benzamide | MS-ESI (m/z): 983 [M + 1]⁺ |
| | or 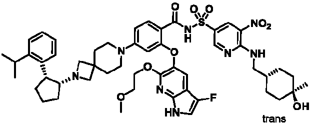 | | |
| A-155 | 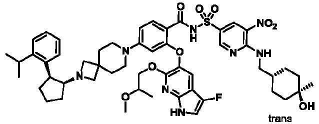 | 2-((3-fluoro-6-(2-methoxypropoxy)-1*H*-pyrrolo[2,3-*b*]pyridin-5-yl)oxy)-*N*-((6-((((1*r*,4*r*)-4-hydroxy-4-methy cyclohexyl)methyl)amino)-5-nitrop yridin-3-yl)sulfonyl)-4-(2-((1*S*,2*S* or 1*R*,2*R*)-2-(2-isopropylphenyl)cyclop entyl)-2,7-diazaspiro[3.5]nonan-7-yl )benzamide | MS-ESI (m/z): 997 [M + 1]⁺ |
| | or 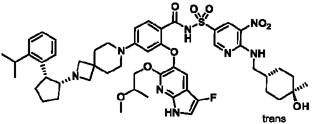 | | |
| A-155a | 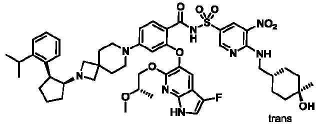 | 2-((3-fluoro-6-((*S*)-2-methoxypropo xy)-1*H*-pyrrolo[2,3-*b*]pyridin-5-yl)o xy)-*N*-((6-((((1*r*,4*r*)-4-hydroxy-4-m ethylcyclohexyl)methyl)amino)-5-ni tropyridin-3-yl)sulfonyl)-4-(2-((1*S*,2 S or 1*R*,2*R*)-2-(2-isopropylphenyl)cyclop entyl)-2,7-diazaspiro[3.5]nonan-7-yl )benzamide | MS-ESI (m/z): 997 [M + 1]⁺ |
| | or 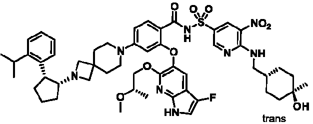 | | |
| A-155b | 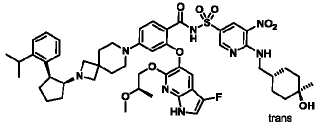 | 2-((3-fluoro-6-((*R*)-2-methoxypropo xy)-1*H*-pyrrolo[2,3-*b*]pyridin-5-yl)o xy)-*N*-((6-((((1*r*,4*r*)-4-hydroxy-4-m ethylcyclohexyl)methyl)amino)-5-ni tropyridin-3-yl)sulfonyl)-4-(2-((1*S*,2 *S* or 1*R*,2*R*)-2-(2-isopropylphenyl)cyclop entyl)-2,7-diazaspiro[3.5]nonan-7-yl )benzamide | MS-ESI (m/z): 997 [M + 1]⁺ |
| | or 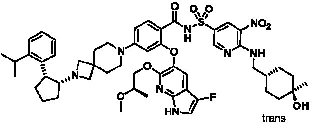 | | |
| A-156 | 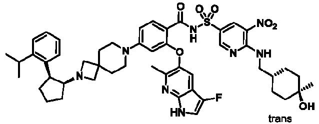 | 2-((3-fluoro-6-methyl-1*H*-pyrrolo[2, 3-*b*]pyridin-5-yl)oxy)-*N*-((6-((((1*r*,4 *r*)-4-hydroxy-4-methylcyclohexyl)m ethyl)amino)-5-nitropyridin-3-yl)sul fonyl)-4-(2-((1*S*,2*S* or 1*R*,2*R*)-2-(2-isopropylphenyl)cyclop entyl)-2,7-diazaspiro[3.5]nonan-7-yl )benzamide | MS-ESI (m/z): 923 [M + 1]⁺ |
| | or 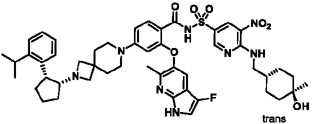 | | |
| A-157 | 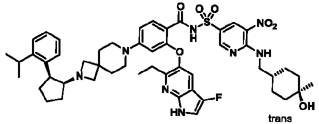 | 2-((6-ethyl-3-fluoro-1*H*-pyrrolo[2,3-*b*]pyridin-5-yl)oxy)-*N*-((6-((((1*r*,4*r*)-4-hydroxy-4-methylcyclohexyl)met nyl)amino)-5-nitropyridin-3-yl)sulfo nyl)-4-(2-((1*S*,2*S* or 1*R*,2*R*)-2-(2-isopropylphenyl)cyclop entyl)-2,7-diazaspiro[3.5]nonan-7-yl )benzamide | MS-ESI (m/z): 937 [M + 1]⁺ |
| | or 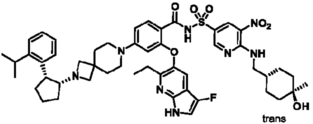 | | |
| A-158 | 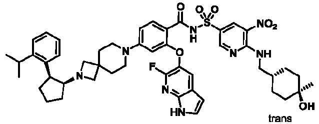 | 2-((6-fluoro-1*H*-pyrrolo[2,3-b]pyrid in-5-yl)oxy)-*N*-((6-((((1*r*,4*r*)-4-hydr oxy-4-methylcyclohexyl)methyl)am ino)-5-nitropyridin-3-yl)sulfonyl)-4-(2-((1*S*,2*S* or 1*R*,2*R*)-2-(2-isopropylphenyl)cyclop entyl)-2,7-diazaspiro[3.5]nonan-7-yl )benzamide | MS-ESI (m/z): 909 [M + 1]⁺ |
| | or 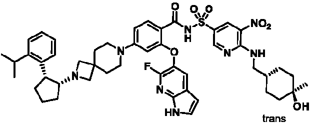 | | |
| A-159 | 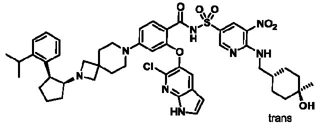 | 2-((6-chloro-1*H*-pyrrolo[2,3-*b*]pyrid in-5-yl)oxy)-*N*-((6-((((1*r*,4*r*)-4-hydr oxy-4-methylcyclohexyl)methyl)am ino)-5-nitropyridin-3-yl)sulfonyl)-4-(2-((1*S*,2*S* or 1*R*,2*R*)-2-(2-isopropylphenyl)cyclop entyl)-2,7-diazaspiro[3.5]nonan-7-yl )benzamide | MS-ESI (m/z): 925 [M + 1]⁺ |
| | or 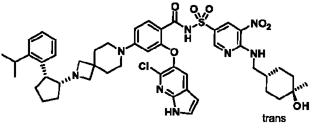 | | |
| A-160 | 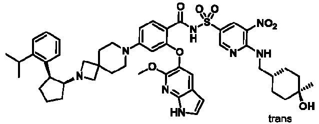 | *N*-((6-((((1*r*,4*r*)-4-hydroxy-4-methyl cyclohexyl)methyl)amino)-5-nitrop yridin-3-yl)sulfonyl)-4-(2-((1*S*,2*S* or 1*R*,2*R*)-2-(2-isopropylphenyl)cyclop entyl)-2,7-diazaspiro[3.5]nonan-7-yl )-2-((6-methoxy-1*H*-pyrrolo[2,3-*b*]p yridin-5-yl)oxy)benzamide | MS-ESI (m/z): 921 [M + 1]⁺ |
| | or 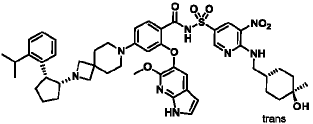 | | |
| A-161 | 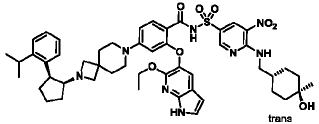 | 2-((6-ethoxy-1*H*-pyrrolo[2,3-*b*]pyri din-5-yl)oxy)-*N*-((6-((((1*r*,4*r*)-4-hyd roxy-4-methylcyclohexyl)methyl)a mino)-5-nitropyridin-3-yl)sulfonyl)-4-(2-((1S,2S or 1*R*,2*R*)-2-(2-isopropylphenyl)cyclop entyl)-2,7-diazaspiro[3.5]nonan-7-yl )benzamide | MS-ESI (m/z): 935 [M + 1]⁺ |
| | or 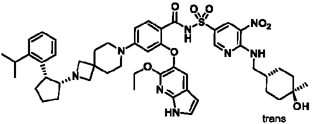 | | |
| A-162 | 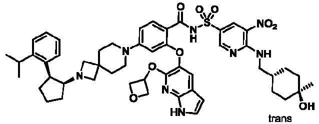 | *N*-((6-((((1*r*,4*r*)-4-hydroxy-4-methyl cyclohexyl)methyl)amino)-5-nitrop yridin-3-yl)sulfonyl)-4-(2-((1*S*,2*S* or 1*R*,2*R*)-2-(2-isopropylphenyl)cyclop entyl)-2,7-diazaspiro[3.5]nonan-7-yl )-2-((6-(oxetan-3-yloxy)-1*H*-pyrrolo [2,3-*b*]pyridin-5-yl)oxy)benzamide | MS-ESI (m/z): 963 [M + 1]⁺ |
| | or 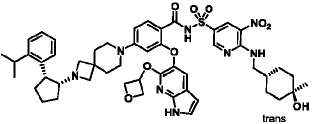 | | |
| A-163 | 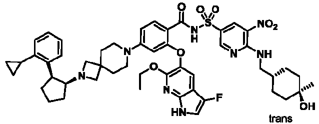 | 4-(2-((1S,2S or 1*R*,2*R*)-2-(2-cyclopropylphenyl)cycl opentyl)-2,7-diazaspiro[3.5]nonan-7 -yl)-2-((6-ethoxy-3-fluoro-1H-pyrro lo[2,3-*b*]pyridin-5-yl)oxy)-*N*-((6-(((( 1*r*,4*r*)-4-hydroxy-4-methylcyclohex yl)methyl)amino)-5-nitropyridin-3-y l)sulfonyl)benzamide | MS-ESI (m/z): 951 [M + 1]⁺ |
| | or 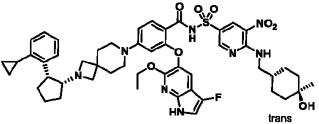 | | |
| A-164 | 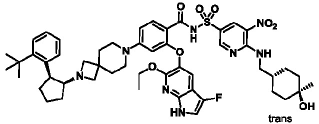 | 4-(2-((1*S*,2*S* or 1R,2R)-2-(2-(tert-butyl)phenyl)cycl opentyl)-2,7-diazaspiro[3.5]nonan-7 -yl)-2-((6-ethoxy-3-fluoro-1*H*-pyrro o[2,3-*b*]pyridin-5-yl)oxy)-*N*-((6-(((( 1r,4r)-4-hydroxy-4-methylcyclohex yl)methyl)amino)-5-nitropyridin-3-y l)sulfonyl)benzamide | MS-ESI (m/z): 967 [M + 1]⁺ |
| | or 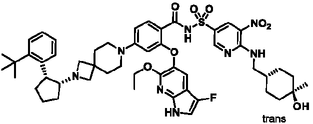 | | |
| A-165 | 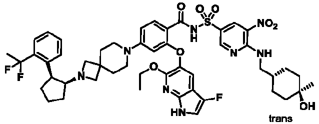 | 4-(2-((1*S*,2*S* or 1R,2R)-2-(2-(1,1-difluoroethyl)phen yl)cyclopentyl)-2,7-diazaspiro[3.5]n onan-7-yl)-2-((6-ethoxy-3-fluoro-1 *H*-pyrrolo[2,3-*b*]pyridin-5-yl)oxy)-*N*-((6-((((1*r*,4*r*)-4-hydroxy-4-methy cyclohexyl)methyl)amino)-5-nitrop yridin-3-yl)sulfonyl)benzamide | MS-ESI (m/z): 975 [M + 1]⁺ |
| | or 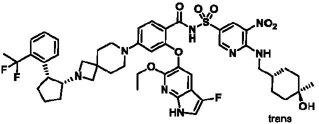 | | |
| A-166 | 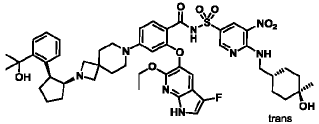 | 2-((6-ethoxy-3-fluoro-1*H*-pyrrolo[2, 3-*b*]pyridin-5-yl)oxy)-*N*-((6-((((1*r*,4 *r*)-4-hydroxy-4-methylcyclohexyl)m ethyl)amino)-5-nitropyridin-3-yl)sul fonyl)-4-(2-((1S,2S or 1*R*,2*R*)-2-(2-(2-hydroxypropan-2-yl) phenyl)cyclopentyl)-2,7-diazaspiro[ 3.5]nonan-7-yl)benzamide | MS-ESI (m/z): 969 [M + 1]⁺ |
| | or 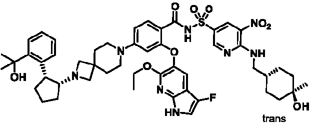 | | |
| A-167 | 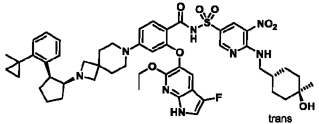 | 2-((6-ethoxy-3-fluoro-1*H*-pyrrolo[2, 3-*b*]pyridin-5-yl)oxy)-*N*-((6-((((1*r*,4 *r*)-4-hydroxy-4-methylcyclohexyl)m ethyl)amino)-5-nitropyridin-3-yl)sul fonyl)-4-(2-((1*S*,2*S* or 1*R*,2*R*)-2-(2-(1-methylcyclopropyl)p henyl)cyclopentyl)-2,7-diazaspiro[3, 5]nonan-7-yl)benzamide | MS-ESI (m/z): 965 [M + 1]⁺ |
| | or 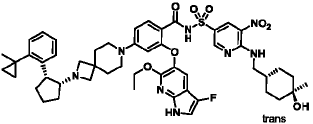 | | |
| A-168 | 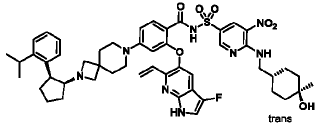 | 2-((3-fluoro-6-vinyl-1*H*-pyrrolo[2,3 -*b*]pyridin-5-yl)oxy)-*N*-((6-((((1*r*,4*r*) -4-hydroxy-4-methylcyclohexyl)met hyl)amino)-5-nitropyridin-3-yl)sulfo nyl)-4-(2-((1*S*,2*S* or 1*R*,2*R*)-2-(2-isopropylphenyl)cyclop entyl)-2,7-diazaspiro[3.5]nonan-7-yl )benzamide | MS-ESI (m/z): 935 [M + 1]⁺ |
| | or 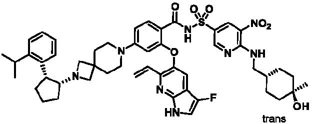 | | |
| A-169 | 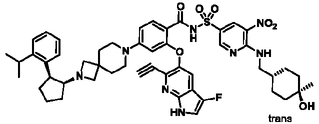 | 2-((6-ethynyl-3-fluoro-1*H*-pyrrolo[2 ,3-*b*]pyridin-5-yl)oxy)-*N*-((6-((((1*r,* 4r)-4-hydroxy-4-methylcyclohexyl) methyl)amino)-5-nitropyridin-3-yl)s ulfonyl)-4-(2-((1*S*,2*S* or 1*R*,2*R*)-2-(2-isopropylphenyl)cyclop entyl)-2,7-diazaspiro[3.5]nonan-7-yl )benzamide | MS-ESI (m/z): 933 [M + 1]⁺ |
| | or 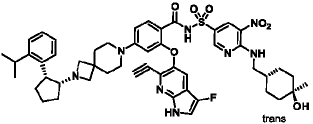 | | |
| A-170 | 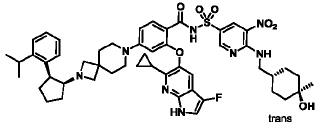 | 2-((6-cyclopropyl-3-fluoro-1*H*-pyrr olo[2,3-*b*]pyridin-5-yl)oxy)-*N*-((6-(( ((1*r*,4*r*)-4-hydroxy-4-methylcyclohe xyl)methyl)amino)-5-nitropyridin-3-yl)sulfonyl)-4-(2-((1*S*,2*S* or 1*R*,2*R*)-2-(2-isopropylphenyl)cyclop entyl)-2,7-diazaspiro[3.5]nonan-7-yl )benzamide | MS-ESI (m/z): 949 [M + 1]⁺ |
| | or 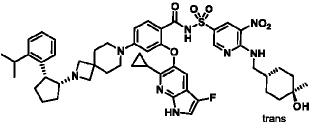 | | |
| A-171 | 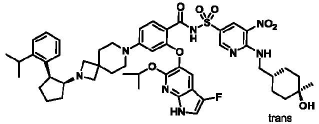 | 2-((3-fluoro-6-isopropoxy-1*H*-pyrro lo[2,3-*b*]pyridin-5-yl)oxy)-*N*-((6-(((( 1*r*,4*r*)-4-hydroxy-4-methylcyclohex yl)methyl)amino)-5-nitropyridin-3-y l)sulfonyl)-4-(2-((1*S*,2*S* or 1*R*,2*R*)-2-(2-isopropylphenyl)cyclop entyl)-2,7-diazaspiro[3.5]nonan-7-yl )benzamide | MS-ESI (m/z): 967 [M + 1]⁺ |
| | or 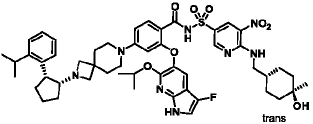 | | |
| A-172 | 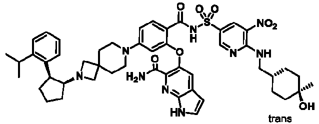 | 5-(2-(((6-((((1*r*,4*r*)-4-hydroxy-4-met hylcyclohexyl)methyl)amino)-5-nitr opyridin-3-yl)sulfonyl)carbamoyl)-5 -(2-((1*S*,2*S* or 1*R*,2*R*)-2-(2-isopropylphenyl)cyclop entyl)-2,7-diazaspiro[3.5]nonan-7-yl )phenoxy)-1*H*-pyrrolo[2,3-*b*]pyridin e-6-carboxamide | MS-ESI (m/z): 934 [M + 1]⁺ |
| | or 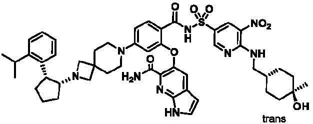 | | |
| A-173 | 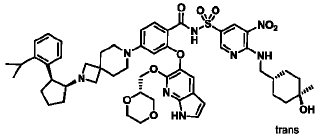 | 2-((6-(((*S*)-1,4-dioxan-2-yl)methoxy )-1*H*-pyrrolo[2,3-b]pyridin-5-yl)oxy )-N-((6-((((1*r*,4*r*)-4-hydroxy-4-meth ylcyclohexyl)methyl)amino)-5-nitro pyridin-3-yl)sulfonyl)-4-(2-((1*S*,2*S* or 1*R*,2*R*)-2-(2-isopropylphenyl)cyclop entyl)-2,7-diazaspiro[3.5]nonan-7-yl )benzamide | MS-ESI (m/z): 1007 [M + 1]⁺ |
| | or 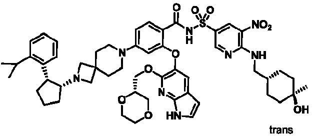 | | |

### Example B-1a

### (S)-2-((1H-pyrrolo[2,3-b]pyridin-5-yl)oxy)-4-(2-(2-(2-cyclopropylphenyl)pyr rolidin-1-yl)-7-azaspiro[3.5]nonan-7-yl)-N-((4-nitro-5-(((tetrahydro-2H-pyran-4-yl)methyl)a mino)pyridin-2-yl)sulfonyl)benzamide (B-1a)

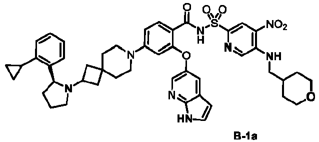

### 2-chloro-5-fluoropyridine 1-oxide (B-1-a)

To a solution of 2-chloro-5-fluoropyridine (7.7 g, 58 mmol) in TFA (180 ml) was added 30% H₂O₂ dropwise at 70°C, the mixture was stirred at the same temperature overnight. The reaction mixture was concentrated under reduced pressure to dryness. The residue was diluted with water, neutralized with 10% aqueous Na₂CO₃ and extracted with DCM / MeOH = 10:1, the extracts were concentrated. The residue was purified by recrystallization with EA : PE = 1:9 to give the title compound 2-chloro-5-fluoropyridine 1-oxide **(B-1-a).** MS-ESI (m/z): 148 [M + 1]⁺.

### 2-chloro-5-fluoro-4-nitropyridine 1-oxide (B-1-b)

A mixture of 2-chloro-5-fluoropyridine 1-oxide **(B-1-a)** (7.2 g, 49 mmol) and KNO₃ (22.0 g, 217 mmol) in H₂SO₄ (100 ml) was stirred at 120°C for 3 h. The mixture was cooled to RT and poured into ice (400 g), after stirred for 10 min and filtered. The wet cake was dissolved in DCM. The organic phase was washed with brine, dried and concentrated to give the title compound 2-chloro-5-fluoro-4-nitropyridine 1-oxide **(B-1-b).** MS-ESI (m/z): 193 [M + 1]⁺.

### 2-chloro-4-nitro-5-(((tetrahydro-2H-pyran-4-yl)methyl)amino)pyridine 1-oxide (B-1-c)

To a solution of 2-chloro-5-fluoro-4-nitropyridine 1-oxide **(B-1-b)** (542 mg, 2.82 mmol) in THF (25 ml) was added (tetrahydro-2H-pyran-4-yl)methanamine (487 mg, 4.23 mmol) and TEA (0.5 ml). The mixture was stirred at 20°C for 15 minutes. The reaction mixture was diluted with water and EtOAc. The mixture were acidified with 2 N HCl and filtrated to give the title compound 2-chloro-4-nitro-5-(((tetrahydro-2H-pyran-4-yl)methyl)amino)pyridine 1-oxide **(B-1-c).** MS-ESI (m/z): 288 [M + 1]⁺.

### 5-((tert-butoxycarbonyl)((tetrahydro-2H-pyran-4-yl)methyl)amino)-2-chloro-4-nitropyridine 1-oxide (B-1-d)

A mixture of 2-chloro-4-nitro-5-(((tetrahydro-2H-pyran-4-yl)methyl)amino)-pyridine 1-oxide **(B-1-c)** (470 mg, 1.64 mmol), (Boc)₂O (1.2 g, 5.5 mmol) and DMAP (300 mg, 2.46 mmol) in acetonitrile (10 ml) was stirred at 50°C for 0.5 h. The mixture was concentrated. The residue was purified by column chromatography on silica gel eluting with EtOAc /PE (1:3) to give the title compound 5-((*tert*-butoxycarbonyl)((tetrahydro-2*H*-pyran-4-yl)methyl)amino)- 2-chloro-4-nitropyridine 1-oxide **(B-1-d).** MS-ESI (m/z): 388 [M + 1]⁺.

### 2-(benzylthio)-5-((tert-butoxycarbonyl)((tetrahydro-2H-pyran-4-yl)methyl)a mino)-4-nitropyridine 1-oxide (B-1-e)

A mixture of 5-((tert-butoxycarbonyl)((tetrahydro-2H-pyran-4-yl)methyl)amino)-2-chloro-4-nitropyridine 1-oxide **(B-1-d)** (10 mg, 0.025 mmol), Na₂S (6 mg, 0.077mmol) and 18-crown-6 (10 mg, 0.038 mmol) in DMA (0.25 ml) was stirred at 20°C for 0.5 h. Then BnBr (30 mg, 0.175 mmol) was added and the mixture was stirred at 20°C for 10 minutes. The mixture was quenched with water and extracted with EtOAc. The extracts were washed with brine, dried with Na₂SO₄ and concentrated. The residue was purified with column chromatography on silica gel eluting with EtOAc / PE (1:1) to give the title compound 2-(benzylthio)-5-((*tert*-butoxycarbonyl)((tetrahydro-2*H*-pyran-4-yl)methyl)amino)-4-nitropyri dine 1-oxide **(B-1-e).** MS-ESI (m/z): 476 [M + 1]⁺.

### 5-((tert-butoxycarbonyl)((tetrahydro-2H-pyran-4-yl)methyl)amino)-4-nitro-2 -sulfamoylpyridine 1-oxide (B-1-f)

To a solution of 2-(benzylthio)-5-((tert-butoxycarbonyl)((tetrahydro-2H-pyran-4-yl)methyl)amino)-4-nitropyridine 1-oxide **(B-1-e)** (167 mg, 0.35 mmol) in acetonitrile (10 ml) was added acetic acid (2 ml) and water (2 ml) at 0°C followed by DCH (276 mg, 1.73 mmol) slowly, the resulting mixture was stirred at 0°C for 10 minutes. The mixture was added into NH₃.H₂O (15 ml) slowly under ice-water bath and extracted with EtOAc. The extracts were concentrated. The residue was purified with column chromatography on silica gel eluting with DCM / MeOH (50 : 1) to give the title compound 5-((*tert-*butoxycarbonyl)((tetrahydro-2*H*-pyran-4-yl)methyl)amino)-4-nitro-2-sulfamoylpyridine 1-oxide **(B-1-f).** MS-ESI (m/z): 433 [M + 1]⁺.

### 4-nitro-5-(((tetrahydro-2H-pyran-4-yl)methyl)amino)pyridine-2-sulfonamide (B-1-g)

To a solution of 5-((tert-butoxycarbonyl)((tetrahydro-2H-pyran-4-yl)methyl)amino)-4-nitro-2-sulfamoylpyridine 1-oxide **(B-1-f)** (100 mg, 0.23 mmol) in DCM (10 ml) was added PCl₃ (3 ml). The mixture was stirred at RT for overnight. Then TFA (10 ml) was added and the mixture was stirred at RT for 30 minutes. The mixture was concentrated, the residue was dissolved in DCM, the organic phase was washed with saturated NaHCO₃ and brine, dried over Na₂SO₄, concentrated. The residue was purified by P-TLC (DCM: MeOH = 20:1) to give the title compound 4-nitro-5-(((tetrahydro-2H-pyran-4-yl)methyl)amino)pyridine- 2-sulfonamide **(B-1-g).** MS-ESI (m/z): 315 [M - 1]⁻.

### (S)-2-((1H-pyrrolo[2,3-b]pyridin-5-yl)oxy)-4-(2-(2-(2-cyclopropylphenyl)pyr rolidin-1-yl)-7-azaspiro[3.5]nonan-7-yl)-N-((4-nitro-5-(((tetrahydro-2H-pyran-4-yl)methyl)a mino)pyridin-2-yl)sulfonyl)benzamide (B-1a)

(*S*)-2-((1*H*-pyrrolo[2,3-*b*]pyridin-5-yl)oxy)-4-(2-(2-(2-cyclopropylphenyl)pyr rolidin-1-yl)-7-azaspiro[3.5]nonan-7-yl)-*N*-((4-nitro-5-(((tetrahydro-2*H*-pyran-4-yl)methyl)a mino)pyridin-2-yl)sulfonyl)benzamide **(B-1a)** was prepared according to the synthetic method of **1** by replacing 6-(((4-hydroxy-4-methylcyclohexyl)methyl)amino)-5-nitropyridine-3-sulfonamide **(C-a)** with 4-nitro-5-(((tetrahydro-2H-pyran-4-yl)methyl)amino)pyridine-2-sulfonamide **(B-1-g).** MS-ESI (m/z): 861 [M + 1]⁺.

### Example B-1b

### (R)-2-((1H-pyrrolo[2,3-b]pyridin-5-yl)oxy)-4-(2-(2-(2-cyclopropylphenyl)pyr rolidin-1-yl)-7-azaspiro[3.5]nonan-7-yl)-N-((4-nitro-5-(((tetrahydro-2H-pyran-4-yl)methyl)a mino)pyridin-2-yl)sulfonyl)benzamide (B-1b)

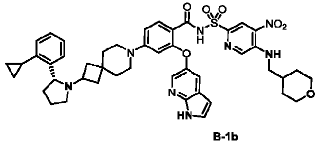

(R)-2-((1H-pyrrolo[2,3-b]pyridin-5-yl)oxy)-4-(2-(2-(2-cyclopropylphenyl)pyr rolidin-1-yl)-7-azaspiro[3.5]nonan-7-yl)-*N*-((4-nitro-5-(((tetrahydro-2*H*-pyran-4-yl)methyl)a mino)pyridin-2-yl)sulfonyl)benzamide **(B-1b)** was prepared according to the synthetic method of **B-1a** by replacing (S)-2-((1H-pyrrolo[2,3-b]pyridin-5-yl)oxy)-4-(2-(2-(2-cyclopropylphenyl)pyrrolidin-1-yl)-7-azaspiro[3.5]nonan-7-yl)benzoic acid **(Intermediate A)** with (R)-2-((1H-pyrrolo[2,3-b]pyridin-5-yl)oxy)-4-(2-(2-(2-cyclopropylphenyl)pyrrolidin-1-yl)-7-azaspiro[3.5]nonan-7-yl)benzoic acid **(Intermediate E).** MS-ESI (m/z): 861 [M + 1]⁺.

### Example B-2a

### 2-((1H-pyrrolo[2,3-b]pyridin-5-yl)oxy)-N-(((S)-2-benzyl-8-nitro-2,3-dihydro-1H-pyrido[2,3-b][1,4]oxazin-6-yl)sulfonyl)-4-(2-((S)-2-(2-cyclopropylphenyl)pyrrolidin-1-yl) -7-azaspiro[3.5]nonan-7-yl)benzamide (B-2a)

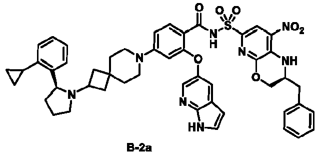

### 2,6-dichloropyridin-4-amine (B-2-a)

A mixture of 2,6-dichloro-4-nitropyridine (2.0 g, 10 mmol), iron powder (5.80 g, 104 mmol), NH₄Cl (1.1 g, 20.7 mmol) and water (8 ml) in EtOH (40) was stirred at 80°C overnight. The mixture was filtrated through Celite and washed with MeOH. The combined filtrate was concentrated, diluted with EtOAc and water. The organic phase was washed with brine, dried over Na₂SO₄, and concentrated to give the title compound 2,6-dichloropyridin-4-amine **(B-2-a).** MS-ESI (m/z): 163 [M + 1]⁺.

### 2,6-dichloro-3-fluoropyridin-4-amine (B-2-b)

A mixture of 2,6-dichloropyridin-4-amine (1.0 g, 6.1 mmol) **(B-2-a)** and select-fluor (2.4 g, 6.8 mmol) in acetonitrile (10 ml) and DMF (10 ml) was stirred at 35°C overnight. The mixture was quenched with ice-water, and the mixture was extracted with EtOAc, the organic layer was washed with brine, dried and concentrated. The residue was purified by column chromatography on silica gel eluting with EtOAc / PE (1:9) to give the title compound 2,6-dichloro-3-fluoropyridin-4-amine **(B-2-b).** MS-ESI (m/z): 181 [M + 1]⁺.

### 2,6-dichloro-3-fluoro-4-nitropyridine 1-oxide (B-2-c)

To a solution of 2,6-dichloro-3-fluoropyridin-4-amine (920 mg, 5.08 mmol) (**B-2-b**) in H₂SO₄ (50 ml) was added 30% H₂O₂ (20 ml) at 0°C slowly. The resulting solution was stirred at 50°C for overnight. The reaction mixture was cooled and quenched with ice, and the mixture was extracted with EtOAc, the organic layer was washed with brine, dried and concentrated to give the title compound 2,6-dichloro-3-fluoro-4-nitropyridine 1-oxide **(B-2-c).**

### (S)-2,6-dichloro-3-((1-hydroxy-3-phenylpropan-2-yl)amino)-4-nitropyridine 1-oxide (B-2-d)

(S)-2,6-dichloro-3-((1-hydroxy-3-phenylpropan-2-yl)amino)-4-nitropyridine 1-oxide **(B-2-d)** was synthesized according to the method described in **(B-1-c)** by replacing 2-chloro-5-fluoro-4-nitropyridine 1-oxide **(B-1-b)** and (tetrahydro-2H-pyran-4-yl)methanamine with 2,6-dichloro-3-fluoro-4-nitropyridine 1-oxide (**B-2-c**) and (*S*)-2-amino-3-phenylpropan-1-ol. MS-ESI (m/z): 358 [M + 1]⁺.

### (S)-2-benzyl-6-chloro-8-nitro-2,3-dihydro-1H-pyrido[2,3-b][1,4]oxazine 5-oxide (B-2-e)

To a solution of (S)-2,6-dichloro-3-((1-hydroxy-3-phenylpropan-2-yl)amino)-4-nitropyridine 1-oxide (600 mg, 1.68 mmol) **(B-2-d)** in THF (20 ml) was added NaH (134 mg, 5.58 mmol). The mixture was stirred at RT for 0.5 h. The reaction was quenched with ice-water, and the mixture was extracted with EtOAc, the organic layer was washed with brine, dried over Na₂SO₄, and concentrated to give the title compound (*S*)-2-benzyl-6-chloro-8-nitro-2,3-dihydro-1*H*-pyrido[2,3-*b*][1,4]oxazine 5-oxide **(B-2-e).** MS-ESI (m/z): 322 [M + 1]⁺.

### 2-((1H-pyrrolo[2,3-b]pyridin-5-yl)oxy)-N-(((S)-2-benzyl-8-nitro-2,3-dihydro-1H-pyrido[2,3-b][1,4]oxazin-6-yl)sulfonyl)-4-(2-((S)-2-(2-cyclopropylphenyl)pyrrolidin-1-yl) -7-azaspiro[3.5]nonan-7-yl)benzamide (B-2a)

2-((1H-pyrrolo[2,3-b]pyridin-5-yl)oxy)-N-(((S)-2-benzyl-8-nitro-2,3-dihydro-1*H*-pyrido[2,3-*b*][1,4]oxazin-6-yl)sulfonyl)-4-(2-((*S*)-2-(2-cyclopropylphenyl)pyrrolidin-1-yl) -7-azaspiro[3.5]nonan-7-yl)benzamide **(B-2a)** was synthesized according to the method described in **(B-1a)** by replacing 2-chloro-4-nitro-5-(((tetrahydro-2H-pyran-4-yl)methyl)amino)pyridine 1-oxide **(B-1-c)** with (S)-2-benzyl-6-chloro-8-nitro-2,3-dihydro-1H-pyrido[2,3-b][1,4]oxazine 5-oxide **(B-2-e).** MS-ESI (m/z): 895 [M + 1]⁺.

### Example B-2b

### 2-((1H-pyrrolo[2,3-b]pyridin-5-yl)oxy)-N-(((S)-2-benzyl-8-nitro-2,3-dihydro-1H-pyrido[2,3-b][1,4]oxazin-6-yl)sulfonyl)-4-(2-((R)-2-(2-cyclopropylphenyl)pyrrolidin-1-yl) -7-azaspiro[3.5]nonan-7-yl)benzamide (B-2b)

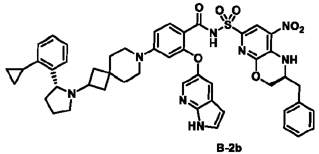

2-((1H-pyrrolo[2,3-b]pyridin-5-yl)oxy)-N-(((S)-2-benzyl-8-nitro-2,3-dihydro-1*H*-pyrido[2,3-*b*][1,4]oxazin-6-yl)sulfonyl)-4-(2-((*R*)-2-(2-cyclopropylphenyl)pyrrolidin-1-yl) -7-azaspiro[3.5]nonan-7-yl)benzamide **(B-2b)** was prepared according to the synthetic method of **B-2a** by replacing (*S*)-2-((1*H*-pyrrolo[2,3-*b*]pyridin-5-yl)oxy)-4-(2-(2-(2-cyclopropylphenyl)-pyrrolidin-1-yl)-7-azaspiro[3.5]nonan-7-yl)benzoic acid **(Intermediate A)** with (*R*)-2-((1*H*-pyrrolo[2,3-b]pyridin-5-yl)oxy)-4-(2-(2-(2-cyclopropylphenyl)pyrrolidin-1-yl)-7-a zaspiro[3.5]nonan-7-yl)benzoic acid **(Intermediate E).** MS-ESI (m/z): 895 [M + 1]⁺.

Following essentially the same procedures described for Examples B-1 ~ B-2 or using similar synthetic strategies or methods. Examples B-3 ~ B-68 listed in Table 3 were prepared using appropriate intermediates, which can be readily synthesized by methods known in the art, and sequential modifications as necessary. The structures and names of Examples B-3 ~ B-68 are given in table 3.

**Table 3**

| EXAMPL E | STRUCTURE | NAME | DATA |
|---|---|---|---|
| B-3a | 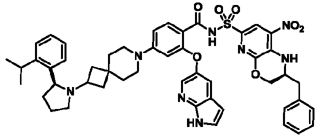 | 2-((1*H*-pyrrolo[2,3-b]pyridin-5-yl )oxy)-*N*-(((*S*)-2-benzyl-8-nitro-2 ,3-dihydro-1*H*-pyrido[2,3-*b*][1,4] oxazin-6-yl)sulfonyl)-4-(2-((*S*)-2 -(2-isopropylphenyl)pyrrolidin-1 -yl)-7-azaspiro[3.5]nonan-7-yl) benzamide | MS-ESI (m/z): 897 [M + 1]⁺ |
| B-3b | 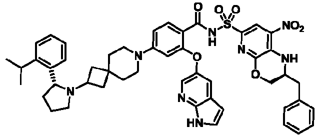 | 2-((1*H*-pyrrolo[2,3-*b*]pyridin-5-yl )oxy)-*N*-(((S)-2-benzyl-8-nitro-2 ,3-dihydro-1H-pyrido[2,3-b] [1,4] oxazin-6-yl)sulfonyl)-4-(2-((R)-2 -(2-isopropylphenyl)pyrrolidin-1 -yl)-7-azaspiro[3.5]nonan-7-yl) benzamide | MS-ESI (m/z): 897 [M + 1]⁺ |
| B-4a | 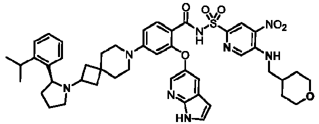 | (*S*)-2-((1*H*-pyrrolo[2,3-b]pyridin -5-yl)oxy)-4-(2-(2-(2-isopropylp henyl)pyrrolidin-1-yl)-7-azaspiro [3.5]nonan-7-yl)-*N*-((4-nitro-5-(( (tetrahydro-2*H*-pyran-4-yl)methyl )amino)pyridin-2-yl)sulfonyl) benzamide | MS-ESI (m/z): 863 [M + 1]⁺ |
| B-4b | 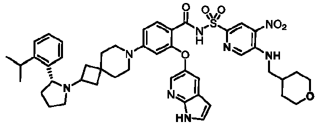 | (*R*)-2-((1*H*-pyrrolo[2,3-*b*]pyridin -5-yl)oxy)-4-(2-(2-(2-isopropylp henyl)pyrrolidin-1-yl)-7-azaspiro [3.5]nonan-7-yl)-*N*-((4-nitro-5-(( (tetrahydro-2*H*-pyran-4-yl)methyl )amino)pyridin-2-yl)sulfonyl) benzamide | MS-ESI (m/z): 863 [M + 1]⁺ |
| B-5a | 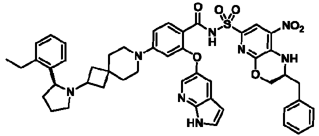 | 2-((1*H*-pyrrolo[2,3-b]pyridin-5-yl )oxy)-*N*-(((*S*)-2-benzyl-8-nitro-2 ,3-dihydro-1*H*-pyrido[2,3-*b*][1,4] oxazin-6-yl)sulfonyl)-4-(2-((*S*)-2 -(2-ethylphenyl)pyrrolidin-1-yl)-7-azaspiro[3.5]nonan-7-yl)benza mide | MS-ESI (m/z): 883 [M + 1]⁺ |
| B-5b | 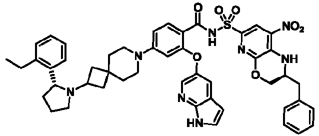 | 2-((1*H*-pyrrolo[2,3-*b*]pyridin-5-y l)oxy)-*N*-(((*S*)-2-benzyl-8-nitro-2 ,3-dihydro-1*H*-pyrido[2,3-*b*][1,4] oxazin-6-yl)sulfonyl)-4-(2-((*R*)-2 -(2-ethylphenyl)pyrrolidin-1-yl)-7-azaspiro[3.5]nonan-7-yl)benza mide | MS-ESI (m/z): 883 [M + 1]⁺ |
| B-6a | 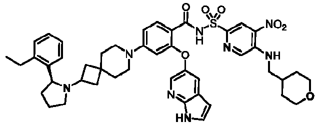 | (*S*)-2-((1*H*-pyrrolo[2,3-*b*]pyridin -5-yl)oxy)-4-(2-(2-(2-ethylphenyl )pyrrolidin-1-yl)-7-azaspiro[3.5] nonan-7-yl)-*N*-((4-nitro-5-(((tetra hydro-2H-pyran-4-yl)methyl)ami no)pyridin-2-yl)sulfonyl)benzamide | MS-ESI (m/z): 849 [M + 1]⁺ |
| B-6b | 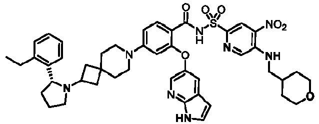 | (*R*)-2-((1*H*-pyrrolo[2,3-*b*]pyridin -5-yl)oxy)-4-(2-(2-(2-ethylphenyl )pyrrolidin-1-yl)-7-azaspiro[3.5] nonan-7-yl)-*N*-((4-nitro-5-(((tetra hydro-2H-pyran-4-yl)methyl) amino)pyridin-2-yl)sulfonyl)benzamide | MS-ESI (m/z): 849 [M + 1]⁺ |
| B-7 | 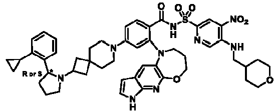 | 4-(2-(2-(2-cyclopropylphenyl) pyrrolidin-1-yl)-7-azaspiro[3.5]non an-7-yl)-2-(3,4-dihydro-2*H*-pyrrolo [3',2':5,6]pyrido[2,3-*b*][1,4]ox azepin-1(7*H*)-yl)-*N*-((4-nitro-5-(( (tetrahydro-2*H*-pyran-4-yl)methy l)amino)pyridin-2-yl)sulfonyl) benzamide | MS-ESI (m/z): 916 [M + 1]⁺ |
| B-8 | 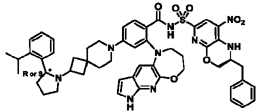 | *N*-(((*S*)-2-benzyl-8-nitro-2,3-dihy dro-1*H*-pyrido[2,3-*b*][1,4]oxazin -6-yl)sulfonyl)-2-(3,4-dihydro-2 *H*-pyrrolo[3',2':5,6]pyrido[2,3-b] [1,4]oxazepin-1(7*H*)-yl)-4-(2-(2-(2-isopropylphenyl)pyrrolidin-1-yl)-7-azaspiro[3.5]nonan-7-yl)be nzamide | MS-ESI (m/z): 952 [M + 1]⁺ |
| B-9 | 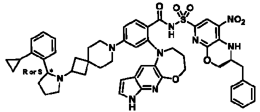 | *N*-(((*S*)-2-benzyl-8-nitro-2,3-dihy dro-1*H*-pyrido[2,3-*b*][1,4]oxazin -6-yl)sulfonyl)-4-(2-(2-(2-cyclop ropylphenyl)pyrrolidin-1-yl)-7-a zaspiro[3.5]nonan-7-yl)-2-(3,4-di hydro-2*H*-pyrrolo[3',2':5,6]pyrid o[2,3-*b*][1,4]oxazepin-1(7*H*)-yl) benzamide | MS-ESI (m/z): 950 [M + 1]⁺ |
| B-10 | 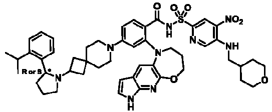 | 2-(3,4-dihydro-2*H*-pyrrolo[3',2':5 ,6]pyrido[2,3-*b*][1,4]oxazepin-1( 7*H*)-yl)-4-(2-(2-(2-isopropylphen yl)pyrrolidin-1-yl)-7-azaspiro[3. 5]nonan-7-yl)-*N*-((4-nitro-5-(((te trahydro-2*H*-pyran-4-yl)methyl)a mino)pyridin-2-yl)sulfonyl)benz amide | MS-ESI (m/z): 918 [M + 1]⁺ |
| B-11 | 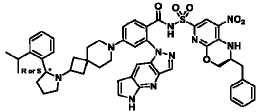 | (*S*)-*N*-((2-benzyl-8-nitro-2,3-dihy dro-1*H*-pyrido[2,3-*b*][1,4]oxazin -6-yl)sulfonyl)-4-(2-(2-(2-isopro pylphenyl)pyrrolidin-1-yl)-7-aza spiro[3.5]nonan-7-yl)-2-(pyrazolo [4,3-*b*]pyrrolo[3,2-*e*]pyridin-1( 5*H*)-yl)benzamide | MS-ESI (m/z): 921 [M + 1]⁺ |
| B-12 | 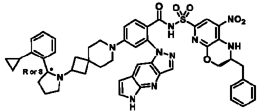 | (*S*)-*N*-((2-benzyl-8-nitro-2,3-dihy dro-1*H*-pyrido[2,3-*b*][1,4]oxazin -6-yl)sulfonyl)-4-(2-(2-(2-cyclop ropylphenyl)pyrrolidin-1-yl)-7-a zaspiro[3.5]nonan-7-yl)-2-(pyraz olo[4,3-b]pyrrolo[3,2-e]pyridin-1 (5*H*)-yl)benzamide | MS-ESI (m/z): 919 [M + 1]⁺ |
| B-13 | 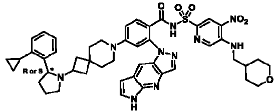 | 4-(2-(2-(2-cyclopropylphenyl)py rrolidin-1-yl)-7-azaspiro[3.5]non an-7-yl)-*N*-((4-nitro-5-(((tetrahyd ro-2*H*-pyran-4-yl)methyl)amino) pyridin-2-yl)sulfonyl)-2-(pyrazol o[4,3-*b*]pyrrolo[3,2-e]pyridin-1( 5*H*)-yl)benzamide | MS-ESI (m/z): 885 [M + 1]⁺ |
| B-14 | 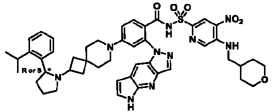 | 4-(2-(2-(2-isopropylphenyl)pyrro lidin-1-yl)-7-azaspiro[3.5]nonan-7-yl)-*N*-((4-nitro-5-(((tetrahydro-2*H*-pyran-4-yl)methyl)amino)pyr idin-2-yl)sulfonyl)-2-(pyrazolo[4 ,3-*b*]pyrrolo[3,2-e]pyridin-1(5*H*) -yl)benzamide | MS-ESI (m/z): 887 [M + 1]⁺ |
| B-15 | 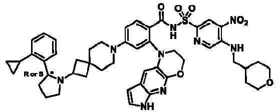 | 4-(2-(2-(2-cyclopropylphenyl) pyrrolidin-1-yl)-7-azaspiro[3.5]non an-7-yl)-2-(2,3-dihydropyrrolo[3' ,2':5,6]pyrido[2,3-*b*][1,4]oxazin-1(6H)-yl)-*N*-((4-nitro-5-(((tetrah ydro-2*H*-pyran-4-yl)methyl)amino )pyridin-2-yl)sulfonyl)benzamide | MS-ESI (m/z): 902 [M + 1]⁺ |
| B-16 | 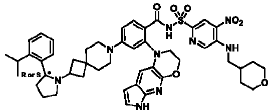 | 2-(2,3-dihydropyrrolo[3',2':5,6] pyrido[2,3-*b*][1,4]oxazin-1(6*H*)-yl )-4-(2-(2-(2-isopropylphenyl)pyr rolidin-1-yl)-7-azaspiro[3.5]nonan -7-yl)-*N*-((4-nitro-5-(((tetrahydro -2*H*-pyran-4-yl)methyl)amino) pyridin-2-yl)sulfonyl)benzamide | MS-ESI (m/z): 904 [M + 1]⁺ |
| B-17 | 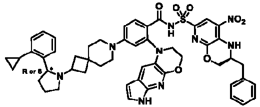 | (*S*)-*N*-((2-benzyl-8-nitro-2,3-dihydro -1*H*-pyrido[2,3-*b*][1,4]oxazin -6-yl)sulfonyl)-4-(2-(2-(2-cyclop ropylphenyl)pyrrolidin-1-yl)-7-azaspiro[3.5]nonan-7-yl)-2-(2,3-di hydropyrrolo[3',2':5,6]pyrido[2,3 -*b*][1,4]oxazin-1(6*H*)-yl)benzamide | MS-ESI (m/z): 936 [M + 1]⁺ |
| B-18 | 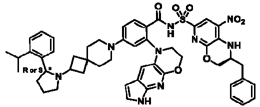 | (*S*)-*N*-((2-benzyl-8-nitro-2,3-dihy dro-1*H*-pyrido[2,3-*b*][1,4]oxazin -6-yl)sulfonyl)-2-(2,3-dihydropyr rolo[3',2':5,6]pyrido[2,3-*b*][1,4] oxazin-1(6*H*)-yl)-4-(2-(2-(2-isopr opylphenyl)pyrrolidin-1-yl)-7-az aspiro[3.5]nonan-7-yl)benzamide | MS-ESI (m/z): 938 [M + 1]⁺ |
| B-19 | 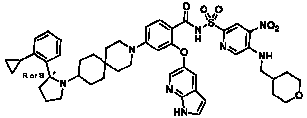 | 2-((1*H*-pyrrolo[2,3-b]pyridin-5-yl )oxy)-4-(9-(2-(2-cyclopropylphe nyl)pyrrolidin-1-yl)-3-azaspiro[5 .5]undecan-3-yl)-*N*-((4-nitro-5-(( (tetrahydro-2*H*-pyran-4-yl)methyl )amino)pyridin-2-yl)sulfonyl)be nzamide | MS-ESI (m/z): 889 [M + 1]⁺ |
| B-20 | 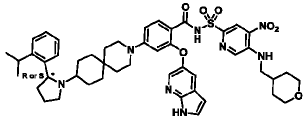 | 2-((1*H*-pyrrolo[2,3-b]pyridin-5-yl )oxy)-4-(9-(2-(2-isopropylphenyl )pyrrolidin-1-yl)-3-azaspiro[5.5] undecan-3-yl)-*N*-((4-nitro-5-(((te trahydro-2*H*-pyran-4-yl)methyl) amino)pyridin-2-yl)sulfonyl)benz amide | MS-ESI (m/z): 891 [M + 1]⁺ |
| B-21 | 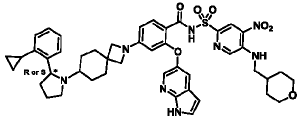 | 2-((1*H*-pyrrolo[2,3-*b*]pyridin-5-y l)oxy)-4-(7-(2-(2-cyclopropylphe nyl)pyrrolidin-1-yl)-2-azaspiro[3 .5]nonan-2-yl)-*N*-((4-nitro-5-(((te trahydro-2H-pyran-4-yl)methyl)a mino)pyridin-2-yl)sulfonyl)benz amide | MS-ESI (m/z): 861 [M + 1]⁺ |
| B-22 | 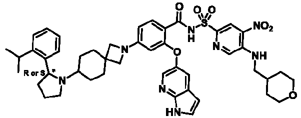 | 2-((1*H*-pyrrolo[2,3-*b*]pyridin-5-yl )oxy)-4-(7-(2-(2-isopropylphenyl )pyrrolidin-1-yl)-2-azaspiro[3.5] nonan-2-yl)-*N*-((4-nitro-5-(((tetra hydro-2*H*-pyran-4-yl)methyl)ami no)pyridin-2-yl)sulfonyl)benzamide | MS-ESI (m/z): 863 [M + 1]⁺ |
| B-23 | 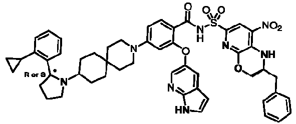 | (*S*)-2-((1*H*-pyrrolo[2,3-*b*]pyridin -5-yl)oxy)-*N*-((2-benzyl-8-nitro-2,3-dihydro-1*H*-pyrido[2,3-*b*][1, 4]oxazin-6-yl)sulfonyl)-4-(9-(2-( 2-cyclopropylphenyl)pyrrolidin-1-yl)-3-azaspiro[5.5]undecan-3-yl )benzamide | MS-ESI (m/z): 923 [M + 1]⁺ |
| B-24 | 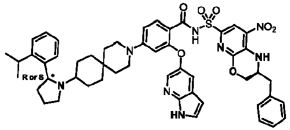 | (*S*)-2-((1*H*-pyrrolo[2,3-*b*]pyridin -5-yl)oxy)-*N*-((2-benzyl-8-nitro-2,3-dihydro-1*H*-pyrido[2,3-*b*][1, 4]oxazin-6-yl)sulfonyl)-4-(9-(2-( 2-isopropylphenyl)pyrrolidin-1-yl )-3-azaspiro[5.5]undecan-3-yl) benzamide | MS-ESI (m/z): 925 [M + 1]⁺ |
| B-25 | 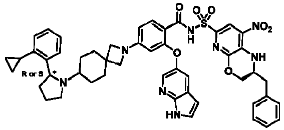 | (*S*)-2-((1*H*-pyrrolo[2,3-b]pyridin -5-yl)oxy)-*N*-((2-benzyl-8-nitro-2,3-dihydro-1*H*-pyrido[2,3-*b*][1, 4]oxazin-6-yl)sulfonyl)-4-(7-(2-( 2-cyclopropylphenyl)pyrrolidin-1-yl)-2-azaspiro[3.5]nonan-2-yl) benzamide | MS-ESI (m/z): 895 [M + 1]⁺ |
| B-26 | 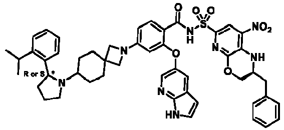 | (*S*)-2-((1*H*-pyrrolo[2,3-b]pyridin -5-yl)oxy)-*N*-((2-benzyl-8-nitro-2,3-dihydro-1*H*-pyrido[2,3-*b*][1, 4]oxazin-6-yl)sulfonyl)-4-(7-(2-( 2-isopropylphenyl)pyrrolidin-1-y l)-2-azaspiro[3.5]nonan-2-yl)ben zamide | MS-ESI (m/z): 897 [M + 1]⁺ |
| B-27 | 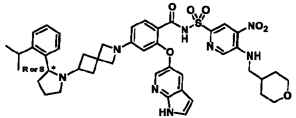 | 2-((1*H*-pyrrolo[2,3-b]pyridin-5-y l)oxy)-4-(6-(2-(2-isopropylpheny l)pyrrolidin-1-yl)-2-azaspiro[3.3] heptan-2-yl)-*N*-((4-nitro-5-(((tetr ahydro-2*H*-pyran-4-yl)methyl)a mino)pyridin-2-yl)sulfonyl)benz amide | MS-ESI (m/z): 835 [M + 1]⁺ |
| B-28 | 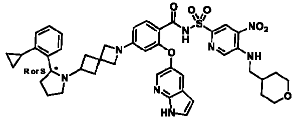 | 2-((1*H*-pyrrolo[2,3-b]pyridin-5-yl )oxy)-4-(6-(2-(2-cyclopropylphe nyl)pyrrolidin-1-yl)-2-azaspiro[3 .3]heptan-2-yl)-*N*-((4-nitro-5-(((t etrahydro-2*H*-pyran-4-yl)methyl) amino)pyridin-2-yl)sulfonyl)ben zamide | MS-ESI (m/z): 833 [M + 1]⁺ |
| B-29 | 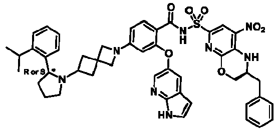 | (*S*)-2-((1*H*-pyrrolo[2,3-*b*]pyridin -5-yl)oxy)-N-((2-benzyl-8-nitro-2,3-dihydro-1H-pyrido[2,3-b][1, 4]oxazin-6-yl)sulfonyl)-4-(6-(2-( 2-isopropylphenyl)pyrrolidin-1-y l)-2-azaspiro[3.3]heptan-2-yl)ben zamide | MS-ESI (m/z): 869 [M + 1]⁺ |
| B-30 | 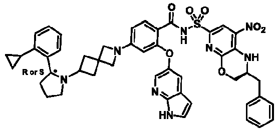 | (*S*)-2-((1*H*-pyrrolo[2,3-*b*]pyridin -5-yl)oxy)-N-((2-benzyl-8-nitro-2,3-dihydro-1H-pyrido[2,3-b][1, 4]oxazin-6-yl)sulfonyl)-4-(6-(2-( 2-cyclopropylphenyl)pyrrolidin-1-yl)-2-azaspiro[3.3]heptan-2-yl) benzamide | MS-ESI (m/z): 867 [M + 1]⁺ |
| B-31a | 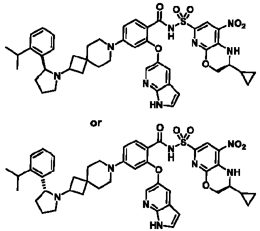 | 2-((1*H*-pyrrolo[2,3-*b*]pyridin-5-yl )oxy)-*N*-(((*S*)-2-cyclopropyl-8-nitro-2,3-dihydro-1*H*-pyrido[2,3-*b* ][1,4]oxazin-6-yl)sulfonyl)-4-(2-((R or *S*)-2-(2-isopropylphenyl)pyrrolid in-1-yl)-7-azaspiro[3.5]nonan-7-yl)benzamide | MS-ESI (m/z): 847 [M + 1]⁺ |
| B-31b | | | |
| B-32a | 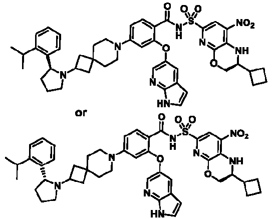 | 2-((1H-pyrrolo[2,3-b]pyridin-5-y l)oxy)-*N*-(((*S*)-2-cyclobutyl-8-nit ro-2,3-dihydro-1*H*-pyrido[2,3-*b*] [1,4]oxazin-6-yl)sulfonyl)-4-(2-( (R or S)-2-(2-isopropylphenyl)pyrrolid in-1-yl)-7-azaspiro[3.5]nonan-7-yl)benzamide | MS-ESI (m/z): 861 [M + 1]⁺ |
| B-32b | | | |
| B-33 | 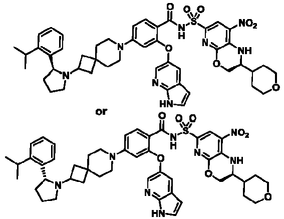 | 2-((1*H*-pyrrolo[2,3-*b*]pyridin-5-yl )oxy)-4-(2-((*R* or *S*)-2-(2-isopropylphenyl)pyrrolid in-1-yl)-7-azaspiro[3.5]nonan-7-yl)-*N*-(((*S*)-8-nitro-2-(tetrahydro-2*H*-pyran-4-yl)-2,3-dihydro-1*H-*pyrido[2,3-*b*][1,4]oxazin-6-yl)sul fonyl)benzamide | MS-ESI (m/z): 891 [M + 1]⁺ |
| B-34 | 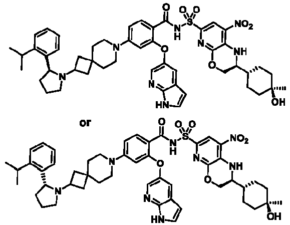 | 2-((1*H*-pyrrolo[2,3-*b*]pyridin-5-yl )oxy)-*N*-(((*S*)-2-((1*r*,4*S*)-4-hydro xy-4-methylcyclohexyl)-8-nitro-2,3-dihydro-1*H*-pyrido[2,3-*b*][1, 4]oxazin-6-yl)sulfonyl)-4-(2-((*R* or *S*)-2-(2-isopropylphenyl)pyrrolid in-1-yl)-7-azaspiro[3.5]nonan-7-yl)benzamide | MS-ESI (m/z): 919 [M + 1]⁺ |
| B-35 | 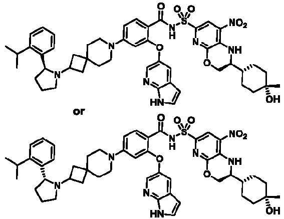 | 2-((1*H*-pyrrolo[2,3-b]pyridin-5-yl )oxy)-*N*-(((*S*)-2-((1*s*,4*R*)-4-hydr oxy-4-methylcyclohexyl)-8-nitro -2,3-dihydro-1*H*-pyrido[2,3-*b*][1, 4]oxazin-6-yl)sulfonyl)-4-(2-((*R* or *S*)-2-(2-isopropylphenyl)pyrrolid in-1-yl)-7-azaspiro[3.5]nonan-7-yl)benzamide | MS-ESI (m/z): 919 [M + 1]⁺ |
| B-36 | 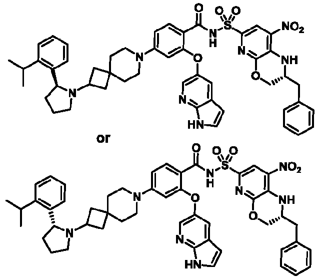 | 2-((1*H*-pyrrolo[2,3-b]pyridin-5-yl )oxy)-*N*-(((*R*)-2-benzyl-8-nitro-2,3-dihydro-1*H*-pyrido[2,3-*b*][1, 4]oxazin-6-yl)sulfonyl)-4-(2-((*R* or *S*)-2-(2-isopropylphenyl)pyrrolid in-1-yl)-7-azaspiro[3.5]nonan-7-yl)benzamide | MS-ESI (m/z): 897 [M + 1]⁺ |
| B-37 | 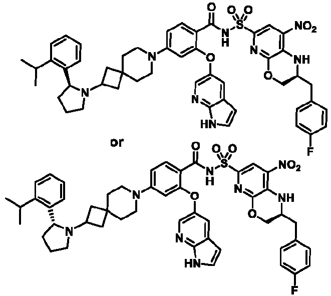 | 2-((1*H*-pyrrolo[2,3-b]pyridin-5-yl )oxy)-*N*-(((*S*)-2-(4-fluorobenzyl) -8-nitro-2,3-dihydro-1*H*-pyrido[2 ,3-*b*][1,4]oxazin-6-yl)sulfonyl)-4 -(2-((*R* or *S*)-2-(2-isopropylphenyl)pyrrolid in-1-yl)-7-azaspiro[3.5]nonan-7-yl)benzamide | MS-ESI (m/z): 915 [M + 1]⁺ |
| B-38 | 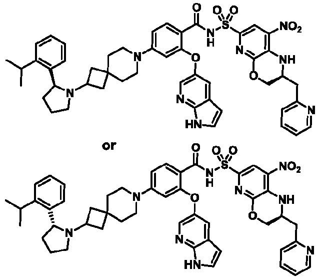 | 2-((1*H*-pyrrolo[2,3-*b*]pyridin-5-yl )oxy)-4-(2-((*R* or *S*)-2-(2-isopropylphenyl)pyrrolid in-1-yl)-7-azaspiro[3.5]nonan-7-yl)-*N*-(((*S*)-8-nitro-2-(pyridin-2-y lmethyl)-2,3-dihydro-1*H*-pyrido[ 2,3-*b*][1,4]oxazin-6-yl)sulfonyl) benzamide | MS-ESI (m/z): 898 [M + 1]⁺ |
| B-39a | 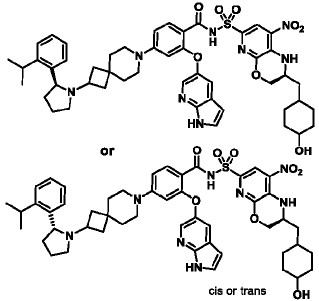 | 2-((1*H*-pyrrolo[2,3-*b*]pyridin-5-y 1)oxy)-*N*-(((*S*)-2-((4-hydroxycycl ohexyl)methyl)-8-nitro-2,3-dihyd ro-1*H*-pyrido[2,3-*b*][1,4]oxazin-6-yl)sulfonyl)-4-(2-((*R* or *S*)-2-(2-isopropylphenyl)pyrrolid in-1-yl)-7-azaspiro[3.5]nonan-7-yl)benzamide | MS-ESI (m/z): 919 [M + 1]⁺ |
| B-39b | | | |
| B-40 | 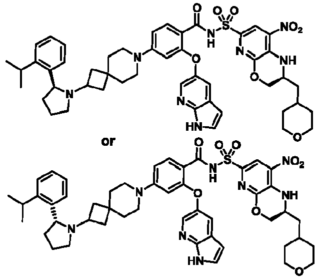 | 2-((1*H*-pyrrolo[2,3-*b*]pyridin-5-yl )oxy)-4-(2-((*R* or *S*)-2-(2-isopropylphenyl)pyrrolid in-1-yl)-7-azaspiro[3.5]nonan-7-yl)-*N*-(((*S*)-8-nitro-2-((tetrahydro -2*H*-pyran-4-yl)methyl)-2,3-dihy dro-1*H*-pyrido[2,3-*b*][1,4]oxazin -6-yl)sulfonyl)benzamide | MS-ESI (m/z): 905 [M + 1]⁺ |
| B-41 | 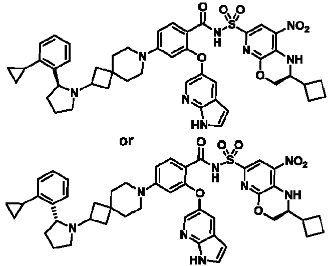 | 2-((1H-pyrrolo[2,3-b]pyridin-5-yl )oxy)-*N*-(((*S*)-2-cyclobutyl-8-nit ro-2,3-dihydro-1*H*-pyrido[2,3-*b*] [1,4]oxazin-6-yl)sulfonyl)-4-(2-( (R or S)-2-(2-cyclopropylphenyl)pyrro lidin-1-yl)-7-azaspiro[3.5]nonan-7-yl)benzamide | MS-ESI (m/z): 859 [M + 1]⁺ |
| B-42 | 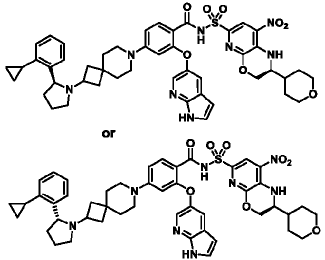 | 2-((1*H*-pyrrolo[2,3-*b*]pyridin-5-yl )oxy)-4-(2-((*R* or *S*)-2-(2-cyclopropylphenyl)pyrro lidin-1-yl)-7-azaspiro[3.5]nonan-7-yl)-*N*-(((*S*)-8-nitro-2-(tetrahydr o-2*H*-pyran-4-yl)-2,3-dihydro-1 *H*-pyrido[2,3-*b*][1,4]oxazin-6-yl) sulfonyl)benzamide | MS-ESI (m/z): 889 [M + 1]⁺ |
| B-43 | 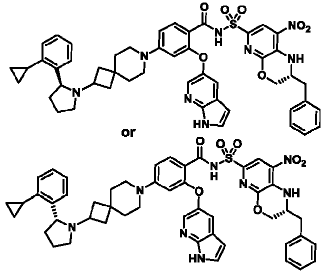 | 2-((1*H*-pyrrolo[2,3-b]pyridin-5-yl oxy)-*N*-(((*R*)-2-benzyl-8-nitro-2,3-dihydro-1*H*-pyrido[2,3-*b*][1, 4]oxazin-6-yl)sulfonyl)-4-(2-((*R* or *S*)-2-(2-cyclopropylphenyl)pyrro lidin-1-yl)-7-azaspiro[3.5]nonan-7-yl)benzamide | MS-ESI (m/z): 895 [M + 1]⁺ |
| B-44 | 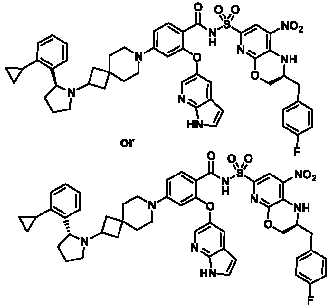 | 2-((1*H*-pyrrolo[2,3-*b*]pyridin-5-yloxy)-4-(2-((*R* or *S*)-2-(2-cyclopropylphenyl)pyrro lidin-1-yl)-7-azaspiro[3.5]nonan-7-yl)-*N*-(((*S*)-2-(4-fluorobenzyl)-8-nitro-2,3-dihydro-1*H*-pyrido[2, 3-*b*][1,4]oxazin-6-yl)sulfonyl)be nzamide | MS-ESI (m/z): 913 [M + 1]⁺ |
| B-45 | 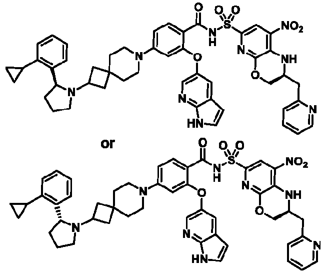 | 2-((1*H*-pyrrolo[2,3-*b*]pyridin-5-yloxy)-4-(2-((*R* or *S*)-2-(2-cyclopropylphenyl)pyrro lidin-1-yl)-7-azaspiro[3.5]nonan-7-yl)-*N*-(((*S*)-8-nitro-2-(pyridin-2 -ylmethyl)-2,3-dihydro-1*H*-pyrid o[2,3-*b*][1,4]oxazin-6-yl)sulfonyl )benzamide | MS-ESI (m/z): 896 [M + 1]⁺ |
| B-46 | 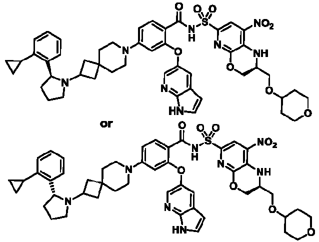 | 2-((1*H*-pyrrolo[2,3-*b*]pyridin-5-yloxy)-4-(2-((*R* or S)-2-(2-cyclopropylphenyl)pyrro lidin-1-yl)-7-azaspiro[3.5]nonan-7-yl)-*N*-(((*S*)-8-nitro-2-(((tetrahy dro-2*H*-pyran-4-yl)oxy)methyl)-2,3-dihydro-1*H*-pyrido[2,3-*b*][1, 4]oxazin-6-yl)sulfonyl)benzamide | MS-ESI (m/z): 919 [M + 1]⁺ |
| B-47 | 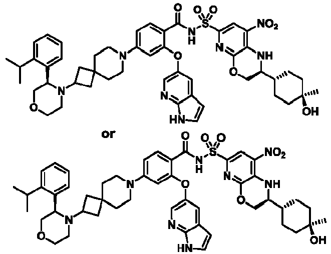 | 2-((1*H*-pyrrolo[2,3-b]pyridin-5-yloxy)-*N*-(((*S*)-2-((1*r*,4*S*)-4-hydro xy-4-methylcyclohexyl)-8-nitro-2,3-dihydro-1*H*-pyrido[2,3-*b*][1, 4]oxazin-6-yl)sulfonyl)-4-(2-((*R* or *S*)-3-(2-isopropylphenyl)morphol ino)-7-azaspiro[3.5]nonan-7-yl) benzamide | MS-ESI (m/z): 935 [M + 1]⁺ |
| B-48 | 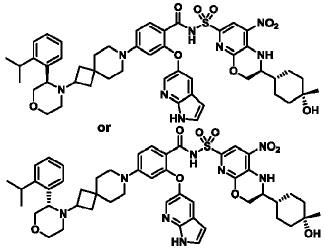 | 2-((1*H*-pyrrolo[2,3-*b*]pyridin-5-yl )oxy)-*N*-(((*S*)-2-((1*s* ,4*R*)-4-hydr oxy-4-methylcyclohexyl)-8-nitro -2,3-dihydro-1*H*-pyrido[2,3-*b*][1, 4]oxazin-6-yl)sulfonyl)-4-(2-((R or *S*)-3-(2-isopropylphenyl)morphol ino)-7-azaspiro[3.5]nonan-7-yl) benzamide | MS-ESI (m/z): 935 [M + 1]⁺ |
| B-49 | 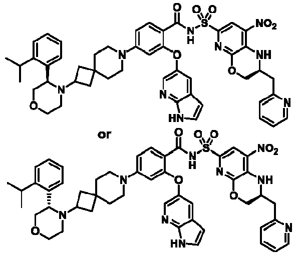 | 2-((1*H*-pyrrolo[2,3-*b*]pyridin-5-yl )oxy)-4-(2-((*R* or *S*)-3-(2-isopropylphenyl)morphol ino)-7-azaspiro[3.5]nonan-7-yl)-*N*-(((*S*)-8-nitro-2-(pyridin-2-ylm ethyl)-2,3-dihydro-1*H*-pyrido[2, 3-*b*][1,4]oxazin-6-yl)sulfonyl) benzamide | MS-ESI (m/z): 914 [M + 1]⁺ |
| B-50a | 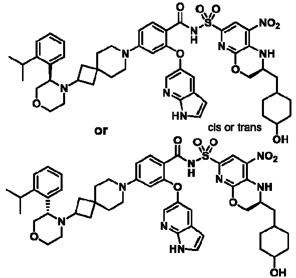 | 2-((1*H*-pyrrolo[2,3-*b*]pyridin-5-y l)oxy)-*N*-(((*S*)-2-((4-hydroxycycl ohexyl)methyl)-8-nitro-2,3-dihyd ro-1*H*-pyrido[2,3-*b*][1,4]oxazin-6-yl)sulfonyl)-4-(2-((R or *S*)-3-(2-isopropylphenyl)morphol ino)-7-azaspiro[3.5]nonan-7-yl)b enzamide | MS-ESI (m/z): 935 [M + 1]⁺ |
| B-50b | | | |
| B-51 | 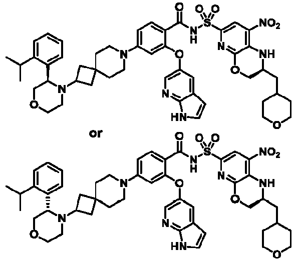 | 2-((1*H*-pyrrolo[2,3-*b*]pyridin-5-y l)oxy)-4-(2-((*R* or *S*)-3-(2-isopropylphenyl)morphol ino)-7-azaspiro[3.5]nonan-7-yl)-*N*-(((*S*)-8-nitro-2-((tetrahydro-2*H* -pyran-4-yl)methyl)-2,3-dihydro-1*H*-pyrido[2,3-*b*][1,4]oxazin-6-y l)sulfonyl)benzamide | MS-ESI (m/z): 921 [M + 1]⁺ |
| B-52 | 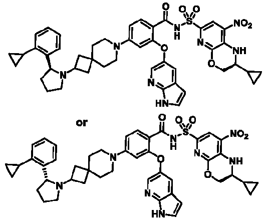 | 2-((1*H*-pyrrolo[2,3-*b*]pyridin-5-y l)oxy)-*N*-(((*S*)-2-cyclopropyl-8-n itro-2,3-dihydro-1*H*-pyrido[2,3-*b* ][1,4]oxazin-6-yl)sulfonyl)-4-(2-((*R* or *S*)-2-(2-cyclopropylphenyl)pyrro lidin-1-yl)-7-azaspiro[3.5]nonan-7-yl)benzamide | MS-ESI (m/z): 845 [M + 1]⁺ |
| B-53 | 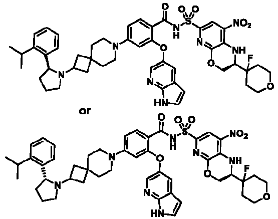 | 2-((1H-pyrrolo[2,3-b]pyridin-5-y l)oxy)-*N*-(((*R*)-2-(4-fluorotetrahy dro-2*H*-pyran-4-yl)-8-nitro-2,3-d ihydro-1*H*-pyrido[2,3-*b*][1,4]oxa zin-6-yl)sulfonyl)-4-(2-((R or S)-2-(2-isopropylphenyl)pyrrolid in-1-yl)-7-azaspiro[3.5]nonan-7-yl)benzamide | MS-ESI (m/z): 909 [M + 1]⁺ |
| B-54 | 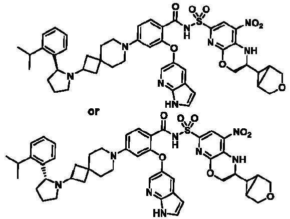 | 2-((1H-pyrrolo[2,3-b]pyridin-5-y l)oxy)-*N*-(((2*S*)-2-(3-oxabicyclo[ 3.1.0]hexan-6-yl)-8-nitro-2,3-dih ydro-1*H*-pyrido[2,3-*b*][1,4]oxazi n-6-yl)sulfonyl)-4-(2-((R or S)-2-(2-isopropylphenyl)pyrrolid in-1-yl)-7-azaspiro[3.5]nonan-7-yl)benzamide | MS**-**ESI (m/z): 889 [M + 1]⁺ |
| B-55 | 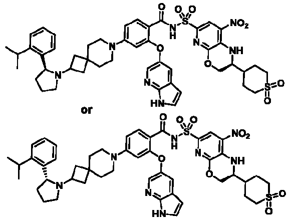 | 2-((1*H*-pyrrolo[2,3-b]pyridin-5-yl)oxy)-*N*-(((*S*)-2-(1,1-dioxidotetr ahydro-2H-thiopyran-4-yl)-8-nitr o-2,3-dihydro-1*H*-pyrido[2,3-*b*][ 1,4]oxazin-6-yl)sulfonyl)-4-(2-(( *R* or S)-2-(2-isopropylphenyl)pyrrolid in-1-yl)-7-azaspiro[3.5]nonan-7-yl)benzamide | MS-ESI (m/z): 939 [M + 1]⁺ |
| B-56 | 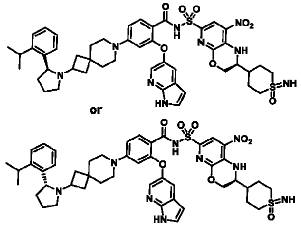 | 2-((1*H*-pyrrolo[2,3*-b*]pyridin-5-yl)oxy)-*N*-(((*S*)-2-(1-imino-1-oxid ohexahydro- 1 λ⁶-thiopyran-4-yl)-8-nitro-2,3-dihydro-1*H*-pyrido[2, 3-*b*][1,4]oxazin-6-yl)sulfonyl)-4-(2-((*R* or *S*)-2-(2-isopropylphenyl)pyrrolid in-1-yl)-7-azaspiro[3.5]nonan-7-yl)benzamide | MS-ESI (m/z): 938 [M + 1]⁺ |
| B-57 | 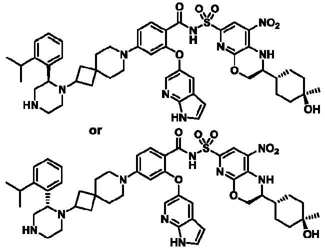 | 2-((1*H*-pyrrolo[2,3-b]pyridin-5-yl )oxy)-*N*-(((*S*)-2-((1*r*,4*S*)-4-hydro xy-4-methylcyclohexyl)-8-nitro-2,3-dihydro-1*H*-pyrido[2,3-*b*][1, 4]oxazin-6-yl)sulfonyl)-4-(2-((*R* or *S*)-2-(2-isopropylphenyl)piperazi n-1-yl)-7-azaspiro[3.5]nonan-7-yl )benzamide | MS-ESI (m/z): 934 [M + 1]⁺ |
| B-58 | 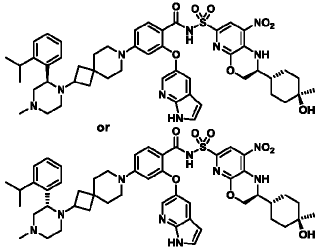 | 2-((1H-pyrrolo[2,3-b]pyridin-5-yl )oxy)-*N*-(((*S*)-2-((1*r*,4*S*)-4-hydro xy-4-methylcyclohexyl)-8-nitro-2,3-dihydro-1*H*-pyrido[2,3-*b*][1, 4]oxazin-6-yl)sulfonyl)-4-(2-((R or S)-2-(2-isopropylphenyl)-4-meth ylpiperazin-1-yl)-7-azaspiro[3.5] nonan-7-yl)benzamide | MS-ESI (m/z): 948 [M + 1]⁺ |
| B-59 | 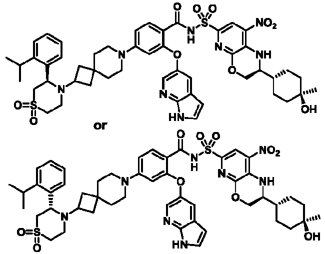 | 2-((1H-pyrrolo[2,3-b]pyridin-5-y l)oxy)-*N*-(((*S*)-2-((1*r*,4*S*)-4-hydro xy-4-methylcyclohexyl)-8-nitro-2,3-dihydro-1*H*-pyrido[2,3-*b*][1, 4]oxazin-6-yl)sulfonyl)-4-(2-((R or S)-3-(2-isopropylphenyl)-1,1-dio xidothiomorpholino)-7-azaspiro[ 3.5]nonan-7-yl)benzamide | MS-ESI (m/z): 983 [M + 1]⁺ |
| B-60 | 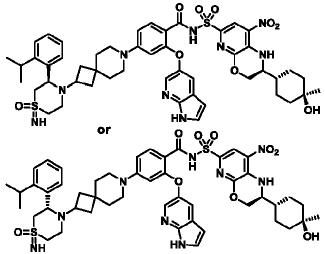 | 2-((1H-pyrrolo[2,3-b]pyridin-5-yl )oxy)-*N*-(((*S*)-2-((1*r*,4*S*)-4-hydro xy-4-methylcyclohexyl)-8-nitro-2,3-dihydro-1*H*-pyrido[2,3-*b*][1, 4]oxazin-6-yl)sulfonyl)-4-(2-((3 *R or* 3*S*)-1-imino-3-(2-isopropylphenyl )-1-oxido-1λ⁶-thiomorpholino)-7 -azaspiro[3.5]nonan-7-yl)benzam ide | MS-ESI (m/z): 982 [M + 1]⁺ |
| B-61 | 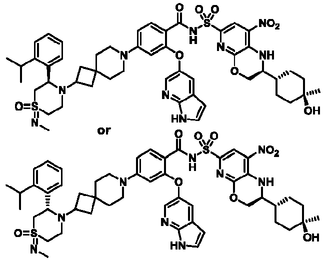 | 2-((1*H*-pyrrolo[2,3-*b*]pyridin-5-yl )oxy)-*N*-(((*S*)-2-((1*r*,4*S*)-4-hydro xy-4-methylcyclohexyl)-8-nitro-2,3-dihydro-1*H*-pyrido[2,3-*b*][1, 4]oxazin-6-yl)sulfonyl)-4-(2-((3 *R* or 3S)-3-(2-isopropylphenyl)-1-(me thylimino)-1-oxido-1λ⁶-thiontorp holino)-7-azaspiro[3.5]nonan-7-yl)benzamide | MS-ESI (m/z): 996 [M + 1]⁺ |
| B-62 | 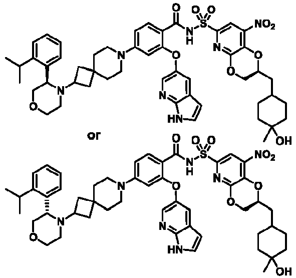 | 2-((1*H*-pyrrolo[2,3-*b*]pyridin-5-yl )oxy)-*N*-(((*S*)-2-((4-hydroxy-4-methylcyclohexyl)methyl)-8-nitr o-2,3-dihydro-[1,4]dioxino[2,3-*b* ]pyridin-6-yl)sulfonyl)-4-(2-((*R* or *S*)-3-(2-isopropylphenyl)morphol ino)-7-azaspiro[3.5]nonan-7-yl)b enzamide | MS-ESI (m/z): 950 [M + 1]⁺ |
| B-63 | 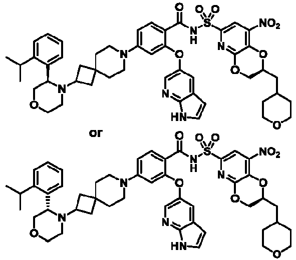 | 2-((1*H*-pyrrolo[2,3-*b*]pyridin-5-yl )oxy)-4-(2-((*R* or *S*)-3-(2-isopropylphenyl)morphol ino)-7-azaspiro[3.5]nonan-7-yl)-*N*-(((*S*)-8-nitro-2-((tetrahydro-2*H* -pyran-4-yl)methyl)-2,3-dihydro-[1,4]dioxino[2,3-b]pyridin-6-yl)s ulfonyl)benzamide | MS-ESI (m/z): 922 [M + 1]⁺ |
| B-64 | 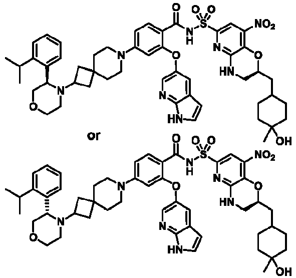 | 2-((1*H*-pyrrolo[2,3-*b*]pyridin-5-y l)oxy)-*N*-(((*S*)-2-((4-hydroxy-4-methylcyclohexyl)methyl)-8-nitr o-3,4-dihydro-2*H*-pyrido[3,2-*b*][ 1,4]oxazin-6-yl)sulfonyl)-4-(2-(( *S*)-3-(2-isopropylphenyl)morphol ino)-7-azaspiro[3.5]nonan-7-yl)b enzamide | MS-ESI (m/z): 949 [M + 1]⁺ |
| B-65 | 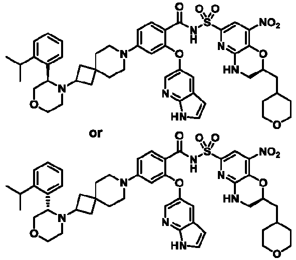 | 2-((1*H*-pyrrolo[2,3-b]pyridin-5-y l)oxy)-4-(2-((*R* or *S*)-3-(2-isopropylphenyl)morphol ino)-7-azaspiro[3.5]nonan-7-yl)-*N*-(((*S*)-8-nitro-2-((tetrahydro-2*H* -pyran-4-yl)methyl)-3,4-dihydro-2*H*-pyrido[3,2-*b*][1,4]oxazin-6-yl )sulfonyl)benzamide | MS-ESI (m/z): 921 [M + 1]⁺ |
| B-66 | 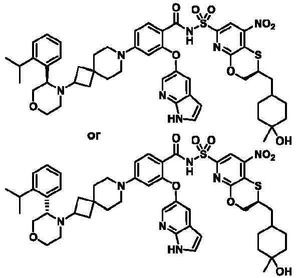 | 2-((1*H*-pyrrolo[2,3-*b*]pyridin-5-yl )oxy)-*N*-(((*S*)-2-((4-hydroxy-4-methylcyclohexyl)methyl)-8-nitro -2,3-dihydro-[1,4]oxathiino[2,3 -*b*]pyridin-6-yl)sulfonyl)-4-(2-(( *R* or *S*)-3-(2-isopropylphenyl)morphol ino)-7-azaspiro[3.5]nonan-7-yl)b enzamide | MS-ESI (m/z): 966 [M + 1]⁺ |
| B-67 | 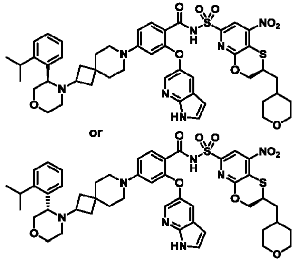 | 2-((1*H*-pyrrolo[2,3-*b*]pyridin-5-yl )oxy)-4-(2-((*R* or *S*)-3-(2-isopropylphenyl)morphol ino)-7-azaspiro[3.5]nonan-7-yl)-*N*-(((*S*)-8-nitro-2-((tetrahydro-2*H* -pyran-4-yl)methyl)-2,3-dihydro-[1,4]oxathiino[2,3-*b*]pyridin-6-yl )sulfonyl)benzamide | MS-ESI (m/z): 938 [M + 1]⁺ |
| B-68 | 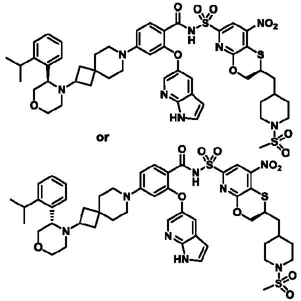 | 2-((1*H*-pyrrolo[2,3-*b*]pyridin-5-yl)oxy)-4-(2-((*R* or *S*)-3-(2-isopropylphenyl)morphol ino)-7-azaspiro[3.5]nonan-7-yl)-*N*-(((*S*)-2-((1-(methylsulfonyl)pi peridin-4-yl)methyl)-8-nitro-2,3-dihydro-[1,4]oxathiino[2,3-b]pyr idin-6-yl)sulfonyl)benzamide | MS-ESI (m/z): 1015 [M + 1]⁺ |

Examples C-1 ~ C-7 listed in Table 4 were prepared using appropriate intermediates, which can be readily synthesized by methods known in the literature or may be prepared by a variety of methods familiar to those skilled in the art. The structures and names of Examples C-1 ~ C-7 are given in table 4.

**Table 4**

| EXAMPLE | STRUCTURE | NAME | DATA |
|---|---|---|---|
| C-1 | 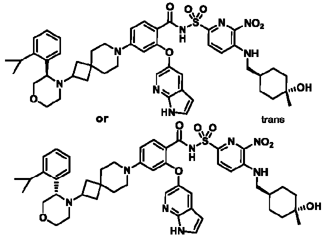 | 2-((1*H*-pyrrolo[2,3-*b*]pyridin-5-yl )oxy)-*N*-((5-((((1*r*,4*r*)-4-hydrox y-4-methylcyclohexyl)methyl) amino)-6-nitropyridin-2-yl)sulfon yl)-4-(2-((*R* or *S*)-3-(2-isopropylphenyl)morphol ino)-7-azaspiro[3.5]nonan-7-yl) benzamide | MS-ESI (m/z): 907 [M + 1]⁺ |
| C-2 | 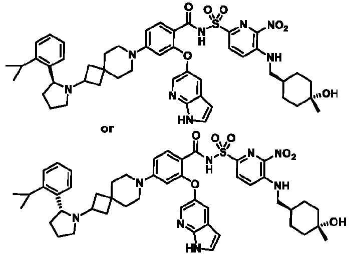 | 2-((1*H*-pyrrolo[2,3-*b*]pyridin-5-yl )oxy)-*N*-((5-((((1*r*,4*r*)-4-hydroxy -4-methylcyclohexyl)methyl) amino)-6-nitropyridin-2-yl)sulfon yl)-4-(2-((*R* or *S*)-2-(2-isopropylphenyl)pyrrolid in-1-yl)-7-azaspiro[3.5]nonan-7-yl)benzamide | MS-ESI (m/z): 891 [M + 1]⁺ |
| C-3 | 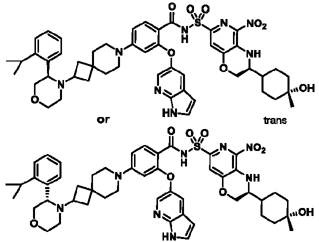 | 2-((1*H*-pyrrolo[2,3-*b*]pyridin-5-yl )oxy)-*N*-(((3*S*)-3-(4-hydroxy-4-methylcyclohexyl)-5-nitro-3,4-di hydro-2*H*-pyrido[4,3-b][1,4]oxaz in-7-yl)sulfonyl)-4-(2-((*R* or *S*)-3-(2-isopropylphenyl)morphol ino)-7-azaspiro[3.5]nonan-7-yl) benzamide | MS-ESI (m/z): 935 [M + 1]⁺ |
| C-4 | 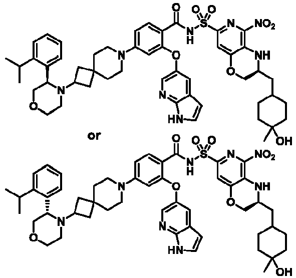 | 2-((1*H*-pyrrolo[2,3-*b*]pyridin-5-y l)oxy)-*N*-(((*S*)-3-((4-hydroxy-4-methylcyclohexyl)methyl)-5-nitr o-3,4-dihydro-2*H*-pyrido[4,3-*b*][ 1,4]oxazin-7-yl)sulfonyl)-4-(2-(( *R* or *S*)-3-(2-isopropylphenyl)morphol ino)-7-azaspiro[3.5]nonan-7-yl)b enzamide | MS-ESI (m/z): 949 [M + 1]⁺ |
| C-5 | 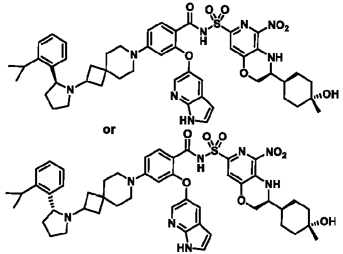 | 2-((1*H*-pyrrolo[2,3-*b*]pyridin-5-y l)oxy)-*N*-(((*S*)-3-((1*r*,4*S*)-4-hydro xy-4-methylcyclohexyl)-5-nitro-3,4-dihydro-2*H*-pyrido[4,3-*b*][1, 4]oxazin-7-yl)sulfonyl)-4-(2-((*R* or *S*)-2-(2-isopropylphenyl)pyrrolid in-1-yl)-7-azaspiro[3.5]nonan-7-yl)benzamide | MS-ESI (m/z): 919 [M + 1]⁺ |
| C-6 | 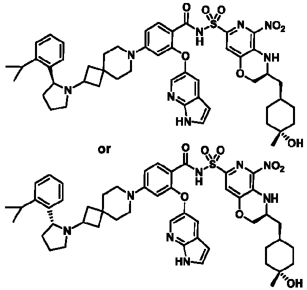 | 2-((1*H*-pyrrolo[2,3-*b*]pyridin-5-y l)oxy)-*N*-(((*S*)-3-(((1*r*,4*S*)-4-hydr oxy-4-methylcyclohexyl)methyl) -5-nitro-3,4-dihydro-2*H*-pyrido[4 ,3-b][1,4]oxazin-7-yl)sulfonyl)-4 -(2-((*R* or *S*)-2-(2-isopropylphenyl)pyrrolid in-1-yl)-7-azaspiro[3.5]nonan-7-yl)benzamide | MS-ESI (m/z): 933 [M + 1]⁺ |
| C-7 | 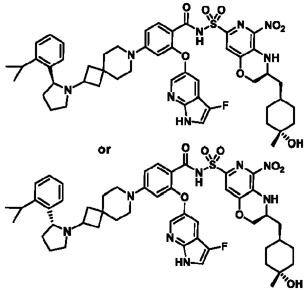 | 2-((3-fluoro-1*H*-pyrrolo[2,3-*b*]py ridin-5-yl)oxy)-*N*-(((*S*)-3-(((1*r*,4 *S*)-4-hydroxy-4-methylcyclohexy l)methyl)-5-nitro-3,4-dihydro-2*H* -pyrido[4,3-*b*][1,4]oxazin-7-yl)s ulfonyl)-4-(2-((*R* or *S*)-2-(2-isopropylphenyl)pyrrolid in-1-yl)-7-azaspiro[3.5]nonan-7-yl)benzamide | MS-ESI (m/z): 951 [M + 1]⁺ |

Examples D-1 ~ D-286 listed in Table 5 were prepared using appropriate intermediates, which can be readily synthesized by methods known in the literature or may be prepared by a variety of methods familiar to those skilled in the art. The structures and names of Reference Examples D-1 ~ D-286 are given in table 5

**Table 5**

| EXAMPLE | STRUCTURE | NAME | DATA |
|---|---|---|---|
| D-1 | 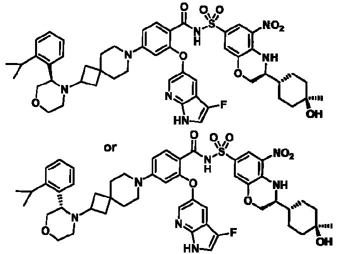 | 2-((3-fluoro-1*H*-pyrrolo[2,3-b]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( *R* or *S*)-3-(2-isopropylphenyl)morphol ino)-7-azaspiro[3.5]nonan-7-yl)b enzamide | MS-ESI (m/z): 952 [M + 1]⁺ |
| D-2 | 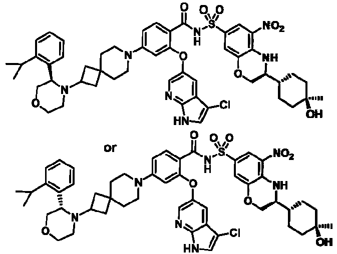 | 2-((3-chloro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( *R* or *S*)-3-(2-isopropylphenyl)morphol ino)-7-azaspiro[3.5]nonan-7-yl)b enzamide | MS-ESI (m/z): 968 [M + 1]⁺ |
| D-3 | 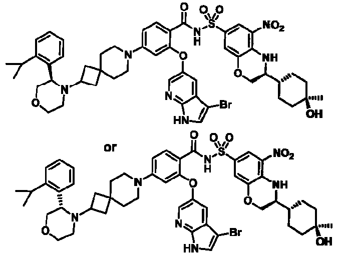 | 2-((3-bromo-1*H*-pyrrolo[2,3-*b*]p yridin-5-yl)oxy)-*N*-(((*S*)-3-((1r,4 *S*)-4-hydroxy-4-methylcyclohexy l)-5-nitro-3,4-dihydro-2*H*-benzo[ *b*][1,4]oxazin-7-yl)sulfonyl)-4-(2 -((*R* or *S*)-3-(2-isopropylphenyl)morphol ino)-7-azaspiro[3.5]nonan-7-yl)b enzamide | MS-ESI (m/z): 1012, 1014 [M + 1]⁺ |
| D-4 | 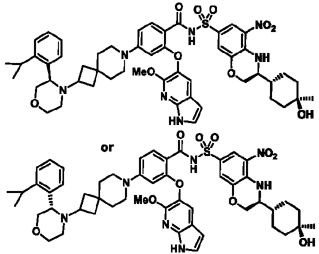 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-3-(2-isopropylphenyl)morphol ino)-7-azaspiro[3.5]nonan-7-yl)-2-((6-methoxy-1*H*-pyrrolo[2,3-*b*] pyridin-5-yl)oxy)benzamide | MS-ESI (m/z): 964 [M + 1]⁺ |
| D-5 | 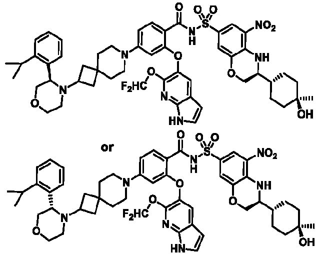 | 2-((6-(difluoromethoxy)-1*H*-pyrr olo[2,3-b]pyridin-5-yl)oxy)-*N*-((( *S*)-3-((1*r*,4*S*)-4-hydroxy-4-methy lcyclohexyl)-5-nitro-3,4-dihydro-2*H*-benzo[*b*][1,4]oxazin-7-yl)sulf onyl)-4-(2-((*R* or *S*)-3-(2-isopropylphenyl)morphol ino)-7-azaspiro[3.5]nonan-7-yl)b enzamide | MS-ESI (m/z): 1000 [M + 1]⁺ |
| D-6 | 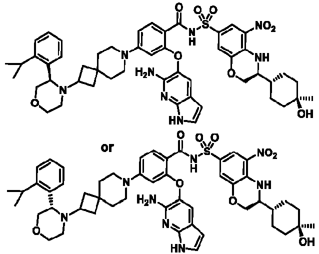 | 2-((6-amino-1*H*-pyrrolo[2,3-b]py ridin-5-yl)oxy)-*N*-(((*S*)-3-((1*r*,4*S*) -4-hydroxy-4-methylcyclohexyl)-5-nitro-3,4-dihydro-2*H*-benzo[b] [1,4]oxazin-7-yl)sulfonyl)-4-(2-(( *R* or *S*)-3-(2-isopropylphenyl)morphol ino)-7-azaspiro[3.5]nonan-7-yl)b enzamide | MS-ESI (m/z): 949 [M + 1]⁺ |
| D-7 | 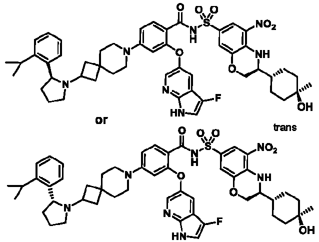 | 2-((3-fluoro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( *R* or *S*)-2-(2-isopropylphenyl)pyrrolid in-1-yl)-7-azaspiro[3.5]nonan-7-yl)benzamide | MS-ESI (m/z): 936 [M + 1]⁺ |
| D-8 | 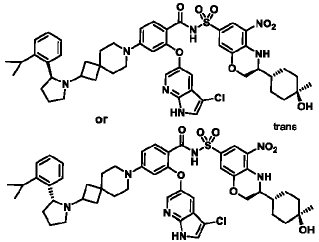 | 2-((3-chloro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2H-benzo[b] [1,4]oxazin-7-yl)sulfonyl)-4-(2-(( *R* or *S*)-2-(2-isopropylphenyl)pyrrolid in-1-yl)-7-azaspiro[3.5]nonan-7-yl)benzamide | MS-ESI (m/z): 952 [M + 1]⁺ |
| D-9 | 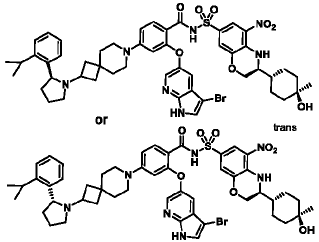 | 2-((3-bromo-1*H*-pyrrolo[2,3-b]p yridin-5-yl)oxy)-*N*-(((*S*)-3-((1*r*,4 *S*)-4-hydroxy-4-methylcyclohexy l)-5-nitro-3,4-dihydro-2*H*-benzo[ *b*][1,4]oxazin-7-yl)sulfonyl)-4-(2 -((*R* or *S*)-2-(2-isopropylphenyl)pyrrolid in-1-yl)-7-azaspiro[3.5]nonan-7-yl)benzamide | MS-ESI (m/z): 996, 998 [M + 1]⁺ |
| D-10 | 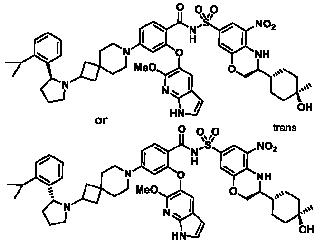 | *N*-(((*S*)-3-((1*r*,4*S*)-4-hydroxy-4-methylcyclohexyl)-5-nitro-3,4-di hydro-2H-benzo[b] [1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-methoxy-1*H*-pyrrolo[2, 3-b]pyridin-5-yl)oxy)benzamide | MS-ESI (m/z): 948 [M + 1]⁺ |
| D-11 | 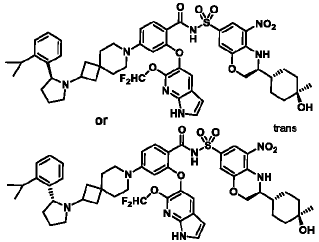 | 2-((6-(difluoromethoxy)-1*H*-pyrr olo[2,3-*b*]pyridin-5-yl)oxy)-*N*-((( *S*)-3-((1*r*,4*S*)-4-hydroxy-4-methy lcyclohexyl)-5-nitro-3,4-dihydro-2*H*-benzo[*b*][1,4]oxazin-7-yl)sulf onyl)-4-(2-((*R* or *S*)-2-(2-isopropylphenyl)pyrrolid in-1-yl)-7-azaspiro[3.5]nonan-7-yl)benzamide | MS-ESI (m/z): 984 [M + 1]⁺ |
| D-12 | 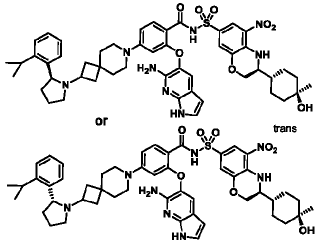 | 2-((6-amino-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( *R* or *S*)-2-(2-isopropylphenyl)pyrrolid in-1-yl)-7-azaspiro[3.5]nonan-7-yl)benzamide | MS-ESI (m/z): 933 [M + 1]⁺ |
| D-13 | 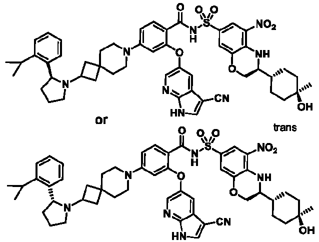 | 2-((3-cyano-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( *R* or *S*)-2-(2-isopropylphenyl)pyrrolid in-1-yl)-7-azaspiro[3.5]nonan-7-yl)benzamide | MS-ESI (m/z): 943 [M + 1]⁺ |
| D-14 | 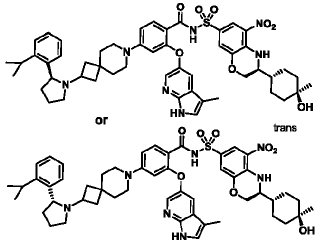 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((3-methyl-1*H*-pyrrolo[2,3-*b*]pyridin-5-yl)oxy)benzamide | MS-ESI (m/z): 932 [M + 1]⁺ |
| D-15 | 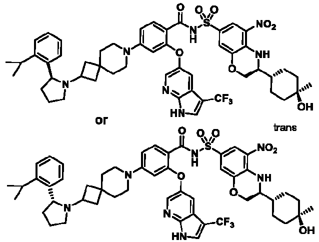 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((3-(trifluoromethyl)-1*H*-py rrolo[2,3-*b*]pyridin-5-yl)oxy)ben zamide | **MS-ESI** (m/z): 986 [M + 1]⁺ |
| D-16 | 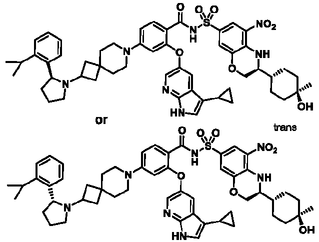 | 2-((3-cyclopropyl-1*H*-pyrrolo[2, 3-b]pyridin-5-yl)oxy)-*N*-(((*S*)-3-( (1*r*,4*S*)-4-hydroxy-4-methylcyclo hexyl)-5-nitro-3,4-dihydro-2*H*-be nzo[*b*][1,4]oxazin-7-yl)sulfonyl)-4-(2-((*R* or *S*)-2-(2-isopropylphenyl)pyrrolid in-1-yl)-7-azaspiro[3.5]nonan-7-yl)benzamide | MS-ESI (m/z): 958 [M + 1]⁺ |
| D-17 | 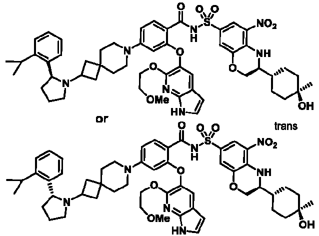 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-(2-methoxyethoxy)-1*H-*pyrrolo[2,3-b]pyridin-5-yl)oxy)b enzamide | MS-ESI (m/z): 992 [M + 1]⁺ |
| D-18 | 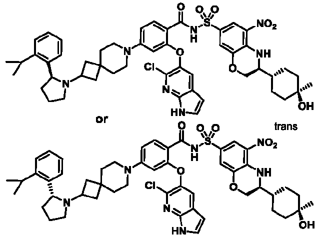 | 2-((6-chloro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( *R* or *S*)-2-(2-isopropylphenyl)pyrrolid in-1-yl)-7-azaspiro[3.5]nonan-7-yl)benzamide | MS-ESI (m/z): 952 [M + 1]⁺ |
| D-19 | 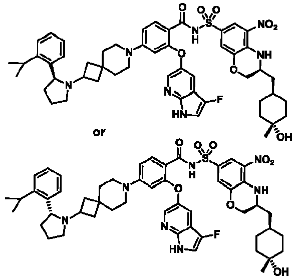 | 2-((3-fluoro-1*H*-pyrrolo[2,3-*b*]py ridin-5-yl)oxy)-*N*-(((*S*)-3-(((1*r*,4*S* )-4-hydroxy-4-methylcyclohexyl) methyl)-5-nitro-3,4-dihydro-2*H-*benzo[b] [1,4]oxazin-7-yl)sulfony l)-4-(2-((*R* or *S*)-2-(2-isopropylphenyl)pyrrolid in-1-yl)-7-azaspiro[3.5]nonan-7-yl)benzamide | MS-ESI (m/z): 950 [M + 1]⁺ |
| D-20 | 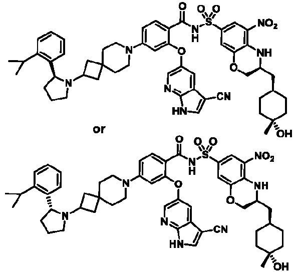 | 2-((3-cyano-1*H*-pyrrolo[2,3-*b*]py ridin-5-yl)oxy)-*N*-(((*S*)-3-(((1*r*,4*S* )-4-hydroxy-4-methylcyclohexyl) methyl)-5-nitro-3,4-dihydro-2*H-*benzo[*b*][1,4]oxazin-7-yl)sulfony l)-4-(2-((*R* or *S*)-2-(2-isopropylphenyl)pyrrolid in-1-yl)-7-azaspiro[3.5]nonan-7-yl)benzamide | MS-ESI (m/z): 957 [M + 1]⁺ |
| D-21 | 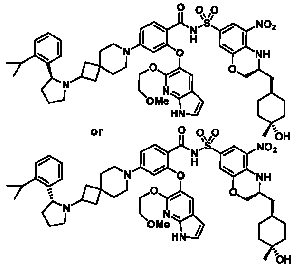 | *N*-(((*S*)-3-(((1*r*,4*S*)-4-hydroxy-4-methylcyclohexyl)methyl)-5-nitr o-3,4-dihydro-2*H*-benzo[*b*][1,4]o xazin-7-yl)sulfonyl)-4-(2-((*R* or *S*)-2-(2-isopropylphenyl)pyrrolid in-1-yl)-7-azaspiro[3.5]nonan-7-yl)-2-((6-(2-methoxyethoxy)-1*H-*pyrrolo[2,3-*b*]pyridin-5-yl)oxy)b enzamide | MS-ESI (m/z): 1006 [M + 1]⁺ |
| D-22 | 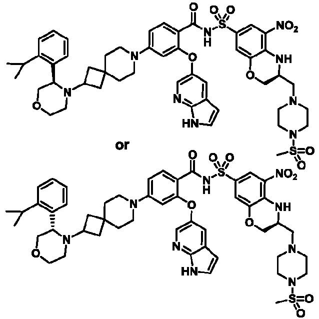 | 2-((1*H*-pyrrolo[2,3-*b*]pyridin-5-y l)oxy)-4-(2-((*R* or *S*)-3-(2-isopropylphenyl)morphol ino)-7-azaspiro[3.5]nonan-7-yl)-N-(((*S*)-3-((4-(methylsulfonyl)pi perazin-1-yl)methyl)-5-nitro-3,4-dihydro-2*H*-benzo[*b*][1,4]oxazin-7-yl)sulfonyl)benzamide | MS-ESI (m/z): 998 [M + 1]⁺ |
| D-23 | 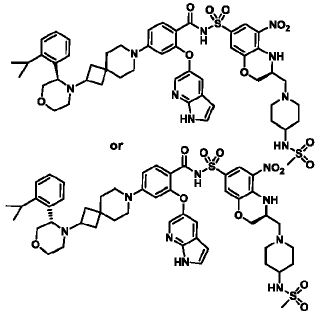 | 2-((1*H*-pyrrolo[2,3-*b*]pyridin-5-y l)oxy)-4-(2-((*R* or *S*)-3-(2-isopropylphenyl)morphol ino)-7-azaspiro[3.5]nonan-7-yl)-*N*-(((*S*)-3-((4-(methylsulfonamid o)piperidin-1-yl)methyl)-5-nitro-3,4-dihydro-2*H*-benzo[*b*][1,4]oxa zin-7-yl)sulfonyl)benzamide | MS-ESI (m/z): 1012 [M + 1]⁺ |
| D-24 | 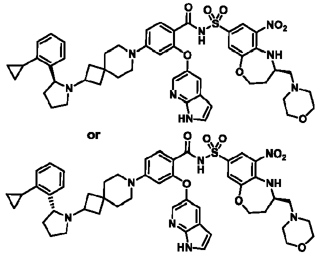 | 2-((1*H*-pyrrolo[2,3-*b*]pyridin-5-y l)oxy)-4-(2-((*R* or *S*)-2-(2-cyclopropylphenyl)pyrrol idin-1-yl)-7-azaspiro[3.5]nonan-7-yl)-*N*-(((*R*)-4-(morpholinometh yl)-6-nitro-2,3,4,5-tetrahydroben zo[*b*][1,4]oxazepin-8-yl)sulfonyl )benzamide | MS-ESI (m/z): 917 [M + 1]⁺ |
| D-25 | 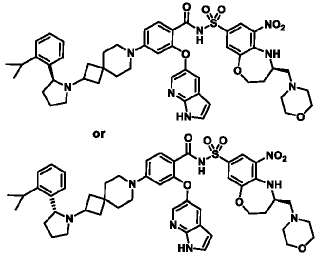 | 2-((1*H*-pyrrolo[2,3-*b*]pyridin-5-y l)oxy)-4-(2-((*R* or *S*)-2-(2-isopropylphenyl)pyrrolid in-1-yl)-7-azaspiro[3.5]nonan-7-yl)-*N*-(((*R*)-4-(morpholinomethyl )-6-nitro-2,3,4,5-tetrahydrobenzo [*b*][1,4]oxazepin-8-yl)sulfonyl)b enzamide | MS-ESI (m/z): 919 [M + 1]⁺ |
| D-26 | 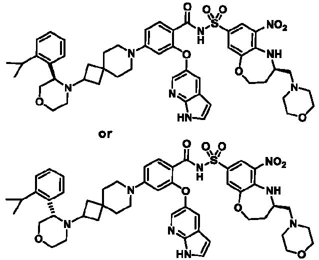 | 2-((1*H*-pyrrolo[2,3-*b*]pyridin-5-y l)oxy)-4-(2-((*R* or S)-3-(2-isopropylphenyl)morphol ino)-7-azaspiro[3.5]nonan-7-yl)-*N*-(((*R*)-4-(morpholinomethyl)-6-nitro-2,3,4,5-tetrahydrobenzo[*b*][ 1,4]oxazepin-8-yl)sulfonyl)benza mide | MS-ESI (m/z): 935 [M + 1]⁺ |
| D-27 | 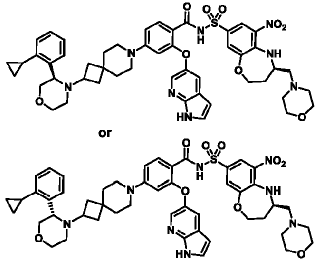 | 2-((1*H*-pyrrolo[2,3-*b*]pyridin-5-y l)oxy)-4-(2-((*R* or *S*)-3-(2-cyclopropylphenyl)morp holino)-7-azaspiro[3.5]nonan-7-y l)-*N*-(((*R*)-4-(morpholinomethyl)-6-nitro-2,3,4,5-tetrahydrobenzo[*b* ][1,4]oxazepin-8-yl)sulfonyl)ben zamide | MS-ESI (m/z): 933 [M + 1]⁺ |
| D-28 | 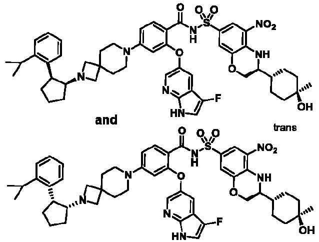 | 2-((3-fluoro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( 1*S*,2*S* or1*R*,2*R*)-2-(2-isopropylphenyl)c yclopentyl)-2,7-diazaspiro[3.5]no nan-7-yl)benzamide | MS-ESI (m/z): 936 [M + 1]⁺ |
| D-28a | 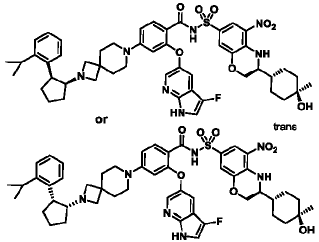 | 2-((3-fluoro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( 1*S*,2*S* or 1*R*,2*R*)-2-(2-isopropylphenyl)cyc lopentyl)-2,7-diazaspiro[3.5]nona n-7-yl)benzamide | MS-ESI (m/z): 936 [M + 1]⁺ |
| D-28b | | | |
| D-29 | 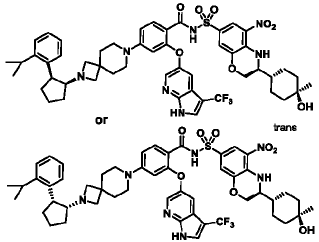 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((1*S*,2*S* or 1*R*,2*R*)-2-(2-isopropylphenyl)cyc lopentyl)-2,7-diazaspiro[3.5]nona n-7-yl)-2-((3-(trifluoromethyl)-1 H-pyrrolo[2,3-b]pyridin-5-yl)oxy )benzamide | MS-ESI (m/z): 986 [M + 1]⁺ |
| D-30 | 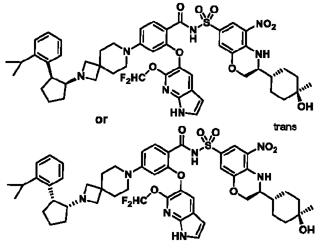 | 2-((6-(difluoromethoxy)-1*H*-pyrr olo[2,3-*b*]pyridin-5-yl)oxy)-*N*-((( *S*)-3-((1*r*,4*S*)-4-hydroxy-4-methyl cyclohexyl)-5-nitro-3,4-dihydro-2*H*-benzo[*b*][1,4]oxazin-7-yl)sulf onyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 984 [M + 1]⁺ |
| D-31 | 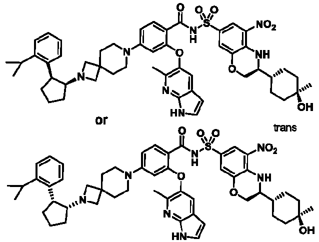 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((1*S*,2*S* or 1*R*,2*R*)-2-(2-isopropylphenyl)cyc lopentyl)-2,7-diazaspiro[3.5]nona n-7-yl)-2-((6-methyl-1*H*-pyrrolo[ 2,3-*b*]pyridin-5-yl)oxy)benzamide | MS-ESI (m/z): 932 [M + 1]⁺ |
| D-32 | 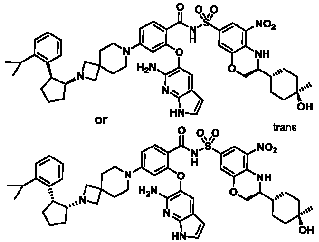 | 2-((6-amino-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( 1*S*,2*S* or 1*R*,2*R*)-2-(2-isopropylphenyl)cyc lopentyl)-2,7-diazaspiro[3.5]nonan -7-yl)benzamide | MS-ESI (m/z): 933 [M + 1]⁺ |
| D-33 | 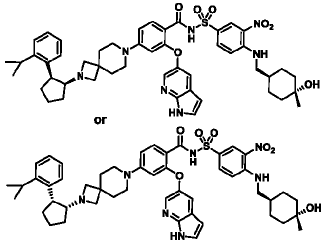 | 2-((1*H*-pyrrolo[2,3-*b*]pyridin-5-yl )oxy)-*N*-((4-((((1*r*,4*r*)-4-hydroxy -4-methylcyclohexyl)methyl)ami no)-3-nitrophenyl)sulfonyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 890 [M + 1]⁺ |
| D-34a | 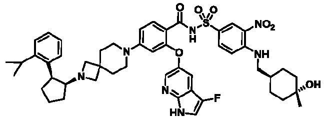 | 2-((3-fluoro-1*H*-pyrrolo[2,3-*b*]py ridin-5-yl)oxy)-*N*-((4-((((1*r*,4*r*)-4 -hydroxy-4-methylcyclohexyl)m ethyl)amino)-3-nitrophenyl)sulfo nyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 908 [M + 1]⁺ |
| | and | | |
| | 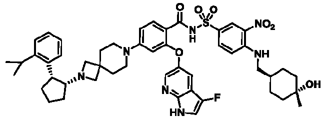 | | |
| D-34b | 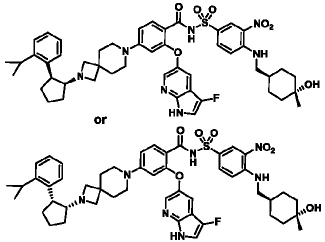 | 2-((3-fluoro-1*H*-pyrrolo[2,3-*b*]py ridin-5-yl)oxy)-*N*-((4-((((1*r*,4*r*)-4 -hydroxy-4-methylcyclohexyl)m ethyl)amino)-3-nitrophenyl)sulfo nyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 908 [M + 1]⁺ |
| D-35 | 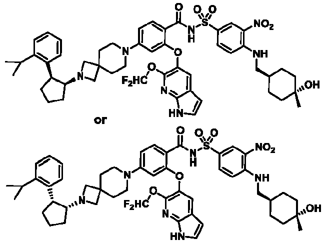 | 2-((6-(difluoromethoxy)-1*H*-pyrr olo[2,3-*b*]pyridin-5-yl)oxy)-*N*-(( 4-((((1r,4r)-4-hydroxy-4-methylc yclohexyl)methyl)amino)-3-nitro phenyl)sulfonyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 956 [M + 1]⁺ |
| D-36 | 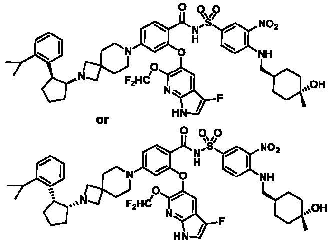 | 2-((6-(difluoromethoxy)-3-fluoro -1*H*-pyrrolo[2,3-b]pyridin-5-yl) oxy)-*N*-((4-((((1r,4*r*)-4-hydroxy-4-methylcyclohexyl)methyl)amino) -3-nitrophenyl)sulfonyl)-4-(2-((1 *S,2S* or 1*R*,2*R*)-2-(2-isopropylphenyl)cyc lopentyl)-2,7-diazaspiro[3.5]nona n-7-yl)benzamide | MS-ESI (m/z): 974 [M + 1]⁺ |
| D-37 | 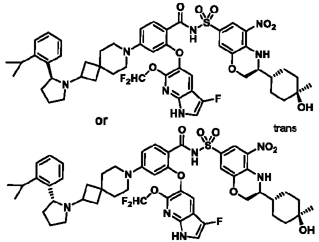 | 2-((6-(difluoromethoxy)-3-fluoro -1*H*-pyrrolo[2,3-b]pyridin-5-yl)o xy)-*N*-(((*S*)-3-((1*r*,4*S*)-4-hydroxy -4-methylcyclohexyl)-5-nitro-3,4 -dihydro-2*H*-benzo[*b*][1,4]oxazin -7-yl)sulfonyl)-4-(2-((*R* or *S*)-2-(2-isopropylphenyl)pyrrolid in-1-yl)-7-azaspiro[3.5]nonan-7-yl)benzamide | MS-ESI (m/z): 1002 [M + 1]⁺ |
| D-38 | 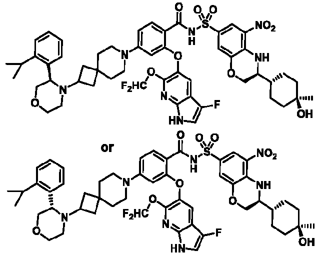 | 2-((6-(difluoromethoxy)-3-fluoro -1*H*-pyrrolo[2,3-*b*]pyridin-5-yl) oxy)-*N*-(((*S*)-3-((1*r*,4*S*)-4-hydroxy -4-methylcyclohexyl)-5-nitro-3,4 -dihydro-2*H*-benzo[*b*][1,4]oxazin -7-yl)sulfonyl)-4-(2-((*R* or *S*)-3-(2-isopropylphenyl)morphol ino)-7-azaspiro[3.5]nonan-7-yl) benzamide | MS-ESI (m/z): 1018 [M + 1]⁺ |
| D-39 | 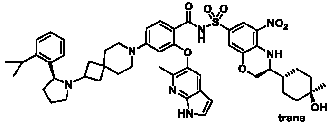 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-methyl-1*H*-pyrrolo[2,3-*b*]pyridin-5-yl)oxy)benzamide | MS-ESI (m/z): 932 [M + 1]⁺ |
| | or | | |
| | 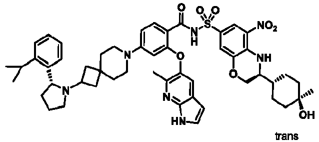 | | |
| D-40 | 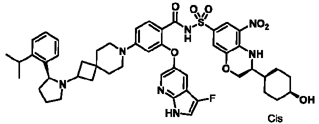 | 2-((3-fluoro-1H-pyrrolo[2,3-b]py ridin-5-yl)oxy)-*N*-(((*S*)-3-((1*s*,4*R* )-4-hydroxycyclohexyl)-5-nitro-3 ,4-dihydro-2*H*-benzo[*b*][1,4]oxaz in-7-yl)sulfonyl)-4-(2-((*R* or *S*)-2-(2-isopropylphenyl)pyrrolid in-1-yl)-7-azaspiro[3.5]nonan-7-yl)benzamide | MS-ESI (m/z): 922 [M + 1]⁺ |
| | or | | |
| | 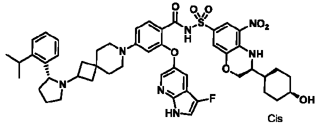 | | |
| D-41 | 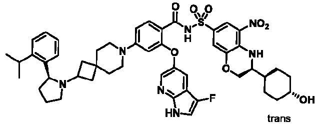 | 2-((3-fluoro-1H-pyrrolo[2,3-b]py ridin-5-yl)oxy)-*N*-(((*S*)-3-((1*r*,4*S*) -4-hydroxycyclohexyl)-5-nitro-3, 4-dihydro-2*H*-benzo[*b*][1,4]oxazi n-7-yl)sulfonyl)-4-(2-((*R* or *S*)-2-(2-isopropylphenyl)pyrrolid in-1-yl)-7-azaspiro[3.5]nonan-7-yl)benzamide | MS-ESI (m/z): 922 [M + 1]⁺ |
| | or | | |
| | 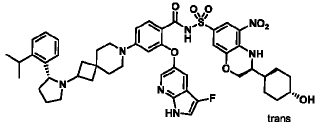 | | |
| D-42 | 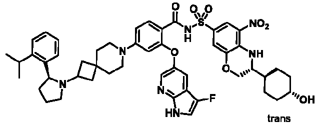 | 2-((3-fluoro-1*H*-pyrrolo[2,3-*b*]py ridin-5-yl)oxy)-*N*-(((*R*)-3-((1*r*,4*R* )-4-hydroxycyclohexyl)-5-nitro-3 ,4-dihydro-2*H*-benzo[*b*][1,4]oxaz in-7-yl)sulfonyl)-4-(2-((*R* or *S*)-2-(2-isopropylphenyl)pyrrolid in-1-yl)-7-azaspiro[3.5]nonan-7-yl)benzamide | MS-ESI (m/z): 922 [M + 1]⁺ |
| | or | | |
| | 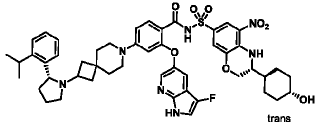 | | |
| D-43 | 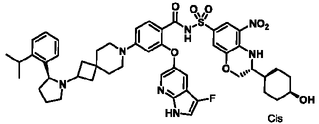 | 2-((3-fluoro-1*H*-pyrrolo[2,3-b]py ridin-5-yl)oxy)-*N*-(((*R*)-3-((1*s*,4*S* )-4-hydroxycyclohexyl)-5-nitro-3 ,4-dihydro-2*H*-benzo[*b*][1,4]oxaz in-7-yl)sulfonyl)-4-(2-((*R* or *S*)-2-(2-isopropylphenyl)pyrrolid in-1-yl)-7-azaspiro[3.5]nonan-7-yl)benzamide | MS-ESI (m/z): 922 [M + 1]⁺ |
| | or | | |
| | 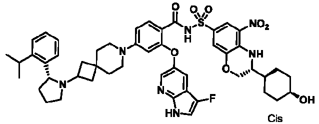 | | |
| D-44 | 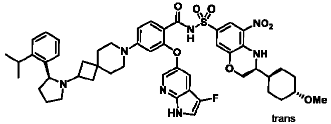 | 2-((3-fluoro-1*H*-pyrrolo[2,3-*b*]py ridin-5-yl)oxy)-4-(2-((*R* or *S*)-2-(2-isopropylphenyl)pyrrolid in-1-yl)-7-azaspiro[3.5]nonan-7-yl)-*N*-(((*S*)-3-((1*r*,4*S*)-4-methoxy cyclohexyl)-5-nitro-3,4-dihydro-2*H*-benzo[*b*][1,4]oxazin-7-yl)sulf onyl)benzamide | MS-ESI (m/z): 936 [M + 1]⁺ |
| | or | | |
| | 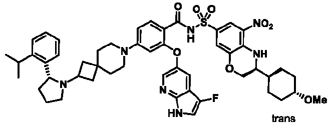 | | |
| D-45 | 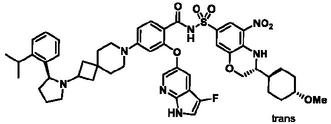 | 2-((3-fluoro-1*H*-pyrrolo[2,3-b]py ridin-5-yl)oxy)-4-(2-((*R* or *S*)-2-(2-isopropylphenyl)pyrrolid in-1-yl)-7-azaspiro[3.5]nonan-7-yl)-*N*-(((*R*)-3-((1*r*,4*S*)-4-methoxy cyclohexyl)-5-nitro-3,4-dihydro-2*H*-benzo[*b*][1,4]oxazin-7-yl)sulf onyl)benzamide | MS-ESI (m/z): 936 [M + 1]⁺ |
| | or | | |
| | 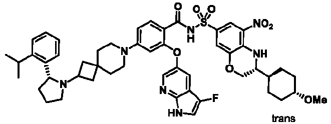 | | |
| D-46 | 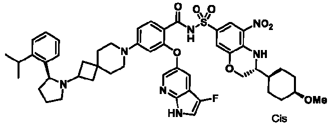 | 2-((3-fluoro-1*H*-pyrrolo[2,3-b]py ridin-5-yl)oxy)-4-(2-((*R* or *S*)-2-(2-isopropylphenyl)pyrrolid in-1-yl)-7-azaspiro[3.5]nonan-7-yl)-*N*-(((*R*)-3-((1*s*,4*S*)-4-methoxy cyclohexyl)-5-nitro-3,4-dihydro-2*H*-benzo[*b*][1,4]oxazin-7-yl)sulf onyl)benzamide | MS-ESI (m/z): 936 [M + 1]⁺ |
| | or | | |
| | 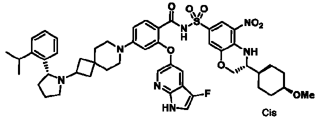 | | |
| D-47 | 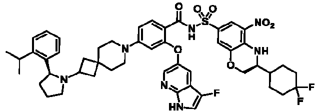 | *N*-(((*S*)-3-(4,4-difluorocyclohexyl )-5-nitro-3,4-dihydro-2*H*-benzo[ b][1,4]oxazin-7-yl)sulfonyl)-2-(( 3-fluoro-1*H*-pyrrolo[2,3-*b*]pyridi n-5-yl)oxy)-4-(2-((*R* or *S*)-2-(2-isopropylphenyl)pyrrolid in-1-yl)-7-azaspiro[3.5]nonan-7-yl)benzamide | MS-ESI (m/z): 942 [M + 1]⁺ |
| | or | | |
| | 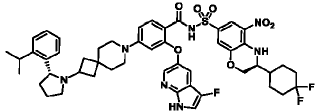 | | |
| D-48 | 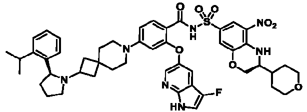 | 2-((3-fluoro-1*H*-pyrrolo[2,3-b]py ridin-5-yl)oxy)-4-(2-((*R* or *S*)-2-(2-isopropylphenyl)pyrrolid in-1-yl)-7-azaspiro[3.5]nonan-7-yl)-*N*-(((*S*)-5-nitro-3-(tetrahydro-2H-pyran-4-yl)-3,4-dihydro-2*H-*benzo[*b*][1,4]oxazin-7-yl)sulfony l)benzamide | MS-ESI (m/z): 908 [M + 1]⁺ |
| | or 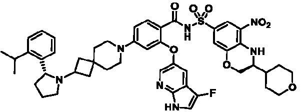 | | |
| D-49a | 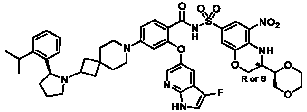 | N-(((*R* or *S*)-3-((*R*)-1,4-dioxan-2-yl)-5-nitr o-3,4-dihydro-2*H*-benzo[*b*][1,4] oxazin-7-yl)sulfonyl)-2-((3-fluoro-1*H*-pyrrolo[2,3-*b*]pyridin-5-yl)ox y)-4-(2-((*R* or *S*)-2-(2-isopropylphenyl)pyrrolid in-1-yl)-7-azaspiro[3.5]nonan-7-yl)benzamide | MS-ESI (m/z): 910 [M + 1]⁺ |
| D-49b | or 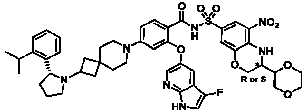 | | |
| D-50a | 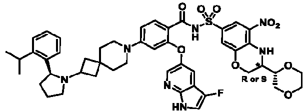 | *N*-(((*R* or *S*)-3-((*S*)-1,4-dioxan-2-yl)-5-nitro -3,4-dihydro-2*H*-benzo[*b*][1,4]ox azin-7-yl)sulfonyl)-2-((3-fluoro-1 *H*-pyrrolo[2,3-*b*]pyridin-5-yl)oxy )-4-(2-((*R* or *S*)-2-(2-isopropylphenyl)pyrrolid in-1-yl)-7-azaspiro[3.5]nonan-7-yl)benzamide | MS-ESI (m/z): 910 [M + 1]⁺ |
| D-50b | or 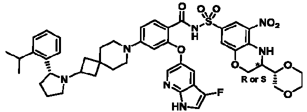 | | |
| D-51 | 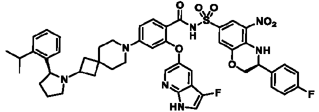 | 2-((3-fluoro-1*H*-pyrrolo[2,3-*b*]py ridin-5-yl)oxy)-*N*-(((*S*)-3-(4-fluor ophenyl)-5-nitro-3,4-dihydro-2*H* -benzo[*b*][1,4]oxazin-7-yl)sulfon yl)-4-(2-((*R* or *S*)-2-(2-isopropylphenyl)pyrrolid in-1-yl)-7-azaspiro[3.5]nonan-7-yl)benzamide | MS-ESI (m/z): 918 [M + 1]⁺ |
| | or | | |
| | 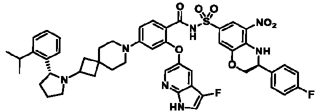 | | |
| D-52 | 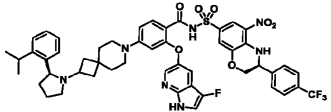 | 2-((3-fluoro-1*H*-pyrrolo[2,3-b]py ridin-5-yl)oxy)-4-(2-((*R* or *S*)-2-(2-isopropylphenyl)pyrrolid in-1-yl)-7-azaspiro[3.5]nonan-7-yl)-*N*-(((*S*)-5-nitro-3-(4-(trifluoro methyl)phenyl)-3,4-dihydro-2*H-*benzo[*b*][1,4]oxazin-7-yl)sulfony l)benzamide | MS-ESI (m/z): 968 [M + 1]⁺ |
| | or | | |
| | 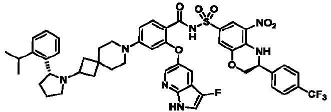 | | |
| D-53 | 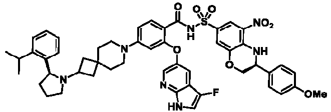 | 2-((3-fluoro-1*H*-pyrrolo[2,3-*b*]py ridin-5-yl)oxy)-4-(2-((*R* or *S*)-2-(2-isopropylphenyl)pyrrolid in-1-yl)-7-azaspiro[3.5]nonan-7-yl)-*N*-(((*S*)-3-(4-methoxyphenyl)-5-nitro-3,4-dihydro-2*H*-benzo[*b*] [1,4]oxazin-7-yl)sulfonyl)benza mide | MS-ESI (m/z): 930 [M + 1]⁺ |
| | or | | |
| | 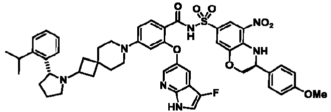 | | |
| D-54 | 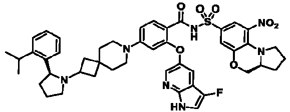 | 2-((3-fluoro-1*H*-pyrrolo[2,3-b]py ridin-5-yl)oxy)-4-(2-((*R* or *S*)-2-(2-isopropylphenyl)pyrrolid in-1-yl)-7-azaspiro[3.5]nonan-7-yl)-*N*-(((*S*)-9-nitro-2,3,3a,4-tetrah ydro-1*H*-benzo[*b*]pyrrolo[1,2-*d*][ 1,4]oxazin-7-yl)sulfonyl)benzam ide | MS-ESI (m/z): 864 [M + 1]⁺ |
| | or 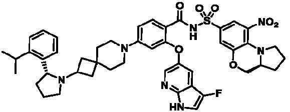 | | |
| D-55 | 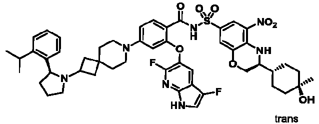 | 2-((3,6-difluoro-1*H*-pyrrolo[2,3-b]pyridin-5-yl)oxy)-*N*-(((*S*)-3-((1 *r*,4*S*)-4-hydroxy-4-methylcycloh exyl)-5-nitro-3,4-dihydro-2*H*-ben zo[*b*][1,4]oxazin-7-yl)sulfonyl)-4 -(2-((*R* or *S*)-2-(2-isopropylphenyl)pyrrolid in-1-yl)-7-azaspiro[3.5]nonan-7-yl)benzamide | MS-ESI (m/z): 954 [M + 1]⁺ |
| | or | | |
| | 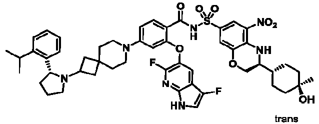 | | |
| D-56 | 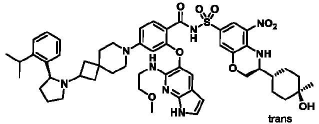 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-((2-methoxyethyl)amin o)-1*H*-pyrrolo[2,3-*b*]pyridin-5-yl )oxy)benzamide | MS-ESI (m/z): 991 [M + 1]⁺ |
| | or | | |
| | 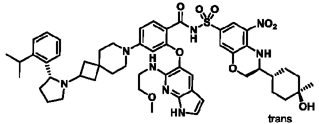 | | |
| D-57 | 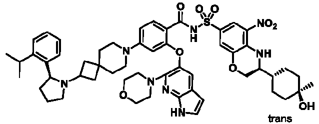 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-morpholino-1*H*-pyrrolo [2,3-*b*]pyridin-5-yl)oxy)benzamide | MS-ESI (m/z): 1003 [M + 1]⁺ |
| | or | | |
| | 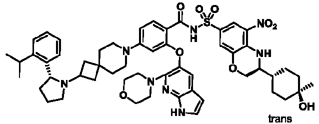 | | |
| D-58 | 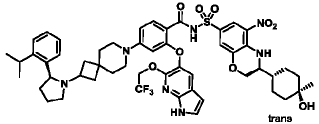 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-(2,2,2-trifluoroethoxy)-1*H*-pyrrolo[2,3-*b*]pyridin-5-yl)ox y)benzamide | MS-ESI (m/z): 1016 [M + 1]⁺ |
| | or | | |
| | 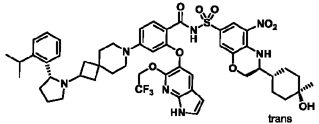 | | |
| D-59 | 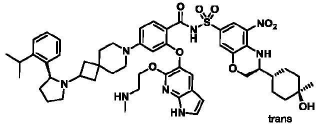 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-(2-(methylamino)ethox y)-1*H*-pyrrolo[2,3-*b*]pyridin-5-yl )oxy)benzamide | MS-ESI (m/z): 991 [M + 1]⁺ |
| | or | | |
| | 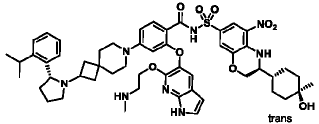 | | |
| D-60 | 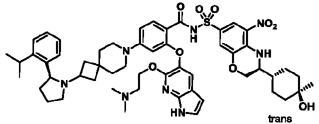 | 2-((6-(2-(dimethylamino)ethoxy) -1*H*-pyrrolo[2,3-*b*]pyridin-5-yl)o xy)-*N*-(((*S*)-3-((1r,4S)-4-hydroxy -4-methylcyclohexyl)-5-nitro-3,4 -dihydro-2*H*-benzo[b][1,4]oxazin -7-yl)sulfonyl)-4-(2-((*R* or *S*)-2-(2-isopropylphenyl)pyrrolid in-1-yl)-7-azaspiro[3.5]nonan-7-yl)benzamide | MS-ESI (m/z): 1005 [M + 1]⁺ |
| | or | | |
| | 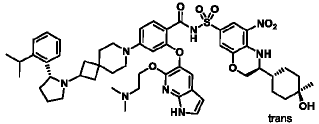 | | |
| D-61 | 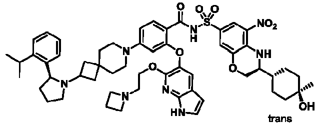 | 2-((6-(2-(azetidin-1-yl)ethoxy)-1 *H*-pyrrolo[2,3-*b*]pyridin-5-yl)oxy )-*N*-(((*S*)-3-((1r,4S)-4-hydroxy-4-methylcyclohexyl)-5-nitro-3,4-di hydro-2H-benzo[b] [1,4]oxazin-7 -yl)sulfonyl)-4-(2-((*R* or *S*)-2-(2-isopropylphenyl)pyrrolid in-1-yl)-7-azaspiro[3.5]nonan-7-yl)benzamide | MS-ESI (m/z): 1017 [M + 1]⁺ |
| | or | | |
| | 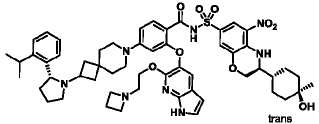 | | |
| D-62 | 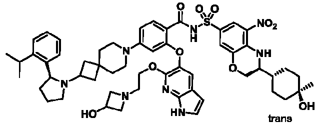 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-2-((6-(2-(3-hydroxy azetidin-1-yl)ethoxy)-1*H*-pyrrolo [2,3-*b*]pyridin-5-yl)oxy)-4-(2-((*R* or *S*)-2-(2-isopropylphenyl)pyrrolid in-1-yl)-7-azaspiro[3.5]nonan-7-yl)benzamide | MS-ESI (m/z): 1033 [M + 1]⁺ |
| | or | | |
| | 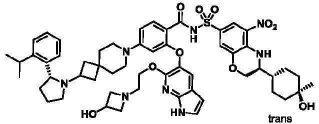 | | |
| D-63 | 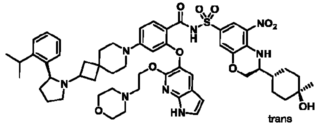 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or S)-2-(2-isopropylphenyl)pyrrolid in-1-yl)-7-azaspiro[3.5]nonan-7-yl)-2-((6-(2-morpholinoethoxy)-1*H*-pyrrolo[2,3-*b*]pyridin-5-yl)ox y)benzamide | MS-ESI (m/z): 1047 [M + 1]⁺ |
| | or | | |
| | 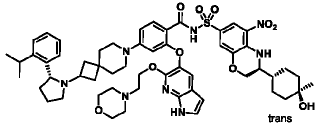 | | |
| D-64 | 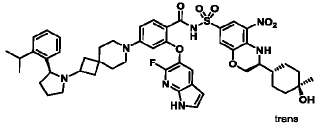 | 2-((6-fluoro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( *R* or *S*)-2-(2-isopropylphenyl)pyrrolid in-1-yl)-7-azaspiro[3.5]nonan-7-yl)benzamide | MS-ESI (m/z): 936 [M + 1]⁺ |
| | or | | |
| | 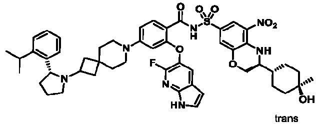 | | |
| D-65 | 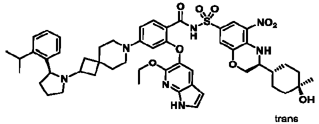 | 2-((6-ethoxy-1*H*-pyrrolo[2,3-*b*]p yridin-5-yl)oxy)-*N*-(((*S*)-3-((1r,4 *S*)-4-hydroxy-4-methylcyclohexy l)-5-nitro-3,4-dihydro-2*H*-benzo[ *b*][1,4]oxazin-7-yl)sulfonyl)-4-(2 -((*R* or *S*)-2-(2-isopropylphenyl)pyrrolid in-1-yl)-7-azaspiro[3.5]nonan-7-yl)benzamide | MS-ESI (m/z): 962 [M + 1]⁺ |
| | or | | |
| | 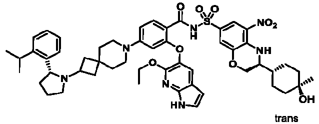 | | |
| D-66 | 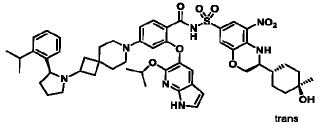 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-2-((6-isopropoxy-1 H-pyrrolo[2,3-*b*]pyridin-5-yl)oxy )-4-(2-((*R* or *S*)-2-(2-isopropylphenyl)pyrrolid in-1-yl)-7-azaspiro[3.5]nonan-7-yl)benzamide | MS-ESI (m/z): 976 [M + 1]⁺ |
| | or | | |
| | 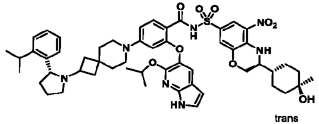 | | |
| D-67 | 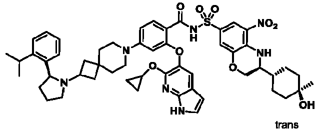 | 2-((6-cyclopropoxy-1*H*-pyrrolo[2 ,3-b]pyridin-5-yl)oxy)-*N*-(((*S*)-3-((1r,4S)-4-hydroxy-4-methylcycl ohexyl)-5-nitro-3,4-dihydro-2*H-*benzo[*b*][1,4]oxazin-7-yl)sulfony l)-4-(2-((*R* or *S*)-2-(2-isopropylphenyl)pyrrolid in-1-yl)-7-azaspiro[3.5]nonan-7-yl)benzamide | MS-ESI (m/z): 974 [M + 1]⁺ |
| | or 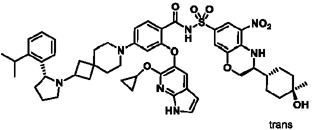 | | |
| D-68 | 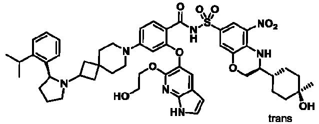 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-2-((6-(2-hydroxyeth oxy)-1*H*-pyrrolo[2,3-*b*]pyridin-5-yl)oxy)-4-(2-((*R* or *S*)-2-(2-isopropylphenyl)pyrrolid in-1-yl)-7-azaspiro[3.5]nonan-7-yl)benzamide | MS-ESI (m/z): 978 [M + 1]⁺ |
| | or 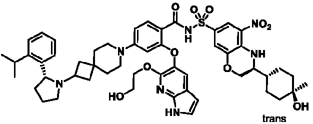 | | |
| D-69 | 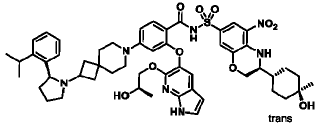 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-2-((6-((*R*)-2-hydrox ypropoxy)-1*H*-pyrrolo[2,3-*b*]pyri din-5-yl)oxy)-4-(2-((*R* or *S*)-2-(2-isopropylphenyl)pyrrolid in-1-yl)-7-azaspiro[3.5]nonan-7-yl)benzamide | MS-ESI (m/z): 992 [M + 1]⁺ |
| | or 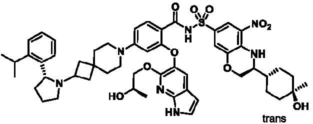 | | |
| D-70 | 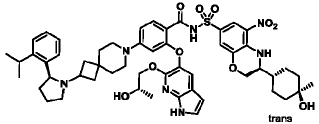 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-2-((6-((*S*)-2-hydrox ypropoxy)-1*H*-pyrrolo[2,3-b]pyri din-5-yl)oxy)-4-(2-((*R* or *S*)-2-(2-isopropylphenyl)pyrrolid in-1-yl)-7-azaspiro[3.5]nonan-7-yl)benzamide | MS-ESI (m/z): 992 [M + 1]⁺ |
| | or 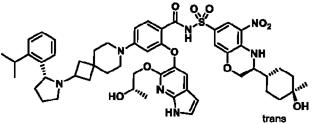 | | |
| D-71 | 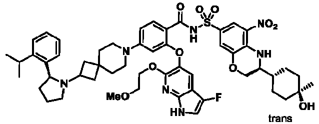 | 2-((3-fluoro-6-(2-methoxyethoxy )-1*H*-pyrrolo[2,3-b]pyridin-5-yl) oxy)-*N*-(((*S*)-3-((1*r*,4*S*)-4-hydrox y-4-methylcyclohexyl)-5-nitro-3, 4-dihydro-2*H*-benzo[*b*][1,4]oxazi n-7-yl)sulfonyl)-4-(2-((*R* or *S*)-2-(2-isopropylphenyl)pyrrolid in-1-yl)-7-azaspiro[3.5]nonan-7-yl)benzamide | MS-ESI (m/z): 1010 [M + 1]⁺ |
| | or 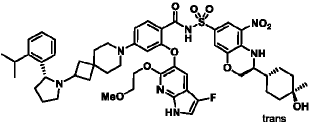 | | |
| D-72 | 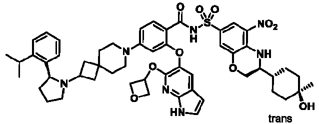 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-(oxetan-3-yloxy)-1*H*-py rrolo[2,3-*b*]pyridin-5-yl)oxy)ben zamide | MS-ESI (m/z): 990 [M + 1]⁺ |
| | or 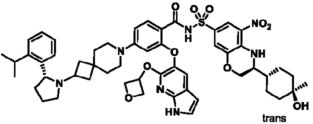 | | |
| D-73 | 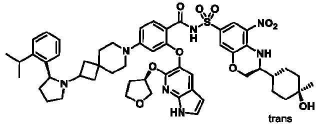 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-(((*R*)-tetrahydrofuran-3-yl)oxy)-1*H*-pyrrolo[2,3-*b*]pyridin -5-yl)oxy)benzamide | MS-ESI (m/z): 992 [M + 1]⁺ |
| | or 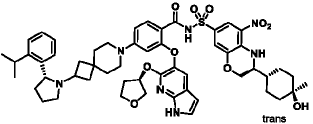 | | |
| D-74 | 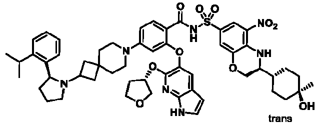 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-(((*S*)-tetrahydrofuran-3-yl)oxy)-1*H*-pyrrolo[2,3-*b*]pyridin -5-yl)oxy)benzamide | MS-ESI (m/z): 1004 [M + 1]⁺ |
| | or 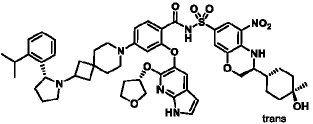 | | |
| D-75a | 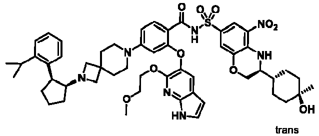 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((1*S*,2*S* and 1*R*,2*R*)-2-(2-isopropylphenyl)cyc lopentyl)-2,7-diazaspiro[3.5]nona n-7-yl)-2-((6-(2-methoxyethoxy)-1*H*-pyrrolo[2,3-*b*]pyridin-5-yl)ox y)benzamide | MS-ESI (m/z): 992 [M + 1]⁺ |
| | and | | |
| | 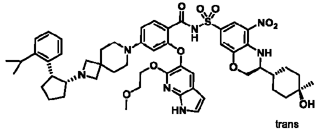 | | |
| D-75b | 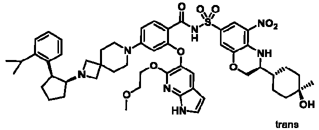 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((1*S*,2*S* or 1*R*,2*R*)-2-(2-isopropylphenyl)cyc lopentyl)-2,7-diazaspiro[3.5]nona n-7-yl)-2-((6-(2-methoxyethoxy)-1*H*-pyrrolo[2,3-*b*]pyridin-5-yl)ox y)benzamide | MS-ESI (m/z): 992 [M + 1]⁺ |
| | or | | |
| | 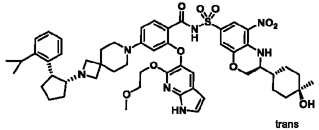 | | |
| D-76 | 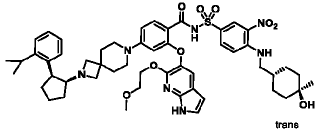 | *N*-((4-((((1*r*,4*r*)-4-hydroxy-4-met hylcyclohexyl)methyl)amino)-3-nitrophenyl)sulfonyl)-4-(2-((1*S*,2 *S* and 1*R*,2*R*)-2-(2-isopropylphenyl)cyc lopentyl)-2,7-diazaspiro[3.5]nona n-7-yl)-2-((6-(2-methoxyethoxy)-1*H*-pyrrolo[2,3-*b*]pyridin-5-yl)ox y)benzamide | MS-ESI (m/z): 964 [M + 1]⁺ |
| | and | | |
| | 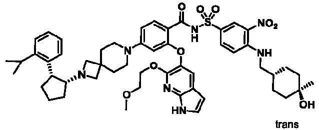 | | |
| D-77 | 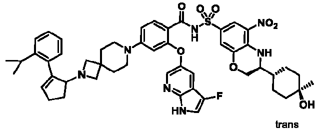 | 2-((3-fluoro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-( 2-(2-isopropylphenyl)cyclopent-2-en-1-yl)-2,7-diazaspiro[3.5]non an-7-yl)benzamide | MS-ESI (m/z): 934 [M + 1]⁺ |
| D-78 | 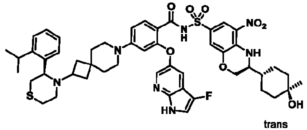 | 2-((3-fluoro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( *R* or *S*)-3-(2-isopropylphenyl)thiomor pholino)-7-azaspiro[3.5]nonan-7-yl)benzamide | MS-ESI (m/z): 968 [M + 1]⁺ |
| | or 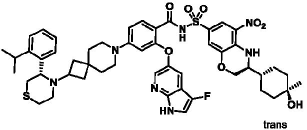 | | |
| D-79 | 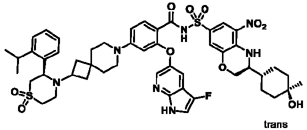 | 2-((3-fluoro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( *R* or *S*)-3-(2-isopropylphenyl)-1,1-dio xidothiomorpholino)-7-azaspiro[ 3.5]nonan-7-yl)benzamide | MS-ESI (m/z): 1000 [M + 1]⁺ |
| | or 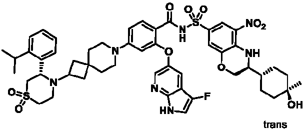 | | |
| D-80 | 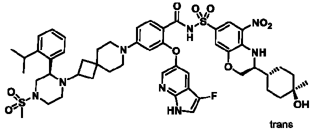 | 2-((3-fluoro-1*H*-pyrrolo[2,3-b]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( *R* or *S*)-2-(2-isopropylphenyl)-4-(met hylsulfonyl)piperazin-1-yl)-7-aza spiro[3.5]nonan-7-yl)benzamide | MS-ESI (m/z): 1029 [M + 1]⁺ |
| | or 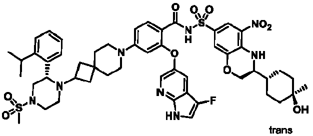 | | |
| D-81 | 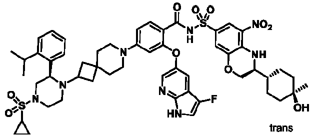 | 4-(2-((*R* or *S*)-4-(cyclopropylsulfonyl)-2-(2-i sopropylphenyl)piperazin-1-yl)-7 -azaspiro[3.5]nonan-7-yl)-2-((3-f luoro-1*H*-pyrrolo[2,3-*b*]pyridin-5 -yl)oxy)-*N*-(((*S*)-3-((1*r*,4*S*)-4-hyd roxy-4-methylcyclohexyl)-5-nitr o-3,4-dihydro-2*H*-benzo[*b*][1,4]o xazin-7-yl)sulfonyl)benzamide | MS-ESI (m/z): 1055 [M + 1]⁺ |
| | or 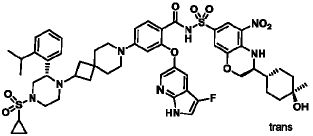 | | |
| D-82 | 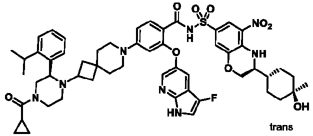 | 4-(2-((*R* or *S*)-4-(cyclopropanecarbonyl)-2-( 2-isopropylphenyl)piperazin-1-yl )-7-azaspiro[3.5]nonan-7-yl)-2-(( 3-fluoro-1*H*-pyrrolo[2,3-*b*]pyridi n-5-yl)oxy)-*N*-(((*S*)-3-((1*r*,4*S*)-4-hydroxy-4-methylcyclohexyl)-5-nitro-3,4-dihydro-2*H*-benzo[*b*][1, 4]oxazin-7-yl)sulfonyl)benzamid e | MS-ESI (m/z): 1019 [M + 1]⁺ |
| | or 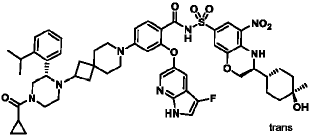 | | |
| D-83 | 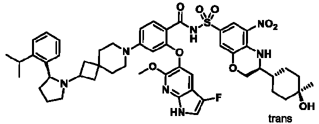 | 2-((3-fluoro-6-methoxy-1*H*-pyrro lo[2,3-*b*]pyridin-5-yl)oxy)-*N*-(((*S* )-3-((1*r*,4*S*)-4-hydroxy-4-methyl cyclohexyl)-5-nitro-3,4-dihydro-2*H*-benzo[*b*][1,4]oxazin-7-yl)sulf onyl)-4-(2-((*R* or *S*)-2-(2-isopropylphenyl)pyrrolid in-1-yl)-7-azaspiro[3.5]nonan-7-yl)benzamide | MS-ESI (m/z): 966 [M + 1]⁺ |
| | or 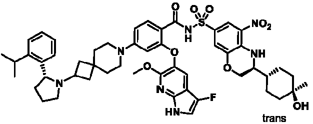 | | |
| D-84 | 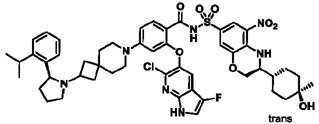 | 2-((6-chloro-3-fluoro-1*H*-pyrrolo [2,3-b]pyridin-5-yl)oxy)-*N*-(((*S*)-3-((1*r*,4*S*)-4-hydroxy-4-methylcy clohexyl)-5-nitro-3,4-dihydro-2*H* -benzo[*b*][1,4]oxazin-7-yl)sulfon yl)-4-(2-((*R* or *S*)-2-(2-isopropylphenyl)pyrrolid in-1-yl)-7-azaspiro[3.5]nonan-7-yl)benzamide | MS-ESI (m/z): 970 [M + 1]⁺ |
| | or 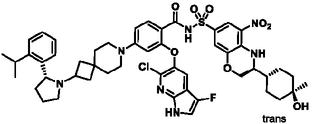 | | |
| D-85 | 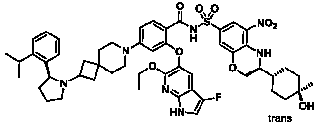 | 2-((6-ethoxy-3-fluoro-1*H*-pyrrolo [2,3-b]pyridin-5-yl)oxy)-*N*-(((*S*)-3-((1r,4S)-4-hydroxy-4-methylcy clohexyl)-5-nitro-3,4-dihydro-2*H* -benzo[*b*][1,4]oxazin-7-yl)sulfon yl)-4-(2-((*R* or *S*)-2-(2-isopropylphenyl)pyrrolid in-1-yl)-7-azaspiro[3.5]nonan-7-yl)benzamide | MS-ESI (m/z): 980 [M + 1]⁺ |
| | or 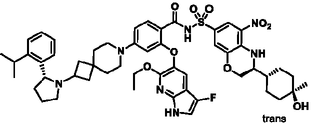 | | |
| D-86 | 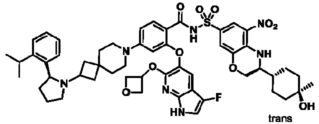 | 2-((3-fluoro-6-(oxetan-3-yloxy)-1*H*-pyrrolo[2,3-*b*]pyridin-5-yl)ox y)-N-(((S)-3-((1*r*,4*S*)-4-hydroxy-4-methylcyclohexyl)-5-nitro-3,4-dihydro-2*H*-benzo[*b*][1,4]oxazin-7-yl)sulfonyl)-4-(2-((*R* or *S*)-2-(2-isopropylphenyl)pyrrolid in-1-yl)-7-azaspiro[3.5]nonan-7-yl)benzamide | MS-ESI (m/z): 1008 [M + 1]⁺ |
| | or 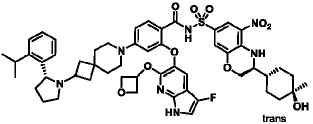 | | |
| D-87 | 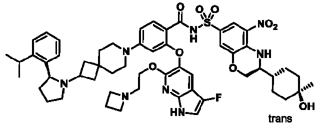 | 2-((6-(2-(azetidin-1-yl)ethoxy)-3-fluoro-1*H*-pyrrolo[2,3-*b*]pyridin-5-yl)oxy)-*N*-(((*S*)-3-((1*r*,4*S*)-4-hy droxy-4-methylcyclohexyl)-5-nit ro-3,4-dihydro-2*H*-benzo[*b*][1,4] oxazin-7-yl)sulfonyl)-4-(2-((*R* or *S*)-2-(2-isopropylphenyl)pyrrolid in-1-yl)-7-azaspiro[3.5]nonan-7-yl)benzamide | MS-ESI (m/z): 1035 [M + 1]⁺ |
| | or 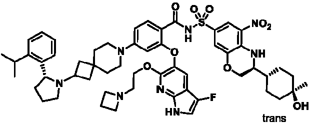 | | |
| D-88 | 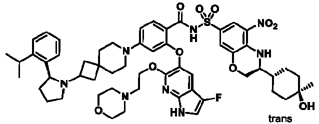 | 2-((3-fluoro-6-(2-morpholinoeth oxy)-1*H*-pyrrolo[2,3-b]pyridin-5-yl)oxy)-*N*-(((*S*)-3-((1*r*,4*S*)-4-hydr oxy-4-methylcyclohexyl)-5-nitro -3,4-dihydro-2*H*-benzo[*b*][1,4]ox azin-7-yl)sulfonyl)-4-(2-((*R* or *S*)-2-(2-isopropylphenyl)pyrrolid in-1-yl)-7-azaspiro[3.5]nonan-7-yl)benzamide | MS-ESI (m/z): 1065 [M + 1]⁺ |
| | or 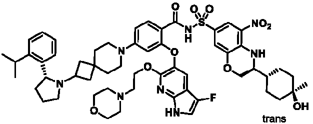 | | |
| D-89 | 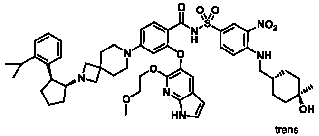 | *N*-((4-((((1*r*,4*r*)-4-hydroxy-4-met hylcyclohexyl)methyl)amino)-3-nitrophenyl)sulfonyl)-4-(2-((1*S*,2 *S* or 1*R*,2*R*)-2-(2-isopropylphenyl)cyc lopentyl)-2,7-diazaspiro[3.5]nona n-7-yl)-2-((6-(2-methoxyethoxy)-1*H*-pyrrolo[2,3-*b*]pyridin-5-yl)ox y)benzamide | MS-ESI (m/z): 964 [M + 1]⁺ |
| | or | | |
| | 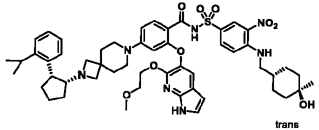 | | |
| D-90 | 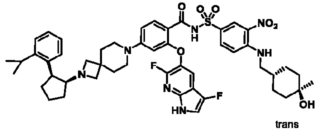 | 2-((3,6-difluoro-1*H*-pyrrolo[2,3-*b*]pyridin-5-yl)oxy)-*N*-((4-((((1*r*, 4*r*)-4-hydroxy-4-methylcyclohex yl)methyl)amino)-3-nitrophenyl) sulfonyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 926 [M + 1]⁺ |
| | or 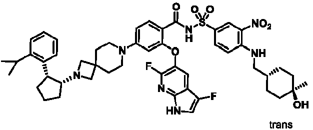 | | |
| D-91 | 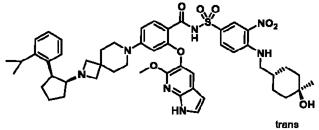 | *N*-((4-((((1*r*,4*r*)-4-hydroxy-4-met hylcyclohexyl)methyl)amino)-3-nitrophenyl)sulfonyl)-4-(2-((1*S*,2 *S* or 1*R*,2*R*)-2-(2-isopropylphenyl)cyc lopentyl)-2,7-diazaspiro[3.5]nona n-7-yl)-2-((6-methoxy-1*H*-pyrrol o[2,3-*b*]pyridin-5-yl)oxy)benzam ide | MS-ESI (m/z): 920 [M + 1]⁺ |
| | or 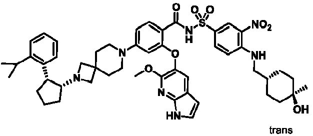 | | |
| D-92 | 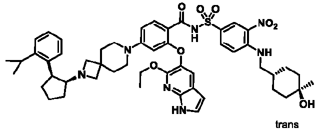 | 2-((6-ethoxy-1*H*-pyrrolo[2,3-*b*] pyridin-5-yl)oxy)-*N*-((4-((((1r,4r)-4-hydroxy-4-methylcyclohexyl) methyl) amino)-3 -nitrophenyl) sul fonyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 934 [M + 1]⁺ |
| | or 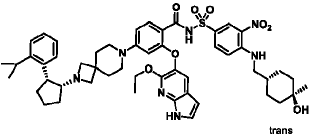 | | |
| D-93 | 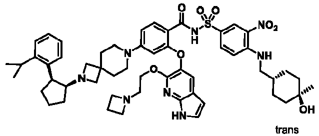 | 2-((6-(2-(azetidin-1-yl)ethoxy)-1 *H*-pyrrolo[2,3-*b*]pyridin-5-yl)oxy )-*N*-((4-((((1*r*,4*r*)-4-hydroxy-4-methylcyclohexyl)methyl)amino)-3 -nitrophenyl)sulfonyl)-4-(2-((1*S*, 2*S* or 1*R*,2*R*)-2-(2-isopropylphenyl)cyc lopentyl)-2,7-diazaspiro[3.5]nona n-7-yl)benzamide | MS-ESI (m/z): 989 [M + 1]⁺ |
| | or 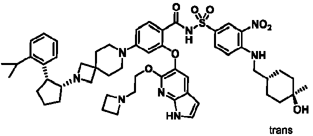 | | |
| D-94 | 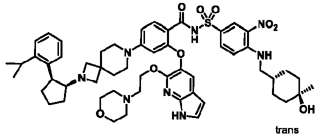 | *N*-((4-((((1*r*,4*r*)-4-hydroxy-4-met hylcyclohexyl)methyl)amino)-3-nitrophenyl)sulfonyl)-4-(2-((1*S*,2 *S* or 1*R*,2*R*)-2-(2-isopropylphenyl)cyc lopentyl)-2,7-diazaspiro[3.5]nona n-7-yl)-2-((6-(2-morpholinoetho xy)-1*H*-pyrrolo[2,3-*b*]pyridin-5-y l)oxy)benzamide | MS-ESI (m/z): 1019 [M + 1]⁺ |
| | or 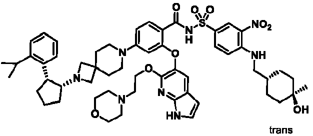 | | |
| D-95 | 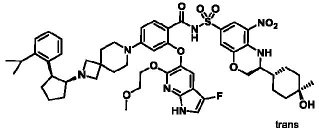 | 2-((3-fluoro-6-(2-methoxyethoxy )-1*H*-pyrrolo[2,3-b]pyridin-5-yl) oxy)-*N*-(((*S*)-3-((1*r*,4*S*)-4-hydrox y-4-methylcyclohexyl)-5-nitro-3, 4-dihydro-2*H*-benzo[*b*][1,4]oxazi n-7-yl)sulfonyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 1010 [M + 1]⁺ |
| | or | | |
| | 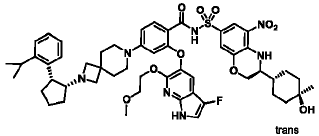 | | |
| D-96 | 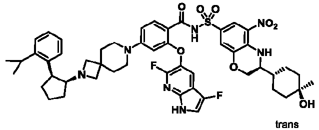 | 2-((3,6-difluoro-1*H*-pyrrolo[2,3-*b*]pyridin-5-yl)oxy)-*N*-(((*S*)-3-((1 *r*,4*S*)-4-hydroxy-4-methylcycloh exyl)-5-nitro-3,4-dihydro-2*H*-ben zo[*b*][1,4]oxazin-7-yl)sulfonyl)-4 -(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 954 [M + 1]⁺ |
| | or 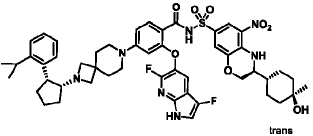 | | |
| D-97 | 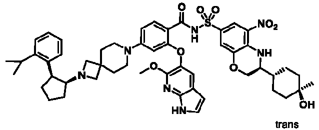 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((1*S*,2*S* or 1*R*,2*R*)-2-(2-isopropylphenyl)cyc lopentyl)-2,7-diazaspiro[3.5]nonan -7-yl)-2-((6-methoxy-1*H*-pyrrol o[2,3-*b*]pyridin-5-yl)oxy)benzam ide | MS-ESI (m/z): 948 [M + 1]⁺ |
| | or 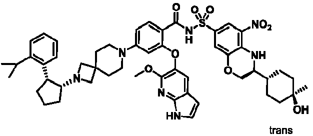 | | |
| D-98 | 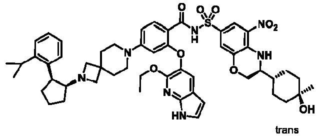 | 2-((6-ethoxy-1*H*-pyrrolo[2,3-*b*]p yridin-5-yl)oxy)-*N*-(((*S*)-3-((1r,4 *S*)-4-hydroxy-4-methylcyclohexy l)-5-nitro-3,4-dihydro-2*H*-benzo[ *b*][1,4]oxazin-7-yl)sulfonyl)-4-(2 -((1*S*,2*S* or 1*R*,2*R*)-2-(2-isopropylphenyl)cyc lopentyl)-2,7-diazaspiro[3.5]nona n-7-yl)benzamide | MS-ESI (m/z): 962 [M + 1]⁺ |
| | or 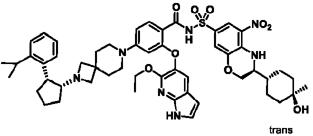 | | |
| D-99 | 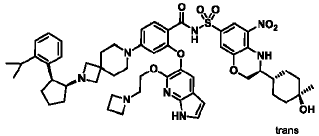 | 2-((6-(2-(azetidin-1-yl)ethoxy)-1 *H*-pyrrolo[2,3-*b*]pyridin-5-yl)oxy )-*N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 1017 [M + 1]⁺ |
| | or 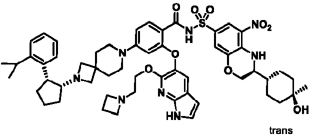 | | |
| D-100 | 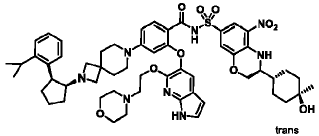 | *N*-(((*S*)-3-((1*r*,4*S*)-4-hydroxy-4-methylcyclohexyl)-5-nitro-3,4-di hydro-2*H*-benzo[b] [1,4]oxazin-7 -yl)sulfonyl)-4-(2-((1*S*,2*S* or 1*R*,2*R*)-2-(2-isopropylphenyl)cyc lopentyl)-2,7-diazaspiro[3.5]nona n-7-yl)-2-((6-(2-morpholinoetho xy)-1*H*-pyrrolo[2,3-*b*]pyridin-5-yl)oxy)benzamide | MS-ESI (m/z): 1047 [M + 1]⁺ |
| | or 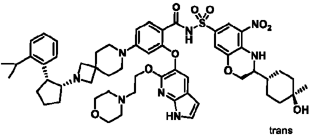 | | |
| D-101 | 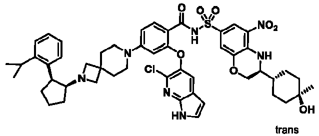 | 2-((6-chloro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( 1*S*,2*S* or 1*R*,2*R*)-2-(2-isopropylphenyl)cyc lopentyl)-2,7-diazaspiro[3.5]nonan -7-yl)benzamide | MS-ESI (m/z): 952 [M + 1]⁺ |
| | or 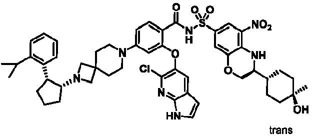 | | |
| D-102 | 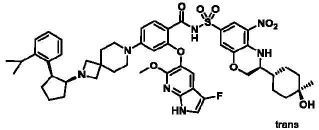 | 2-((3-fluoro-6-methoxy-1*H*-pyrro lo[2,3-*b*]pyridin-5-yl)oxy)-*N*-(((*S* )-3-((1*r*,4*S*)-4-hydroxy-4-methyl cyclohexyl)-5-nitro-3,4-dihydro-2*H*-benzo[*b*][1,4]oxazin-7-yl)sulf onyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 996 [M + 1]⁺ |
| | or 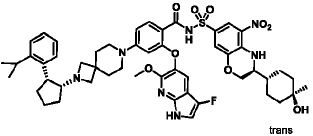 | | |
| D-103 | 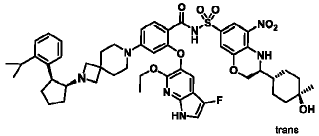 | 2-((6-ethoxy-3-fluoro-1*H*-pyrrolo [2,3-*b*]pyridin-5-yl)oxy)-*N*-(((*S*)-3-((1*r*,4*S*)-4-hydroxy-4-methylcy clohexyl)-5-nitro-3,4-dihydro-2*H* -benzo[b][1,4]oxazin-7-yl)sulfon yl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 980 [M + 1]⁺ |
| | or 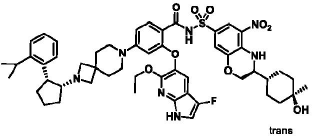 | | |
| D-104 | 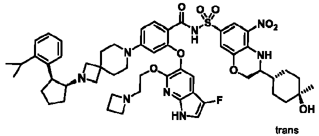 | 2-((6-(2-(azetidin-1-yl)ethoxy)-3-fluoro-1*H*-pyrrolo[2,3-*b*]pyridin-5-yl)oxy)-*N*-(((*S*)-3-((1*r*,4*S*)-4-hy droxy-4-methylcyclohexyl)-5-nit ro-3,4-dihydro-2*H*-benzo[*b*][1,4] oxazin-7-yl)sulfonyl)-4-(2-((1*S*,2 *S* or 1*R*,2*R*)-2-(2-isopropylphenyl)cyc lopentyl)-2,7-diazaspiro[3.5]nonan -7-yl)benzamide | MS-ESI (m/z): 1035 [M + 1]⁺ |
| | or 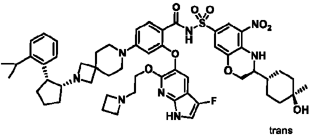 | | |
| D-105 | 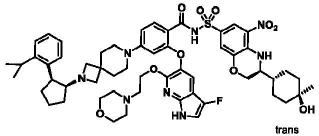 | 2-((3-fluoro-6-(2-morpholinoeth oxy)-1H-pyrrolo[2,3-b]pyridin-5-yl)oxy)-*N*-(((*S*)-3-((1*r*,4*S*)-4-hydr oxy-4-methylcyclohexyl)-5-nitro -3,4-dihydro-2*H*-benzo[*b*][1,4]ox azin-7-yl)sulfonyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 1065 [M + 1]⁺ |
| | or 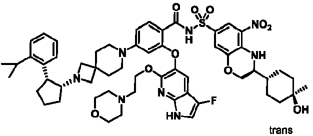 | | |
| D-106 | 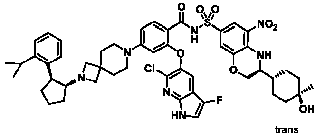 | 2-((6-chloro-3-fluoro-1*H*-pyrrolo [2,3-*b*]pyridin-5-yl)oxy)-*N*-(((*S*)-3-((1*r*,4*S*)-4-hydroxy-4-methylcy clohexyl)-5-nitro-3,4-dihydro-2*H* -benzo[b][1,4]oxazin-7-yl)sulfon yl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 970 [M + 1]⁺ |
| | or 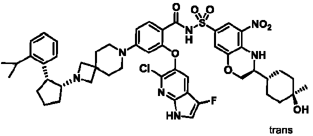 | | |
| D-107 | 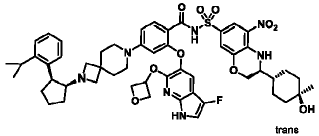 | 2-((3-fluoro-6-(oxetan-3-yloxy)-1*H*-pyrrolo[2,3-*b*]pyridin-5-yl)ox y)-*N*-(((*S*)-3-((1*r*,4*S*)-4-hydroxy-4-methylcyclohexyl)-5-nitro-3,4-dihydro-2*H*-benzo[*b*][1,4]oxazin-7-yl)sulfonyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 1008 [M + 1]⁺ |
| | or 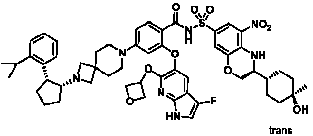 | | |
| D-108 | 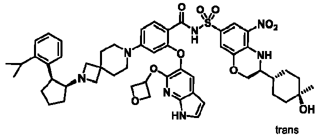 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((1*S*,2*S* or 1*R*,2*R*)-2-(2-isopropylphenyl)cyc lopentyl)-2,7-diazaspiro[3.5]nona n-7-yl)-2-((6-(oxetan-3-yloxy)-1 *H*-pyrrolo[2,3-*b*]pyridin-5-yl)oxy )benzamide | MS-ESI (m/z): 990 [M + 1]⁺ |
| | or 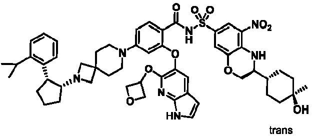 | | |
| D-109 | 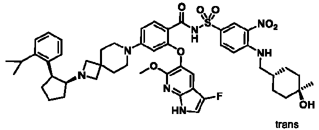 | 2-((3-fluoro-6-methoxy-1*H*-pyrro lo[2,3-*b*]pyridin-5-yl)oxy)-*N*-((4-((((1*r*,4*r*)-4-hydroxy-4-methylcy clohexyl)methyl)amino)-3-nitrop henyl)sulfonyl)-4-(2-((1*S*,2*S* or 1*R*,2*R*)-2-(2-isopropylphenyl)cyc lopentyl)-2,7-diazaspiro[3.5]nonan -7-yl)benzamide | MS-ESI (m/z): 938 [M + 1]⁺ |
| | or 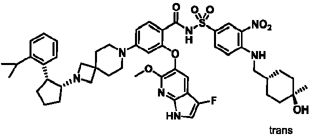 | | |
| D-110 | 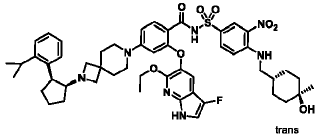 | 2-((6-ethoxy-3-fluoro-1*H*-pyrrolo [2,3-*b*]pyridin-5-yl)oxy)-*N*-((4-(( ((1*r*,4*r*)-4-hydroxy-4-methylcycl ohexyl)methyl)amino)-3-nitroph enyl)sulfonyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 952 [M + 1]⁺ |
| | or 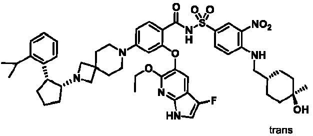 | | |
| D-111 | 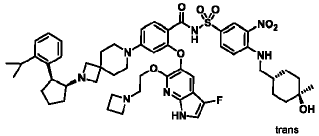 | 2-((6-(2-(azetidin-1-yl)ethoxy)-3-fluoro-1*H*-pyrrolo[2,3-b]pyridin-5-yl)oxy)-*N*-((4-((((1*r*,4*r*)-4-hydr oxy-4-methylcyclohexyl)methyl) amino)-3-nitrophenyl)sulfonyl)-4 -(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 1007 [M + 1]⁺ |
| | or 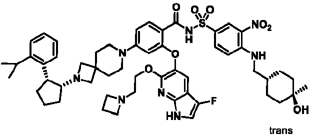 | | |
| D-112 | 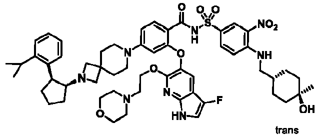 | 2-((3-fluoro-6-(2-morpholinoeth oxy)-1*H*-pyrrolo[2,3-*b*]pyridin-5-yl)oxy)-*N*-((4-((((1*r*,4*r*)-4-hydrox y-4-methylcyclohexyl)methyl)am ino)-3-nitrophenyl)sulfonyl)-4-(2 -((1*S*,2*S* or 1*R*,2*R*)-2-(2-isopropylphenyl)cyc lopentyl)-2,7-diazaspiro[3.5]nonan -7-yl)benzamide | MS-ESI (m/z): 1037 [M + 1]⁺ |
| | or 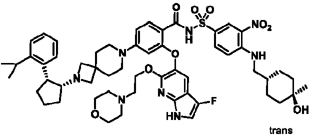 | | |
| D-113 | 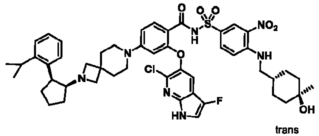 | 2-((6-chloro-3-fluoro-1*H*-pyrrolo [2,3-*b*]pyridin-5-yl)oxy)-*N*-((4-(( ((1*r*,4*r*)-4-hydroxy-4-methylcycl ohexyl)methyl)amino)-3-nitroph enyl)sulfonyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 942 [M + 1]⁺ |
| | or 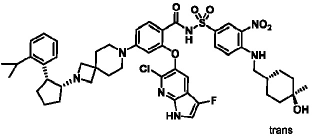 | | |
| D-114 | 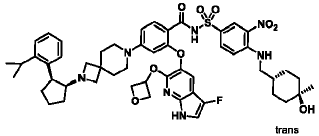 | 2-((3-fluoro-6-(oxetan-3-yloxy)-1*H*-pyrrolo[2,3-b]pyridin-5-yl)ox y)-*N*-((4-((((1*r*,4*r*)-4-hydroxy-4-methylcyclohexyl)methyl)amino) -3-nitrophenyl)sulfonyl)-4-(2-((1 *S*,2*S* or 1*R*,2*R*)-2-(2-isopropylphenyl)cyc lopentyl)-2,7-diazaspiro[3.5]nona n-7-yl)benzamide | MS-ESI (m/z): 980 [M + 1]⁺ |
| | or 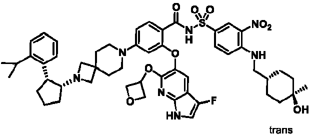 | | |
| D-115 | 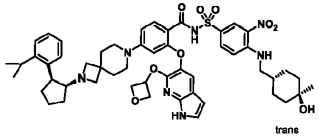 | *N*-((4-((((1*r*,4*r*)-4-hydroxy-4-met hylcyclohexyl)methyl)amino)-3-nitrophenyl)sulfonyl)-4-(2-((1*S*,2 *S* or 1*R*,2*R*)-2-(2-isopropylphenyl)cyc lopentyl)-2,7-diazaspiro[3.5]nona n-7-yl)-2-((6-(oxetan-3-yloxy)-1 *H*-pyrrolo[2,3-*b*]pyridin-5-yl)oxy )benzamide | MS-ESI (m/z): 962 [M + 1]⁺ |
| | or 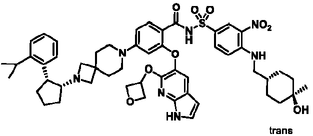 | | |
| D-116 | 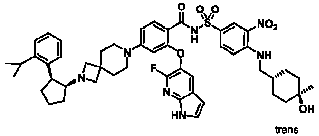 | 2-((6-fluoro-1*H*-pyrrolo[2,3-b]py ridin-5-yl)oxy)-*N*-((4-((((1*r*,4*r*)-4 -hydroxy-4-methylcyclohexyl)m ethyl)amino)-3-nitrophenyl)sulfo nyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 908 [M + 1]⁺ |
| | or 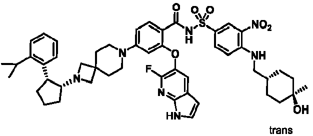 | | |
| D-117 | 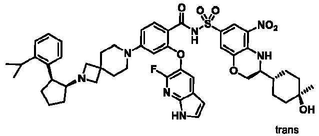 | 2-((6-fluoro-1*H*-pyrrolo[2,3-*b*]py ridin-5-yl)oxy)-*N*-(((*S*)-3-((1r,4*S*) -4-hydroxy-4-methylcyclohexyl)-5-nitro-3,4-dihydro-2*H*-benzo[*b*] [1,4]oxazin-7-yl)sulfonyl)-4-(2-(( 1*S*,2*S* or 1*R*,2*R*)-2-(2-isopropylphenyl)cyc lopentyl)-2,7-diazaspiro[3.5]nona n-7-yl)benzamide | MS-ESI (m/z): 936 [M + 1]⁺ |
| | or 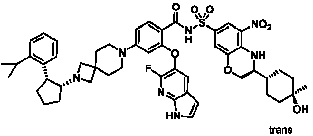 | | |
| D-118 | 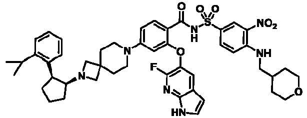 | 2-((6-fluoro-1*H*-pyrrolo[2,3-*b*]py ridin-5-yl)oxy)-4-(2-((1*S*,2*S* or 1*R*,2*R*)-2-(2-isopropylphenyl)cyc lopentyl)-2,7-diazaspiro[3.5]nona n-7-yl)-*N*-((3-nitro-4-(((tetrahydr o-2*H*-pyran-4-yl)methyl)amino)p henyl)sulfonyl)benzamide | MS-ESI (m/z): 880 [M + 1]⁺ |
| | or 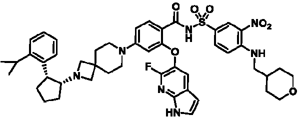 | | |
| D-119 | 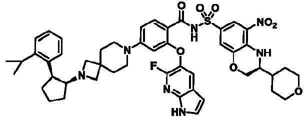 | 2-((6-fluoro-1*H*-pyrrolo[2,3-*b*]py ridin-5-yl)oxy)-4-(2-((1*S*,2*S* or 1*R*,2*R*)-2-(2-isopropylphenyl)cyc lopentyl)-2,7-diazaspiro[3.5]nona n-7-yl)-*N*-(((*S*)-5-nitro-3-(tetrahy dro-2*H*-pyran-4-yl)-3,4-dihydro-2*H*-benzo[*b*][1,4]oxazin-7-yl)sulf onyl)benzamide | MS-ESI (m/z): 908 [M + 1]⁺ |
| | or 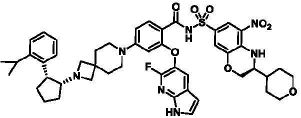 | | |
| D-120 | 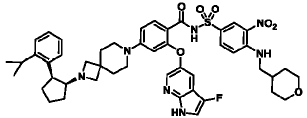 | 2-((3-fluoro-1*H*-pyrrolo[2,3-b]py ridin-5-yl)oxy)-4-(2-((1*S*,2*S* or 1*R*,2*R*)-2-(2-isopropylphenyl)cyc lopentyl)-2,7-diazaspiro[3.5]nona n-7-yl)-*N*-((3-nitro-4-(((tetrahydr o-2*H*-pyran-4-yl)methyl)amino)p henyl)sulfonyl)benzamide | MS-ESI (m/z): 880 [M + 1]⁺ |
| | or 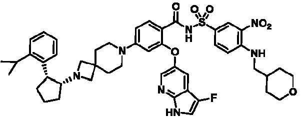 | | |
| D-121 | 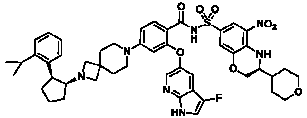 | 2-((3-fluoro-1*H*-pyrrolo[2,3-*b*]py ridin-5-yl)oxy)-4-(2-((1*S*,2*S* or 1*R*,2*R*)-2-(2-isopropylphenyl)cyc lopentyl)-2,7-diazaspiro[3.5]nona n-7-yl)-*N*-(((*S*)-5-nitro-3-(tetrahy dro-2*H*-pyran-4-yl)-3,4-dihydro-2*H*-benzo[*b*][1,4]oxazin-7-yl)sulf onyl)benzamide | MS-ESI (m/z): 908 [M + 1]⁺ |
| | or 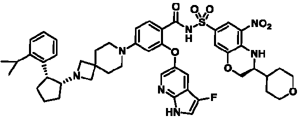 | | |
| D-122 | 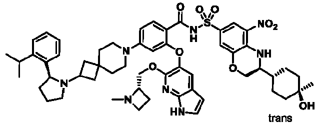 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-(((*S*)-1-methylazetidin-2-yl)methoxy)-1*H*-pyrrolo[2,3-*b*] pyridin-5-yl)oxy)benzamide | MS-ESI (m/z): 1017 [M + 1]⁺ |
| | or 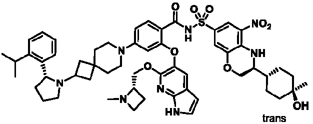 | | |
| D-123 | 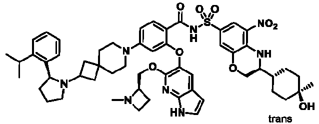 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-(((*R*)-1-methylazetidin-2-yl)methoxy)-1*H*-pyrrolo[2,3-*b*] pyridin-5-yl)oxy)benzamide | MS-ESI (m/z): 1017 [M + 1]⁺ |
| | or 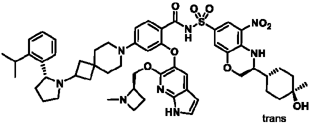 | | |
| D-124 | 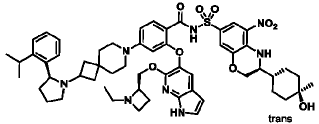 | 2-((6-(((*R*)-1-ethylazetidin-2-yl) methoxy)-1*H*-pyrrolo[2,3-*b*]pyri din-5-yl)oxy)-*N*-(((*S*)-3-((1*r*,4*S*)-4-hydroxy-4-methylcyclohexyl)-5-nitro-3,4-dihydro-2*H*-benzo[b] [1,4]oxazin-7-yl)sulfonyl)-4-(2-(( *R* or *S*)-2-(2-isopropylphenyl)pyrrolid in-1-yl)-7-azaspiro[3.5]nonan-7-yl)benzamide | MS-ESI (m/z): 1031 [M + 1]⁺ |
| | or 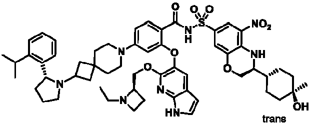 | | |
| D-125 | 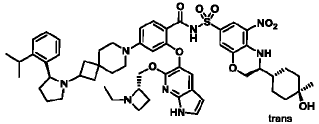 | 2-((6-(((*S*)-1-ethylazetidin-2-yl) methoxy)-1*H*-pyrrolo[2,3-b]pyri din-5-yl)oxy)-*N*-(((*S*)-3-((1*r*,4*S*)-4-hydroxy-4-methylcyclohexyl)-5-nitro-3,4-dihydro-2*H*-benzo[*b*] [1,4]oxazin-7-yl)sulfonyl)-4-(2-(( *R* or *S*)-2-(2-isopropylphenyl)pyrrolid in-1-yl)-7-azaspiro[3.5]nonan-7-yl)benzamide | MS-ESI (m/z): 1031 [M + 1]⁺ |
| | or 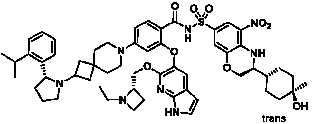 | | |
| D-126 | 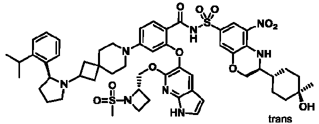 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-(((*S*)-1-(methylsulfonyl) azetidin-2-yl)methoxy)-1*H*-pyrro lo[2,3-*b*]pyridin-5-yl)oxy)benza mide | MS-ESI (m/z): 1081 [M + 1]⁺ |
| | or 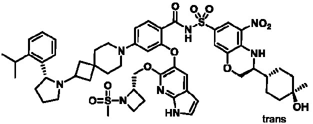 | | |
| D-127 | 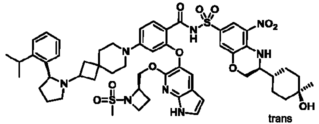 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-(((*R*)-1-(methylsulfonyl )azetidin-2-yl)methoxy)-1*H*-pyrr olo[2,3-*b*]pyridin-5-yl)oxy)benza mide | MS-ESI (m/z): 1081 [M + 1]⁺ |
| | or 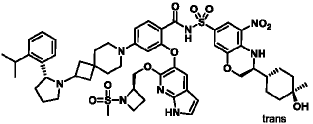 | | |
| D-128 | 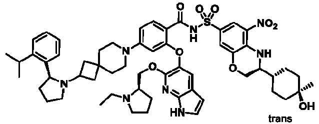 | 2-((6-(((*R*)-1-ethylpyrrolidin-2-yl )methoxy)-1*H*-pyrrolo[2,3-*b*]pyri din-5-yl)oxy)-*N*-(((*S*)-3-((1*r*,4*S*)-4-hydroxy-4-methylcyclohexyl)-5-nitro-3,4-dihydro-2*H*-benzo[*b*] [1,4]oxazin-7-yl)sulfonyl)-4-(2-(( *R* or *S*)-2-(2-isopropylphenyl)pyrrolid in-1-yl)-7-azaspiro[3.5]nonan-7-yl)benzamide | MS-ESI (m/z): 1045 [M + 1]⁺ |
| | or 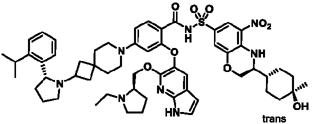 | | |
| D-129 | 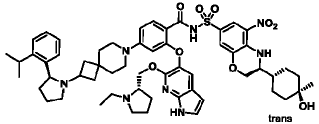 | 2-((6-(((*S*)-1-ethylpyrrolidin-2-yl )methoxy)-1*H*-pyrrolo[2,3-b]pyri din-5-yl)oxy)-*N*-(((*S*)-3-((1*r*,4*S*)-4-hydroxy-4-methylcyclohexyl)-5-nitro-3,4-dihydro-2*H*-benzo[*b*] [1,4]oxazin-7-yl)sulfonyl)-4-(2-(( *R* or *S*)-2-(2-isopropylphenyl)pyrrolid in-1-yl)-7-azaspiro[3.5]nonan-7-yl)benzamide | MS-ESI (m/z): 1045 [M + 1]⁺ |
| | or 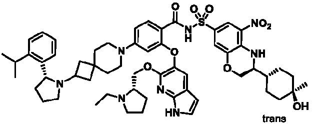 | | |
| D-130 | 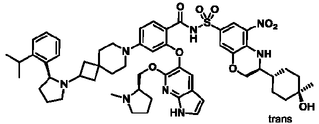 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-(((*R*)-1-methylpyrrolidi n-2-yl)methoxy)-1*H*-pyrrolo[2,3-*b*]pyridin-5-yl)oxy)benzamide | MS-ESI (m/z): 1031 [M + 1]⁺ |
| | or 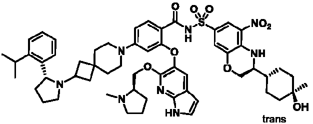 | | |
| D-131 | 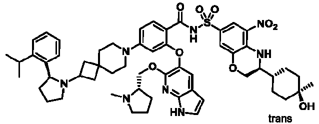 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-(((*S*)-1-methylpyrrolidi n-2-yl)methoxy)-1*H-*pyrrolo[2,3-*b*]pyridin-5-yl)oxy)benzamide | MS-ESI (m/z): 1031 [M + 1]⁺ |
| | or 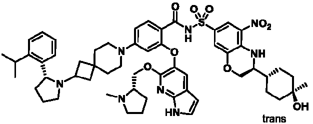 | | |
| D-132 | 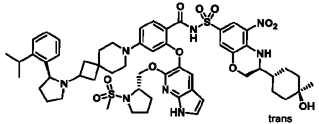 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or S)-2-(2-isopropylphenyl)pyrrolid in-1-yl)-7-azaspiro[3.5]nonan-7-yl)-2-((6-(((*S*)-1-(methylsulfonyl) pyrrolidin-2-yl)methoxy)-1*H*-pyr rolo[2,3-*b*]pyridin-5-yl)oxy)benz amide | MS-ESI (m/z): 1095 [M + 1]⁺ |
| | or 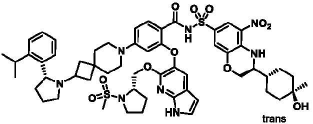 | | |
| D-133 | 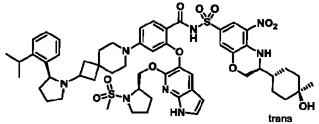 | *N*-(((*S*)-3-((1r,4*S*)-4-hydroxy-4-methylcyclohexyl)-5-nitro-3,4-di hydro-2*H*-benzo[*b*][1,4]oxazin-7 -yl)sulfonyl)-4-(2-((*R* or S)-2-(2-isopropylphenyl)pyrrolid in-1-yl)-7-azaspiro[3.5]nonan-7-yl)-2-((6-(((*R*)-1-(methylsulfonyl )pyrrolidin-2-yl)methoxy)-1*H*-py rrolo[2,3-*b*]pyridin-5-yl)oxy)ben zamide | MS-ESI (m/z): 1095 [M + 1]⁺ |
| | or 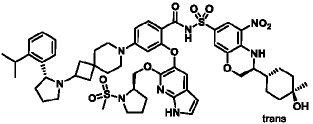 | | |
| D-134 | 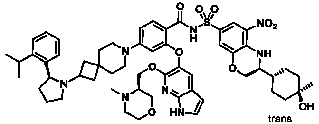 | *N*-(((*S*)-3-((1r,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-(((*R*)-4-methylmorpholi n-3-yl)methoxy)-1*H*-pyrrolo[2,3-*b*]pyridin-5-yl)oxy)benzamide | MS-ESI (m/z): 1047 [M + 1]⁺ |
| | or 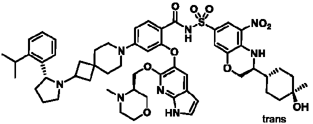 | | |
| D-135 | 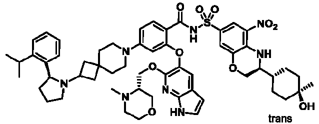 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-(((*S*)-4-methylmorpholi n-3-yl)methoxy)-1*H*-pyrrolo[2,3-*b*]pyridin-5-yl)oxy)benzamide | MS-ESI (m/z): 1047 [M + 1]⁺ |
| | or 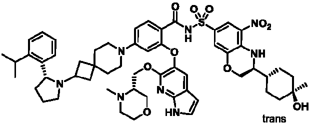 | | |
| D-136 | 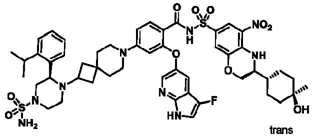 | 2-((3-fluoro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( *R* or *S*)-2-(2-isopropylphenyl)-4-sulfa moylpiperazin-1-yl)-7-azaspiro[3 .5]nonan-7-yl)benzamide | MS-ESI (m/z): 1030 [M + 1]⁺ |
| | or | | |
| | 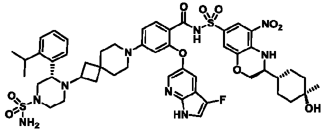 | | |
| D-137 | 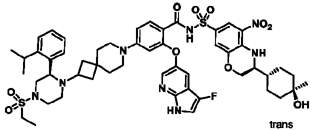 | 4-(2-((*R* or *S*)-4-(ethylsulfonyl)-2-(2-isoprop ylphenyl)piperazin-1-yl)-7-azaspi ro[3.5]nonan-7-yl)-2-((3-fluoro-1 *H*-pyrrolo[2,3-*b*]pyridin-5-yl)oxy )-*N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)benzamide | MS-ESI (m/z): 1043 [M + 1]⁺ |
| | or | | |
| | 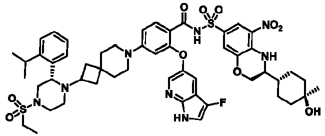 | | |
| D-138 | 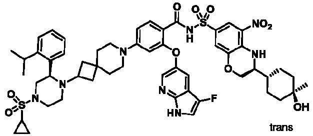 | 4-(2-((*R* or *S*)-4-(cyclopropylsulfonyl)-2-(2-i sopropylphenyl)piperazin-1-yl)-7 -azaspiro[3.5]nonan-7-yl)-2-((3-f luoro-1*H*-pyrrolo[2,3-b]pyridin-5 -yl)oxy)-*N*-(((*S*)-3-((1*r*,4*S*)-4-hyd roxy-4-methylcyclohexyl)-5-nitr o-3,4-dihydro-2*H*-benzo[*b*][1,4]o xazin-7-yl)sulfonyl)benzamide | MS-ESI (m/z): 1055 [M + 1]⁺ |
| | or | | |
| | 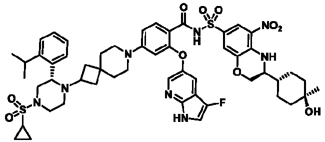 | | |
| D-139 | 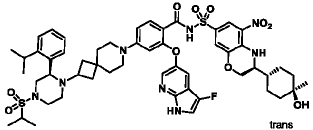 | 2-((3-fluoro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( *R* or *S*)-2-(2-isopropylphenyl)-4-(isop ropylsulfonyl)piperazin-1-yl)-7-azaspiro[3.5]nonan-7-yl)benzamide | MS-ESI (m/z): 1057 [M + 1]⁺ |
| | or 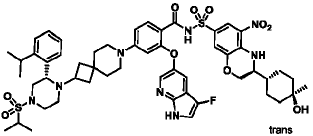 | | |
| D-140 | 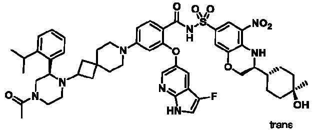 | 4-(2-((*R* or *S*)-4-acetyl-2-(2-isopropylphenyl )piperazin-1-yl)-7-azaspiro[3.5]n onan-7-yl)-2-((3-fluoro-1*H*-pyrro lo[2,3-b]pyridin-5-yl)oxy)-*N*-(((*S* )-3-((1*r*,4*S*)-4-hydroxy-4-methyl cyclohexyl)-5-nitro-3,4-dihydro-2*H*-benzo[*b*][1,4]oxazin-7-yl)sulf onyl)benzamide | MS-ESI (m/z): 993 [M + 1]⁺ |
| | or 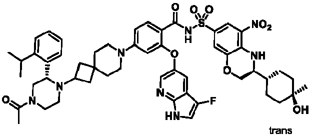 | | |
| D-141 | 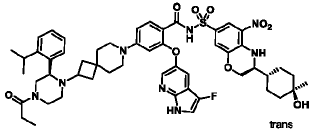 | 2-((3-fluoro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( *R* or *S*)-2-(2-isopropylphenyl)-4-propi onylpiperazin-1-yl)-7-azaspiro[3. 5]nonan-7-yl)benzamide | MS-ESI (m/z): 1007 [M + 1]⁺ |
| | or 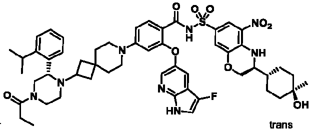 | | |
| D-142 | 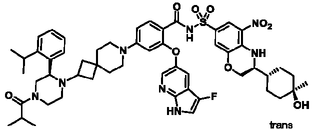 | 2-((3-fluoro-1*H*-pyrrolo[2,3-b]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( *R* or *S*)-4-isobutyryl-2-(2-isopropylph enyl)piperazin-1-yl)-7-azaspiro[3 .5]nonan-7-yl)benzamide | MS-ESI (m/z): 1021 [M + 1]⁺ |
| | or 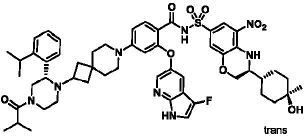 | | |
| D-143 | 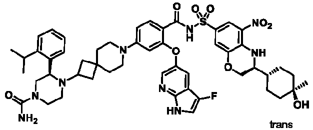 | (*R* or *S*)-4-(7-(3-((3-fluoro-1*H*-pyrrolo[ 2,3-b]pyridin-5-yl)oxy)-4-((((*S*)-3-((1*r*,4*S*)-4-hydroxy-4-methylcy clohexyl)-5-nitro-3,4-dihydro-2*H* -benzo[*b*][1,4]oxazin-7-yl)sulfon yl)carbamoyl)phenyl)-7-azaspiro [3.5]nonan-2-yl)-3-(2-isopropylp henyl)piperazine-1-carboxamide | MS-ESI (m/z): 994 [M + 1]⁺ |
| | or 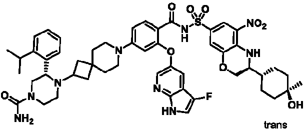 | | |
| D-144 | 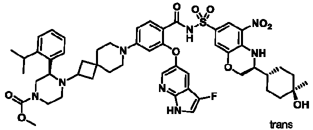 | methyl (*R* or *S*)-4-(7-(3-((3-fluoro-1*H*-pyrrolo] 2,3-*b*]pyridin-5-yl)oxy)-4-((((*S*)-3-((1*r*,4*S*)-4-hydroxy-4-methylcy clohexyl)-5-nitro-3,4-dihydro-2*H* -benzo[*b*][1,4]oxazin-7-yl)sulfon yl)carbamoyl)phenyl)-7-azaspiro [3.5]nonan-2-yl)-3-(2-isopropylp henyl)piperazine-1-carboxylate | MS-ESI (m/z): 1009 [M + 1]⁺ |
| | or 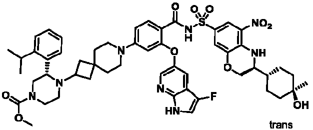 | | |
| D-145 | 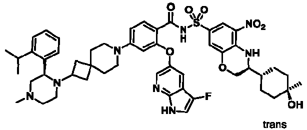 | 2-((3-fluoro-1*H*-pyrrolo[2,3-b]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( *R* or *S*)-2-(2-isopropylphenyl)-4-meth ylpiperazin-1-yl)-7-azaspiro[3.5] nonan-7-yl)benzamide | MS-ESI (m/z): 965 [M + 1]⁺ |
| | of 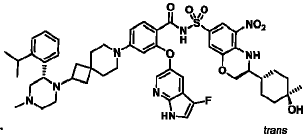 | | |
| D-146a | 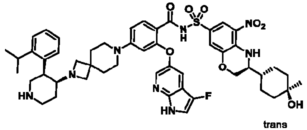 | 2-((3-fluoro-1*H*-pyrrolo[2,3-b]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( 3*R*,4*S* and 3*S*,4*R*)-3-(2-isopropylphenyl)pip eridin-4-yl)-2,7-diazaspiro[3.5]n onan-7-yl)benzamide | MS-ESI (m/z): 951 [M + 1]⁺ |
| | and | | |
| | 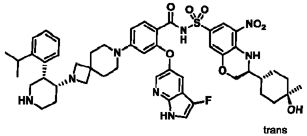 | | |
| D-146b | 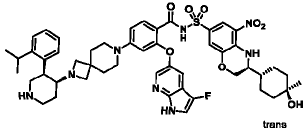 | 2-((3-fluoro-1*H*-pyrrolo[2,3-b]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( 3*R*,4*S* or 3*S*,4*R*)-3-(2-isopropylphenyl)pip eridin-4-yl)-2,7-diazaspiro[3.5]n onan-7-yl)benzamide | MS-ESI (m/z): 951 [M + 1]⁺ |
| | or 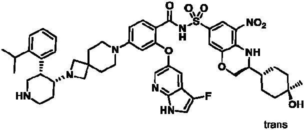 | | |
| D-147 | 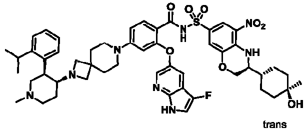 | 2-((3-fluoro-1*H*-pyrrolo[2,3-b]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( 3*R*,4*S* or 3*S*,4*R*)-3-(2-isopropylphenyl)-1-methylpiperidin-4-yl)-2,7-diazas piro[3.5]nonan-7-yl)benzamide | MS-ESI (m/z): 965 [M + 1]⁺ |
| | or 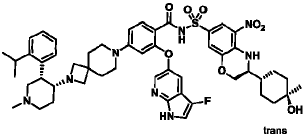 | | |
| D-148 | 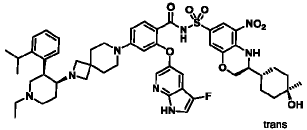 | 4-(2-((3*R*,4*S* or 3*S*,4*R*)-1-ethyl-3-(2-isopropylphe nyl)piperidin-4-yl)-2,7-diazaspir o[3.5]nonan-7-yl)-2-((3-fluoro-1 *H*-pyrrolo[2,3-*b*]pyridin-5-yl)oxy )-*N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)benzamide | MS-ESI (m/z): 979 [M + 1]⁺ |
| | or 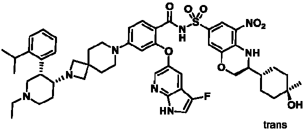 | | |
| D-149 | 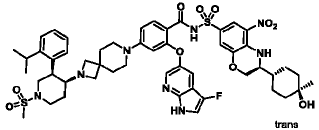 | 2-((3-fluoro-1*H*-pyrrolo[2,3-*b*]py ridin-5-yl)oxy)-*N*-(((*S*)-3-((1r,4S) -4-hydroxy-4-methylcyclohexyl)-5-nitro-3,4-dihydro-2*H*-benzo[*b*] [1,4]oxazin-7-yl)sulfonyl)-4-(2-(( 3*R*,4*S* or 3*S*,4*R*)-3-(2-isopropylphenyl)-1-( methylsulfonyl)piperidin-4-yl)-2, 7-diazaspiro[3.5]nonan-7-yl)benz amide | MS-ESI (m/z): 1029 [M + 1]⁺ |
| | or 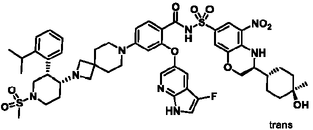 | | |
| D-150 | 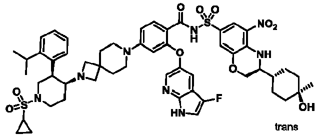 | 4-(2-((3*R*,4*S* or 3*S*,4*R*)-1-(cyclopropylsulfonyl)-3 -(2-isopropylphenyl)piperidin-4-yl)-2,7-diazaspiro[3.5]nonan-7-yl )-2-((3-fluoro-1*H*-pyrrolo[2,3-*b*] pyridin-5-yl)oxy)-*N*-(((*S*)-3-((1*r*, 4S)-4-hydroxy-4-methylcyclohex yl)-5-nitro-3,4-dihydro-2*H*-benzo [*b*][1,4]oxazin-7-yl)sulfonyl)ben zamide | MS-ESI (m/z): 1055 [M + 1]⁺ |
| | or 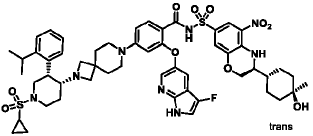 | | |
| D-151 | 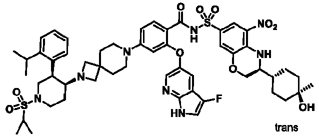 | 2-((3-fluoro-1*H*-pyrrolo[2,3-b]py ridin-5-yl)oxy)-*N*-(((*S*)-3-((1*r*,4*S*) -4-hydroxy-4-methylcyclohexyl)-5-nitro-3,4-dihydro-2*H*-benzo[b] [1,4]oxazin-7-yl)sulfonyl)-4-(2-(( 3*R*,4*S* or 3*S*,4*R*)-3-(2-isopropylphenyl)-1-( isopropylsulfonyl)piperidin-4-yl) -2,7-diazaspiro[3.5]nonan-7-yl) benzamide | MS-ESI (m/z): 1057 [M + 1]⁺ |
| | or 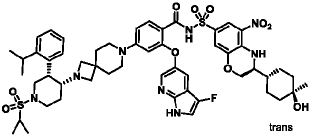 | | |
| D-152 | 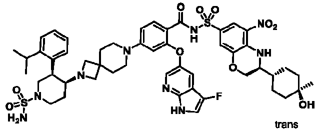 | 2-((3-fluoro-1*H*-pyrrolo[2,3-b]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( 3*R*,4*S* or 3*S*,4*R*)-3-(2-isopropylphenyl)-1-sulfamoylpiperidin-4-yl)-2,7-diaz aspiro[3.5]nonan-7-yl)benzamide | MS-ESI (m/z): 1030 [M + 1]⁺ |
| | or 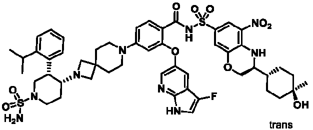 | | |
| D-153 | 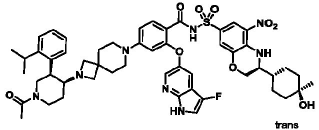 | 4-(2-((3*R*,4*S* or 3*S*,4*R*)-1-acetyl-3-(2-isopropylph enyl)piperidin-4-yl)-2,7-diazaspir o[3.5]nonan-7-yl)-2-((3-fluoro-1 *H*-pyrrolo[2,3-*b*]pyridin-5-yl)oxy )-*N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)benzamide | MS-ESI (m/z): 993 [M + 1]⁺ |
| | or 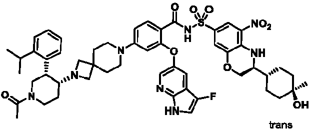 | | |
| D-154 | 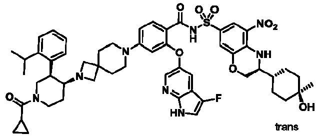 | 4-(2-((3*R*,4*S* or 3*S*,4*R*)-1-(cyclopropanecarbonyl) -3-(2-isopropylphenyl)piperidin-4-yl)-2,7-diazaspiro[3.5]nonan-7-yl)-2-((3-fluoro-1*H*-pyrrolo[2,3-*b* ]pyridin-5-yl)oxy)-*N*-(((*S*)-3-((1r, 4S)-4-hydroxy-4-methylcyclohex yl)-5-nitro-3,4-dihydro-2*H*-benzo [*b*][1,4]oxazin-7-yl)sulfonyl)ben zamide | MS-ESI (m/z): 1019 [M + 1]⁺ |
| | or 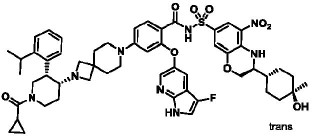 | | |
| D-155 | 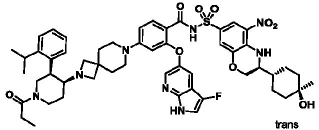 | 2-((3-fluoro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( 3*R*,4*S* or 3*S*,4*R*)-3-(2-isopropylphenyl)-1-propionylpiperidin-4-yl)-2,7-diaz aspiro[3.5]nonan-7-yl)benzamide | MS-ESI (m/z): 1007 [M + 1]⁺ |
| | or 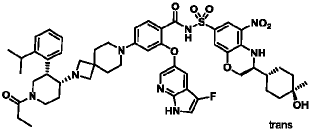 | | |
| D-156 | 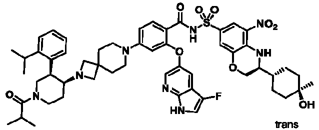 | 2-((3-fluoro-1*H*-pyrrolo[2,3-b]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( 3*R*,4*S* or 3*S*,4*R*)-1-isobutyryl-3-(2-isoprop ylphenyl)piperidin-4-yl)-2,7-diaz aspiro[3.5]nonan-7-yl)benzamide | MS-ESI (m/z): 1021 [M + 1]⁺ |
| | or 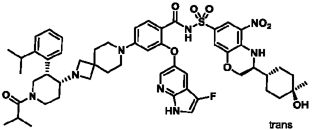 | | |
| D-157 | 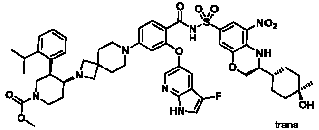 | methyl (3*R*,4*S* or 3*S*,4*R*)-4-(7-(3-((3-fluoro-1*H*-pyr rolo[2,3-b]pyridin-5-yl)oxy)-4-(( ((*S*)-3-((1r,4*S*)-4-hydroxy-4-meth ylcyclohexyl)-5-nitro-3,4-dihydr o-2*H*-benzo[*b*][1,4]oxazin-7-yl)s ulfonyl)carbamoyl)phenyl)-2,7-d iazaspiro[3.5]nonan-2-yl)-3-(2-is opropylphenyl)piperidine-1-carb oxylate | MS-ESI (m/z): 1009 [M + 1]⁺ |
| | or 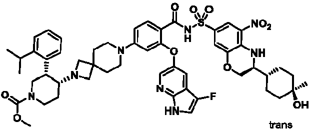 | | |
| D-158 | 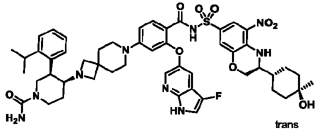 | (3*R*,4*S* or 3*S*,4*R*)-4-(7-(3-((3-fluoro-1*H*-pyr rolo[2,3-b]pyridin-5-yl)oxy)-4-(( ((*S*)-3-((1*r*,4*S*)-4-hydroxy-4-meth ylcyclohexyl)-5-nitro-3,4-dihydr o-2*H*-benzo[*b*][1,4]oxazin-7-yl) sulfonyl)carbamoyl)phenyl)-2,7-diazaspiro[3.5]nonan-2-yl)-3-(2-is opropylphenyl)piperidine-1-carb oxamide | MS-ESI (m/z): 994 [M + 1]⁺ |
| | or 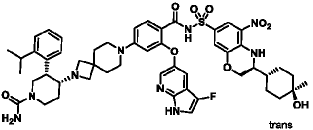 | | |
| D-159 | 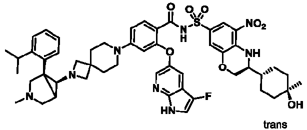 | 2-((3-fluoro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( 1*S*,6*S* or 1*R*, 6*R*)-1-(2-isopropylphenyl)-3-met hyl-3-azabicyclo[3.1.0]hexan-6-y l)-2,7-diazaspiro[3.5]nonan-7-yl) benzamide | MS-ESI (m/z): 963 [M + 1]⁺ |
| | or 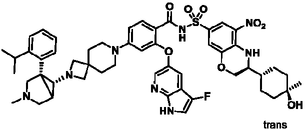 | | |
| D-160 | 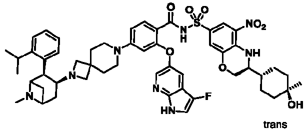 | 2-((3-fluoro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( 2*S*,3*S* or 2*R*, 3*R*)-2-(2-isopropylphenyl)-6-met hyl-6-azabicyclo[3.1.1]heptan-3-yl)-2,7-diazaspiro[3.5]nonan-7-yl )benzamide | MS-ESI (m/z): 977 [M + 1]⁺ |
| | or 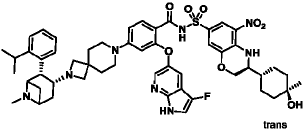 | | |
| D-161 | 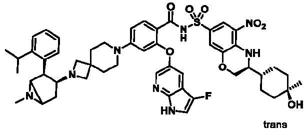 | 2-((3-fluoro-1*H*-pyrrolo[2,3-b]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( 2*S*,3*S* or 2*R*, 3*R*)-2-(2-isopropylphenyl)-6-met hyl-6-azabicyclo[3.1.0]hexan-3-y l)-2,7-diazaspiro[3.5]nonan-7-yl) benzamide | MS-ESI (m/z): 963 [M + 1]⁺ |
| | or 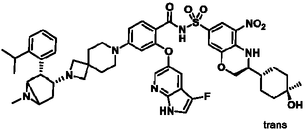 | | |
| D-162 | 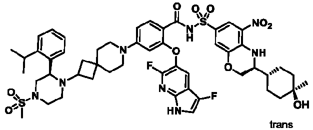 | 2-((3,6-difluoro-1*H*-pyrrolo[2,3-b]pyridin-5-yl)oxy)-*N*-(((*S*)-3-((1 r,4*S*)-4-hydroxy-4-methylcyclohe xyl)-5-nitro-3,4-dihydro-2*H*-benz o[*b*][1,4]oxazin-7-yl)sulfonyl)-4-(2-((*R* or *S*)-2-(2-isopropylphenyl)-4-(met hylsulfonyl)piperazin-1-yl)-7-aza spiro[3.5]nonan-7-yl)benzamide | MS-ESI (m/z): 1047 [M + 1]⁺ |
| | or 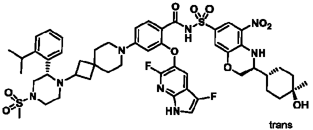 | | |
| D-163 | 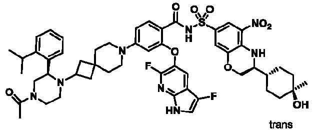 | 4-(2-((*R* or *S*)-4-acetyl-2-(2-isopropylphenyl )piperazin-1-yl)-7-azaspiro[3.5]n onan-7-yl)-2-((3,6-difluoro-1H-p yrrolo[2,3-b]pyridin-5-yl)oxy)-*N* -(((*S*)-3-((1*r*,4*S*)-4-hydroxy-4-me thylcyclohexyl)-5-nitro-3,4-dihy dro-2*H*-benzo[*b*][1,4]oxazin-7-yl )sulfonyl)benzamide | MS-ESI (m/z): 1011 [M + 1]⁺ |
| | or 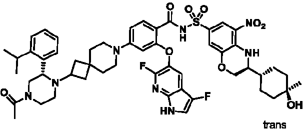 | | |
| D-164 | 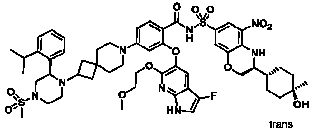 | 2-((3-fluoro-6-(2-methoxyethoxy )-1*H*-pyrrolo[2,3-*b*]pyridin-5-yl) oxy)-*N*-(((*S*)-3-((1*r,*4*S*)-4-hydrox y-4-methylcyclohexyl)-5-nitro-3, 4-dihydro-2*H*-benzo[*b*][1,4]oxazi n-7-yl)sulfonyl)-4-(2-((*R* or *S*)-2-(2-isopropylphenyl)-4-(met hylsulfonyl)piperazin-1-yl)-7-aza spiro[3.5]nonan-7-yl)benzamide | MS-ESI (m/z): 1103 [M + 1]⁺ |
| | or 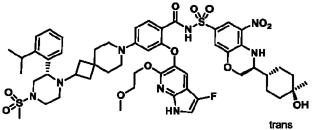 | | |
| D-165 | 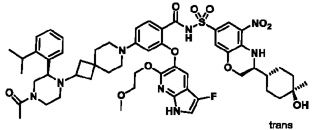 | 4-(2-((*R* or *S*)-4-acetyl-2-(2-isopropylphenyl )piperazin-1-yl)-7-azaspiro[3.5]n onan-7-yl)-2-((3-fluoro-6-(2-met hoxyethoxy)-1*H*-pyrrolo[2,3-*b*]p yridin-5-yl)oxy)-*N*-(((*S*)-3-((1*r*,4 *S*)-4-hydroxy-4-methylcyclohexy l)-5-nitro-3,4-dihydro-2*H*-benzo[ *b*][1,4]oxazin-7-yl)sulfonyl)benz amide | MS-ESI (m/z): 1067 [M + 1]⁺ |
| | or 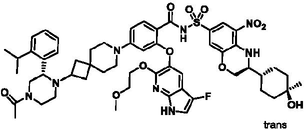 | | |
| D-166 | 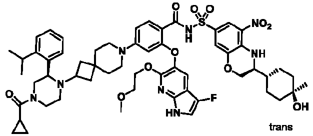 | 4-(2-((*R* or *S*)-4-(cyclopropanecarbonyl)-2-( 2-isopropylphenyl)piperazin-1-yl )-7-azaspiro[3.5]nonan-7-yl)-2-(( 3-fluoro-6-(2-methoxyethoxy)-1 *H*-pyrrolo[2,3-*b*]pyridin-5-yl)oxy )-*N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)benzamide | MS-ESI (m/z): 1093 [M + 1]⁺ |
| | or 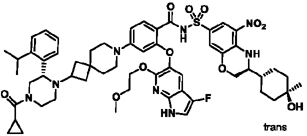 | | |
| D-167 | 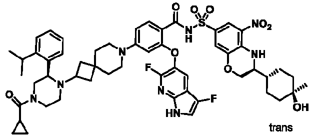 | 4-(2-((*R* or *S*)-4-(cyclopropanecarbonyl)-2-( 2-isopropylphenyl)piperazin-1-yl )-7-azaspiro[3.5]nonan-7-yl)-2-(( 3,6-difluoro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)benza mide | MS-ESI (m/z): 1037 [M + 1]⁺ |
| | or 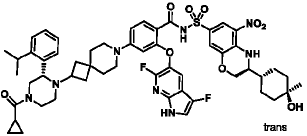 | | |
| D-168 | 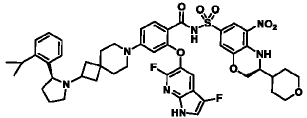 | 2-((3,6-difluoro-1*H*-pyrrolo[2,3-b]pyridin-5-yl)oxy)-4-(2-((*R* or *S*)-2-(2-isopropylphenyl)pyrrolid in-1-yl)-7-azaspiro[3.5]nonan-7-yl)-*N*-(((*S*)-5-nitro-3-(tetrahydro-2*H*-pyran-4-yl)-3,4-dihydro-2*H-*benzo[*b*][1,4]oxazin-7-yl)sulfony l)benzamide | MS-ESI (m/z): 926 [M + 1]⁺ |
| | or 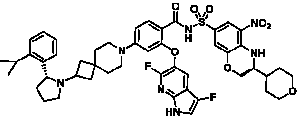 | | |
| D-169 | 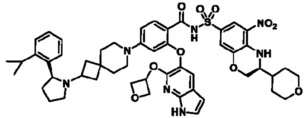 | 4-(2-((*R* or *S*)-2-(2-isopropylphenyl)pyrrolid in-1-yl)-7-azaspiro[3.5]nonan-7-yl)-*N*-(((*S*)-5-nitro-3-(tetrahydro-2*H*-pyran-4-yl)-3,4-dihydro-2*H-*benzo[*b*][1,4]oxazin-7-yl)sulfony 1)-2-((6-(oxetan-3-yloxy)-1*H*-pyr rolo[2,3-*b*]pyridin-5-yl)oxy)benz amide | MS-ESI (m/z): 962 [M + 1]⁺ |
| | or 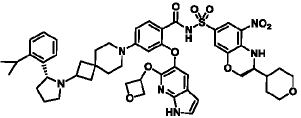 | | |
| D-170 | 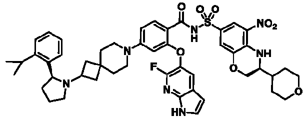 | 2-((6-fluoro-1*H*-pyrrolo[2,3-b]py ridin-5-yl)oxy)-4-(2-((*R* or *S*)-2-(2-isopropylphenyl)pyrrolid in-1-yl)-7-azaspiro[3.5]nonan-7-yl)-*N*-(((*S*)-5-nitro-3-(tetrahydro-2*H*-pyran-4-yl)-3,4-dihydro-2*H-*benzo[*b*][1,4]oxazin-7-yl)sulfony l)benzamide | MS-ESI (m/z): 908 [M + 1]⁺ |
| | or 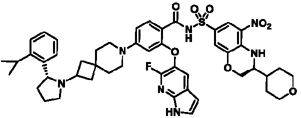 | | |
| D-171 | 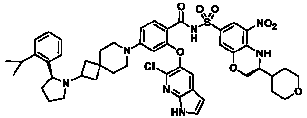 | 2-((6-chloro-1*H*-pyrrolo[2,3-*b*]py ridin-5-yl)oxy)-4-(2-((*R* or *S*)-2-(2-isopropylphenyl)pyrrolid in-1-yl)-7-azaspiro[3.5]nonan-7-yl)-*N*-(((*S*)-5-nitro-3-(tetrahydro-2*H*-pyran-4-yl)-3,4-dihydro-2*H-*benzo[*b*][1,4]oxazin-7-yl)sulfony l)benzamide | MS-ESI (m/z): 925 [M + 1]⁺ |
| | or 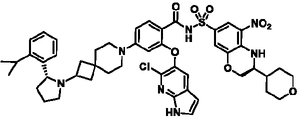 | | |
| D-172 | 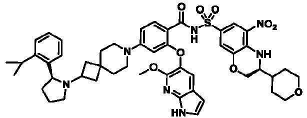 | 4-(2-((*R* or *S*)-2-(2-isopropylphenyl)pyrrolid in-1-yl)-7-azaspiro[3.5]nonan-7-yl)-2-((6-methoxy-1*H*-pyrrolo[2, 3-b]pyridin-5-yl)oxy)-*N*-(((*S*)-5-n itro-3-(tetrahydro-2H-pyran-4-yl) -3,4-dihydro-2*H*-benzo[b][1,4]ox azin-7-yl)sulfonyl)benzamide | MS-ESI (m/z): 920 [M + 1]⁺ |
| | or 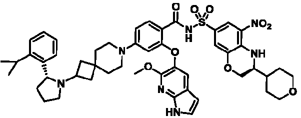 | | |
| D-173 | 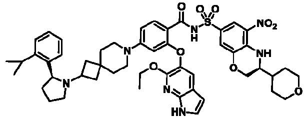 | 2-((6-ethoxy-1*H*-pyrrolo[2,3-b]p yridin-5-yl)oxy)-4-(2-((*R* or *S*)-2-(2-isopropylphenyl)pyrrolid in-1-yl)-7-azaspiro[3.5]nonan-7-yl)-*N*-(((*S*)-5-nitro-3-(tetrahydro-2*H*-pyran-4-yl)-3,4-dihydro-2*H-*benzo[*b*][1,4]oxazin-7-yl)sulfony l)benzamide | MS-ESI (m/z): 934 [M + 1]⁺ |
| | or 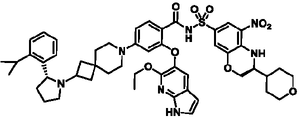 | | |
| D-174 | 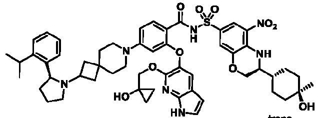 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-2-((6-((1-hydroxycy clopropyl)methoxy)-1*H*-pyrrolo[ 2,3-*b*]pyridin-5-yl)oxy)-4-(2-((*R* or *S*)-2-(2-isopropylphenyl)pyrrolid in-1-yl)-7-azaspiro[3.5]nonan-7-yl)benzamide | MS-ESI (m/z): 1004 [M + 1]⁺ |
| | or | | |
| | 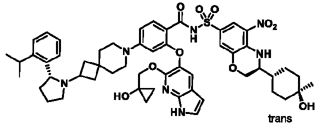 | | |
| D-175 | 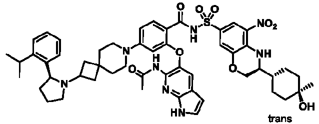 | 2-((6-acetamido-1*H*-pyrrolo[2,3-*b*]pyridin-5-yl)oxy)-*N*-(((*S*)-3-((1 *r*,4*S*)-4-hydroxy-4-methylcycloh exyl)-5-nitro-3,4-dihydro-2*H*-ben zo[*b*][1,4]oxazin-7-yl)sulfonyl)-4 -(2-((*R* or *S*)-2-(2-isopropylphenyl)pyrrolid in-1-yl)-7-azaspiro[3.5]nonan-7-yl)benzamide | MS-ESI (m/z): 975 [M + 1]⁺ |
| | or | | |
| | 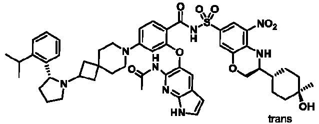 | | |
| D-176 | 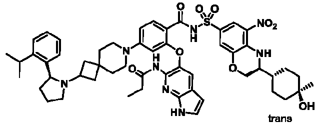 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-propionamido-1*H*-pyrro lo[2,3-*b*]pyridin-5-yl)oxy)benza mide | MS-ESI (m/z): 989 [M + 1]⁺ |
| | or | | |
| | 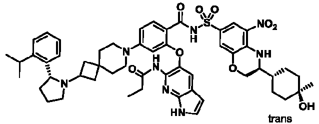 | | |
| D-177 | 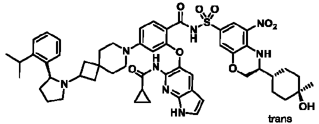 | 2-((6-(cyclopropanecarboxamido )-1*H*-pyrrolo[2,3-*b*]pyridin-5-yl) oxy)-*N*-(((*S*)-3-((1*r*,4*S*)-4-hydrox y-4-methylcyclohexyl)-5-nitro-3, 4-dihydro-2*H*-benzo[*b*][1,4]oxazi n-7-yl)sulfonyl)-4-(2-((*R* or *S*)-2-(2-isopropylphenyl)pyrrolid in-1-yl)-7-azaspiro[3.5]nonan-7-yl)benzamide | MS-ESI (m/z): 1001 [M + 1]⁺ |
| | or | | |
| | 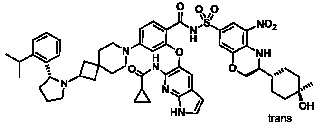 | | |
| D-178 | 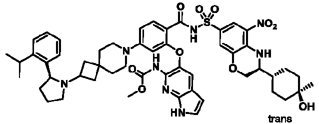 | methyl (5-(2-((((*S*)-3-((1*r*,4*S*)-4-hydroxy -4-methylcyclohexyl)-5-nitro-3,4 -dihydro-2*H*-benzo[*b*][1,4]oxazin -7-yl)sulfonyl)carbamoyl)-5-(2-(( *R* or *S*)-2-(2-isopropylphenyl)pyrrolid in-1-yl)-7-azaspiro[3.5]nonan-7-yl)phenoxy)-1*H*-pyrrolo[2,3-*b*]py ridin-6-yl)carbamate | MS-ESI (m/z): 991 [M + 1]⁺ |
| | or | | |
| | 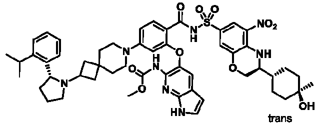 | | |
| D-179 | 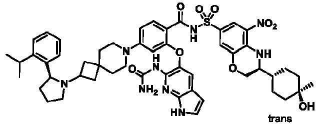 | N-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-ureido-1*H*-pyrrolo[2,3-*b*]pyridin-5-yl)oxy)benzamide | MS-ESI (m/z): 976 [M + 1]⁺ |
| | or | | |
| | 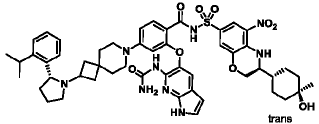 | | |
| D-180 | 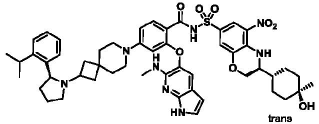 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-(methylamino)-1*H*-pyrr olo[2,3-*b*]pyridin-5-yl)oxy)benza mide | MS-ESI (m/z): 947 [M + 1]⁺ |
| | or | | |
| | 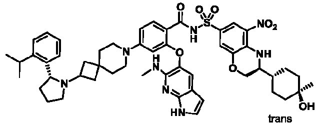 | | |
| D-181 | 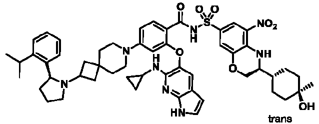 | 2-((6-(cyclopropylamino)-1*H*-pyr rolo[2,3-b]pyridin-5-yl)oxy)-*N*-(( (*S*)-3-((1*r*,4*S*)-4-hydroxy-4-meth ylcyclohexyl)-5-nitro-3,4-dihydr o-2*H*-benzo[*b*][1,4]oxazin-7-yl)s ulfonyl)-4-(2-((*R* or *S*)-2-(2-isopropylphenyl)pyrrolid in-1-yl)-7-azaspiro[3.5]nonan-7-yl)benzamide | MS-ESI (m/z): 973 [M + 1]⁺ |
| | or | | |
| | 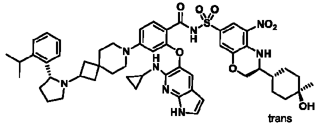 | | |
| D-182 | 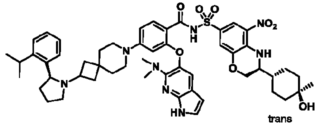 | 2-((6-(dimethylamino)-1H-pyrrol o[2,3-b]pyridin-5-yl)oxy)-*N*-(((*S*) -3-((1*r*,4*S*)-4-hydroxy-4-methylc yclohexyl)-5-nitro-3,4-dihydro-2 *H*-benzo[*b*][1,4]oxazin-7-yl)sulfo nyl)-4-(2-((*R* or *S*)-2-(2-isopropylphenyl)pyrrolid in-1-yl)-7-azaspiro[3.5]nonan-7-yl)benzamide | MS-ESI (m/z): 961 [M + 1]⁺ |
| | or | | |
| | 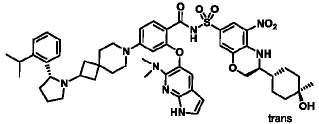 | | |
| D-183 | 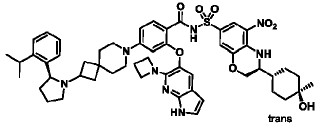 | 2-((6-(azetidin-1-yl)-1H-pyrrolo[ 2,3-*b*]pyridin-5-yl)oxy)-*N*-(((*S*)-3 -((1*r*,4*S*)-4-hydroxy-4-methylcyc lohexyl)-5-nitro-3,4-dihydro-2*H-*benzo[*b*][1,4]oxazin-7-yl)sulfony l)-4-(2-((*R* or *S*)-2-(2-isopropylphenyl)pyrrolid in-1-yl)-7-azaspiro[3.5]nonan-7-yl)benzamide | MS-ESI (m/z): 973 [M + 1]⁺ |
| | or | | |
| | 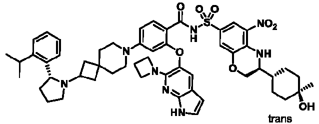 | | |
| D-184 | 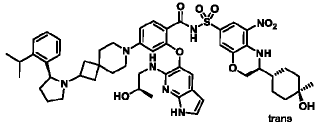 | *N*-(((*S*)-3-((1*r*,4S)-4-hydroxy-4-methylcyclohexyl)-5-nitro-3,4-di hydro-2*H*-benzo[*b*][1,4]oxazin-7 -yl)sulfonyl)-2-((6-(((*R*)-2-hydro xypropyl)amino)-1*H*-pyrrolo[2,3 -*b*]pyridin-5-yl)oxy)-4-(2-((*R* or *S*)-2-(2-isopropylphenyl)pyrrolid in-1-yl)-7-azaspiro[3.5]nonan-7-yl)benzamide | MS-ESI (m/z): 991 [M + 1]⁺ |
| | or | | |
| | 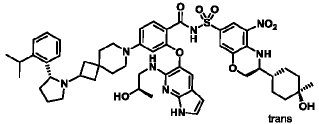 | | |
| D-185 | 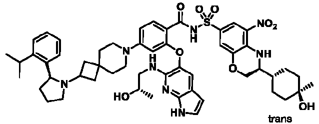 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-2-((6-(((*S*)-2-hydro xypropyl)amino)-1*H*-pyrrolo[2,3 -*b*]pyridin-5-yl)oxy)-4-(2-((*R* or *S*)-2-(2-isopropylphenyl)pyrrolid in-1-yl)-7-azaspiro[3.5]nonan-7-yl)benzamide | MS-ESI (m/z): 991 [M + 1]⁺ |
| | or | | |
| | 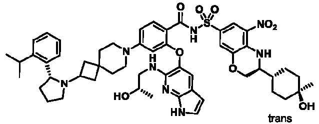 | | |
| D-186 | 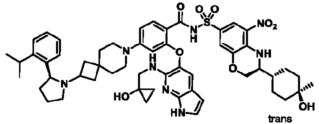 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-2-((6-(((1-hydroxyc yclopropyl)methyl)amino)-1*H*-p yrrolo[2,3-*b*]pyridin-5-yl)oxy)-4-(2-((*R* or *S*)-2-(2-isopropylphenyl)pyrrolid in-1-yl)-7-azaspiro[3.5]nonan-7-yl)benzamide | MS-ESI (m/z): 1003 [M + 1]⁺ |
| | or | | |
| | 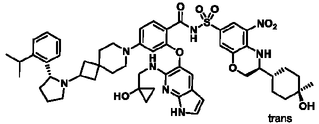 | | |
| D-187 | 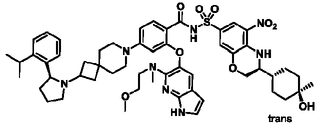 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-((2-methoxyethyl)(meth yl)amino)-1*H*-pyrrolo[2,3-*b*]pyri din-5-yl)oxy)benzamide | MS-ESI (m/z): 1005 [M + 1]⁺ |
| | or | | |
| | 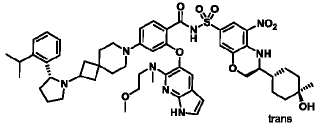 | | |
| D-188 | 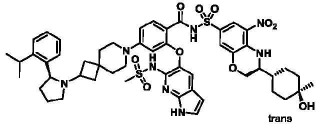 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-(methylsulfonamido)-1 *H*-pyrrolo[2,3-*b*]pyridin-5-yl)oxy )benzamide | MS-ESI (m/z): 1011 [M + 1]⁺ |
| | or | | |
| | 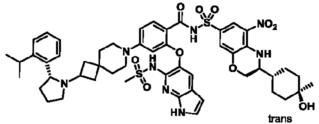 | | |
| D-189 | 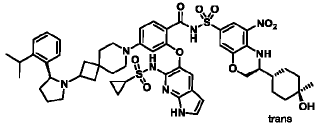 | 2-((6-(cyclopropanesulfonamido) -1*H*-pyrrolo[2,3-*b*]pyridin-5-yl)o xy)-*N*-(((*S*)-3-((1*r*,4*S*)-4-hydroxy -4-methylcyclohexyl)-5-nitro-3,4 -dihydro-2*H*-benzo[*b*][1,4]oxazin -7-yl)sulfonyl)-4-(2-((*R* or S)-2-(2-isopropylphenyl)pyrrolid in-1-yl)-7-azaspiro[3.5]nonan-7-yl)benzamide | MS-ESI (m/z): 1037 [M + 1]⁺ |
| | or | | |
| | 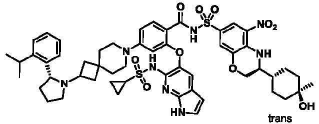 | | |
| D-190 | 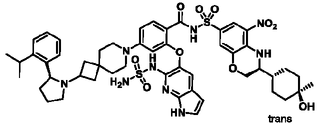 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-(sulfamoylamino)-1*H*-p yrrolo[2,3-*b*]pyridin-5-yl)oxy)be nzamide | MS-ESI (m/z): 1012 [M + 1]⁺ |
| | or | | |
| | 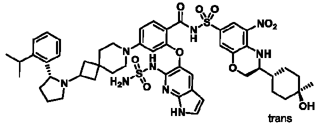 | | |
| D-191 | 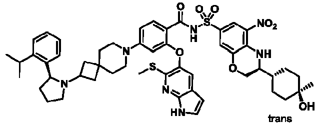 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-(methylthio)-1*H*-pyrrolo [2,3-*b*]pyridin-5-yl)oxy)benzam ide | MS-ESI (m/z): 964 [M + 1]⁺ |
| | or | | |
| | 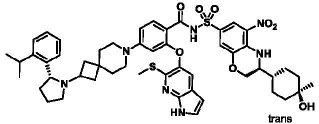 | | |
| D-192 | 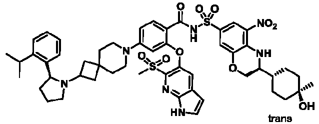 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-(methylsulfonyl)-1*H*-py rrolo[2,3-*b*]pyridin-5-yl)oxy)ben zamide | MS-ESI (m/z): 996 [M + 1]⁺ |
| | or | | |
| | 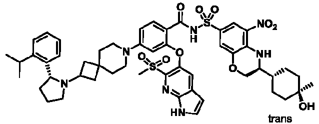 | | |
| D-193 | 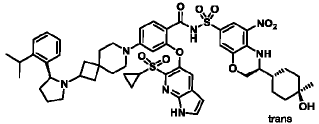 | 2-((6-(cyclopropylsulfonyl)-1*H-*pyrrolo[2,3-*b*]pyridin-5-yl)oxy)-*N* -(((*S*)-3-((1*r*,4*S*)-4-hydroxy-4-me thylcyclohexyl)-5-nitro-3,4-dihy dro-2*H*-benzo[*b*][1,4]oxazin-7-yl )sulfonyl)-4-(2-((*R* or *S*)-2-(2-isopropylphenyl)pyrrolid in-1-yl)-7-azaspiro[3.5]nonan-7-yl)benzamide | MS-ESI (m/z): 1022 [M + 1]⁺ |
| | or | | |
| | 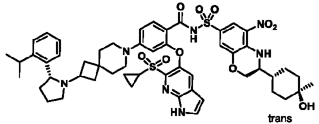 | | |
| D-194 | 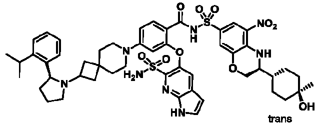 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-sulfamoyl-1*H*-pyrrolo[2 ,3-*b*]pyridin-5-yl)oxy)benzamide | MS-ESI (m/z): 997 [M + 1]⁺ |
| | or | | |
| | 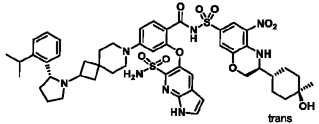 | | |
| D-195 | 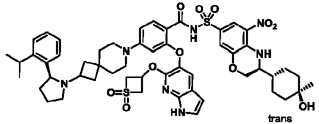 | 2-((6-((1,1-dioxidothietan-3-yl)o xy)-1*H*-pyrrolo[2,3-*b*]pyridin-5-y l)oxy)-*N*-(((*S*)-3-((1*r*,4*S*)-4-hydro xy-4-methylcyclohexyl)-5-nitro-3,4-dihydro-2*H*-benzo[*b*][1,4]oxa zin-7-yl)sulfonyl)-4-(2-((*R* or *S*)-2-(2-isopropylphenyl)pyrrolid in-1-yl)-7-azaspiro[3.5]nonan-7-yl)benzamide | MS-ESI (m/z): 1038 [M + 1]⁺ |
| | or | | |
| | 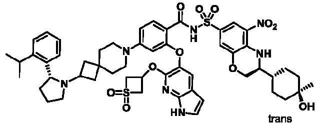 | | |
| D-196 | 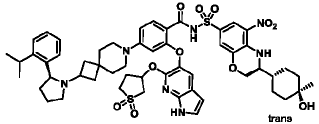 | 2-((6-((1,1-dioxidotetrahydrothio phen-3-yl)oxy)-1*H*-pyrrolo[2,3-b ]pyridin-5-yl)oxy)-*N*-(((*S*)-3-((1*r*, 4*S*)-4-hydroxy-4-methylcyclohex yl)-5-nitro-3,4-dihydro-2*H*-benzo [*b*][1,4]oxazin-7-yl)sulfonyl)-4-( 2-((*R* or *S*)-2-(2-isopropylphenyl)pyrrolid in-1-yl)-7-azaspiro[3.5]nonan-7-yl)benzamide | MS-ESI (m/z): 1052 [M + 1]⁺ |
| | or | | |
| | 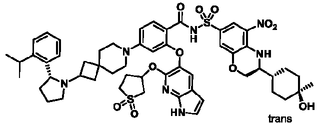 | | |
| D-197 | 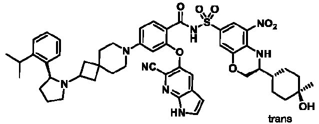 | 2-((6-cyano-1*H*-pyrrolo[2,3-b]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( *R* or *S*)-2-(2-isopropylphenyl)pyrrolid in-1-yl)-7-azaspiro[3.5]nonan-7-yl)benzamide | MS-ESI (m/z): 943 [M + 1]⁺ |
| | or | | |
| | 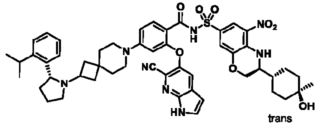 | | |
| D-198 | 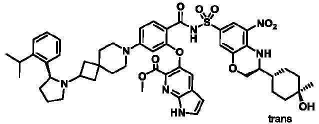 | methyl 5-(2-((((*S*)-3-((1*r*,4*S*)-4-hydroxy-4-methylcyclohexyl)-5-nitro-3,4-dihydro-2*H*-benzo[*b*][1,4]oxazin-7-yl)sulfonyl)carbamoyl)-5-(2-(( *R* or *S*)-2-(2-isopropylphenyl)pyrrolid in-1-yl)-7-azaspiro[3.5]nonan-7-yl)phenoxy)-1*H*-pyrrolo[2,3-*b*]py ridine-6-carboxylate | MS-ESI (m/z): 976 [M + 1]⁺ |
| | or | | |
| | 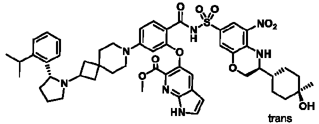 | | |
| D-199 | 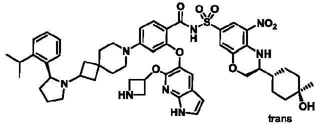 | 2-((6-(azetidin-3-yloxy)-1*H*-pyrr olo[2,3-*b*]pyridin-5-yl)oxy)-*N*-((( *S*)-3-((1*r*,4*S*)-4-hydroxy-4-methy lcyclohexyl)-5-nitro-3,4-dihydro-2*H*-benzo[*b*][1,4]oxazin-7-yl)sulf onyl)-4-(2-((*R* or *S*)-2-(2-isopropylphenyl)pyrrolid in-1-yl)-7-azaspiro[3.5]nonan-7-yl)benzamide | MS-ESI (m/z): 989 [M + 1]⁺ |
| | or | | |
| | 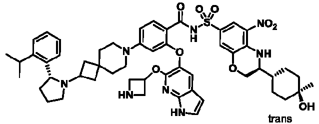 | | |
| D-200 | 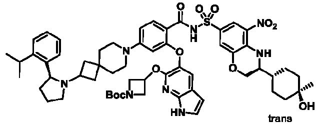 | *tert-*butyl 3-((5-(2-((((*S*)-3-((1*r*,4*S*)-4-hydro xy-4-methylcyclohexyl)-5-nitro-3,4-dihydro-2*H*-benzo[*b*][1,4]oxa zin-7-yl)sulfonyl)carbamoyl)-5-( 2-((*R* or *S*)-2-(2-isopropylphenyl)pyrrolid in-1-yl)-7-azaspiro[3.5]nonan-7-yl)phenoxy)-1*H*-pyrrolo[2,3-*b*]py ridin-6-yl)oxy)azetidine-1-carbox ylate | MS-ESI (m/z): 1090 [M + 1]⁺ |
| | or | | |
| | 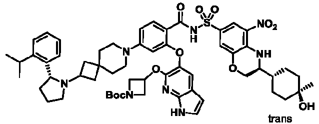 | | |
| D-201 | 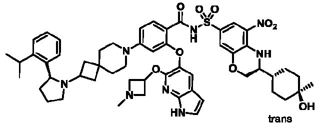 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-((1-methylazetidin-3-yl) oxy)-1*H*-pyrrolo[2,3-*b*]pyridin-5-yl)oxy)benzamide | MS-ESI (m/z): 1003 [M + 1]⁺ |
| | or | | |
| | 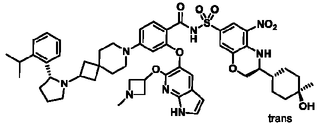 | | |
| D-202 | 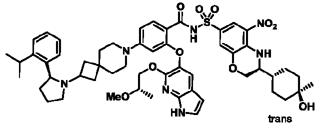 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-((*S*)-2-methoxypropoxy )-1*H*-pyrrolo[2,3-*b*]pyridin-5-yl) oxy)benzamide | MS-ESI (m/z): 1006 [M + 1]⁺ |
| | or | | |
| | 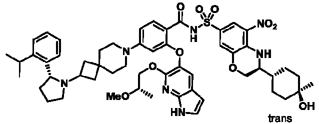 | | |
| D-203 | 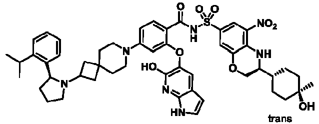 | 2-((6-hydroxy-1*H*-pyrrolo[2,3-*b*] pyridin-5-yl)oxy)-*N*-(((*S*)-3-((1*r*, 4*S*)-4-hydroxy-4-methylcyclohex yl)-5-nitro-3,4-dihydro-2*H*-benzo [*b*][1,4]oxazin-7-yl)sulfonyl)-4-( *2-((R* or *S*)-2-(2-isopropylphenyl)pyrrolid in-1-yl)-7-azaspiro[3.5]nonan-7-yl)benzamide | MS-ESI (m/z): 934 [M + 1]⁺ |
| | or | | |
| | 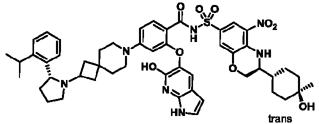 | | |
| D-204 | 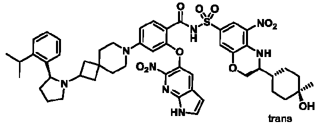 | *N-*(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-nitro-1*H*-pyrrolo[2,3-*b*] pyridin-5-yl)oxy)benzamide | MS-ESI (m/z): 963 [M + 1]⁺ |
| | or | | |
| | 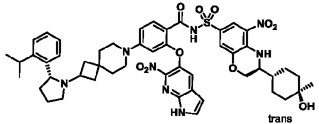 | | |
| D-205 | 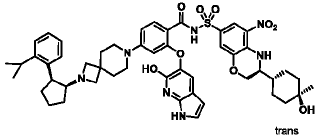 | 2-((6-hydroxy-1*H*-pyrrolo[2,3-*b*] pyridin-5-yl)oxy)-*N*-(((*S*)-3-((1*r*, 4*S*)-4-hydroxy-4-methylcyclohex yl)-5-nitro-3,4-dihydro-2*H*-benzo [*b*][1,4]oxazin-7-yl)sulfonyl)-4-( 2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 934 [M + 1]⁺ |
| | or | | |
| | 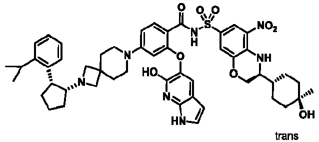 | | |
| D-206 | 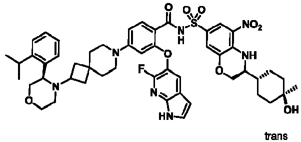 or | 2-((6-fluoro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( *R* or *S*)-3-(2-isopropylphenyl)morphol ino)-7-azaspiro[3.5]nonan-7-yl)b enzamide | MS-ESI (m/z): 952 [M + 1]⁺ |
| | 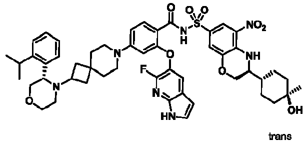 | | |
| D-207 | 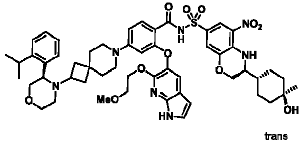 or | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-3-(2-isopropylphenyl)morphol ino)-7-azaspiro[3.5]nonan-7-yl)-2-((6-(2-methoxyethoxy)-1*H*-pyr rolo[2,3-*b*]pyridin-5-yl)oxy)benz amide | MS-ESI (m/z): 1008 [M + 1]⁺ |
| | 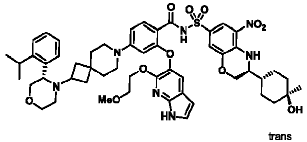 | | |
| D-208 | 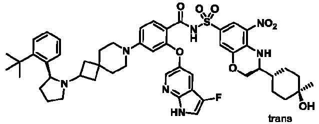 | 4-(2-((*R* or *S*)-2-(2-(tert-butyl)phenyl)pyrroli din-1-yl)-7-azaspiro[3.5]nonan-7 -yl)-2-((3-fluoro-1*H*-pyrrolo[2,3-*b*]pyridin-5-yl)oxy)-*N*-(((*S*)-3-((1 *r*,4*S*)-4-hydroxy-4-methylcycloh exyl)-5-nitro-3,4-dihydro-2*H*-ben zo[*b*][1,4]oxazin-7-yl)sulfonyl)b enzamide | MS-ESI (m/z): 950 [M + 1]⁺ |
| | or | | |
| | 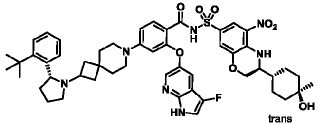 | | |
| D-209 | 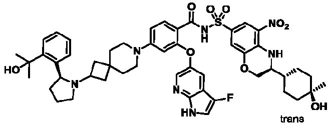 | 2-((3-fluoro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( *R* or *S*)-2-(2-(2-hydroxypropan-2-yl)p henyl)pyrrolidin-1-yl)-7-azaspiro [3.5]nonan-7-yl)benzamide | MS-ESI (m/z): 952 [M + 1]⁺ |
| | or | | |
| | 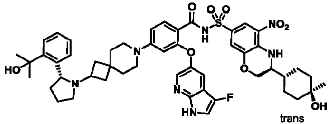 | | |
| D-210 | 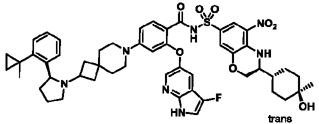 | 2-((3-fluoro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( *R* or *S*)-2-(2-(1-methylcyclopropyl)ph enyl)pyrrolidin-1-yl)-7-azaspiro[ 3.5]nonan-7-yl)benzamide | MS-ESI (m/z): 948 [M + 1]⁺ |
| | or | | |
| | 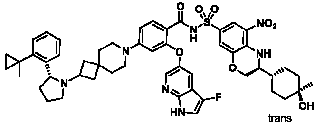 | | |
| D-211 | 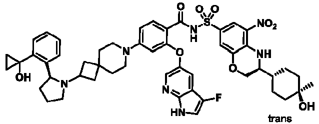 | 2-((3-fluoro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( *R* or *S*)-2-(2-(1-hydroxycyclopropyl)p henyl)pyrrolidin-1-yl)-7-azaspiro [3.5]nonan-7-yl)benzamide | MS-ESI (m/z): 950 [M + 1]⁺ |
| | or | | |
| | 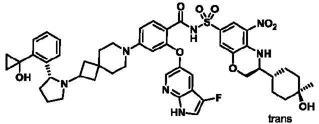 | | |
| D-212 | 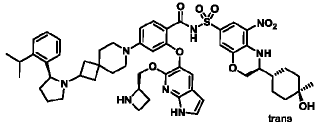 | 2-((6-(((*R*)-azetidin-2-yl)methox y)-1*H*-pyrrolo[2,3-*b*]pyridin-5-yl )oxy)-*N*-(((*S*)-3-((1*r*,4*S*)-4-hydro xy-4-methylcyclohexyl)-5-nitro-3,4-dihydro-2*H*-benzo[*b*][1,4]oxa zin-7-yl)sulfonyl)-4-(2-((*R* or *S*)-2-(2-isopropylphenyl)pyrrolid in-1-yl)-7-azaspiro[3.5]nonan-7-yl)benzamide | MS-ESI (m/z): 1003 [M + 1]⁺ |
| | or | | |
| | 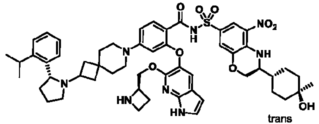 | | |
| D-213 | 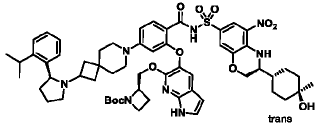 | *tert-*butyl (*R*)-2-(((5-(2-((((*S*)-3-((1*r*,4*S*)-4-hydroxy-4-methylcyclohexyl)-5-nitro-3,4-dihydro-2*H*-benzo[*b*][1, 4]oxazin-7-yl)sulfonyl)carbamoy 1)-5-(2-((*R* or *S*)-2-(2-isopropylphenyl)pyrrolid in-1-yl)-7-azaspiro[3.5]nonan-7-yl)phenoxy)-1*H*-pyrrolo[2,3-*b*]py ridin-6-yl)oxy)methyl)azetidine-1-carboxylate | MS-ESI (m/z): 1104 [M + 1]⁺ |
| | or | | |
| | 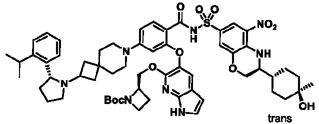 | | |
| D-214 | 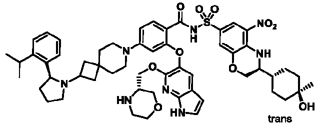 | *N*-(((*S*)-3-((1*r*,4*S*)-4-hydroxy-4-m ethylcyclohexyl)-5-nitro-3,4-dihy dro-2*H*-benzo[*b*][1,4]oxazin-7-yl )sulfonyl)-4-(2-((*R* or *S*)-2-(2-isopropylphenyl)pyrrolid in-1-yl)-7-azaspiro[3.5]nonan-7-yl)-2-((6-(((*S*)-morpholin-3-yl)m ethoxy)-1*H*-pyrrolo[2,3-*b*]pyridi n-5-yl)oxy)benzamide | MS-ESI (m/z): 1033 [M + 1]⁺ |
| | or | | |
| | 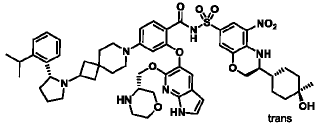 | | |
| D-215 | 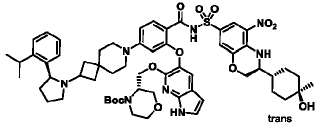 | *tert-*butyl (*S*)-3-(((5-(2-((((*S*)-3-((1*r*,4*S*)-4-h ydroxy-4-methylcyclohexyl)-5-ni tro-3,4-dihydro-2*H*-benzo[*b*][1,4] oxazin-7-yl)sulfonyl)carbamoyl)-5-(2-((*R* or S)-2-(2-isopropylphenyl)pyrrolid in-1-yl)-7-azaspiro[3.5]nonan-7-yl)phenoxy)-1*H*-pyrrolo[2,3-*b*]py ridin-6-yl)oxy)methyl)morpholin e-4-carboxylate | MS-ESI (m/z): 1134 [M + 1]⁺ |
| | or | | |
| | 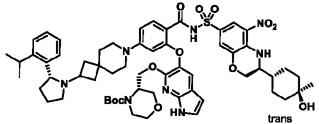 | | |
| D-216 | 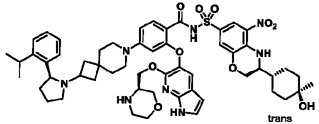 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((R or *S*)-2-(2-isopropylphenyl)pyrrolid in-1-yl)-7-azaspiro[3.5]nonan-7-yl)-2-((6-(((*R*)-morpholin-3-yl)m ethoxy)-1*H*-pyrrolo[2,3-b]pyridi n-5-yl)oxy)benzamide | MS-ESI (m/z): 1033 [M + 1]⁺ |
| | or | | |
| | 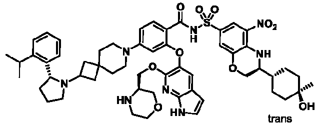 | | |
| D-217 | 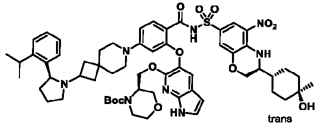 | *tert-*butyl (*R*)-3-(((5-(2-((((*S*)-3-((1*r*,4*S*)-4-hydroxy-4-methylcyclohexyl)-5-nitro-3,4-dihydro-2*H*-benzo[*b*][1, 4]oxazin-7-yl)sulfonyl)carbamoy 1)-5-(2-((*R* or S)-2-(2-isopropylphenyl)pyrrolid in-1-yl)-7-azaspiro[3.5]nonan-7-yl)phenoxy)-1*H*-pyrrolo[2,3-*b*]py ridin-6-yl)oxy)methyl)morpholin e-4-carboxylate | MS-ESI (m/z): 1134 [M + 1]⁺ |
| | or | | |
| | 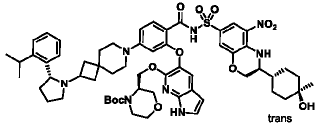 | | |
| D-218 | 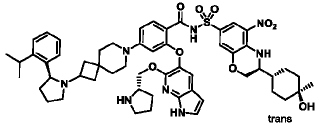 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-(((*S*)-pyrrolidin-2-yl)me thoxy)-1*H*-pyrrolo[2,3-*b*]pyridin-5-yl)oxy)benzamide | MS-ESI (m/z): 1017 [M + 1]⁺ |
| | or | | |
| | 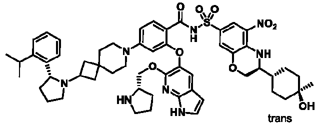 | | |
| D-219 | 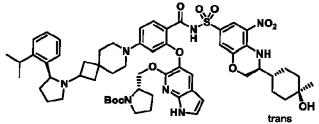 | *tert-*butyl (*S*)-2-(((5-(2-((((*S*)-3-((1*r*,4*S*)-4-h ydroxy-4-methylcyclohexyl)-5-ni tro-3,4-dihydro-2*H*-benzo[*b*][1,4] oxazin-7-yl)sulfonyl)carbamoyl)-5-(2-((*R* or *S*)-2-(2-isopropylphenyl)pyrrolid in-1-yl)-7-azaspiro[3.5]nonan-7-yl)phenoxy)-1*H*-pyrrolo[2,3-*b*]py ridin-6-yl)oxy)methyl)pyrrolidin e-1-carboxylate | MS-ESI (m/z): 1118 [M + 1]⁺ |
| | or | | |
| | 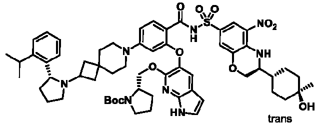 | | |
| D-220 | 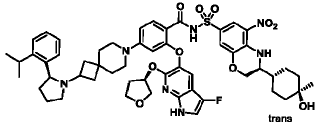 | 2-((3-fluoro-6-(((*R*)-tetrahydrofur an-3-yl)oxy)-1*H*-pyrrolo[2,3-b]p yridin-5-yl)oxy)-*N*-(((*S*)-3-((1*r*,4 *S*)-4-hydroxy-4-methylcyclohexy l)-5-nitro-3,4-dihydro-2*H*-benzo[ *b*][1,4]oxazin-7-yl)sulfonyl)-4-(2 -((*R* or *S*)-2-(2-isopropylphenyl)pyrrolid in-1-yl)-7-azaspiro[3.5]nonan-7-yl)benzamide | MS-ESI (m/z): 1022 [M + 1]⁺ |
| | or 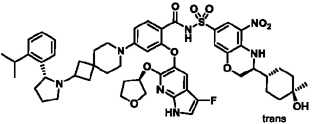 | | |
| D-221 | 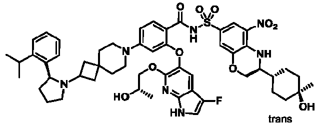 | 2-((3-fluoro-6-((*S*)-2-hydroxypro poxy)-1*H*-pyrrolo[2,3-*b*]pyridin-5-yl)oxy)-*N*-(((*S*)-3-((1*r*,4*S*)-4-hy droxy-4-methylcyclohexyl)-5-nit ro-3,4-dihydro-2*H*-benzo[*b*][1,4] oxazin-7-yl)sulfonyl)-4-(2-((*R* or *S*)-2-(2-isopropylphenyl)pyrrolid in-1-yl)-7-azaspiro[3.5]nonan-7-yl)benzamide | MS-ESI (m/z): 1010 [M + 1]⁺ |
| | or 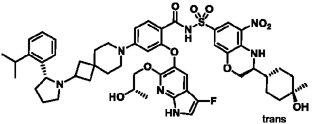 | | |
| D-222 | 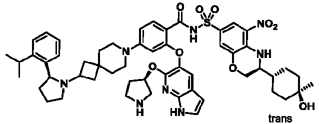 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-(((*R*)-pyrrolidin-3-yl)ox y)-1*H*-pyrrolo[2,3-*b*]pyridin-5-yl )oxy)benzamide | MS-ESI (m/z): 1003 [M + 1]⁺ |
| | or 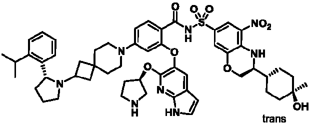 | | |
| D-223 | 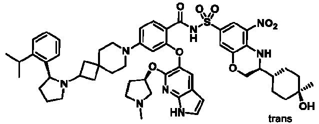 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-(((*R*)-1-methylpyrrolidi n-3-yl)oxy)-1*H*-pyrrolo[2,3-*b*]py ridin-5-yl)oxy)benzamide | MS-ESI (m/z): 1017 [M + 1]⁺ |
| | or 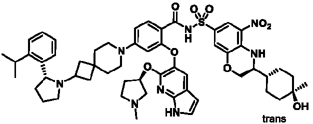 | | |
| D-224 | 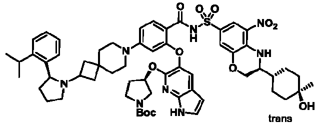 | *tert-*butyl (*R*)-3-((5-(2-((((*S*)-3-((1*r*,4*S*)-4-h ydroxy-4-methylcyclohexyl)-5-ni tro-3,4-dihydro-2*H*-benzo[*b*] [1,4] oxazin-7-yl)sulfonyl)carbamoyl)-5-(2-((*R* or S)-2-(2-isopropylphenyl)pyrrolid in-1-yl)-7-azaspiro[3.5]nonan-7-yl)phenoxy)-1*H*-pyrrolo[2,3-*b*]py ridin-6-yl)oxy)pyrrolidine-1-carb oxylate | MS-ESI (m/z): 1103 [M + 1]⁺ |
| | or 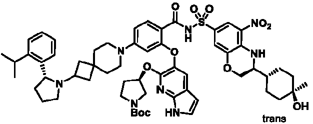 | | |
| D-225 | 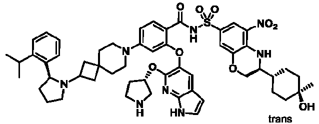 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-(((*S*)-pyrrolidin-3-yl)ox y)-1*H*-pyrrolo[2,3-*b*]pyridin-5-yl )oxy)benzamide | MS-ESI (m/z): 1003 [M + 1]⁺ |
| | or 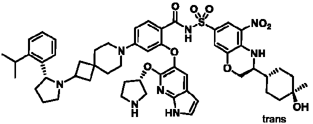 | | |
| D-226 | 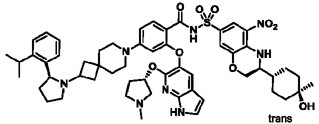 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-(((*S*)-1-methylpyrrolidi n-3-yl)oxy)-1*H*-pyrrolo[2,3-*b*]py ridin-5-yl)oxy)benzamide | MS-ESI (m/z): 1017 [M + 1]⁺ |
| | or 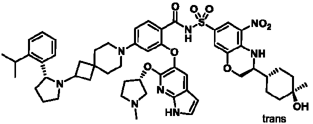 | | |
| D-227 | 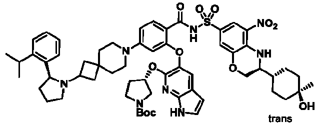 | *tert-*butyl (*S*)-3-((5-(2-((((*S*)-3-((1*r*,4*S*)-4-h ydroxy-4-methylcyclohexyl)-5-ni tro-3,4-dihydro-2*H*-benzo[*b*][1,4] oxazin-7-yl)sulfonyl)carbamoyl)-5-(2-((*R* or *S*)-2-(2-isopropylphenyl)pyrrolid in-1-yl)-7-azaspiro[3.5]nonan-7-yl)phenoxy)-1*H*-pyrrolo[2,3-*b*]py ridin-6-yl)oxy)pyrrolidine-1-carb oxylate | MS-ESI (m/z): 1103 [M + 1]⁺ |
| | or 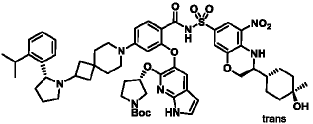 | | |
| D-228 | 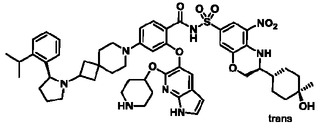 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-(piperidin-4-yloxy)-1*H-*pyrrolo[2,3-*b*]pyridin-5-yl)oxy)b enzamide | MS-ESI (m/z): 1017 [M + 1]⁺ |
| | or 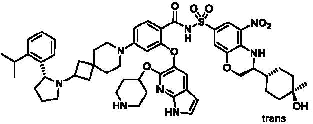 | | |
| D-229 | 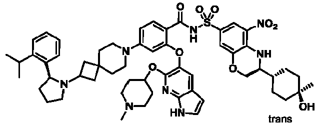 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((6-((1-methylpiperidin-4-y 1)oxy)-1*H*-pyrrolo[2,3-*b*]pyridin-5-yl)oxy)benzamide | MS-ESI (m/z): 1031 [M + 1]⁺ |
| | or 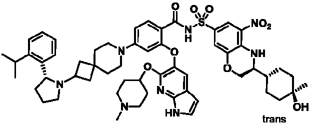 | | |
| D-230 | 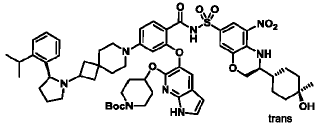 | *tert-*butyl 4-((5-(2-((((*S*)-3-((1*r*,4*S*)-4-hydro xy-4-methylcyclohexyl)-5-nitro-3,4-dihydro-2*H*-benzo[*b*][1,4]oxa zin-7-yl)sulfonyl)carbamoyl)-5-( 2-((*R* or *S*)-2-(2-isopropylphenyl)pyrrolid in-1-yl)-7-azaspiro[3.5]nonan-7-yl)phenoxy)-1*H*-pyrrolo[2,3-*b*]py ridin-6-yl)oxy)piperidine-1-carbo xylate | MS-ESI (m/z): 1118 [M + 1]⁺ |
| | or 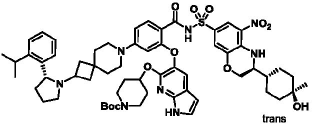 | | |
| D-231 | 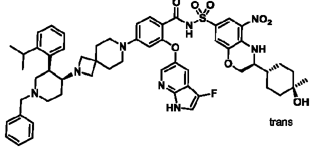 | 4-(2-((3*R*,4*S* and 3*S*,4*R*)-1-benzyl-3-(2-isopropylp henyl)piperidin-4-yl)-2,7-diazasp iro[3.5]nonan-7-yl)-2-((3-fluoro-1*H*-pyrrolo[2,3-b]pyridin-5-yl)ox y)-*N*-(((*S*)-3-((1*r*,4*S*)-4-hydroxy-4-methylcyclohexyl)-5-nitro-3,4-dihydro-2*H*-benzo[*b*][1,4]oxazin-7-yl)sulfonyl)benzamide | MS-ESI (m/z): 1041 [M + 1]⁺ |
| | and | | |
| | 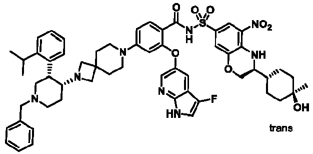 | | |
| D-232 | 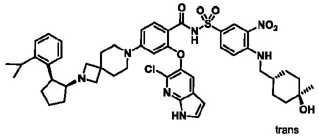 | 2-((6-chloro-1*H*-pyrrolo[2,3-*b*]py ridin-5-yl)oxy)-*N*-((4-((((1*r*,4*r*)-4 -hydroxy-4-methylcyclohexyl)m ethyl)amino)-3-nitrophenyl)sulfo nyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 924 [M + 1]⁺ |
| | or 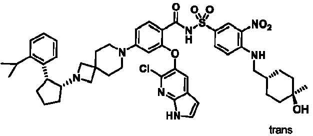 | | |
| D-233 | 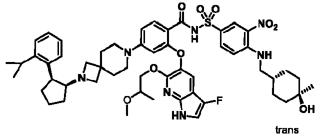 | 2-((3-fluoro-6-(2-methoxypropox y)-1*H*-pyrrolo[2,3-*b*]pyridin-5-yl )oxy)-*N*-((4-((((1*r*,4*r*)-4-hydroxy -4-methylcyclohexyl)methyl)ami no)-3-nitrophenyl)sulfonyl)-4-(2-*((1S,2S* or 1*R*,2*R*)-2-(2-isopropylphenyl)cyc lopentyl)-2,7-diazaspiro[3.5]nona n-7-yl)benzamide | MS-ESI (m/z): 996 [M + 1]⁺ |
| | or 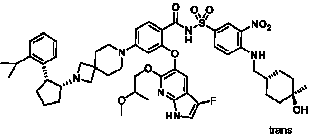 | | |
| D-233a | 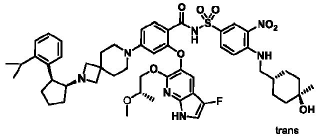 | 2-((3-fluoro-6-((*S*)-2-methoxypro poxy)-1*H*-pyrrolo[2,3-*b*]pyridin-5-yl)oxy)-*N*-((4-((((1*r*,4*r*)-4-hydr oxy-4-methylcyclohexyl)methyl) amino)-3-nitrophenyl)sulfonyl)-4 *-*(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 996 [M + 1]⁺ |
| | or 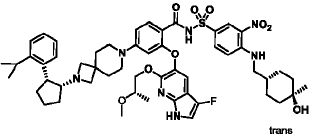 | | |
| D-233b | 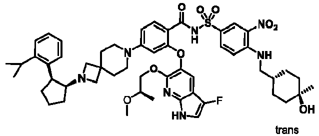 | 2-((3-fluoro-6-((*R*)-2-methoxypr opoxy)-1*H*-pyrrolo[2,3-*b*]pyridin -5-yl)oxy)-*N*-((4-((((1*r*,4*r*)-4-hyd roxy-4-methylcyclohexyl)methyl )amino)-3-nitrophenyl)sulfonyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 996 [M + 1]⁺ |
| | or 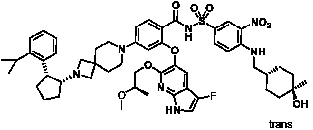 | | |
| D-234 | 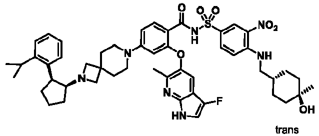 | 2-((3-fluoro-6-methyl-1*H-*pyrrol o[2,3-*b*]pyridin-5-yl)oxy)-*N*-((4-( (((1*r*,4*r*)-4-hydroxy-4-methylcycl ohexyl)methyl)amino)-3-nitroph enyl)sulfonyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 922 [M + 1]⁺ |
| | or 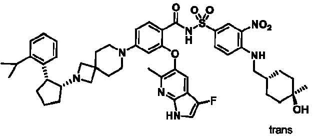 | | |
| D-235 | 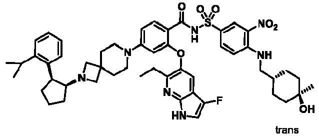 | 2-((6-ethyl-3-fluoro-1*H*-pyrrolo[ 2,3-b]pyridin-5-yl)oxy)-*N*-((4-((( (1*r*,4*r*)-4-hydroxy-4-methylcyclo hexyl)methyl)amino)-3-nitrophe nyl)sulfonyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 936 [M + 1]⁺ |
| | or 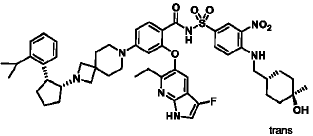 | | |
| D-236 | 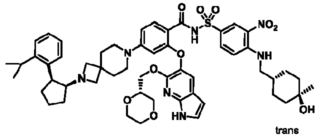 | 2-((6-(((*S*)-1,4-dioxan-2-yl)meth oxy)-1*H*-pyrrolo[2,3-b]pyridin-5-yl)oxy)-*N*-((4-((((1*r*,4*r*)-4-hydrox y-4-methylcyclohexyl)methyl)am ino)-3-nitrophenyl)sulfonyl)-4-(2 -((1*S*,2*S* or 1*R*,2*R*)-2-(2-isopropylphenyl)cyc lopentyl)-2,7-diazaspiro[3.5]nona n-7-yl)benzamide | MS-ESI (m/z): 1006 [M + 1]⁺ |
| | or 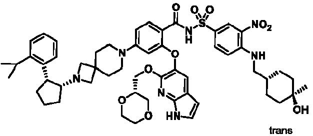 | | |
| D-237 | 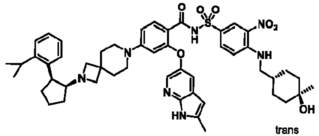 | *N*-((4-((((1*r*,4*r*)-4-hydroxy-4-met hylcyclohexyl)methyl)amino)-3-nitrophenyl)sulfonyl)-4-(2-((1*S*,2 *S* or 1*R*,2*R*)-2-(2-isopropylphenyl)cyc lopentyl)-2,7-diazaspiro[3.5]nona n-7-yl)-2-((2-methyl-1*H*-pyrrolo[ 2,3-*b*]pyridin-5-yl)oxy)benzamide | MS-ESI (m/z): 904 [M + 1]⁺ |
| | or 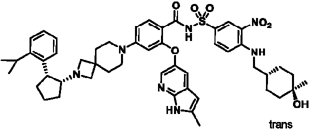 | | |
| D-238 | 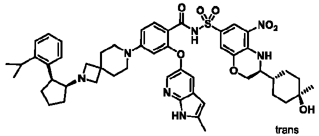 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((1*S*,2*S* or 1*R*,2*R*)-2-(2-isopropylphenyl)cyc lopentyl)-2,7-diazaspiro[3.5]nona n-7-yl)-2-((2-methyl-1*H*-pyrrolo[ 2,3-*b*]pyridin-5-yl)oxy)benzamide | MS-ESI (m/z): 932 [M + 1]⁺ |
| | or 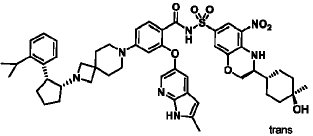 | | |
| D-239 | 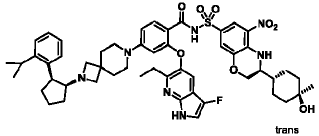 | 2-((6-ethyl-3-fluoro-1*H*-pyrrolo[ 2,3-b]pyridin-5-yl)oxy)-*N*-(((*S*)-3 -((1*r*,4*S*)-4-hydroxy-4-methylcyc lohexyl)-5-nitro-3,4-dihydro-2*H-*benzo[*b*][1,4]oxazin-7-yl)sulfony 1)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 964 [M + 1]⁺ |
| | or 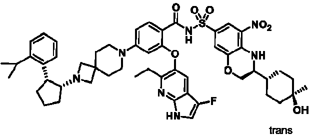 | | |
| D-240 | 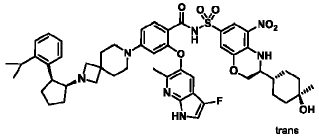 | 2-((3-fluoro-6-methyl-1*H*-pyrrol o[2,3-b]pyridin-5-yl)oxy)-*N*-(((*S*) -3-((1*r*,4*S*)-4-hydroxy-4-methylc yclohexyl)-5-nitro-3,4-dihydro-2 *H*-benzo[*b*][1,4]oxazin-7-yl)sulfo nyl)-4-(2-((1*S*,2*S* or 1*R*,2*R*)-2-(2-isopropylphenyl)cyc lopentyl)-2,7-diazaspiro[3.5]nonan -7-yl)benzamide | MS-ESI (m/z): 950 [M + 1]⁺ |
| | or 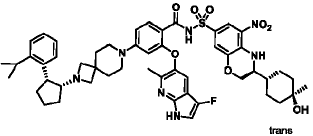 | | |
| D-241 | 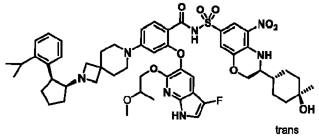 | 2-((3-fluoro-6-(2-methoxypropox y)-1*H*-pyrrolo[2,3-*b*]pyridin-5-yl )oxy)-*N*-(((*S*)-3-((1*r*,4*S*)-4-hydro xy-4-methylcyclohexyl)-5-nitro-3,4-dihydro-2*H*-benzo[b][1,4]oxa zin-7-yl)sulfonyl)-4-(2-((1*S*,2*S* or 1*R*,2*R*)-2-(2-isopropylphenyl)cyc lopentyl)-2,7-diazaspiro[3.5]nonan -7-yl)benzamide | MS-ESI (m/z): 1024 [M + 1]⁺ |
| | or 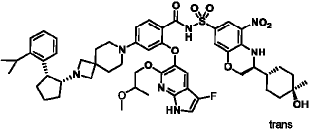 | | |
| D-241a | 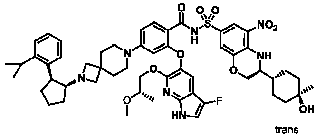 | 2-((3-fluoro-6-((*S*)-2-methoxypro poxy)-1*H*-pyrrolo[2,3-*b*]pyridin-5-yl)oxy)-*N*-(((*S*)-3-((1*r*,4*S*)-4-hy droxy-4-methylcyclohexyl)-5-nit ro-3,4-dihydro-2*H*-benzo[*b*][1,4] oxazin-7-yl)sulfonyl)-4-(2-((1*S*,2 *S* or 1*R*,2*R*)-2-(2-isopropylphenyl)cyc lopentyl)-2,7-diazaspiro[3.5]nona n-7-yl)benzamide | MS-ESI (m/z): 1024 [M + 1]⁺ |
| | or 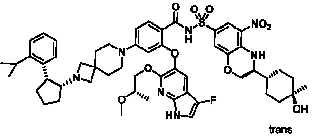 | | |
| D-241b | 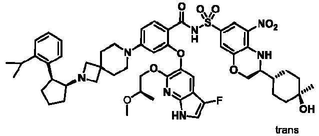 | 2-((3-fluoro-6-((*R*)-2-methoxypr opoxy)-1*H*-pyrrolo[2,3-b]pyridin -5-yl)oxy)-*N*-(((*S*)-3-((1*r*,4*S*)-4-h ydroxy-4-methylcyclohexyl)-5-ni tro-3,4-dihydro-2*H*-benzo[*b*][1,4] oxazin-7-yl)sulfonyl)-4-(2-((1S,2 *S* or 1*R*,2*R*)-2-(2-isopropylphenyl)cyc lopentyl)-2,7-diazaspiro[3.5]nona n-7-yl)benzamide | MS-ESI (m/z): 1024 [M + 1]⁺ |
| | or 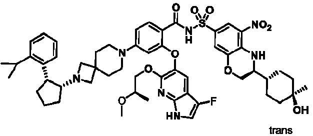 | | |
| D-242 | 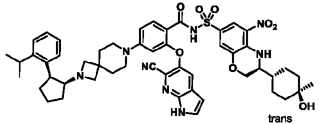 | 2-((6-cyano-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( *1S,2S* or 1*R*,2*R*)-2-(2-isopropylphenyl)cyc lopentyl)-2,7-diazaspiro[3.5]nona n-7-yl)benzamide | MS-ESI (m/z): 943 [M + 1]⁺ |
| | or | | |
| | 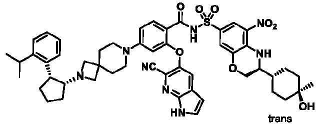 | | |
| D-243 | 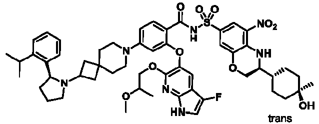 | 2-((3-fluoro-6-(2-methoxypropox y)-1*H*-pyrrolo[2,3-*b*]pyridin-5-yl )oxy)-*N*-(((*S*)-3-((1*r*,4*S*)-4-hydro xy-4-methylcyclohexyl)-5-nitro-3,4-dihydro-2*H*-benzo[*b*][1,4]oxa zin-7-yl)sulfonyl)-4-(2-((*R* or *S*)-2-(2-isopropylphenyl)pyrrolid in-1-yl)-7-azaspiro[3.5]nonan-7-yl)benzamide | MS-ESI (m/z): 1024 [M + 1]⁺ |
| | or | | |
| | 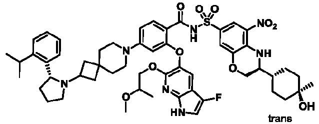 | | |
| D-243a | 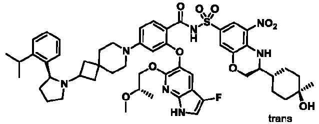 | 2-((3-fluoro-6-((*S*)-2-methoxypropox y)-1*H*-pyrrolo[2,3-b]pyridin-5-yl)oxy) -*N*-(((*S*)-3-((1*r*,4*S*)-4-hydroxy-4-meth ylcyclohexyl)-5-nitro-3,4-dihydro-2*H* -benzo[*b*][1,4]oxazin-7-yl)sulfonyl)-4 -(2-((*R* or *S*)-2-(2-isopropylphenyl)pyrrolidin-1-yl)-7-azaspiro[3.5]nonan-7-yl)benzamide | MS-ESI (m/z): 1024 [M + 1]⁺ |
| | or | | |
| | 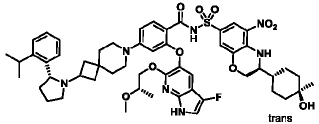 | | |
| D-243b | 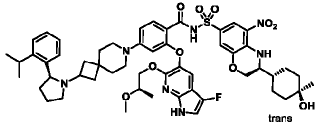 | 2-((3-fluoro-6-((*R*)-2-methoxypropox y)-1*H*-pyrrolo[2,3-b]pyridin-5-yl)oxy) -*N*-(((*S*)-3-((1*r*,4*S*)-4-hydroxy-4-meth ylcyclohexyl)-5-nitro-3,4-dihydro-2*H* -benzo[*b*][1,4]oxazin-7-yl)sulfonyl)-4 -(2-((*R* or *S*)-2-(2-isopropylphenyl)pyrrolidin-1-yl)-7-azaspiro[3.5]nonan-7-yl)benzamide | MS-ESI (m/z): 1024 [M + 1]⁺ |
| | or | | |
| | 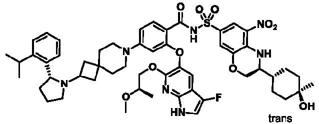 | | |
| D-244 | 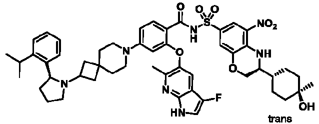 | 2-((3-fluoro-6-methyl-1*H*-pyrrol o[2,3-b]pyridin-5-yl)oxy)-*N*-(((*S*) -3-((1*r*,4*S*)-4-hydroxy-4-methylc yclohexyl)-5-nitro-3,4-dihydro-2 *H*-benzo[*b*][1,4]oxazin-7-yl)sulfo nyl)-4-(2-((*R* or *S*)-2-(2-isopropylphenyl)pyrrolid in-1-yl)-7-azaspiro[3.5]nonan-7-yl)benzamide | MS-ESI (m/z): 950 [M + 1]⁺ |
| | or | | |
| | 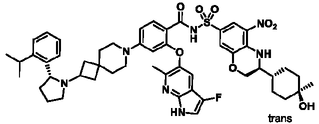 | | |
| D-245 | 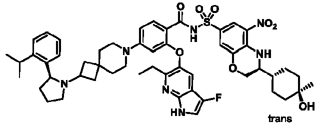 | 2-((6-ethyl-3-fluoro-1*H*-pyrrolo[ 2,3-b]pyridin-5-yl)oxy)-*N*-(((*S*)-3 -((1*r*,4*S*)-4-hydroxy-4-methylcyc lohexyl)-5-nitro-3,4-dihydro-2*H-*benzo[*b*][1,4]oxazin-7-yl)sulfony 1)-4-(2-((*R* or *S*)-2-(2-isopropylphenyl)pyrrolid in-1-yl)-7-azaspiro[3.5]nonan-7-yl)benzamide | MS-ESI (m/z): 964 [M + 1]⁺ |
| | or | | |
| | 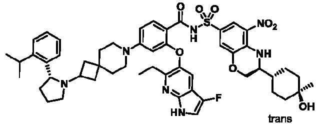 | | |
| D-246 | 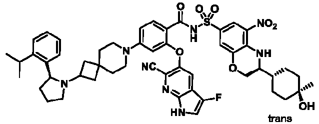 | 2-((6-cyano-3-fluoro-1*H*-pyrrolo[ 2,3-b]pyridin-5-yl)oxy)-*N*-(((*S*)-3 -((1*r*,4*S*)-4-hydroxy-4-methylcyc lohexyl)-5-nitro-3,4-dihydro-2*H-*benzo[*b*][1,4]oxazin-7-yl)sulfony 1)-4-(2-((*R* or *S*)-2-(2-isopropylphenyl)pyrrolid in-1-yl)-7-azaspiro[3.5]nonan-7-yl)benzamide | MS-ESI (m/z): 961 [M + 1]⁺ |
| | or | | |
| | 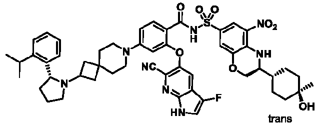 | | |
| D-247 | 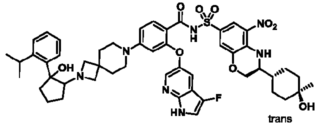 | 2-((3-fluoro-1*H*-pyrrolo[2,3-*b*]py ridin-5-yl)oxy)-4-(2-(2-hydroxy-2-(2-isopropylphenyl)cyclopenty 1)-2,7-diazaspiro[3.5]nonan-7-yl) -*N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)benzamide | MS-ESI (m/z): 952 [M + 1]⁺ |
| D-248 | 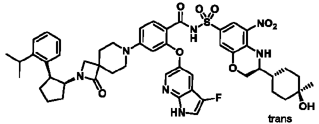 | 2-((3-fluoro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( 1*S,*2*S* or 1*R*,2*R*)-2-(2-isopropylphenyl)cyc lopentyl)-1-oxo-2,7-diazaspiro[3. 5]nonan-7-yl)benzamide | MS-ESI (m/z): 950 [M + 1]⁺ |
| | or | | |
| | 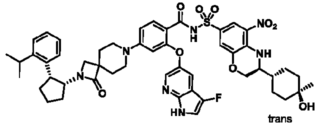 | | |
| D-249 | 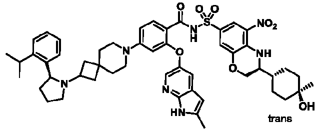 | *N*-(((*S*)-3-((1*r*,4*S*)-4-hydroxy-4-methylcyclohexyl)-5-nitro-3,4-di hydro-2*H*-benzo[*b*][1,4]oxazin-7 -yl)sulfonyl)-4-(2-((*R* or *S*)-2-(2-isopropylphenyl)pyrrolid in-1-yl)-7-azaspiro[3.5]nonan-7-yl)-2-((2-methyl-1*H*-pyrrolo[2,3-*b*]pyridin-5-yl)oxy)benzamide | MS-ESI (m/z): 932 [M + 1]⁺ |
| | or | | |
| | 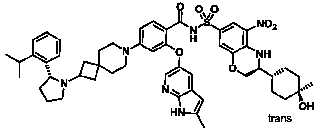 | | |
| D-250 | 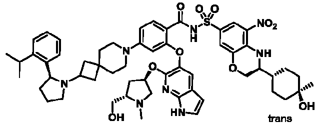 | *N*-(((*S*)-3-((1*r*,4*S*)-4-hydroxy-4-methylcyclohexyl)-5-nitro-3,4-di hydro-2*H*-benzo[b][1,4]oxazin-7 -yl)sulfonyl)-2-((6-(((3*R*,5*S*)-5-(h ydroxymethyl)-1-methylpyrrolidi n-3-yl)oxy)-1*H*-pyrrolo[2,3-*b*]py ridin-5-yl)oxy)-4-(2-((*R* or *S*)-2-(2-isopropylphenyl)pyrrolid in-1-yl)-7-azaspiro[3.5]nonan-7-yl)benzamide | MS-ESI (m/z): 1047 [M + 1]⁺ |
| | or | | |
| | 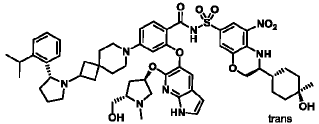 | | |
| D-251 | 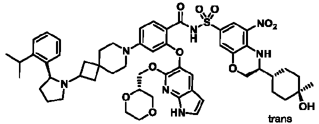 | 2-((6-(((S)-1,4-dioxan-2-yl)meth oxy)-1*H*-pyrrolo[2,3*-b*]pyridin-5-yl)oxy)-*N*-(((*S*)-3-((1*r*,4*S*)-4-hydr oxy-4-methylcyclohexyl)-5-nitro -3,4-dihydro-2*H*-benzo[*b*][1,4]ox azin-7-yl)sulfonyl)-4-(2-((*R* or *S*)-2-(2-isopropylphenyl)pyrrolid in-1-yl)-7-azaspiro[3.5]nonan-7-yl)benzamide | MS-ESI (m/z): 1034 [M + 1]⁺ |
| | or | | |
| | 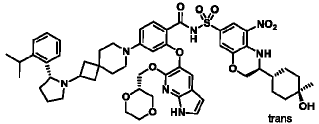 | | |
| D-252 | 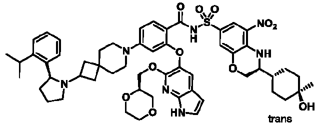 | 2-((6-(((*R*)-1,4-dioxan-2-yl)meth oxy)-1*H*-pyrrolo[2,3-*b*]pyridin-5-yl)oxy)-*N*-(((*S*)-3-((1*r*,4*S*)-4-hydr oxy-4-methylcyclohexyl)-5-nitro -3,4-dihydro-2*H*-benzo[*b*][1,4]ox azin-7-yl)sulfonyl)-4-(2-((*R* or *S*)-2-(2-isopropylphenyl)pyrrolid in-1-yl)-7-azaspiro[3.5]nonan-7-yl)benzamide | MS-ESI (m/z): 1034 [M + 1]⁺ |
| | or | | |
| | 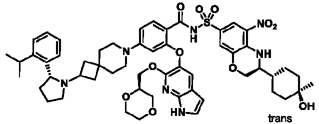 | | |
| D-253 | 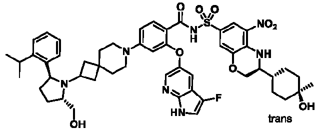 | 2-((3-fluoro-1*H*-pyrrolo[2,3-b]py ridin-5-yl)oxy)-*N*-(((*S*)-3-((1*r*,4*S*) -4-hydroxy-4-methylcyclohexyl)-5-nitro-3,4-dihydro-2*H*-benzo[b] [1,4]oxazin-7-yl)sulfonyl)-4-(2-(( 2*S*,5*S*)-2-(hydroxymethyl)-5-(2-i sopropylphenyl)pyrrolidin-1-yl)-7-azaspiro[3.5]nonan-7-yl)benza mide | MS-ESI (m/z): 966 [M + 1]⁺ |
| D-254 | 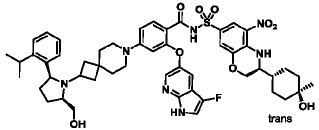 | 2-((3-fluoro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2H-benzo[b] [1,4]oxazin-7-yl)sulfonyl)-4-(2-(( 2*R*,5*S*)-2-(hydroxymethyl)-5-(2-i sopropylphenyl)pyrrolidin-1-yl)-7-azaspiro[3.5]nonan-7-yl)benza mide | MS-ESI (m/z): 966 [M + 1]⁺ |
| D-255 | 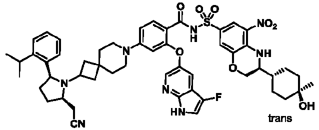 | 4-(2-((2*R*,5*S*)-2-(cyanomethyl)-5 -(2-isopropylphenyl)pyrrolidin-1 -yl)-7-azaspiro[3.5]nonan-7-yl)-2 -((3-fluoro-1*H*-pyrrolo[2,3-*b*]pyri din-5-yl)oxy)-*N*-(((*S*)-3-((1*r*,4*S*)-4-hydroxy-4-methylcyclohexyl)-5-nitro-3,4-dihydro-2*H*-benzo[*b*] [1,4]oxazin-7-yl)sulfonyl)benza mide | MS-ESI (m/z): 975 [M + 1]⁺ |
| D-256 | 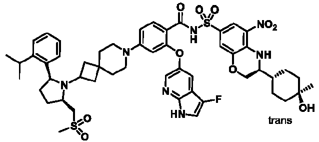 | 2-((3-fluoro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( 2*S*,5*R*)-2-(2-isopropylphenyl)-5-( (methylsulfonyl)methyl)pyrrolidi n-1-yl)-7-azaspiro[3.5]nonan-7-y l)benzamide | MS-ESI (m/z): 1028 [M + 1]⁺ |
| D-257 | 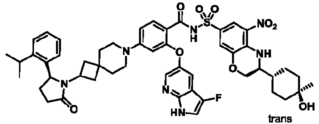 | 2-((3-fluoro-1*H*-pyrrolo[2,3-*b*]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( *S*)-2-(2-isopropylphenyl)-5-oxop yrrolidin-1-yl)-7-azaspiro[3.5]no nan-7-yl)benzamide | MS-ESI (m/z): 950 [M + 1]⁺ |
| D-258 | 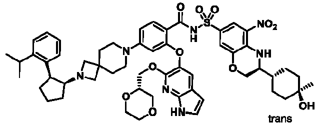 | 2-((6-(((*S*)-1,4-dioxan-2-yl)meth oxy)-1H-pyrrolo[2,3-b]pyridin-5-yl)oxy)-*N*-(((*S*)-3-((1*r*,4*S*)-4-hydr oxy-4-methylcyclohexyl)-5-nitro -3,4-dihydro-2*H*-benzo[*b*][1,4]ox azin-7-yl)sulfonyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 1034 [M + 1]⁺ |
| | or | | |
| | 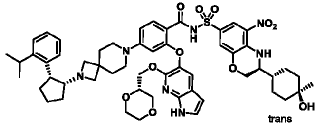 | | |
| D-259 | 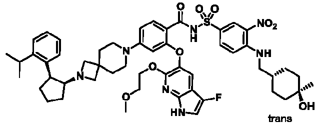 | 2-((3-fluoro-6-(2-methoxyethoxy )-1H-pyrrolo[2,3-*b*]pyridin-5-yl) oxy)-*N*-((4-((((1*r*,4*r*)-4-hydroxy-4-methylcyclohexyl)methyl)amin o)-3-nitrophenyl)sulfonyl)-4-(2-( (1*S*,2*S* or 1*R*,2*R*)-2-(2-isopropylphenyl)cyc lopentyl)-2,7-diazaspiro[3.5]nona n-7-yl)benzamide | MS-ESI (m/z): 982 [M + 1]⁺ |
| | or | | |
| | 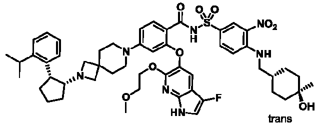 | | |
| D-260 | 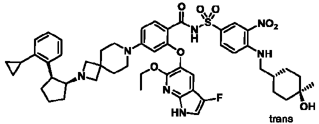 | 4-(2-((1*S*,2*S* or 1*R*,2*R*)-2-(2-cyclopropylphenyl)c yclopentyl)-2,7-diazaspiro[3.5]no nan-7-yl)-2-((6-ethoxy-3-fluoro-1H-pyrrolo[2,3-*b*]pyridin-5-yl)ox y)-N-((4-((((1*r*,4*r*)-4-hydroxy-4-methylcyclohexyl)methyl)amino) -3-nitrophenyl)sulfonyl)benzami de | MS-ESI (m/z): 950 [M + 1]⁺ |
| | or | | |
| | 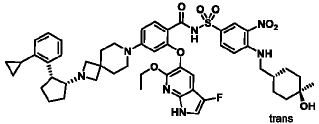 | | |
| D-261 | 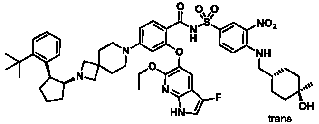 | 4-(2-((1*S*,2*S* or 1*R*,2*R*)-2-(2-(tert-butyl)phenyl)c yclopentyl)-2,7-diazaspiro[3.5]no nan-7-yl)-2-((6-ethoxy-3-fluoro-1H-pyrrolo[2,3-b]pyridin-5-yl)ox y)-*N*-((4-((((1*r*,4*r*)-4-hydroxy-4-methylcyclohexyl)methyl)amino) -3-nitrophenyl)sulfonyl)benzami de | MS-ESI (m/z): 966 [M + 1]⁺ |
| | or | | |
| | 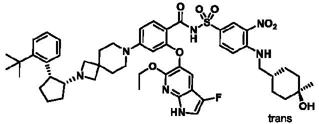 | | |
| D-262 | 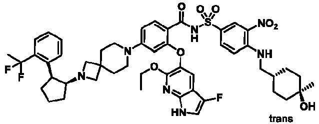 | 4-(2-((1*S*,2S or 1*R*,2*R*)-2-(2-(1,1-difluoroethyl)p henyl)cyclopentyl)-2,7-diazaspir o[3.5]nonan-7-yl)-2-((6-ethoxy-3 -fluoro-1H-pyrrolo[2,3-b]pyridin -5-yl)oxy)-*N*-((4-((((1*r*,4*r*)-4-hyd roxy-4-methylcyclohexyl)methyl )amino)-3-nitrophenyl)sulfonyl)b enzamide | MS-ESI (m/z): 974 [M + 1]⁺ |
| | or | | |
| | 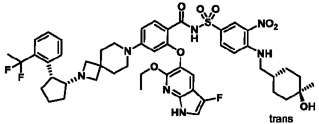 | | |
| D-263 | 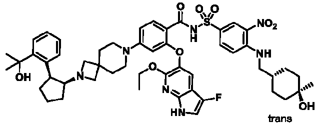 | 2-((6-ethoxy-3-fluoro-1*H*-pyrrolo [2,3-*b*]pyridin-5-yl)oxy)-*N*-((4-(( ((1*r*,4*r*)-4-hydroxy-4-methylcycl ohexyl)methyl)amino)-3-nitroph enyl)sulfonyl)-4-(2-((1*S*,2*S* or 1*R*,2*R*)-2-(2-(2-hydroxypropan-2 -yl)phenyl)cyclopentyl)-2,7-diaz aspiro[3.5]nonan-7-yl)benzamide | MS-ESI (m/z): 968 [M + 1]⁺ |
| | or | | |
| | 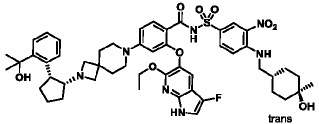 | | |
| D-264 | 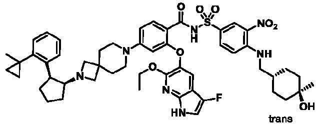 | 2-((6-ethoxy-3-fluoro-1*H*-pyrrolo [2,3-*b*]pyridin-5-yl)oxy)-*N*-((4-(( ((1*r*,4*r*)-4-hydroxy-4-methylcycl ohexyl)methyl)amino)-3-nitroph enyl)sulfonyl)-4-(2-((1*S*,2*S* or 1*R*,2*R*)-2-(2-(1-methylcycloprop yl)phenyl)cyclopentyl)-2,7-diaza spiro[3.5]nonan-7-yl)benzamide | MS-ESI (m/z): 964 [M + 1]⁺ |
| | or | | |
| | 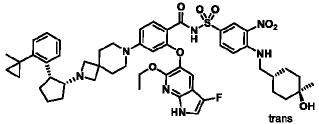 | | |
| D-265 | 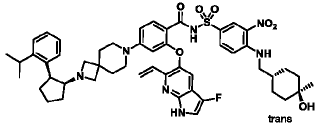 | 2-((3-fluoro-6-vinyl-1*H*-pyrrolo[ 2,3-*b*]pyridin-5-yl)oxy)-*N*-((4-((( (1*r*,4*r*)-4-hydroxy-4-methylcyclo hexyl)methyl)amino)-3-nitrophe nyl)sulfonyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 934 [M + 1]⁺ |
| | or | | |
| | 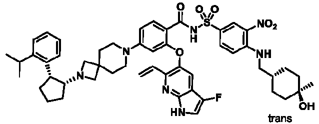 | | |
| D-266 | 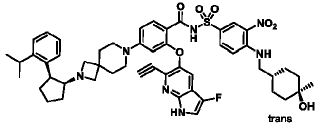 | 2-((6-ethynyl-3-fluoro-1*H*-pyrrol o[2,3-*b*]pyridin-5-yl)oxy)-*N*-((4-( (((1*r*,4*r*)-4-hydroxy-4-methylcycl ohexyl)methyl)amino)-3-nitroph enyl)sulfonyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 932 [M + 1]⁺ |
| | or | | |
| | 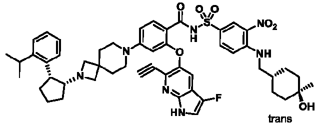 | | |
| D-267 | 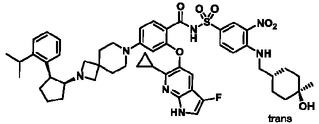 | 2-((6-cyclopropyl-3-fluoro-1*H*-p yrrolo[2,3-*b*]pyridin-5-yl)oxy)-*N* -((4-((((1*r*,4*r*)-4-hydroxy-4-meth ylcyclohexyl)methyl)amino)-3-ni trophenyl)sulfonyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 948 [M + 1]⁺ |
| | or | | |
| | 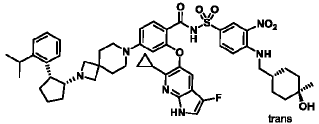 | | |
| D-268 | 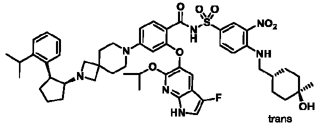 | 2-((3-fluoro-6-isopropoxy-1*H*-py rrolo[2,3-*b*]pyridin-5-yl)oxy)-*N*-( (4-((((1*r*,4*r*)-4-hydroxy-4-methyl cyclohexyl)methyl)amino)-3-nitr ophenyl)sulfonyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 966 [M + 1]⁺ |
| | or | | |
| | 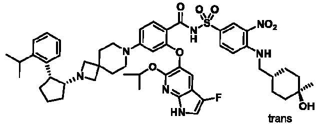 | | |
| D-269 | 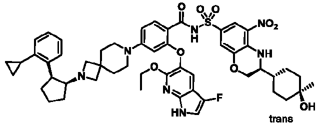 | 4-(2-((1*S*,2*S* or 1*R*,2*R*)-2-(2-cyclopropylphenyl)c yclopentyl)-2,7-diazaspiro[3.5]no nan-7-yl)-2-((6-ethoxy-3-fluoro-1H-pyrrolo[2,3-b]pyridin-5-yl)ox y)-*N*-(((*S*)-3-((1*r*,4*S*)-4-hydroxy-4-methylcyclohexyl)-5-nitro-3,4-dihydro-2*H*-benzo[*b*][1,4]oxazin-7-yl)sulfonyl)benzamide | MS-ESI (m/z): 978 [M + 1]⁺ |
| | or | | |
| | 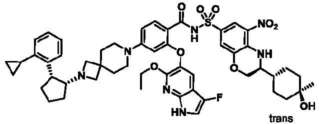 | | |
| D-270 | 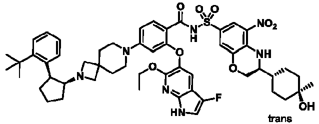 | 4-(2-((1*S*,2*S* or 1*R*,2*R*)-2-(2-(tert-butyl)phenyl)c yclopentyl)-2,7-diazaspiro[3.5]no nan-7-yl)-2-((6-ethoxy-3-fluoro-1H-pyrrolo[2,3-b]pyridin-5-yl)ox y)-*N*-(((*S*)-3-((1*r*,4*S*)-4-hydroxy-4-methylcyclohexyl)-5-nitro-3,4-dihydro-2*H*-benzo[*b*][1,4]oxazin-7-yl)sulfonyl)benzamide | MS-ESI (m/z): 994 [M + 1]⁺ |
| | or | | |
| | 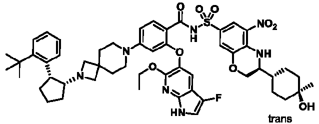 | | |
| D-271 | 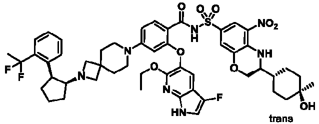 | 4-(2-((1*S*,2*S* or 1*R*,2*R*)-2-(2-(1,1-difluoroethyl)p henyl)cyclopentyl)-2,7-diazaspir o[3.5]nonan-7-yl)-2-((6-ethoxy-3 -fluoro-1*H*-pyrrolo[2,3-*b*]pyridin -5-yl)oxy)-*N*-(((*S*)-3-((1*r*,4*S*)-4-h ydroxy-4-methylcyclohexyl)-5-ni tro-3,4-dihydro-2*H*-benzo[*b*][1,4] oxazin-7-yl)sulfonyl)benzamide | MS-ESI (m/z): 1002 [M + 1]⁺ |
| | or | | |
| | 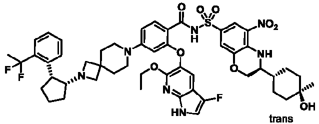 | | |
| D-272 | 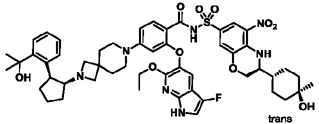 | 2-((6-ethoxy-3-fluoro-1*H*-pyrrolo [2,3-*b*]pyridin-5-yl)oxy)-*N*-(((*S*)-3-((1*r*,4*S*)-4-hydroxy-4-methylcy clohexyl)-5-nitro-3,4-dihydro-2*H* -benzo[*b*][1,4]oxazin-7-yl)sulfon yl)-4-(2-((1*S*,2*S* or 1*R*,2*R*)-2-(2-(2-hydroxypropan-2 -yl)phenyl)cyclopentyl)-2,7-diaz aspiro[3.5]nonan-7-yl)benzamide | MS-ESI (m/z): 996 [M + 1]⁺ |
| | or | | |
| | 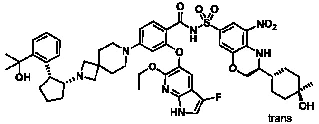 | | |
| D-273 | 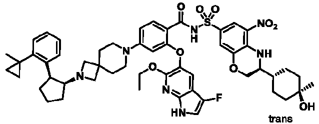 | 2-((6-ethoxy-3-fluoro-1*H*-pyrrolo [2,3-b]pyridin-5-yl)oxy)-*N*-(((*S*)-3-((1*r*,4*S*)-4-hydroxy-4-methylcy clohexyl)-5-nitro-3,4-dihydro-2*H* -benzo[*b*][1,4]oxazin-7-yl)sulfon yl)-4-(2-((1S,2S or 1*R*,2*R*)-2-(2-(1-methylcycloprop yl)phenyl)cyclopentyl)-2,7-diaza spiro[3.5]nonan-7-yl)benzamide | MS-ESI (m/z): 992 [M + 1]⁺ |
| | or | | |
| | 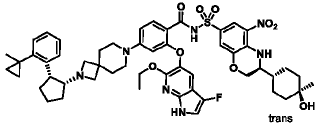 | | |
| D-274 | 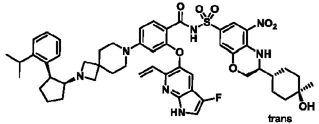 | 2-((3-fluoro-6-vinyl-1*H*-pyrrolo[ 2,3-b]pyridin-5-yl)oxy)-*N*-(((*S*)-3 -((1*r*,4*S*)-4-hydroxy-4-methylcyc lohexyl)-5-nitro-3,4-dihydro-2*H-*benzo[*b*][1,4]oxazin-7-yl)sulfony 1)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 962 [M + 1]⁺ |
| | or | | |
| | 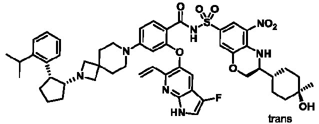 | | |
| D-275 | 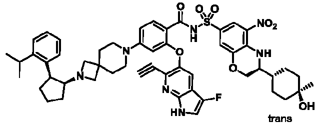 | 2-((6-ethynyl-3-fluoro-1*H*-pyrrol o[2,3-*b*]pyridin-5-yl)oxy)-*N*-(((*S*) -3-((1*r*,4*S*)-4-hydroxy-4-methylc yclohexyl)-5-nitro-3,4-dihydro-2 *H*-benzo[*b*] 1,4]oxazin-7-yl)sulfo nyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 960 [M + 1]⁺ |
| | or | | |
| | 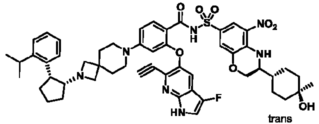 | | |
| D-276 | 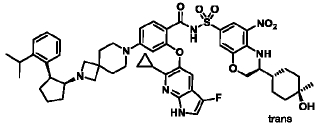 | 2-((6-cyclopropyl-3-fluoro-1*H*-p yrrolo[2,3-*b*]pyridin-5-yl)oxy)-*N* -(((*S*)-3-((1*r*,4*S*)-4-hydroxy-4-me thylcyclohexyl)-5-nitro-3,4-dihy dro-2*H*-benzo[*b*][1,4]oxazin-7-yl )sulfonyl)-4-(2-((1S,2S or 1*R*,2*R*)-2-(2-isopropylphenyl)cyc lopentyl)-2,7-diazaspiro[3.5]nona n-7-yl)benzamide | MS-ESI (m/z): 976 [M + 1]⁺ |
| | or | | |
| | 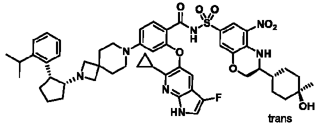 | | |
| D-277 | 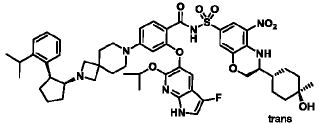 | 2-((3-fluoro-6-isopropoxy-1*H*-py rrolo[2,3-*b*]pyridin-5-yl)oxy)-*N*-( ((*S*)-3-((1*r*,4*S*)-4-hydroxy-4-meth ylcyclohexyl)-5-nitro-3,4-dihydr o-2*H*-benzo[*b*][1,4]oxazin-7-yl)s ulfonyl)-4-(2-((1*S*,2*S* or 1*R*,2*R*)-2-(2-isopropylphenyl)cyc lopentyl)-2,7-diazaspiro[3.5]nona n-7-yl)benzamide | MS-ESI (m/z): 994 [M + 1]⁺ |
| | or | | |
| | 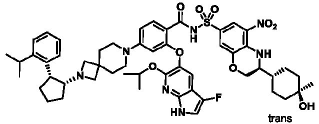 | | |
| D-278 | 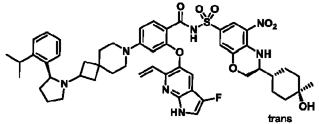 | 2-((3-fluoro-6-vinyl-1*H*-pyrrolo[ 2,3-*b*]pyridin-5-yl)oxy)-*N*-(((*S*)-3 -((1*r*,4*S*)-4-hydroxy-4-methylcyc lohexyl)-5-nitro-3,4-dihydro-2*H-*benzo[b] [1,4]oxazin-7-yl)sulfony l)-4-(2-((*R* or *S*)-2-(2-isopropylphenyl)pyrrolid in-1-yl)-7-azaspiro[3.5]nonan-7-yl)benzamide | MS-ESI (m/z): 962 [M + 1]⁺ |
| | or | | |
| | 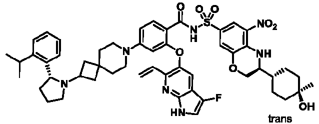 | | |
| D-279 | 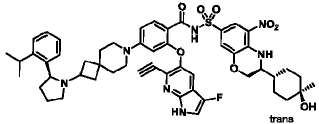 | 2-((6-ethynyl-3-fluoro-1*H*-pyrrol o[2,3-b]pyridin-5-yl)oxy)-*N*-(((*S*) -3-((1*r*,4*S*)-4-hydroxy-4-methylc yclohexyl)-5-nitro-3,4-dihydro-2 *H*-benzo[*b*][1,4]oxazin-7-yl)sulfo nyl)-4-(2-((*R* or *S*)-2-(2-isopropylphenyl)pyrrolid in-1-yl)-7-azaspiro[3.5]nonan-7-yl)benzamide | MS-ESI (m/z): 960 [M + 1]⁺ |
| | or | | |
| | 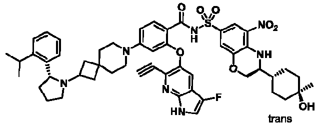 | | |
| D-280 | 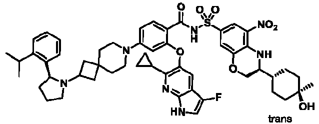 | 2-((6-cyclopropyl-3-fluoro-1*H*-p yrrolo[2,3-*b*]pyridin-5-yl)oxy)-*N* -(((*S*)-3-((1*r*,4*S*)-4-hydroxy-4-me thylcyclohexyl)-5-nitro-3,4-dihy dro-2*H*-benzo[*b*][1,4]oxazin-7-yl )sulfonyl)-4-(2-((R or *S*)-2-(2-isopropylphenyl)pyrrolid in-1-yl)-7-azaspiro[3.5]nonan-7-yl)benzamide | MS-ESI (m/z): 976 [M + 1]⁺ |
| | or | | |
| | 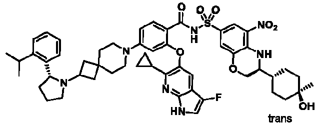 | | |
| D-281 | 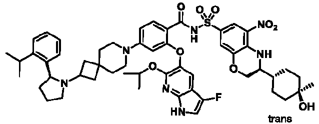 | 2-((3-fluoro-6-isopropoxy-1*H*-py rrolo[2,3-*b*]pyridin-5-yl)oxy)-*N*-( ((*S*)-3-((1*r*,4*S*)-4-hydroxy-4-meth ylcyclohexyl)-5-nitro-3,4-dihydr o-2*H*-benzo[*b*][1,4]oxazin-7-yl)s ulfonyl)-4-(2-((*R* or *S*)-2-(2-isopropylphenyl)pyrrolid in-1-yl)-7-azaspiro[3.5]nonan-7-yl)benzamide | MS-ESI (m/z): 994 [M + 1]⁺ |
| | or | | |
| | 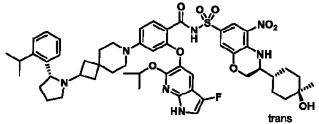 | | |
| D-282 | 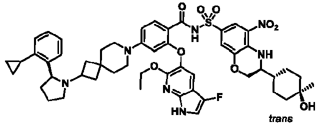 | 4-(2-((*R* or *S*)-2-(2-cyclopropylphenyl)pyrrol idin-1-yl)-7-azaspiro[3.5]nonan-7-yl)-2-((6-ethoxy-3-fluoro-1H-p yrrolo[2,3-*b*]pyridin-5-yl)oxy)-*N* -(((*S*)-3-((1*r*,4*S*)-4-hydroxy-4-me thylcyclohexyl)-5-nitro-3,4-dihy dro-2*H*-benzo[*b*][1,4]oxazin-7-yl )sulfonyl)benzamide | MS-ESI (m/z): 978 [M + 1]⁺ |
| | or | | |
| | 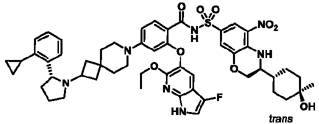 | | |
| D-283 | 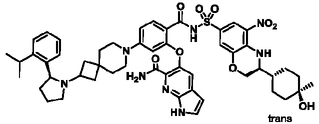 | 5-(2-((((*S*)-3-((1*r*,4*S*)-4-hydroxy-4-methylcyclohexyl)-5-nitro-3,4-dihydro-2*H*-benzo[*b*][1,4]oxazin-7-yl)sulfonyl)carbamoyl)-5-(2-(( *R* or *S*)-2-(2-isopropylphenyl)pyrrolid in-1-yl)-7-azaspiro[3.5]nonan-7-yl)phenoxy)-1*H*-pyrrolo[2,3-*b*]py ridine-6-carboxamide | MS-ESI (m/z): 961 [M + 1]⁺ |
| | or | | |
| | 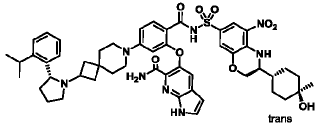 | | |
| D-284 | 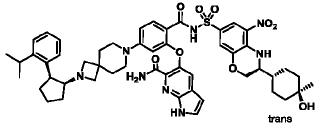 | 5-(2-((((*S*)-3-((1*r*,4*S*)-4-hydroxy-4-methylcyclohexyl)-5-nitro-3,4-dihydro-2*H*-benzo[*b*][1,4]oxazin-7-yl)sulfonyl)carbamoyl)-5-(2-(( 1*S*,2*S* or 1*R*,2*R*)-2-(2-isopropylphenyl)cyc lopentyl)-2,7-diazaspiro[3.5]nona n-7-yl)phenoxy)-1*H*-pyrrolo[2,3-*b*]pyridine-6-carboxamide | MS-ESI (m/z): 961 [M + 1]⁺ |
| | or | | |
| | 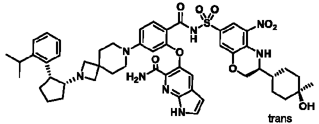 | | |
| D-285 | 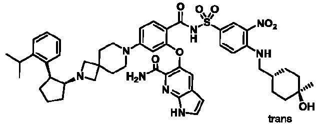 | 5-(2-(((4-((((1*r*,4r)-4-hydroxy-4-methylcyclohexyl)methyl)amino) -3-nitrophenyl)sulfonyl)carbamo yl)-5-(2-((1*S*,2*S* or 1*R*,2*R*)-2-(2-isopropylphenyl)cyc lopentyl)-2,7-diazaspiro[3.5]nona n-7-yl)phenoxy)-1*H*-pyrrolo[2,3-*b*]pyridine-6-carboxamide | MS-ESI (m/z): 933 [M + 1]⁺ |
| | or | | |
| | 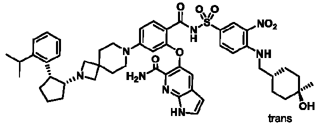 | | |
| D-286 | 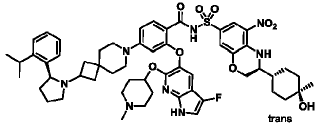 | 2-((3-fluoro-6-((1-methylpiperidi n-4-yl)oxy)-1*H*-pyrrolo[2,3-b]py ridin-5-yl)oxy)-*N*-(((*S*)-3-((1*r*,4*S*) -4-hydroxy-4-methylcyclohexyl)-5-nitro-3,4-dihydro-2*H*-benzo[*b*] [1,4]oxazin-7-yl)sulfonyl)-4-(2-(( *R* or *S*)-2-(2-isopropylphenyl)pyrrolid in-1-yl)-7-azaspiro[3.5]nonan-7-yl)benzamide | MS-ESI (m/z): 1049 [M + 1]⁺ |
| | or | | |
| | 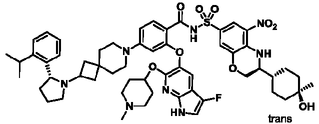 | | |

Examples E-1 ~ E-54 listed in Table 6 were prepared using appropriate intermediates, which can be readily synthesized by methods known in the literature or may be prepared by a variety of methods familiar to those skilled in the art. The structures and names of Reference Examples E-1 ~ E-54 are given in table 6.

**Table 6**

| EXAMPLE | STRUCTURE | NAME | DATA |
|---|---|---|---|
| E-1 | 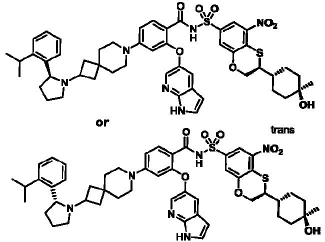 | 2-((1*H*-pyrrolo[2,3-*b*]pyridin-5 *-*yl)oxy)-*N*-(((*S*)-3-((1*r*,4*S*)-4-h ydroxy-4-methylcyclohexyl)-5-nitro-2,3-dihydrobenzo[*b*][1,4] oxathiin-7-yl)sulfonyl)-4-(2-((R or S)-2-(2-isopropylphenyl)pyrroli din-1-yl)-7-azaspiro[3.5]nonan-7-yl)benzamide | MS-ESI (m/z): 935 [M + 1]⁺ |
| E-2 | 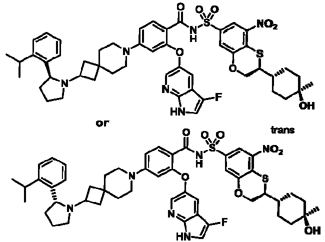 | 2-((3-fluoro-1H-pyrrolo[2,3-b] pyridin-5-yl)oxy)-N-(((S)-3-((1 *r*,4*S*)-4-hydroxy-4-methylcyclo hexyl)-5-nitro-2,3-dihydrobenz o[b][1,4]oxathiin-7-yl)sulfonyl) -4-(2-((R or S)-2-(2-isopropylphenyl)pyrroli din-1-yl)-7-azaspiro[3.5]nonan-7-yl)benzamide | MS-ESI (m/z): 953 [M + 1]⁺ |
| E-3 | 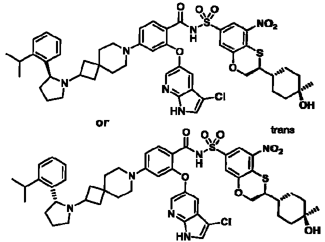 | 2-((3-chloro-1H-pyrrolo[2,3-b] pyridin-5-yl)oxy)-N-(((S)-3-((1 *r*,4*S*)-4-hydroxy-4-methylcyclo hexyl)-5-nitro-2,3-dihydrobenz o[*b*][1,4]oxathiin-7-yl)sulfonyl) -4-(2-((R or S)-2-(2-isopropylphenyl)pyrroli din-1-yl)-7-azaspiro[3.5]nonan-7-vl)benzamide | MS-ESI (m/z): 969 [M + 1]⁺ |
| E-4 | 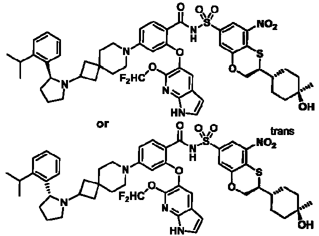 | 2-((6-(difluoromethoxy)-1H-py rrolo[2,3-b]pyridin-5-yl)oxy)-*N* -(((*S*)-3-((1*r*,4*S*)-4-hydroxy-4-methylcyclohexyl)-5-nitro-2,3-dihydrobenzo[*b*][1,4]oxathiin-7 -yl)sulfonyl)-4-(2-((*R* or S)-2-(2-isopropylphenyl)pyrroli din-1-yl)-7-azaspiro[3.5]nonan-7-yl)benzamide | MS-ESI (m/z): 1001 [M + 1]⁺ |
| E-5 | 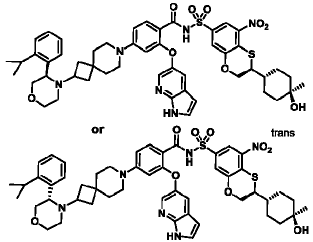 | 2-((1*H*-pyrrolo[2,3-*b*]pyridin-5 -yl)oxy)-4-(2-((R or S)-3-(2-isopropylphenyl)morph olino)-7-azaspiro[3.5]nonan-7-yl)-N-(((*S*)-3-((1*r*,4*S*)-4-hydrox y-4-methylcyclohexyl)-5-nitro-2,3-dihydrobenzo[b][1,4]oxathi in-7-yl)sulfonyl)benzamide | MS-ESI (m/z): 951 [M + 1]⁺ |
| E-6 | 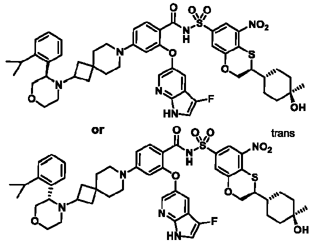 | 2-((3-fluoro-1*H*-pyrrolo[2,3-*b*] pyridin-5-yl)oxy)-N-(((S)-3-((1 *r*,4*S*)-4-hydroxy-4-methylcyclo hexyl)-5-nitro-2,3-dihydrobenzo [*b*][1,4]oxathiin-7-yl)sulfonyl) -4-(2-((R or S)-3-(2-isopropylphenyl)morph olino)-7-azaspiro[3.5]nonan-7-yl)benzamide | MS-ESI (m/z): 969 [M + 1]⁺ |
| E-7 | 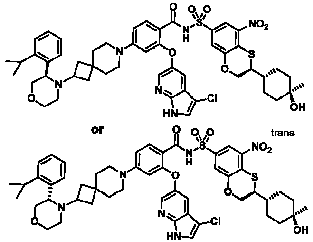 | 2-((3-chloro-1*H*-pyrrolo[2,3*-b*] pyridin-5-yl)oxy)-*N*-(((*S*)-3-((1 *r*,4*S*)-4-hydroxy-4-methylcyclo hexyl)-5-nitro-2,3-dihydrobenz o[b][1,4]oxathiin-7-yl)sulfonyl) -4-(2-((*R* or *S*)-3-(2-isopropylphenyl)morph olino)-7-azaspiro[3.5]nonan-7-yl)benzamide | MS-ESI (m/z): 985 [M + 1]⁺ |
| E-8 | 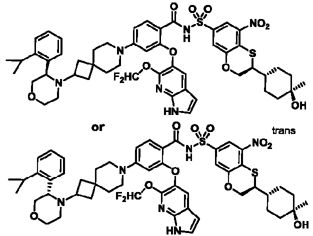 | 2-((6-(difluoromethoxy)-1*H*-py rrolo[2,3-b]pyridin-5-yl)oxy)-*N* -(((*S*)-3-((1*r*,4*S*)-4-hydroxy-4-methylcyclohexyl)-5-nitro-2,3-dihydrobenzo[*b*][1,4]oxathiin-7 -yl)sulfonyl)-4-(2-((R or S)-3-(2-isopropylphenyl)morph olino)-7-azaspiro[3.5]nonan-7-yl)benzamide | MS-ESI (m/z): 1017 [M + 1]⁺ |
| E-9 | 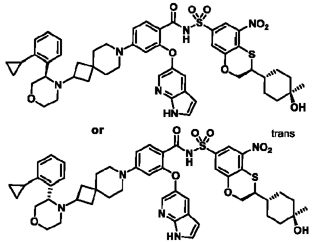 | 2-((1*H*-pyrrolo[2,3-*b*]pyridin-5 -yl)oxy)-4-(2-((R or S)-3-(2-cyclopropylphenyl)mor pholino)-7-azaspiro[3.5]nonan-7-yl)-*N*-(((*S*)-3-((1*r*,4*S*)-4-hydr oxy-4-methylcyclohexyl)-5-nitr o-2,3-dihydrobenzo[*b*][1,4]oxat hiin-7-yl)sulfonyl)benzamide | MS-ESI (m/z): 949 [M + 1]⁺ |
| E-10 | 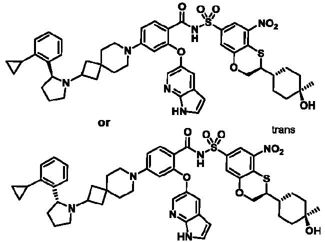 | 2-((1*H*-pyrrolo[2,3-*b*]pyridin-5 -yl)oxy)-4-(2-((*R* or *S*)-2-(2-cyclopropylphenyl)pyrr olidin-1-yl)-7-azaspiro[3.5]non an-7-yl)-*N*-(((*S*)-3-((1*r*,4*S*)-4-h ydroxy-4-methylcyclohexyl)-5-nitro-2,3-dihydrobenzo[*b*][1,4] oxathiin-7-yl)sulfonyl)benzamide | MS-ESI (m/z): 933 [M + 1]⁺ |
| E-11 | 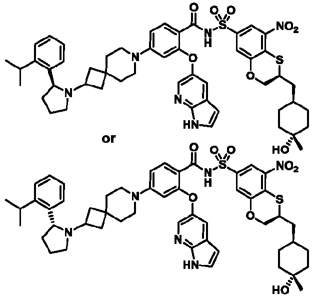 | 2-((1*H*-pyrrolo[2,3-*b*]pyridin-5 -yl)oxy)-*N*-(((*S*)-3-(((1*r*,4*S*)-4-h ydroxy-4-methylcyclohexyl)me thyl)-5-nitro-2,3-dihydrobenzo[ *b*][1,4]oxathiin-7-yl)sulfonyl)-4 -(2-((*R* or *S*)-2-(2-isopropylphenyl)pyrroli din-1-yl)-7-azaspiro[3.5]nonan-7-yl)benzamide | MS-ESI (m/z): 949 [M + 1]⁺ |
| E-12 | 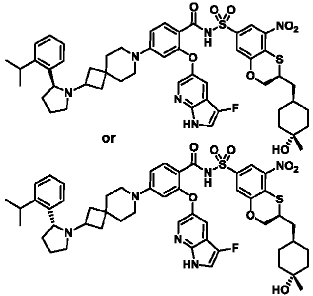 | 2-((3-fluoro-1*H*-pyrrolo[2,3-*b*] pyridin-5-yl)oxy)-*N*-(((*S*)-3-((( 1*r*,4*S*)-4-hydroxy-4-methylcycl ohexyl)methyl)-5-nitro-2,3-dih ydrobenzo[*b*][1,4]oxathiin-7-yl )sulfonyl)-4-(2-((*R* or *S*)-2-(2-isopropylphenyl)pyrroli din-1-yl)-7-azaspiro[3.5]nonan-7-yl)benzamide | MS-ESI (m/z): 967 [M + 1]⁺ |
| E-13 | 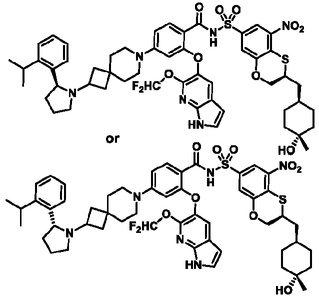 | 2-((6-(difluoromethoxy)-1*H*-py rrolo[2,3-b]pyridin-5-yl)oxy)-*N* -(((*S*)-3-(((1*r*,4*S*)-4-hydroxy-4-methylcyclohexyl)methyl)-5-ni tro-2,3-dihydrobenzo[*b*][1,4]ox athiin-7-yl)sulfonyl)-4-(2-((*R* or *S*)-2-(2-isopropylphenyl)pyrroli din-1-yl)-7-azaspiro[3.5]nonan-7-yl)benzamide | MS-ESI (m/z): 1015 [M + 1]⁺ |
| E-14 | 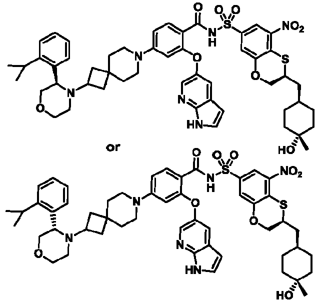 | 2-((1*H*-pyrrolo[2,3-*b*]pyridin-5 -yl)oxy)-*N*-(((*S*)-3-((4-hydroxy-4-methylcyclohexyl)methyl)-5-nitro-2,3-dihydrobenzo[*b*][1,4] oxathiin-7-yl)sulfonyl)-4-(2-((*R* or *S*)-3-(2-isopropylphenyl)morph olino)-7-azaspiro[3.5]nonan-7-yl)benzamide | MS-ESI (m/z): 965 [M + 1]⁺ |
| E-15 | 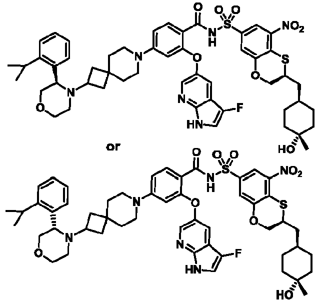 | 2-((3-fluoro-1*H*-pyrrolo[2,3-*b*] pyridin-5-yl)oxy)-*N*-(((*S*)-3-((( 1*r*,4*S*)-4-hydroxy-4-methylcycl ohexyl)methyl)-5-nitro-2,3-dih ydrobenzo[*b*][1,4]oxathiin-7-yl )sulfonyl)-4-(2-((*R* or *S*)-3-(2-isopropylphenyl)morph olino)-7-azaspiro[3.5]nonan-7-yl)benzamide | MS-ESI (m/z): 983 [M + 1]⁺ |
| E-16 | 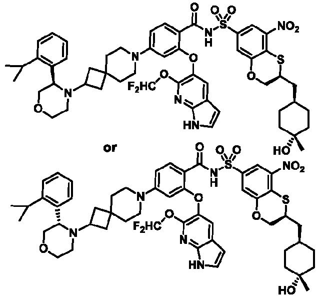 | 2-((6-(difluoromethoxy)-1*H*-py rrolo[2,3-b]pyridin-5-yl)oxy)-*N* -(((*S*)-3-(((1*r*,4*S*)-4-hydroxy-4-methylcyclohexyl)methyl)-5-ni tro-2,3-dihydrobenzo[*b*][1,4]ox athiin-7-yl)sulfonyl)-4-(2-((*R* or *S*)-3-(2-isopropylphenyl)morph olino)-7-azaspiro[3.5]nonan-7-yl)benzamide | MS-ESI (m/z): 1031 [M + 1]⁺ |
| E-17 | 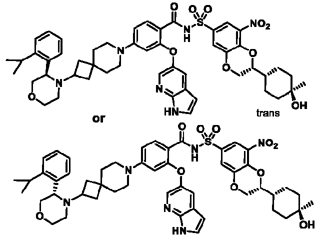 | 2-((1*H*-pyrrolo[2,3-*b*]pyridin-5 -yl)oxy)-*N*-(((*R*)-2-((1*r*,4*R*)-4-h ydroxy-4-methylcyclohexyl)-8-nitro-2,3-dihydrobenzo[*b*][1,4] dioxin-6-yl)sulfonyl)-4-(2-((*R* or *S*)-3-(2-isopropylphenyl)morph olino)-7-azaspiro[3.5]nonan-7-yl)benzamide | MS-ESI (m/z): 935 [M + 1]⁺ |
| E-18 | 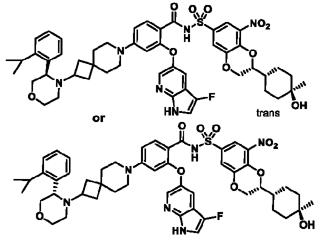 | 2-((3-fluoro-1H-pyrrolo[2,3-b] pyridin-5-yl)oxy)-N-(((R)-2-((1 *r*,4*S*)-4-hydroxy-4-methylcyclo hexyl)-8-nitro-2,3-dihydrobenz o[*b*][1,4]dioxin-6-yl)sulfonyl)-4-(2-((R or S)-3-(2-isopropylphenyl)morph olino)-7-azaspiro[3.5]nonan-7-yl)benzamide | MS-ESI (m/z): 953 [M + 1]⁺ |
| E-19 | 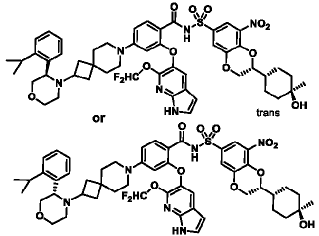 | 2-((6-(difluoromethoxy)-1H-py rrolo[2,3-b]pyridin-5-yl)oxy)-*N* -(((*R*)-2-((1*r*,4*R*)-4-hydroxy-4-methylcyclohexyl)-8-nitro-2,3-dihydrobenzo[b] [1,4]dioxin-6-yl)sulfonyl)-4-(2-((R or S)-3-(2-isopropylphenyl)morph olino)-7-azaspiro[3.5]nonan-7-yl)benzamide | MS-ESI (m/z): 1001 [M + 1]⁺ |
| E-20 | 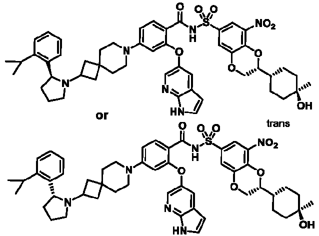 | 2-((1*H*-pyrrolo[2,3-*b*]pyridin-5 -yl)oxy)-*N*-(((*R*)-2-((1*r*,4*R*)-4-hydroxy-4-methylcyclohexyl)-8-nitro-2,3-dihydrobenzo[*b*][1,4] dioxin-6-yl)sulfonyl)-4-(2-((*R* or *S*)-2-(2-isopropylphenyl)pyrroli din-1-yl)-7-azaspiro[3.5]nonan-7-yl)benzamide | MS-ESI (m/z): 919 [M + 1]⁺ |
| E-21 | 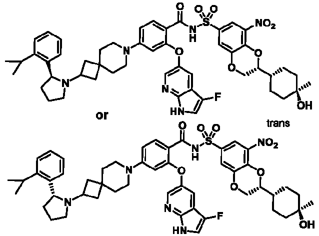 | 2-((3-fluoro-1*H*-pyrrolo[2,3-*b*] pyridin-5-yl)oxy)-*N*-(((*R*)-2-((1 *r*,4*R*)-4-hydroxy-4-methylcyclo hexyl)-8-nitro-2,3-dihydrobenz o[*b*][1,4]dioxin-6-yl)sulfonyl)-4-(2-((*R* or *S*)-2-(2-isopropylphenyl)pyrroli din-1-yl)-7-azaspiro[3.5]nonan-7-yl)benzamide | MS-ESI (m/z): 937 [M + 1]⁺ |
| E-22 | 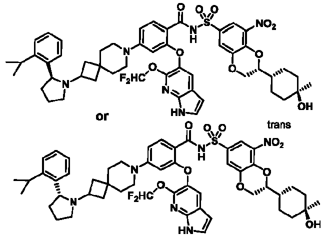 | 2-((6-(difluoromethoxy)-1*H*-py rrolo[2,3-*b*]pyridin-5-yl)oxy)-*N* -(((*R*)-2-((1*r*,4*R*)-4-hydroxy-4-methylcyclohexyl)-8-nitro-2,3-dihydrobenzo[b] [1,4]dioxin-6-yl)sulfonyl)-4-(2-((*R* or *S*)-2-(2-isopropylphenyl)pyrroli din-1-yl)-7-azaspiro[3.5]nonan-7-yl)benzamide | MS-ESI (m/z): 985 [M + 1]⁺ |
| E-23 | 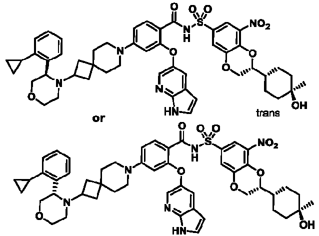 | 2-((1*H*-pyrrolo[2,3-*b*]pyridin-5 -yl)oxy)-4-(2-((*R* or *S*)-3-(2-cyclopropylphenyl)mor pholino)-7-azaspiro[3.5]nonan-7-yl)-*N*-(((*R*)-2-((1*r*,4*S*)-4-hydr oxy-4-methylcyclohexyl)-8-nitr o-2,3-dihydrobenzo[*b*][1,4]diox in-6-yl)sulfonyl)benzamide | MS-ESI (m/z): 933 [M + 1]⁺ |
| E-24 | 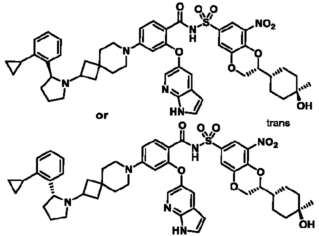 | 2-((1*H*-pyrrolo[2,3-*b*]pyridin-5 -yl)oxy)-4-(2-((*R* or *S*)-2-(2-cyclopropylphenyl)pyrr olidin-1-yl)-7-azaspiro[3.5]non an-7-yl)-*N*-(((*R*)-2-((1*r*,4*S*)-4-h ydroxy-4-methylcyclohexyl)-8-nitro-2,3-dihydrobenzo[*b*][1,4] dioxin-6-yl)sulfonyl)benzamide | MS-ESI (m/z): 917 [M + 1]⁺ |
| E-25 | 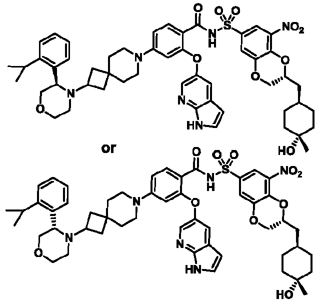 | 2-((1*H*-pyrrolo[2,3-*b*]pyridin-5 -yl)oxy)-*N*-(((*R*)-2-(((1*r*,4*R*)-4-hydroxy-4-methylcyclohexyl)m ethyl)-8-nitro-2,3-dihydrobenz o[*b*][1,4]dioxin-6-yl)sulfonyl)-4-(2-((*R* or *S*)-3-(2-isopropylphenyl)morph olino)-7-azaspiro[3.5]nonan-7-yl)benzamide | MS-ESI (m/z): 949 [M + 1]⁺ |
| E-26 | 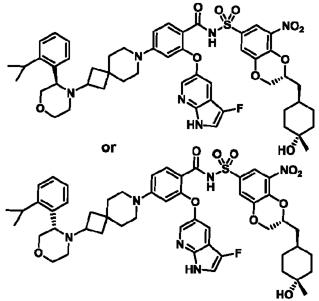 | 2-((3-fluoro-1*H*-pyrrolo[2,3-*b*] pyridin-5-yl)oxy)-*N*-(((*R*)-2-((( 1*r*,4*R*)-4-hydroxy-4-methylcycl ohexyl)methyl)-8-nitro-2,3-dih ydrobenzo[*b*][1,4]dioxin-6-yl)s ulfonyl)-4-(2-((*R* or *S*)-3-(2-isopropylphenyl)morph olino)-7-azaspiro[3.5]nonan-7-yl)benzamide | MS-ESI (m/z): 967 [M + 1]⁺ |
| E-27 | 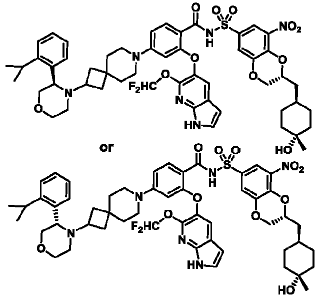 | 2-((6-(difluoromethoxy)-1H-py rrolo[2,3-b]pyridin-5-yl)oxy)-*N* -(((*R*)-2-(((1*r*,4*R*)-4-hydroxy-4-methylcyclohexyl)methyl)-8-ni tro-2,3-dihydrobenzo[*b*][1,4]di oxin-6-yl)sulfonyl)-4-(2-((R or S)-3-(2-isopropylphenyl)morph olino)-7-azaspiro[3.5]nonan-7-yl)benzamide | MS-ESI (m/z): 1015 [M + 1]⁺ |
| E-28 | 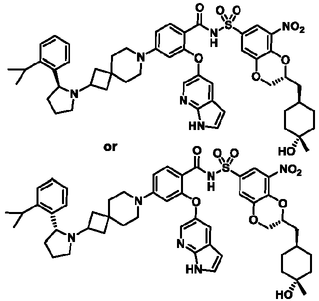 | 2-((1*H*-pyrrolo[2,3-*b*]pyridin-5 -yl)oxy)-*N*-(((*R*)-2-(((1*r*,4*R*)-4-hydroxy-4-methylcyclohexyl)m ethyl)-8-nitro-2,3-dihydrobenz o[*b*][1,4]dioxin-6-yl)sulfonyl)-4-(2-((R or S)-2-(2-isopropylphenyl)pyrroli din-1-yl)-7-azaspiro[3.5]nonan-7-yl)benzamide | MS-ESI (m/z): 933 [M + 1]⁺ |
| E-29 | 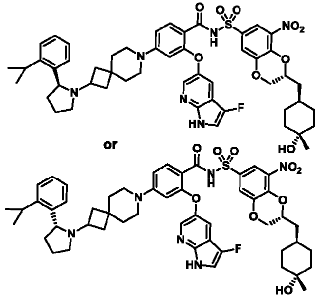 | 2-((3-fluoro-1*H*-pyrrolo[2,3-*b*] pyridin-5-yl)oxy)-*N*-(((*R*)-2-((( 1*r*,4*R*)-4-hydroxy-4-methylcycl ohexyl)methyl)-8-nitro-2,3-dih ydrobenzo[b][1,4]dioxin-6-yl)s ulfonyl)-4-(2-((*R* or *S*)-2-(2-isopropylphenyl)pyrroli din-1-yl)-7-azaspiro[3.5]nonan-7-yl)benzamide | MS-ESI (m/z): 951 [M + 1]⁺ |
| E-30 | 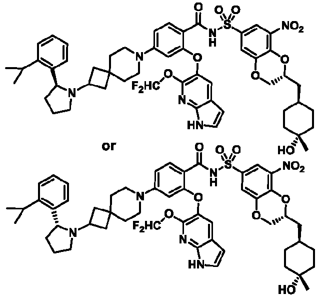 | 2-((6-(difluoromethoxy)-1*H*-py rrolo[2,3-b]pyridin-5-yl)oxy)-*N* -(((*R*)-2-(((1*r*,4*R*)-4-hydroxy-4-methylcyclohexyl)methyl)-8-ni tro-2,3-dihydrobenzo[*b*][1,4]di oxin-6-yl)sulfonyl)-4-(2-((*R* or *S*)-2-(2-isopropylphenyl)pyrroli din-1-yl)-7-azaspiro[3.5]nonan-7-yl)benzamide | MS-ESI (m/z): 949 [M + 1]⁺ |
| E-31 | 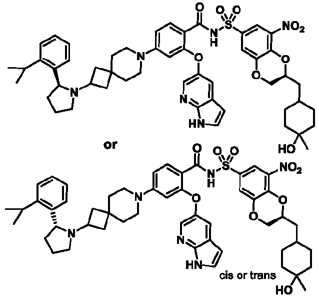 | 2-((1*H*-pyrrolo[2,3-*b*]pyridin-5 -yl)oxy)-*N*-(((*S*)-2-((4-hydroxy-4-methylcyclohexyl)methyl)-8-nitro-2,3-dihydrobenzo[*b*][1,4] dioxin-6-yl)sulfonyl)-4-(2-((*R* or *S*)-2-(2-isopropylphenyl)pyrroli din-1-yl)-7-azaspiro[3.5]nonan-7-yl)benzamide | MS-ESI (m/z): 933 [M + 1]⁺ |
| E-32 | 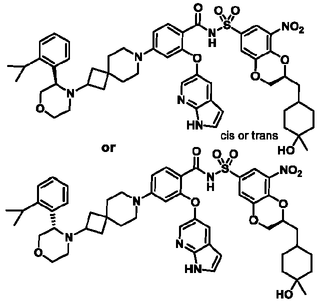 | 2-((1*H*-pyrrolo[2,3-*b*]pyridin-5 -yl)oxy)-*N*-(((*S*)-2-((4-hydroxy-4-methylcyclohexyl)methyl)-8-nitro-2,3-dihydrobenzo[*b*][1,4] dioxin-6-yl)sulfonyl)-4-(2-((*R* or *S*)-3-(2-isopropylphenyl)morph olino)-7-azaspiro[3.5]nonan-7-yl)benzamide | MS-ESI (m/z): 949 [M + 1]⁺ |
| E-33 | 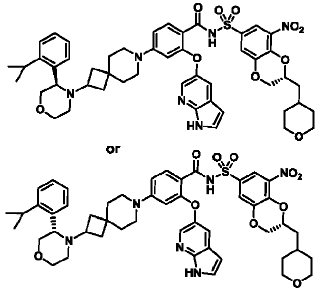 | 2-((1*H*-pyrrolo[2,3-*b*]pyridin-5 -yl)oxy)-4-(2-((R or S)-3-(2-isopropylphenyl)morph olino)-7-azaspiro[3.5]nonan-7-yl)-N-(((R)-8-nitro-2-((tetrahyd ro-2*H*-pyran-4-yl)methyl)-2,3-dihydrobenzo[*b*][1,4]dioxin-6-yl)sulfonyl)benzamide | MS-ESI (m/z): 921 [M + 1]⁺ |
| E-34 | 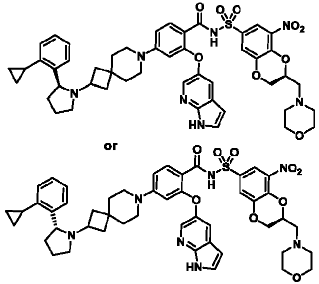 | 2-((1*H*-pyrrolo[2,3-*b*]pyridin-5 -yl)oxy)-4-(2-((R or S)-2-(2-cyclopropylphenyl)pyrr olidin-1-yl)-7-azaspiro[3.5]non an-7-yl)-N-(((S)-2-(morpholino methyl)-8-nitro-2,3-dihydroben zo[*b*][1,4]dioxin-6-yl)sulfonyl) benzamide | MS-ESI (m/z): 904 [M + 1]⁺ |
| E-35 | 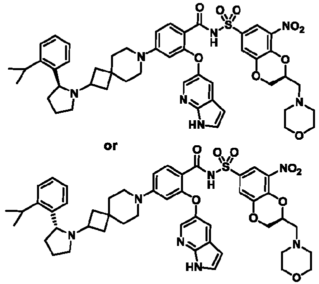 | 2-((1*H*-pyrrolo[2,3-*b*]pyridin-5 -yl)oxy)-4-(2-((*R* or *S*)-2-(2-isopropylphenyl)pyrroli din-1-yl)-7-azaspiro[3.5]nonan-7-yl)-*N*-(((*S*)-2-(morpholinomet hyl)-8-nitro-2,3-dihydrobenzo[ *b*][1,4]dioxin-6-yl)sulfonyl)ben zamide | MS-ESI (m/z): 906 [M + 1]⁺ |
| E-36 | 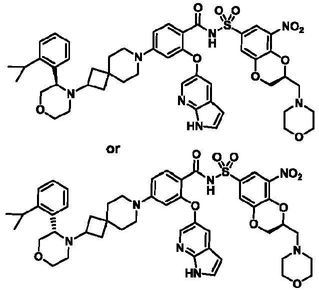 | 2-((1*H*-pyrrolo[2,3-*b*]pyridin-5 -yl)oxy)-4-(2-((*R* or *S*)-3-(2-isopropylphenyl)morph olino)-7-azaspiro[3.5]nonan-7-yl)-*N*-(((*S*)-2-(morpholinometh yl)-8-nitro-2,3-dihydrobenzo[*b*] [1,4]dioxin-6-yl)sulfonyl)benza mide | MS-ESI (m/z): 922 [M + 1]⁺ |
| E-37 | 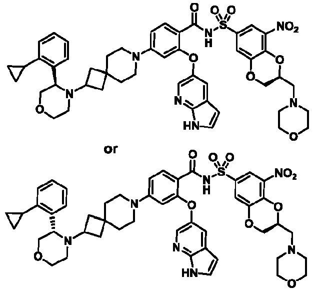 | 2-((1*H*-pyrrolo[2,3-*b*]pyridin-5 -yl)oxy)-4-(2-((*R* or *S*)-3-(2-cyclopropylphenyl)mor pholino)-7-azaspiro[3.5]nonan-7-yl)-*N*-(((*S*)-2-(morpholinomet hyl)-8-nitro-2,3-dihydrobenzo[ *b*][1,4]dioxin-6-yl)sulfonyl)ben zamide | MS-ESI (m/z): 920 [M + 1]⁺ |
| E-38 | 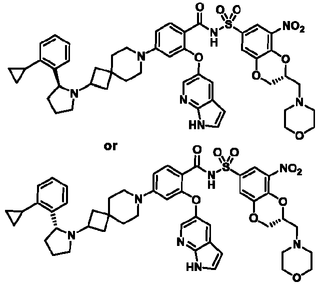 | 2-((1*H*-pyrrolo[2,3-*b*]pyridin-5 -yl)oxy)-4-(2-((*R* or *S*)-2-(2-cyclopropylphenyl)pyrr olidin-1-yl)-7-azaspiro[3.5]non an-7-yl)-*N*-(((*R*)-2-(morpholino methyl)-8-nitro-2,3-dihydroben zo[*b*][1,4]dioxin-6-yl)sulfonyl) benzamide | MS-ESI (m/z): 904 [M + 1]⁺ |
| E-39 | 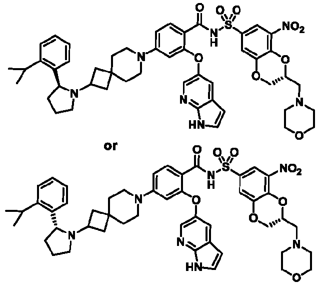 | 2-((1*H*-pyrrolo[2,3-*b*]pyridin-5 -yl)oxy)-4-(2-((*R* or *S*)-2-(2-isopropylphenyl)pyrroli din-1-yl)-7-azaspiro[3.5]nonan-7-yl)-*N*-(((*R*)-2-(morpholinome thyl)-8-nitro-2,3-dihydrobenzo[ *b*][1,4]dioxin-6-yl)sulfonyl)ben zamide | MS-ESI (m/z): 906 [M + 1]⁺ |
| E-40 | 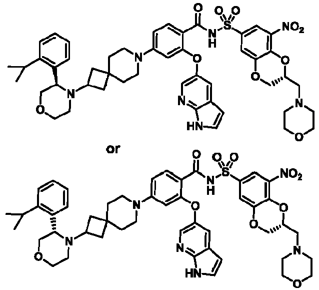 | 2-((1*H*-pyrrolo[2,3-*b*]pyridin-5 -yl)oxy)-4-(2-((*R* or *S*)-3-(2-isopropylphenyl)morph olino)-7-azaspiro[3.5]nonan-7-yl)-*N*-(((*R*)-2-(morpholinometh yl)-8-nitro-2,3-dihydrobenzo[*b*] [1,4]dioxin-6-yl)sulfonyl)benza mide | MS-ESI (m/z): 922 [M + 1]⁺ |
| E-41 | 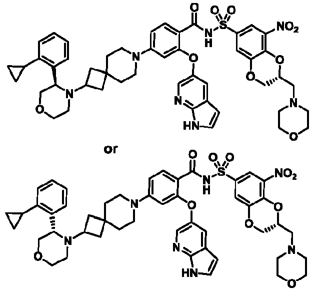 | 2-((1*H*-pyrrolo[2,3-*b*]pyridin-5 -yl)oxy)-4-(2-((*R* or *S*)-3-(2-cyclopropylphenyl)mor pholino)-7-azaspiro[3.5]nonan-7-yl)-*N*-(((*R*)-2-(morpholinome thyl)-8-nitro-2,3-dihydrobenzo[ *b*][1,4]dioxin-6-yl)sulfonyl)ben zamide | MS-ESI (m/z): 920 [M + 1]⁺ |
| E-42 | 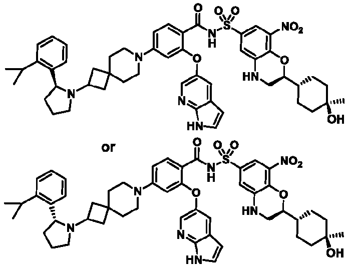 | 2-((1*H*-pyrrolo[2,3-*b*]pyridin-5 -yl)oxy)-*N*-(((*S*)-2-((1*r*,4*S*)-4-h ydroxy-4-methylcyclohexyl)-8-nitro-3,4-dihydro-2*H*-benzo[*b*][ 1,4]oxazin-6-yl)sulfonyl)-4-(2-((*R* or *S*)-2-(2-isopropylphenyl)pyrroli din-1-yl)-7-azaspiro[3.5]nonan-7-yl)benzamide | MS-ESI (m/z): 918 [M + 1]⁺ |
| E-43 | 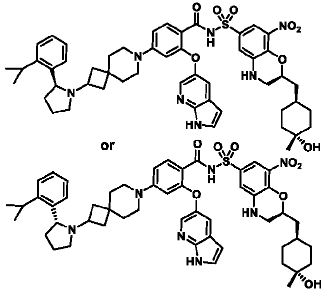 | 2-((1*H*-pyrrolo[2,3-*b*]pyridin-5 -yl)oxy)-*N*-(((*S*)-2-(((1*r*,4*S*)-4-h ydroxy-4-methylcyclohexyl)me thyl)-8-nitro-3,4-dihydro-2*H*-b enzo[*b*][1,4]oxazin-6-yl)sulfon yl)-4-(2-((*R* or *S*)-2-(2-isopropylphenyl)pyrroli din-1-yl)-7-azaspiro[3.5]nonan-7-yl)benzamide | MS-ESI (m/z): 932 [M + 1]⁺ |
| E-44 | 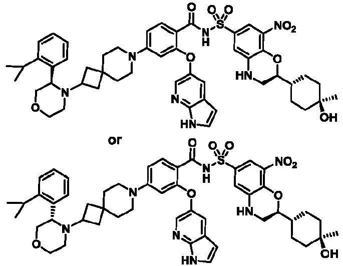 | 2-((1*H*-pyrrolo[2,3-*b*]pyridin-5 -yl)oxy)-*N*-(((*S*)-2-((1*r*,4*S*)-4-h ydroxy-4-methylcyclohexyl)-8-nitro-3,4-dihydro-2*H*-benzo[*b*][ 1,4]oxazin-6-yl)sulfonyl)-4-(2-((*R* or *S*)-3-(2-isopropylphenyl)morph olino)-7-azaspiro[3.5]nonan-7-yl)benzamide | MS-ESI (m/z): 934 [M + 1]⁺ |
| E-45 | 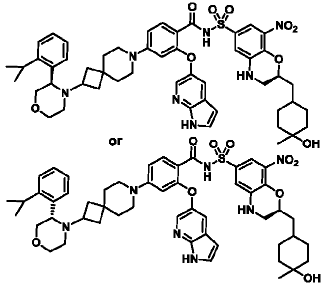 | 2-((1*H*-pyrrolo[2,3-*b*]pyridin-5 -yl)oxy)-*N*-(((*S*)-2-((4-hydroxy-4-methylcyclohexyl)methyl)-8-nitro-3,4-dihydro-2*H*-benzo[*b*][ 1,4]oxazin-6-yl)sulfonyl)-4-(2-((*R* or *S*)-3-(2-isopropylphenyl)morph olino)-7-azaspiro[3.5]nonan-7-yl)benzamide | MS-ESI (m/z): 948 [M + 1]⁺ |

### Cell Proliferation Assays

MTS testing kit was purchased from Promega. The RPMI-1640, Fetal bovine serum and Penicillin-Streptomycin were purchased from Gibco. Dimethyl sulfoxide (DMSO) was purchased from Sigma.

To investigate whether a compound is able to inhibit the activity of Bcl-2 in cells, a mechanism-based assay using RS4;11 (ATCC^{®} CRL-1873^{™}) cell was developed. In this assay, inhibition of Bcl-2 was detected by the inhibition of RS4;11 cells proliferation. RS4;11 cells were cultured in culture flasks to 40-80% confluence in RPMI-1640 plus 10% fetal bovine serum. Cells were collected and plated onto 96-well plates at desired cell density (30000 cells/well). Plates were incubated overnight at 37°C, with 5% CO₂ to adhere. Compounds were added to the plates, and the final compound concentrations were 10000, 3333, 1111, 270, 124, 41, 14, 4.6 and 1.5 nM. Plates plated with RS4;11 cells were placed at 37°C, with 5% CO₂ for 72 h. 20 µl MTS/ 100 µl medium mixture solution were added to each well and incubate the plates for exactly 2 hours. The reaction was stopped by adding 25 µl 10% SDS per well. Measure absorbance at 490 nm and 650 nm (reference wavelength). IC₅₀ was calculated using GraphPad Prism 5.0.

Select compounds prepared as described above were assayed according to the biological procedures described herein. The results are given in the table 7.

**Table 7**

| Example | RS4;11 IC₅₀ (nM) | Example | RS4;11 IC₅₀ (nM) | Example | RS4;11 IC₅₀ (nM) |
|---|---|---|---|---|---|
| A-1a | 1 | A-27b | 1 | A-98 | 1 |
| A-1b | 1 | A-30a | 1 | A-121 | 1 |
| A-2b | 1 | A-30b | 1 | A-131 | 1 |
| A-3b | 1 | A-31b | 1 | A-142 | 1 |
| A-5a | 1 | A-32a | 1 | B-1b | 1 |
| A-5b | 1 | A-32b | 1 | B-3b | 1 |
| A-6a | 1 | A-34 | 1 | B-4b | 1 |
| A-6b | 1 | A-36a | 1 | B-31a | 1 |
| A-7a | 1 | A-37 | 1 | B-33 | 1 |
| A-7b | 1 | A-39a | 1 | B-34 | 1 |
| A-8a | 1 | A-40 | 1 | B-38 | 1 |
| A-9b | 1 | A-55a | 1 | B-45 | 1 |
| A-10b | 1 | A-56a | 1 | B-47 | 1 |
| A-13b | 1 | A-56b | 1 | B-52 | 1 |
| A-17b | 1 | A-75 | 1 | E-38 | 1 |
| A-21b | 1 | A-97a | 1 | E-39 | 1 |
| A-23b | 1 | A-97b | 1 | E-40 | 1 |
| A-27a | 1 | / | / | / | / |

MTS testing kit was purchased from Promega (Madison, WI, USA). The RPMI-1640, Fetal bovine serum and Penicillin-Streptomycin were purchased from BI (Biological Industries, Beit Haemek, Israel). Dimethyl sulfoxide (DMSO) was purchased from Sigma (St. Louis., MO, USA). DoHH2 (DSMZ catalog#: ACC47) cells were cultured in RPMI-1640 supplemented with Penicillin-Streptomycin and 10% FBS.

To investigate whether a compound is able to inhibit the activity of BCL-2 in cells, a mechanism-based assay using DoHH2 lines was developed. In this assay, the inhibition of BCL-2 was reflected by the inhibition of cell proliferation of DoHH2 cells. Cells were plated into 96-well plates at the optimized cell density of 5000 cells/well. Plates were incubated at 37°C, with 5 % CO₂ for 4h. Compounds were serially diluted and added to the plates with the final concentrations of 10000, 3333.3, 1111.1, 370.4, 123.5, 41.2, 13.7, 4.6 and 1.5 nM. Plates were incubated at 37°C, with 5% CO₂ for 120 h. 20 µl MTS was added into each well and the plates were incubated at 37°C, with 5 % CO₂ for exactly 2 h. The absorbance was measured by a microplate reader at 490 nm. IC₅₀ was calculated using GraphPad Prism 5.0 software.

Select compounds prepared as described above were assayed according to the biological procedures described herein. The results are given in the table 8.

**Table 8**

| Example | DoHH2 IC₅₀ (nM) | Example | DoHH2 IC₅₀ (nM) | Example | DoHH2 IC₅₀ (nM) |
|---|---|---|---|---|---|
| A-1b | 1 | A-39a | 1 | B-34 | 1 |
| A-2b | 1 | A-40 | 1 | B-35 | 1 |
| A-3b | 1 | A-55a | 1 | B-36 | 1 |
| A-5a | 1 | A-56a | 1 | B-38 | 1 |
| A-5b | 1 | A-56b | 1 | B-40 | 1 |
| A-6b | 1 | A-57 | 1 | B-45 | 1 |
| A-7a | 1 | A-62 | 1 | B-46 | 1 |
| A-7b | 1 | A-75 | 1 | B-47 | 1 |
| A-10b | 1 | A-97a | 1 | B-48 | 1 |
| A-13b | 1 | A-97b | 1 | B-51 | 1 |
| A-17b | 1 | A-121 | 1 | B-52 | 1 |
| A-21b | 1 | A-128 | 1 | D-23 | 1 |
| A-23b | 1 | A-129 | 1 | D-24 | 1 |
| A-27b | 1 | A-131 | 1 | E-34 | 1 |
| A-30b | 1 | A-136 | 1 | E-35 | 1 |
| A-31b | 1 | A-142 | 1 | E-36 | 1 |
| A-32a | 1 | B-1b | 1 | E-37 | 1 |
| A-32b | 1 | B-3b | 1 | E-38 | 1 |
| A-34 | 1 | B-4b | 1 | E-39 | 1 |
| A-37 | 1 | B-31a | 1 | E-40 | 1 |
| / | / | B-33 | 1 | E-41 | 1 |

MTS testing kit was purchased from Promega (Madison, WI, USA). The RPMI-1640, Fetal bovine serum and Penicillin-Streptomycin were purchased from BI (Biological Industries, Beit Haemek, Israel). Dimethyl sulfoxide (DMSO) was purchased from Sigma (St. Louis., MO, USA). Toledo (ATCC catalog#: CRL-2631) cells were cultured in RPMI-1640 supplemented with Penicillin-Streptomycin and 10% FBS.

To investigate whether a compound is able to inhibit the activity of BCL-2 in cells, a mechanism-based assay using Toledo lines was developed. In this assay, the inhibition of BCL-2 was reflected by the inhibition of cell proliferation of Toledo cells. Cells were plated into 96-well plates at the optimized cell density of 30000 cells/well. Plates were incubated at 37°C, with 5 % CO₂ for 4h. Compounds were serially diluted and added to the plates with the final concentrations of 10000, 3333.3, 1111.1, 370.4, 123.5, 41.2, 13.7, 4.6 and 1.5 nM. Plates were incubated at 37°C, with 5% CO₂ for 72 h. 20 µl MTS was added into each well and the plates were incubated at 37°C, with 5 % CO₂ for exactly 2 h. The absorbance was measured by a microplate reader at 490 nm. IC₅₀ was calculated using GraphPad Prism 5.0 software.

Select compounds prepared as described above were assayed according to the biological procedures described herein. The results are given in the table 9.

**Table 9**

| Example | Toledo IC₅₀ (nM) | Example | Toledo IC₅₀ (nM) | Example | Toledo IC₅₀ (nM) | Example | Toledo IC₅₀ (nM) |
|---|---|---|---|---|---|---|---|
| A-3b | 1 | D-3 | 1 | D-70 | 1 | D-144 | 1 |
| A-5b | 1 | D-7 | 1 | D-71 | 1 | D-183 | 1 |
| A-7a | 1 | D-10 | 1 | D-72 | 1 | D-191 | 1 |
| A-7b | 1 | D-11 | 1 | D-73 | 1 | D-201 | 1 |
| A-27b | 1 | D-17 | 1 | D-74 | 1 | D-202 | 1 |
| A-30b | 1 | D-18 | 1 | D-75a | 1 | D-213 | 1 |
| A-31b | 2 | D-19 | 9 | D-75b | 1 | D-214 | 1 |
| A-34 | 7 | D-21 | 1 | D-76 | 1 | D-215 | 1 |
| A-37 | 1 | D-28 | 1 | D-77 | 1 | D-216 | 1 |
| A-39a | 1 | D-28a | 1 | D-78 | 1 | D-218 | 1 |
| A-40 | 1 | D-28b | 1 | D-79 | 1 | D-220 | 1 |
| A-55a | 5 | D-34a | 1 | D-80 | 1 | D-221 | 1 |
| A-56a | 1 | D-34b | 1 | D-81 | 1 | D-223 | 1 |
| A-56b | 1 | D-39 | 1 | D-82 | 1 | D-225 | 1 |
| A-57 | 2 | D-40 | 1 | D-83 | 1 | D-226 | 1 |
| A-62 | 1 | D-41 | 1 | D-84 | 1 | D-229 | 1 |
| A-97b | 6 | D-42 | 1 | D-85 | 1 | D-232 | 1 |
| A-121 | 1 | D-43 | 1 | D-90 | 1 | D-234 | 1 |
| A-125 | 2 | D-44 | 1 | D-91 | 1 | D-236 | 1 |
| A-126 | 1 | D-45 | 1 | D-92 | 1 | D-240 | 1 |
| A-128 | 1 | D-46 | 1 | D-95 | 1 | D-244 | 1 |
| A-129 | 1 | D-47 | 1 | D-96 | 1 | D-247 | 1 |
| A-136 | 1 | D-48 | 1 | D-97 | 1 | D-248 | 1 |
| A-142 | 9 | D-49a | 1 | D-98 | 1 | D-250 | 1 |
| A-145 | 1 | D-49b | 1 | D-101 | 1 | D-251 | 1 |
| A-149 | 1 | D-50a | 1 | D-102 | 1 | D-252 | 1 |
| A-150 | 1 | D-50b | 1 | D-103 | 1 | D-253 | 1 |
| A-151 | 1 | D-55 | 1 | D-106 | 1 | D-254 | 1 |
| A-152 | 1 | D-56 | 1 | D-108 | 1 | D-257 | 1 |
| A-153 | 1 | D-57 | 1 | D-109 | 1 | D-258 | 1 |
| A-154 | 1 | D-58 | 1 | D-110 | 1 | D-286 | 1 |
| A-156 | 1 | D-59 | 1 | D-113 | 1 | D-287 | 1 |
| A-158 | 1 | D-60 | 1 | D-115 | 1 | E-2 | 1 |
| A-159 | 1 | D-61 | 1 | D-116 | 1 | E-6 | 1 |
| A-160 | 1 | D-63 | 1 | D-117 | 1 | E-35 | 7 |
| A-161 | 1 | D-64 | 1 | D-123 | 1 | E-38 | 1 |
| A-162 | 1 | D-65 | 1 | D-130 | 1 | E-39 | 1 |
| C-1 | 1 | D-66 | 1 | D-131 | 1 | E-40 | 1 |
| C-2 | 1 | D-67 | 1 | D-134 | 1 | E-41 | 1 |
| D-1 | 1 | D-68 | 1 | D-135 | 1 | / | / |
| D-2 | 1 | D-69 | 1 | D-140 | 1 | / | / |

MTS testing kit was purchased from Promega (Madison, WI, USA). The RPMI-1640, Fetal bovine serum and Penicillin-Streptomycin were purchased from BI (Biological Industries, Beit Haemek, Israel). Puromycin was purchased from Beyotime (shanghai, China). Dimethyl sulfoxide (DMSO) was purchased from Sigma (St. Louis., MO, USA). RS4;11-BCL-2 G101V and RS4;11-BCL-2 F104L cells were cultured in RPMI1640 supplemented with 1 ug/mL puromycin, 100 U/mL Penicillin-Streptomycin and 10% FBS.

To investigate whether a compound is able to inhibit the activity of BCL-2 mutation in cells, a mechanism-based assay using engineered cell lines stably overexpressing BCL-2 mutation (RS4;11-BCL-2 G101V and RS4;11-BCL-2 F104L) was developed. In this assay, the inhibition of BCL-2 mutation was reflected by the inhibition of cell proliferation of RS4;11-BCL-2 G101V and RS4;11-BCL-2 F104L cells. Cells were plated into 96-well plates at optimized cell density (RS4;11-BCL-2 G101V: 5000 cells/well; RS4;11-BCL-2 F104L: 5000 cells/well). Plates were incubated at 37 °C, with 5 % CO₂ for 4 h. Compounds were serially diluted to the final-concentrations of 10000, 3333.3, 1111.1, 370.4, 123.5, 41.2, 13.7, 4.6 and 1.4 nM and added to the plates. Plates were incubated at 37 °C, with 5 % CO₂ for 168 h. 20 µl MTS was added into each well and the plates were incubated at 37 °C, with 5 % CO₂ exactly for 2 h. The absorbance was measured by a microplate reader at 490 nm. IC₅₀ values were calculated using GraphPad Prism 5.0 software.

Select compounds prepared as described above were assayed according to the biological procedures described herein. The results are given in the table 10 and table 11.

**Table 10**

| Example | RS4;11 BCL2-G101V | Example | RS4;11 BCL2-G101V | Example | RS4;11 BCL2-G101V |
|---|---|---|---|---|---|
| A-149 | 1 | D-64 | 1 | D-123 | 1 |
| A-150 | 6 | D-65 | 1 | D-130 | 1 |
| A-151 | 3 | D-71 | 13 | D-131 | 1 |
| A-152 | 40 | D-72 | 17 | D-134 | 1 |
| A-153 | 25 | D-75a | 4 | D-135 | 1 |
| A-154 | 57 | D-75b | 16 | D-201 | 1 |
| A-155 | 13 | D-80 | 8 | D-202 | 2 |
| A-161 | 2 | D-82 | 19 | D-214 | 1 |
| D-7 | 1 | D-85 | 28 | D-216 | 1 |
| D-17 | 1 | D-90 | 5 | D-223 | 1 |
| D-18 | 14 | D-92 | 1 | D-226 | 13 |
| D-28 | 13 | D-95 | 3 | D-229 | 1 |
| D-28a | 1 | D-96 | 4 | D-233 | 14 |
| D-34a | 18 | D-97 | 12 | D-241 | 4 |
| D-34b | 7 | D-98 | 1 | D-243 | 10 |
| D-55 | 14 | D-101 | 19 | D-250 | 1 |
| D-59 | 15 | D-102 | 16 | D-251 | 6 |
| D-60 | 1 | D-103 | 1 | D-286 | 1 |
| D-61 | 1 | D-106 | 14 | / | / |
| D-63 | 1 | D-113 | 19 | / | / |

**Table 11**

| Example | RS4;11 BCL2-F104L | Example | RS4;11 BCL2-F104L | Example | RS4;11 BCL2-F104L |
|---|---|---|---|---|---|
| A-149 | 1 | D-65 | 20 | D-216 | 1 |
| A-150 | 1 | D-71 | 12 | D-223 | 1 |
| A-151 | 1 | D-83 | 8 | D-229 | 1 |
| A-152 | 9 | D-85 | 5 | D-233 | 4 |
| A-153 | 6 | D-90 | 1 | D-234 | 8 |
| A-154 | 19 | D-95 | 8 | D-235 | 10 |
| A-155 | 1 | D-96 | 1 | D-239 | 5 |
| A-155a | 27 | D-102 | 1 | D-240 | 10 |
| A-156 | 16 | D-103 | 1 | D-241 | 1 |
| A-157 | 23 | D-109 | 8 | D-241a | 11 |
| A-158 | 8 | D-110 | 15 | D-243 | 1 |
| D-7 | 14 | D-113 | 1 | D-243a | 13 |
| D-10 | 34 | D-116 | 1 | D-243b | 20 |
| D-17 | 22 | D-117 | 1 | D-244 | 1 |
| D-18 | 33 | D-123 | 11 | D-245 | 15 |
| D-34b | 1 | D-130 | 1 | D-250 | 1 |
| D-55 | 19 | D-131 | 1 | D-251 | 1 |
| D-60 | 1 | D-134 | 1 | D-252 | 1 |
| D-61 | 11 | D-135 | 1 | D-286 | 1 |
| D-64 | 25 | D-214 | 1 | / | / |

## Claims

1. A compound of formula (I): 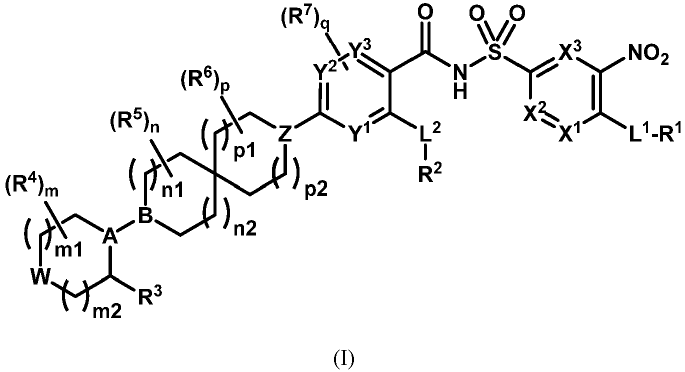 or a pharmaceutically acceptable salt thereof, wherein:
X¹ is selected from N and CR⁸ X² is selected from N and CR⁹; X³ is selected from N and CR¹⁰; wherein at least one of X¹, X² and X³ is N;
Y¹, Y² and Y³ are CH;
A and B are independently selected from N and CH;
W is selected from -CHR¹¹-, -NR¹²-, -O-, -S(O)ᵣ- and -S(O)(=NR¹²)-;
Z is selected from N and CH;
L¹ is selected from -CH₂-, -O-, -NH- and -S-;
L² is selected from a bond and -O-;
R¹ is selected from C₁₋₁₀ alkyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁-₄ alkyl, wherein alkyl, cycloalkyl, heterocyclyl, aryl and heteroaryl are each unsubstituted or substituted with at least one substituent, independently selected from R^{X};
R² is selected from 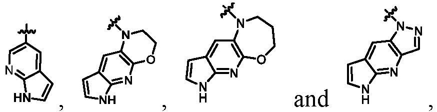 which are unsubstituted or substituted with at least one substituent, independently selected from R^{X};
R³ is phenyl, wherein phenyl is unsubstituted or substituted with at least one substituent, independently selected from R^{X};
each R⁴ is independently selected from hydrogen, halogen, -OH, methyl, ethyl, isopropyl and cyclopropyl, wherein methyl, ethyl, isopropyl and cyclopropyl are each unsubstituted or substituted with at least one substituent, independently selected from R^{X}; or any two of R⁴ together with the atoms to which they are attached form cyclopropyl, wherein cyclopropyl is unsubstituted or substituted with 1, 2 or 3 R^{X} groups;
or "R⁴ and R¹¹" or "R⁴ and R¹²" together with the atoms to which they are attached form a C₃₋₈ cycloalkyl or heterocyclic ring of 4 to 10 members containing 1, 2 or 3 heteroatoms independently selected from oxygen, sulfur, nitrogen and phosphorus, and optionally substituted with 1, 2 or 3 R^{X} groups;
each R⁵ and R⁶ are independently selected from hydrogen, halogen, C₁₋₁₀ alkyl, C₃₋₁₀ cycloalkyl, CN, NO₂, -NH₂ and -OH, wherein alkyl and cycloalkyl are each unsubstituted or substituted with at least one substituent, independently selected from R^{X};
each R⁷ is independently selected from hydrogen, halogen, C₁₋₁₀ alkyl, C₃₋₁₀ cycloalkyl, CN, NO₂, -NR^{A7}R^{B7}, -OR^{A7} and -C(O)R^{A7}, wherein alkyl and cycloalkyl are each unsubstituted or substituted with at least one substituent, independently selected from R^{X};
R⁸ is selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, CN, NO₂, -NR^{A8}R^{B8}, -OR^{A8}, - C(O)R^{A8}, -C(O)OR^{A8}, -OC(O)R^{A8} and -S(O)ᵣR^{A8}, wherein alkyl, alkenyl, alkynyl, cycloalkyl and heterocyclyl are each unsubstituted or substituted with at least one substituent, independently selected from R^{X};
or R¹ and R⁸ together with the atoms to which they are attached form a C₅₋₁₀ cycloalkyl or heterocyclic ring of 5 to 12 members containing 1, 2 or 3 heteroatoms independently selected from oxygen, sulfur, nitrogen and phosphorus, and optionally substituted with 1, 2 or 3 R^{X} groups;
R⁹ is selected from hydrogen, halogen, CN, OH, methyl, ethyl and trifluoromethyl;
R¹⁰ is selected from hydrogen, halogen, C₁₋₁₀ alkyl, CN, NO₂, -NH₂ and -OH, wherein alkyl is unsubstituted or substituted with at least one substituent, independently selected from R^{X};
R¹¹ is selected from hydrogen, C₁₋₁₀ alkyl and C₃₋₁₀ cycloalkyl, wherein alkyl and cycloalkyl are each unsubstituted or substituted with at least one substituent, independently selected from R^{X};
R¹² is selected from hydrogen, C₁₋₁₀ alkyl and C₃₋₁₀ cycloalkyl, heterocyclyl, heterocyclyl-C₁₋₄ alkyl, aryl, aryl-C₁₋₄ alkyl, heteroaryl, -C(O)R^{A12}, -C(O)NR^{A12}R^{B12}, -C(O)OR^{A12}, -S(O)ᵣR^{A12} and - S(O)ᵣNR^{A12}R^{B12}, wherein alkyl, cycloalkyl heterocyclyl, aryl and heteroaryl are each unsubstituted or substituted with at least one substituent, independently selected from R^{X};
each R^{A7}, R^{A8}, R^{A12}, R^{B7}, R^{B8}, and R^{B12} are independently selected from hydrogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl and heteroaryl-C₁-₄ alkyl, wherein alkyl, alkenyl, alkynyl, cycloalkyl, heterocyclyl, aryl and heteroaryl are each unsubstituted or substituted with at least one substituent, independently selected from R^{X};
or "R^{A7} and R^{B7}" or "R^{A8} and R^{B8}" or "R^{A12} and R^{B12}" together with the atom(s) to which they are attached form a heterocyclic ring of 4 to 12 members containing 0, 1 or 2 additional heteroatoms independently selected from oxygen, sulfur, nitrogen and phosphorus and optionally substituted with **1, 2** or 3 R^{X} groups;
each R^{X} is independently selected from hydrogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, halogen, CN, NO₂, -(CR^{c1}R^{d1})ₜNR^{a1}R^{b1}, -(CR^{c1}R^{d1})ₜOR^{b1}, - (CR^{c1}R^{d1})ₜC(O)R^{a1}, -(CR^{c1}R^{d1})ₜC(=NR^{e1})R^{a1}, -(CR^{c1}R^{d1})ₜC(=N-OR^{b1})R^{a1}, -(CR^{c1}R^{d1})ₜC(O)OR^{b1}, - (CR^{c1}R^{d1})ₜOC(O)R^{b1}, -(CR^{c1}R^{d1})ₜC(O)NR^{a1}R^{b1}, -(CR^{c1}R^{d1})ₜNR^{a1}C(O)R^{b1}, - (CR^{c1}R^{d1})ₜC(=NR^{e1})NR^{a1}R^{b1}, -(CR^{c1}R^{d1})ₜNR^{a1}C(=NR^{e1})R^{b1}, -(CR^{c1}R^{d1})ₜOC(O)NR^{a1}R^{b1}, - (CR^{c1}R^{d1})ₜNR^{a1}C(O)OR^{b1}, -(CR^{c1}R^{d1})ₜNR^{a1}C(O)NR^{a1}R^{b1}, -(CR^{c1}R^{d1})ₜNR^{a1}C(S)NR^{a1}R^{b1}, - (CR^{c1}R^{d1})ₜNR^{a1}C(=NR^{c1})NR^{a1}R^{b1}, -(CR^{c1}R^{d1})ₜS(O)ᵣR^{b1}, -(CR^{c1}R^{d1})ₜS(O)(=NR^{e1})R^{b1}, - (CR^{c1}R^{d1})ₜN=S(O)R^{a1}R^{b1}, -(CR^{c1}R^{d1})ₜS(O)₂OR^{b1}, -(CR^{c1}R^{d1})ₜOS(O)2R^{b1}, -(CR^{c1}R^{d1})ₜNR^{a1}S(O)ᵣR^{b1}, - (CR^{c1}R^{d1})ₜNR^{a1}S(O)(=NR^{e1})R^{b1}, -(CR^{c1}R^{d1})ₜS(O)ᵣNR^{a1}R^{b1}, -(CR^{c1}R^{d1})ₜS(O)(=NR^{e1})NR^{a1}R^{b1}, - (CR^{c1}R^{d1})ₜNR^{a1}S(O)₂NR^{a1}R^{b1}, -(CR^{c1}R^{d1})ₜNR^{a1}S(O)(=NR^{c1})NR^{a1}R^{b1}, -(CR^{c1}R^{d1})ₜP(O)R^{a1}R^{b1} and - (CR^{c1}R^{d1})ₜP(O)(OR^{a1})(OR^{b1}), wherein alkyl, alkenyl, alkynyl, cycloalkyl, heterocyclyl, aryl and heteroaryl are each unsubstituted or substituted with at least one substituent, independently selected from R^{Y};
each R^{a1} and each R^{b1} are independently selected from hydrogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl and heteroaryl-C₁-₄ alkyl, wherein alkyl, alkenyl, alkynyl, cycloalkyl, heterocyclyl, aryl and heteroaryl are each unsubstituted or substituted with at least one substituent, independently selected from R^{Y};
or R^{a1} and R^{b1} together with the atom(s) to which they are attached form a heterocyclic ring of 4 to 12 members containing 0, 1 or 2 additional heteroatoms independently selected from oxygen, sulfur, nitrogen and phosphorus, and optionally substituted with 1, 2 or 3 R^{Y} groups;
each R^{c1} and each R^{d1} are independently selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl and heteroaryl-C₁-₄ alkyl, wherein alkyl, alkenyl, alkynyl, cycloalkyl, heterocyclyl, aryl and heteroaryl are each unsubstituted or substituted with at least one substituent, independently selected from R^{Y};
each R^{e1} is independently selected from hydrogen, C₁₋₁₀ alkyl, C₃₋₁₀ cycloalkyl, C₃₋₁₀ cycloalkyl-C₁₋₄ alkyl, CN, NO₂, -OR^{a2}, -SR^{a2}, -S(O)ᵣR^{a2}, -C(O)R^{a2}, -C(O)OR^{a2}, -S(O)ᵣNR^{a2}R^{b2} and - C(O)NR^{a2}R^{b2};
each R^{Y} is independently selected from C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁-₄ alkyl, halogen, CN, NO₂, -(CR^{c2}R^{d2})ₜNR^{a2}R^{b2}, -(CR^{c2}R^{d2})ₜOR^{b2}, -(CR^{c2}R^{d2})ₜC(O)R^{a2}, -(CR^{c2}R^{d2})ₜC(=NR^{e2})R^{a2}, -(CR^{c2}R^{d2})ₜC(=N-OR^{b2})R^{a2}, -(CR^{c2}R^{d2})ₜC(O)OR^{b2}, -(CR^{c2}R^{d2})ₜOC(O)R^{b2}, - (CR^{c2}R^{d2})ₜC(O)NR^{a2}R^{b2}, -(CR^{c2}R^{d2})ₜNR^{a2}C(O)R^{b2}, -(CR^{c2}R^{d2})ₜC(=NR^{c2})NR^{a2}R^{b2}, - (CR^{c2}R^{d2})NR^{a2}C(=NR^{e2})R^{b2}, -(CR^{c2}R^{d2})ₜOC(O)NR^{a2}R^{b2}, -(CR^{c2}R^{d2})ₜNR^{a2}C(O)OR^{b2}, - (CR^{c2}R^{d2})ₜNR^{a2}C(O)NR^{a2}R^{b2}, -(CR^{c2}R^{d2})ₜNR^{a2}C(S)NR^{a2}R^{b2}, -(CR^{c2}R^{d2})ₜNR^{a2}C(=NR^{e2})NR^{a2}R^{b2}, - (CR^{c2}R^{d2})ₜS(O)ᵣR^{b2}, -(CR^{c2}R^{d2})ₜS(O)(=NR^{e2})R^{b2}, -(CR^{c2}R^{d2})ₜN=S(O)R^{a2}R^{b2}, -(CR^{c2}R^{d2})ₜS(O)₂OR^{b2}, - (CR^{c2}R^{d2})ₜOS(O)₂R^{b2}, -(CR^{c2}R^{d2})ₜNR^{a2}S(O)ᵣR^{b2}, -(CR^{c2}R^{d2})ₜNR^{a2}S(O)(=NR^{e2})R^{b2}, - (CR^{c2}R^{d2})ₜS(O)ᵣNR^{a2}R^{b2}, -(CR^{c2}R^{d2})ₜS(O)(=NR^{e2})NR^{a2}R^{b2}, -(CR^{c2}R^{d2})ₜNR^{a2}S(O)₂NR^{a2}R^{b2}, - (CR^{c2}R^{d2})ₜNR^{a2}S(O)(=NR^{c2})NR^{a2}R^{b2}, -(CR^{c2}R^{d2})ₜP(O)R^{a2}R^{b2} and -(CR^{c2}R^{d2})ₜP(O)(OR^{a2})(OR^{b2}), wherein alkyl, alkenyl, alkynyl, cycloalkyl, heterocyclyl, aryl and heteroaryl are each unsubstituted or substituted with at least one substituent, independently selected from OH, CN, amino, halogen, C₁₋₁₀ alkyl, C₂₋₁₀ alkenyl, C₂₋₁₀ alkynyl, C₃₋₁₀ cycloalkyl, C₁₋₁₀ alkoxy, C₃₋₁₀ cycloalkoxy, C₁₋₁₀ alkylthio, C₃₋₁₀ cycloalkylthio, C₁₋₁₀ alkylamino, C₃₋₁₀ cycloalkylamino and di(C₁₋₁₀ alkyl)amino;
each R^{a2} and each R^{b2} are independently selected from hydrogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, C₁₋₁₀ alkoxy, C₃₋₁₀ cycloalkoxy, C₁₋₁₀ alkylthio, C₃₋₁₀ cycloalkylthio, C₁₋₁₀ alkylamino, C₃₋₁₀ cycloalkylamino, di(C₁₋₁₀ alkyl)amino, heterocyclyl, heterocyclyl-C₁-₄ alkyl, aryl, aryl-C₁₋₄ alkyl, heteroaryl and heteroaryl-C₁-₄ alkyl, wherein alkyl, alkenyl, alkynyl, cycloalkyl, alkoxy, cycloalkoxy, alkylthio, cycloalkylthio, alkylamino, cycloalkylamino, heterocyclyl, aryl and heteroaryl are each unsubstituted or substituted with at least one substituent, independently selected from halogen, CN, C₁₋₁₀ alkyl, C₂₋₁₀ alkenyl, C₂₋₁₀ alkynyl, C₃₋₁₀ cycloalkyl, OH, C₁₋₁₀ alkoxy, C₃₋₁₀ cycloalkoxy, C₁₋₁₀ alkylthio, C₃₋₁₀ cycloalkylthio, amino, C₁₋₁₀ alkylamino, C₃₋₁₀ cycloalkylamino and di(C₁₋₁₀ alkyl)amino;
each R^{c2} and each R^{d2} are independently selected from hydrogen, halogen, C₁₋₁₀ alkyl, C₂₋₁₀ alkenyl, C₂₋₁₀ alkynyl, C₃₋₁₀ cycloalkyl, C₃₋₁₀ cycloalkyl-C₁₋₄ alkyl, C₁₋₁₀ alkoxy, C₃₋₁₀ cycloalkoxy, C₁₋₁₀ alkylthio, C₃₋₁₀ cycloalkylthio, C₁₋₁₀ alkylamino, C₃₋₁₀ cycloalkylamino, di(C₁₋₁₀ alkyl)amino, heterocyclyl, heterocyclyl-C₁₋₄ alkyl, aryl, aryl-C₁₋₄ alkyl, heteroaryl and heteroaryl-C₁-₄ alkyl, wherein alkyl, alkenyl, alkynyl, cycloalkyl, alkoxy, cycloalkoxy, alkylthio, cycloalkylthio, alkylamino, cycloalkylamino, heterocyclyl, aryl and heteroaryl are each unsubstituted or substituted with at least one substituent, independently selected from halogen, CN, C₁₋₁₀ alkyl, C₂₋₁₀ alkenyl, C₂₋₁₀ alkynyl, C₃₋₁₀ cycloalkyl, OH, C₁₋₁₀ alkoxy, C₃₋₁₀ cycloalkoxy, C₁₋₁₀ alkylthio, C₃₋₁₀ cycloalkylthio, amino, C₁₋₁₀ alkylamino, C₃₋₁₀ cycloalkylamino and di(C₁₋₁₀ alkyl)amino;
each R^{e2} is independently selected from hydrogen, CN, NO₂, C₁₋₁₀ alkyl, C₃₋₁₀ cycloalkyl, C₃₋₁₀ cycloalkyl-C₁₋₄ alkyl, C₁₋₁₀ alkoxy, C₃₋₁₀ cycloalkoxy, -C(O)C₁₋₄ alkyl, -C(O)C₃₋₁₀ cycloalkyl,-C(O)OC₁₋₄ alkyl, -C(O)OC₃₋₁₀ cycloalkyl, -C(O)N(C₁₋₄ alkyl)₂, -C(O)N(C₃₋₁₀ cycloalkyl)₂, -S(O)₂C₁₋₄ alkyl, -S(O)₂C₃₋₁₀ cycloalkyl, -S(O)₂N(C₁₋₄ alkyl)₂ and -S(O)₂N(C₃₋₁₀ cycloalkyl)₂;
m, n, p, and q are independently selected from 0, 1, 2 and 3;
m1, m2, n1, n2, p1 and p2 are independently selected from 0 and 1;
each r is independently selected from 0, 1 and 2;
each t is independently selected from 0, 1, 2, 3 and 4;
each u is independently selected from 0, 1, 2, 3 and 4.

2. A compound of claim 1 or a pharmaceutically acceptable salt thereof, wherein
X³ is CH, A is N, B is CH, Z is N, Y¹, Y² and Y³ are CH, shown as formula (II), 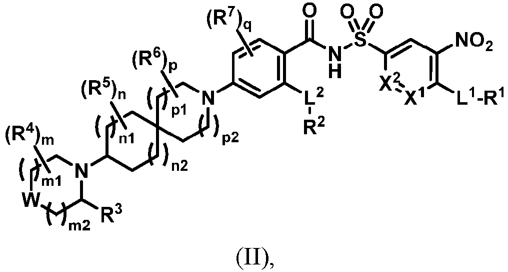
wherein
X¹ is selected from N and CR⁸, X² is selected from N and CR⁹, wherein at least one of X¹ and X² is N; W is selected from -CHR¹¹-, -NR¹²-, -O- and -S(O)ᵣ-; L¹, L² , R¹, R² , R³ , R⁴ , R⁵, R⁶, R⁷, R⁸, R⁹, R¹¹, R¹², m, m1, m2, n, n1, n2, p, p1, p2 and q are as defined in formula (I).

3. A compound of any one of claims 1-2 or a pharmaceutically acceptable salt thereof, wherein
(i) R¹ is selected from 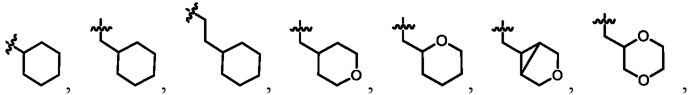 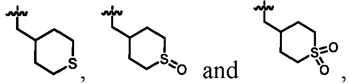 which are unsubstituted or substituted with at least one substituent, independently selected from R^{X};
and/or
(ii) each substituent R^{X} of R¹ is independently selected from C₁₋₁₀ alkyl, C₃₋₁₀ cycloalkyl, halogen, CN, NO₂, -(CR^{c1}R^{d1})ₜNR^{a1}R^{b1}, -(CR^{c1}R^{d1})ₜOR^{b1}, -(CR^{c1}R^{d1})ₜC(O)R^{a1} and - (CR^{c1}R^{d1})ₜN=S(O)R^{a1}R^{b1}, wherein alkyl, cycloalkyl are each unsubstituted or substituted with at least one substituent, independently selected from R^{Y};
for example,
wherein each R^{a1} and each R^{b1} are independently selected from hydrogen, C₁₋₁₀ alkyl, C₂₋₁₀ alkenyl and C₂₋₁₀ alkynyl, wherein alkyl, alkenyl and alkynyl are each unsubstituted or substituted with at least one substituent, independently selected from R^{Y};
or
wherein R^{a1} and R^{b1} together with the atom(s) to which they are attached form a heterocyclic ring of 4 to 8 members containing 0, 1 or 2 additional heteroatoms independently selected from oxygen, sulfur, nitrogen and phosphorus, and optionally substituted with 1, 2 or 3 R^{Y} groups;
or
each substituent R^{X} of R¹ is independently selected from OH, CN, halogen, C₁₋₁₀ alkyl,

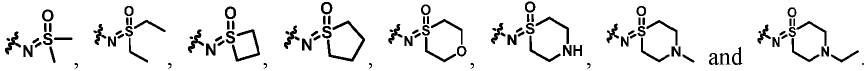

4. A compound of any one of claims 1-3 or a pharmaceutically acceptable salt thereof, wherein
(i) R¹ is selected from 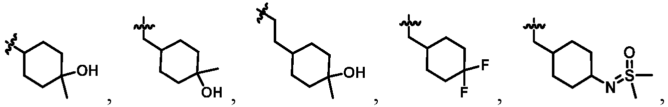 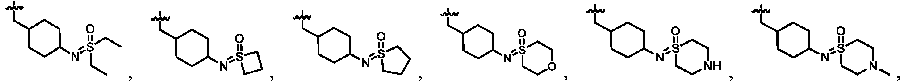 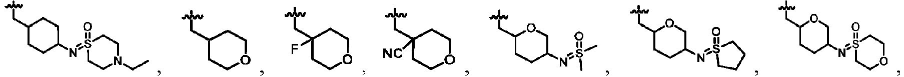 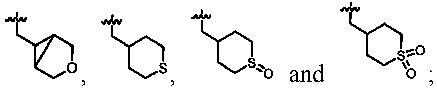 and/or
(ii) R⁸ is hydrogen.

5. A compound of any one of claims 1-4 or a pharmaceutically acceptable salt thereof, wherein
R¹ and R⁸ together with the atoms to which they are attached form a heterocyclic ring of 5 to 12 members containing 1, 2 or 3 heteroatoms independently selected from oxygen, sulfur and nitrogen, and optionally substituted with 1, 2 or 3 R^{X} groups;
for example,
wherein the moiety 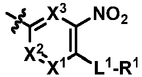 in formula (I) and the moiety 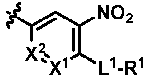 in formula (II) are selected from 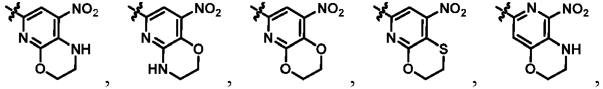 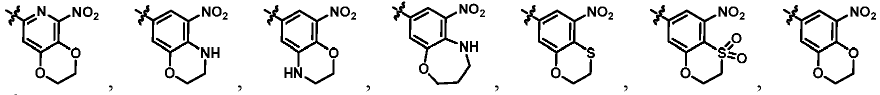 and 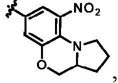 wherein the heterocyclic ring formed by R¹ and R⁸ is optionally substituted with 1, 2 or 3 R^{X} groups, wherein the 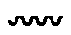 symbol indicates the point of attachment to the rest of the molecule.

6. A compound of claim 5 or a pharmaceutically acceptable salt thereof, wherein
each substituent R^{X} of the 5 to 12 membered heterocyclic ring is independently selected from C₁₋₁₀ alkyl, C₃₋₁₀ cycloalkyl, C₃₋₁₀ cycloalkyl-C₁₋₄ alkyl, heterocyclyl, heterocyclyl-C₁₋₄ alkyl, aryl, aryl-C₁₋₄ alkyl, heteroaryl, heteroaryl-C₁₋₄ alkyl, halogen, CN, NO₂, (CR^{c1}R^{d1})ₜNR^{a1}R^{b1} and - (CR^{c1}R^{d1})ₜOR^{b1}, wherein alkyl, cycloalkyl, heterocyclyl, aryl and heteroaryl are each unsubstituted or substituted with at least one substituent, independently selected from R^{Y};
for example,
each substituent R^{X} of the 5 to 12 membered heterocyclic ring is selected from halogen, CN, NO₂, OH, C₁₋₈ alkyl,

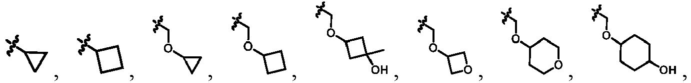

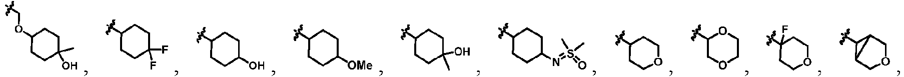

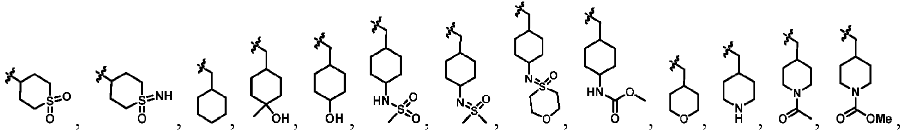

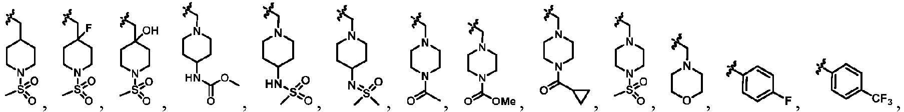

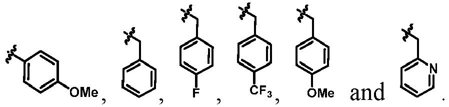

7. A compound of any one of claims 1-6 or a pharmaceutically acceptable salt thereof, wherein
(i) each substituent R^{X} of R² is independently selected from C₁₋₁₀ alkyl, C₂₋₁₀ alkenyl, C₂₋₁₀ alkynyl, C₃₋₁₀ cycloalkyl, heterocyclyl, heterocyclyl-C₁₋₄ alkyl, halogen, CN, NO₂, - (CR^{c1}R^{d1})ₜNR^{a1}R^{b1} , -(CR^{c1}R^{d1})ₜOR^{b1}, (CR^{c1}R^{d1})ₜC(O)OR^{b1}, -(CR^{c1}R^{d1})ₜC(O)NR^{a1}R^{b1}, - (CR^{c1}R^{d1})ₜNR^{a1}C(O)R^{b1}, -(CR^{c1}R^{d1})ₜNR^{a1}C(O)OR^{b1}, -(CR^{c1}R^{d1})ₜNR^{a1}C(O)NR^{a1}R^{b1}, - (CR^{c1}R^{d1})ₜS(O)₂OR^{b1}, -(CR^{c1}R^{d1})ₜS(O)ᵣR^{b1}, -(CR^{c1}R^{d1})ₜNR^{a1}S(O)ᵣR^{b1}, -(CR^{c1}R^{d1})ₜS(O)ᵣNR^{a1}R^{b1} and - (CR^{c1}R^{d1})ₜNR^{a1}S(O)₂NR^{a1}R^{b1}, wherein alkyl, alkenyl, alkynyl, cycloalkyl and heterocyclyl are each unsubstituted or substituted with at least one substituent, independently selected from R^{Y};
for example,
each substituent R^{X} of R² is selected from halogen, CN, NH₂, methyl, trifluoromethyl, cyclopropyl, methoxy, methoxyethoxy, and difluoromethoxy;
for example,
wherein R^{a1} and R^{b1} are independently selected from hydrogen, C₁₋₁₀ alkyl, C₃₋₁₀ cycloalkyl, C₃₋₁₀ cycloalkyl-C₁₋₄ alkyl, heterocyclyl and heterocyclyl-C₁₋₄ alkyl, wherein alkyl, cycloalkyl and heterocyclyl are each unsubstituted or substituted with at least one substituent, independently selected from R^{Y};
or
(ii) R² is selected from

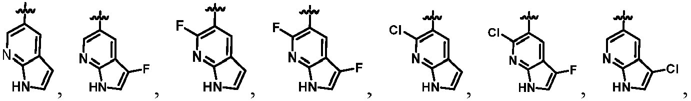

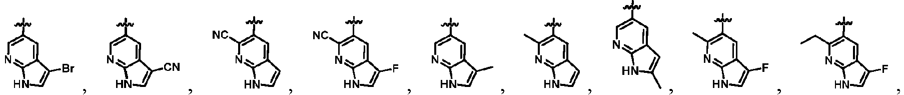

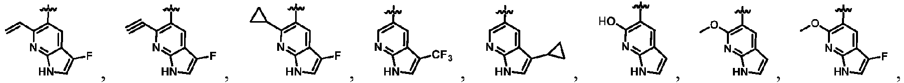

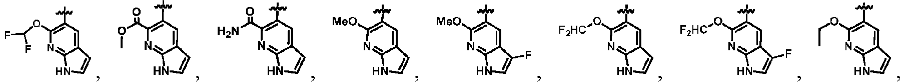

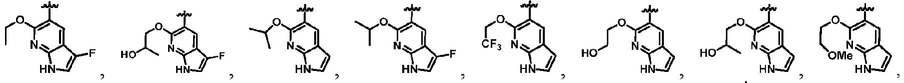

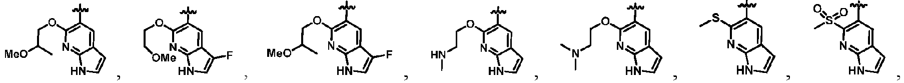

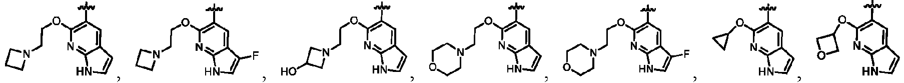

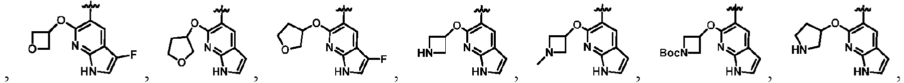

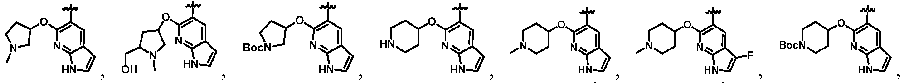

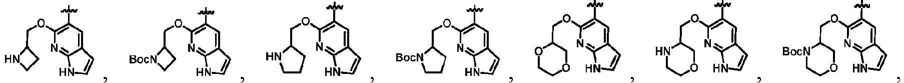

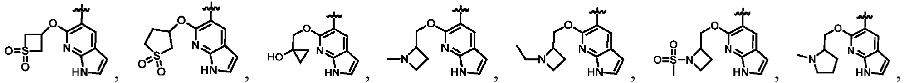

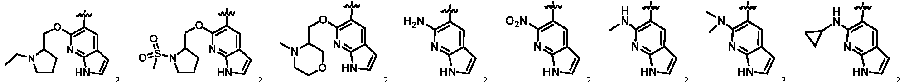

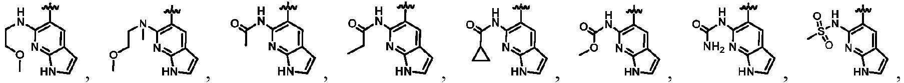

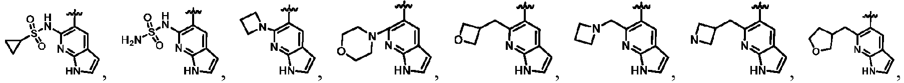

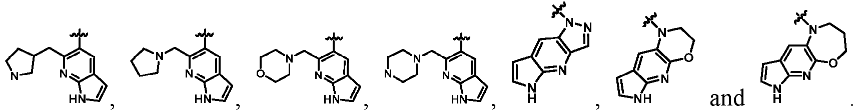

8. A compound of any one of claims 1-7 or a pharmaceutically acceptable salt thereof, wherein
W is selected from -CH₂-, -NR¹²-, -O-, -S(O)ᵣ- and -S(O)(=NR¹²)-.

9. A compound of any one of claims 1-8 or a pharmaceutically acceptable salt thereof, wherein
(i) R¹¹ is hydrogen;
and/or
(ii) R¹² is selected from hydrogen, methyl, ethyl, cyclopropyl, -C(O)CH₃, -C(O)OCH₃, - S(O)₂CH₃,

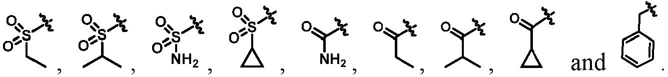

10. A compound of any one of claims 1-9 or a pharmaceutically acceptable salt thereof, wherein
(i) each substituent R^{X} of R³ is selected from halogen, C₁₋₁₀ alkyl, C₂₋₁₀ alkenyl, C₃₋₁₀ cycloalkyl, wherein alkyl, alkenyl and cycloalkyl are each unsubstituted or substituted with at least one substituent, independently selected from R^{Y};
for example,
each substituent R^{X} of R³ is selected from halogen, methyl, ethyl, isopropyl, tert-butyl, propenyl and cyclopropyl, wherein methyl, ethyl, isopropyl, propenyl and cyclopropyl are each unsubstituted or substituted with at least one substituent, independently selected from halogen, C₁₋₁₀ alkyl, CN, NO₂, -NH₂ and -OH;
or
(ii) R³ is selected from 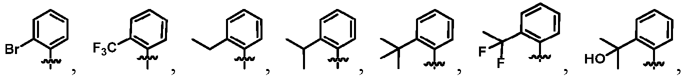 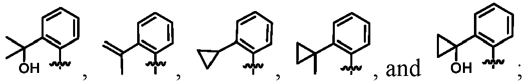 and/or
(iii) m is selected from 0, 1 and 2.

11. A compound of any one of claims 1-8 or a pharmaceutically acceptable salt thereof, wherein the moiety 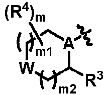 in formula (I) and formula (II) is selected from 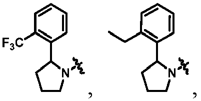 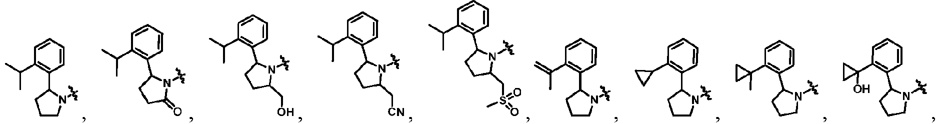 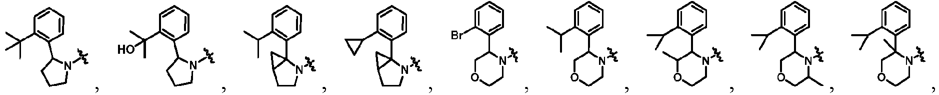 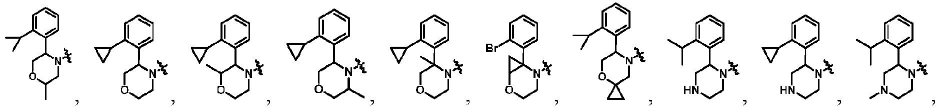 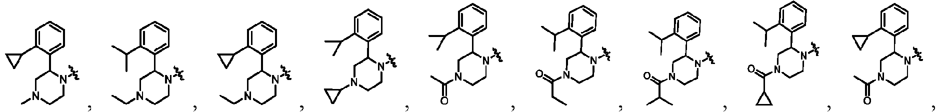 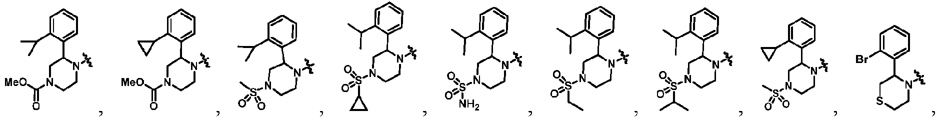 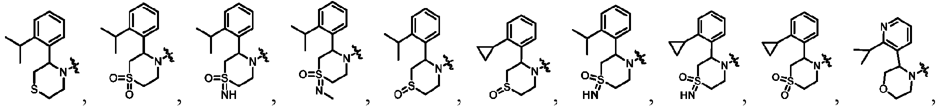 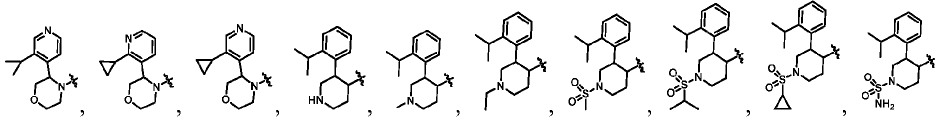 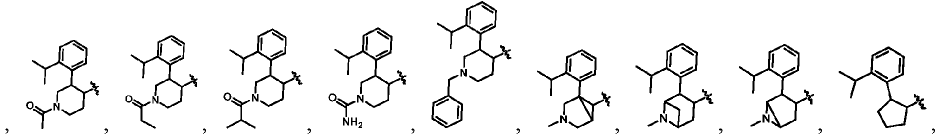 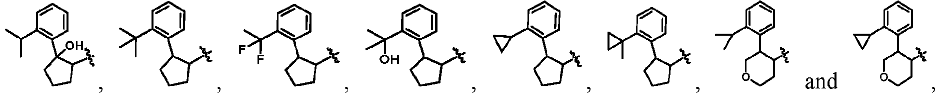 wherein the 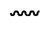 symbol indicates the point of attachment to the rest of the molecule.

12. A compound of any one of claims 1-9 or a pharmaceutically acceptable salt thereof, wherein
(i) R⁵ and R⁶ are H;
and/or
(ii) the moiety 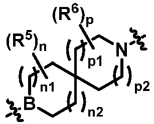 in formula (I) and formula (II) is selected from 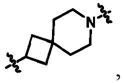 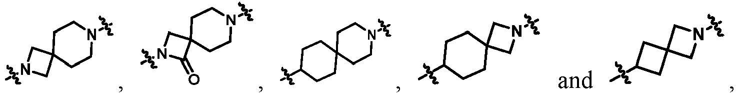 wherein the 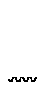 symbol indicates the point of attachment to the rest of the molecule;
and/or
(iii) each R⁷ is hydrogen.

13. A compound, wherein the compound is selected from 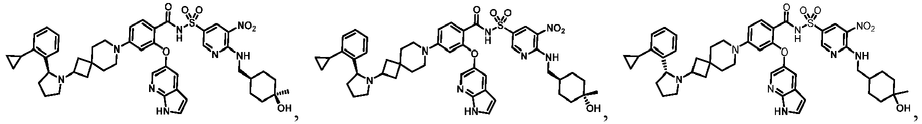 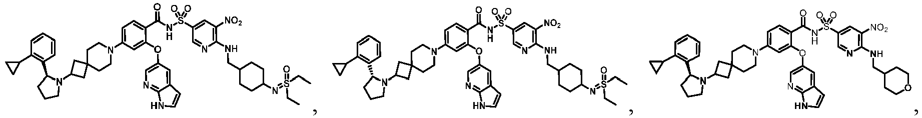 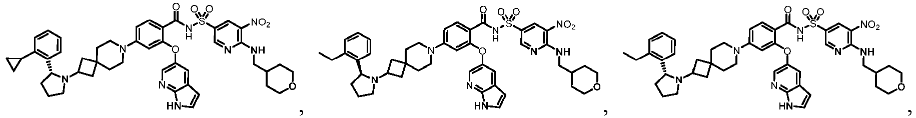 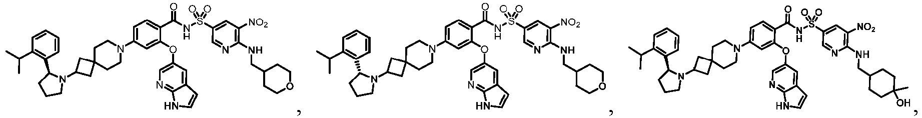 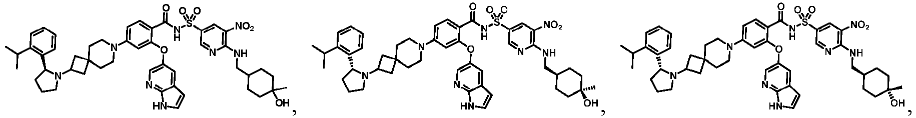 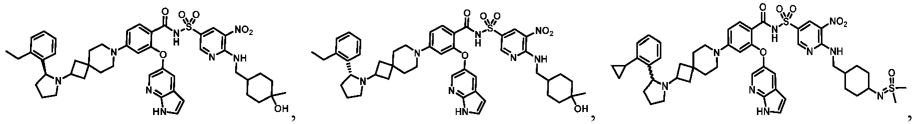 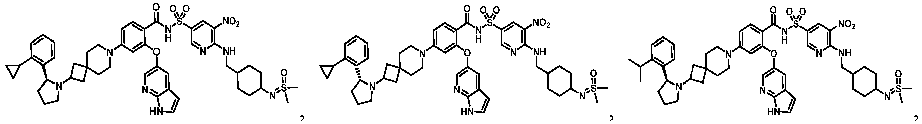 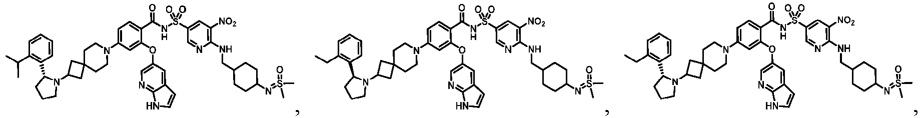 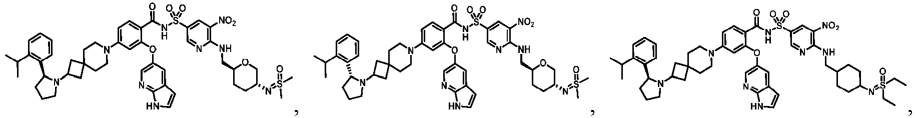 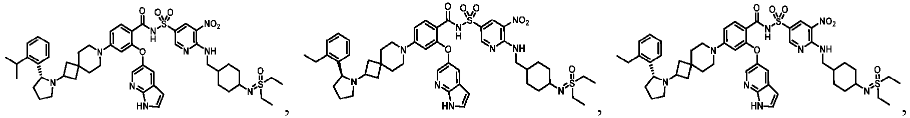 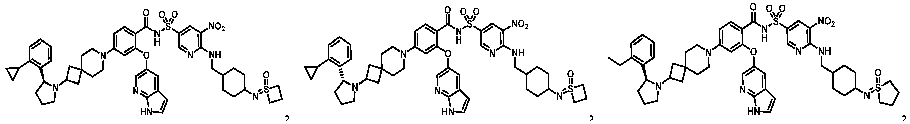 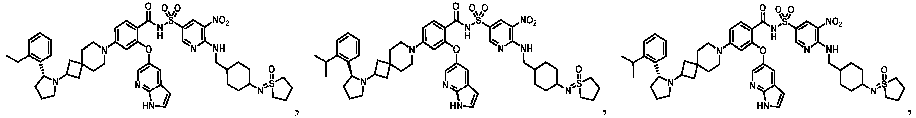 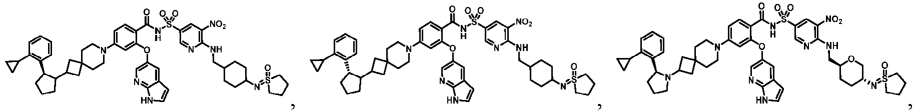 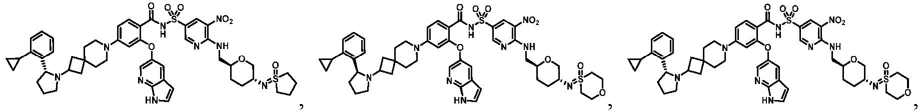 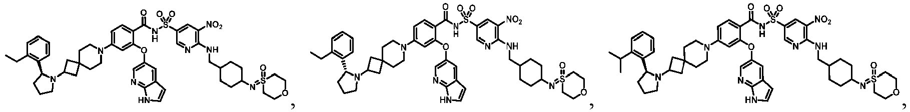 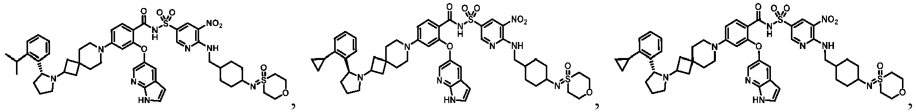 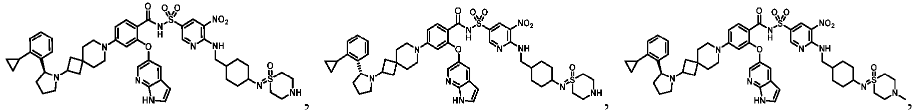 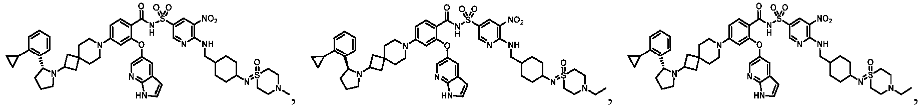 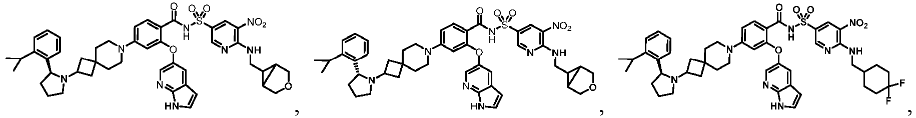 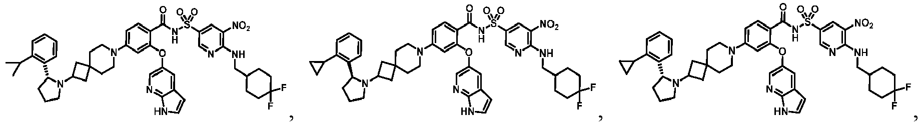 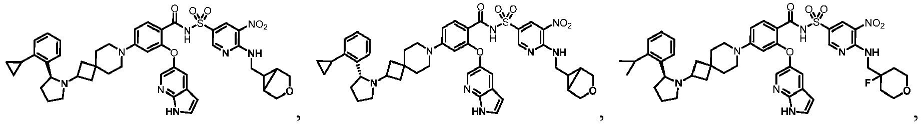 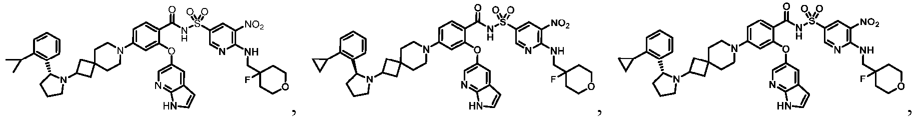 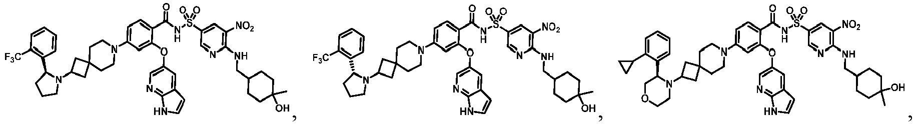 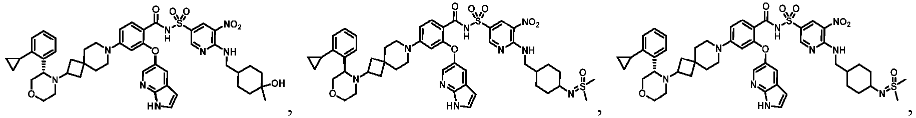 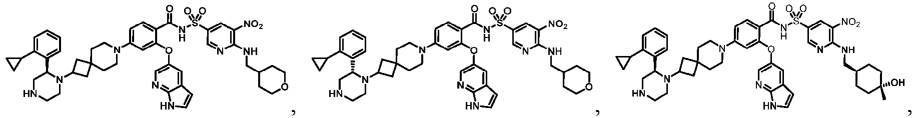 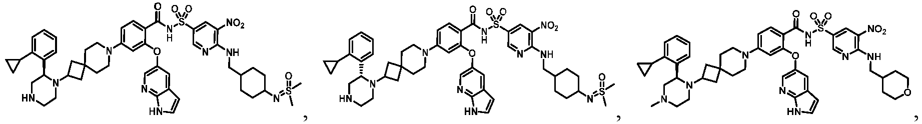 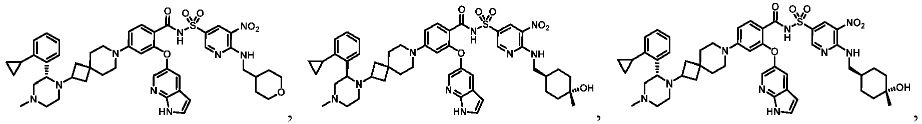 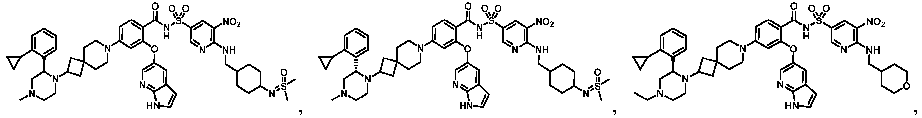 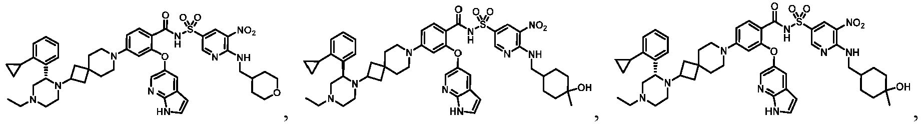 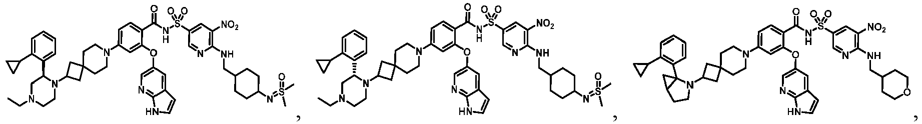 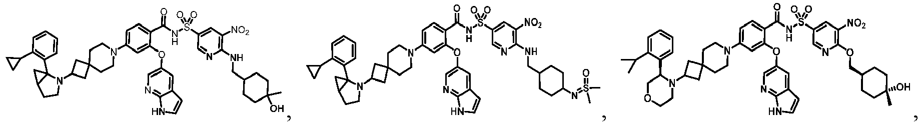 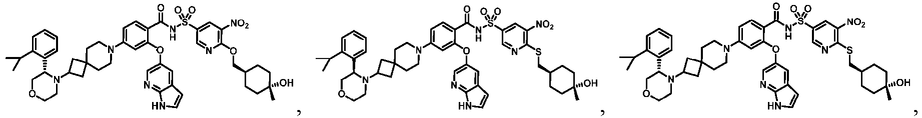 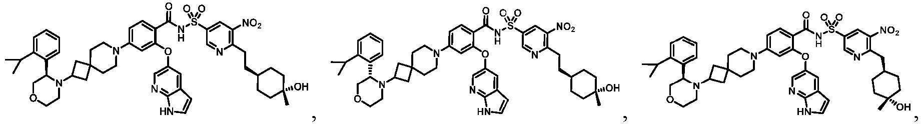 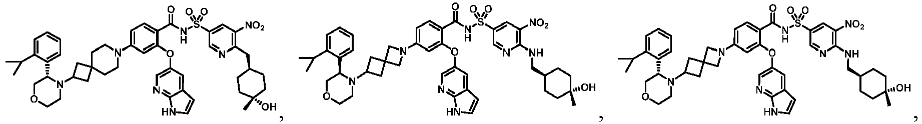 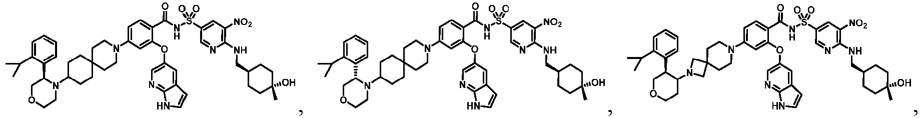 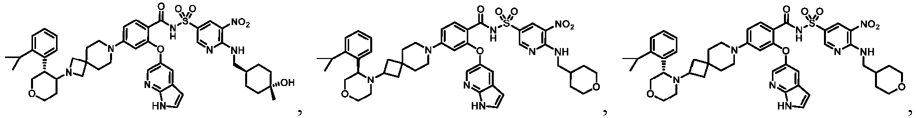 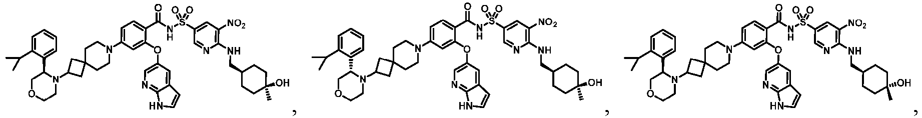 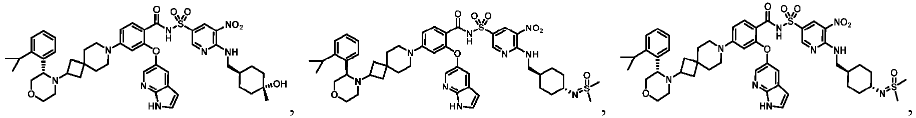 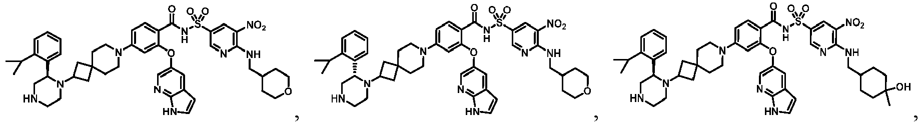 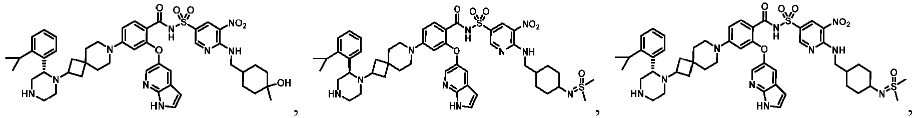 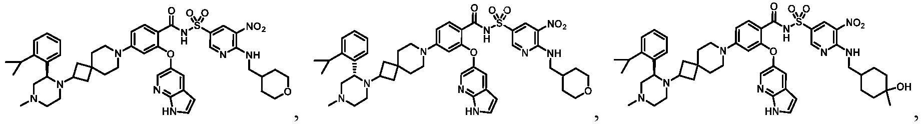 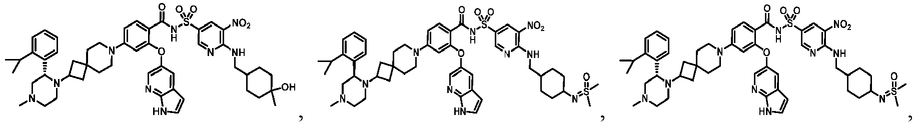 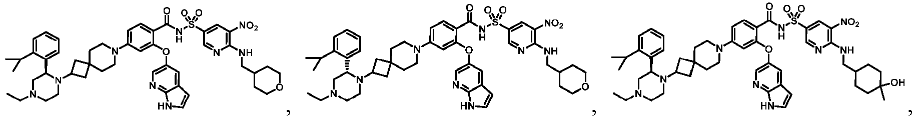 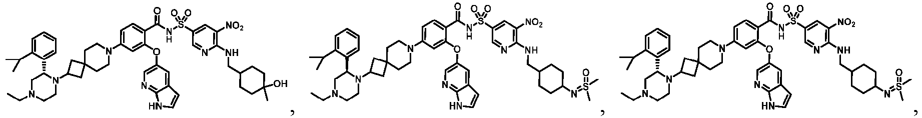 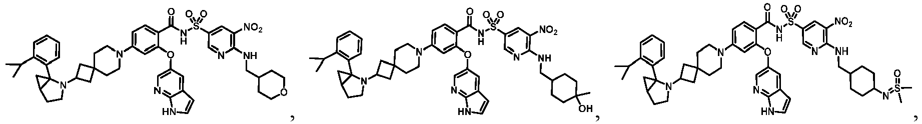 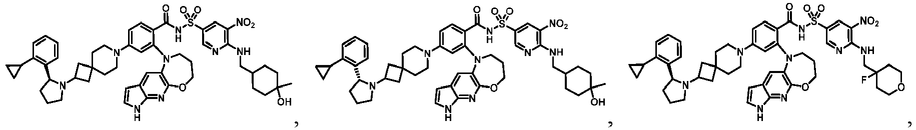 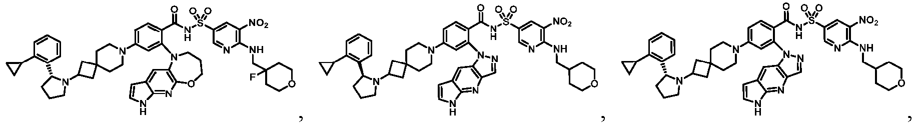 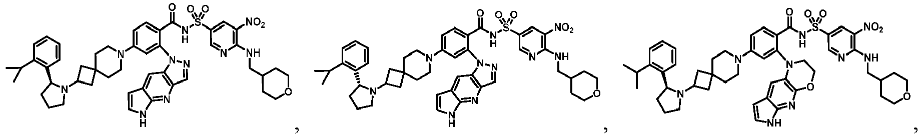 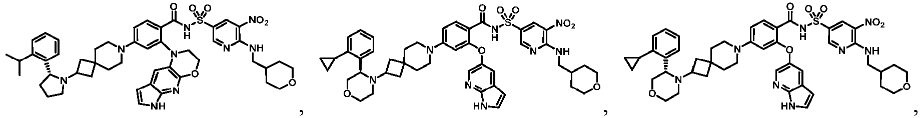 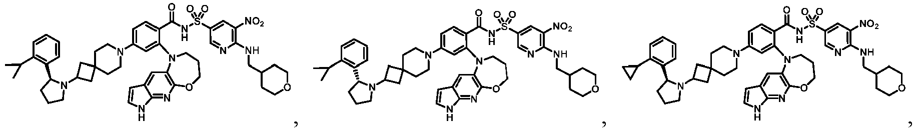 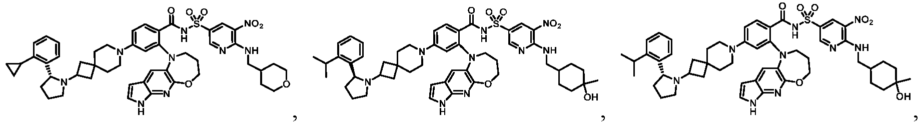 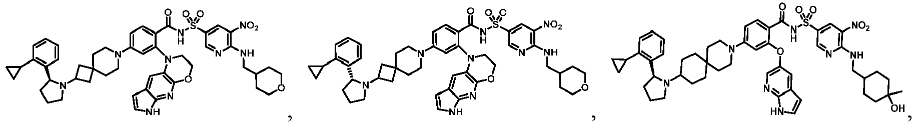 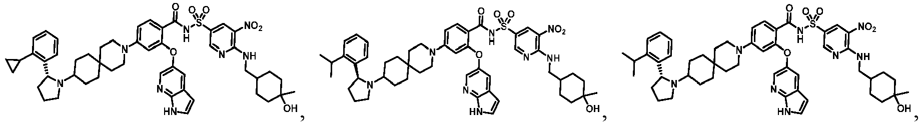 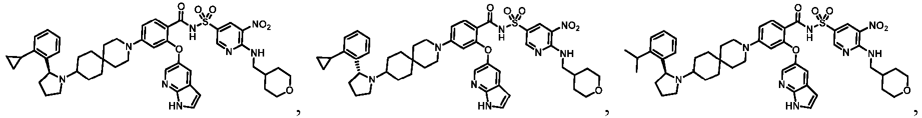 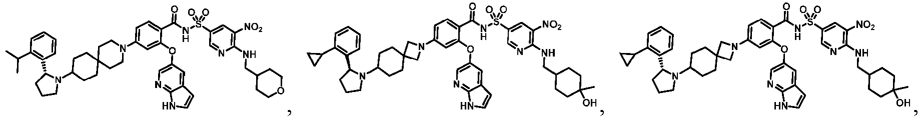 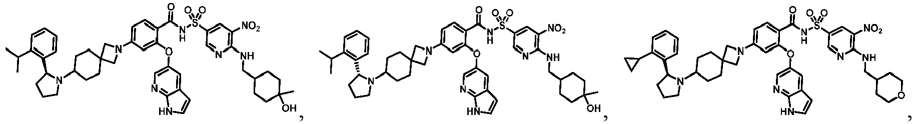 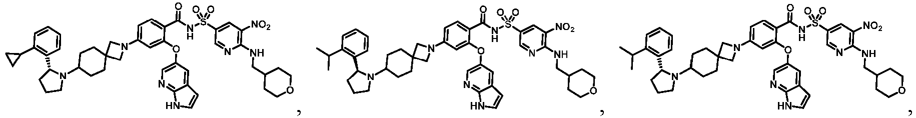 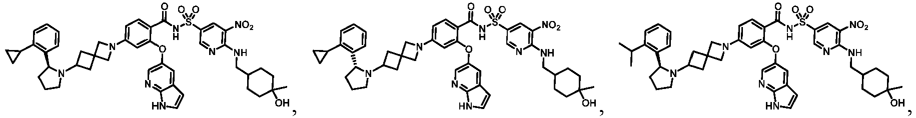 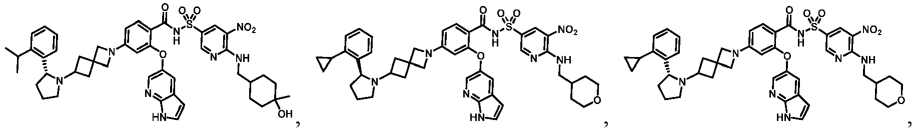 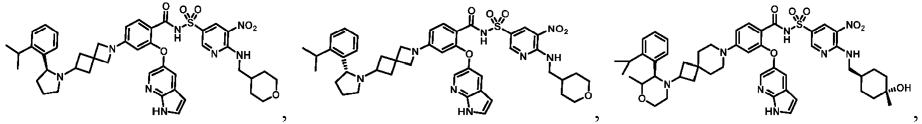 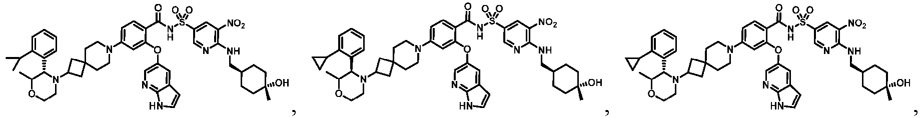 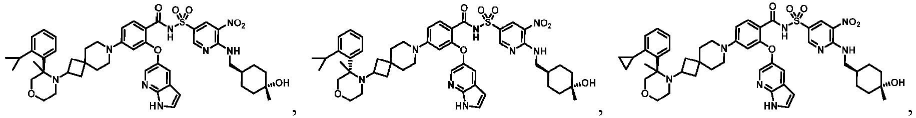 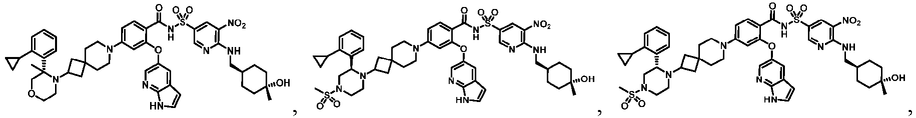 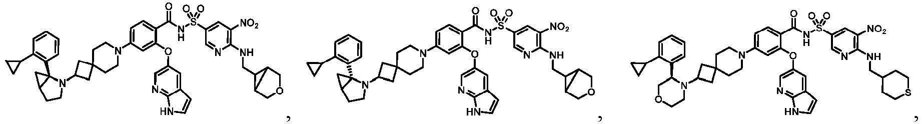 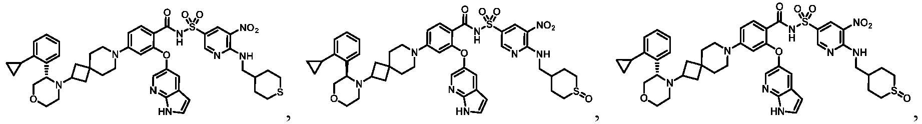 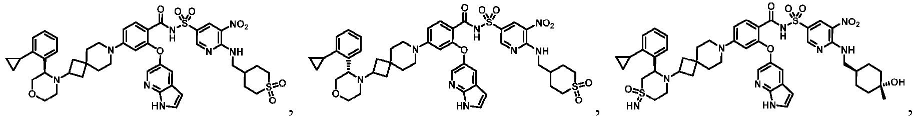 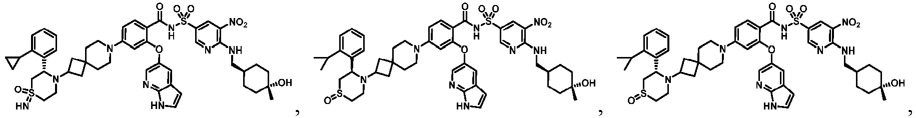 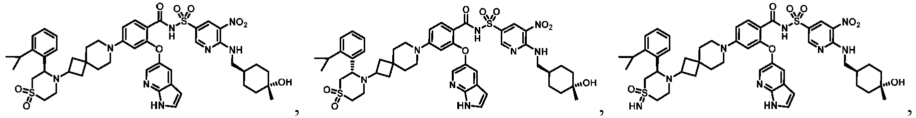 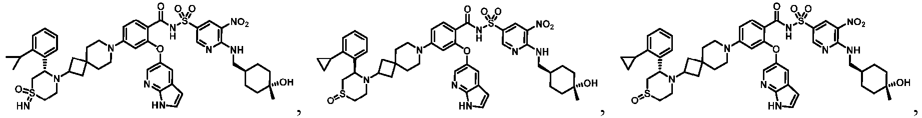 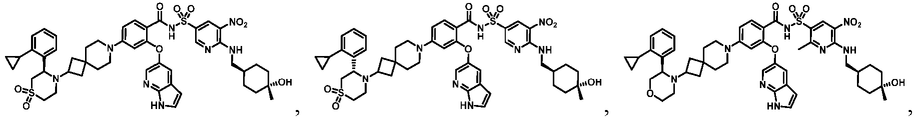 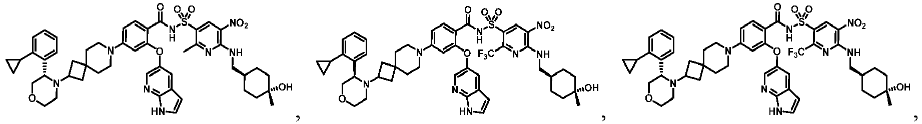 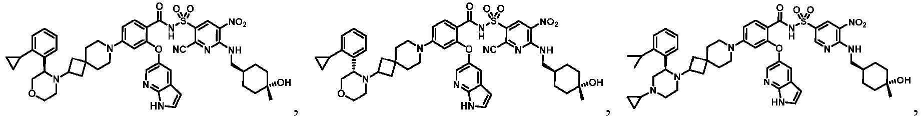 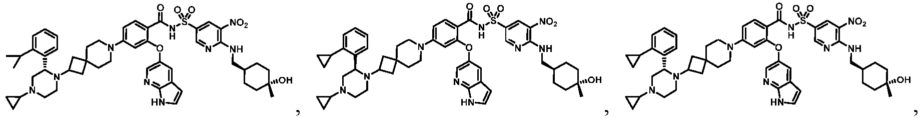 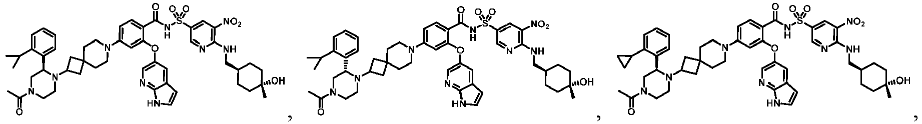 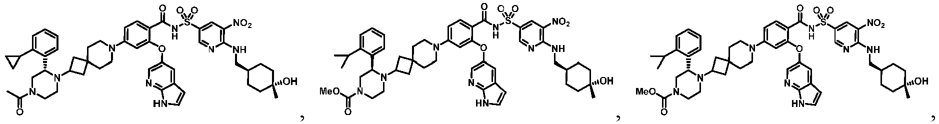 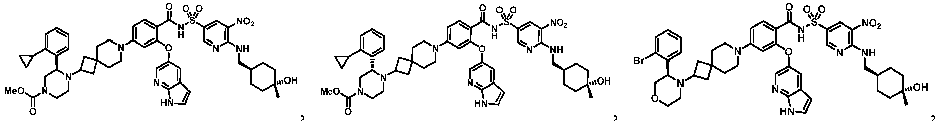 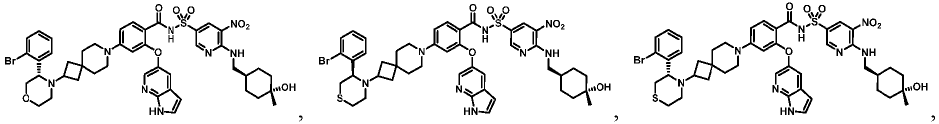 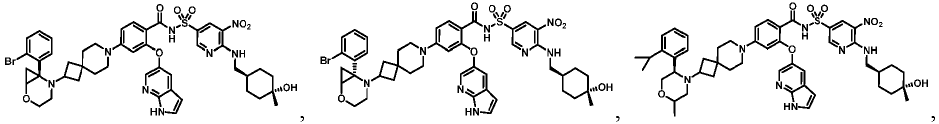 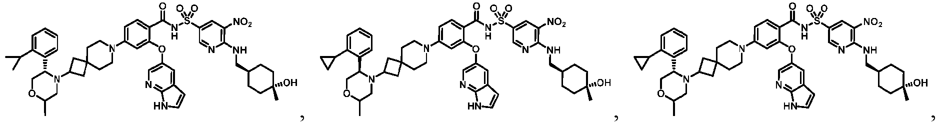 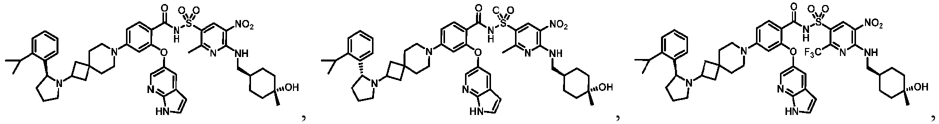 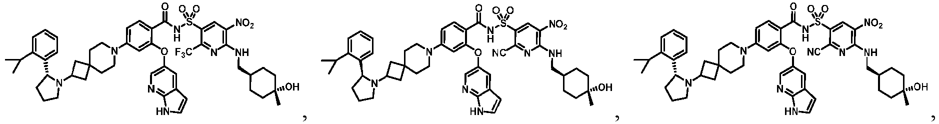 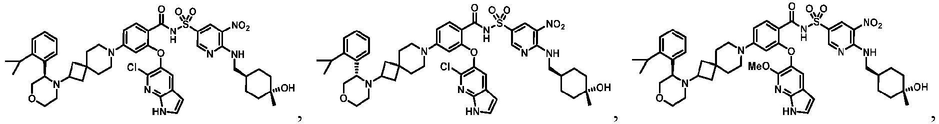 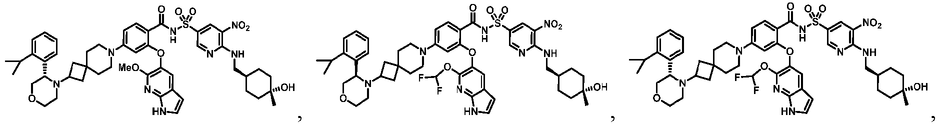 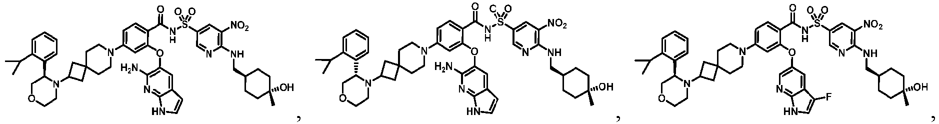 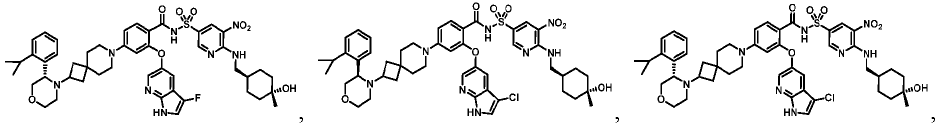 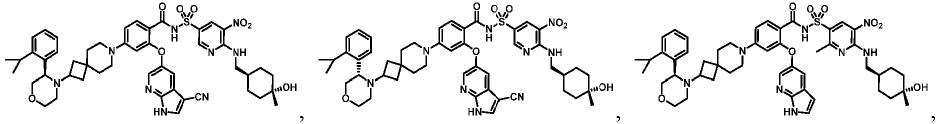 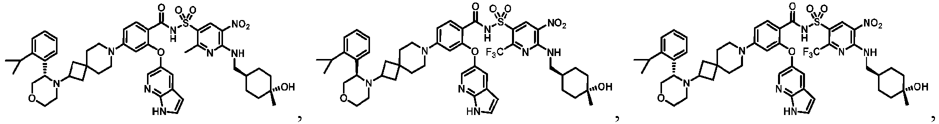 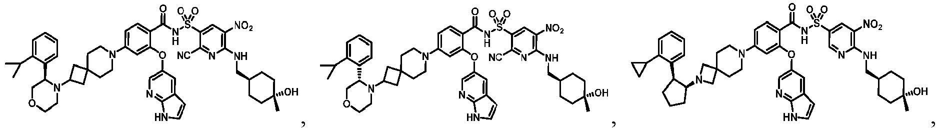 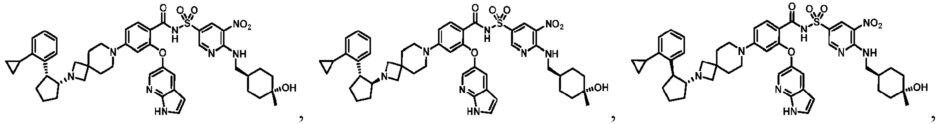 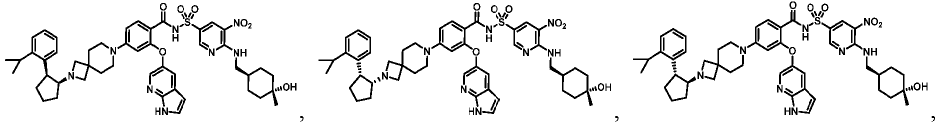 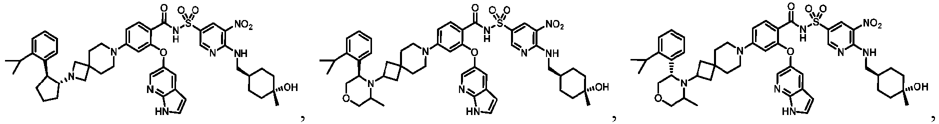 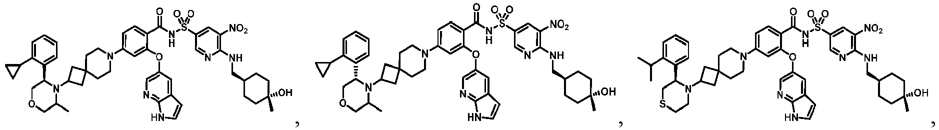 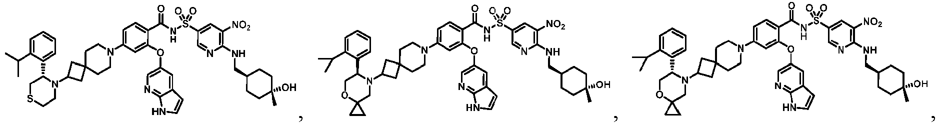 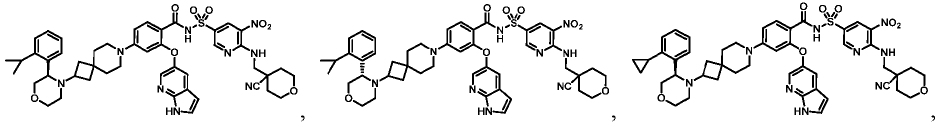 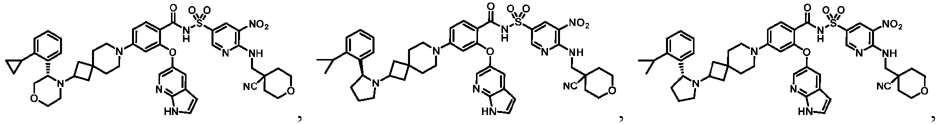 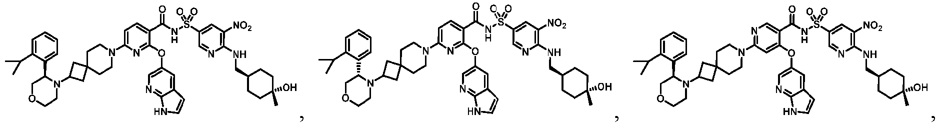 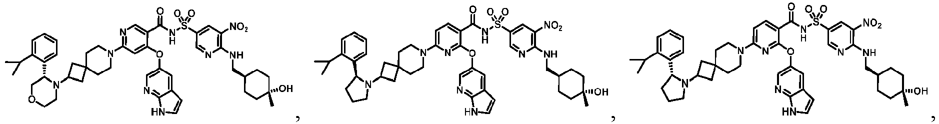 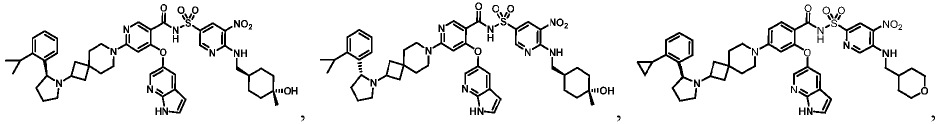 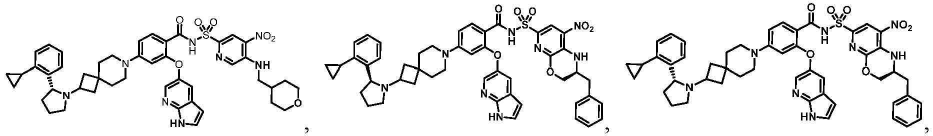 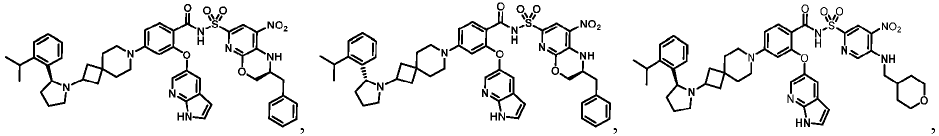 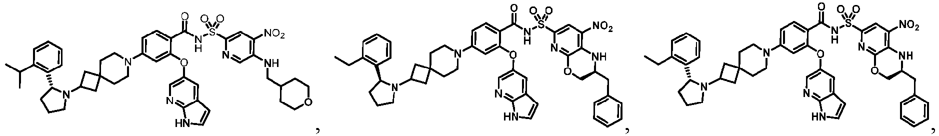 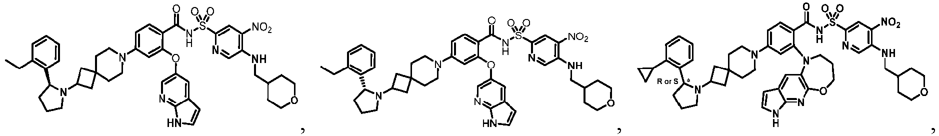 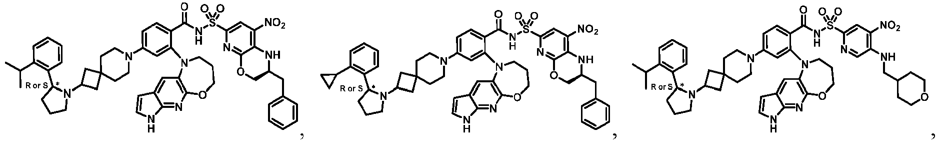 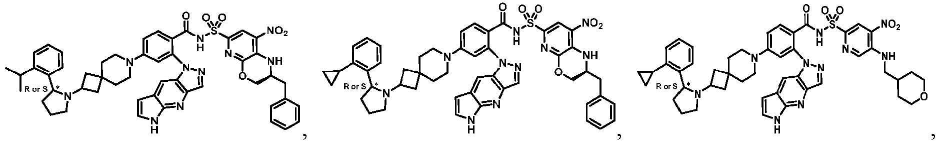 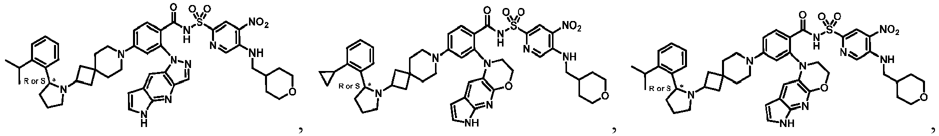 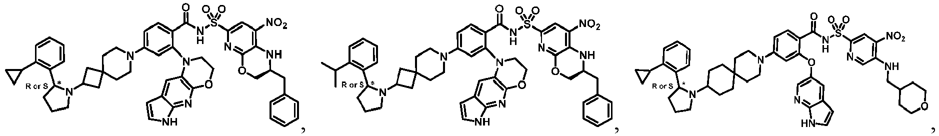 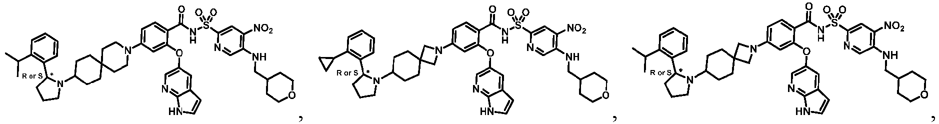 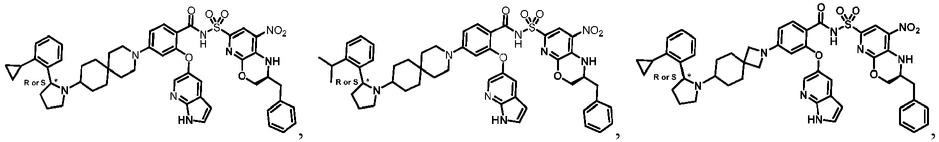 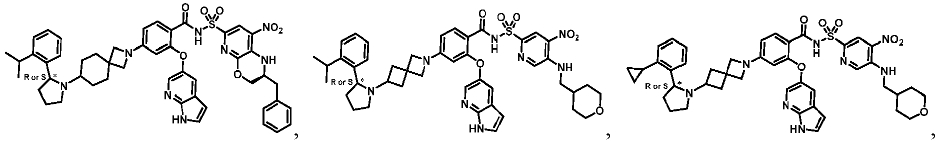 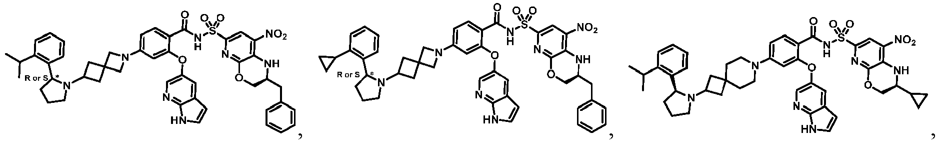 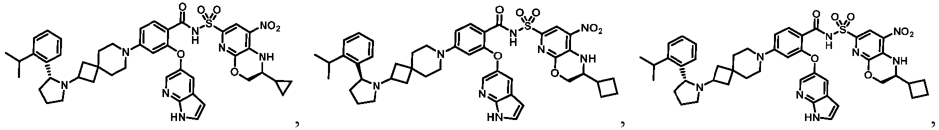 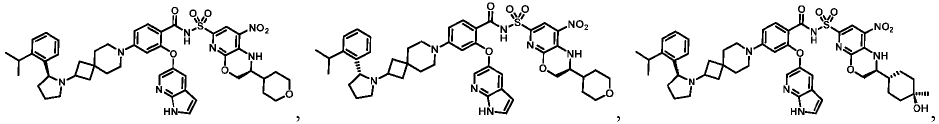 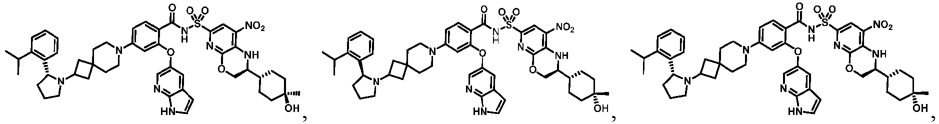 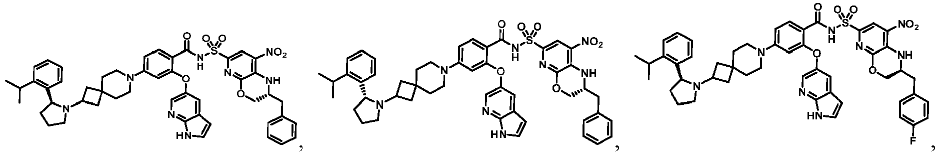 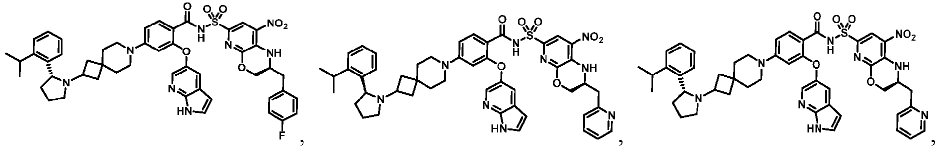 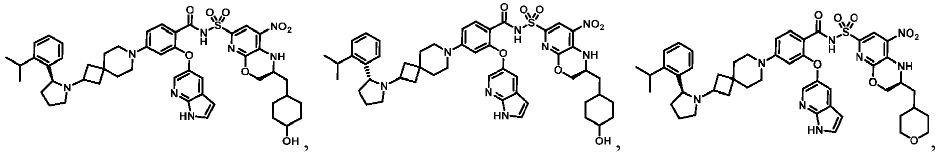 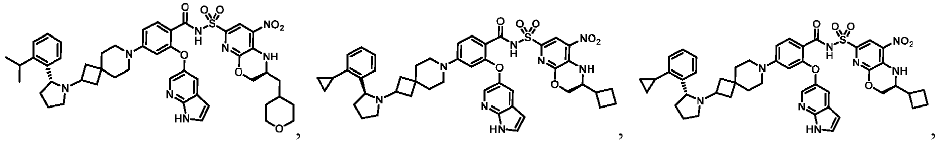 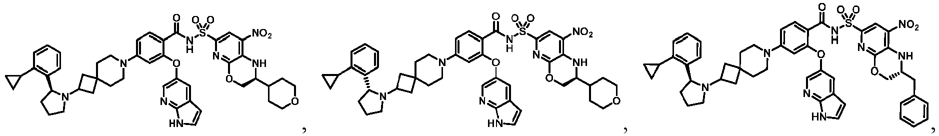 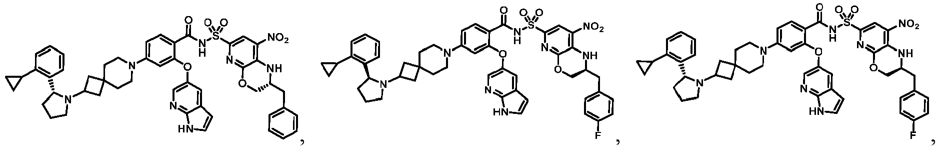 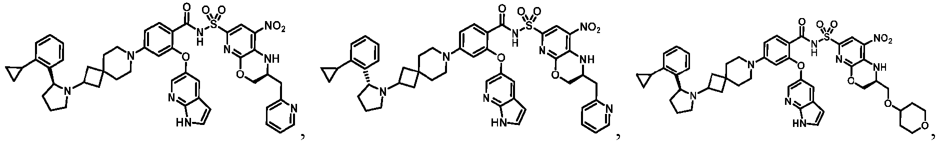 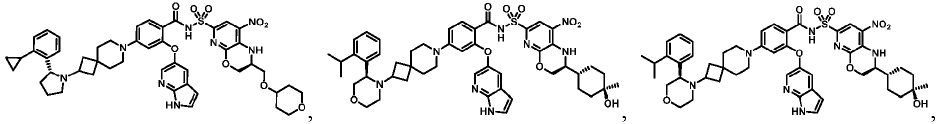 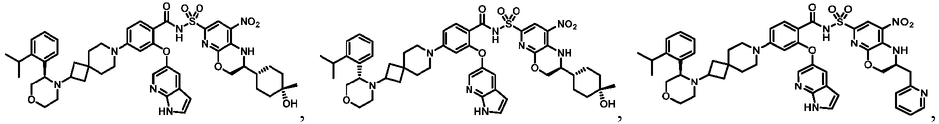 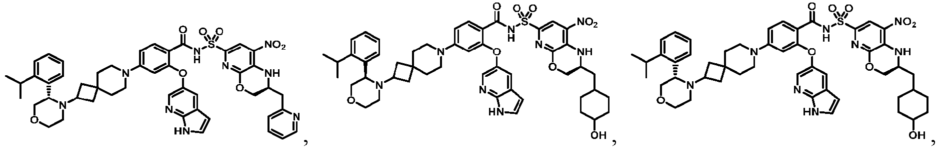 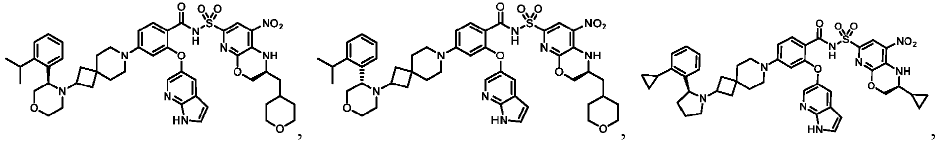 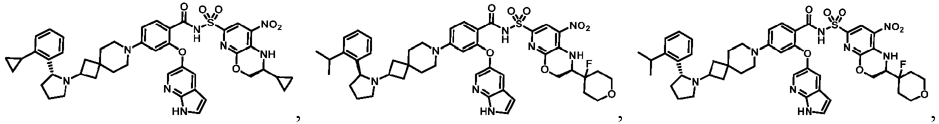 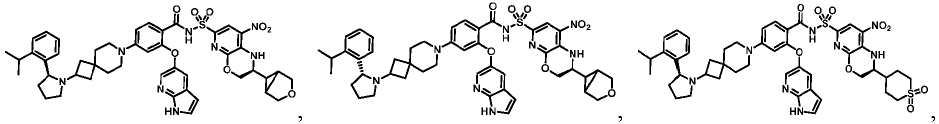 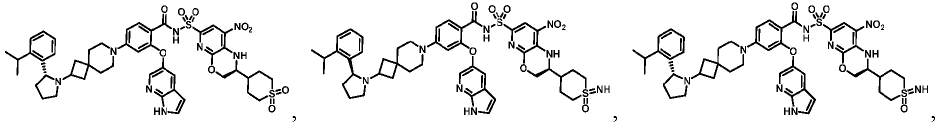 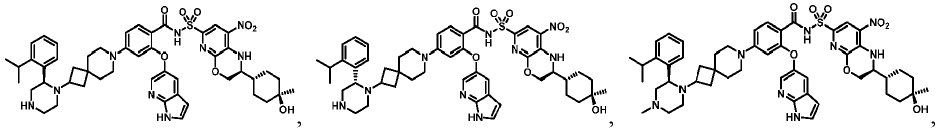 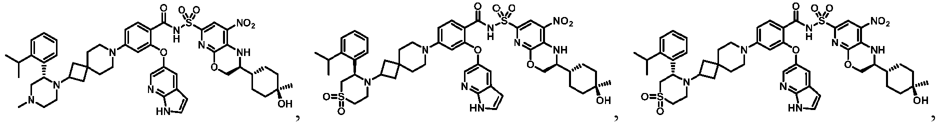 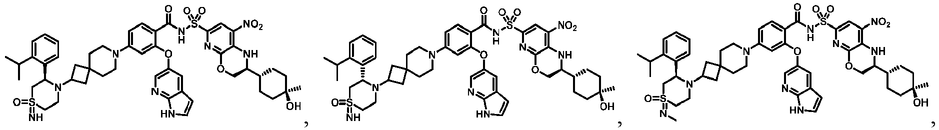 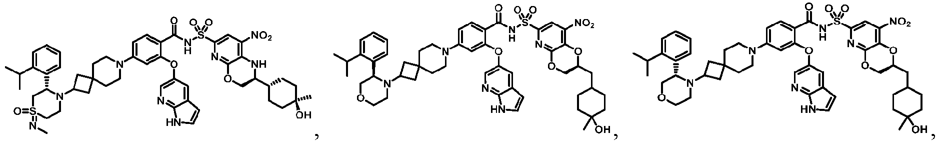 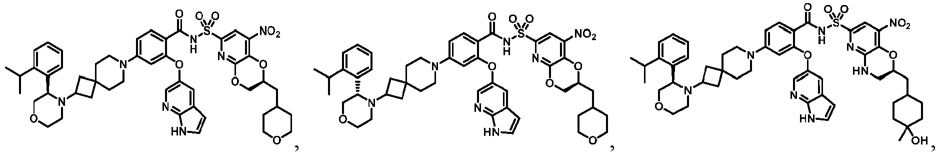 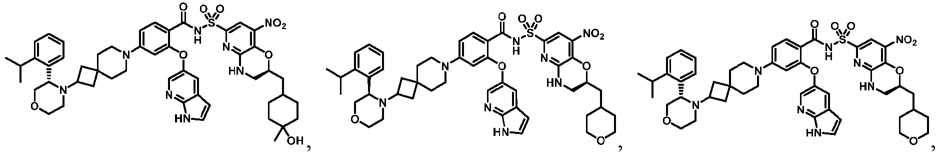 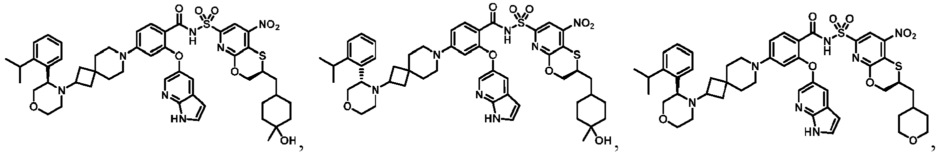 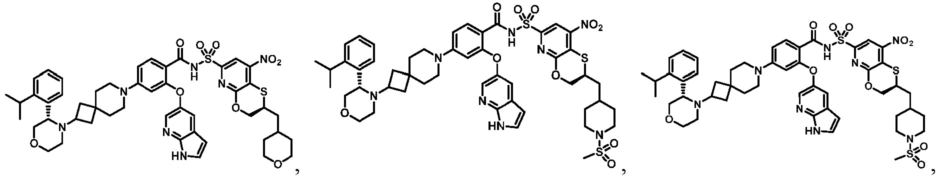 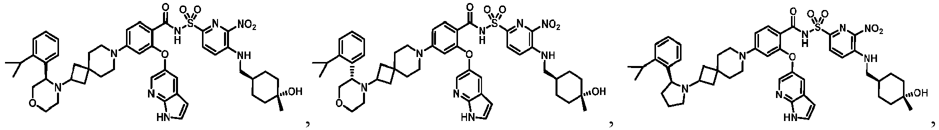 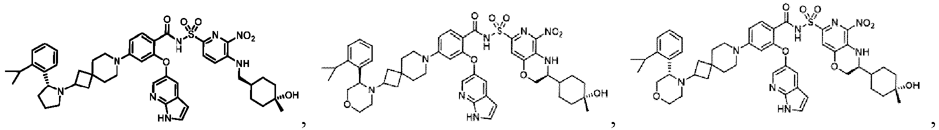 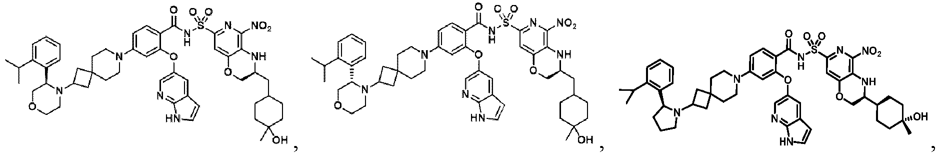 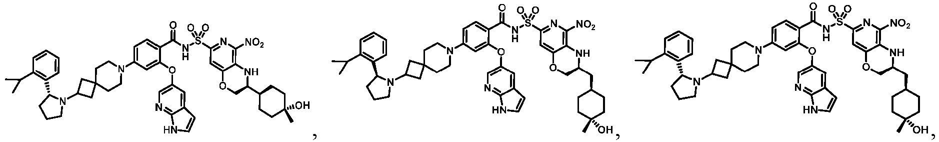 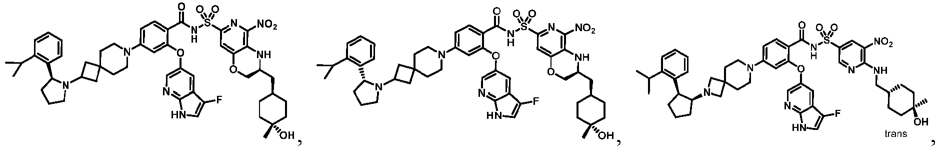 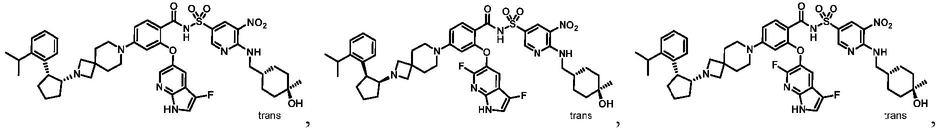 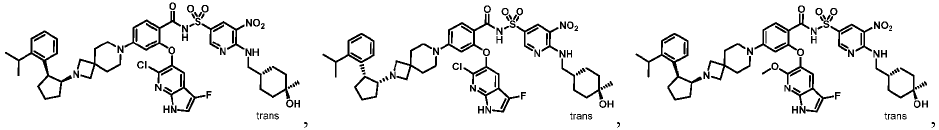 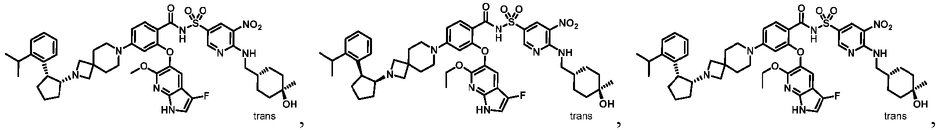 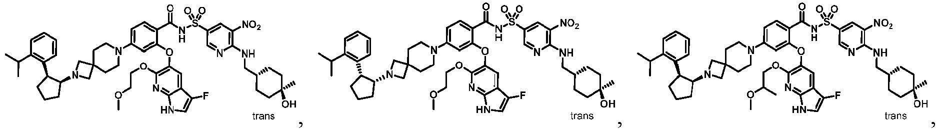 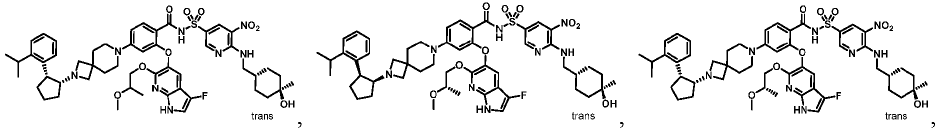 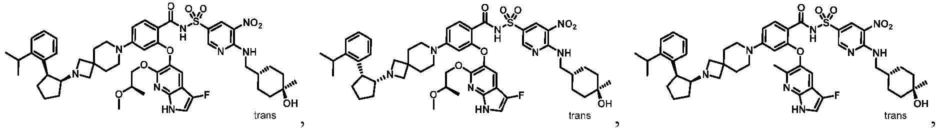 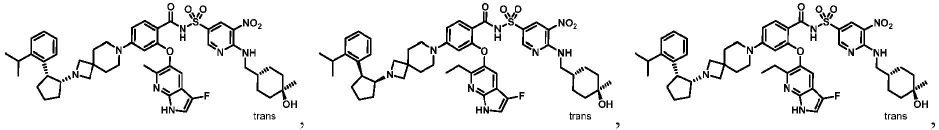 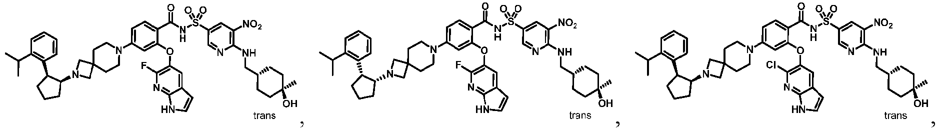 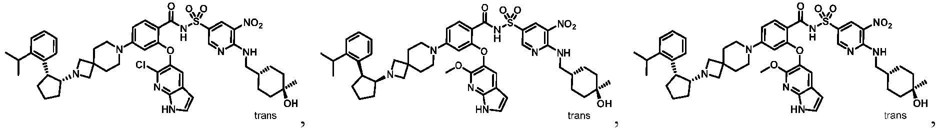 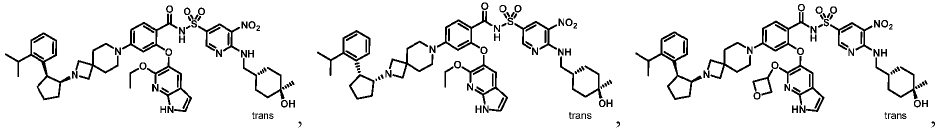 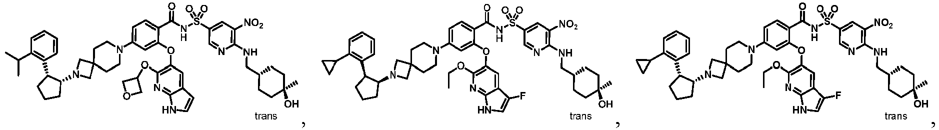 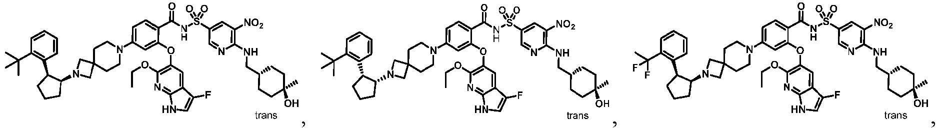 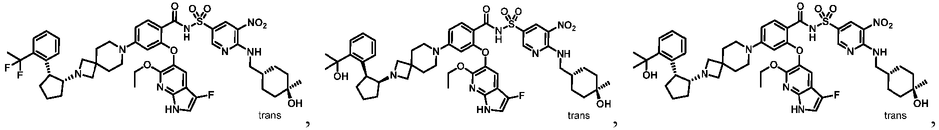 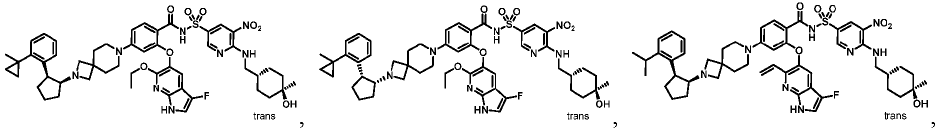 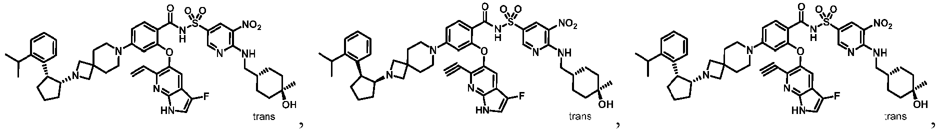 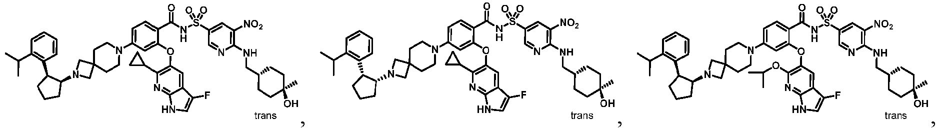 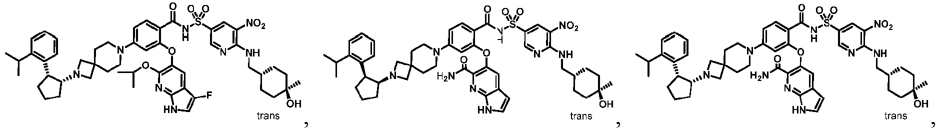 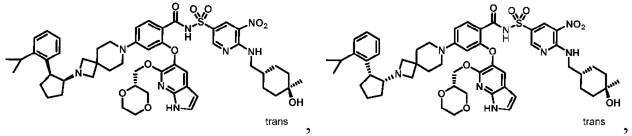 and pharmaceutically acceptable salts thereof.

14. A pharmaceutical composition, comprising the compound of any one of claims 1-13 or the pharmaceutically acceptable salt thereof and at least one pharmaceutically acceptable carrier.

15. The compound of any one of claims 1-13 or the pharmaceutically acceptable salt thereof or the pharmaceutical composition of claim 14, for use in treating a cell-proliferative disorder or autoimmune disease;
for example, wherein
(i) the cell-proliferative disorder is selected from breast cancer, ovarian cancer, bladder cancer, uterine cancer, prostate cancer, testicular cancer, lung cancer (for example, NSCLC, SCLC, squamous cell carcinoma or adenocarcinoma), esophageal cancer, head and neck cancer, colorectal cancer, kidney cancer (for example, RCC), liver cancer (for example, HCC), pancreatic cancer, stomach (i.e., gastric) cancer, thyroid cancer, chronic lymphocytic leukemia (CLL), lymphoblastic leukemia, follicular lymphoma, lymphoid malignancies of T-cell or B-cell origin, melanoma, myelogenous leukemia and myeloma;
and/or
(ii) the autoimmune disease is selected from allergy, Alzheimer's disease, acute disseminated encephalomyelitis, Addison's disease, ankylosing spondylitis, antiphospholipid antibody syndrome, asthma, atherosclerosis, autoimmune hemolytic anemia, autoimmune hemolytic and thrombocytopenic states, autoimmune hepatitis, autoimmune inner ear disease, bullous pemphigoid, coeliac disease, chagas disease, chronic obstructive pulmonary disease, chronic Idiopathic thrombocytopenic purpura (ITP), churg-strauss syndrome, Crohn's disease, dermatomyositis, diabetes mellitus type 1, endometriosis, Goodpasture's syndrome and associated glomerulonephritis and pulmonary hemorrhage, graves' disease, guillainbarre syndrome, hashimoto's disease, hidradenitis suppurativa, idiopathic thrombocytopenic purpura, interstitial cystitis, irritable bowel syndrome, lupus erythematosus, morphea, multiple sclerosis, myasthenia gravis, narcolepsy, neuromyotonia, Parkinson's disease, pemphigus vulgaris, pernicious anaemia, polymyositis, primary biliary cirrhosis, psoriasis, psoriatic arthritis, rheumatoid arthritis, schizophrenia, septic shock, scleroderma, Sjogren's disease, systemic lupus erythematosus and associated glomerulonephritis, temporal arteritis, tissue graft rejection and hyperacute rejection of transplanted organs, vasculitis (for example, ANCA-associated and other vasculitides), vitiligo, and wegener's granulomatosis.

## Patentansprüche

1. Verbindung der Formel (I): 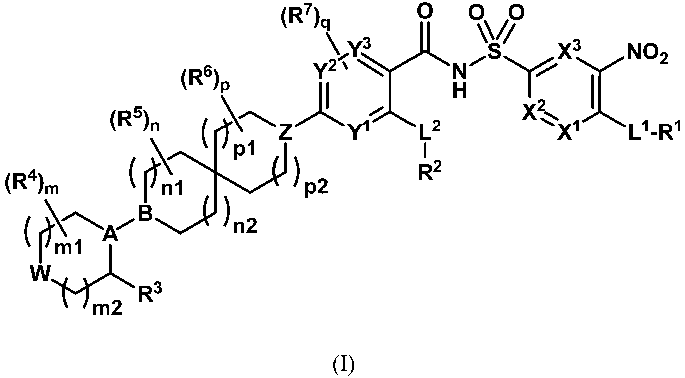 oder ein pharmazeutisch verträgliches Salz davon, worin:
X¹ ausgewählt ist aus N und CR⁸; X² ausgewählt ist aus N und CR⁹; X³ ausgewählt ist aus N und
CR¹⁰, wobei mindestens eines von X¹, X² und X³ N ist;
Y¹, Y² und Y³ CH sind;
A und B unabhängig ausgewählt sind aus N und CH;
W ausgewählt ist aus -CHR¹¹-, -NR¹²-, -O-, -S(O)ᵣ- und -S(O)(=NR¹²)-;
Z ausgewählt ist aus N und CH;
L¹ ausgewählt ist aus -CH₂-, -O-, -NH- und -S-;
L² ausgewählt ist aus einer Bindung und -O-;
R¹ ausgewählt ist aus C₁₋₁₀-Alkyl-, C₃₋₁₀-Cycloalkyl-, C₃₋₁₀-Cycloalkyl-C₁₋₄-alkyl-, Heterocyclyl-, Heterocyclyl-C₁₋₄-alkyl-, Aryl-, Aryl-C₁₋₄-alkyl-, Heteroaryl-, Heteroaryl-C₁₋₄-alkylgruppe, wobei die Alkyl-, Cycloalkyl-, Heterocyclyl-, Aryl- und Heteroarylgruppen jeweils unsubstituiert oder mit mindestens einem Substituenten, unabhängig ausgewählt aus R^{X}, substituiert sind;
R² ausgewählt ist aus 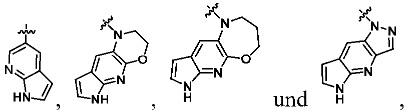 die unsubstituiert oder mit mindestens einem Substituenten, unabhängig ausgewählt aus R^{X}, substituiert sind;
R³ eine Phenylgruppe ist, wobei die Phenylgruppe unsubstituiert oder mit mindestens einem Substituenten, unabhängig ausgewählt aus R^{X}, substituiert ist;
jedes R⁴ unabhängig ausgewählt ist aus Wasserstoff-, Halogenatom, -OH, Methyl-, Ethyl-, Isopropyl- und Cyclopropylgruppe, wobei die Methyl-, Ethyl-, Isopropyl- und Cyclopropylgruppen jeweils unsubstituiert oder mit mindestens einem Substituenten, unabhängig ausgewählt aus R^{X}, substituiert sind; oder jegliche zwei von R⁴ zusammen mit den Atomen, an die sie gebunden sind, eine Cyclopropylgruppe bilden, wobei die Cyclopropylgruppe unsubstituiert oder mit 1, 2 oder 3 R^{X}-Gruppen substituiert ist;
oder "R⁴ und R¹¹" oder "R⁴ und R¹²" zusammen mit den Atomen, an die sie gebunden sind, eine C₃₋₈-Cycloalkylgruppe oder einen 4- bis 10-gliedrigen heterocyclischen Ring, enthaltend 1, 2 oder 3 Heteroatome, unabhängig ausgewählt aus Sauerstoff-, Schwefel-, Stickstoff- und Phosphoratom, und optional substituiert mit 1, 2 oder 3 R^{X}-Gruppen, bilden;
jedes Rund R⁶ unabhängig ausgewählt ist aus Wasserstoff-, Halogenatom, C₁₋₁₀-Alkyl-, C₃₋₁₀-Cycloalkylgruppe, CN, NO₂, -NH₂ und -OH, wobei die Alkyl- und Cycloalkylgruppen jeweils unsubstituiert oder mit mindestens einem Substituenten, unabhängig ausgewählt aus R^{X}, substituiert sind;
jedes R⁷ unabhängig ausgewählt ist aus Wasserstoff-, Halogenatom, C₁₋₁₀-Alkyl-, C₃₋₁₀-Cycloalkylgruppe, CN, NO₂, -NR^{A7}R^{B7}, -OR^{A7} und -C(O)R^{A7}, wobei die Alkyl- und Cycloalkylgruppen jeweils unsubstituiert oder mit mindestens einem Substituenten, unabhängig ausgewählt aus R^{X}, substituiert sind;
R⁸ ausgewählt ist aus Wasserstoff-, Halogenatom, C₁₋₁₀-Alkyl-, C₂₋₁₀-Alkenyl-, C₂₋₁₀-Alkinyl-, C₃₋₁₀-Cycloalkyl-, C₃₋₁₀-Cycloalkyl-C₁₋₄-alkyl-, Heterocyclyl-, Heterocyclyl-C₁₋₄-alkylgruppe, CN, NO₂, -NR^{A8}R^{B8}, -OR^{A8}, ^{_}C(O)R^{A8}, -C(O)OR^{A8}, -OC(O)R^{A8} und -S(O)ᵣR^{A8}, wobei die Alkyl-, Alkenyl-, Alkinyl-, Cycloalkyl- und Heterocyclylgruppen jeweils unsubstituiert oder mit mindestens einem Substituenten, unabhängig ausgewählt aus R^{X}, substituiert sind;
oder R¹ und R⁸ zusammen mit den Atomen, an die sie gebunden sind, eine C₅₋₁₀-Cycloalkylgruppe oder einen 5- bis 12-gliedrigen heterocyclischen Ring, enthaltend 1, 2 oder 3 Heteroatome, unabhängig ausgewählt aus Sauerstoff-, Schwefel-, Stickstoff- und Phosphoratom, und optional substituiert mit 1, 2 oder 3 R^{X}-Gruppen, bilden;
R⁹ ausgewählt ist aus Wasserstoff-, Halogenatom, CN, OH, Methyl-, Ethyl- und Trifluormethylgruppe;
R¹⁰ ausgewählt ist aus Wasserstoff-, Halogenatom, C₁₋₁₀-Alkylgruppe, CN, NO₂, -NH₂ und -OH, wobei die Alkylgruppe unsubstituiert oder mit mindestens einem Substituenten, unabhängig ausgewählt aus R^{X}, substituiert ist;
R¹¹ ausgewählt ist aus Wasserstoffatom, C₁₋₁₀-Alkyl- und C₃₋₁₀-Cycloalkylgruppe, wobei die Alkyl- und Cycloalkylgruppen jeweils unsubstituiert oder mit mindestens einem Substituenten, unabhängig ausgewählt aus R^{X}, substituiert sind;
R¹² ausgewählt ist aus Wasserstoffatom, C₁₋₁₀-Alkyl- und C₃₋₁₀-Cycloalkyl-, Heterocyclyl-, Heterocyclyl-C₁₋₄-alkyl-, Aryl-, Aryl-C₁₋₄-alkyl-, Heteroarylgruppe, -C(O)R^{A2}, -C(O)NR^{A12}R^{B12}, -C(O)OR^{A12}, -S(O)ᵣR^{A12} und -S(O)ᵣNR^{A12}R^{B12}, wobei die Alkyl-, Cycloalkyl-, Heterocyclyl-, Aryl- und Heteroarylgruppen jeweils unsubstituiert oder mit mindestens einem Substituenten, unabhängig ausgewählt aus R^{X}, substituiert sind;
jedes R^{A7}, R^{A8}, R^{A12}, R^{B7}, R^{B8} und R^{B12} unabhängig ausgewählt ist aus Wasserstoffatom, C₁₋₁₀-Alkyl-, C₂₋₁₀-Alkenyl-, C₂₋₁₀-Alkinyl-, C₃₋₁₀-Cycloalkyl-, C₃₋₁₀-Cycloalkyl-C₁₋₄-alkyl-, Heterocyclyl-, Heterocyclyl-C₁₋₄-alkyl-, Aryl-, Aryl-C₁₋₄-alkyl-, Heteroaryl- und Heteroaryl-C₁₋₄-alkylgruppe, wobei die Alkyl-, Alkenyl-, Alkinyl-, Cycloalkyl-, Heterocyclyl-, Aryl- und Heteroarylgruppen jeweils unsubstituiert oder mit mindestens einem Substituenten, unabhängig ausgewählt aus R^{X}, substituiert sind;
oder "R^{A7} und R^{B7}" oder "R^{A8} und R^{E8}" oder "R^{A12} und R^{B12}" zusammen mit dem/den Atom(en), an das/die sie gebunden sind, einen 4- bis 12-gliedrigen heterocyclischen Ring, enthaltend 0, 1 oder 2 zusätzliche Heteroatome, unabhängig ausgewählt aus Sauerstoff-, Schwefel-, Stickstoff- und Phosphoratom, und optional substituiert mit 1, 2 oder 3 R^{X}-Gruppen, bilden;
jedes R^{X} unabhängig ausgewählt ist aus Wasserstoffatom, C₁₋₁₀-Alkyl-, C₂₋₁₀-Alkenyl-, C₂₋₁₀-Alkinyl-, C₃₋₁₀-Cycloalkyl-, C₃₋₁₀-Cycloalkyl-C₁₋₄-alkyl-, Heterocyclyl-, Heterocyclyl-C₁₋₄-alkyl-, Aryl-, Aryl-C₁₋₄-alkyl-, Heteroaryl-, Heteroaryl-C₁₋₄-alkylgruppe, Halogenatom, CN, NO₂, -(CR^{c1}R^{d1})ₜNR^{a1}R^{b1}, -(CR^{c1}R^{d1})ₜOR^{b1}, -(CR^{c1}R^{d1})ₜC(O)R^{a1}, -(CR^{c1}R^{d1})ₜC(=NR^{e1})R^{a1}, -(CR^{c1}R^{d1})ₜC(=N-OR^{b1})R^{a1}, -(CR^{c1}R^{d1})ₜC(O)OR^{b1}, -(CR^{c1}R^{d1})ₜOC(O)R^{b1}, -(CR^{c1}R^{d1})ₜC(O)NR^{a1}R^{b1}, -(CR^{c1}R^{d1})ₜNR^{a1}C(O)R^{b1}, -(CR^{c1}R^{d1})ₜC(=NR^{e1})NR^{a1}R^{b1}, -(CR^{c1}R^{d1})ₜNR^{a1}C(=NR^{e1})R^{b1}, -(CR^{c1}R^{d1})ₜOC(O)NR^{a1}R^{b1}, -(CR^{c1}R^{d1})ₜNR^{a1}C(O)OR^{b1}, -(CR^{c1}R^{d1})ₜNR^{a1}C(O)NR^{a1}R^{b1}, -(CR^{c1}R^{d1})ₜNR^{a1}C(S)NR^{a1}R^{b1}, -(CR^{c1}R^{d1})ₜNR^{a1}C(=NR^{e1})NR^{a1}R^{b1}, -(CR^{c1}R^{d1})ₜS(O)ᵣR^{b1}, -(CR^{c1}R^{d1})ₜS(O)(=NR^{e1})R^{b1}, -(CR^{c1}R^{d1})ₜN=S(O)R^{a1}R^{b1}, -(CR^{c1}R^{d1})ₜS(O)₂OR^{b1}, -(CR^{c1}R^{d1})ₜOS(O)₂R^{b1}, -(CR^{c1}R^{d1})ₜNR^{a1}S(O)ᵣR^{b1}, -(CR^{c1}R^{d1})ₜNR^{a1}S(O)(=NR^{e1})R^{b1}, -(CR^{c1}R^{d1})ₜS(O)ᵣNR^{a1}R^{b1}, -(CR^{c1}R^{d1})ₜS(O)(=NR^{e1})NR^{a1}R^{b1}, -(CR^{c1}R^{d1})ₜNR^{a1}S(O)₂NR^{a1}R^{b1}, -(CR^{c1}R^{d1})ₜNR^{a1}S(O)(=NR^{e1})NR^{a1}R^{b1}, -(CR^{c1}R ^{d1})ₜP(O)R^{a1}R^{b1} und -(CR^{c1}R^{d1})ₜP(O)(OR^{a1})(OR^{b1}), wobei die Alkyl-, Alkenyl-, Alkinyl-, Cycloalkyl-, Heterocyclyl-, Aryl- und Heteroarylgrupen jeweils unsubstituiert oder mit mindestens einem Substituenten, unabhängig ausgewählt aus R^{Y}, substituiert sind;
jedes R^{a1} und jedes R^{b1} unabhängig ausgewählt ist aus Wasserstoffatom, C₁₋₁₀-Alkyl-, C₂₋₁₀-Alkenyl-, C₂₋₁₀-Alkinyl-, C₃₋₁₀-Cycloalkyl-, C₃₋₁₀-Cycloalkyl-C₁₋₄-alkyl-, Heterocyclyl-, Heterocyclyl-C₁₋₄-alkyl-, Aryl-, Aryl-C₁₋₄-alkyl-, Heteroaryl- und Heteroaryl-C₁₋₄-alkylgruppe, wobei die Alkyl-, Alkenyl-, Alkinyl-, Cycloalkyl-, Heterocyclyl-, Aryl- und Heteroarylgruppen jeweils unsubstituiert oder mit mindestens einem Substituenten, unabhängig ausgewählt aus R^{Y}, substituiert sind;
oder R^{a1} und R^{b1} zusammen mit dem/den Atom(en), an das/die sie gebunden sind, einen 4- bis 12-gliedrigen heterocyclischen Ring, enthaltend 0, 1 oder 2 zusätzliche Heteroatome, unabhängig ausgewählt aus Sauerstoff-, Schwefel-, Stickstoff- und Phosphoratom, und optional substituiert mit 1, 2 oder 3 R^{Y}-Gruppen, bilden;
jedes R^{c1} und jedes R^{d1} unabhängig ausgewählt ist aus Wasserstoff-, Halogenatom, C₁₋₁₀-Alkyl-, C₂₋₁₀-Alkenyl-, C₂₋₁₀-Alkinyl-, C₃₋₁₀-Cycloalkyl-, C₃₋₁₀-Cycloalkyl-C₁₋₄-alkyl-, Heterocyclyl-, Heterocyclyl-C₁₋₄-alkyl-, Aryl-, Aryl-C₁₋₄-alkyl-, Heteroaryl- und Heteroaryl-C₁₋₄-alkylgruppe, wobei die Alkyl-, Alkenyl-, Alkinyl-, Cycloalkyl-, Heterocyclyl-, Aryl- und Heteroarylgruppen jeweils unsubstituiert oder mit mindestens einem Substituenten, unabhängig ausgewählt aus R^{Y}, substituiert sind;
jedes R^{e1} unabhängig ausgewählt ist aus Wasserstoffatom, C₁₋₁₀-Alkyl-, C₃₋₁₀-Cycloalkyl-, C₃₋₁₀-Cycloalkyl-C₁₋₄-alkylgruppe, CN, NO₂, -OR^{a2}, -SR^{a2}, -S(O)ᵣR^{a2}, -C(O)R^{a2}, -C(O)OR^{a2}, -S(O)ᵣNR^{a2}R^{b2} und -C(O)NR^{a2}R^{b2};
jedes R^{Y} unabhängig ausgewählt ist aus C₁₋₁₀-Alkyl-, C₂₋₁₀-Alkenyl-, C₂₋₁₀-Alkinyl-, C₃₋₁₀-Cycloalkyl-, C₃₋₁₀-Cycloalkyl-C₁₋₄-alkyl-, Heterocyclyl-, Heterocyclyl-C₁₋₄-alkyl-, Aryl-, Aryl-C₁₋₄-alkyl-, Heteroaryl-, Heteroaryl-C₁₋₄-alkylgruppe, Halogenatom, CN, NO₂, -(CR^{c2}R^{d2})ₜNR^{a2}R^{b2}, -(CR^{c2}R^{d2})ₜOR^{b2}, -(CR^{c2}R^{d2})ₜC(O)R^{a2}, -(CR^{c2}R^{d2})ₜC(=NR^{e2})R^{a2}, -(CR^{c2}R^{d2})ₜC(=N-OR^{b2})R^{a2}, -(CR^{c2}R^{d2})ₜC(O)OR^{b2}, -(CR^{c2}R^{d2})ₜOC(O)R^{b2}, -(CR^{c2}R^{d2})ₜC(O)NR^{a2}R^{b2}, -(CR^{c2}R^{d2})ₜNR^{a2}C(O)R^{b2}, -(CR^{c2}R^{d2})ₜC(=NR^{c2})NR^{a2}R^{b2}, -(CR^{c2}R^{d2})ₜNR^{a2}C(=NR^{e2})R^{b2}, -(CR^{c2}R^{d2})ₜOC(O)NR^{a2}R^{b2}, -(CR^{c2}R^{d2})ₜNR^{a2}C(O)OR^{b2}, -(CR^{c2}R^{d2})ₜNR^{a2}C(O)NR^{a2}R^{b2}, ^{_}(CR^{c2}R^{d2})ₜNR^{a2}C(S)NR^{a2}R^{b2}, -(CR^{c2}R^{d2})ₜNR^{a2}C(=NR^{e2})NR^{a2}R^{b2}, -(CR^{c2}R^{d2})ₜS(O)ᵣR^{b2}, -(CR^{c2}R^{d2})ₜS(O)(=NR^{e2})R^{b2}, -(CR^{c2}R^{d2})ₜN=S(O)R^{a2}R^{b2}, -(CR^{c2}R^{d2})ₜS(O)₂OR^{b2}, -(CR^{c2}R^{d2})ₜOS(O)₂R^{b2}, -(CR^{c2}R^{d2})ₜNR^{a2}S(O)ᵣR^{b2}, -(CR^{c2}R^{d2})ₜNR^{a2}S(O)(=NR^{e2})R^{b2}, -(CR^{c2}R^{d2})ₜS(O)ᵣNR^{a2}R^{b2}, -(CR^{c2}R^{d2})ₜS(O)(=NR^{e2})NR^{a2}R^{b2}, -(CR^{c2}R^{d2})ₜNR^{a2}S(O)₂NR^{a2}R^{b2}, -(CR^{c2}R^{d2})ₜNR^{a2}S(O)(=NR^{e2})NR^{a2}R^{b2}, -(CR^{c2}R^{d2})ₜP(O)R^{a2}R^{b2} und -(CR^{c2}R^{d2})ₜP(O)(OR^{a2})(OR^{b2}), wobei die Alkyl-, Alkenyl-, Alkinyl-, Cycloalkyl-, Heterocyclyl-, Aryl- und Heteroarylgruppen jeweils unsubstituiert oder mit mindestens einem Substituenten, unabhängig ausgewählt aus OH, CN, Aminogruppe, Halogenatom, C₁₋₁₀-Alkyl-, C₂₋₁₀-Alkenyl-, C₂₋₁₀-Alkinyl-, C₃₋₁₀-Cycloalkyl-, C₁₋₁₀-Alkoxy-, C₃₋₁₀-Cycloalkoxy-, C₁₋₁₀-Alkylthio-, C₃₋₁₀-Cycloalkylthio-, C₁₋₁₀-Alkylamino-, C₃₋₁₀-Cycloalkylamino- und Di(C₁₋₁₀-alkyl)aminogruppe, substituiert sind;
jedes R^{a2} und jedes R^{b2} unabhängig ausgewählt ist aus Wasserstoffatom, C₁₋₁₀-Alkyl-, C₂₋₁₀-Alkenyl-, C₂₋₁₀-Alkinyl-, C₃₋₁₀-Cycloalkyl-, C₃₋₁₀-Cycloalkyl-C₁₋₄-alkyl-, C₁₋₁₀-Alkoxy-, C₃₋₁₀-Cycloalkoxy-, C₁₋₁₀-Alkylthio-, C₃₋₁₀-Cycloalkylthio-, C₁₋₁₀-Alkylamino-, C₃₋₁₀-Cycloalkylamino-, Di(C₁₋₁₀-alkyl)-amino-, Heterocyclyl-, Heterocyclyl-C₁₋₄-alkyl-, Aryl-, Aryl-C₁₋₄-alkyl-, Heteroaryl- und Heteroaryl-C₁₋₄-alkylgruppe, wobei die Alkyl-, Alkenyl-, Alkinyl-, Cycloalkyl-, Alkoxy-, Cycloalkoxy-, Alkylthio-, Cycloalkylthio-, Alkylamino-, Cycloalkylamino-, Heterocyclyl-, Aryl- und Heteroarylgruppen jeweils unsubstituiert oder mit mindestens einem Substituenten, unabhängig ausgewählt aus Halogenatom, CN, C₁₋₁₀-Alkyl-, C₂₋₁₀-Alkenyl-, C₂₋₁₀-Alkinyl-, C₃₋₁₀-Cycloalkylgruppe, OH, C₁₋₁₀-Alkoxy-, C₃₋₁₀-Cycloalkoxy-, C₁₋₁₀-Alkylthio-, C₃₋₁₀-Cycloalkylthio-, Amino-, C₁₋₁₀-Alkylamino-, C₃₋₁₀-Cycloalkylamino- und Di(C₁₋₁₀-alkyl)aminogruppe, substituiert sind;
jedes R^{c2} und jedes R^{d2} unabhängig ausgewählt ist aus Wasserstoff-, Halogenatom, C₁₋₁₀-Alkyl-, C₂₋₁₀-Alkenyl-, C₂₋₁₀-Alkinyl-, C₃₋₁₀-Cycloalkyl-, C₃₋₁₀-Cycloalkyl-C₁₋₄-alkyl-, C₁₋₁₀-Alkoxy-, C₃₋₁₀-Cycloalkoxy-, C₁₋₁₀-Alkylthio-, C₃₋₁₀-Cycloalkylthio-, C₁₋₁₀-Alkylamino-, C₃₋₁₀-Cycloalkylamino-, Di(C₁₋₁₀-alkyl)amino-, Heterocyclyl-, Heterocyclyl-C₁₋₄-alkyl-, Aryl-, Aryl-C₁₋₄-alkyl-, Heteroaryl- und Heteroaryl-C₁₋₄-alkylgruppe, wobei die Alkyl-, Alkenyl-, Alkinyl-, Cycloalkyl-, Alkoxy-, Cycloalkoxy-, Alkylthio-, Cycloalkylthio-, Alkylamino-, Cycloalkylamino-, Heterocyclyl-, Aryl- und Heteroarylgruppen jeweils unsubstituiert oder mit mindestens einem Substituenten, unabhängig ausgewählt aus Halogenatom, CN, C₁₋₁₀-Alkyl-, C₂₋₁₀-Alkenyl-, C₂₋₁₀-Alkinyl-, C₃₋₁₀-Cycloalkylgruppe, OH, C₁₋₁₀-Alkoxy-, C₃₋₁₀-Cycloalkoxy-, C₁₋₁₀-Alkylthio-, C₃₋₁₀-Cycloalkylthio-, Amino-, C₁₋₁₀-Alkylamino-, C₃₋₁₀-Cycloalkylamino- und Di(C₁₋₁₀-alkyl)aminogruppe, substituiert sind;
jedes R^{e2} unabhängig ausgewählt ist aus Wasserstoffatom, CN, NO₂, C₁₋₁₀-Alkyl-, C₃₋₁₀-Cycloalkyl-, C₃₋₁₀-Cycloalkyl-C₁₋₄-alkyl-, C₁₋₁₀-Alkoxy-, C₃₋₁₀-Cycloalkoxy-, -C(O)C₁₋₄-Alkyl-, -C(O)C₃₋₁₀-Cycloalkyl-, -C(O)OC₁₋₄-Alkyl-, -C(O)OC₃₋₁₀-Cycloalkyl-, -C(O)N(C₁₋₄-Alkyl)₂-, -C(O)N(C₃₋₁₀-Cycloalkyl)₂-, -S(O)₂C₁₋₄-Alkyl-, -S(O)₂C₃₋₁₀-Cycloalkyl-, -S(O)₂N(C₁₋₄-Alkyl)₂- und -S(O)₂N(C₃₋₁₀-Cycloalkyl)₂-Gruppe;
m, n, p und q unabhängig ausgewählt sind aus 0, 1, 2 und 3;
m1, m2, n1, n2, p1 und p2 unabhängig ausgewählt sind aus 0 und 1;
jedes r unabhängig ausgewählt ist aus 0, 1 und 2;
jedes t unabhängig ausgewählt ist aus 0, 1, 2, 3 und 4;
jedes u unabhängig ausgewählt ist aus 0, 1, 2, 3 und 4.

2. Verbindung nach Anspruch 1 oder ein pharmazeutisch verträgliches Salz davon, wobei
X³ CH ist, AN ist, B CH ist, Z N ist, Y¹, Y² und Y³ CH sind, gezeigt als Formel (II) 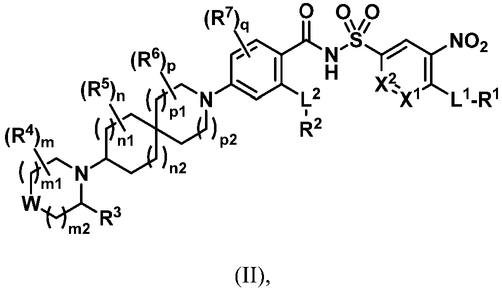
worin
X¹ ausgewählt ist aus N und CR⁸, X² ausgewählt ist aus N und CR⁹, wobei mindestens eines von X¹ und X² N ist; W ausgewählt ist aus -CHR¹¹-, -NR¹²-, -O- und -S(O)ᵣ-; L¹, L², R¹, R², R³, R⁴, R⁵, R⁶, R⁷, R⁸, R⁹, R¹¹, R¹², m, m1, m2, n, n1, n2, p, p1, p2 und q wie in Formel (I) definiert sind.

3. Verbindung nach einem jeglichen der Ansprüche 1-2 oder ein pharmazeutisch verträgliches Salz davon, wobei
(i) R¹ ausgewählt ist aus 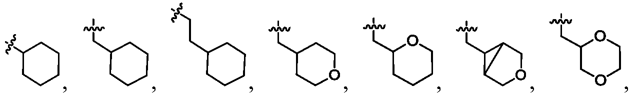 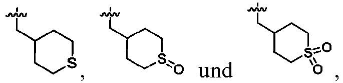 die unsubstituiert oder mit mindestens einem Substituenten, unabhängig ausgewählt aus R^{X}, substituiert sind;
und/oder
(ii) jeder Substituent R^{X} von R¹ unabhängig ausgewählt ist aus C₁₋₁₀-Alkyl-, C₃₋₁₀-Cycloalkylgruppe, Halogenatom, CN, NO₂, -(CR^{c1}R^{d1})ₜNR^{a1}R^{b1}, -(CR^{c2}R^{d1})ₜOR^{b1}, -(CR^{c1}R^{d1})ₜC(O)R^{a1} und -(CR^{c1}R^{d1})ₜN=S(O)R^{a1}R^{b1}, wobei die Alkyl-, Cycloalkylgruppen jeweils unsubstituiert oder mit mindestens einem Substituenten, unabhängig ausgewählt aus R^{Y}, substituiert sind;
zum Beispiel
wobei jedes R^{a1} und jedes R^{b1} unabhängig ausgewählt ist aus Wasserstoffatom, C₁₋₁₀-Alkyl-, C₂₋₁₀-Alkenyl- und C₂₋₁₀-Alkinylgruppe, wobei die Alkyl-, Alkenyl- und Alkinylgruppen jeweils unsubstituiert oder mit mindestens einem Substituenten, unabhängig ausgewählt aus R^{Y}, substituiert sind; oder
wobei R^{a1} und R^{b1} zusammen mit dem/den Atom(en), an das/die sie gebunden sind, einen 4- bis 8-gliedrigen heterocyclischen Ring, enthaltend 0, 1 oder 2 zusätzliche Heteroatome, unabhängig ausgewählt aus Sauerstoff-, Schwefel-, Stickstoff- und Phosphoratom, und optional substituiert mit 1, 2 oder 3 R^{Y}-Gruppen, bilden;
oder
jeder Substituent R^{X} von R¹ unabhängig ausgewählt ist aus OH, CN, Halogenatom, C₁₋₁₀-Alkylgruppe,

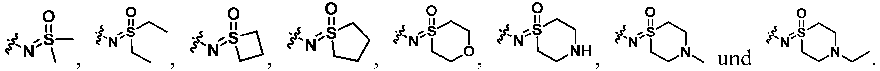

4. Verbindung nach einem jeglichen der Ansprüche 1-3 oder ein pharmazeutisch verträgliches Salz davon, wobei
(i) R¹ ausgewählt ist aus 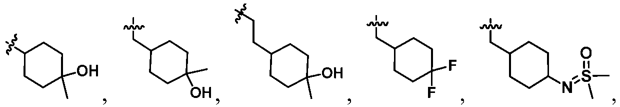 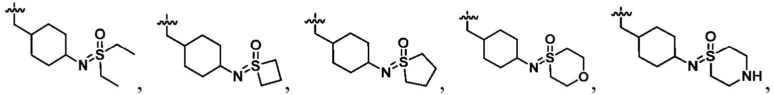 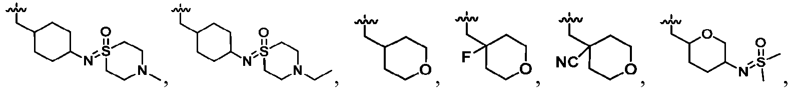 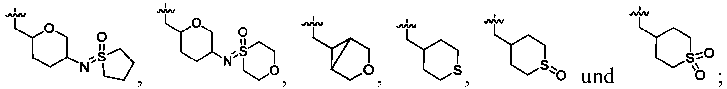 und/oder
(ii) R⁸ ein Wasserstoffatom ist.

5. Verbindung nach einem jeglichen der Ansprüche 1-4 oder ein pharmazeutisch verträgliches Salz davon, wobei
R¹ und R⁸ zusammen mit den Atomen, an die sie gebunden sind, einen 5- bis 12-gliedrigen heterocyclischen Ring, enthaltend 1, 2 oder 3 Heteroatome, unabhängig ausgewählt aus Sauerstoff-, Schwefel- und Stickstoffatom, und optional substituiert mit 1, 2 oder 3 R^{X}-Gruppen, bilden;
zum Beispiel
wobei der Rest 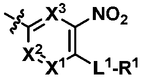 in Formel (I) und der Rest 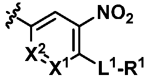 in Formel (II) ausgewählt sind aus 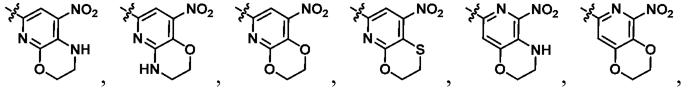 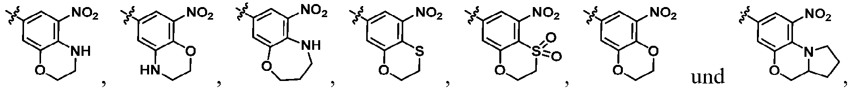 wobei der heterocyclische Ring, der durch R¹ und R⁸ gebildet wird, optional mit 1, 2 oder 3 R^{X}-Gruppen substituiert ist, wobei das Symbol 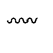 den Anbindungspunkt an den Rest des Moleküls anzeigt.

6. Verbindung nach Anspruch 5 oder ein pharmazeutisch verträgliches Salz davon, wobei
jeder Substituent R^{X} des 5- bis 12-gliedrigen heterocyclischen Rings unabhängig ausgewählt ist aus C₁₋₁₀-Alkyl-, C₃₋₁₀-Cycloalkyl-, C₃₋₁₀-Cycloalkyl-C₁₋₄-alkyl-, Heterocyclyl-, Heterocyclyl-C₁₋₄-alkyl-, Aryl-, Aryl-C₁₋₄-alkyl-, Heteroaryl-, Heteroaryl-C₁₋₄-alkylgruppe, Halogenatom, CN, NO₂, -(CR^{c1}R^{d1})ₜNR^{a1}R^{b1} und -(CR^{c1}R^{d1})ₜOR^{b1}, wobei die Alkyl-, Cycloalkyl-, Heterocyclyl-, Aryl- und Heteroarylgruppen jeweils unsubstituiert oder mit mindestens einem Substituenten, unabhängig ausgewählt aus R^{Y}, substituiert sind;
zum Beispiel
jeder Substituent R^{X} des 5- bis 12-gliedrigen heterocyclischen Rings ausgewählt ist aus Halogenatom, CN, NO₂, OH, C₁₋₈-Alkylgruppe,

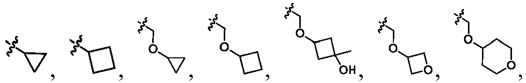

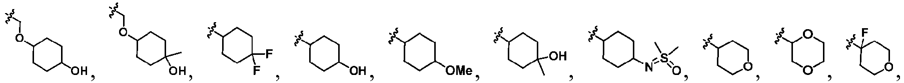

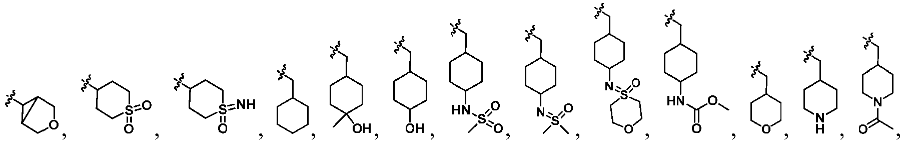

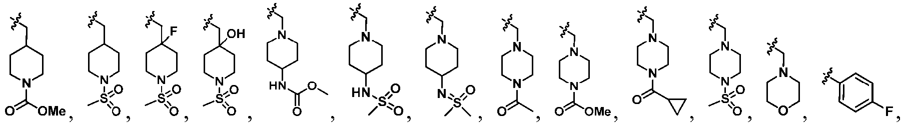

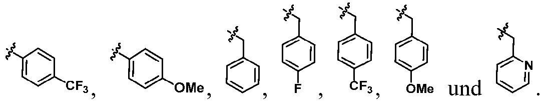

7. Verbindung nach einem jeglichen der Ansprüche 1-6 oder ein pharmazeutisch verträgliches Salz davon, wobei
(i) jeder Substituent R^{X} von R² unabhängig ausgewählt ist aus C₁₋₁₀-Alkyl-, C₂₋₁₀-Alkenyl-, C₂₋₁₀-Alkinyl-, C₃₋₁₀-Cycloalkyl-, Heterocyclyl-, Heterocyclyl-C₁₋₄-alkylgruppe, Halogenatom, CN, NO₂, -(CR^{c1}R^{d1})ₜNR^{a1}R^{b1} , -(CR^{c1}R^{d1})ₜOR^{b1}, -(CR^{c1}R^{d1})ₜC(O)OR^{b1}, -(CR^{c1}R^{d1})ₜC(O)NR^{a1}R^{b1}, -(CR^{c1}R^{d1})ₜNR^{a1}C(O)R^{b1}, -(CR^{c1}R^{d1})ₜNR^{a1}C(O)OR^{b1}, -(CR^{c1}R^{d1})ₜNR^{a1}C(O)NR^{a1}R^{b1}, -(CR^{c1}R^{d1})ₜS(O)₂OR^{b1}, -(CR^{c2}R^{d1})ₜS(O)ᵣR^{b1}, -(CR^{c1}R^{d1})ₜNR^{a1}S(O)ᵣR^{b1}, -(CR^{c1}R^{d1})ₜS(O)ᵣNR^{a1}R^{b1} und -(CR^{c1}R^{d1})ₜNR^{a1}S(O)₂NR^{a1}R^{b1}, wobei die Alkyl-, Alkenyl-, Alkinyl-, Cycloalkyl- und Heterocyclylgruppen jeweils unsubstituiert oder mit mindestens einem Substituenten, unabhängig ausgewählt aus R^{Y}, substituiert sind;
zum Beispiel
jeder Substituent R^{X} von R² ausgewählt ist aus Halogenatom, CN, NH₂, Methyl-, Trifluormethyl-, Cyclopropyl-, Methoxy-, Methoxyethoxy- und Difluormethoxygruppe;
zum Beispiel
wobei R^{a1} und R^{b1} unabhängig ausgewählt sind aus Wasserstoffatom, C₁₋₁₀-Alkyl-, C₃₋₁₀-Cycloalkyl-, C₃₋₁₀-Cycloalkyl-C₁₋₄-alkyl-, Heterocyclyl- und Heterocyclyl-C₁₋₄-alkylgruppe, wobei die Alkyl-, Cycloalkyl- und Heterocyclylgruppen jeweils unsubstituiert oder mit mindestens einem Substituenten, unabhängig ausgewählt aus R^{Y}, substituiert sind;
oder
(ii) R² ausgewählt ist aus

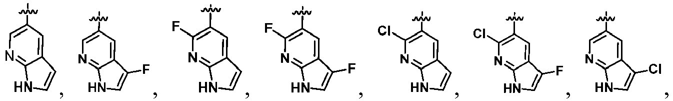

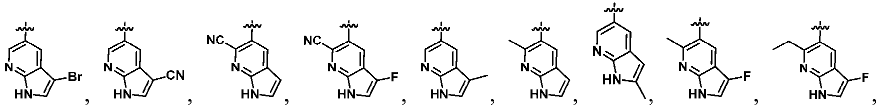

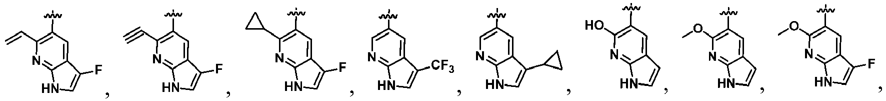

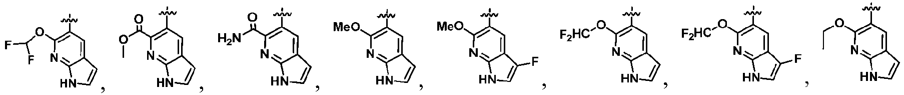

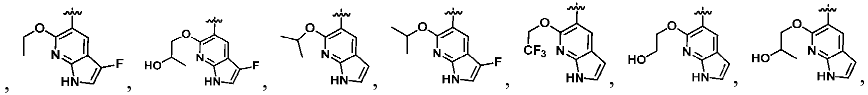

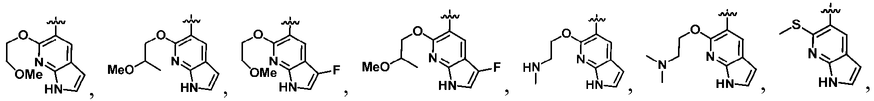

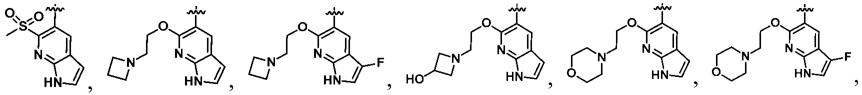

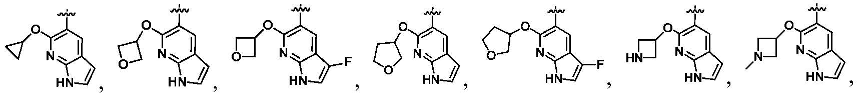

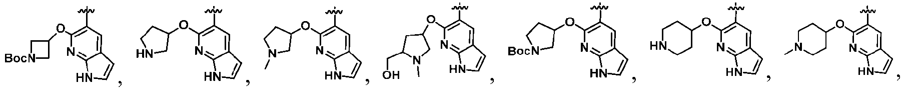

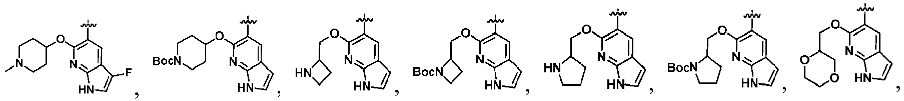

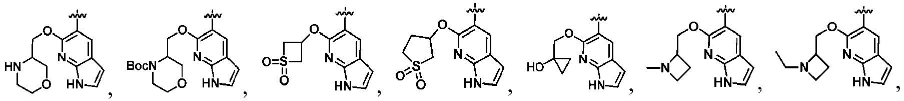

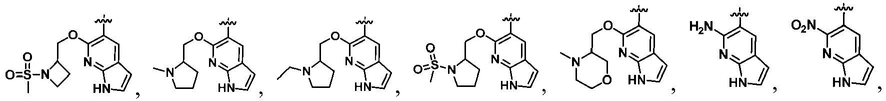

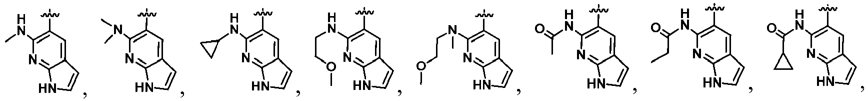

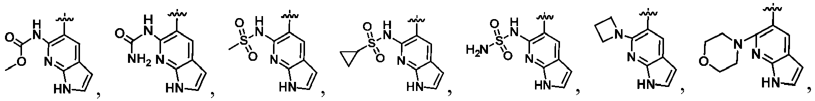

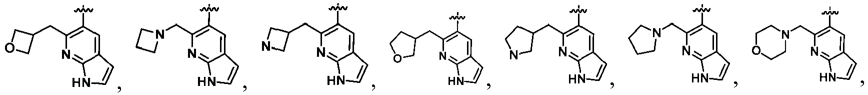

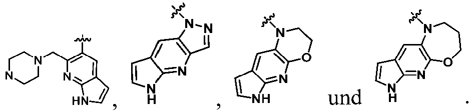

8. Verbindung nach einem jeglichen der Ansprüche 1-7 oder ein pharmazeutisch verträgliches Salz davon, wobei
W ausgewählt ist aus -CH₂-, -NR¹²-, -O-, -S(O)ᵣ- und -S(O)(=NR¹²)-.

9. Verbindung nach einem jeglichen der Ansprüche 1-8 oder ein pharmazeutisch verträgliches Salz davon, wobei
(i) R¹¹ ein Wasserstoffatom ist;
und/oder
(ii) R¹² ausgewählt ist aus Wasserstoffatom, Methyl-, Ethyl-, Cyclopropylgruppe, -C(O)CH₃, -C(O)OCH₃, -S(O)₂CH₃,

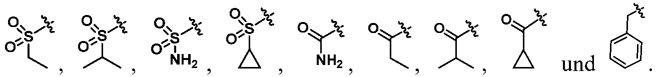

10. Verbindung nach einem jeglichen der Ansprüche 1-9 oder ein pharmazeutisch verträgliches Salz davon, wobei
(i) jeder Substituent R^{X} von R³ ausgewählt ist aus Halogenatom, C₁₋₁₀-Alkyl-, C₂₋₁₀-Alkenyl-, C₃₋₁₀-Cycloalkylgruppe, wobei die Alkyl-, Alkenyl- und Cycloalkylgruppen jeweils unsubstituiert oder mit mindestens einem Substituenten, unabhängig ausgewählt aus R^{Y}, substituiert sind;
zum Beispiel
jeder Substituent R^{X} von R³ ausgewählt ist aus Halogenatom, Methyl-, Ethyl-, Isopropyl-, tert-Butyl-, Propenyl- und Cyclopropylgruppe, wobei die Methyl-, Ethyl-, Isopropyl-, Propenyl- und Cyclopropylgruppen jeweils unsubstituiert oder mit mindestens einem Substituenten, unabhängig ausgewählt aus Halogenatom, C₁₋₁₀-Alkylgruppe, CN, NO₂, -NH₂ und -OH, substituiert sind;
oder
(ii) R³ ausgewählt ist aus 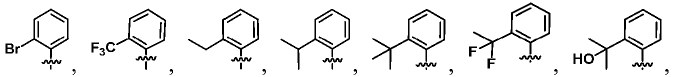 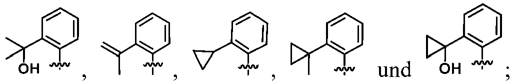 und/oder
(iii) m ausgewählt ist aus 0, 1 und 2.

11. Verbindung nach einem jeglichen der Ansprüche 1-8 oder ein pharmazeutisch verträgliches Salz davon, wobei der Rest 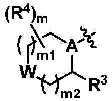 in Formel (I) und Formel (II) ausgewählt ist aus 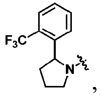 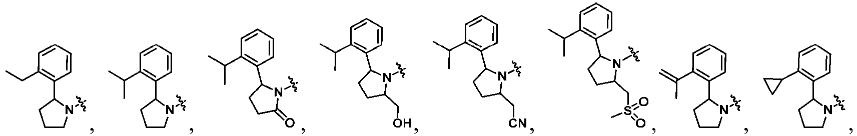 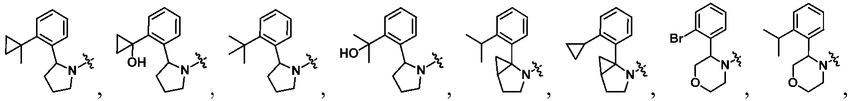 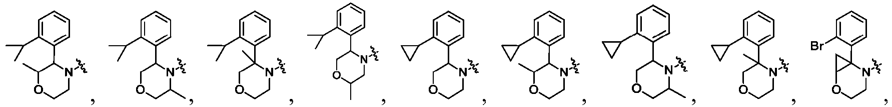 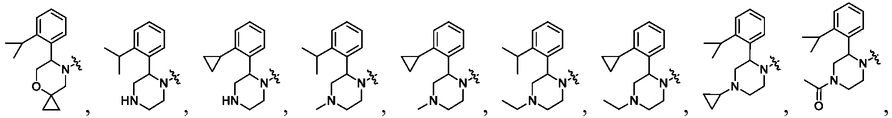 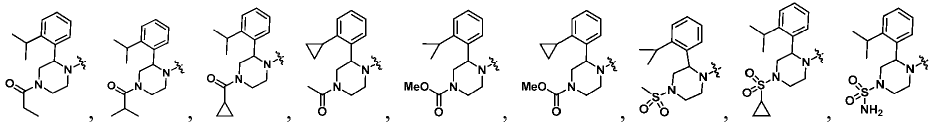 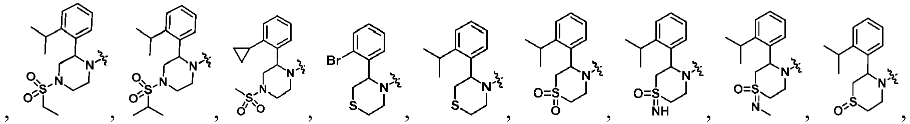 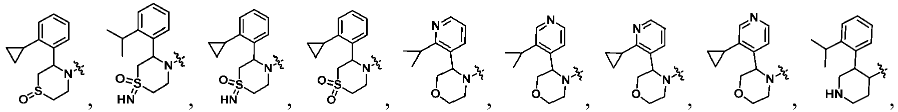 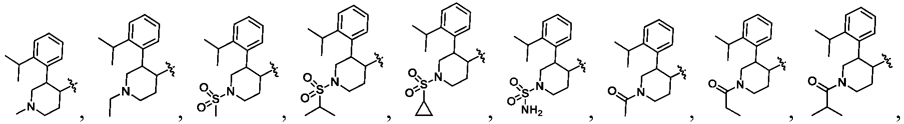 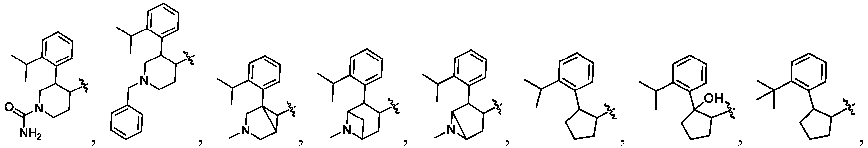 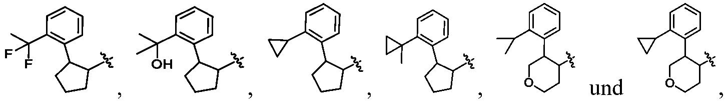 wobei das Symbol 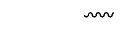 den Anbindungspunkt an den Rest des Moleküls anzeigt.

12. Verbindung nach einem jeglichen der Ansprüche 1-9 oder ein pharmazeutisch verträgliches Salz davon, wobei
(i) R⁵ und R⁶ H sind;
und/oder
(ii) der Rest 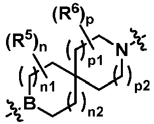 in Formel (I) und Formel (II) ausgewählt ist aus 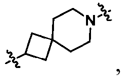 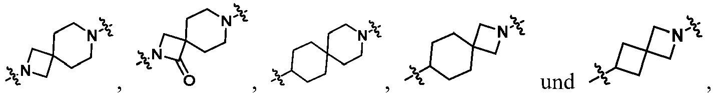 wobei das Symbol 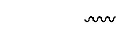 den Anbindungspunkt an den Rest des Moleküls anzeigt;
und/oder
(iii) jedes R⁷ ein Wasserstoffatom ist.

13. Verbindung, wobei die Verbindung ausgewählt ist aus 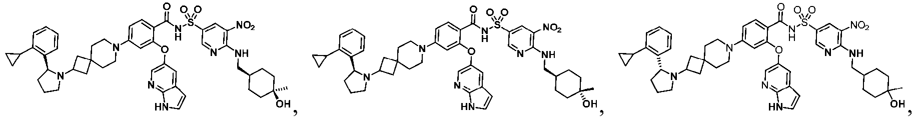 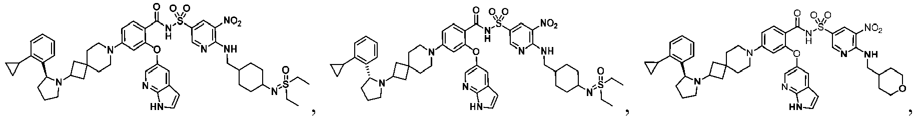 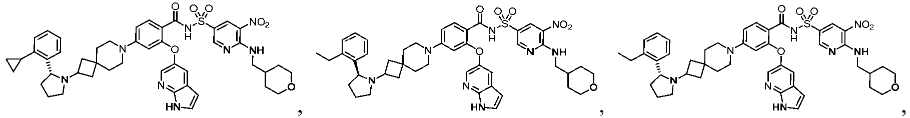 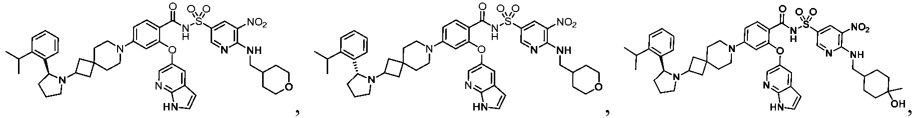 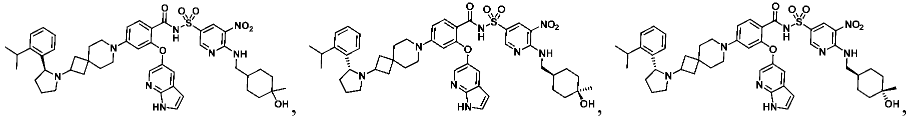 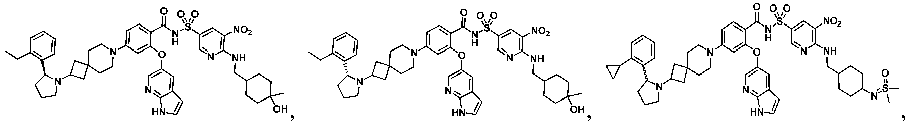 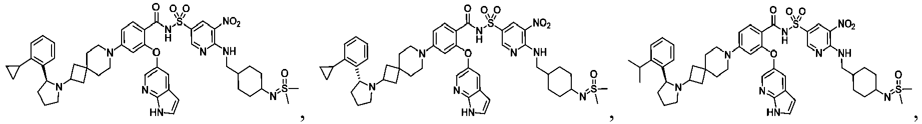 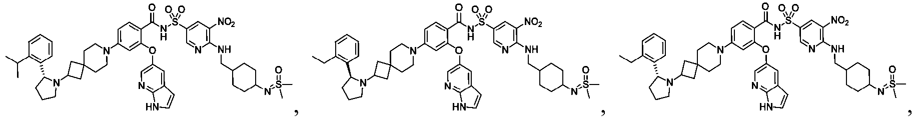 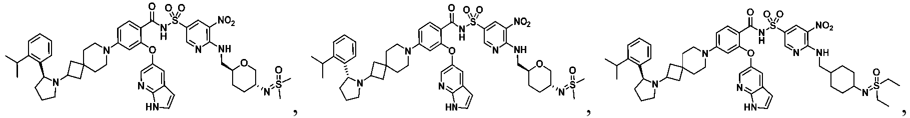 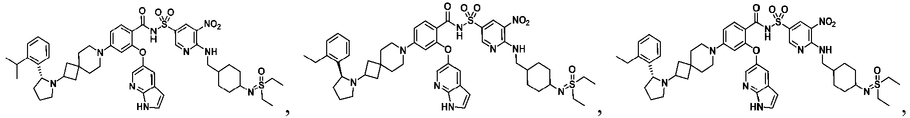 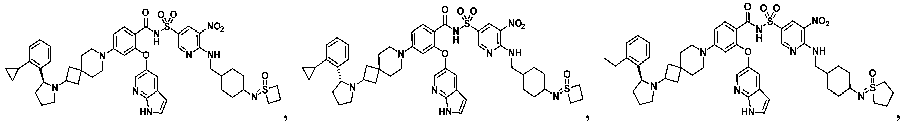 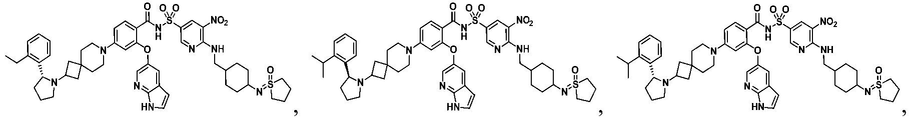 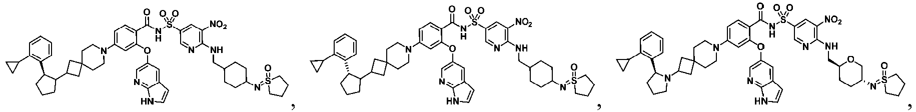 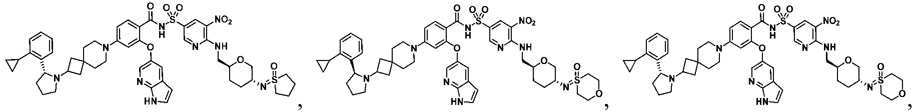 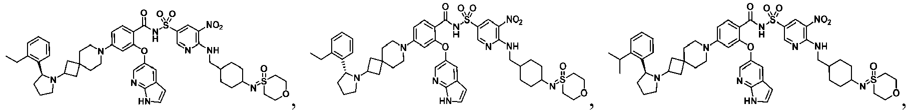 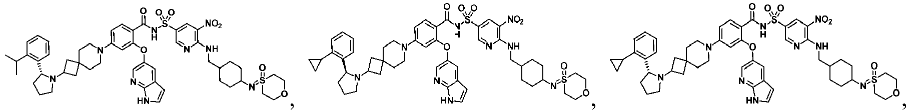 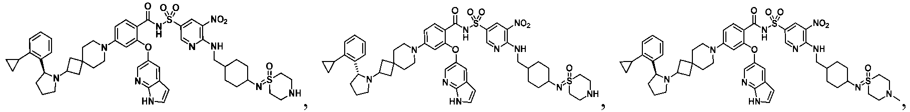 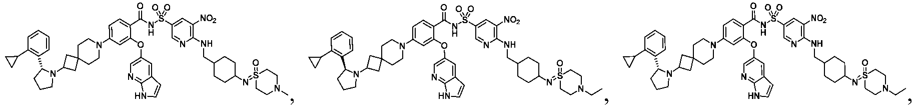 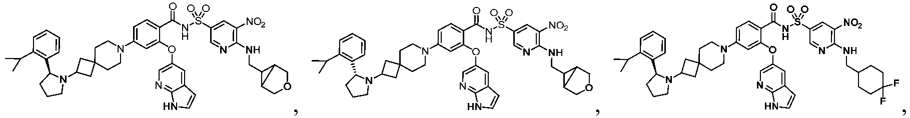 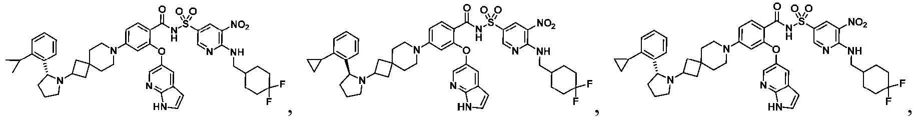 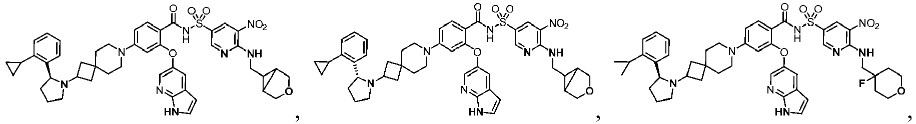 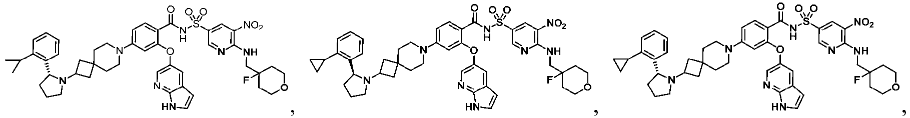 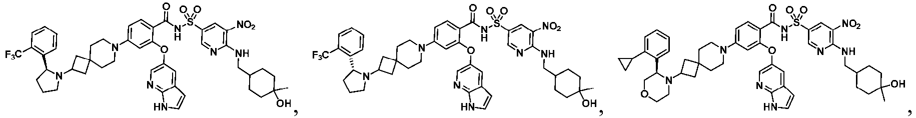 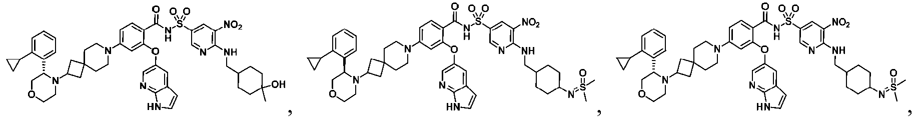 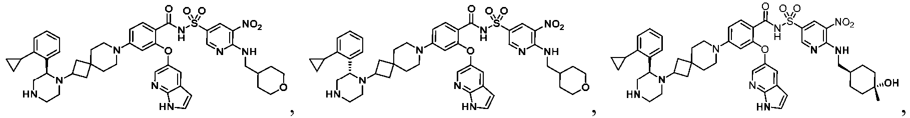 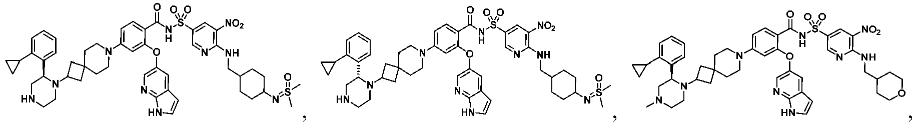 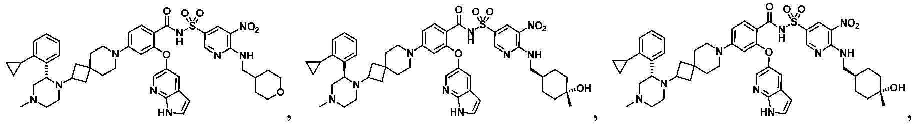 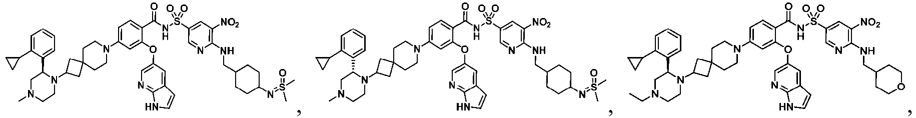 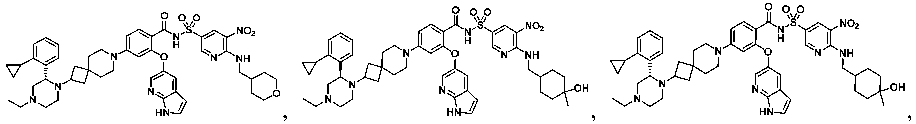 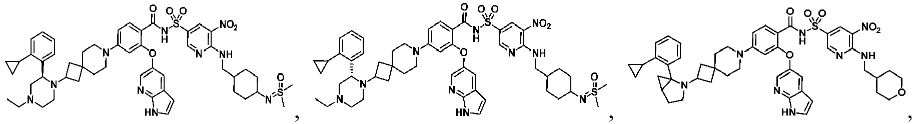 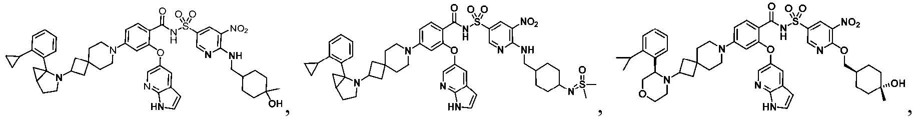 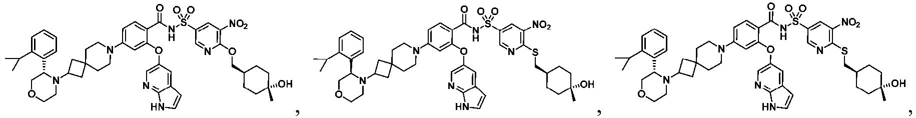 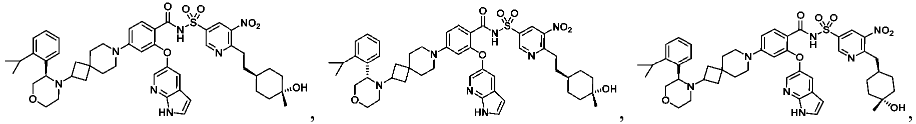 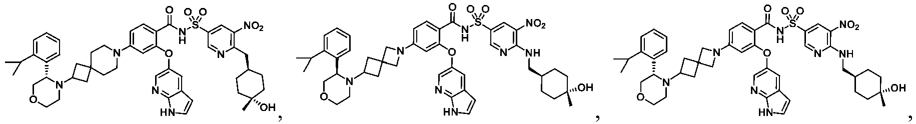 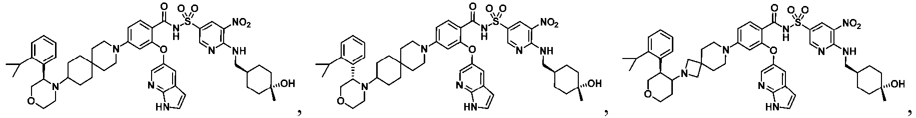 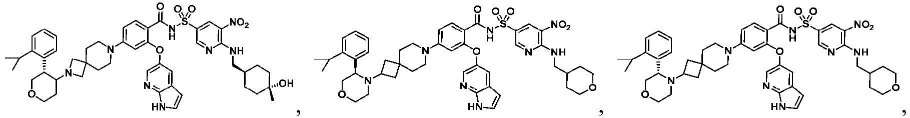 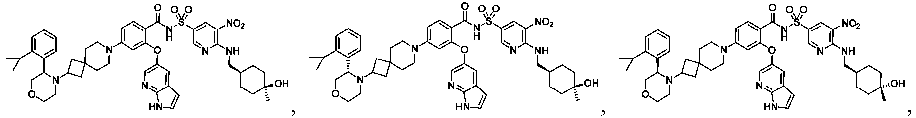 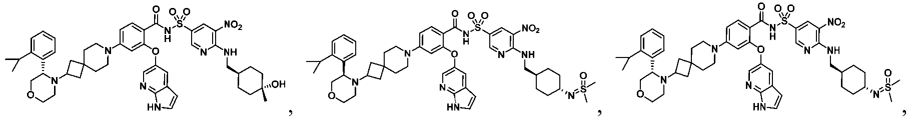 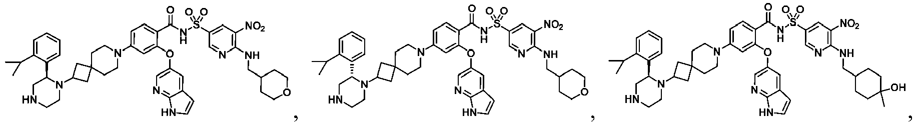 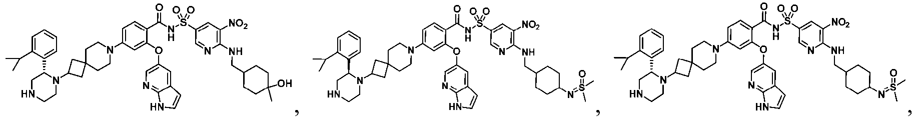 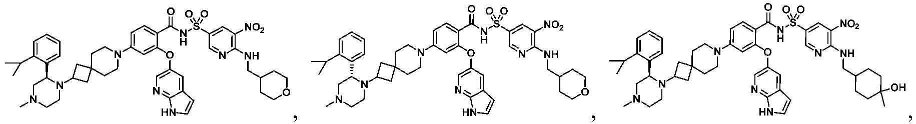 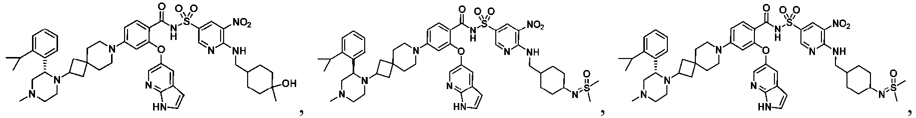 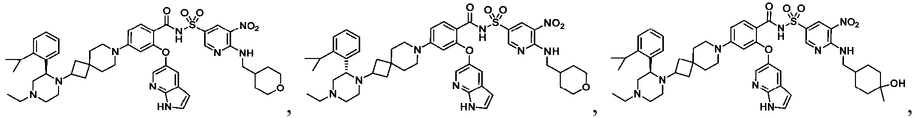 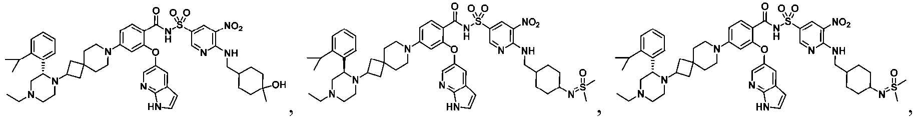 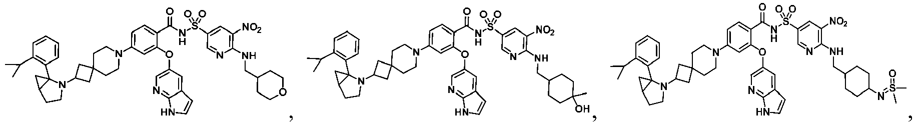 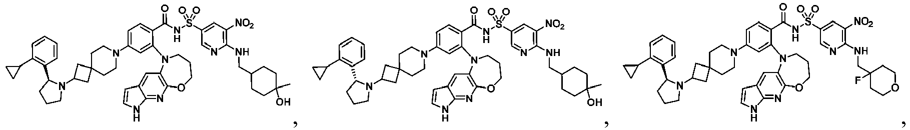 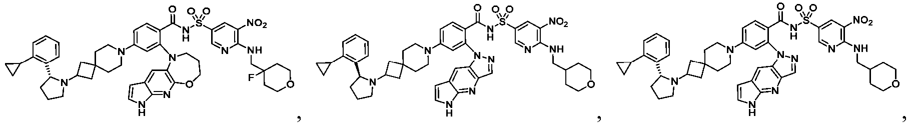 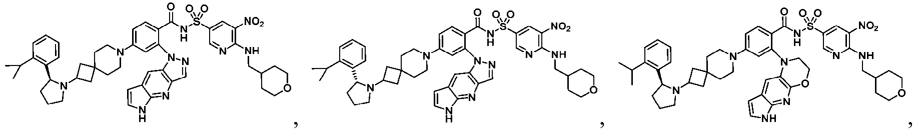 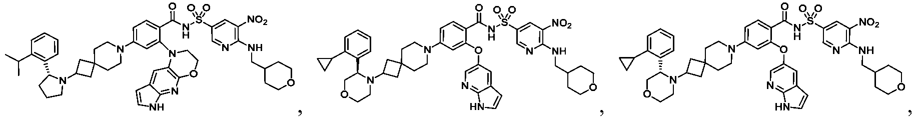 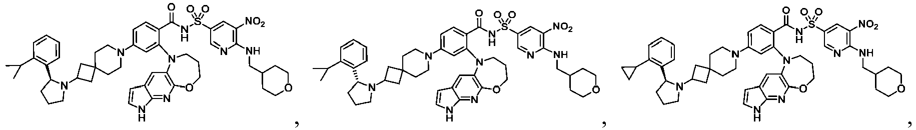 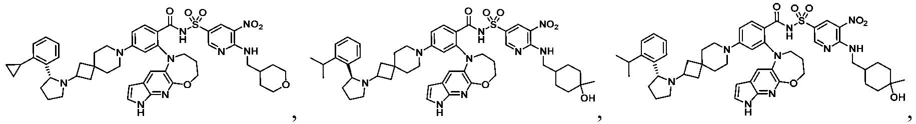 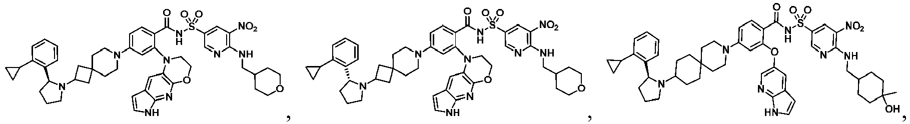 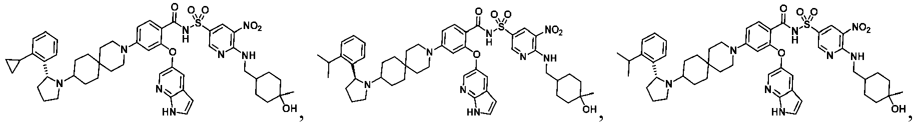 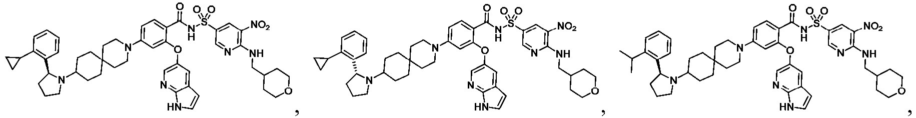 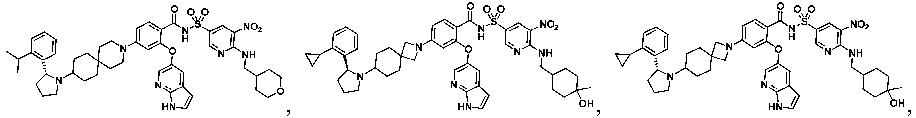 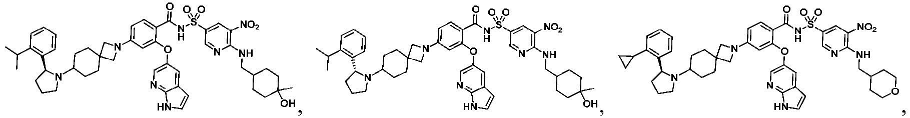 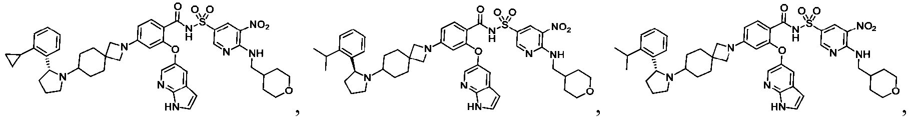 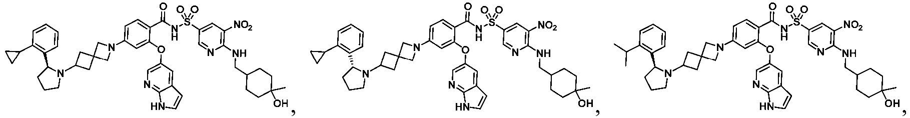 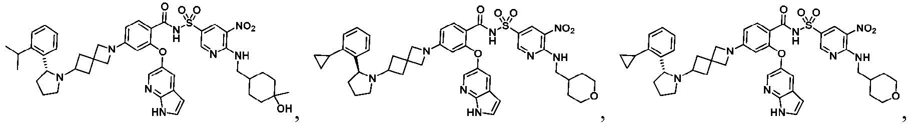 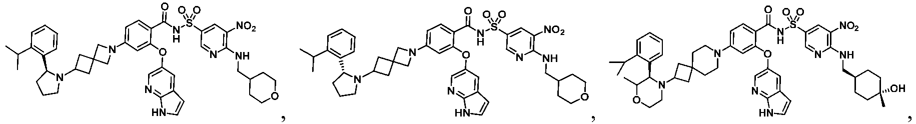 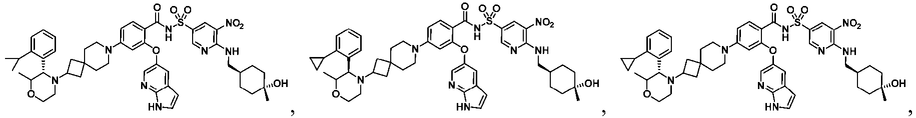 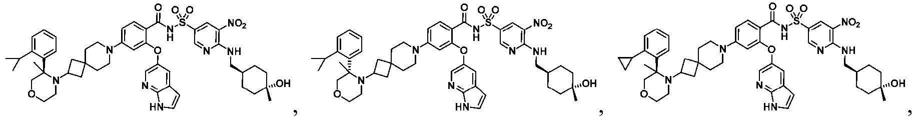 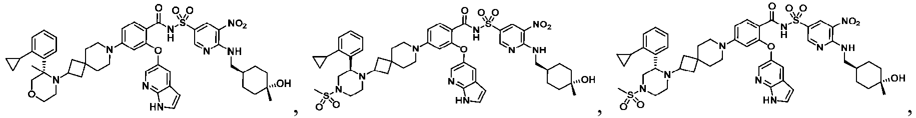 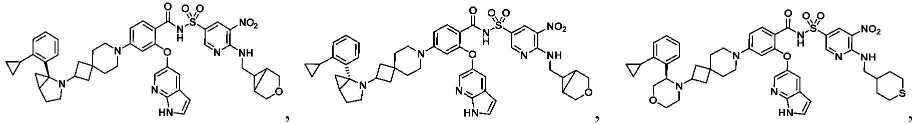 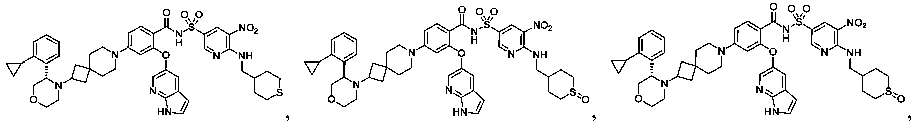 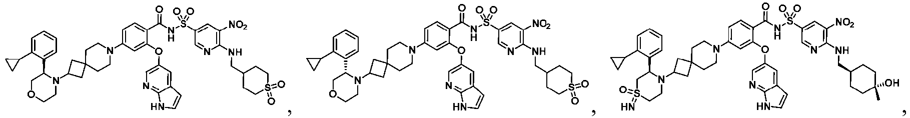 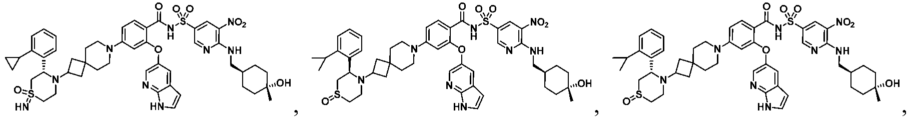 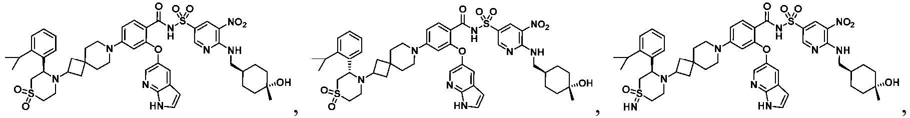 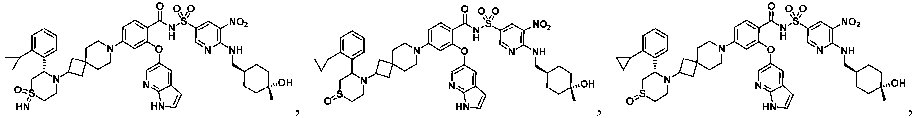 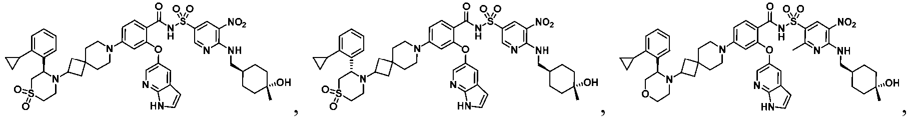 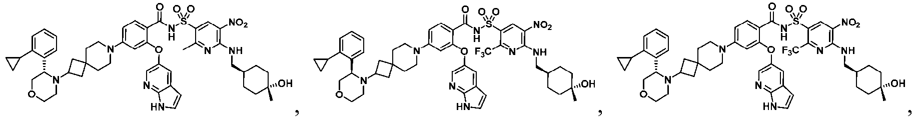 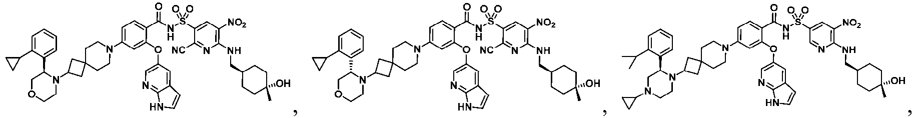 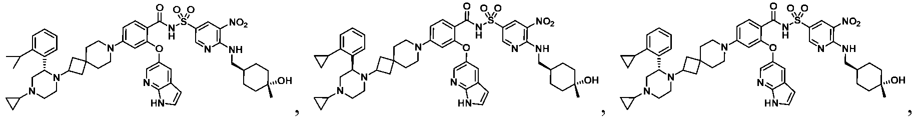 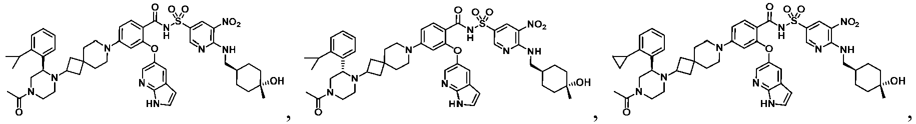 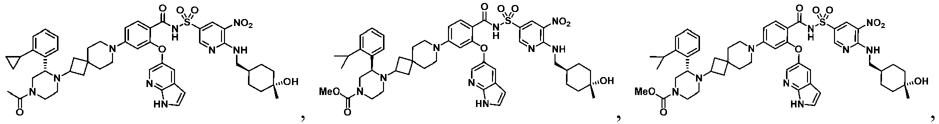 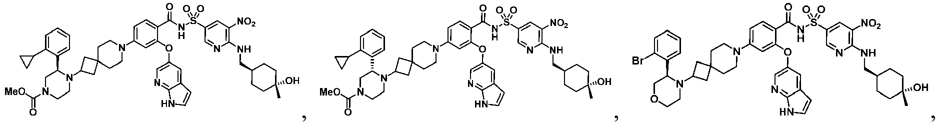 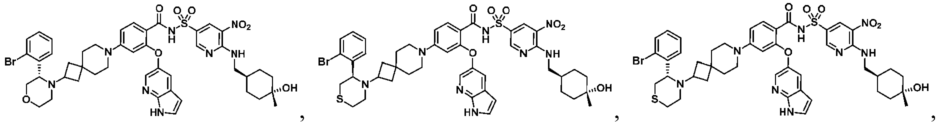 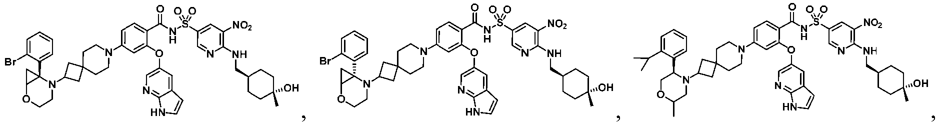 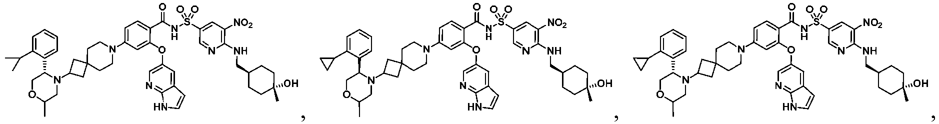 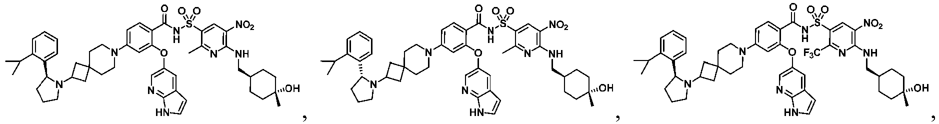 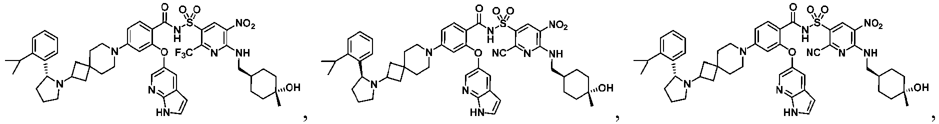 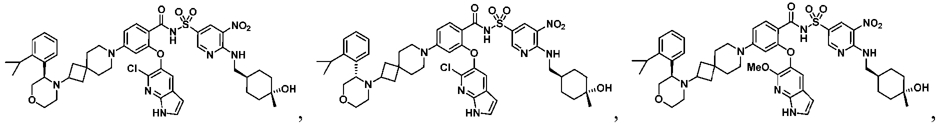 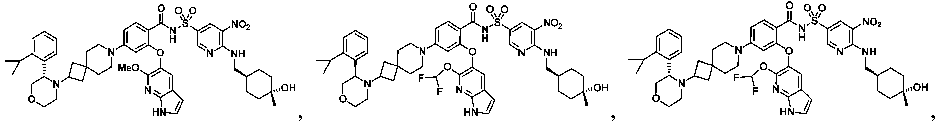 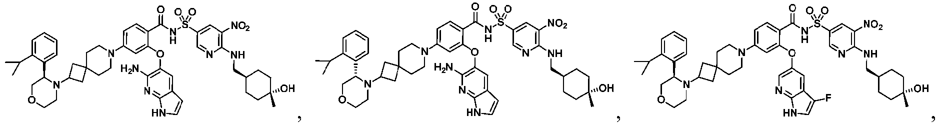 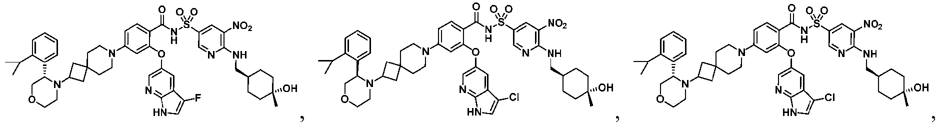 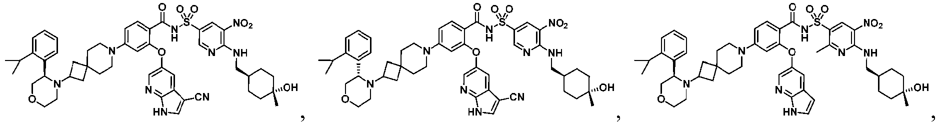 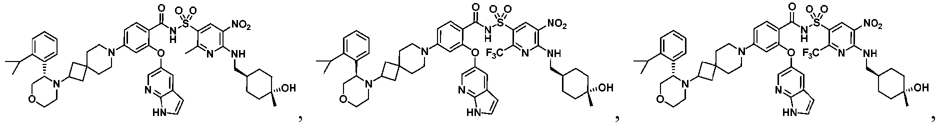 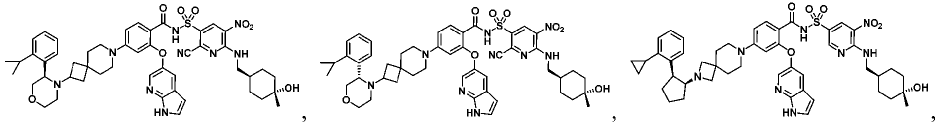 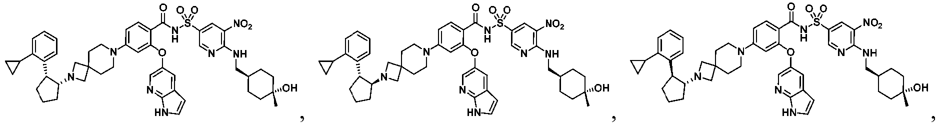 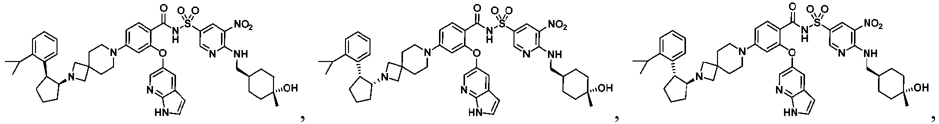 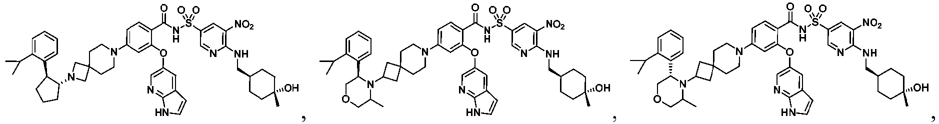 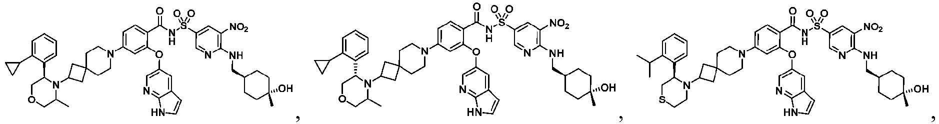 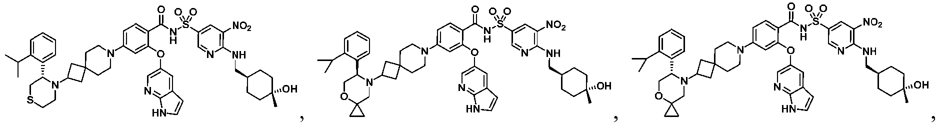 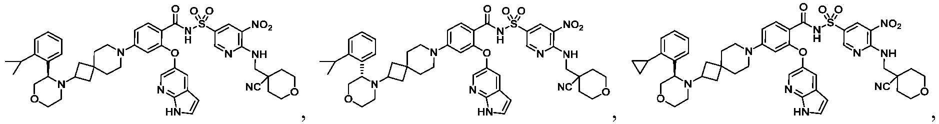 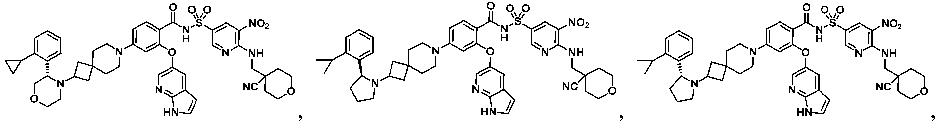 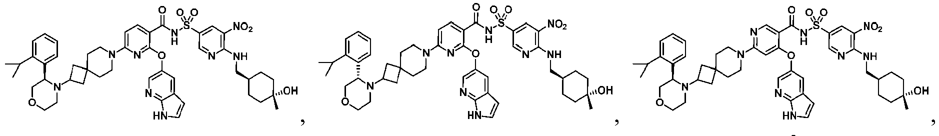 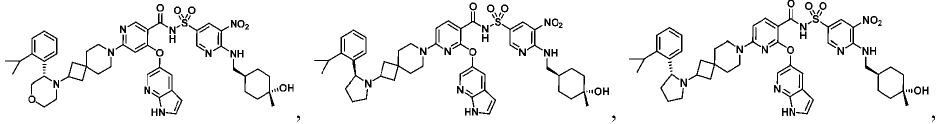 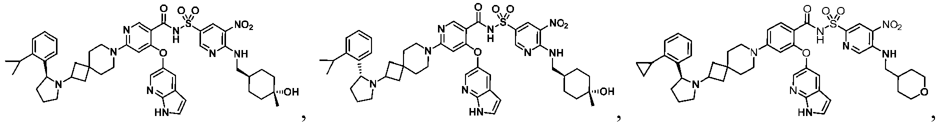 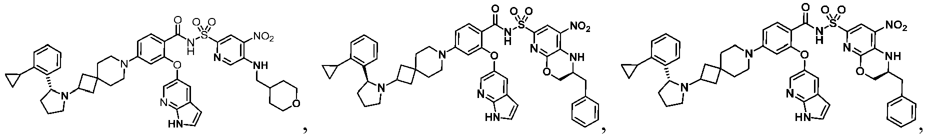 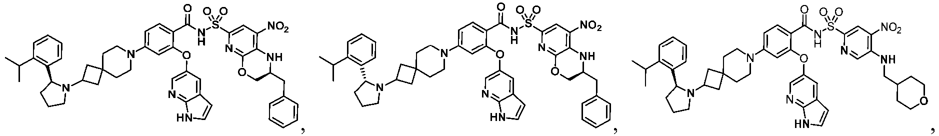 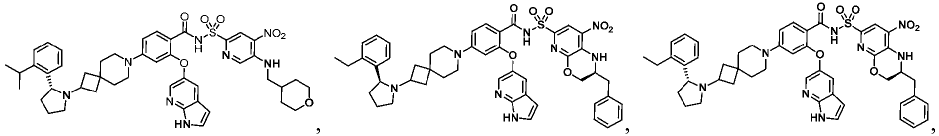 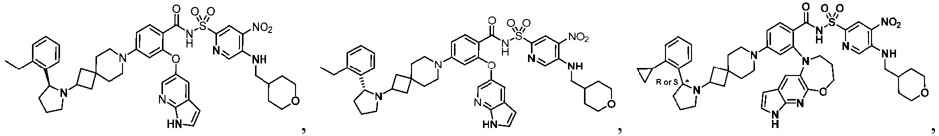 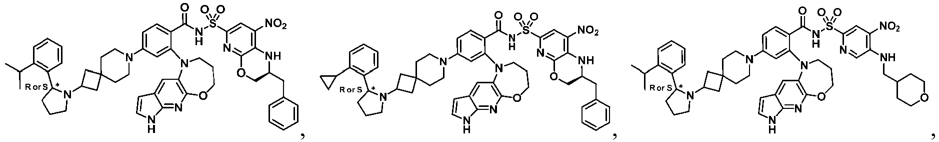 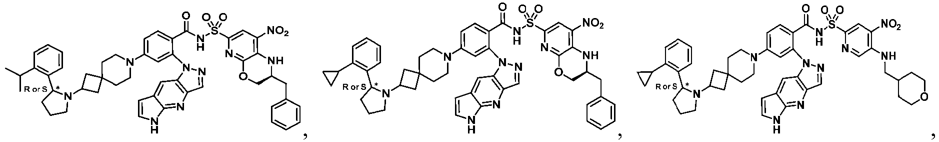 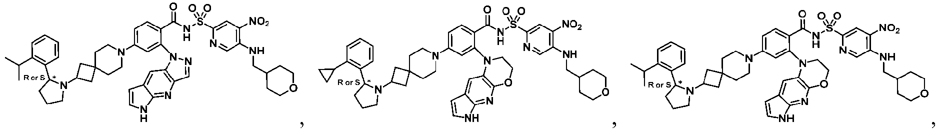 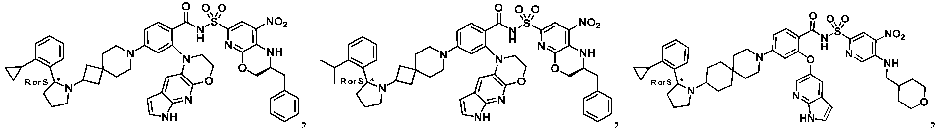 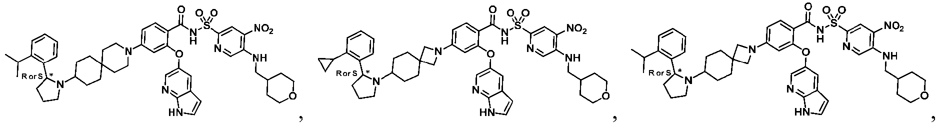 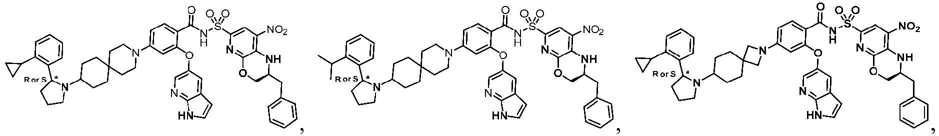 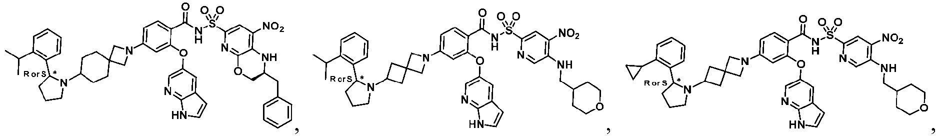 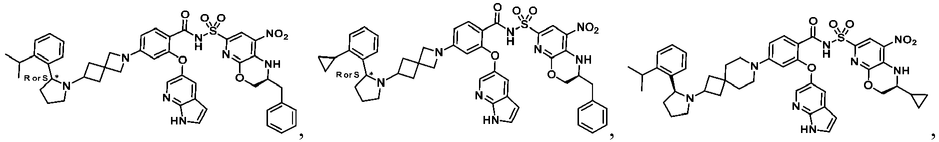 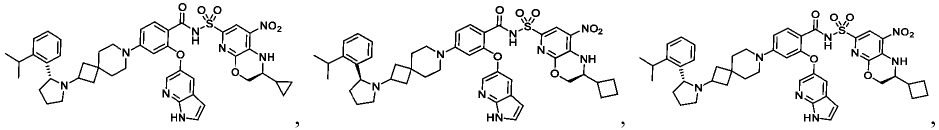 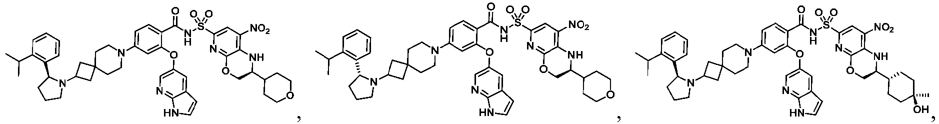 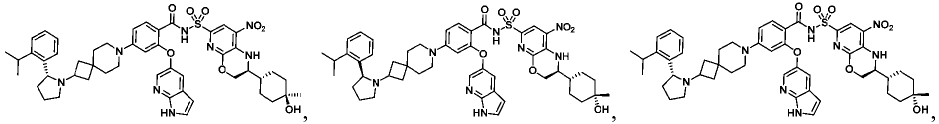 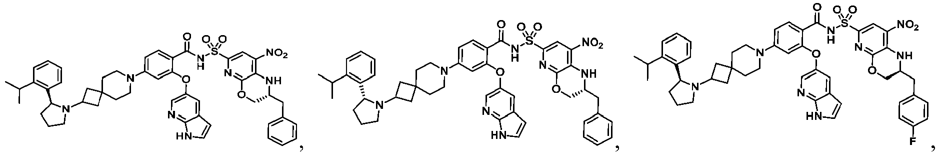 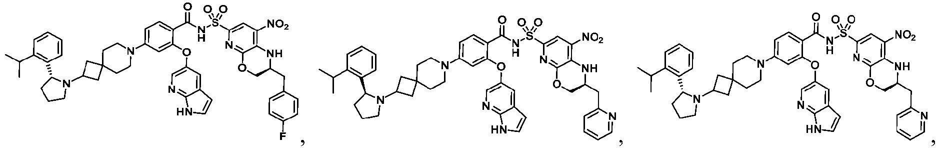 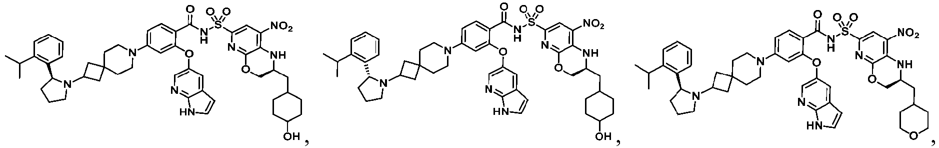 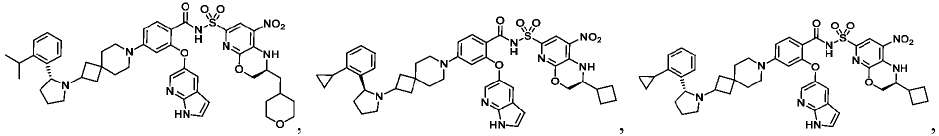 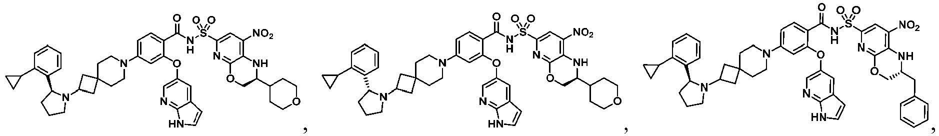 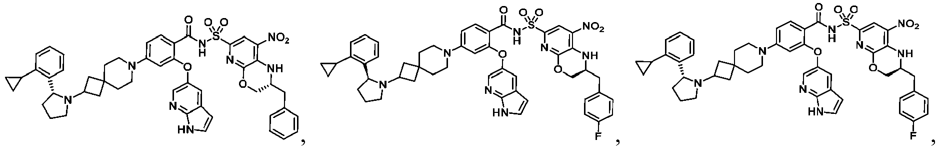 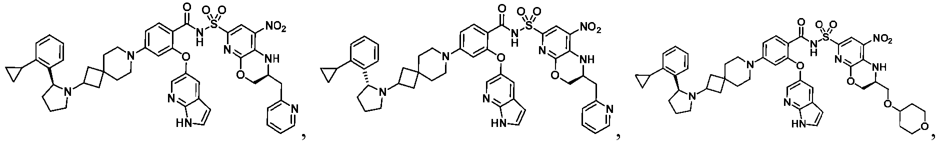 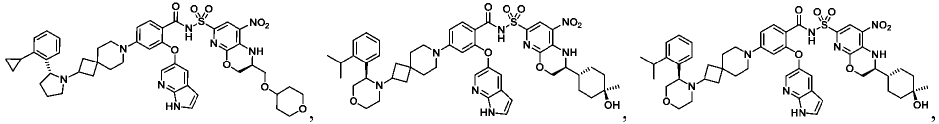 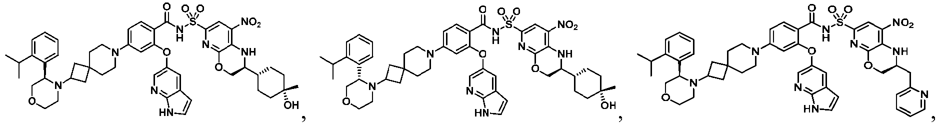 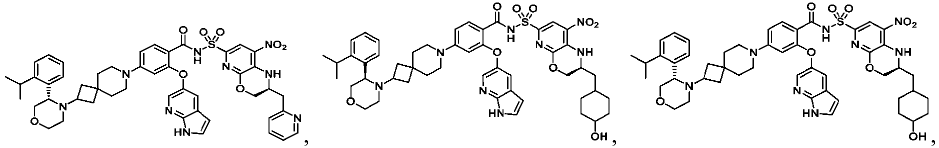 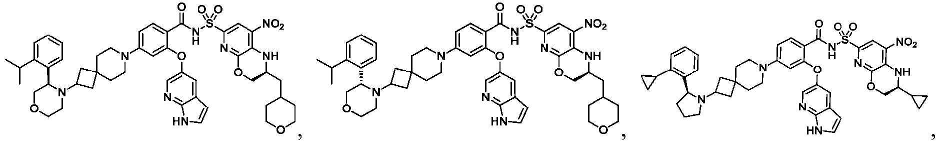 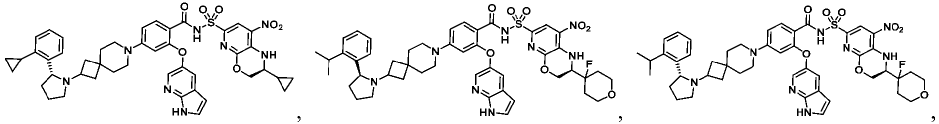 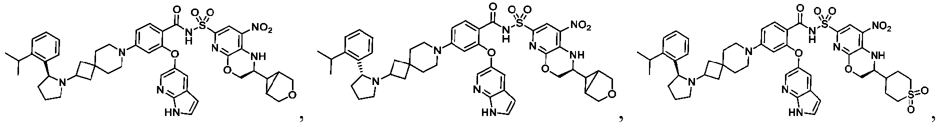 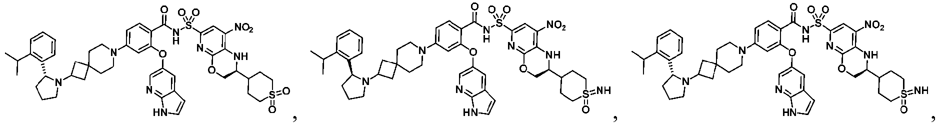 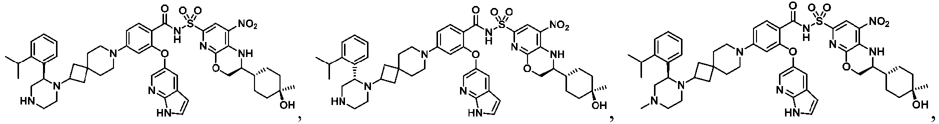 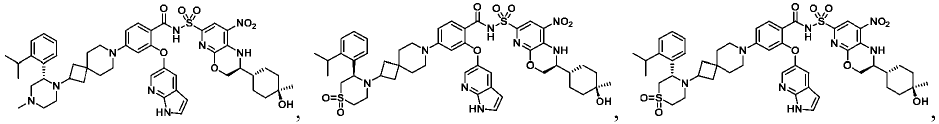 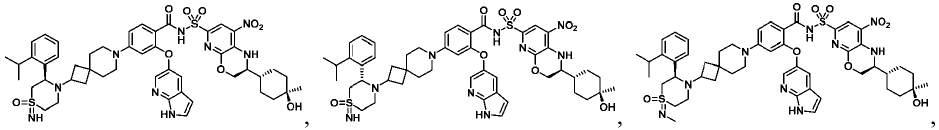 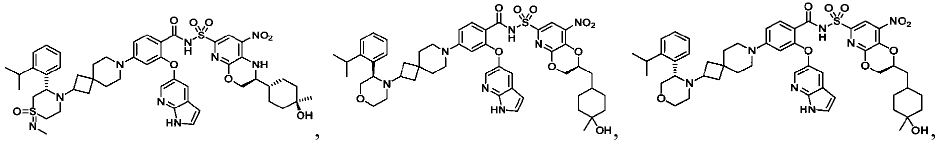 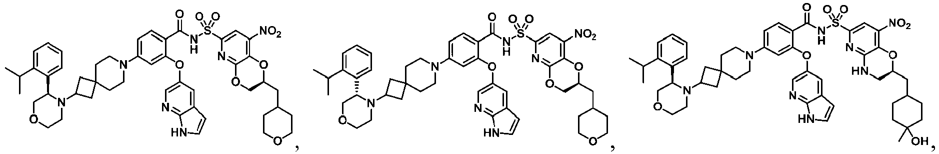 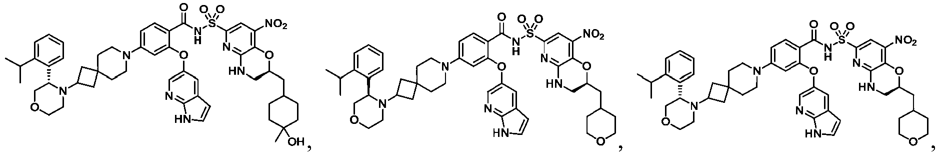 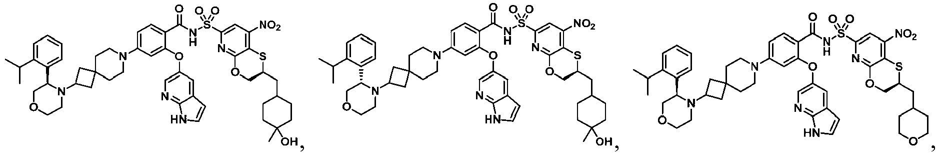 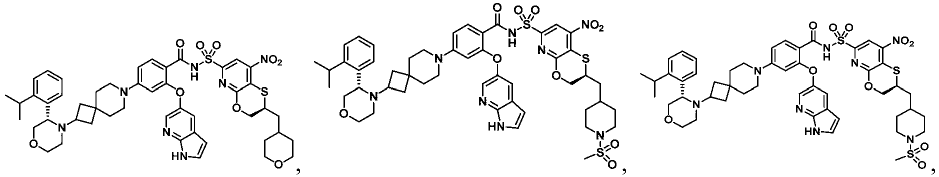 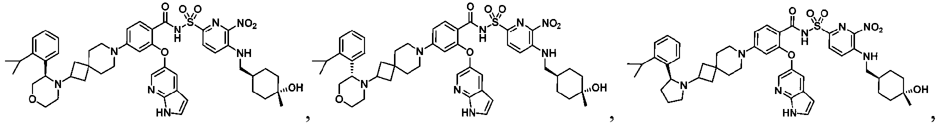 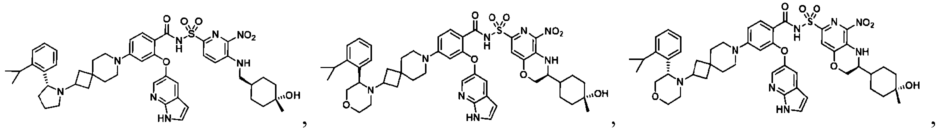 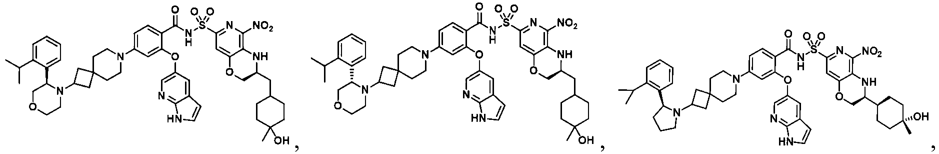 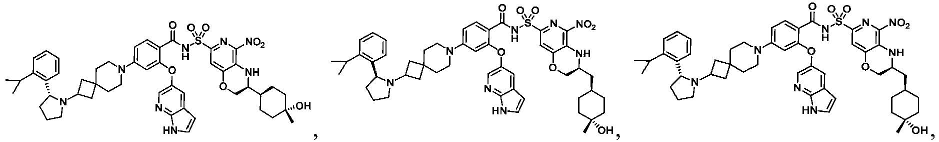 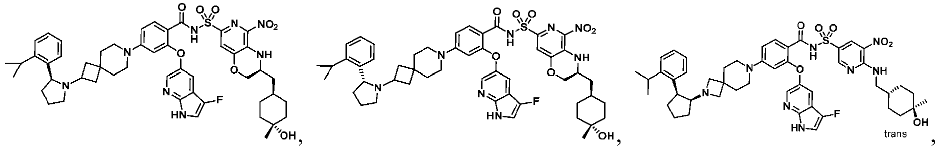 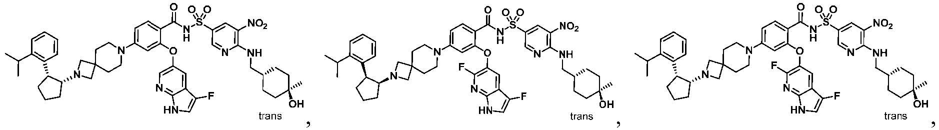 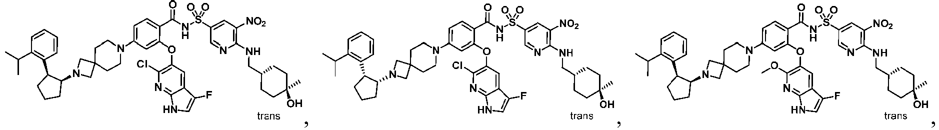 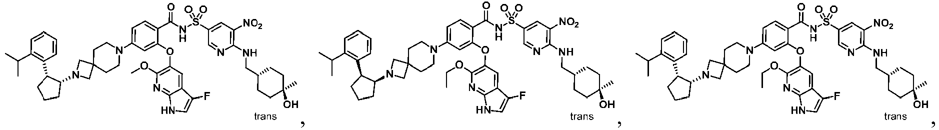 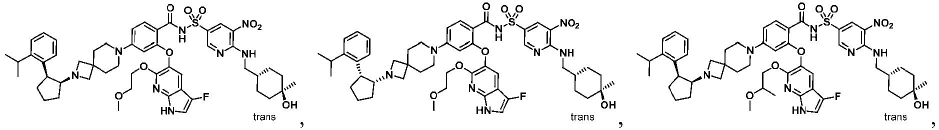 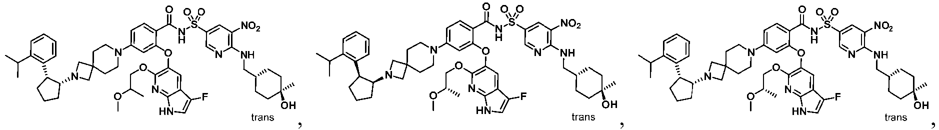 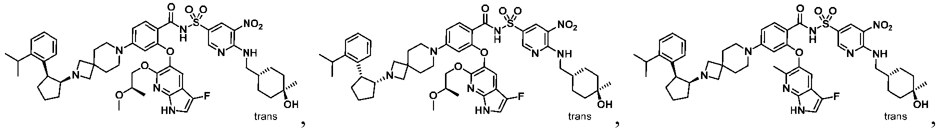 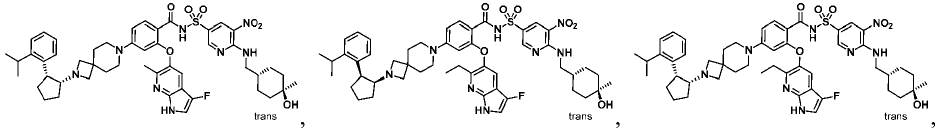 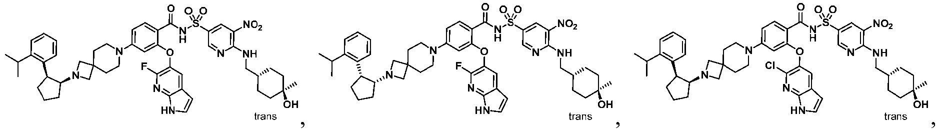 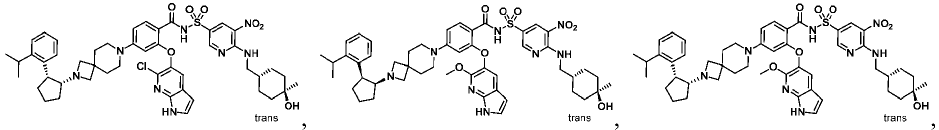 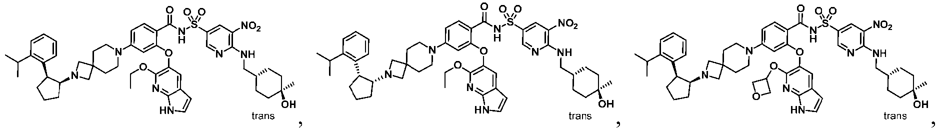 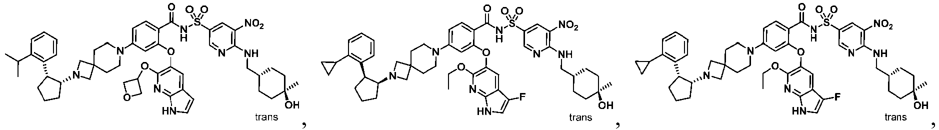 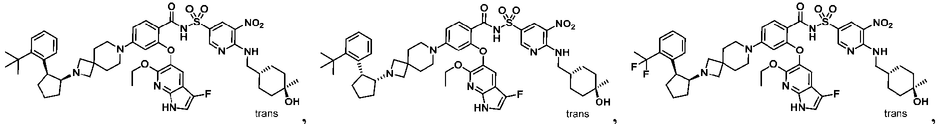 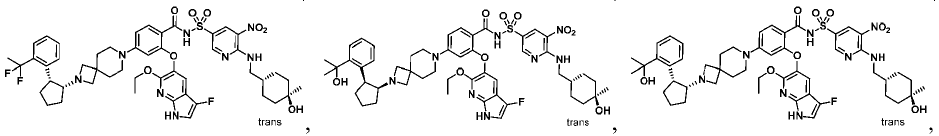 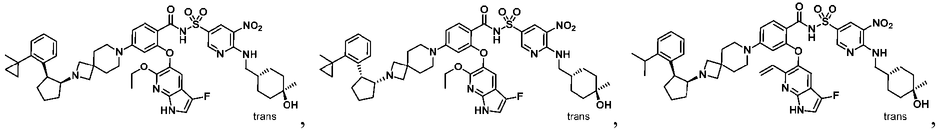 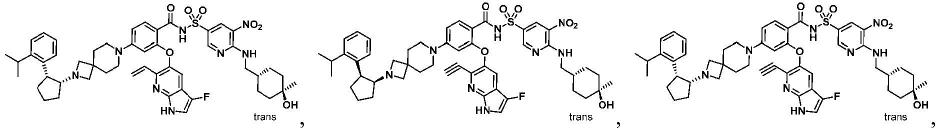 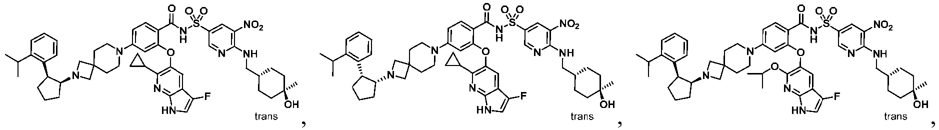 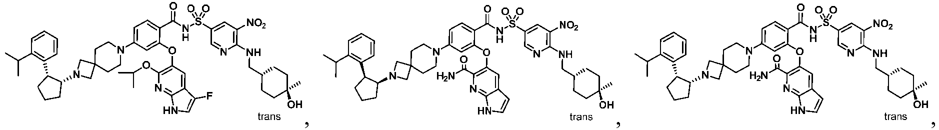 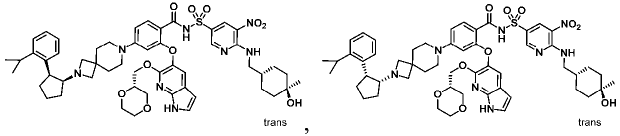 und pharmazeutisch verträglichen Salzen davon.

14. Pharmazeutische Zusammensetzung, die die Verbindung nach einem jeglichen der Ansprüche 1-13 oder das pharmazeutisch verträgliche Salz davon und mindestens einen pharmazeutisch verträglichen Träger umfasst.

15. Verbindung nach einem jeglichen der Ansprüche 1-13 oder pharmazeutisch verträgliches Salz davon oder pharmazeutische Zusammensetzung nach Anspruch 14 zur Verwendung beim Behandeln einer Zellproliferationsstörung oder einer Autoimmunerkrankung,
zum Beispiel wobei
(i) die Zellproliferationsstörung ausgewählt ist aus Brustkrebs, Eierstockkrebs, Blasenkrebs, Gebärmutterkrebs, Prostatakrebs, Hodenkrebs, Lungenkrebs (z.B. NSCLC, SCLC, Plattenepithelkarzinom oder Adenokarzinom), Speiseröhrenkrebs, Kopf-Hals-Krebs, Kolorektalkrebs, Nierenkrebs (z.B. RCC), Leberkrebs (z.B. HCC), Bauchspeicheldrüsenkrebs, Magenkrebs (z.B. Magenkarzinom), Schilddrüsenkrebs, chronischer lymphatischer Leukämie (CLL), lymphoblastischer Leukämie, follikulärem Lymphom, malignen lymphatischen Erkrankungen T- oder B-Zell-Ursprungs, Melanom, myeloischer Leukämie und Myelom;
und/oder
(ii) die Autoimmunerkrankung ausgewählt ist aus Allergie, Alzheimer-Krankheit, akuter disseminierter Enzephalomyelitis, Morbus Addison, ankylosierender Spondylitis, Antiphospholipid-Antikörper-Syndrom, Asthma, Atherosklerose, autoimmuner hämolytischer Anämie, autoimmunen hämolytischen und thrombozytopenischen Zuständen, autoimmuner Hepatitis, autoimmuner Innenohrerkrankung, bullösem Pemphigoid, Zöliakie, Chagas-Krankheit, chronisch obstruktiver Lungenerkrankung, chronischer idiopathischer thrombozytopenischer Purpura (ITP), Churg-Strauss-Syndrom, Morbus Crohn, Dermatomyositis, Diabetes mellitus Typ 1, Endometriose, Goodpasture-Syndrom mit assoziierter Glomerulonephritis und Lungenblutung, Morbus Basedow, Guillain-Barré-Syndrom, Hashimoto-Thyreoiditis, Hidradenitis suppurativa, idiopathischer thrombozytopenischer Purpura, interstitieller Zystitis, Reizdarmsyndrom, Lupus erythematodes, Morphea, Multipler Sklerose, Myasthenia gravis, Narkolepsie, Neuromyotonie, Parkinson-Krankheit, Pemphigus vulgaris, pemiziöser Anämie, Polymyositis, primärer biliärer Zirrhose, Psoriasis, Psoriasis-Arthritis, rheumatoider Arthritis, Schizophrenie, septischem Schock, Sklerodermie, Sjögren-Syndrom, systemischem Lupus erythematodes und damit verbundener Glomerulonephritis, Arteriitis temporalis, Gewebetransplantatabstoßung und hyperakuter Abstoßung transplantierter Organe, Vaskulitis (z.B. ANCA-assoziierter und anderen Vaskulitiden), Vitiligo und Wegener-Granulomatose.

## Revendications

1. Composé de formule (I) : 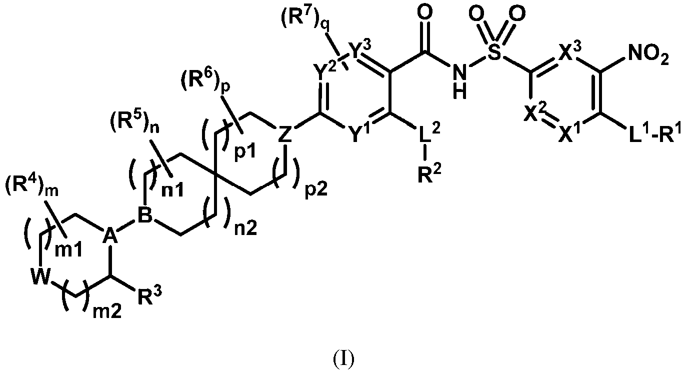 ou sel acceptable pharmaceutiquement de celui-ci, où :
X¹ est choisi parmi N et CR⁸ ; X² est choisi parmi N et CR⁹ ; X³ est choisi parmi N et CR¹⁰ ; où au moins l'un de X¹, X² et X³ est N ;
Y¹, Y² et Y³ sont CH ;
A et B sont indépendamment choisis parmi N et CH ;
W est choisi parmi -CHR¹¹-, -NR¹²-, -O-, -S(O)ᵣ- et -S(O)(=NR¹²)- ;
Z est choisi parmi N et CH ;
L¹ est choisi parmi -CH₂-, -O-, -NH- et -S- ;
L² est choisi parmi une liaison et -O- ;
R¹ est choisi parmi un alkyle en C₁₋₁₀, un cycloalkyle en C₃₋₁₀, un cycloalkyle en C₃₋₁₀-alkyle en C₁₋₄, un hétérocyclyle, un hétérocyclyle-alkyle en C₁₋₄, un aryle, un aryle-alkyle en C₁₋₄, un hétéroaryle, un hétéroaryle-alkyle en C₁₋₄, où l'alkyle, le cycloalkyle, l'hétérocyclyle, l'aryle et l'hétéroaryle sont chacun non substitués ou substitués par au moins un substituant, indépendamment choisi parmi R^{X} ;
R² est choisi parmi 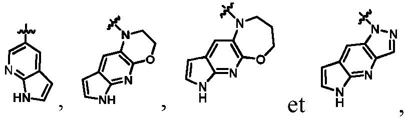 qui sont non substitués ou substitués par au moins un substituant, indépendamment choisi parmi R^{X} ;
R³ est un phényle, où le phényle est non substitué ou substitué par au moins un substituant, indépendamment choisi parmi R^{X} ;
chaque R⁴ est indépendamment choisi parmi un hydrogène, un halogène, -OH, un méthyle, un éthyle, un isopropyle et un cyclopropyle, où le méthyle, l'éthyle, l'isopropyle et le cyclopropyle sont chacun non substitués ou substitués par au moins un substituant, indépendamment choisi parmi R^{X} ; ou deux quelconques de R⁴ conjointement avec les atomes auxquels ils sont liés forment un cyclopropyle, où le cyclopropyle est non substitué ou substitué par 1, 2 ou 3 groupes R^{X} ;
ou « R⁴ et R¹¹ » ou « R⁴ et R¹² » conjointement avec les atomes auxquels ils sont liés forment un cycloalkyle en C₃₋₈ ou un cycle hétérocyclique à 4 à 10 chaînons contenant 1, 2 ou 3 hétéroatomes indépendamment choisis parmi l'oxygène, le soufre, l'azote et le phosphore, et éventuellement substitué par 1, 2 ou 3 groupes R^{X} ;
chaque R⁵ et R⁶ sont indépendamment choisis parmi un hydrogène, un halogène, un alkyle en C₁₋₁₀, un cycloalkyle en C₃₋₁₀, CN, NO₂, -NH₂ et -OH, où l'alkyle et le cycloalkyle sont chacun non substitués ou substitués par au moins un substituant, indépendamment choisi parmi R^{X} ;
chaque R⁷ est indépendamment choisi parmi un hydrogène, un halogène, un alkyle en C₁₋₁₀, un cycloalkyle en C₃₋₁₀, CN, NO₂, -NR^{A7}R^{B7}, -OR^{A7} et -C(O)R^{A7}, où l'alkyle et le cycloalkyle sont chacun non substitués ou substitués par au moins un substituant, indépendamment choisi parmi R^{X} ;
R⁸ est choisi parmi un hydrogène, un halogène, un alkyle en C₁₋₁₀, un alcényle en C₂₋₁₀, un alcynyle en C₂₋₁₀, un cycloalkyle en C₃₋₁₀, un cycloalkyle en C₃₋₁₀-alkyle en C₁₋₄, un hétérocyclyle, un hétérocyclyle-alkyle en C₁₋₄, CN, NO₂, -NR^{A8}R^{B8}, -OR^{A8}, -C(O)R^{A8}, -C(O)OR^{A8}, -OC(O)R^{A8} et -S(O)ᵣR^{A8}, où l'alkyle, l'alcényle, l'alcynyle, le cycloalkyle et l'hétérocyclyle sont chacun non substitués ou substitués par au moins un substituant, indépendamment choisi parmi R^{X} ;
ou R¹ et R⁸ conjointement avec les atomes auxquels ils sont liés forment un cycloalkyle en C₅₋₁₀ ou un cycle hétérocyclique à 5 à 12 chaînons contenant 1, 2 ou 3 hétéroatomes indépendamment choisis parmi l'oxygène, le soufre, l'azote et le phosphore, et éventuellement substitué par 1, 2 ou 3 groupes R^{X} ;
R⁹ est choisi parmi un hydrogène, un halogène, CN, OH, un méthyle, un éthyle et un trifluorométhyle ;
R¹⁰ est choisi parmi un hydrogène, un halogène, un alkyle en C₁₋₁₀, CN, NO₂, -NH₂ et - OH, où l'alkyle est non substitué ou substitué par au moins un substituant, indépendamment choisi parmi R^{X} ;
R¹¹ est choisi parmi un hydrogène, un alkyle en C₁₋₁₀ et un cycloalkyle en C₃₋₁₀, où l'alkyle et le cycloalkyle sont chacun non substitués ou substitués par au moins un substituant, indépendamment choisi parmi R^{X} ;
R¹² est choisi parmi un hydrogène, un alkyle en C₁₋₁₀ et un cycloalkyle en C₃₋₁₀, un hétérocyclyle, un hétérocyclyle-alkyle en C₁₋₄, un aryle, un aryle-alkyle en C₁₋₄, un hétéroaryle, - C(O)R^{A12}, -C(O)NR^{A12}R^{B12}, -C(0)OR^{A12}, -S(O)ᵣR^{A12} et -S(O)ᵣNR^{A12}R^{B12}, où l'alkyle, le cycloalkyle, l'hétérocyclyle, l'aryle et l'hétéroaryle sont chacun non substitués ou substitués par au moins un substituant, indépendamment choisi parmi R^{X} ;
chaque R^{A7}, R^{A8}, R^{A12}, R^{B7}, R^{B8} et R^{B12} est indépendamment choisi parmi un hydrogène, un alkyle en C₁₋₁₀, un alcényle en C₂₋₁₀, un alcynyle en C₂₋₁₀, un cycloalkyle en C₃₋₁₀, un cycloalkyle en C₃₋₁₀-alkyle en C₁₋₄, un hétérocyclyle, un hétérocyclyle-alkyle en C₁₋₄, un aryle, un aryle-alkyle en C₁₋₄, un hétéroaryle et un hétéroaryle-alkyle en C₁₋₄, où l'alkyle, l'alcényle, l'alcynyle, le cycloalkyle, l'hétérocyclyle, l'aryle et l'hétéroaryle sont chacun non substitués ou substitués par au moins un substituant, indépendamment choisi parmi R^{X} ;
ou « R^{A7} et R^{B7} » ou « R^{A8} et R^{B8} » ou « R^{A12} et R^{B12} » conjointement avec le ou les atomes auxquels ils sont liés forment un cycle hétérocyclique à 4 à 12 chaînons contenant 0, 1 ou 2 hétéroatomes supplémentaires indépendamment choisis parmi l'oxygène, le soufre, l'azote et le phosphore et éventuellement substitués par 1, 2 ou 3 groupes R^{X} ;
chaque R^{X} est indépendamment choisi parmi un hydrogène, un alkyle en C₁₋₁₀, un alcényle en C₂₋₁₀, un alcynyle en C₂₋₁₀, un cycloalkyle en C₃₋₁₀, un cycloalkyle en C₃₋₁₀-alkyle en C₁₋₄, un hétérocyclyle, un hétérocyclyle-alkyle en C₁₋₄, un aryle, un aryle-alkyle en C₁₋₄, un hétéroaryle, un hétéroaryle-alkyle en C₁₋₄, un halogène, CN, NO₂, -(CR^{c1}R^{d1})ₜNR^{a1}R^{b1}, -(CR^{c1}R^{d1})ₜOR^{b1}, - (CR^{c1}R^{d1})ₜC(O)R^{a1}, -(CR^{c1}R^{d1})ₜC(=NR^{e1})R^{a1}, -(CR^{c1}R^{d1})ₜC(=N-OR^{b1})R^{a1}, -(CR^{c1}R^{d1})ₜC(O)OR^{b1}, - (CR^{c1}R^{d1})ₜOC(O)R^{b1}, -(CR^{c1}R^{d1})ₜC(O)NR^{a1}R^{b1}, -(CR^{c1}R^{d1})ₜNR^{a1}C(O)R^{b1}, - (CR^{c1}R^{d1})ₜC(=NR^{e1})NR^{a1}Rb^{c1}, -(CR^{c1}R^{d1})ₜNR^{a1}C(=NR^{e1})R^{b1}, -(CR^{c1}R^{d1})ₜOC(O)NR^{a1}R^{b1}, - (CR^{c1}R^{d1})ₜNR^{a1}C(O)OR^{b1}, -(CR^{c1}R^{d1})ₜNR^{a1}C(O)NR^{a1}R^{b1}, -(CR^{c1}R^{d1})ₜNR^{a1}C(S)NR^{a1}R^{b1}, - (CR^{c1}R^{d1})ₜNR^{a1}C(=NR^{e1})NR^{a1}R^{b1}, -(CR^{c1}R^{d1})ₜS(O)ᵣR^{b1}, -(CR^{c1}R^{d1})ₜS(O)(=NR^{e1})R^{b1}, - (CR^{c1}R^{d1})ₜN=S(O)R^{a1}R^{b1}, -(CR^{c1}R^{d1})ₜS(O)₂OR^{b1}, -(CR^{c1}R^{d1})ₜOS(O)₂R^{b1}, - (CR^{c1}R^{d1})ₜNR^{a1}S(O)ᵣR^{b1}, -(CR^{c1}R^{d1})ₜNR^{a1}S(O)(=NR^{e1})R^{b1}, -(CR^{c1}R^{d1})ₜS(O)ᵣNR^{a1}R^{b1}, - (CR^{c1}R^{d1})ₜS(O)(=NR^{e1})NR^{a1}R^{b1}, -(CR^{c1}R^{d1})ₜNR^{a1}S(O)₂NR^{a1}R^{b1}, - (CR^{c1}R^{d1})ₜNR^{a1}S(O)(=NR^{e1})NR^{a1}R^{b1}, -(CR^{c1}R^{d1})ₜP(O)R^{a1}R^{b1} et -(CR^{c1}R^{d1})ₜP(O)(OR^{a1})(OR^{b1}), où l'alkyle, l'alcényle, l'alcynyle, le cycloalkyle, l'hétérocyclyle, l'aryle et l'hétéroaryle sont chacun non substitués ou substitués par au moins un substituant, indépendamment choisi parmi R^{Y} ;
chaque R^{a1} et chaque R^{b1} sont indépendamment choisis parmi un hydrogène, un alkyle en C₁₋₁₀, un alcényle en C₂₋₁₀, un alcynyle en C₂₋₁₀, un cycloalkyle en C₃₋₁₀, un cycloalkyle en C₃₋₁₀-alkyle en C₁₋₄, un hétérocyclyle, un hétérocyclyle-alkyle en C₁₋₄, un aryle, un aryle-alkyle en C₁₋₄, un hétéroaryle et un hétéroaryle-alkyle en C₁₋₄, où l'alkyle, l'alcényle, l'alcynyle, le cycloalkyle, l'hétérocyclyle, l'aryle et l'hétéroaryle sont chacun non substitués ou substitués par au moins un substituant, indépendamment choisi parmi R^{Y} ;
ou R^{a1} et R^{b1} conjointement avec le ou les atomes auxquels ils sont liés forment un cycle hétérocyclique à 4 à 12 chaînons contenant 0, 1 ou 2 hétéroatomes supplémentaires indépendamment choisis parmi l'oxygène, le soufre, l'azote et le phosphore, et éventuellement substitués par 1, 2 ou 3 groupes R^{Y} ;
chaque R^{c1} et chaque R^{d1} sont indépendamment choisis parmi un hydrogène, un halogène, un alkyle en C₁₋₁₀, un alcényle en C₂₋₁₀, un alcynyle en C₂₋₁₀, un cycloalkyle en C₃₋₁₀, un cycloalkyle en C₃₋₁₀-alkyle en C₁₋₄, un hétérocyclyle, un hétérocyclyle-alkyle en C₁₋₄, un aryle, un aryle-alkyle en C₁₋₄, un hétéroaryle et un hétéroaryle-alkyle en C₁₋₄, où l'alkyle, l'alcényle, l'alcynyle, le cycloalkyle, l'hétérocyclyle, l'aryle et l'hétéroaryle sont chacun non substitués ou substitués par au moins un substituant, indépendamment choisi parmi R^{Y} ;
chaque R^{e1} est indépendamment choisi parmi un hydrogène, un alkyle en C₁₋₁₀, un cycloalkyle en C₃₋₁₀, un cycloalkyle en C₃₋₁₀-alkyle en C₁₋₄, CN, NO₂, -OR^{a2}, -SR^{a2}, -S(O)ᵣR^{a2}, - C(O)R^{a2}, -C(O)OR^{a2}, -S(O)ᵣNR^{a2}R^{b2} et -C(O)NR^{a2}R^{b2} ;
chaque R^{Y} est indépendamment choisi parmi un alkyle en C₁₋₁₀, un alcényle en C₂₋₁₀, un alcynyle en C₂₋₁₀, un cycloalkyle en C₃₋₁₀, un cycloalkyle en C₃₋₁₀-alkyle en C₁₋₄, un hétérocyclyle, un hétérocyclyle-alkyle en C₁₋₄, un aryle, un aryle-alkyle en C₁₋₄, un hétéroaryle, un hétéroaryle-alkyle en C₁₋₄, un halogène, CN, NO₂, -(CR^{c2}R^{d2})ₜNR^{a2}R^{b2}, -(CR^{c2}R^{d2})ₜOR^{b2}, -(CR^{c2}R^{d2})ₜC(O)R^{a2}, -(CR^{c2}R^{d2})ₜC(=NR^{e2})R^{a2}, -(CR^{c2}R^{d2})ₜC(=N-OR^{b2})R^{a2}, -(CR^{c2}R^{d2})ₜC(O)OR^{b2}, - (CR^{c2}R^{d2})ₜOC(O)R^{b2}, -(CR^{c2}R^{d2})ₜC(O)NR^{a2}R^{b2}, -(CR^{c2}R^{d2})ₜNR^{a2}C(O)R^{b2}, - (CR^{c2}R^{d2})ₜC(=NR^{e2})NR^{a2}R^{b2}, -(CR^{c2}R^{d2})ₜNR^{a2}C(=NR^{e2})R^{b2}, -(CR^{c2}R^{d2})ₜOC(O)NR^{a2}R^{b2}, - (CR^{c2}R^{d2})ₜNR^{a2}C(O)OR^{b2}, -(CR^{c2}R^{d2})ₜNR^{a2}C(O)NR^{a2}R^{b2}, -(CR^{c2}R^{d2})ₜNR^{a2}C(S)NR^{a2}R^{b2}, - (CR^{c2}R^{d2})ₜNR^{a2}C(=NR^{e2})NR^{a2}R^{b2}, -(CR^{c2}R^{d2})ₜS(O)ᵣR^{b2}, -(CR^{c2}R^{d2})ₜS(O)(=NR^{e2})R^{b2}, - (CR^{c2}R^{d2})ₜN=S(O)R^{a2}R^{b2}, -(CR^{c2}R^{d2})ₜS(O)₂OR^{b2}, -(CR^{c2}R^{d2})ₜOS(O)₂R^{b2}, - (CR^{c2}R^{d2})ₜNR^{a2}S(O)ᵣR^{b2}, -(CR^{c2}R^{d2})ₜNR^{a2}S(O)(=NR^{e2})R^{b2}, -(CR^{c2}R^{d2})ₜS(O)ᵣNR^{a2}R^{b2}, - (CR^{c2}R^{d2})ₜS(O)(=NR^{e2})NR^{a2}R^{b2}, -(CR^{c2}R^{d2})ₜNR^{a2}S(O)₂NR^{a2}R^{b2}, - (CR^{c2}R^{d2})ₜNR^{a2}S(O)(=NR^{e2})NR^{a2}R^{b2}, -(CR^{c2}R^{d2})ₜP(O)R^{a2}R^{b2} et -(CR^{c2}R^{d2})ₜP(O)(OR^{a2})(OR^{b2}), où l'alkyle, l'alcényle, l'alcynyle, le cycloalkyle, l'hétérocyclyle, l'aryle et l'hétéroaryle sont chacun non substitués ou substitués par au moins un substituant, indépendamment choisi parmi OH, CN, un amino, un halogène, un alkyle en C₁₋₁₀, un alcényle en C₂₋₁₀, un alcynyle en C₂₋₁₀, un cycloalkyle en C₃₋₁₀, un alcoxy en C₁₋₁₀, un cycloalcoxy en C₃₋₁₀, un alkylthio en C₁₋₁₀, un cycloalkylthio en C₃₋₁₀, un alkylamino en C₁₋₁₀, un cycloalkylamino en C₃₋₁₀ et un di(alkyle en C₁₋₁₀)amino ;
chaque R^{a2} et chaque R^{b2} sont indépendamment choisis parmi un hydrogène, un alkyle en C₁₋₁₀, un alcényle en C₂₋₁₀, un alcynyle en C₂₋₁₀, un cycloalkyle en C₃₋₁₀, un cycloalkyle en C₃₋₁₀-alkyle en C₁₋₄, un alcoxy en C₁₋₁₀, un cycloalcoxy en C₃₋₁₀, un alkylthio en C₁₋₁₀, un cycloalkylthio en C₃₋₁₀, un alkylamino en C₁₋₁₀, un cycloalkylamino en C₃₋₁₀, un di(alkyle en C₁₋₁₀)amino, un hétérocyclyle, un hétérocyclyle-alkyle en C₁₋₄, un aryle, un aryle-alkyle en C₁₋₄, un hétéroaryle et un hétéroaryle-alkyle en C₁₋₄, où l'alkyle, l'alcényle, l'alcynyle, le cycloalkyle, l'alcoxy, le cycloalcoxy, l'alkylthio, le cycloalkylthio, l'alkylamino, le cycloalkylamino, l'hétérocyclyle, l'aryle et l'hétéroaryle sont chacun non substitués ou substitués par au moins un substituant, indépendamment choisi parmi un halogène, CN, un alkyle en C₁₋₁₀, un alcényle en C₂₋₁₀, un alcynyle en C₂₋₁₀, un cycloalkyle en C₃₋₁₀, OH, un alcoxy en C₁₋₁₀, un cycloalcoxy en C₃₋₁₀, un alkylthio en C₁₋₁₀, un cycloalkylthio en C₃₋₁₀, un amino, un alkylamino en C₁₋₁₀, un cycloalkylamino en C₃₋₁₀ et un di(alkyle en C₁₋₁₀)amino ;
chaque R^{c2} et chaque R^{d2} sont indépendamment choisis parmi un hydrogène, un halogène, un alkyle en C₁₋₁₀, un alcényle en C₂₋₁₀, un alcynyle en C₂₋₁₀, un cycloalkyle en C₃₋₁₀, un cycloalkyle en C₃₋₁₀-alkyle en C₁₋₄, un alcoxy en C₁₋₁₀, un cycloalcoxy en C₃₋₁₀, un alkylthio en C₁₋₁₀, un cycloalkylthio en C₃₋₁₀, un alkylamino en C₁₋₁₀, un cycloalkylamino en C₃₋₁₀, un di(alkyle en C₁₋₁₀)amino, un hétérocyclyle, un hétérocyclyle-alkyle en C₁₋₄, un aryle, un aryle-alkyle en C₁₋₄, un hétéroaryle et un hétéroaryle-alkyle en C₁₋₄, où l'alkyle, l'alcényle, l'alcynyle, le cycloalkyle, l'alcoxy, le cycloalcoxy, l'alkylthio, le cycloalkylthio, l'alkylamino, le cycloalkylamino, l'hétérocyclyle, l'aryle et l'hétéroaryle sont chacun non substitués ou substitués par au moins un substituant, indépendamment choisi parmi un halogène, CN, un alkyle en C₁₋₁₀, un alcényle en C₂₋₁₀, un alcynyle en C₂₋₁₀, un cycloalkyle en C₃₋₁₀, OH, un alcoxy en C₁₋₁₀, un cycloalcoxy en C₃₋₁₀, un alkylthio en C₁₋₁₀, un cycloalkylthio en C₃₋₁₀, un amino, un alkylamino en C₁₋₁₀, un cycloalkylamino en C₃₋₁₀ et un di(alkyle en C₁₋₁₀)amino ;
chaque R^{e2} est indépendamment choisi parmi un hydrogène, CN, NO₂, un alkyle en C₁₋₁₀, un cycloalkyle en C₃₋₁₀, un cycloalkyle en C₃₋₁₀-alkyle en C₁₋₄, un alcoxy en C₁₋₁₀, un cycloalcoxy en C₃₋₁₀, -C(O)-alkyle en C₁₋₄, -C(O)-cycloalkyle en C₃₋₁₀, -C(O)O-alkyle en C₁₋₄, -C(O)O-cycloalkyle en C₃₋₁₀, -C(O)N(alkyle en C₁₋₄)₂, -C(O)N(cycloalkyle en C₃₋₁₀)₂, -S(O)₂-alkyle en C₁₋₄, -S(O)₂-cycloalkyle en C₃₋₁₀, -S(O)₂N(alkyle en C₁₋₄)₂ et -S(O)₂N(cycloalkyle en C₃₋₁₀)₂ ;
m, n, p et q sont indépendamment choisis parmi 0, 1, 2 et 3 ;
m1, m2, n1, n2, p1 et p2 sont indépendamment choisis parmi 0 et 1 ;
chaque r est indépendamment choisi parmi 0, 1 et 2 ;
chaque t est indépendamment choisi parmi 0, 1, 2, 3 et 4 ;
chaque u est indépendamment choisi parmi 0, 1, 2, 3 et 4.

2. Composé de la revendication 1 ou sel acceptable pharmaceutiquement de celui-ci, dans lequel
X³ est CH, A est N, B est CH, Z est N, Y¹, Y² et Y³ sont CH, représenté par la formule (II), 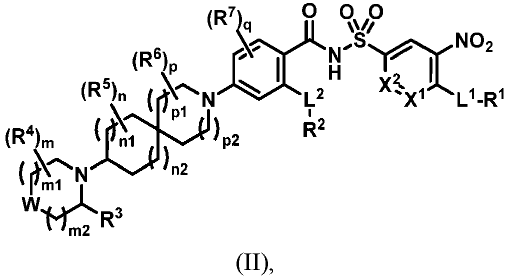 où
X¹ est choisi parmi N et CR⁸, X² est choisi parmi N et CR⁹, où au moins l'un de X¹ et X² est N ; W est choisi parmi -CHR¹¹-, -NR¹²-, -O- et -S(O)ᵣ- ; L¹, L², R¹, R², R³, R⁴, R⁵, R⁶, R⁷, R⁸, R⁹, R¹¹, R¹², m, m1, m2, n, n1, n2, p, p1, p2 et q sont définis comme dans la formule (I).

3. Composé de l'une quelconque des revendications 1 et 2, ou sel acceptable pharmaceutiquement de celui-ci, dans lequel
(i) R¹ est choisi parmi 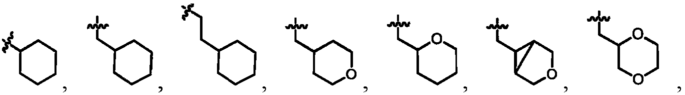 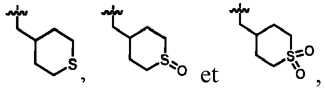 qui sont non substitués ou substitués par au moins un substituant, indépendamment choisi parmi R^{X} ;
et/ou
(ii) chaque substituant R^{X} de R¹ est indépendamment choisi parmi un alkyle en C₁₋₁₀, un cycloalkyle en C₃₋₁₀, un halogène, CN, NO₂, -(CR^{c1}R^{d1})ₜNR^{a1}R^{b1}, -(CR^{c1}R^{a1})ₜOR^{b1}, - (CR^{c1}R^{d1})ₜC(O)R^{a1} et -(CR^{c1}R^{d1})ₜN=S(O)R^{a1}R^{b1}, où l'alkyle, le cycloalkyle sont chacun non substitués ou substitués par au moins un substituant, indépendamment choisi parmi R^{Y} ;
par exemple,
où chaque R^{a1} et chaque R^{b1} sont indépendamment choisis parmi un hydrogène, un alkyle en C₁₋₁₀, un alcényle en C₂₋₁₀ et un alcynyle en C₂₋₁₀, où l'alkyle, l'alcényle et l'alcynyle sont chacun non substitués ou substitués par au moins un substituant, indépendamment choisi parmi R^{Y} ;
ou
où R^{a1} et R^{b1} conjointement avec le ou les atomes auxquels ils sont liés forment un cycle hétérocyclique à 4 à 8 chaînons contenant 0, 1 ou 2 hétéroatomes supplémentaires indépendamment choisis parmi l'oxygène, le soufre, l'azote et le phosphore, et éventuellement substitué par 1, 2 ou 3 groupes R^{Y} ;
ou
chaque substituant R^{X} de R¹ est indépendamment choisi parmi OH, CN, un halogène, un alkyle en C₁₋₁₀, 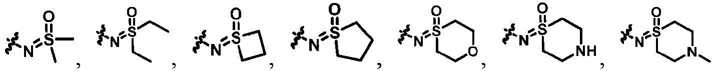 et

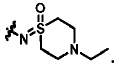

4. Composé de l'une quelconque des revendications 1 à 3, ou sel acceptable pharmaceutiquement de celui-ci, dans lequel
(i) R¹ est choisi parmi 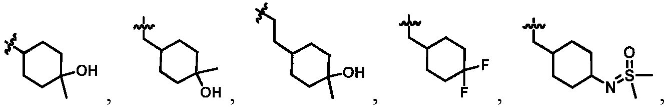 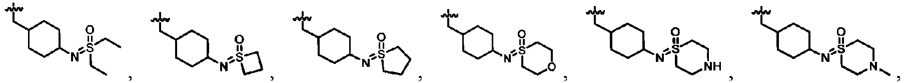 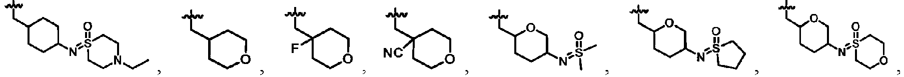 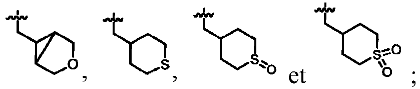 et/ou
(ii) R⁸ est un hydrogène.

5. Composé de l'une quelconque des revendications 1 à 4, ou sel acceptable pharmaceutiquement de celui-ci, dans lequel
R¹ et R⁸ conjointement avec les atomes auxquels ils sont liés forment un cycle hétérocyclique à 5 à 12 chaînons contenant 1, 2 ou 3 hétéroatomes indépendamment choisis parmi l'oxygène, le soufre et l'azote, et éventuellement substitués par 1, 2 ou 3 groupes R^{X} ;
par exemple,
où le fragment 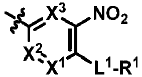 de la formule (I) et le fragment 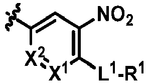 de la formule (II) sont choisis parmi 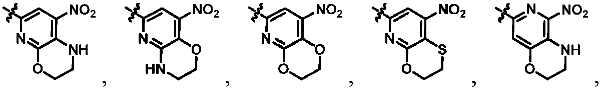 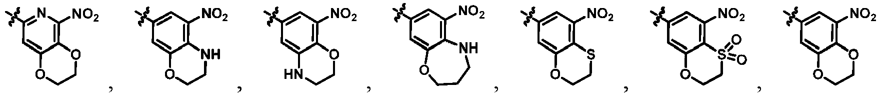 et 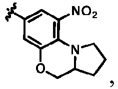 où le cycle hétérocyclique formé par R¹ et R⁸ est éventuellement substitué par 1, 2 ou 3 groupes R^{X}, où le symbole 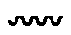 indique le point de liaison au reste de la molécule.

6. Composé de la revendication 5, ou sel acceptable pharmaceutiquement de celui-ci, dans lequel
chaque substituant R^{X} du cycle hétérocyclique à 5 à 12 chaînons est indépendamment choisi parmi un alkyle en C₁₋₁₀, un cycloalkyle en C₃₋₁₀, un cycloalkyle en C₃₋₁₀-alkyle en C₁₋₄, un hétérocyclyle, un hétérocyclyle-alkyle en C₁₋₄, un aryle, un aryle-alkyle en C₁₋₄, un hétéroaryle, un hétéroaryle-alkyle en C₁₋₄, un halogène, CN, NO₂, -(CR^{c1}R^{d1})ₜNR^{a1}R^{b1} et -(CR^{c1}R^{d1})ₜOR^{b1}, où l'alkyle, le cycloalkyle, l'hétérocyclyle, l'aryle et l'hétéroaryle sont chacun non substitués ou substitués par au moins un substituant, indépendamment choisi parmi R^{Y} ;
par exemple,
chaque substituant R^{X} du cycle hétérocyclique à 5 à 12 chaînons est choisi parmi un halogène, CN, NO₂, OH, un alkyle en C₁₋₈,

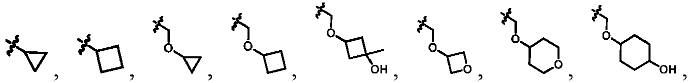

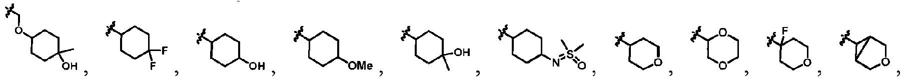

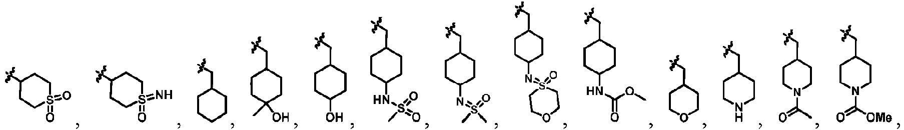

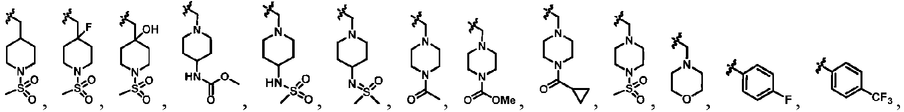

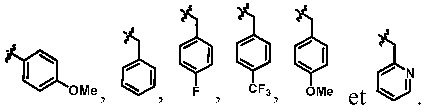

7. Composé de l'une quelconque des revendications 1 à 6, ou sel acceptable pharmaceutiquement de celui-ci, dans lequel
(i) chaque substituant R^{X} de R² est indépendamment choisi parmi un alkyle en C₁₋₁₀, un alcényle en C₂₋₁₀, un alcynyle en C₂₋₁₀, un cycloalkyle en C₃₋₁₀, un hétérocyclyle, un hétérocyclyle-alkyle en C₁₋₄, un halogène, CN, NO₂, -(CR^{c1}R^{d1})ₜNR^{a1}R^{b1}, -(CR^{c1}R^{d1})ₜOR^{b1}, - (CR^{c1}R^{a1})ₜC(O)OR^{b1}, -(CR^{c1}R^{d1})ₜC(O)NR^{a1}R^{b1}, -(CR^{c1}R^{d1})ₜNR^{a1}C(O)R^{b1}, - (CR^{c1}R^{a1})ₜNR^{a1}C(O)OR^{b1}, -(CR^{c1}R^{d1})ₜNR^{a1}C(O)NR^{a1}R^{b1}, -(CR^{c1}R^{a1})ₜS(O)₂OR^{b1}, - (CR^{c1}R^{d1})ₜS(O)ᵣR^{b1}, -(CR^{c1}R^{d1})ₜNR^{a1}S(O)ᵣR^{b1}, -(CR^{c1}R^{d1})ₜS(O)ᵣNR^{a1}R^{b1} et - (CR^{c1}R^{d1})ₜNR^{a1}S(O)₂NR^{a1}R^{b1}, où l'alkyle, l'alcényle, l'alcynyle, le cycloalkyle et l'hétérocyclyle sont chacun non substitués ou substitués par au moins un substituant, indépendamment choisi parmi R^{Y} ;
par exemple,
chaque substituant R^{x} de R² est choisi parmi un halogène, CN, NH₂, un méthyle, un trifluorométhyle, un cyclopropyle, un méthoxy, un méthoxyéthoxy et un difluorométhoxy ;
par exemple,
où R^{a1} et R^{b1} sont indépendamment choisis parmi un hydrogène, un alkyle en C₁₋₁₀, un cycloalkyle en C₃₋₁₀, un cycloalkyle en C₃₋₁₀-alkyle en C₁₋₄, un hétérocyclyle et un hétérocyclyle-alkyle en C₁₋₄, où l'alkyle, le cycloalkyle et l'hétérocyclyle sont chacun non substitués ou substitués par au moins un substituant, indépendamment choisi parmi R^{Y} ;
ou
(ii) R² est choisi parmi

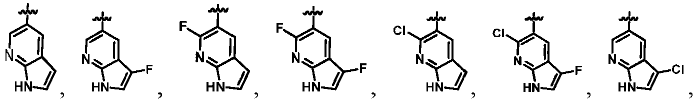

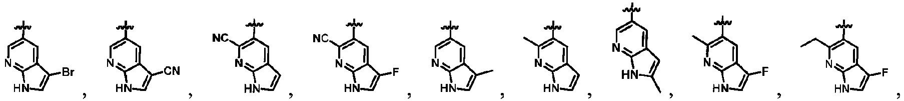

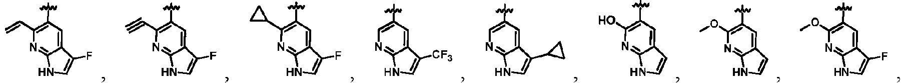

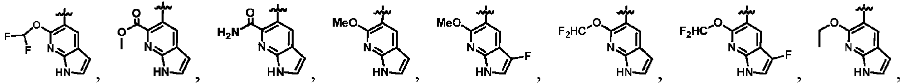

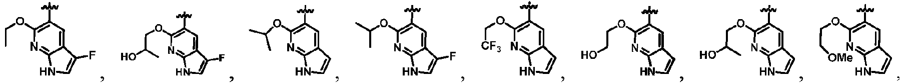

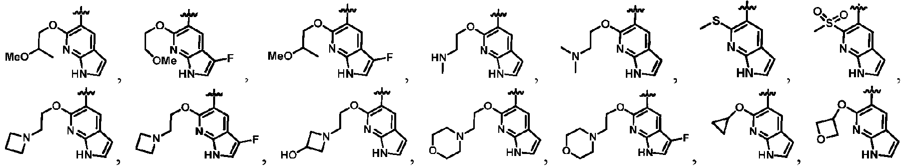

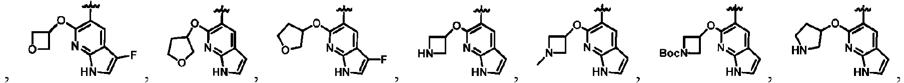

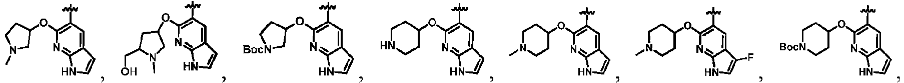

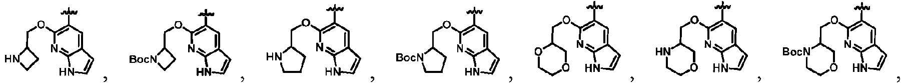

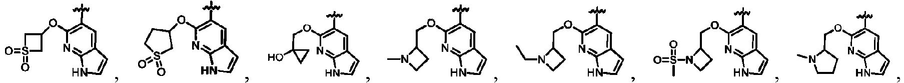

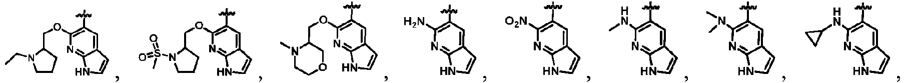

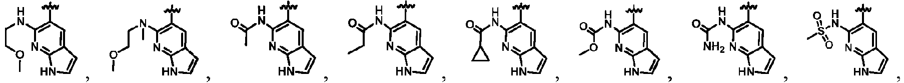

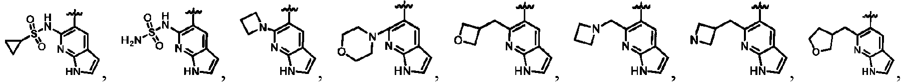

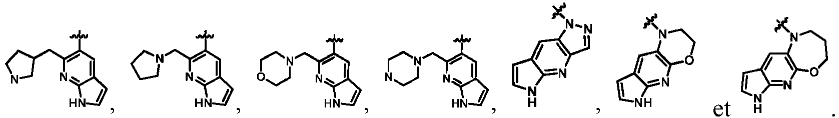

8. Composé de l'une quelconque des revendications 1 à 7, ou sel acceptable pharmaceutiquement de celui-ci, dans lequel
W est choisi parmi -CH₂-, -NR¹²-, -O-, -S(O)ᵣ- et -S(O)(=NR¹²)-.

9. Composé de l'une quelconque des revendications 1 à 8, ou sel acceptable pharmaceutiquement de celui-ci, dans lequel
(i) R¹¹ est un hydrogène ;
et/ou
(ii) R¹² est choisi parmi un hydrogène, un méthyle, un éthyle, un cyclopropyle, -C(O)CH₃, -C(O)OCH₃, -S(O)₂CH₃,

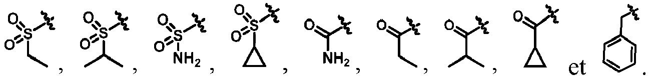

10. Composé de l'une quelconque des revendications 1 à 9, ou sel acceptable pharmaceutiquement de celui-ci, dans lequel
(i) chaque substituant R^{X} de R³ est choisi parmi un halogène, un alkyle en C₁₋₁₀, un alcényle en C₂₋₁₀, un cycloalkyle en C₃₋₁₀, où l'alkyle, l'alcényle et le cycloalkyle sont chacun non substitués ou substitués par au moins un substituant, indépendamment choisi parmi R^{Y} ;
par exemple,
chaque substituant R^{X} de R³ est choisi parmi un halogène, un méthyle, un éthyle, un isopropyle, un tert-butyle, un propényle et un cyclopropyle, où le méthyle, l'éthyle, l'isopropyle, le propényle et le cyclopropyle sont chacun non substitués ou substitués par au moins un substituant, indépendamment choisi parmi un halogène, un alkyle en C₁₋₁₀, CN, NO₂, -NH₂ et - OH ;
ou
(ii) R³ est choisi parmi 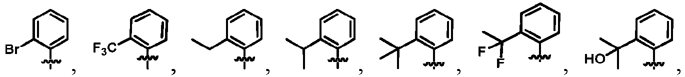 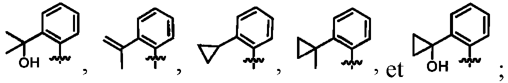 et/ou
(iii) m est choisi parmi 0, 1 et 2.

11. Composé de l'une quelconque des revendications 1 à 8 ou sel acceptable pharmaceutiquement de celui-ci, dans lequel le fragment 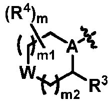 dans la formule (I) et la formule (II) est choisi parmi 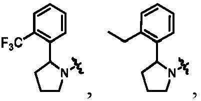 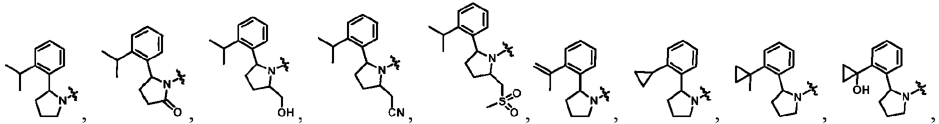 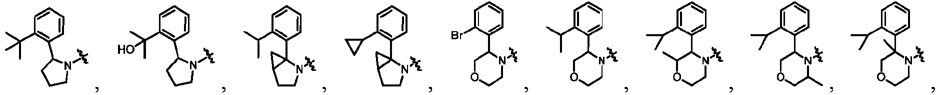 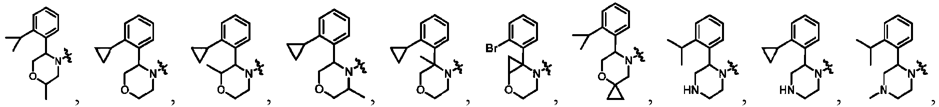 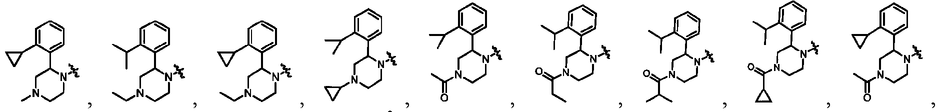 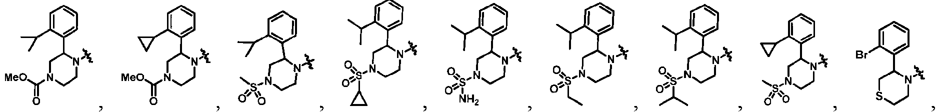 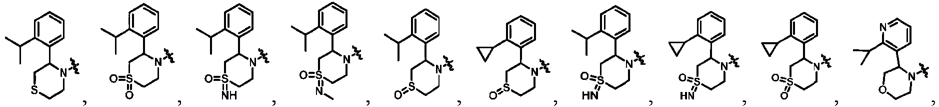 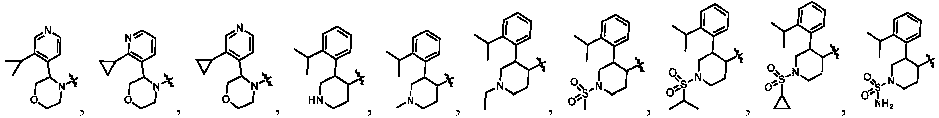 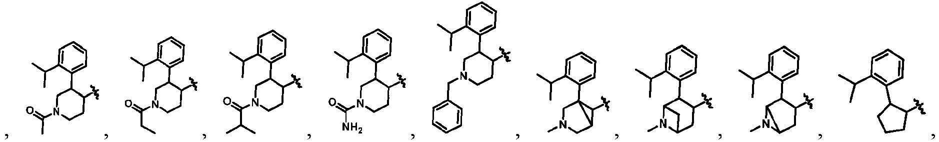 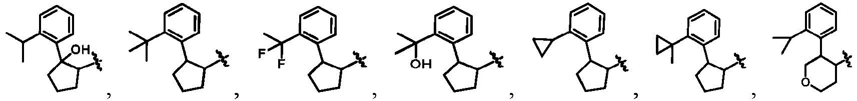 et 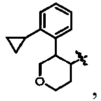 où le symbole 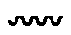 indique le point de liaison au reste de la molécule.

12. Composé de l'une quelconque des revendications 1 à 9, ou sel acceptable pharmaceutiquement de celui-ci, dans lequel
(i) R⁵ et R⁶ sont H ;
et/ou
(ii) le fragment 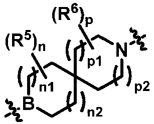 dans la formule (I) et la formule (II) est choisi parmi 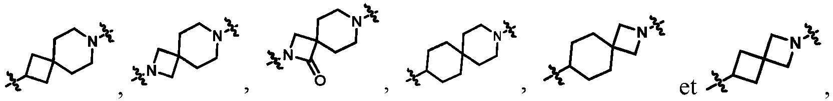 où le symbole 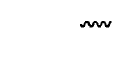 indique le point de liaison au reste de la molécule ;
et/ou
(iii) chaque R⁷ est un hydrogène.

13. Composé, ledit composé étant choisi parmi 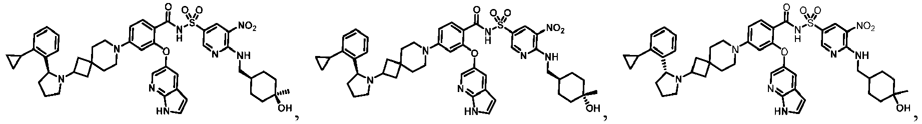 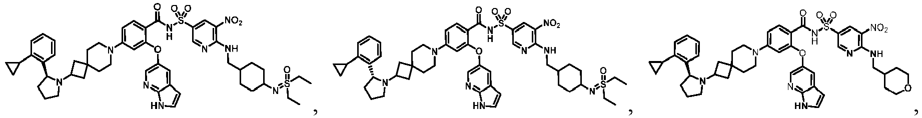 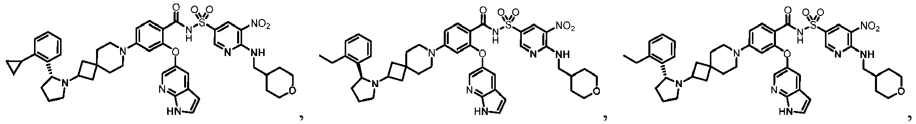 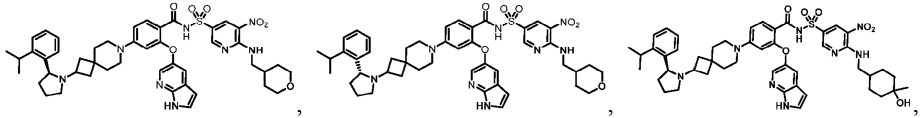 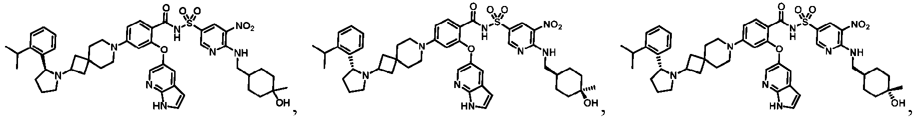 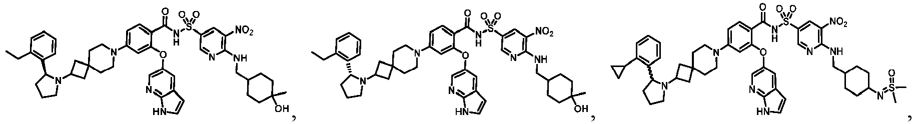 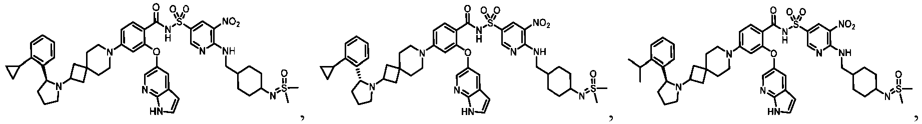 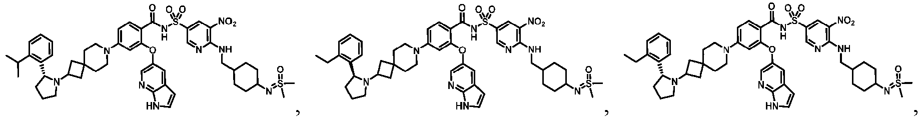 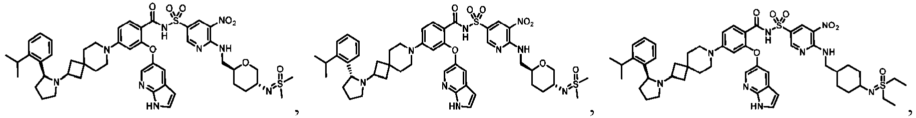 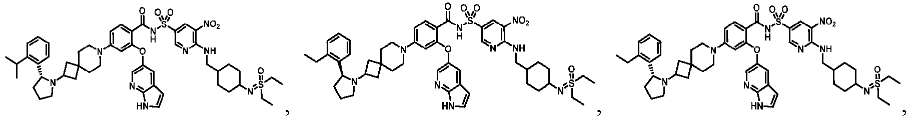 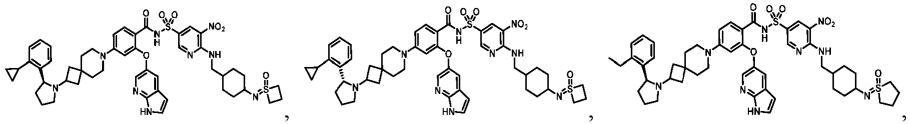 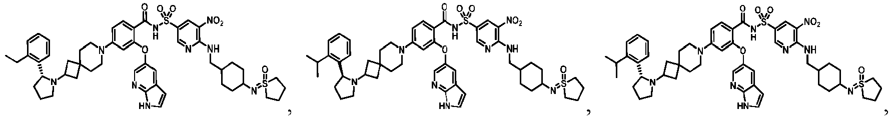 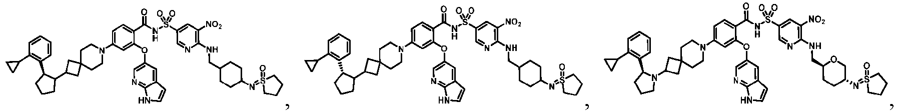 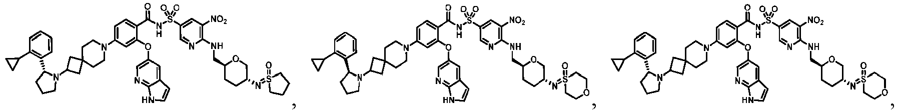 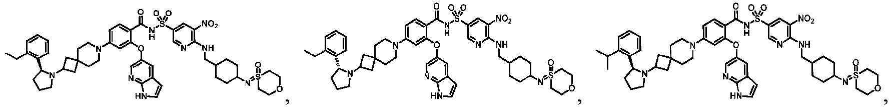 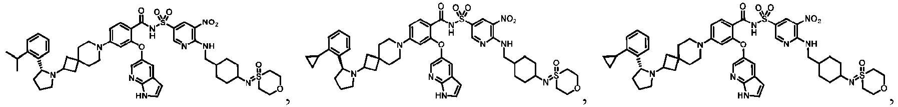 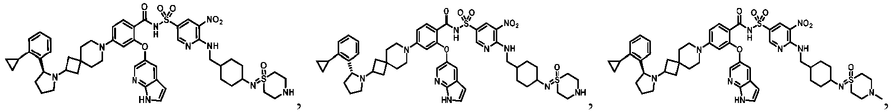 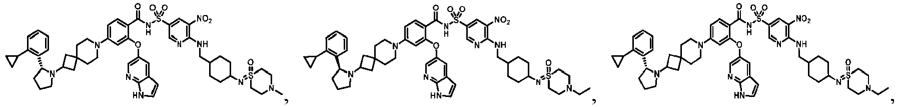 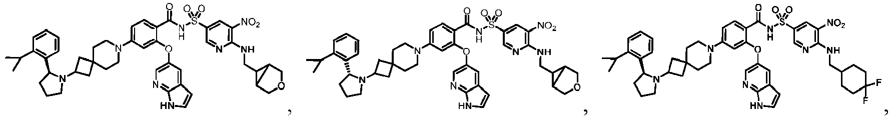 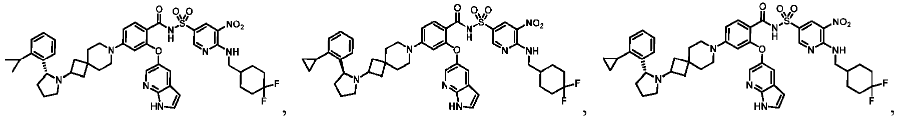 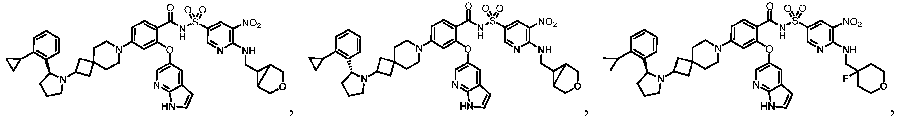 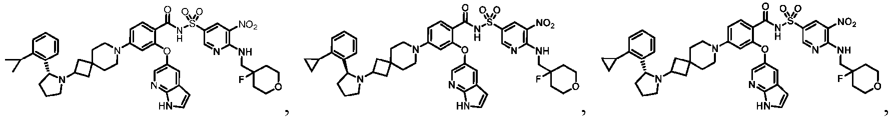 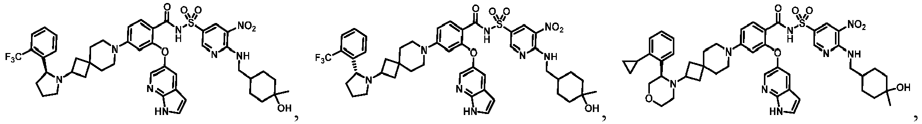 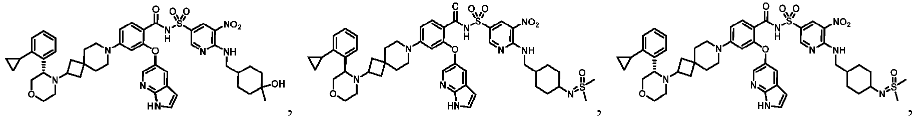 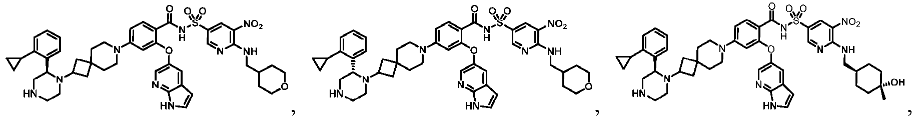 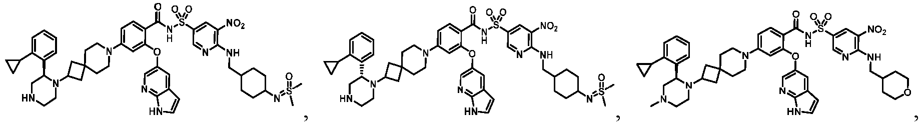 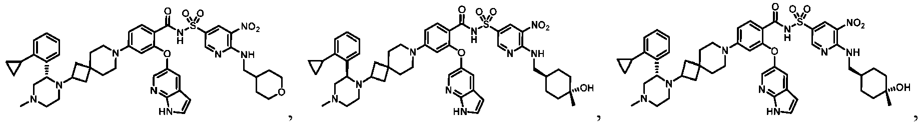 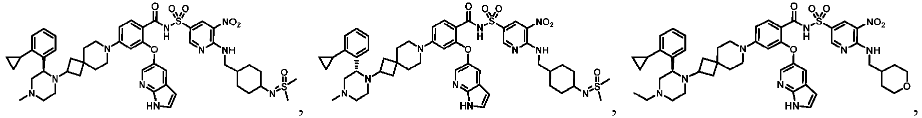 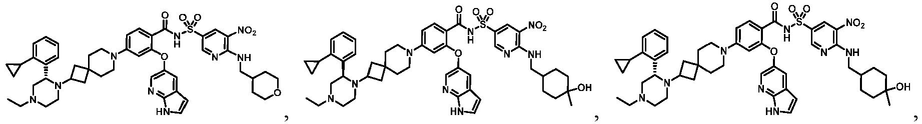 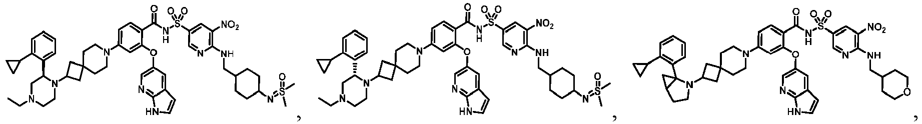 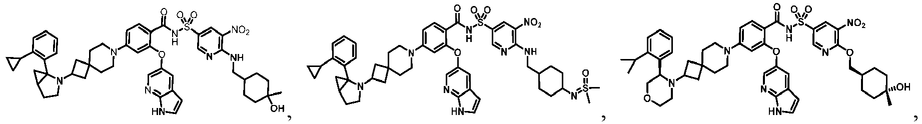 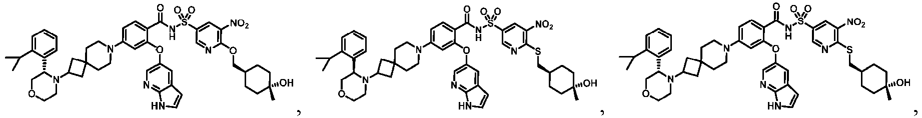 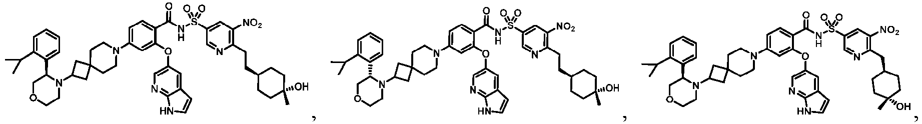 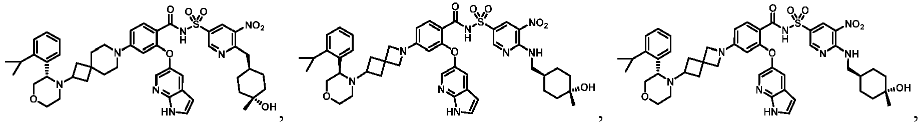 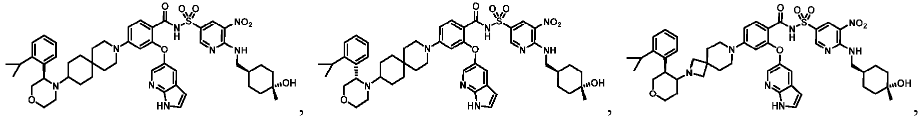 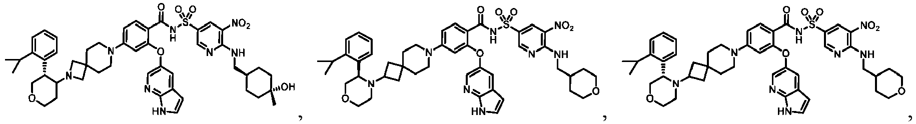 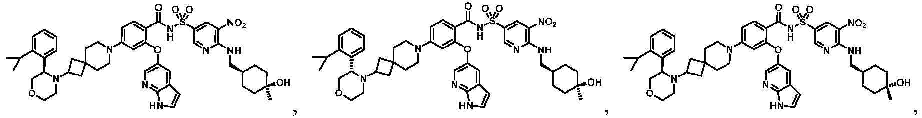 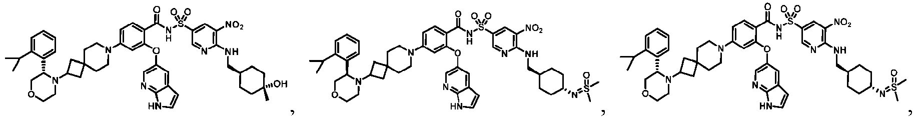 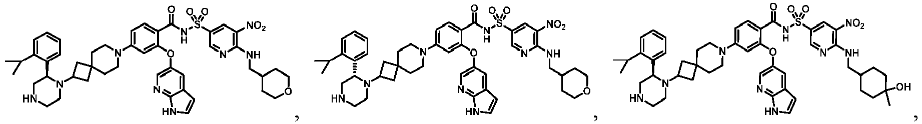 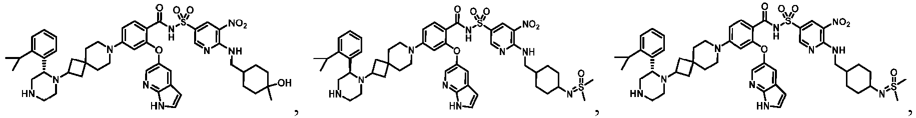 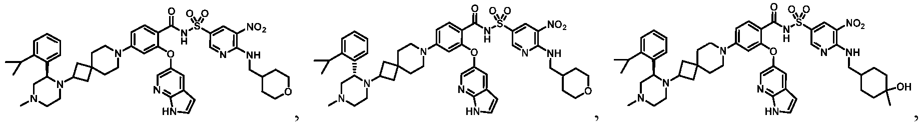 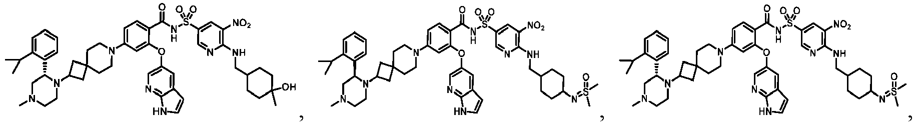 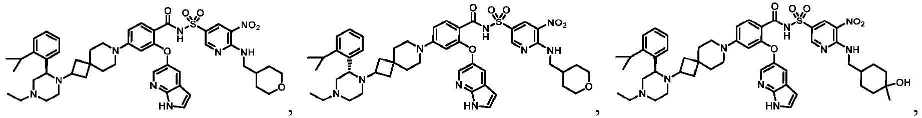 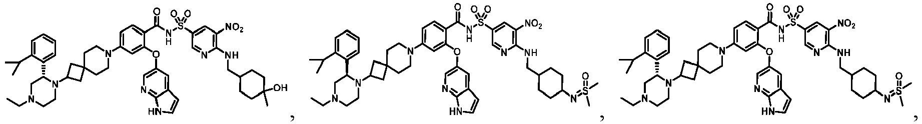 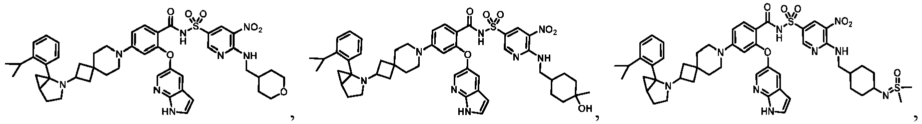 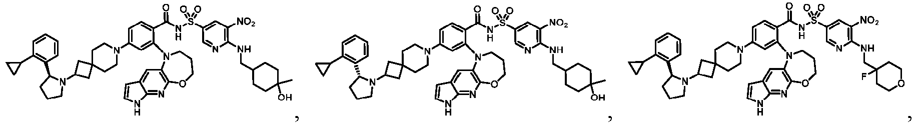 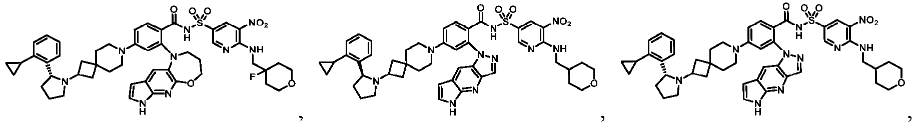 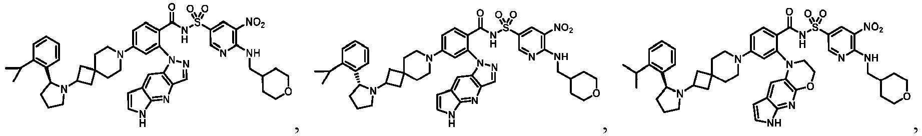 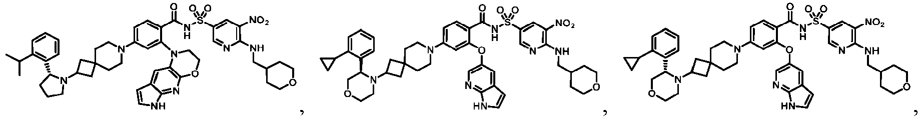 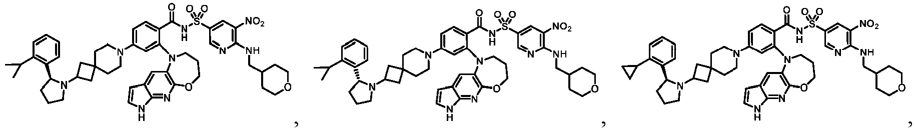 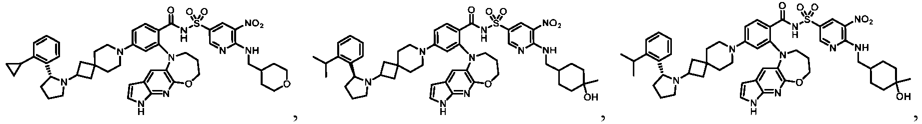 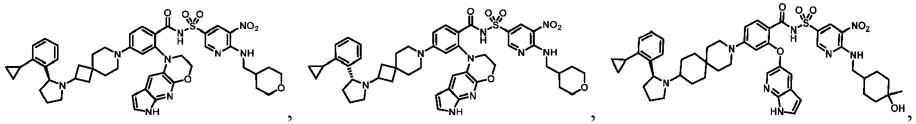 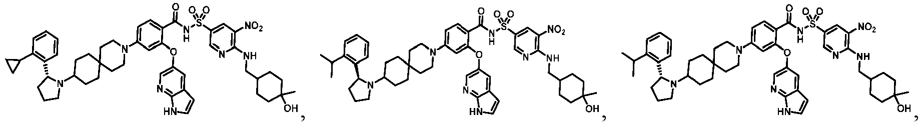 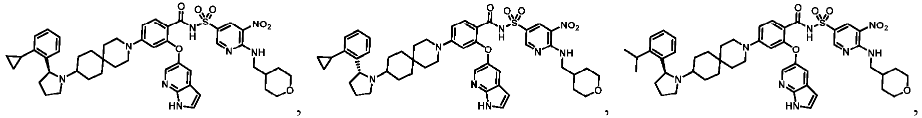 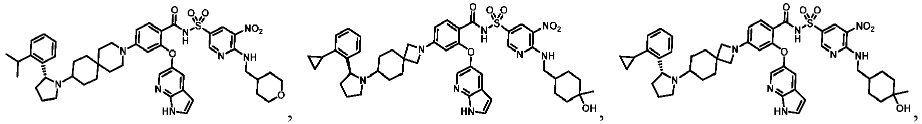 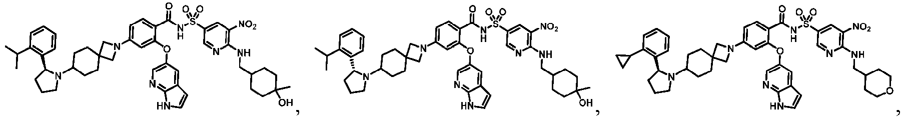 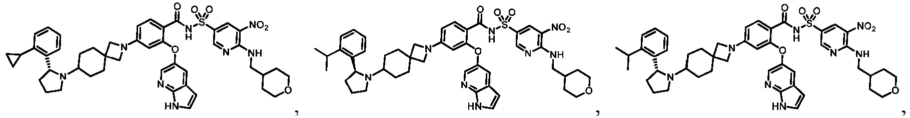 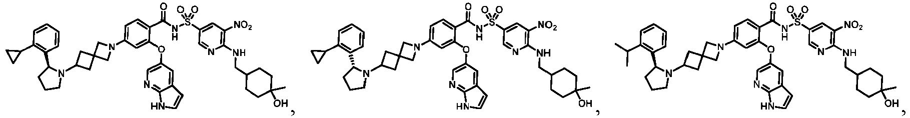 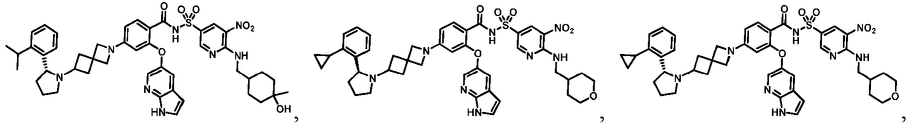 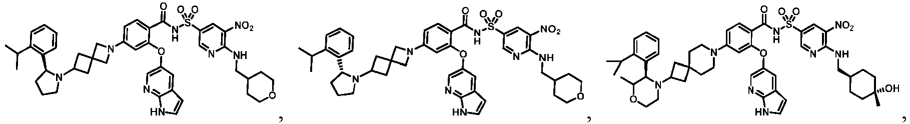 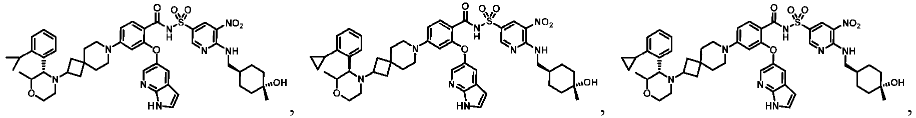 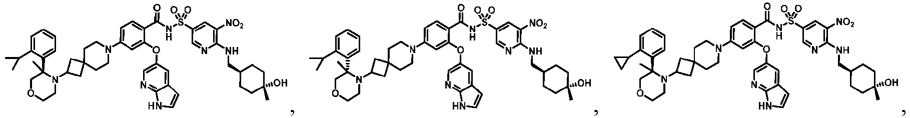 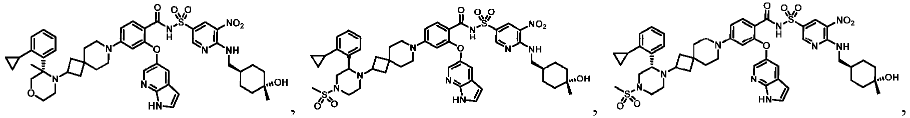 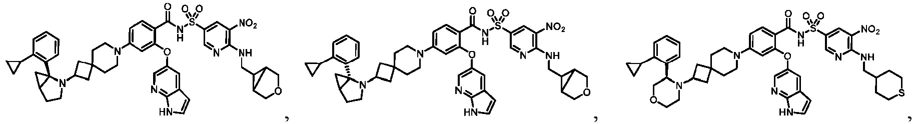 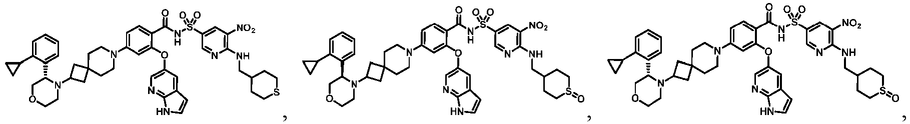 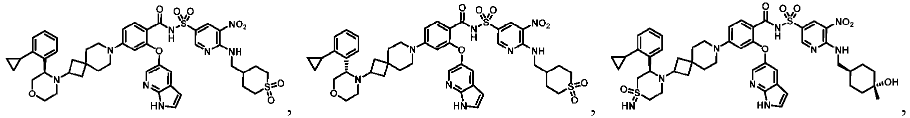 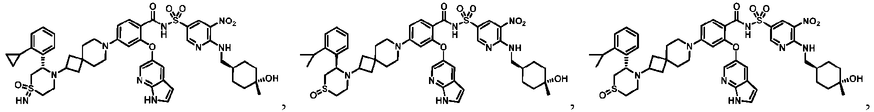 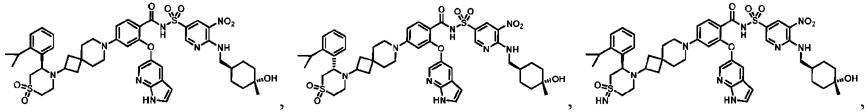 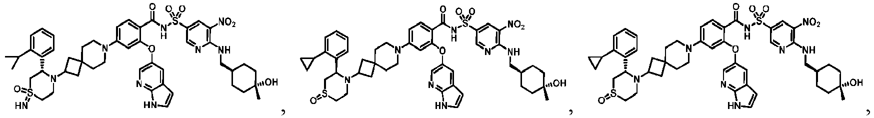 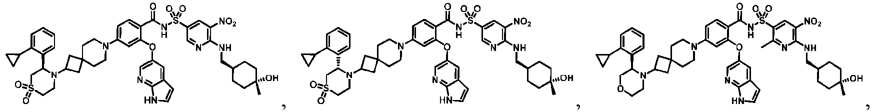 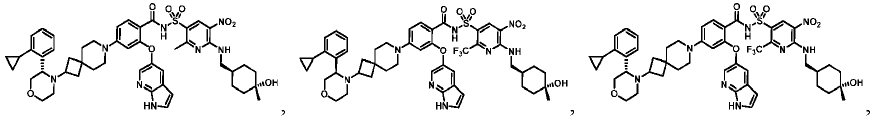 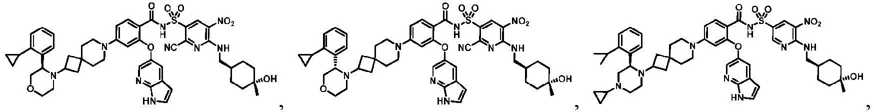 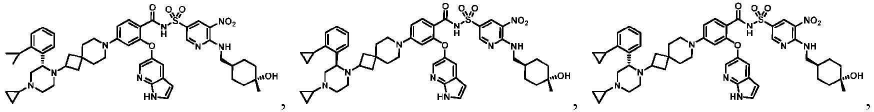 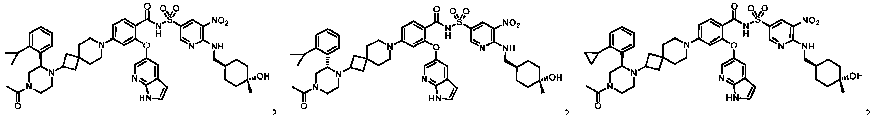 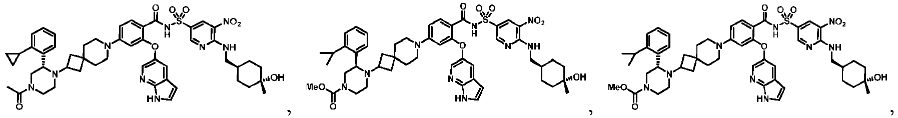 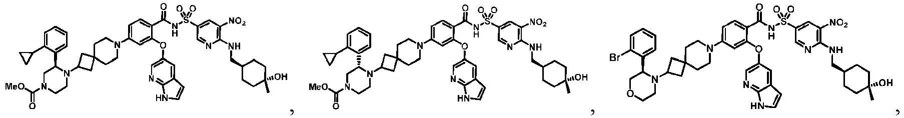 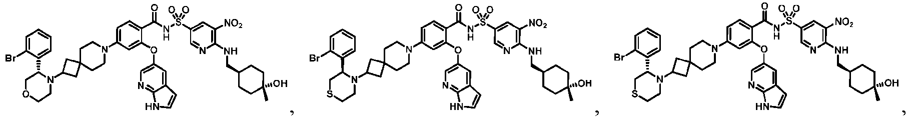 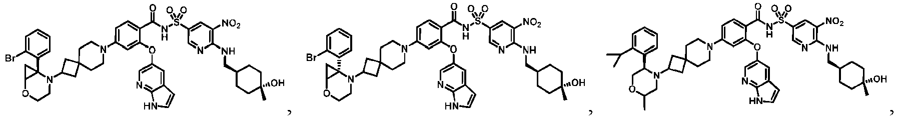 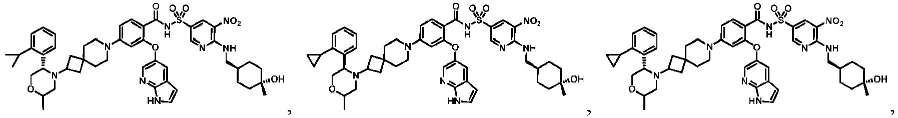 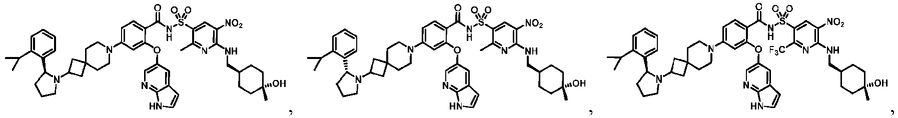 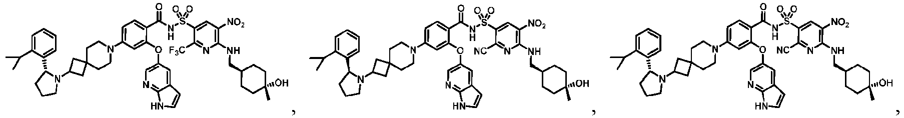 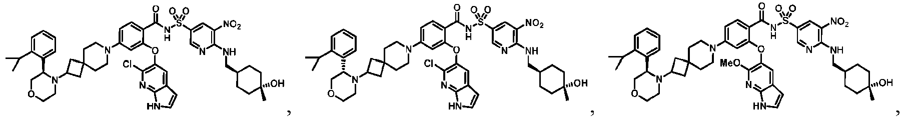 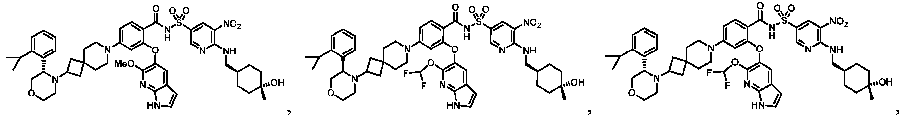 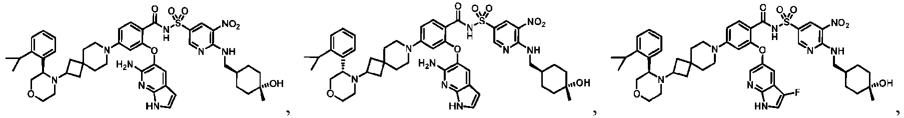 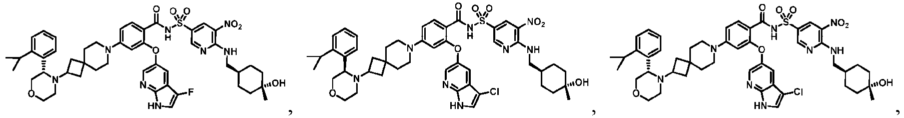 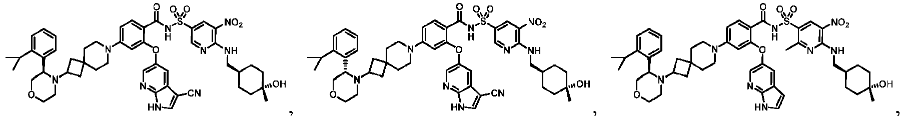 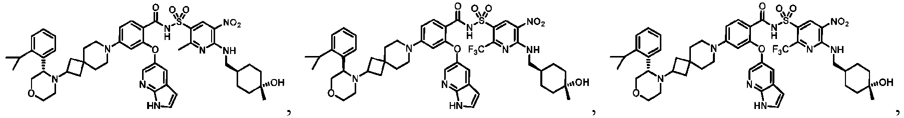 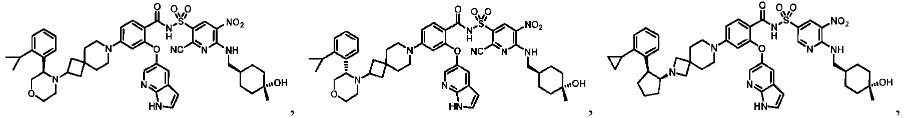 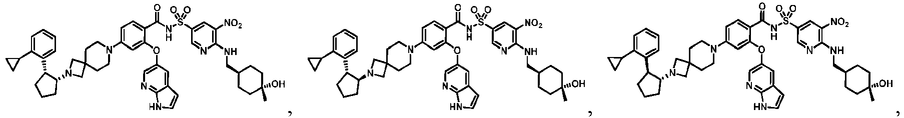 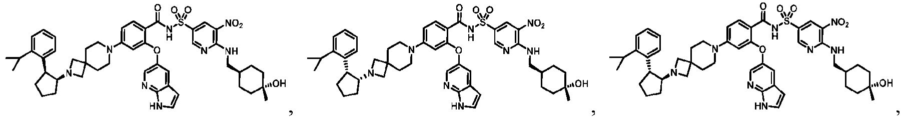 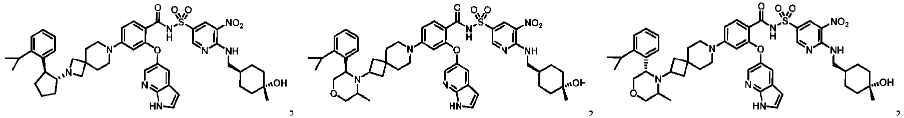 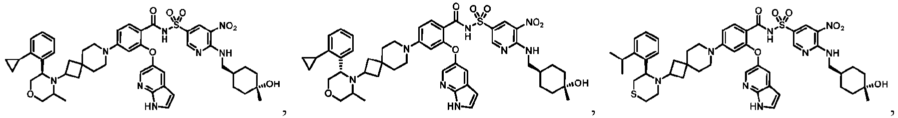 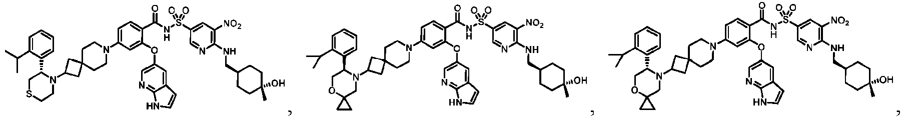 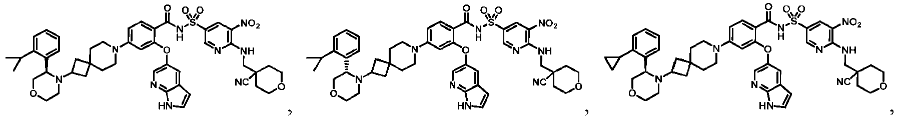 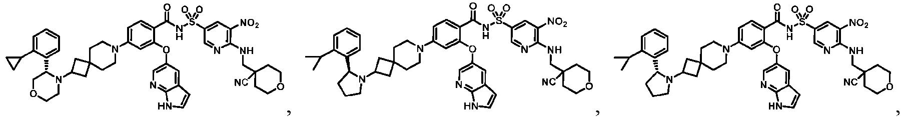 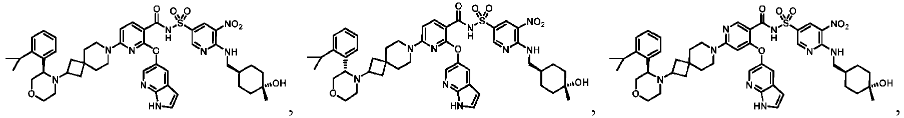 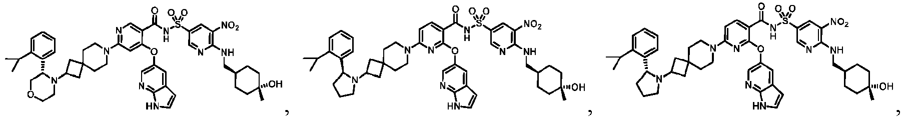 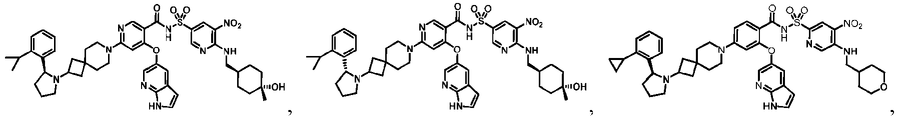 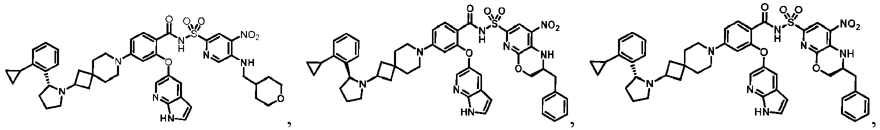 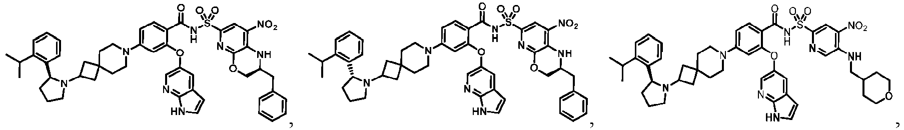 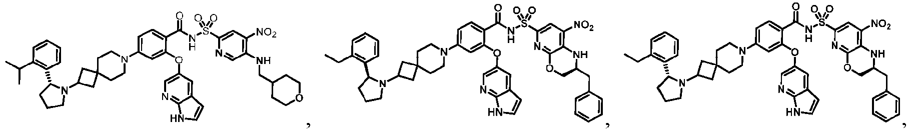 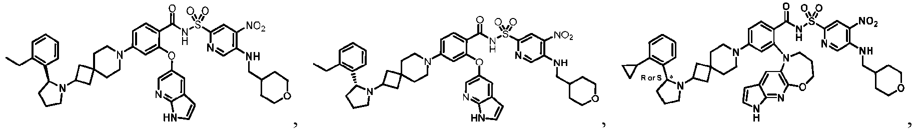 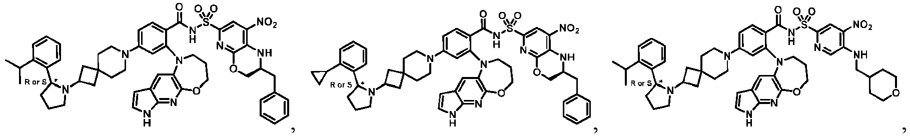 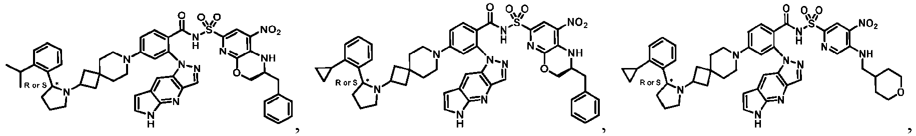 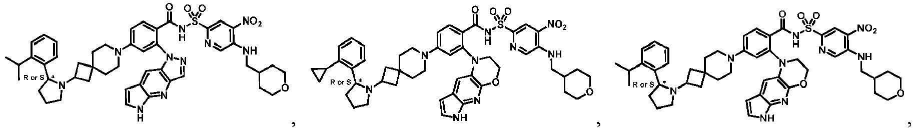 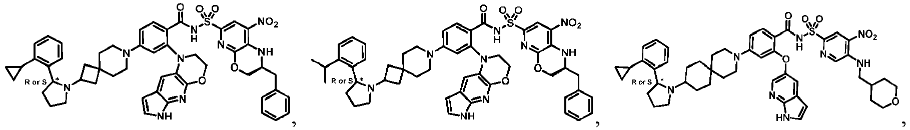 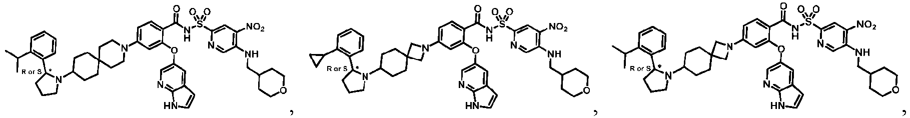 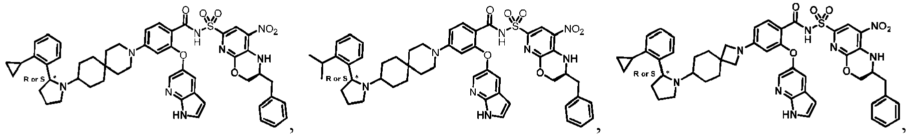 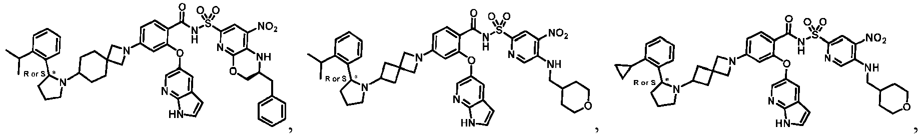 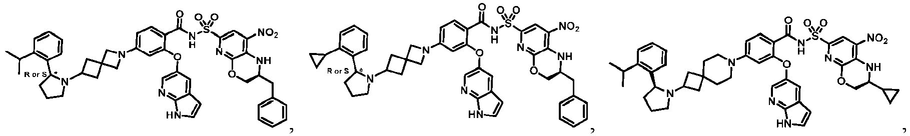 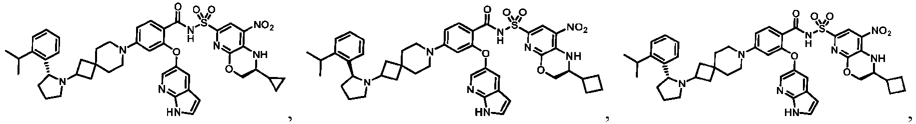 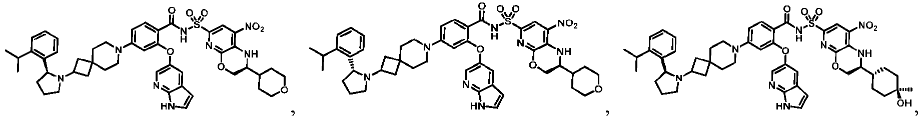 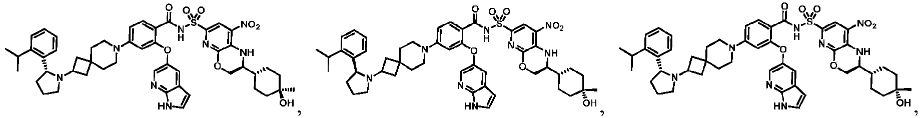 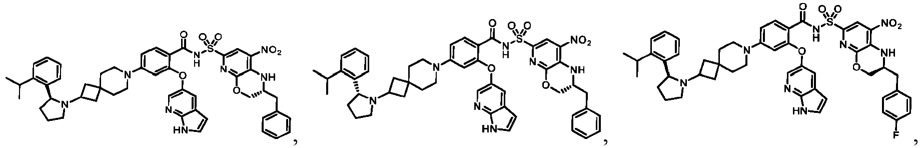 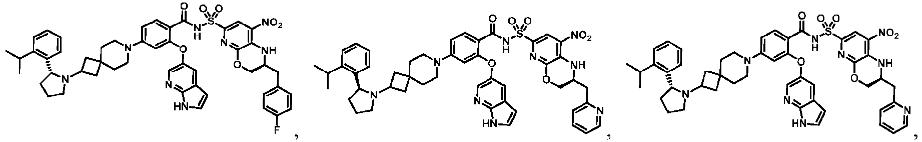 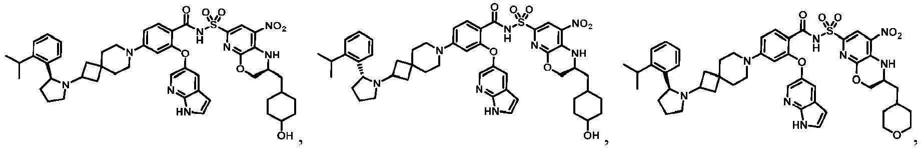 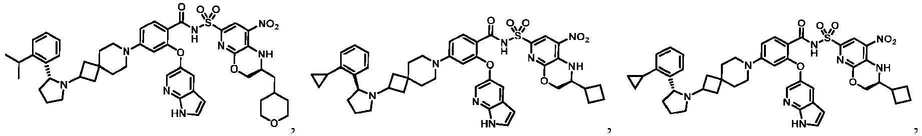 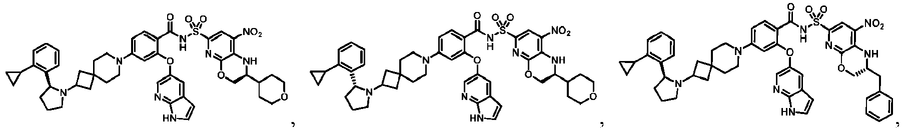 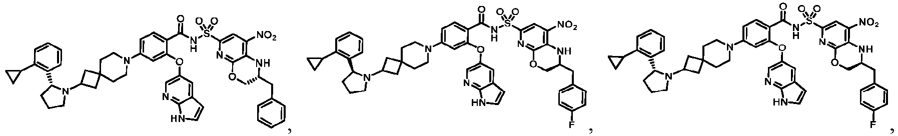 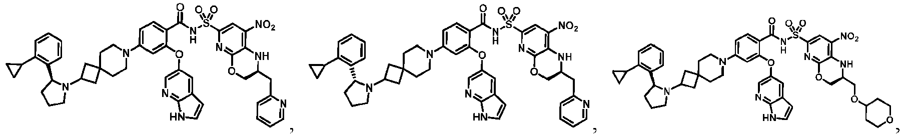 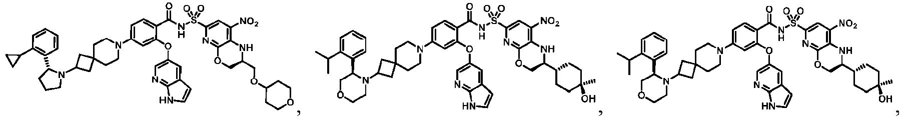 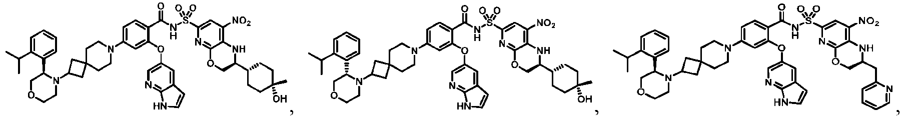 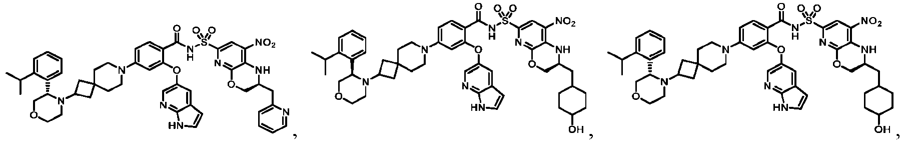 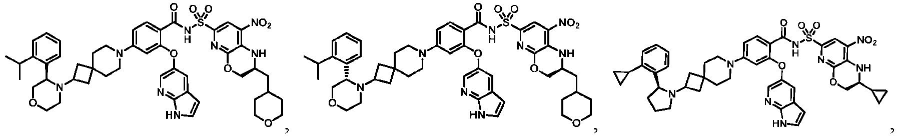 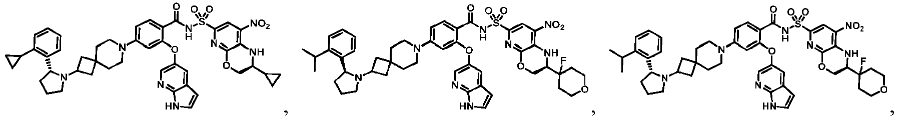 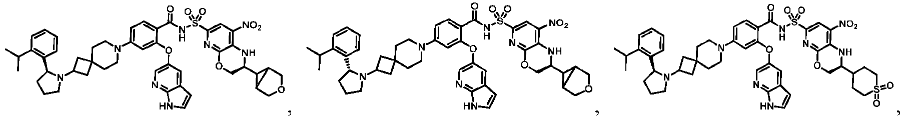 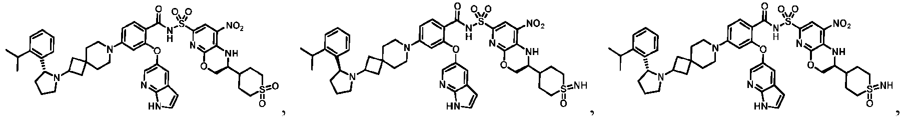 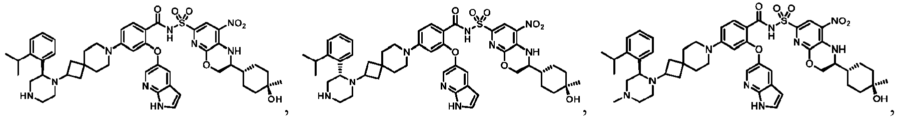 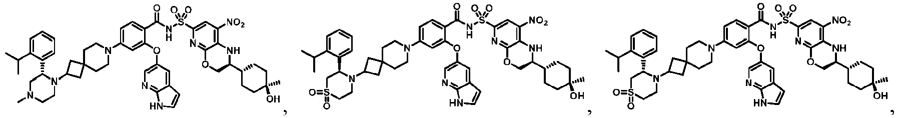 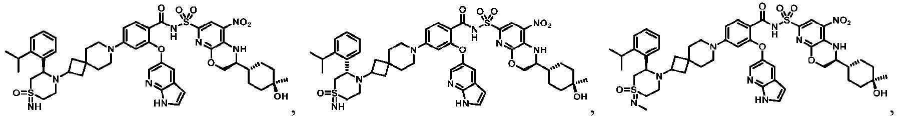 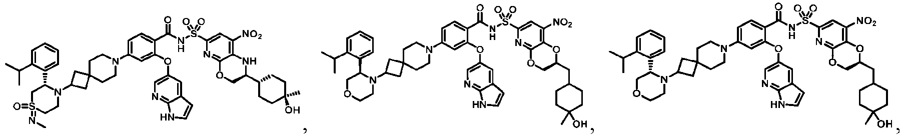 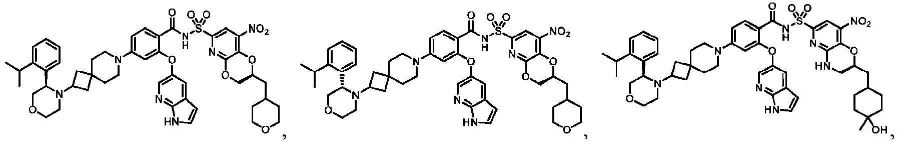 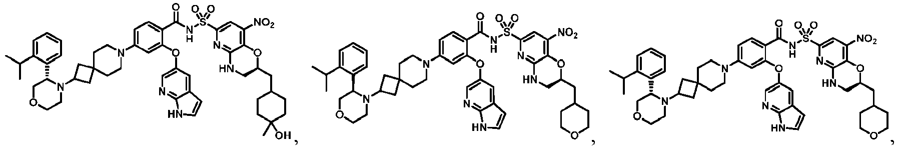 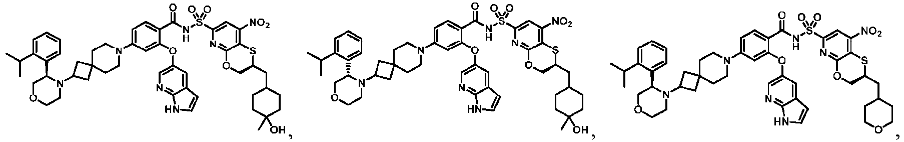 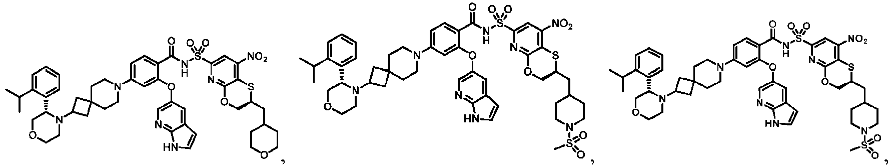 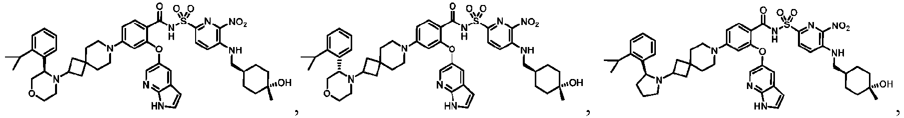 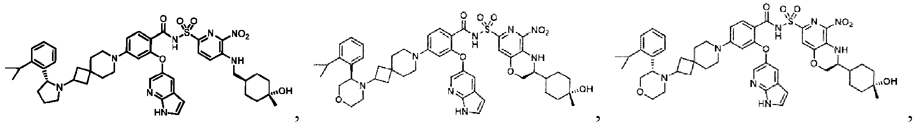 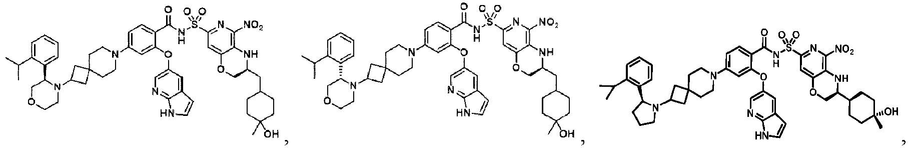 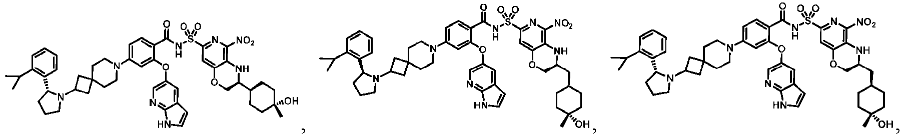 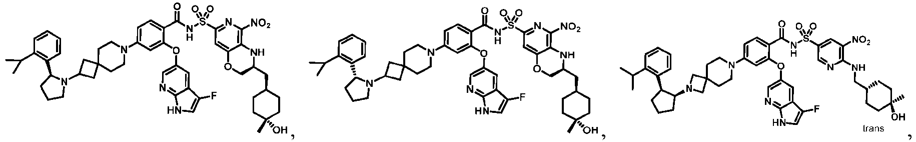 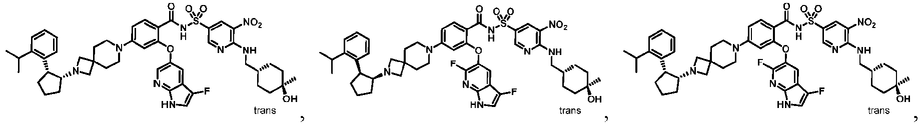 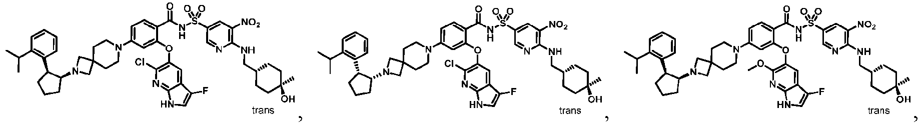 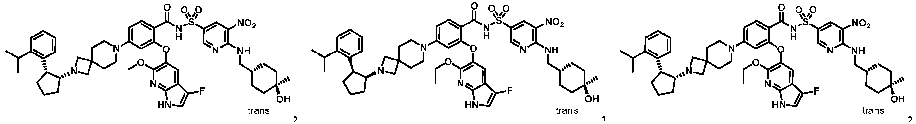 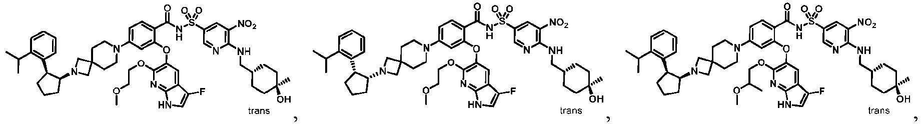 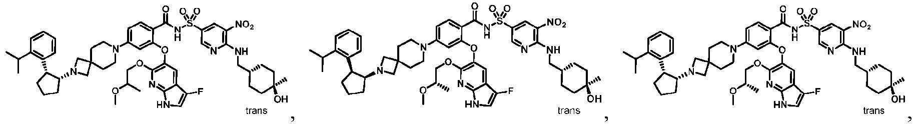 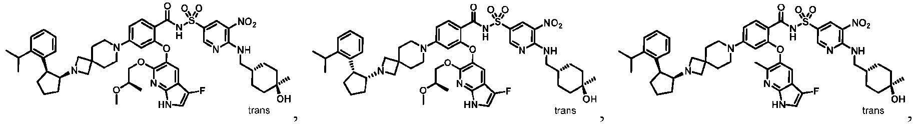 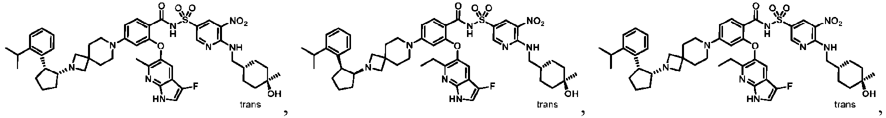 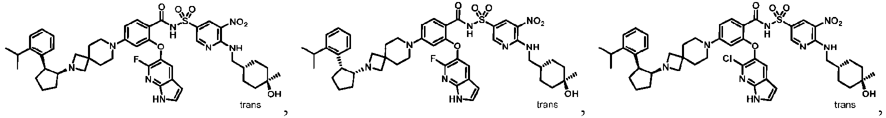 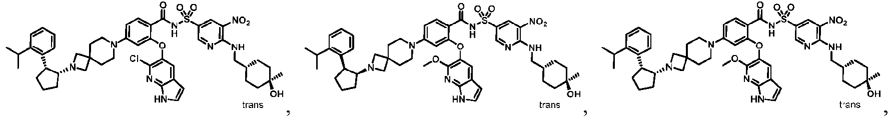 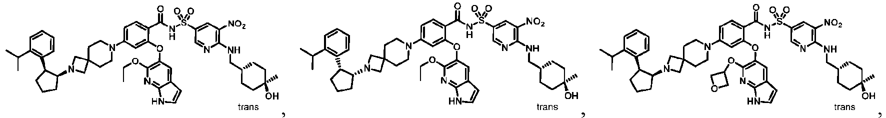 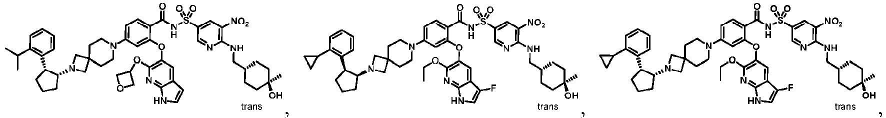 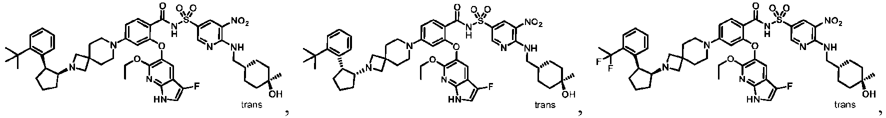 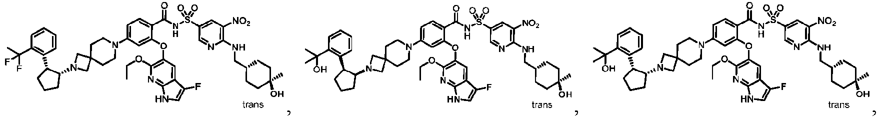 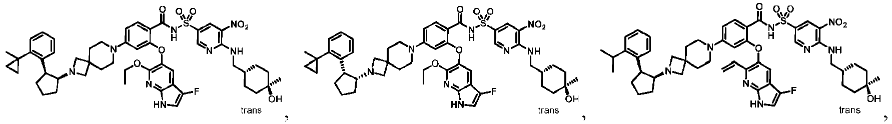 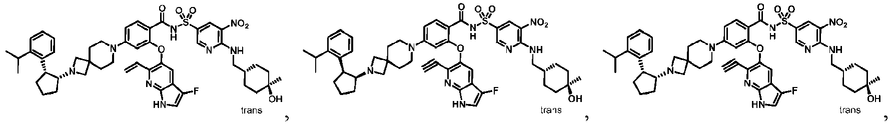 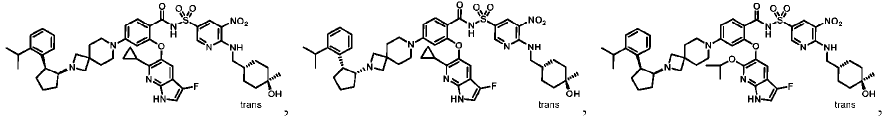 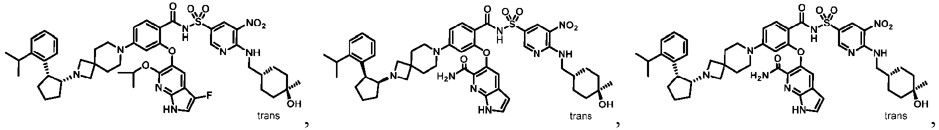 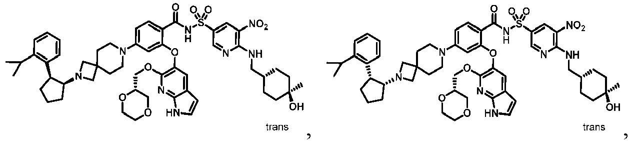 et des sels acceptables pharmaceutiquement de ceux-ci.

14. Composition pharmaceutique comprenant le composé de l'une quelconque des revendications 1 à 13 ou le sel acceptable pharmaceutiquement de celui-ci et au moins un support acceptable pharmaceutiquement.

15. Composé de l'une quelconque des revendications 1 à 13, ou sel acceptable pharmaceutiquement de celui-ci, ou composition pharmaceutique de la revendication 14, destiné(e) à être utilisé(e) dans le traitement d'un trouble de prolifération cellulaire ou d'une maladie auto-immune ;
par exemple, où
(i) le trouble de prolifération cellulaire est choisi parmi le cancer du sein, le cancer de l'ovaire, le cancer de la vessie, le cancer de l'utérus, le cancer de la prostate, le cancer des testicules, le cancer du poumon (par exemple, NSCLC, SCLC, le carcinome épidermoïde ou l'adénocarcinome), le cancer de l'œsophage, le cancer de la tête et du cou, le cancer colorectal, le cancer du rein (par exemple, RCC), le cancer du foie (par exemple, HCC), le cancer du pancréas, le cancer de l'estomac (c'est-à-dire, le cancer gastrique), le cancer de la thyroïde, la leucémie lymphoïde chronique (LLC), la leucémie lymphoblastique, le lymphome folliculaire, les tumeurs malignes lymphoïdes d'origine lymphocytaire T ou B, le mélanome, la leucémie myéloïde et le myélome ;
et/ou
(ii) la maladie auto-immune est choisie parmi les allergies, la maladie d'Alzheimer, l'encéphalomyélite aiguë disséminée, la maladie d'Addison, la spondylarthrite ankylosante, le syndrome des antiphospholipides, l'asthme, l'athérosclérose, l'anémie hémolytique auto-immune, les états hémolytiques et thrombocytopéniques auto-immuns, l'hépatite auto-immune, la maladie auto-immune de l'oreille interne, la pemphigoïde bulleuse, la maladie cœliaque, la maladie de Chagas, la bronchopneumopathie chronique obstructive, le purpura thrombocytopénique idiopathique chronique (PTI), le syndrome de Churg-Strauss, la maladie de Crohn, la dermatomyosite, le diabète sucré de type 1, l'endométriose, le syndrome de Goodpasture et la glomérulonéphrite et l'hémorragie pulmonaire associées, la maladie de Graves, le syndrome de Guillain-Barré, la maladie de Hashimoto, l'hidradénite suppurée, le purpura thrombocytopénique idiopathique, la cystite interstitielle, le syndrome de l'intestin irritable, le lupus érythémateux, la morphée, la sclérose en plaques, la myasthénie grave, la narcolepsie, la neuromyotonie, la maladie de Parkinson, le pemphigus vulgaire, l'anémie pernicieuse, la polymyosite, la cirrhose biliaire primitive, le psoriasis, l'arthrite psoriasique, la polyarthrite rhumatoïde, la schizophrénie, le choc septique, la sclérodermie, la maladie de Sjogren, le lupus érythémateux systémique et la glomérulonéphrite associée, l'artérite temporale, le rejet de greffe tissulaire et le rejet hyperaigu d'organes transplantés, la vasculite (par exemple, associée aux ANCA et autres vasculites), le vitiligo, et la granulomatose de Wegener.
